# EUROPEAN PATENT APPLICATION

(11) **EP 2 272 566 A2**
(43) Date of publication of application: **12.01.2011**
(21) Application number: 10185161.6
(22) Date of filing: 18.08.2004
(51) Int. Cl.: A61P 35/00

(54) **Humanisation of antibodies**

(30) Priority: 18.08.2003 US 496367 P
(62) Divisional of application: 04816814.0
(71) Applicant: MedImmune, LLC, Gaithersburg, MD 20878 (US)
(72) Inventor: Dall-Acqua, William, Gaithersburg, MD 20878 (US); Damschroder, Melissa, Germantown, MD 20874 (US); Wu, Herren, Boyds, MD 20841 (US)
(74) Representative: Bates, Rosica Florence

(57) **Abstract**

The present invention relates to methods of reengineering or reshaping antibodies to reduce the immunogenicity of the antibodies, while maintaining the immunospecificity of the antibodies for an antigen. In particular, the present invention provides methods of producing antibodies immunospecific for an antigen by synthesizing a combinatorial library comprising complementarity determining regions (CDRs) from a donor antibody fused in frame to framework regions from a sub-bank of framework regions. The present invention also provides antibodies produced by the methods of the invention.

## Description

### FIELD OF THE INVENTION

The present invention relates to methods of reengineering or reshaping antibodies to reduce the immunogenicity of the antibodies, while maintaining the immunospecificity of the antibodies for an antigen. In particular, the present invention provides methods of producing antibodies immunospecific for an antigen by synthesizing a combinatorial library comprising complementarity determining regions (CDRs) from a donor antibody fused in frame to framework regions from a sub-bank of framework regions. The present invention also provides antibodies produced by the methods of the invention.

### 1. BACKGROUND OF THE INVENTION

Antibodies play a vital role in our immune responses. They can inactivate viruses and bacterial toxins, and are essential in recruiting the complement system and various types of white blood cells to kill invading microorganisms and large parasites. Antibodies are synthesized exclusively by B lymphocytes, and are produced in millions of forms, each with a different amino acid sequence and a different binding site for an antigen. Antibodies, collectively called immunoglobulins (Ig), are among the most abundant protein components in the blood. Alberts et al., Molecular Biology of the Cell, 2nd ed., 1989, Garland Publishing, Inc.

A typical antibody is a Y-shaped molecule with two identical heavy (H) chains (each containing about 440 amino acids) and two identical light (L) chains (each containing about 220 amino acids). The four chains are held together by a combination of noncovalent and covalent (disulfide) bonds. The proteolytic enzymes, such as papain and pepsin, can split an antibody molecule into different characteristic fragments. Papain produces two separate and identical Fab fragments, each with one antigen-binding site, and one Fc fragment. Pepsin produces one F (ab')₂ fragment. Alberts et al., Molecular Biology of the Cell, 2nd ed., 1989, Garland Publishing, Inc.

Both L and H chains have a variable sequence at their amino-terminal ends but a constant sequence at their carboxyl-terminal ends. The L chains have a constant region about 110 amino acids long and a variable region of the same size. The H chains also have a variable region about 110 amino acids long, but the constant region of the H chains is about 330 or 440 amino acid long, depending on the class of the H chain. Alberts et al., Molecular Biology of the Cell, 2nd ed., 1989, Garland Publishing, Inc. at pp1019.

Only part of the variable region participates directly in the binding of antigen. Studies have shown that the variability in the variable regions of both L and H chains is for the most part restricted to three small hypervariable regions (also called complementarity-determining regions, or CDRs) in each chain. The remaining parts of the variable region, known as framework regions (FR), are relatively constant. Alberts et al., Molecular Biology of the Cell, 2nd ed., 1989, Garland Publishing, Inc. at pp 1019 - 1020.

Natural immunoglobulins have been used in assays, diagnosis and, to a more limited extent, therapy. However, such uses, especially in therapy, have been hindered by the polyclonal nature of natural immunoglobulins. The advent of monoclonal antibodies of defined specificity increased the opportunities for therapeutic use. However, most monoclonal antibodies are produced following immunization of a rodent host animal with the target protein, and subsequent fusion of a rodent spleen cell producing the antibody of interest with a rodent myeloma cell. They are, therefore, essentially rodent proteins and as such are naturally immunogenic in humans, frequently giving rise to an undesirable immune response termed the HAMA (Human Anti-Mouse Antibody) response.

Many groups have devised techniques to decrease the immunogenicity of therapeutic antibodies. Traditionally, a human template is selected by the degree of homology to the donor antibody, *i.e*., the most homologous human antibody to the non-human antibody in the variable region is used as the template for humanization. The rationale is that the framework sequences serve to hold the CDRs in their correct spatial orientation for interaction with an antigen, and that framework residues can sometimes even participate in antigen binding. Thus, if the selected human framework sequences are most similar to the sequences of the donor frameworks, it will maximize the likelihood that affinity will be retained in the humanized antibody. Winter (EP No. 0239400), for instance, proposed generating a humanized antibody by site-directed mutagenesis using long oligonucleotides in order to graft three complementarity determining regions (CDR1, CDR2 and CDR3) from each of the heavy and light chain variable regions. Although this approach has been shown to work, it limits the possibility of selecting the best human template supporting the donor CDRs.

Although a humanized antibody is less immunogenic than its natural or chimeric counterpart in a human, many groups find that a CDR grafted humanized antibody may demonstrate a significantly decreased binding affinity (*e.g*., Riechmann et al., 1988, Nature 332:323-327). For instance, Reichmann and colleagues found that transfer of the CDR regions alone was not sufficient to provide satisfactory antigen binding activity in the CDR-grafted product, and that it was also necessary to convert a serine residue at position 27 of the human sequence to the corresponding rat phenylalanine residue. These results indicated that changes to residues of the human sequence outside the CDR regions may be necessary to obtain effective antigen binding activity. Even so, the binding affinity was still significantly less than that of the original monoclonal antibody.

For example, Queen et al (U.S. Patent No. 5,530,101) described the preparation of a humanized antibody that binds to the interleukin-2 receptor, by combining the CDRs of a murine monoclonal (anti-Tac MAb) with human immunoglobulin framework and constant regions. The human framework regions were chosen to maximize homology with the anti-Tac MAb sequence. In addition, computer modeling was used to identify framework amino acid residues which were likely to interact with the CDRs or antigen, and mouse amino acids were used at these positions in the humanized antibody. The humanized anti-Tac antibody obtained was reported to have an affinity for the interleukin-2 receptor (p55) of 3 X 10⁹ M⁻¹, which was still only about one-third of that of the murine MAb.

Other groups identified further positions within the framework of the variable regions (*i.e*., outside the CDRs and structural loops of the variable regions) at which the amino acid identities of the residues may contribute to obtaining CDR-grafted products with satisfactory binding affinity. See, *e.g*., U.S. Patent Nos. 6,054,297 and 5,929,212. Still, it is impossible to know beforehand how effective a particular CDR grafting arrangement will be for any given antibody of interest.

Leung (U.S. Patent Application Publication No. US 2003/0040606) describes a framework patching approach, in which the variable region of the immunoglobulin is compartmentalized into FR1, CDR1, FR2, CDR2, FR3, CDR3 and FR4, and the individual FR sequence is selected by the best homology between the non-human antibody and the human antibody template. This approach, however, is labor intensive, and the optimal framework regions may not be easily identified.

As more therapeutic antibodies are being developed and are holding more promising results, it is important to be able to reduce or eliminate the body's immune response elicited by the administered antibody. Thus, new approaches allowing efficient and rapid engineering of antibodies to be human-like, and/or allowing a reduction in labor to humanize an antibody provide great benefits and medical value.

Citation or discussion of a reference herein shall not be construed as an admission that such is prior art to the present invention.

### 2. SUMMARY OF THE INVENTION

The invention is based, in part, on the synthesis of framework region sub-banks for the variable heavy chain framework regions and the variable light chain framework regions of antibodies and on the synthesis of combinatorial libraries of antibodies comprising a variable heavy chain region and/or a variable light chain region with the variable chain region(s) produced by fusing together in frame complementarity determining regions (CDRs) derived from a donor antibody and framework regions derived from framework region sub-banks. The synthesis of framework region sub-banks allows for the fast, less labor intensive production of combinatorial libraries of antibodies (with or without constant regions) which can be readily screened for their immunospecificity for an antigen of interest, as well as their immunogenicity in an organism of interest. The library approach described in the invention allows for efficient selection and identification of acceptor frameworks (*e.g*., human frameworks). In addition to the synthesis of framework region sub-banks, sub-banks of CDRs can be generated and randomly fused in frame with framework regions from framework region sub-banks to produce combinatorial libraries of antibodies (with or without constant regions) that can be screened for their immunospecificity for an antigen of interest, as well as their immunogenicity in an organism of interest. The combinatorial library methodology of the invention is exemplified herein for the production of humanized antibodies for use in human beings. However, the combinatorial library methodology of the invention can readily be applied to the production of antibodies for use in any organism of interest.

The present invention provides for a framework region sub-bank for each framework region of the variable light chain and variable heavy chain. Accordingly, the invention provides a framework region sub-bank for variable light chain framework region 1, variable light chain framework region 2, variable light chain framework region 3, and variable light chain framework region 4 for each species of interest and for each definition of a CDR (*e.g*., Kabat and Chothia). The invention also provides a framework region sub-bank for variable heavy chain framework region 1, variable heavy chain framework region 2, variable heavy chain framework region 3, and variable heavy chain framework region 4 for each species of interest and for each definition of a CDR (*e.g*., Kabat and Chothia). The framework region sub-banks may comprise framework regions from germline framework sequences and/or framework regions from functional antibody sequences. The framework region sub-banks may comprise framework regions from germline framework sequences and/or framework regions from functional antibody sequences into which one or more mutations have been introduced. The framework region sub-banks can be readily used to synthesize a combinatorial library of antibodies which can be screened for their immunospecificity for an antigen of interest, as well as their immunogencity in an organism of interest.

The present invention provides for a CDR sub-bank for each CDR of the variable light chain and variable heavy chain. Accordingly, the invention provides a CDR region sub-bank for variable light chain CDR1, variable light chain CDR2, and variable light CDR3 for each species of interest and for each definition of a CDR (*e.g*., Kabat and Chothia). The invention also provides a CDR sub-bank for variable heavy chain CDR1, variable heavy CDR2, and variable heavy chain CDR3 for each species of interest and for each definition of a CDR (*e.g*., Kabat and Chothia). The CDR sub-banks may comprise CDRs that have been identified as part of an antibody that immunospecifically to an antigen of interest. The CDR sub-banks can be readily used to synthesize a combinatorial library of antibodies which can be screened for their immunospecificity for an antigen of interest, as well as their immunogencity in an organism of interest.

The present invention provides a nucleic acid sequence comprising a nucleotide sequence encoding a heavy chain variable region and/or a nucleotide sequence encoding a light chain variable region with the variable region(s) produced by fusing together CDRs 1-3 derived from a donor antibody in frame with framework regions 1-4 from framework region sub-banks. In some embodiments, one or more of the CDRs derived from the donor antibody heavy and/or light chain variable region(s) contain(s) one or more mutations relative to the nucleic acid sequence encoding the corresponding CDR in the donor antibody. The present invention also provides a nucleic acid sequence comprising a nucleotide sequence encoding a heavy chain variable region and/or a nucleotide sequence encoding a light chain variable region with the variable region(s) produced by fusing together CDRs 1-3 derived from CDR sub-banks (preferably, sub-banks of CDRs that immunospecifically bind to an antigen of interest) in frame with framework regions 1-4 from framework region sub-banks.

In one embodiment, the present invention provides a nucleic acid sequence comprising a first nucleotide sequence encoding a heavy chain variable region (preferably, a humanized heavy chain variable region), said first nucleotide sequence encoding the heavy chain variable region produced by fusing together a nucleic acid sequence encoding a heavy chain framework region 1, a nucleic acid sequence encoding a heavy chain complementarity determining region (CDR) 1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, a nucleic acid sequence encoding a heavy chain CDR3, and a nucleic acid sequence encoding a heavy chain framework region 4, wherein the CDRs are derived from a donor antibody heavy chain variable region (preferably, a non-human donor antibody heavy chain variable region) and at least one heavy chain framework region is from a sub-bank of heavy chain framework regions (preferably, a sub-bank of human heavy chain framework regions). In accordance with this embodiment, the nucleic acid sequence may further comprise a second nucleotide sequence encoding a donor light chain variable region (preferably, a non-human donor light chain variable region). Alternatively, in accordance with this embodiment, the nucleic acid sequence may further comprise a second nucleotide sequence encoding a light chain variable region (preferably, a humanized light chain variable region), said second nucleotide sequence encoding the light chain variable region produced by fusing together a nucleic acid sequence encoding a light chain framework region 1, a nucleic acid sequence encoding a light chain CDR1, a nucleic acid sequence encoding a light chain framework region 2, a nucleic acid encoding a light chain CDR2, a nucleic acid sequence encoding a light chain framework region 3, a nucleic acid sequence encoding a light chain CDR3, and a nucleic acid sequence encoding a light chain framework region 4, wherein the CDRs are derived from a donor antibody light chain variable region (preferably, a non-human donor antibody light chain variable region) and at least one light chain framework region is from a sub-bank of light chain framework regions (preferably, sub-bank of human light chain framework regions).

In another embodiment, the present invention provides a nucleic acid sequence comprising a first nucleotide sequence encoding a light chain variable region (preferably, a humanized light chain variable region), said first nucleotide sequence encoding the light chain variable region produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the CDRs are derived from a donor antibody light chain variable region (preferably, a non-human donor antibody light chain variable region) and at least one light chain framework region is from a sub-bank of light chain framework regions (preferably, a sub-bank of human light chain framework regions). In accordance with this embodiment, the nucleic acid sequence may further comprise a second nucleotide sequence encoding a donor heavy chain variable region (preferably, a non-human donor heavy chain variable region).

In another embodiment, the present invention provides a nucleic acid sequence comprising a first nucleotide sequence encoding a heavy chain variable region (preferably, a humanized heavy chain variable region), said first nucleotide acid sequence encoding the heavy chain variable region produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, wherein at least one CDR is from a sub-bank of heavy chain CDRs derived from donor antibodies (preferably, non-human donor antibodies) and at least one heavy chain framework region is from a sub-bank of heavy chain framework regions (preferably, a sub-bank of human heavy chain framework regions). In accordance with this embodiment, the nucleic acid may further comprise a second nucleotide sequence encoding a donor light chain variable region (preferably, a non-human donor light chain variable region). Alternatively, in accordance with this embodiment, the nucleic acid sequence may further comprise a second nucleotide sequence encoding a light chain variable region (preferably, a humanized light chain variable region), said second nucleotide sequence encoding the light chain variable region produced by fusing together a nucleic acid sequence encoding a light chain framework region 1, a nucleic acid sequence encoding a light chain CDR1, a nucleic acid sequence encoding a light chain CDR2, a nucleic acid sequence encoding a light chain framework region 2, a nucleic acid sequence encoding a light chain framework region 3, a nucleic acid sequence encoding a light chain CDR3, and a nucleic acid sequence encoding a light chain framework region 4, wherein the CDRs are derived from a donor antibody light chain variable region (preferably, a non-human donor antibody light chain variable region) or at least one CDR is from a sub-bank of light chain CDRs derived from donor antibodies (preferably, non-human antibodies) and at least one light chain framework region is from a sub-bank of human light chain framework regions (preferably, a sub-bank of human light chain framework regions).

In another embodiment, the present invention provides a nucleic acid sequence comprising a first nucleotide sequence encoding a light chain variable region (preferably, a humanized light chain variable region), said first nucleotide sequence encoding the humanized light chain variable region produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one CDR is from a sub-bank of light chain CDRs derived from donor antibodies (preferably, non-human donor antibodies) and at least one light chain framework region is from a sub-bank of light chain framework regions (preferably, a sub-bank of human light chain framework regions). In accordance with this embodiment, the nucleic acid sequence may further comprise a second nucleotide sequence encoding a donor heavy chain variable region (preferably, a non-human heavy chain variable region). Alternatively, in accordance with this embodiment, the nucleic acid sequence may further comprise a second nucleotide sequence encoding a heavy chain variable region (preferably, a humanized heavy chain variable region), said second nucleotide sequence encoding the heavy chain variable region produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, wherein the CDRs are derived from a donor antibody heavy chain variable region (preferably, a non-human donor antibody heavy chain variable region) and at least one heavy chain framework region is from a sub-bank of heavy chain framework regions (preferably, a sub-bank of human heavy chain framework regions).

The present invention also provides cells comprising, containing or engineered to express the nucleic acid sequences described herein. In one embodiment, the present invention provides a cell comprising a first nucleic acid sequence comprising a first nucleotide sequence encoding a heavy chain variable region (preferably, a humanized heavy chain variable region), said cell produced by the process comprising introducing into a cell a nucleic acid sequence comprising a nucleotide sequence encoding a heavy chain variable region (preferably, a humanized heavy chain variable region) synthesized by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, wherein the CDRs are derived from a donor antibody heavy chain variable region (preferably, a non-human donor antibody heavy chain variable region) and at least one heavy chain framework region is from a sub-bank of heavy chain framework regions (preferably, a sub-bank of human heavy chain framework regions). In accordance with this embodiment, the cell may further comprise a second nucleic acid sequence comprising a second nucleotide sequence encoding a light chain variable region (preferably, a humanized or human light chain variable region).

In another embodiment, the present invention provides a cell comprising a first nucleic acid sequence comprising a first nucleotide sequence encoding a light chain variable region (preferably, a humanized light chain variable region), said cell produced by the process comprising introducing into a cell a nucleic acid sequence comprising a nucleotide sequence encoding a light chain variable region (preferably, a humanized light chain variable region) synthesized by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the CDRs are derived from a donor antibody light chain variable region (preferably, a non-human donor antibody light chain variable region) and at least one light chain framework region is from a sub-bank of light chain framework regions (preferably, a sub-bank of human light chain framework regions). In accordance with this embodiment, the cell may further comprise a second nucleic acid sequence comprising a second nucleotide sequence encoding a heavy chain variable region (preferably, a human or humanized heavy chain variable region).

In another embodiment, the present invention provides a cell comprising a nucleic acid sequence comprising a first nucleotide sequence encoding a heavy chain variable region (preferably, a humanized heavy chain variable region) and a second nucleotide sequence encoding a light chain variable region (preferably, a humanized light chain variable region), said cell produced by the process comprising introducing into a cell a nucleic acid sequence comprising: (i) a first nucleotide sequence encoding a heavy chain variable region synthesized by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4; and (ii) a second nucleotide sequence encoding a light chain variable region synthesized by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the CDRs of the heavy chain variable region are derived from a donor antibody heavy chain variable region (preferably, a non-human donor antibody heavy chain variable region), the CDRs of the light chain variable region are derived from a donor light chain variable region (preferably, a non-human donor light chain variable region), at least one heavy chain framework region is from a sub-bank of heavy chain framework regions (preferably, a sub-bank of human heavy chain framework regions), and at least one light chain framework region is from a sub-bank of light chain framework regions (preferably, a sub-bank of human light chain framework regions).

In another embodiment, the present invention provides a cell comprising a first nucleic acid sequence comprising a first nucleotide sequence encoding a heavy chain variable region (preferably, a humanized heavy chain variable region), said cell produced by the process comprising introducing into a cell a nucleic acid sequence comprising a nucleotide sequence encoding a heavy chain variable region synthesized by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, wherein at least one CDR is from a sub-bank of heavy chain CDRs derived from donor antibodies (preferably, non-human donor antibodies) and at least one heavy chain framework region is from a sub-bank of heavy chain framework regions (preferably, a sub-bank of human heavy chain framework regions). In accordance with this embodiment, the cell may further comprise a second nucleic acid sequence comprising a second nucleotide sequence encoding a light chain variable region (preferably, a humanized or human light chain variable region).

In another embodiment, the present invention provides a cell comprising a first nucleic acid sequence comprising a first nucleotide sequence encoding a light chain variable region (preferably, a humanized light chain variable region), said cell produced by the process comprising introducing into a cell a nucleic acid sequence comprising a nucleotide sequence encoding a light chain variable region synthesized by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one CDR is from a sub-bank of light chain CDRs derived from donor antibodies (preferably, non-human donor antibodies) and at least one light chain framework region is from a sub-bank of light chain framework regions (preferably, a sub-bank of human light chain framework regions). In accordance with this embodiment, the cell may further comprise a second nucleic acid sequence comprising a second nucleotide sequence encoding a heavy chain variable region (preferably, a humanized or human heavy chain variable region).

In another embodiment, the present invention provides a cell comprising a nucleic acid sequence comprising a first nucleotide sequence encoding a heavy chain variable region (preferably, a humanized heavy chain variable region) and a second nucleotide sequence encoding a light chain variable region (preferably, a humanized light chain region), said cell produced by the process comprising introducing into a cell a nucleic acid sequence comprising: (i) a first nucleotide sequence encoding a heavy chain variable region synthesized by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4; and (ii) a second nucleotide sequence encoding a light chain variable region synthesized by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one heavy chain variable region CDR is from a sub-bank of heavy chain CDRs derived from donor antibodies (preferably, non-human donor antibodies), at least one light chain variable region CDR is from a sub-bank of light chain CDRs derived from donor antibodies (preferably, non-human donor antibodies), at least one heavy chain framework region is from a sub-bank of heavy chain framework regions (preferably, a sub-bank of human heavy chain framework regions), and at least one light chain framework region is from a sub-bank of light chain framework regions (preferably, a sub-bank of human light chain framework regions).

In another embodiment, the present invention provides a cell comprising a nucleic acid sequence comprising a first nucleotide sequence encoding a heavy chain variable region (preferably, a humanized heavy chain variable region) and a second nucleotide sequence encoding a light chain variable region (preferably, a humanized light chain variable region), said cell produced by the process comprising introducing into a cell a nucleic acid sequence comprising: (i) a first nucleotide sequence encoding a heavy chain variable region synthesized by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4; and (ii) a second nucleotide sequence encoding a light chain variable region synthesized by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the heavy chain variable region CDRs are derived from a donor antibody heavy chain variable region (preferably, a non-human donor antibody heavy chain variable region), at least one light chain variable region CDR is from a sub-bank of light chain CDRs derived from donor antibodies (preferably, non-human donor antibodies), at least one heavy chain framework region is from a sub-bank of heavy chain framework regions (preferably, a sub-bank of human heavy chain framework regions), and at least one light chain framework region is from a sub-bank of light chain framework regions (preferably, a sub-bank of human light chain framework regions).

In another embodiment, the present invention provides a cell comprising a nucleic acid sequence comprising a first nucleotide sequence encoding a heavy chain variable region (preferably, a humanized heavy chain variable region) and a second nucleotide sequence encoding a light chain variable region (preferably, a humanized light chain variable region), said cell produced by the process comprising introducing into a cell a nucleic acid sequence comprising: (i) a first nucleotide sequence encoding a heavy chain variable region synthesized by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4; and (ii) a second nucleotide sequence encoding a light chain variable region synthesized by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one heavy chain variable region CDR is from a sub-bank of heavy chain CDRs derived from donor antibodies (preferably, non-human donor antibodies), the light chain variable region CDRs are derived from a donor antibody light chain variable region (preferably, a non-human donor antibody light chain variable region), at least one heavy chain framework region is from a sub-bank of heavy chain framework regions (preferably, a sub-bank of human heavy chain framework regions), and at least one light chain framework region is from a sub-bank of light chain framework regions (preferably, a sub-bank of human light chain framework regions).

The present invention provides a cell containing nucleic acid sequences encoding an antibody (preferably, a humanized antibody) that immunospecifically binds to an antigen, said cell produced by the process comprising: (a) introducing into a cell a nucleic acid sequence comprising a nucleotide sequence encoding a heavy chain variable region (preferably, a humanized heavy chain variable region), said first nucleotide sequence synthesized by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, wherein the CDRs are derived from a donor antibody heavy chain variable region (preferably, a non-human donor antibody heavy chain variable region) and at least one heavy chain framework region is from a sub-bank of heavy chain framework regions (preferably, a sub-bank of human heavy chain framework regions); and (b) introducing into a cell a nucleic acid sequence comprising a nucleotide sequence encoding a light chain variable region (preferably, a humanized light chain variable region), said nucleotide sequence synthesized by fusing together a nucleic acid sequence encoding a light chain framework region 1, a nucleic acid sequence encoding a light chain complementarity determining region (CDR) 1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the CDRs are derived from a donor antibody light chain variable region (preferably, a non-human donor antibody light chain variable region) and at least one light chain framework region is from a sub-bank of light chain framework region (preferably, a sub-bank of human light chain framework region).

The present invention provides a cell containing nucleic acid sequences encoding an antibody (preferably, a humanized antibody) that immunospecifically binds to an antigen, said cell produced by the process comprising: (a) introducing into a cell a nucleic acid sequence comprising a nucleotide sequence encoding a heavy chain variable region (preferably, a heavy chain variable region), said nucleotide sequence synthesized by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, wherein at least one CDR is from a sub-bank of heavy chain CDRs derived from donor antibodies (preferably, non-human donor antibodies) and at least one heavy chain framework region is from a sub-bank of heavy chain framework regions (preferably, a sub-bank of human heavy chain framework regions); and (b) introducing into a cell a nucleic acid sequence comprising a nucleotide sequence encoding a light chain variable region (preferably, a humanized light chain variable region), said nucleotide sequence synthesized by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the CDRs are derived from a donor antibody light chain variable region (preferably, a non-human donor antibody light chain variable region) and at least one light chain framework region is from a sub-bank of light chain framework region (preferably, a sub-bank of human light chain framework region).

The present invention provides a cell containing nucleic acid sequences encoding an antibody (preferably, a humanized antibody) that immunospecifically binds to an antigen, said cell produced by the process comprising: (a) introducing into a cell a nucleic acid sequence comprising a nucleotide acid sequence encoding a heavy chain variable region (preferably, a humanized heavy chain variable region), said nucleotide sequence synthesized by fusing together a nucleic acid sequence encoding a heavy chain framework region 1, a nucleic acid sequence encoding a heavy chain complementarity determining region (CDR) 1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, wherein at least one CDR is from a sub-bank of heavy chain CDRs derived from donor antibodies (preferably, non-human donor antibodies) and at least one heavy chain framework region is from a sub-bank of heavy chain framework regions (preferably, a sub-bank of human heavy chain framework regions); and (b) introducing into a cell a nucleic acid sequence comprising a nucleotide sequence encoding a light chain variable region (preferably, a humanized light chain variable region), said nucleotide sequence synthesized by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one CDR is from a sub-bank of light chain CDRs derived from donor antibodies (preferably, non-human donor antibodies) and at least one light chain framework region is from a sub-bank of light chain framework regions (preferably, a sub-bank of human light chain framework regions).

The present invention provides a cell containing nucleic acid sequences encoding an antibody (preferably, a humanized antibody) that immunospecifically binds to an antigen, said cell produced by the process comprising: (a) introducing into a cell a nucleic acid sequence comprising a nucleotide sequence encoding a heavy chain variable region (preferably, a humanized heavy chain variable region), said nucleotide sequence synthesized by fusing together a nucleic acid sequence encoding a heavy chain framework region 1, a nucleic acid sequence encoding a heavy chain complementarity determining region (CDR) 1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, wherein the CDRs are derived from a donor antibody heavy chain variable region (preferably, a non-human donor antibody heavy chain variable region) and at least one heavy chain framework region is from a sub-bank of heavy chain framework regions (preferably, a sub-bank of human heavy chain framework regions); and (b) introducing into a cell a nucleic acid sequence comprising a nucleotide sequence encoding a light chain variable region (preferably, a humanized light chain variable region), said nucleotide sequence synthesized by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one CDR is from a sub-bank of light chain CDRs derived from donor antibodies (preferably, non-human donor antibodies) and at least one light chain framework region is from a sub-bank of light chain framework regions (preferably, a sub-bank of human light chain framework regions).

The present invention provides a method of producing a heavy chain variable region (preferably, a humanized heavy chain variable region), said method comprising expressing the nucleotide sequence encoding a heavy chain variable region (preferably, a humanized heavy chain variable region) in a cell described herein. The present invention provides a method of producing an light chain variable region (preferably, a humanized light chain variable region), said method comprising expressing the nucleotide sequence encoding a light chain variable region (preferably, a humanized light chain variable region) in a cell described herein. The present invention also provides a method of producing an antibody (preferably, a humanized antibody) that immunospecifically binds to an antigen, said method comprising expressing the nucleic acid sequence(s) encoding the humanized antibody contained in the cell described herein.

In one embodiment, the present invention provides a method of producing an antibody (preferably, a humanized antibody) that immunospecifically binds to an antigen, said method comprising: (a) generating sub-banks of heavy chain framework regions; (b) synthesizing a nucleic acid sequence comprising a nucleotide sequence encoding a humanized heavy chain variable region, said nucleotide sequence produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding heavy chain CDR2, a nucleic acid sequence encoding a heavy chain framework region 3, a nucleic acid sequence encoding a heavy chain CDR3, and a nucleic acid sequence encoding a heavy chain framework region 4, wherein the CDRs are derived from a donor antibody heavy chain variable region (preferably, a non-human donor antibody heavy chain variable region) and at least one heavy chain framework region is from a sub-bank of heavy chain framework regions (preferably, a sub-bank of human heavy chain framework regions); (c) introducing the nucleic acid sequence into a cell containing a nucleic acid sequence comprising a nucleotide sequence encoding a variable light chain variable region (preferably, a humanized or human variable light chain variable region); and (d) expressing the nucleotide sequences encoding the heavy chain variable region (preferably, the humanized heavy chain variable region) and the light chain variable region (preferably, the humanized or human light chain variable region). In accordance with this embodiment, the method may further comprise a step (e) comprising screening for an antibody (preferably, a humanized antibody) that immunospecifically binds to the antigen.

In another embodiment, the present invention provides a method of producing an antibody (preferably, a humanized antibody) that immunospecifically binds to an antigen, said method comprising: (a) generating sub-banks of heavy chain framework regions; (b) synthesizing a nucleic acid sequence comprising a nucleotide sequence encoding a heavy chain variable region (preferably, a humanized heavy chain variable region), said nucleotide sequence produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding heavy chain CDR2, a nucleic acid sequence encoding a heavy chain framework region 3, a nucleic acid sequence encoding a heavy chain CDR3, and a nucleic acid sequence encoding a heavy chain framework region 4, wherein at least one CDR is from a sub-bank of heavy chain CDRs derived from donor antibodies (preferably, non-human donor antibodies) and at least one heavy chain framework region is from a sub-bank of heavy chain framework regions (preferably, a sub-bank of human heavy chain framework regions); (c) introducing the nucleic acid sequence into a cell containing a nucleic acid sequence comprising a nucleotide sequence encoding a variable light chain variable region (preferably, a humanized or human variable light chain variable region); and (d) expressing the nucleotide sequences encoding the heavy chain variable region (preferably, the humanized heavy chain variable region) and the light chain variable region (preferably, the humanized or human light chain variable region). In accordance with this embodiment, the method may further comprise a step (e) comprising screening for an antibody (preferably, a humanized antibody) that immunospecifically binds to the antigen.

In another embodiment, the present invention provides a method of producing an antibody (preferably, a humanized antibody) that immunospecifically binds to an antigen, said method comprising: (a) generating sub-banks of light chain framework regions; (b) synthesizing a nucleic acid sequence comprising a nucleotide sequence encoding a light chain variable region (preferably, a humanized light chain variable region), said nucleotide sequence produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the CDRs are derived from a donor antibody light chain variable region (preferably, a non-human donor antibody light chain variable region) and at least one light chain framework region is from a sub-bank of light chain framework regions (preferably, a sub-bank of human light chain framework regions); (c) introducing the nucleic acid sequence into a cell containing a nucleic acid sequence comprising a nucleotide sequence encoding a variable heavy chain variable region (preferably, a humanized or human variable heavy chain variable region); and (d) expressing the nucleotide sequences encoding the heavy chain variable region (preferably, the humanized heavy chain variable region) and the light chain variable region (preferably, the humanized or human light chain variable region). In accordance with this embodiment, the method may further comprise a step (e) comprising screening for an antibody (preferably, a humanized antibody) that immunospecifically binds to the antigen.

In another embodiment, the present invention provides a method of producing an antibody (preferably, a humanized antibody) that immunospecifically binds to an antigen, said method comprising: (a) generating sub-banks of light chain framework regions; (b) synthesizing a nucleic acid sequence comprising a nucleotide sequence encoding a light chain variable region (preferably, a humanized light chain variable region), said nucleotide sequence produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one CDR is from a sub-bank of light chain CDRs derived from donor antibodies (preferably, non-human donor antibodies) and at least one light chain framework region is from a sub-bank of light chain framework regions (preferably, a sub-bank of human light chain framework regions); (c) introducing the nucleic acid sequence into a cell containing a nucleic acid sequence comprising a nucleotide sequence encoding a variable heavy chain variable region (preferably, a humanized or human variable heavy chain variable region); and (d) expressing the nucleotide sequences encoding the heavy chain variable region (preferably, the humanized heavy chain variable region) and the light chain variable region (preferably, the humanized or human light chain variable region). In accordance with this embodiment, the method may further comprise a step (e) comprising screening for an antibody (preferably, a humanized antibody) that immunospecifically binds to the antigen.

In another embodiment, the present invention provides a method of producing an antibody (preferably, a humanized antibody) that immunospecifically binds to an antigen, said method comprising: (a) generating sub-banks of light chain framework regions; (b) generating sub-banks of heavy chain framework regions; (c) synthesizing a nucleic acid sequence comprising a nucleotide sequence encoding a heavy chain variable region (preferably, a humanized heavy chain variable region), said nucleotide sequence produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding heavy chain CDR2, a nucleic acid sequence encoding a heavy chain framework region 3, a nucleic acid sequence encoding a heavy chain CDR3, and a nucleic acid sequence encoding a heavy chain framework region 4, wherein the CDRs are derived from a donor antibody heavy chain variable region (preferably, a non-human donor antibody heavy chain variable region) and at least one heavy chain framework region is from a sub-bank of heavy chain framework regions (preferably, a sub-bank of human heavy chain framework regions); (d) synthesizing a nucleic acid sequence comprising a nucleotide sequence encoding a light chain variable region (preferably, a humanized light chain variable region), said nucleotide sequence produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the CDRs are derived from a donor antibody light chain variable region (preferably, a non-human donor antibody light chain variable region) and at least one light chain framework region is from a sub-bank of light chain framework regions (preferably, a sub-bank of human light chain framework regions); (e) introducing the nucleic acid sequences into a cell; and (f) expressing the nucleotide sequences encoding the heavy chain variable region (preferably, the humanized heavy chain variable region) and the humanized light chain variable region (preferably, the humanized light chain variable region). In accordance with this embodiment, the method may further comprise a step (g) comprising screening for an antibody (preferably, a humanized antibody) that immunospecifically binds to the antigen.

In another embodiment, the present invention provides a method of producing an antibody (preferably, a humanized antibody) that immunospecifically binds to an antigen, said method comprising: (a) generating sub-banks of light chain framework regions; (b) generating sub-banks of heavy chain framework regions; (c) synthesizing a nucleic acid sequence comprising a nucleotide sequence encoding a heavy chain variable region (preferably, a humanized heavy chain variable region), said nucleotide sequence produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding heavy chain CDR2, a nucleic acid sequence encoding a heavy chain framework region 3, a nucleic acid sequence encoding a heavy chain CDR3, and a nucleic acid sequence encoding a heavy chain framework region 4, wherein at least one CDR is from a sub-bank of heavy chain CDRs derived from donor antibodies (preferably, non-human antibodies) and at least one heavy chain framework region is from a sub-bank of heavy chain framework regions (preferably, a sub-bank of human heavy chain framework regions); (d) synthesizing a nucleic acid sequence comprising a nucleotide sequence encoding a light chain variable region (preferably a humanized light chain variable region), said nucleotide sequence produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the CDRs are derived from a donor antibody light chain variable region and at least one light chain framework region is from a sub-bank of human light chain framework regions; (e) introducing the nucleic acid sequences into a cell; and (f) expressing the nucleotide sequences encoding the heavy chain variable region (preferably, the humanized heavy chain variable region) and the light chain variable region (preferably, the humanized light chain variable region). In accordance with this embodiment, the method may further comprise a step (g) comprising screening for an antibody (preferably, a humanized antibody) that immunospecifically binds to the antigen.

In another embodiment, the present invention provides a method of producing an antibody (preferably, a humanized antibody) that immunospecifically binds to an antigen, said method comprising: (a) generating sub-banks of light chain framework regions; (b) generating sub-banks of heavy chain framework regions; (c) synthesizing a nucleic acid sequence comprising a nucleotide sequence encoding a humanized heavy chain variable region, said nucleotide sequence produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding heavy chain CDR2, a nucleic acid sequence encoding a heavy chain framework region 3, a nucleic acid sequence encoding a heavy chain CDR3, and a nucleic acid sequence encoding a heavy chain framework region 4, wherein the CDRs are derived from a donor antibody heavy chain variable region (preferably, a non-human donor antibody heavy chain variable region) and at least one heavy chain framework region is from a sub-bank of heavy chain framework regions (preferably, a sub-bank of human heavy chain framework regions); (d) synthesizing a nucleic acid sequence comprising a nucleotide sequence encoding a light chain variable region (preferably, a humanized light chain variable region), said nucleotide sequence produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one CDR is from a sub-bank of light chain CDRs derived from donor antibodies (preferably, non-human donor antibodies) and at least one light chain framework region is from a sub-bank of light chain framework regions (preferably, a sub-bank of human light chain framework regions); (e) introducing the nucleic acid sequences into a cell; and (f) expressing the nucleotide sequences encoding the heavy chain variable region (preferably, the humanized heavy chain variable region) and the light chain variable region (preferably, the humanized light chain variable region). In accordance with this embodiment, the method may further comprise a step (g) comprising screening for an antibody (preferably, a humanized antibody) that immunospecifically binds to the antigen.

In another embodiment, the present invention provides a method of producing an antibody (preferably, a humanized antibody) that immunospecifically binds to an antigen, said method comprising: (a) generating sub-banks of light chain framework regions; (b) generating sub-banks of heavy chain framework regions; (c) synthesizing a nucleic acid sequence comprising a nucleotide sequence encoding a heavy chain variable region (preferably, a humanized heavy chain variable region), said nucleotide sequence produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding heavy chain CDR2, a nucleic acid sequence encoding a heavy chain framework region 3, a nucleic acid sequence encoding a heavy chain CDR3, and a nucleic acid sequence encoding a heavy chain framework region 4, wherein at least one CDR is from a sub-bank of heavy chain CDRs derived from donor antibodies (preferably, non-human antibodies) and at least one heavy chain framework region is from a sub-bank of heavy chain framework regions (preferably, a sub-bank of human heavy chain framework regions); (d) synthesizing a nucleic acid sequence comprising a nucleotide sequence encoding a light chain variable region (preferably, a humanized light chain variable region), said nucleotide sequence produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one CDR is from a sub-bank of light chain CDRs derived from donor antibodies (preferably, non-human donor antibodies) and at least one light chain framework region is from a sub-bank of light chain framework regions (preferably, a sub-bank of human light chain framework regions); (e) introducing the nucleic acid sequences into a cell; and (f) expressing the nucleotide sequences encoding the heavy chain variable region (preferably, the humanized heavy chain variable region) and the light chain variable region (preferably, the humanized light chain variable region). In accordance with this embodiment, the method may further comprise a step (g) comprising screening for an antibody (preferably, a humanized antibody) that immunospecifically binds to the antigen.

In another embodiment, the present invention provides a method of producing an antibody (preferably, a humanized antibody) that immunospecifically binds to an antigen, said method comprising: (a) generating sub-banks of light chain framework regions; (b) generating sub-banks of heavy chain framework regions; (c) synthesizing a nucleic acid sequence comprising: (i) a first nucleotide sequence encoding a heavy chain variable region (preferably, a humanized heavy chain variable region), said first nucleotide sequence produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding heavy chain CDR2, a nucleic acid sequence encoding a heavy chain framework region 3, a nucleic acid sequence encoding a heavy chain CDR3, and a nucleic acid sequence encoding a heavy chain framework region 4, and (ii) a second nucleotide sequence encoding a light chain variable region (preferably, a humanized light chain variable region), said second nucleotide sequence produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the heavy chain variable region CDRs are derived from a donor antibody heavy chain variable region (preferably, a non-human donor antibody heavy chain variable region), the light chain variable region CDRs are derived from a donor antibody light chain variable region (preferably, a non-human donor antibody light chain variable region), at least one heavy chain framework region is from a sub-bank of heavy chain framework regions (preferably, a sub-bank of human heavy chain framework regions) and at least one light chain framework region is from a sub-bank of light chain framework regions (preferably, a sub-bank of human light chain framework regions); (d) introducing the nucleic acid sequence into a cell; and (e) expressing the nucleotide sequences encoding the heavy chain variable region (preferably, the humanized heavy chain variable region) and the light chain variable region (preferably, the humanized light chain variable region). In accordance with this embodiment, the method may further comprise a step (f) comprising screening for an antibody (preferably, a humanized antibody) that immunospecifically binds to the antigen.

The present invention provides a method of producing a humanized antibody that immunospecifically binds to an antigen, said method comprising: (a) generating sub-banks of light chain framework regions; (b) generating sub-banks of heavy chain framework regions; (c) synthesizing a nucleic acid sequence comprising: (i) a first nucleotide sequence encoding a humanized heavy chain variable region, said first nucleotide sequence produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding heavy chain CDR2, a nucleic acid sequence encoding a heavy chain framework region 3, a nucleic acid sequence encoding a heavy chain CDR3, and a nucleic acid sequence encoding a heavy chain framework region 4, and (ii) a second nucleotide sequence encoding a humanized light chain variable region, said second nucleotide sequence produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one heavy chain variable region CDR is from a sub-bank of heavy chain CDRs derived from donor antibodies that immunospecifically bind to an antigen, the light chain variable region CDRs are derived from a donor antibody light chain variable region, at least one heavy chain framework region is from a sub-bank of human heavy chain framework regions and at least one light chain framework region is from a sub-bank of human light chain framework regions; (d) introducing the nucleic acid sequence into a cell; and (e) expressing the nucleotide sequences encoding the humanized heavy chain variable region and the humanized light chain variable region. In accordance with this embodiment, the method may further comprise a step (f) comprising screening for an antibody (preferably, a humanized antibody) that immunospecifically binds to the antigen.

The present invention provides a method of producing a humanized antibody that immunospecifically binds to an antigen, said method comprising: (a) generating sub-banks of light chain framework regions; (b) generating sub-banks of heavy chain framework regions; (c) synthesizing a nucleic acid sequence comprising: (i) a first nucleotide sequence encoding a humanized heavy chain variable region, said first nucleotide sequence produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding heavy chain CDR2, a nucleic acid sequence encoding a heavy chain framework region 3, a nucleic acid sequence encoding a heavy chain CDR3, and a nucleic acid sequence encoding a heavy chain framework region 4, and (ii) a second nucleotide sequence encoding a humanized light chain variable region, said second nucleotide sequence produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the heavy chain variable region CDRs are derived from a donor antibody heavy chain variable region, at least one light chain variable region CDR is from a sub-bank of light chain CDRs derived from donor antibodies that immunospecifically bind to an antigen, at least one heavy chain framework region is from a sub-bank of human heavy chain framework regions and at least one light chain framework region is from a sub-bank of human light chain framework regions; (d) introducing the nucleic acid sequence into a cell; and (e) expressing the nucleotide sequences encoding the humanized heavy chain variable region and the humanized light chain variable region. In accordance with this embodiment, the method may further comprise a step (f) comprising screening for an antibody (preferably, a humanized antibody) that immunospecifically binds to the antigen.

In another embodiment, the present invention provides a method of producing an antibody (preferably, a humanized antibody) that immunospecifically binds to an antigen, said method comprising: (a) generating sub-banks of light chain framework regions; (b) generating sub-banks of heavy chain framework regions; (c) synthesizing a nucleic acid sequence comprising: (i) a first nucleotide sequence encoding a heavy chain variable region (preferably, a humanized heavy chain variable region), said first nucleotide sequence produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding heavy chain CDR2, a nucleic acid sequence encoding a heavy chain framework region 3, a nucleic acid sequence encoding a heavy chain CDR3, and a nucleic acid sequence encoding a heavy chain framework region 4, and (ii) a second nucleotide sequence encoding a light chain variable region (preferably, a humanized light chain variable region), said second nucleotide sequence produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one heavy chain variable region CDR is from a sub-bank of heavy chain CDRs derived from donor antibodies (preferably, non-human donor antibodies), at least one light chain variable region CDR is from a sub-bank of light chain CDRs derived from donor antibodies (preferably, non-human donor antibodies), at least one heavy chain framework region is from a sub-bank of heavy chain framework regions (preferably, a sub-bank of human heavy chain framework regions) and at least one light chain framework region is from a sub-bank of light chain framework regions (preferably, a sub-bank of human light chain framework regions); (d) introducing the nucleic acid sequence into a cell; and (e) expressing the nucleotide sequences encoding the heavy chain variable region (preferably, the humanized heavy chain variable region) and the humanized light chain variable region (preferably, the humanized light chain variable region). In accordance with this embodiment, the method may further comprise a step (f) comprising screening for an antibody (preferably, a humanized antibody) that immunospecifically binds to the antigen.

The present invention provides antibodies produced by the methods described herein. In a preferred embodiment, the invention provides humanized antibodies produced by the methods described herein. The present invention also provides a composition comprising an antibody produced by the methods described herein and a carrier, diluent or excipient. In a preferred embodiment, the invention provides a composition comprising a humanized antibody produced by the methods described herein and a carrier, diluent or excipient. Preferably, the compositions of the invention are pharmaceutical compositions in a form for its intended use.

The present invention provides a plurality of nucleic acid sequences comprising nucleotide sequences encoding heavy chain variable regions (preferably, humanized heavy chain variable regions), said nucleotide sequences encoding the heavy chain variable regions each produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, wherein the CDRs are derived from a donor antibody heavy chain variable region (preferably, a non-humanized donor antibody heavy chain variable region) and at least one heavy chain framework region is from a sub-bank of heavy chain framework regions (preferably, a sub-bank of human heavy chain framework regions). The present invention also provides a plurality of nucleic acid sequences comprising nucleotide sequences encoding heavy chain variable regions (preferably, humanized heavy chain variable regions), said nucleotide sequences encoding the heavy chain variable regions each produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, wherein at least one CDR is from a sub-bank of heavy chain CDRs derived from donor antibodies (preferably, non-human donor antibodies) and at least one heavy chain framework region is from a sub-bank of heavy chain framework regions (preferably, a sub-bank of human heavy chain framework regions).

The present invention provides a plurality of nucleic acid sequences comprising nucleotide sequences encoding light chain variable regions (preferably, humanized light chain variable regions), said nucleotide sequences encoding the light chain variable regions each produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the CDRs are derived from a donor antibody light chain variable region (preferably, a non-human donor antibody light chain variable region) and at least one light chain framework region is from a sub-bank of light chain framework regions (preferably, a sub-bank of human light chain framework regions). The present invention also provides a plurality of nucleic acid sequences comprising nucleotide sequences encoding light chain variable regions (preferably, humanized light chain variable regions), said nucleotide sequences encoding the light chain variable regions each produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one CDR is from a sub-bank of light chain CDRs derived from donor antibodies (preferably, non-human donor antibodies) and at least one light chain framework region is from a sub-bank of light chain framework regions (preferably, a sub-bank of human light chain framework regions).

The present invention provides a plurality of nucleic acid sequences comprising: (i) a first set of nucleotide sequences encoding heavy chain variable regions (preferably, humanized heavy chain variable regions), said first set of nucleotide sequences encoding the heavy chain variable regions each produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, and (ii) a second set of nucleotide encoding light chain variable regions (preferably, humanized light chain variable regions), said second set of nucleotide sequences encoding the light chain variable regions each produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the heavy chain variable region CDRs are derived from a donor antibody heavy chain variable region (preferably, a non-human donor antibody heavy chain variable region), the light chain variable region CDRs are derived from a donor antibody light chain variable region (preferably, a non-human donor antibody light chain variable region), at least one heavy chain framework region is from a sub-bank of heavy chain framework regions (preferably, a sub-bank of human heavy chain framework regions) and at least one light chain framework region is from a sub-bank of light chain framework regions (preferably, a sub-bank of human light chain framework regions).

The present invention provides a plurality of nucleic acid sequences comprising: (i) a first set of nucleotide sequences encoding heavy chain variable regions (preferably, humanized heavy chain variable regions), said first set of nucleotide sequences encoding the heavy chain variable regions each produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, and (ii) a second set of nucleotide encoding light chain variable regions (preferably, humanized light chain variable regions), said second set of nucleotide sequences encoding the light chain variable regions each produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one heavy chain variable region CDR is from a sub-bank of heavy chain CDRs derived from donor antibodies (preferably, non-human donor antibodies), the light chain variable region CDRs are derived from a donor antibody light chain variable region (preferably, a non-human donor antibody light chain variable region), at least one heavy chain framework region is from a sub-bank of heavy chain framework regions (preferably, a sub-bank of human heavy chain framework regions) and at least one light chain framework region is from a sub-bank of light chain framework regions (preferably, a sub-bank of human light chain framework regions).

The present invention provides a plurality of nucleic acid sequences comprising: (i) a first set of nucleotide sequences encoding heavy chain variable regions (preferably, humanized heavy chain variable regions), said first set of nucleotide sequences encoding the heavy chain variable regions each produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, and (ii) a second set of nucleotide encoding light chain variable regions (preferably, humanized light chain variable regions), said second set of nucleotide sequences encoding the light chain variable regions each produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the heavy chain variable region CDRs are derived from a donor antibody heavy chain variable region (preferably, a non-human donor antibody heavy chain variable region), at least one light chain variable region CDR is from a sub-bank of light chain CDRs derived from donor antibodies (preferably, non-human donor antibodies), at least one heavy chain framework region is from a sub-bank of heavy chain framework regions (preferably, a)sub-bank of human heavy chain framework regions) and at least one light chain framework region is from a sub-bank of light chain framework regions (preferably, human light chain framework regions).

The present invention provides a plurality of nucleic acid sequences comprising: (i) a first set of nucleotide sequences encoding heavy chain variable regions (preferably, humanized heavy chain variable regions), said first set of nucleotide sequences encoding the heavy chain variable regions each produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, and (ii) a second set of nucleotide encoding light chain variable regions (preferably, humanized light chain variable regions), said second set of nucleotide sequences encoding the light chain variable regions each produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one heavy chain variable region CDR is from a sub-bank of heavy chain CDRs derived from donor antibodies (preferably, non-human antibodies), at least one light chain variable region CDR is from a sub-bank of light chain CDRs derived from donor antibodies (preferably, non-human antibodies), at least one heavy chain framework region is from a sub-bank of heavy chain framework regions (preferably, a sub-bank of human heavy chain framework regions )and at least one light chain framework region is from a sub-bank of light chain framework regions (preferably, a sub-bank of human light chain framework regions).

The present invention provides a population of cells comprising the nucleic acid sequences described herein. In one embodiment, the present invention provides a population of cells comprising nucleic acid sequences comprising nucleotide sequences encoding a plurality of heavy chain variable regions (preferably, humanized heavy chain variable regions), said cells produced by the process comprising introducing into cells nucleic acid sequences comprising nucleotide sequences encoding heavy chain variable regions each synthesized by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, wherein the CDRs are derived from a donor antibody heavy chain variable region (preferably, a non-human donor antibody heavy chain variable region) and at least one heavy chain framework region is from a sub-bank of heavy chain framework regions (preferably, a sub-bank of human heavy chain framework regions). In accordance with this embodiment, the cells may further comprise a nucleic acid sequence comprising a nucleotide sequence encoding a light chain variable region (preferably, a humanized or human light chain variable region).

In another embodiment, the present invention provides a population of cells comprising nucleic acid sequences comprising nucleotide acid sequences encoding a plurality of heavy chain variable regions (preferably, humanized heavy chain variable regions), said cells produced by the process comprising introducing into cells nucleic acid sequences comprising nucleotide sequences encoding heavy chain variable regions each synthesized by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, wherein at least one CDR is from a sub-bank of heavy chain CDRs derived from donor antibodies (preferably, non-human donor antibodies) and at least one heavy chain framework region is from a sub-bank of heavy chain framework regions (preferably, a sub-bank of human heavy chain framework regions). In accordance with this embodiment, the cells may further comprise a nucleic acid sequence comprising a nucleotide sequence encoding a light chain variable region (preferably, a humanized or human light chain variable region).

In another embodiment, the present invention provides a population of cells comprising nucleic sequences comprising nucleotide sequences encoding a plurality of light chain variable regions (preferably, humanized light chain variable regions), said cells produced by the process comprising introducing into cells nucleic acid sequences comprising nucleotide sequences encoding light chain variable regions each synthesized by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the CDRs are derived from a donor antibody light chain variable region (preferably, a non-human donor antibody light chain variable region) and at least one light chain framework region is from a sub-bank of light chain framework regions (preferably, a sub-bank of human light chain framework regions). In accordance with this embodiment, the cells may further comprise a nucleic acid sequence comprising a nucleotide sequence encoding a light chain variable region (preferably, a humanized or human light chain variable region).

In another embodiment, the present invention provides a population of cells comprising nucleic acid sequences comprising nucleotide sequences encoding a plurality of light chain variable regions (preferably, humanized light chain variable regions), said cells produced by the process comprising introducing into cells nucleic acid sequences comprising nucleotide sequences encoding light chain variable regions each synthesized by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one CDR is from a sub-bank of light chain CDRs derived from donor antibodies (preferably, non-human donor antibodies) and at least one light chain framework region is from a sub-bank of light chain framework regions (preferably, a sub-bank of human light chain framework regions). In accordance with this embodiment, the cells may further comprise a nucleic acid sequence comprising a nucleotide sequence encoding a light chain variable region (preferably, a humanized or human light chain variable region).

In another embodiment, the present invention provides a population of cells comprising nucleic acid sequences comprising nucleotide sequences encoding a plurality of heavy chain variable regions (preferably, humanized heavy chain variable regions) and a plurality of light chain variable regions (preferably, humanized light chain variable regions), said cells each produced by the process comprising introducing into cells nucleic acid sequences comprising: (i) a first set of nucleotide sequences encoding heavy chain variable regions each synthesized by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, and (ii) a second set of nucleotide sequences encoding light chain variable regions each synthesized by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the heavy chain variable region CDRs are derived from a donor antibody heavy chain variable region (preferably, a non-human donor antibody heavy chain variable region), the light chain variable region CDRs are derived from a donor antibody light chain variable region (preferably, a non-human donor antibody light chain variable region), at least one heavy chain framework region is from a sub-bank of heavy chain framework regions (preferably, a sub-bank of human heavy chain framework regions) and at least one light chain framework region is from a sub-bank of light chain framework regions (preferably, a sub-bank of human light chain framework regions).

In another embodiment, the present invention provides a population of cells comprising nucleic acid sequences comprising nucleotide sequences encoding a plurality of heavy chain variable regions (preferably, humanized heavy chain variable regions) and a plurality of light chain variable regions (preferably, humanized light chain variable regions), said cells each produced by the process comprising introducing into cells nucleic acid sequences comprising: (i) a first set of nucleotide sequences encoding heavy chain variable regions each synthesized by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, and (ii) a second set of nucleotide sequences encoding light chain variable regions each synthesized by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one heavy chain variable region CDR is from a sub-bank of heavy chain CDRs derived from donor antibodies (preferably, non-human donor antibodies), the light chain variable region CDRs are derived from a donor antibody light chain variable region (preferably, a non-human donor antibody light chain variable region), at least one heavy chain framework region is from a sub-bank of heavy chain framework regions (preferably, a sub-bank of human heavy chain framework regions) and at least one light chain framework region is from a sub-bank of light chain framework regions (preferably, a sub-bank of human light chain framework regions).

In another embodiment, the present invention provides a population of cells comprising nucleic acid sequences comprising nucleotide sequences encoding a plurality of heavy chain variable regions (preferably, humanized heavy chain variable regions) and a plurality of light chain variable regions (preferably, humanized light chain variable regions), said cells each produced by the process comprising introducing into cells nucleic acid sequences comprising: (i) a first set of nucleotide sequences encoding heavy chain variable regions each synthesized by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, and (ii) a second set of nucleotide sequences encoding light chain variable regions each synthesized by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the heavy chain variable region CDRs are derived from a donor antibody heavy chain variable region (preferably, a non-human donor antibody heavy chain variable region), at least one light chain variable region CDR is from a sub-bank of light chain CDRs derived from donor antibodies (preferably, non-human donor antibodies), at least one heavy chain framework region is from a sub-bank of heavy chain framework regions (preferably, a sub-bank of human heavy chain framework regions) and at least one light chain framework region is from a sub-bank of light chain framework regions (preferably, a sub-bank of human light chain framework regions).

In another embodiment, the present invention provides a population of cells comprising nucleic acid sequences comprising nucleotide sequences encoding a plurality of heavy chain variable regions (preferably, humanized heavy chain variable regions) and a plurality of light chain variable regions (preferably, humanized light chain variable regions), said cells each produced by the process comprising introducing into cells nucleic acid sequences comprising: (i) a first set of nucleotide sequences encoding heavy chain variable regions each synthesized by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, and (ii) a second set of nucleotide sequences encoding light chain variable regions each synthesized by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one heavy chain variable region CDR is from a sub-bank of heavy chain CDRs derived from donor antibodies (preferably, non-human donor antibodies), at least one light chain variable region CDR is from a sub-bank of light chain CDRs derived from donor antibodies (preferably, non-human donor antibodies), at least one heavy chain framework region is from a sub-bank of heavy chain framework regions (preferably, a sub-bank of human heavy chain framework regions) and at least one light chain framework region is from a sub-bank of light chain framework regions (preferably, a sub-bank of human light chain framework regions).

The present invention provides a method of identifying an antibody that immunospecifically binds to an antigen, said method comprising expressing the nucleic acid sequences in the cells as described herein and screening for an antibody that has an affinity of at least 1 x 10⁶ M⁻¹, at least 1 x 10⁷ M⁻¹, at least 1 x 10⁸ M⁻¹, at least 1 x 10⁹ M⁻¹, at least 1 x 10¹⁰ M⁻¹ or above for said antigen. In a preferred embodiment, the invention provides a method of identifying a humanized antibody that immunospecifically to an antigen, said method comprising expressing the nucleic acid sequences in the cells as described herein and screening for a humanized antibody that has an affinity of at least 1 x 10⁶ M⁻¹, at least 1 x 10⁷ M⁻¹, at least 1 x 10⁸ M⁻¹, at least 1 x 10⁹ M⁻¹, at least 1 x 10¹⁰ M⁻¹ or above for said antigen. The present invention provides an antibody identified by the methods described herein. In a preferred embodiment, the invention provides a humanized antibody identified by the methods described herein.

In accordance with the present invention, the antibodies generated as described herein (*e.g*., a humanized antibody) comprise a light chain variable region and/or a heavy chain variable region. In some embodiments, the antibodies generated as described herein further comprise a constant region(s).

The present invention provides antibodies (preferably, humanized antibodies) generated in accordance with the invention conjugated or fused to a moiety (*e.g*., a therapeutic agent or drug). The present invention also provides compositions, preferably pharmaceutical compositions, comprising an antibody generated and/or identified in accordance with the present invention and a carrier, diluent or excipient. In certain preferred embodiments, the present invention provides compositions, preferably pharmaceutical compositions, comprising a humanized antibody as described herein and a carrier, diluent or excipient. The present invention also provides compositions, preferably pharmaceutical compositions, comprising an antibody generated and/or identified in accordance with the present invention conjugated or fused to a moiety (*e.g*., a therapeutic agent or drug), and a carrier, diluent or excepient. In certain preferred embodiments, the present invention provides compositions comprising a humanized antibody (or fragment thereof) conjugated or fused to a moiety (*e.g*., a therapeutic agent or drug), and a carrier, diluent or excepient. The present invention further provides uses of an antibody generated and/or identified in accordance with the present invention (*e.g*., a humanized antibody) alone or in combination with other therapies to prevent, treat, manage or ameliorate a disorder or a symptom thereof.

The pharmaceutical compositions of the invention may be used for the prevention, management, treatment or amelioration of a disease or one or more symptoms thereof. Preferably, the pharmaceutical compositions of the invention are sterile and in suitable form for a particular method of administration to a subject with a disease.

The invention further provides methods of detecting, diagnosing and/or monitoring the progression of a disorder utilizing one or more antibodies (preferably, one or more humanized antibodies) generated and/or identified in accordance with the methods of the invention.

The invention provides kits comprising sub-banks of antibody framework regions of a species of interest. The invention also provides kits comprising sub-banks of CDRs of a species of interest. The invention also provides kits comprising combinatorial sub-libraries of nucleic acids, wherein the nucleic acids comprise nucleotide sequences that contain one framework region (*e.g*., FR1) fused in frame to one corresponding CDR (*e.g*., CDR1). The invention further provides kits comprising combinatorial libraries of nucleic acids, wherein the nucleic acids comprise nucleotide sequences that contain the framework regions and CDRs of the variable heavy chain region or variable light chain region fused in frame (*e.g*., FR1+CDR1+FR2+CDR2+FR3+CDR3+FR4).

In some preferred embodiments, the invention provides kits comprising sub-banks of human immunoglobulin framework regions, sub-banks of CDRs, combinatorial sub-libraries, and/or combinatorial libraries. In one embodiment, the invention provides a kit comprising a framework region sub-bank for variable light chain framework region 1, 2, 3, and/or 4, wherein the framework region is defined according to the Kabat system. In another embodiment, the invention provides a kit comprising a framework region sub-bank for variable light chain framework region 1, 2, 3, and/or 4, wherein the framework region is defined according to the Chothia system. In another embodiment, the invention provides a kit comprising a framework region sub-bank for variable heavy chain framework region 1, 2, 3, and/or 4, wherein the framework region is defined according to the Kabat system. In another embodiment, the invention provides a kit comprising a framework region sub-bank for variable heavy chain framework region 1, 2, 3, and/or 4, wherein the framework region is defined according to the Chothia system. In yet another embodiment, the invention provides a kit comprising sub-banks of both the variable light chain and the variable heavy chain framework regions.

The invention also provides a pharmaceutical pack or kit comprising one or more containers filled with a humanized antibody of the invention. The pharmaceutical pack or kit may further comprises one or more other prophylactic or therapeutic agents useful for the prevention, treatment, management or amelioration of a particular disease or a symptom thereof. The invention also provides a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects approval by the agency of manufacture, use or sale for human administration.

The present invention also provides articles of manufacture.

### 2.1 Terminology

As used herein, the terms "acceptor" and "acceptor antibody" refer to the antibody or nucleic acid sequence providing or encoding at least 80%, at least 85%, at least 90%, or at least 95% amino acid sequences of one or more of the framework regions. In some embodiments, the term "acceptor" refers to the antibody or nucleic acid sequence providing or encoding the constant region(s). In a specific embodiment, the term "acceptor" refers to a human antibody or nucleic acid sequence that provides or encodes at least 80%, preferably, at least 85%, at least 90%, or at least 95% amino acid sequences of one or more of the framework regions. An acceptor framework region and/or acceptor constant region(s) may be, *e.g*., derived or obtained from a germline antibody gene, a mature antibody gene, a functional antibody (*e.g*., antibodies well-known in the art, antibodies in development, or antibodies commercially available).

As used herein, the terms "antibody" and "antibodies" refer to monoclonal antibodies, multispecific antibodies, human antibodies, humanized antibodies, camelised antibodies, chimeric antibodies, single-chain Fvs (scFv), single chain antibodies, single domain antibodies, Fab fragments, F(ab) fragments, disulfide-linked Fvs (sdFv), antiidiotypic (anti-Id) antibodies, and epitope-binding fragments of any of the above. In particular, antibodies include immunoglobulin molecules and immunologically active fragments of immunoglobulin molecules, *i.e.,* molecules that contain an antigen binding site. Immunoglobulin molecules can be of any type (*e.g*., IgG, IgE, IgM, IgD, IgA and IgY), class (*e.g*., IgG₁, IgG₂, IgG₃, IgG₄, IgA₁ and IgA₂) or subclass.

A typical antibody contains two heavy chains paired with two light chains. A full-length heavy chain is about 50 kD in size (approximately 446 amino acids in length), and is encoded by a heavy chain variable region gene (about 116 amino acids) and a constant region gene. There are different constant region genes encoding heavy chain constant region of different isotypes such as alpha, gamma (IgG1, IgG2, IgG3, IgG4), delta, epsilon, and mu sequences. A full-length light chain is about 25 Kd in size (approximately 214 amino acids in length), and is encoded by a light chain variable region gene (about 110 amino acids) and a kappa or lambda constant region gene. The variable regions of the light and/or heavy chain are responsible for binding to an antigen, and the constant regions are responsible for the effector functions typical of an antibody.

As used herein, the term "CDR" refers to the complement determining region within antibody variable sequences. There are three CDRs in each of the variable regions of the heavy chain and the light chain, which are designated CDR1, CDR2 and CDR3, for each of the variable regions. The exact boundaries of these CDRs have been defined differently according to different systems. The system described by Kabat (Kabat et al., Sequences of Proteins of Immunological Interest (National Institutes of Health, Bethesda, MD (1987) and (1991)) not only provides an unambiguous residue numbering system applicable to any variable region of an antibody, but also provides precise residue boundaries defining the three CDRs. These CDRs may be referred to as Kabat CDRs. Chothia and coworkers (Chothia & Lesk, J. Mol. Biol. 196:901-917 (1987) and Chothia et al., Nature 342:877-883 (1989)) found that certain sub-portions within Kabat CDRs adopt nearly identical peptide backbone conformations, despite having great diversity at the level of amino acid sequence. These sub-portions were designated as L1, L2 and L3 or H1, H2 and H3 where the "L" and the "H" designates the light chain and the heavy chains regions, respectively. These regions may be referred to as Chothia CDRs, which have boundaries that overlap with Kabat CDRs. Other boundaries defining CDRs overlapping with the Kabat CDRs have been described by Padlan (FASEB J. 9:133-139 (1995)) and MacCallum (J Mol Biol 262(5):732-45 (1996)). Still other CDR boundary definitions may not strictly follow one of the above systems, but will nonetheless overlap with the Kabat CDRs, although they may be shortened or lengthened in light of prediction or experimental findings that particular residues or groups of residues or even entire CDRs do not significantly impact antigen binding. The methods used herein may utilize CDRs defined according to any of these systems, although preferred embodiments use Kabat or Chothia defined CDRs.

As used herein, the term "derivative" in the context of proteinaceous agent (*e.g*., proteins, polypeptides, and peptides, such as antibodies) refers to a proteinaceous agent that comprises an amino acid sequence which has been altered by the introduction of amino acid residue substitutions, deletions, and/or additions. The term "derivative" as used herein also refers to a proteinaceous agent which has been modified, i. e., by the covalent attachment of any type of molecule to the proteinaceous agent. For example, but not by way of limitation, an antibody may be modified, *e.g*., by glycosylation, acetylation, pegylation, phosphorylation, amidation, derivatization by known protecting/blocking groups, proteolytic cleavage, linkage to a cellular ligand or other protein, etc. A derivative of a proteinaceous agent may be produced by chemical modifications using techniques known to those of skill in the art, including, but not limited to specific chemical cleavage, acetylation, formylation, metabolic synthesis oftunicamycin, etc. Further, a derivative of a proteinaceous agent may contain one or more non-classical amino acids. A derivative of a proteinaceous agent possesses a similar or identical function as the proteinaceous agent from which it was derived.

As used herein, the terms "disorder" and "disease" are used interchangeably for a condition in a subject.

As used herein, the term "donor antibody" refers to an antibody providing one or more CDRs. In a preferred embodiment, the donor antibody is an antibody from a species different from the antibody from which the framework regions are derived. In the context of a humanized antibody, the term "donor antibody" refers to a non-human antibody providing one or more CDRs.

As used herein, the term "effective amount" refers to the amount of a therapy which is sufficient to reduce or ameliorate the severity and/or duration of a disorder or one or more symptoms thereof, prevent the advancement of a disorder, cause regression of a disorder, prevent the recurrence, development, onset or progression of one or more symptoms associated with a disorder, detect a disorder, or enhance or improve the prophylactic or therapeutic effect(s) of another therapy (*e.g*., prophylactic or therapeutic agent).

As used herein, the term "epitopes" refers to fragments of a polypeptide or protein having antigenic or immunogenic activity in an animal, preferably in a mammal, and most preferably in a human. An epitope having immunogenic activity is a fragment of a polypeptide or protein that elicits an antibody response in an animal. An epitope having antigenic activity is a fragment of a polypeptide or protein to which an antibody immunospecifically binds as determined by any method well-known to one of skill in the art, for example by immunoassays. Antigenic epitopes need not necessarily be immunogenic.

As used herein, the term "fusion protein" refers to a polypeptide or protein (including, but not limited to an antibody) that comprises an amino acid sequence of a first protein or polypeptide or functional fragment, analog or derivative thereof, and an amino acid sequence of a heterologous protein, polypeptide, or peptide (*i.e.,* a second protein or polypeptide or fragment, analog or derivative thereof different than the first protein or fragment, analog or derivative thereof). In one embodiment, a fusion protein comprises a prophylactic or therapeutic agent fused to a heterologous protein, polypeptide or peptide. In accordance with this embodiment, the heterologous protein, polypeptide or peptide may or may not be a different type of prophylactic or therapeutic agent. For example, two different proteins, polypeptides or peptides with immunomodulatory activity may be fused together to form a fusion protein. In a preferred embodiment, fusion proteins retain or have improved activity relative to the activity of the original protein, polypeptide or peptide prior to being fused to a heterologous protein, polypeptide, or peptide.

As used herein, the term "fragment" refers to a peptide or polypeptide (including, but not limited to an antibody) comprising an amino acid sequence of at least 5 contiguous amino acid residues, at least 10 contiguous amino acid residues, at least 15 contiguous amino acid residues, at least 20 contiguous amino acid residues, at least 25 contiguous amino acid residues, at least 40 contiguous amino acid residues, at least 50 contiguous amino acid residues, at least 60 contiguous amino residues, at least 70 contiguous amino acid residues, at least contiguous 80 amino acid residues, at least contiguous 90 amino acid residues, at least contiguous 100 amino acid residues, at least contiguous 125 amino acid residues, at least 150 contiguous amino acid residues, at least contiguous 175 amino acid residues, at least contiguous 200 amino acid residues, or at least contiguous 250 amino acid residues of the amino acid sequence of another polypeptide or protein. In a specific embodiment, a fragment of a protein or polypeptide retains at least one function of the protein or polypeptide.

As used herein, the term "functional fragment" refers to a peptide or polypeptide (including, but not limited to an antibody) comprising an amino acid sequence of at least 5 contiguous amino acid residues, at least 10 contiguous amino acid residues, at least 15 contiguous amino acid residues, at least 20 contiguous amino acid residues, at least 25 contiguous amino acid residues, at least 40 contiguous amino acid residues, at least 50 contiguous amino acid residues, at least 60 contiguous amino residues, at least 70 contiguous amino acid residues, at least contiguous 80 amino acid residues, at least contiguous 90 amino acid residues, at least contiguous 100 amino acid residues, at least contiguous 125 amino acid residues, at least 150 contiguous amino acid residues, at least contiguous 175 amino acid residues, at least contiguous 200 amino acid residues, or at least contiguous 250 amino acid residues of the amino acid sequence of second, different polypeptide or protein, wherein said polypeptide or protein retains at least one function of the second, different polypeptide or protein. In a specific embodiment, a fragment of a polypeptide or protein retains at least two, three, four, or five functions of the protein or polypeptide. Preferably, a fragment of an antibody that immunospecifically binds to a particular antigen retains the ability to immunospecifically bind to the antigen.

As used herein, the term "framework" or "framework sequence" refers to the remaining sequences of a variable region minus the CDRs. Because the exact definition of a CDR sequence can be determined by different systems, the meaning of a framework sequence is subject to correspondingly different interpretations. The six CDRs (CDR1, 2, and 3 of light chain and CDR1, 2, and 3 of heavy chain) also divide the framework regions on the light chain and the heavy chain into four sub-regions (FR1, FR2, FR3 and FR4) on each chain, in which CDR1 is positioned between FR1 and FR2, CDR2 between FR2 and FR3, and CDR3 between FR3 and FR4. Without specifying the particular sub-regions as FR1, FR2, FR3 or FR4, a framework region, as referred by others, represents the combined FR's within the variable region of a single, naturally occurring immunoglobulin chain. As used herein, a FR represents one of the four sub-regions, and FRs represents two or more of the four sub-regions constituting a framework region. As an example, Table 1-4 list the germline sequences of FR1, 2, 3, and 4 of kappa light chain, respectively. Table 5-7 list the germline sequences of FR1, 2, and 3 of heavy chain according to the Kabat definition, respectively. Table 8-10 list the germline sequences of FR 1, 2 and 3 of heavy chain according to the Chothia definition, respectively. Table 11 lists the germline sequence of FR4 of the heavy chain.

### Tables 1-65

The SEQ ID Number for each sequence described in tables 1-65 is indicated in the first column of each table.

**Table 1. FR1 of Light Chains**

| | |
|---|---|
| 1 | GATGTTGTGATGACTCAGTCTCCACTCTCCCTGCCCGTCACCCTTGGACAGCCGGCCTCCATCTCCTGC |
| 2 | GATGTTGTGATGACTCAGTCTCCACTCTCCCTGCCCGTCACCCTTGGACAGCCGGCCTCCATCTCCTGC |
| 3 | GATATTGTGATGACCCAGACTCCACTCTCTCTGTCCGTCACCCCTGGACAGCCGGCCTCCATCTCCTGC |
| 4 | GATATTGTGATGACTCAGTCTCCACTCTCCCTGCCCGTCACCCCTGGAGAGCCGGCCTCCATCTCCTGC |
| 5 | GATATTGTGATGACCCAGACTCCACTCTCTCTGTCCGTCACCCCTGGACAGCCGGCCTCCATCTCCTGC |
| 6 | GATATTGTGATGACCCAGACTCCACTCTCCTCACCTGTCACCCTTGGACAGCCGGCCTCCATCTCCTGC |
| 7 | GATATTGTGATGACTCAGTCTCCACTCTCCCTGCCCGTCACCCCTGGAGAGCCGGCCTCCATCTCCTGC |
| 8 | GAGATTGTGATGACCCAGACTCCACTCTCCTTGTCTATCACCCCTGGAGAGCAGGCCTCCATCTCCTGC |
| 9 | GATATTGTGATGACCCAGACTCCACTCTCCTCGCCTGTCACCCTTGGACAGCCGGCCTCCATCTCCTTC |
| 10 | GAAATTGTGCTGACTCAGTCTCCAGACTTTCAGTCTGTGACTCCAAAGGAGAAAGTCACCATCACCTGC |
| 11 | GATGTTGTGATGACACAGTCTCCAGCTTTCCTCTCTGTGACTCCAGGGGAGAAAGTCACCATCACCTGC |
| 12 | GACATCCAGATGACCCAGTCTCCATCCTCCCTGTCTGCATCTGTAGGAGACAGAGTCACCATCACTTGC |
| 13 | GAAATTGTGCTGACTCAGTCTCCAGACTTTCAGTCTGTGACTCCAAAGGAGAAAGTCACCATCACCTGC |
| 14 | GACATCCAGATGACCCAGTCTCCATCCTCCCTGTCTGCATCTGTAGGAGACAGAGTCACCATCACTTGC |
| 15 | GAAACGACACTCACGCAGTCTCCAGCATTCATGTCAGCGACTCCAGGAGACAAAGTCAACATCTCCTGC |
| 16 | GACATCCAGATGACCCAGTCTCCATCCTCACTGTCTGCATCTGTAGGAGACAGAGTCACCATCACTTGT |
| 17 | GCCATCCAGATGACCCAGTCTCCATCCTCCCTGTCTGCATCTGTAGGAGACAGAGTCACCATCACTTGC |
| 18 | GACATCCAGATGACCCAGTCTCCTTCCACCCTGTCTGCATCTGTAGGAGACAGAGTCACCATCACTTGC |
| 19 | AACATCCAGATGACCCAGTCTCCATCTGCCATGTCTGCATCTGTAGGAGACAGAGTCACCATCACTTGT |
| 20 | GACATCCAGATGACCCAGTCTCCATCCTCACTGTCTGCATCTGTAGGAGACAGAGTCACCATCACTTGT |
| 21 | GAAATAGTGATGATGCAGTCTCCAGCCACCCTGTCTGTGTCTCCAGGGGAAAGAGCCACCCTCTCCTGC |
| 22 | GCCATCCAGTTGACCCAGTCTCCATCCTCCCTGTCTGCATCTGTAGGAGACAGAGTCACCATCACTTGC |
| 23 | GACATCCAGATGACCCAGTCTCCATCTTCTGTGTCTGCATCTGTAGGAGACAGAGTCACCATCACTTGT |
| 24 | GAAATAGTGATGACGCAGTCTCCAGCCACCCTGTCTGTGTCTCCAGGGGAAAGAGCCACCCTCTCCTGC |
| 25 | GAAATTGTGTTGACACAGTCTCCAGCCACCCTGTCTTTGTCTCCAGGGGAAAGAGCCACCCTCTCCTGC |
| 26 | GACATCCAGATGATCCAGTCTCCATCTTTCCTGTCTGCATCTGTAGGAGACAGAGTCAGTATCATTTGC |
| 27 | GCCATCCGGATGACCCAGTCTCCATTCTCCCTGTCTGCATCTGTAGGAGACAGAGTCACCATCACTTGC |
| 28 | GTCATCTGGATGACCCAGTCTCCATCCTTACTCTCTGCATCTACAGGAGACAGAGTCACCATCAGTTGT |
| 29 | GCCATCCAGTTGACCCAGTCTCCATCCTCCCTGTCTGCATCTGTAGGAGACAGAGTCACCATCACTTGC |
| 30 | GACATCCAGATGACCCAGTCTCCATCTTCCGTGTCTGCATCTGTAGGAGACAGAGTCACCATCACTTGT |
| 31 | GAAATTGTGTTGACACAGTCTCCAGCCACCCTGTCTTTGTCTCGAGGGGAAAGAGCCACCCTCTCCTGC |
| 32 | GACATCCAGTTGACCCAGTCTCCATCCTTCCTGTCTGCATCTGTAGGAGACAGAGTCACCATCACTTGC |
| 33 | GCCATCCGGATGACCCAGTCTCCATCCTCATTCTCTGCATCTACAGGAGACAGAGTCACCATCACTTGT |
| 34 | GACATCCAGATGACCCAGTCTCCATCCTCCCTGTCTGCATCTGTAGGAGACAGAGTCACCATCACTTGC |
| 35 | GACATCCAGTTGACCCAGTCTCCATCCTCCCTGTCTGCATCTGTAGGAGACAGAGTCACCATCAGTTGC |
| 36 | GACATCCAGATGAGCCAGTCTCCATCCTCCCTGTCTGCATCTGTAGGAGACAGAGTCACCATCACTTGC |
| 37 | GACATCCAGATGACCCAGTCTCCATCCTCCCTGTCTGCATCTGTAGGAGACAGAGTCACCATCACTTGC |
| 38 | GACATCCAGTTGACCCAGTCTCCATCCTCCCTGTCTGCATCTGTAGGAGACAGAGTCACCATCACTTGC |
| 39 | GACATCCAGATGACCCAGTCTCCATCCTCCCTGTCTGCATCTGTAGGAGACAGAGTCACCATCACTTGC |
| 40 | GAAATTGTAATGACACAGTCTCCACCCACCCTGTCTTTGTCTCCAGGGGAAAGAGTCACCCTCTCCTGC |
| 41 | GAAATTGTAATGACACAGTCTCCAGCCACCCTGTCTTTGTCTCCAGGGGAAAGAGCCACCCTCTCCTGC |
| 42 | GAAATTGTGTTGACGCAGTCTCCAGCCACCCTGTCTTTGTCTCCAGGGGAAAGAGCCACCCTCTCCTGC |
| 43 | GAAATTGTGTTGACGCAGTCTCCAGGCACCCTGTCTTTGTCTCCAGGGGAAAGAGCCACCCTCTCCTGC |
| 44 | GACATCGTGATGACCCAGTCTCCAGACTCCCTGGCTGTGTCTCTGGGCGAGAGGGCCACCATCAACTGC |
| 45 | GATATTGTGATGACCCAGACTCCACTCTCCCTGCCCGTCACCCCTGGAGAGCCGGCCTCCATCTCCTGC |
| 46 | GATATTGTGATGACCCAGACTCCACTCTCCCTGCCCGTCACCCCTGGAGAGCCGGCCTCCATCTCCTGC |

**Table 2. FR2 of Light Chains**

| | |
|---|---|
| 47 | TGGTTTCAGCAGAGGCCAGGCCAATCTCCAAGGCGCCTAATTTAT |
| 48 | TGGTTTCAGCAGAGGCCAGGCCAATCTCCAAGGCGCCTAATTTAT |
| 49 | TGGTACCTGCAGAAGCCAGGCCAGTCTCCACAGCTCCTGATCTAT |
| 50 | TGGTACCTGCAGAAGCCAGGGCAGTCTCCACAGCTCCTGATCTAT |
| 51 | TGGTACCTGCAGAAGCCAGGCCAGCCTCCACAGCTCCTGATCTAT |
| 52 | TGGCTTCAGCAGAGGCCAGGCCAGCCTCCAAGACTCCTAATTTAT |
| 53 | TGGTACCTGCAGAAGCCAGGGCAGTCTCCACAGCTCCTGATCTAT |
| 54 | TGGTTTCTGCAGAAAGCCAGGCCAGTCTCCACACTCCTGATCTAT |
| 55 | TGGCTTCAGCAGAGGCCAGGCCAGCCTCCAAGACTCCTAATTTAT |
| 56 | TGGTACCAGCAGAAACCAGATCAGTCTCCAAAGCTCCTCATCAAG |
| 57 | TGGTACCAGCAGAAACCAGATCAAGCCCCAAAGCTCGTCATCAAG |
| 58 | TGGTATCAGCAGAAACCAGGGAAAGTTCCTAAGCTCCTGATCTAT |
| 59 | TGGTACCAGCAGAAACCAGATCAGTCTCCAAAGCTCCTCATCAAG |
| 60 | TGGTATCAGCAGAAACCAGGGAAAGCCCCTAAGCGCCTGATCTAT |
| 61 | TGGTACCAACAGAAACCAGGAGAAGCTGCTATTTTCATTATTCAA |
| 62 | TGGTTTCAGCAGAAACCAGGGAAAGCCCCTAAGTCCCTGATCTAT |
| 63 | TGGTATCAGCAGAAACCAGGGAAAGCCCCTAAGCTCCTGATCTAT |
| 64 | TGGTATCAGCAGAAACCAGGGAAAGCCCCTAAGCTCCTGATCTAT |
| 65 | TGGTTTCAGCAGAAACCAGGGAAAGTCCCTAAGCACCTGATCTAT |
| 66 | TGGTATCAGCAGAAACCAGAGAAAGCCCCTAAGTCCCTGATCTAT |
| 67 | TGGTACCAGCAGAAACCTGGCCAGGCTCCCAGGCTCCTCATCTAT |
| 68 | TGGTATCAGCAGAAACCAGGGAAAGCTCCTAAGCTCCTGATCTAT |
| 69 | TGGTATCAGCAGAAACCAGGGAAAGCCCCTAAGCTCCTGATCTAT |
| 70 | TGGTACCAGCAGAAACCTGGCCAGGCTCCCAGGCTCCTCATCTAT |
| 71 | TGGTACCAGCAGAAACCTGGCCAGGCTCCCAGGCTCCTCATCTAT |
| 72 | TGGTATCTGCAGAAACCAGGGAAATCCCCTAAGCTCTTCCTCTAT |
| 73 | TGGTATCAGCAAAAACCAGCAAAAGCCCCTAAGCTCTTCATCTAT |
| 74 | TGGTATCAGCAAAAACCAGGGAAAGCCCCTGAGCTCCTGATCTAT |
| 75 | TGGTATCAGCAGAAACCAGGGAAAGCTCCTAAGCTCCTGATCTAT |
| 76 | TGGTATCAGCAGAAACCAGGGAAAGCCCCTAAGCTCCTGATCTAT |
| 77 | TGGTACCAACAGAAACCTGGCCAGGCTCCCAGGCTCCTCATCTAT |
| 78 | TGGTATCAGCAAAAACCAGGGAAAGCCCCTAAGCTCCTGATCTAT |
| 79 | TGGTATCAGCAAAAACCAGGGAAAGCCCCTAAGCTCCTGATCTAT |
| 80 | TGGTATCAGCAGAAACCAGGGAAAGCCCCTAAGCTCCTGATCTAT |
| 81 | TGGTATCGGCAGAAACCAGGGAAAGTTCCTAAGCTCCTGATCTAT |
| 82 | TGGTATCAGCAGAAACCAGGGAAAGCCCCTAAGCTCCTGATCTAC |
| 83 | TGGTATCAGCAGAAACCAGGGAAAGCCCCTAAGCTCCTGATCTAT |
| 84 | TGGTATCGGCAGAAACCAGGGAAAGTTCCTAAGCTCCTGATCTAT |
| 85 | TGGTATCAGCAGAAACCAGGGAAAGCCCCTAAGCTCCTGATCTAC |
| 86 | TGGTATCAGCAGAAACCTGGCCAGGCGCCCAGGCTCCTCATCTAT |
| 87 | TGGTACCAGCAGAAACCTGGGCAGGCTCCCAGGCTCCTCATCTAT |
| 88 | TGGTACCAGCAGAAACCTGGCCTGGCGCCCAGGCTCCTCATCTAT |
| 89 | TGGTACCAGCAGAAACCTGGCCAGGCTCCCAGGCTCCTCATCTAT |
| 90 | TGGTACCAGCAGAAACCAGGACAGCCTCCTAAGCTGCTCATTTAC |
| 91 | TGGTACCTGCAGAAGCCAGGGCAGTCTCCACAGCTCCTGATCTAT |
| 92 | TGGTACCTGCAGAAGCCAGGGCAGTCTCCACAGCTCCTGATCTAT |

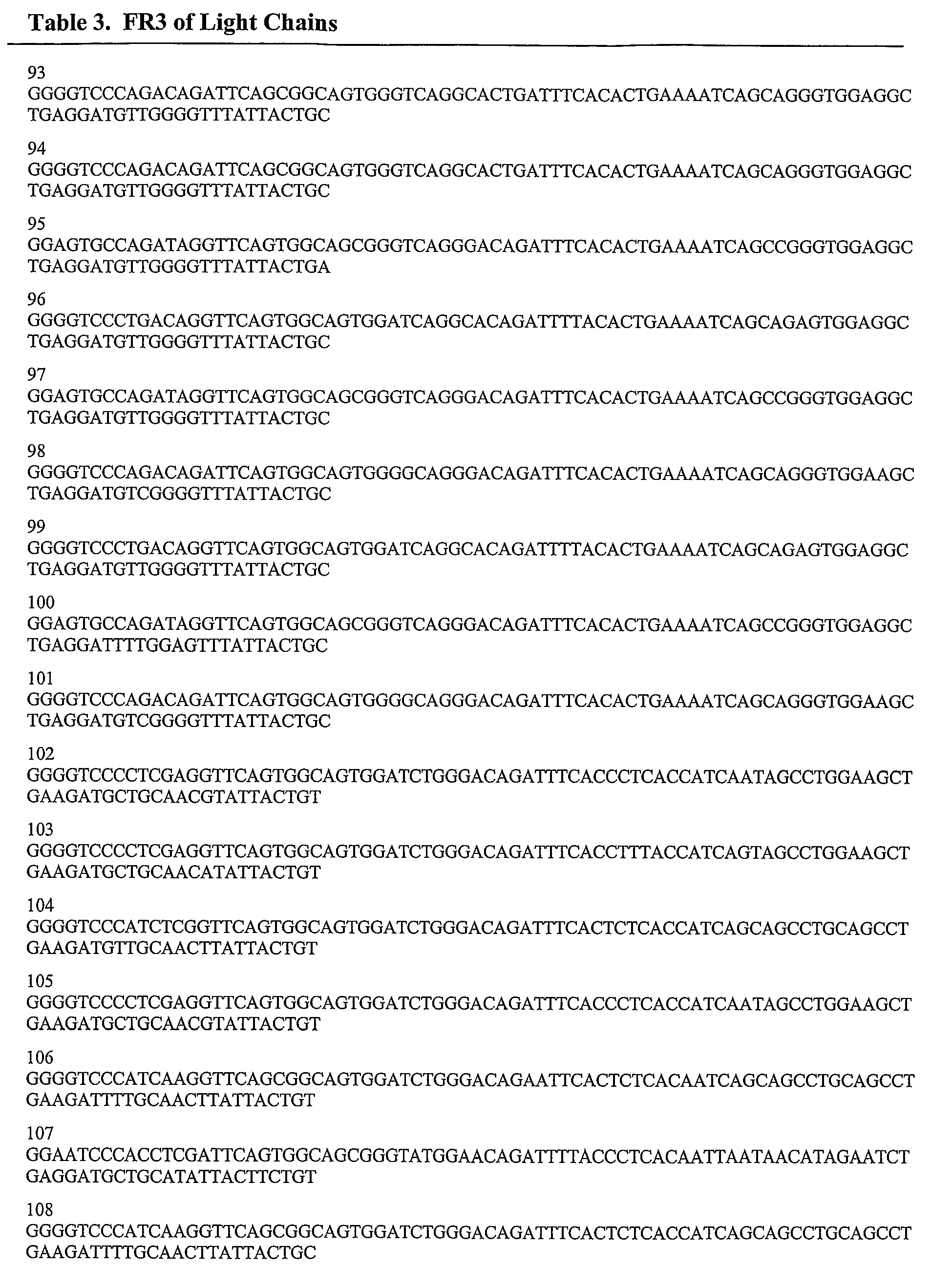

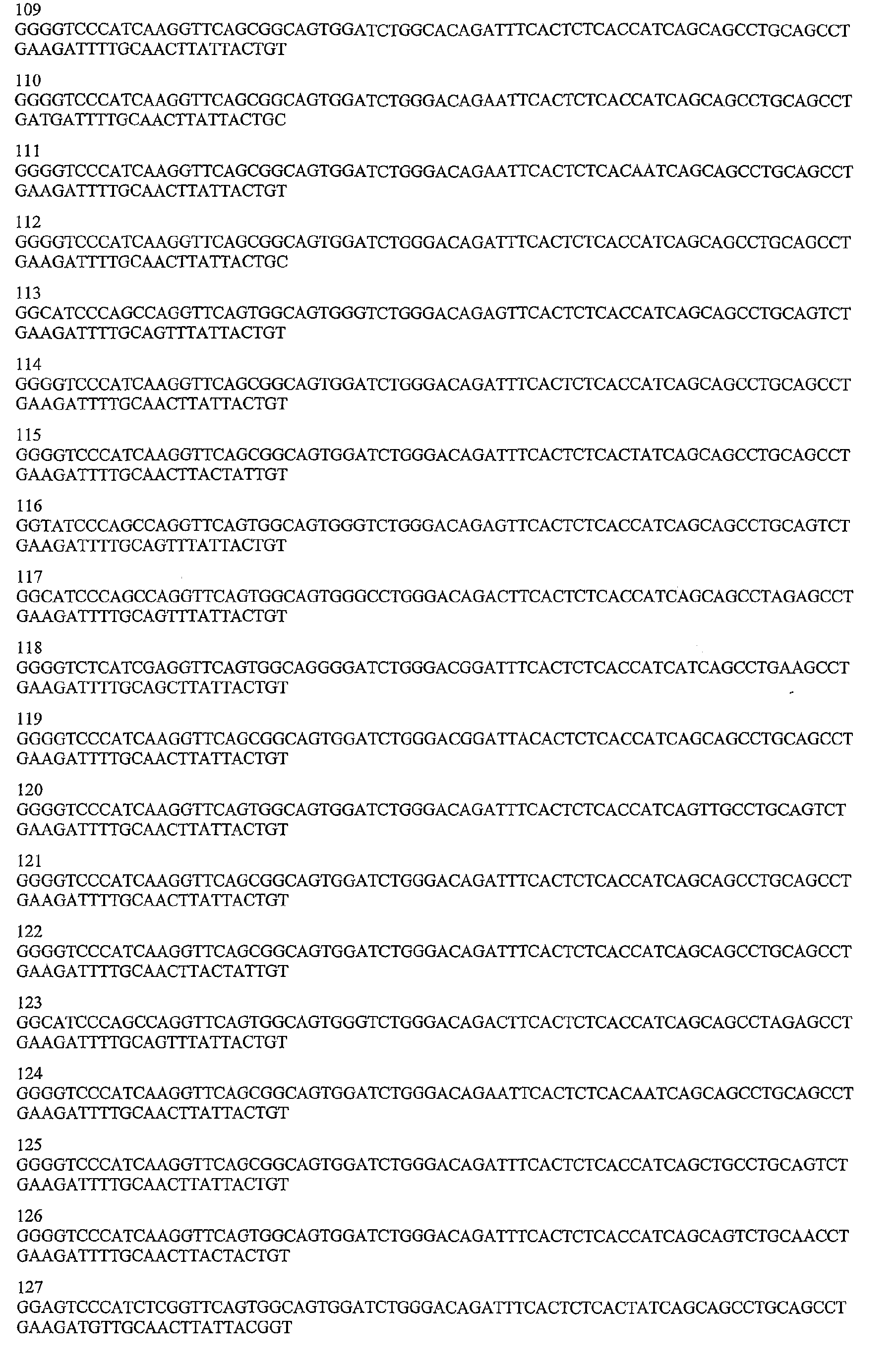

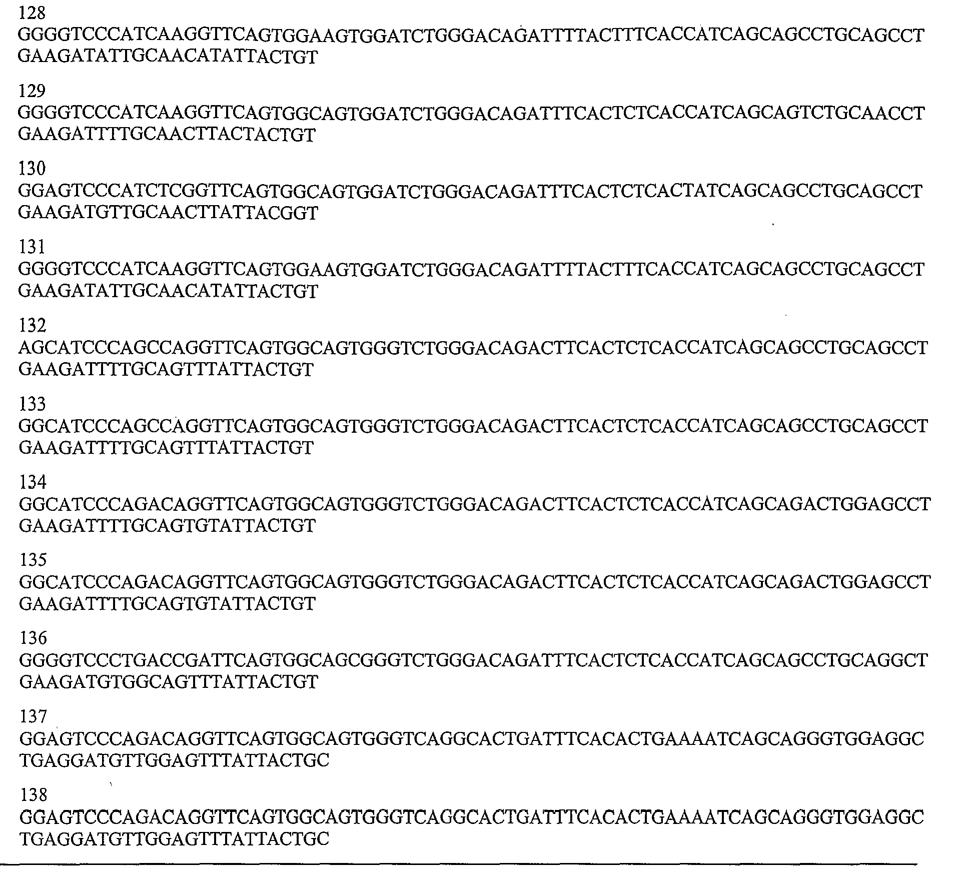

**Table 4. FR4 of Light Chains**

| | |
|---|---|
| 139 | TTCGGCCAAGGGACCAAGGTGGAAATCAAA |
| 140 | TTTGGCCAGGGGACCAAGCTGGAGATCAAA |
| 141 | TTCGGCCCTGGGACCAAAGTGGATATCAAA |
| 142 | TTCGGCGGAGGGACCAAGGTGGAGATCAAA |
| 143 | TTCGGCCAAGGGACACGACTGGAGATTAAA |

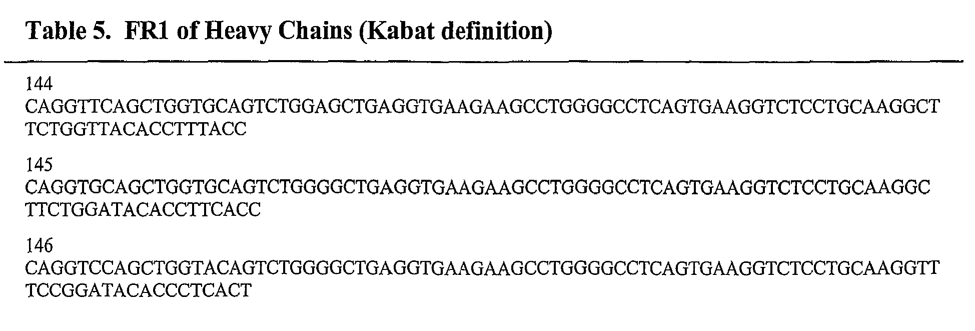

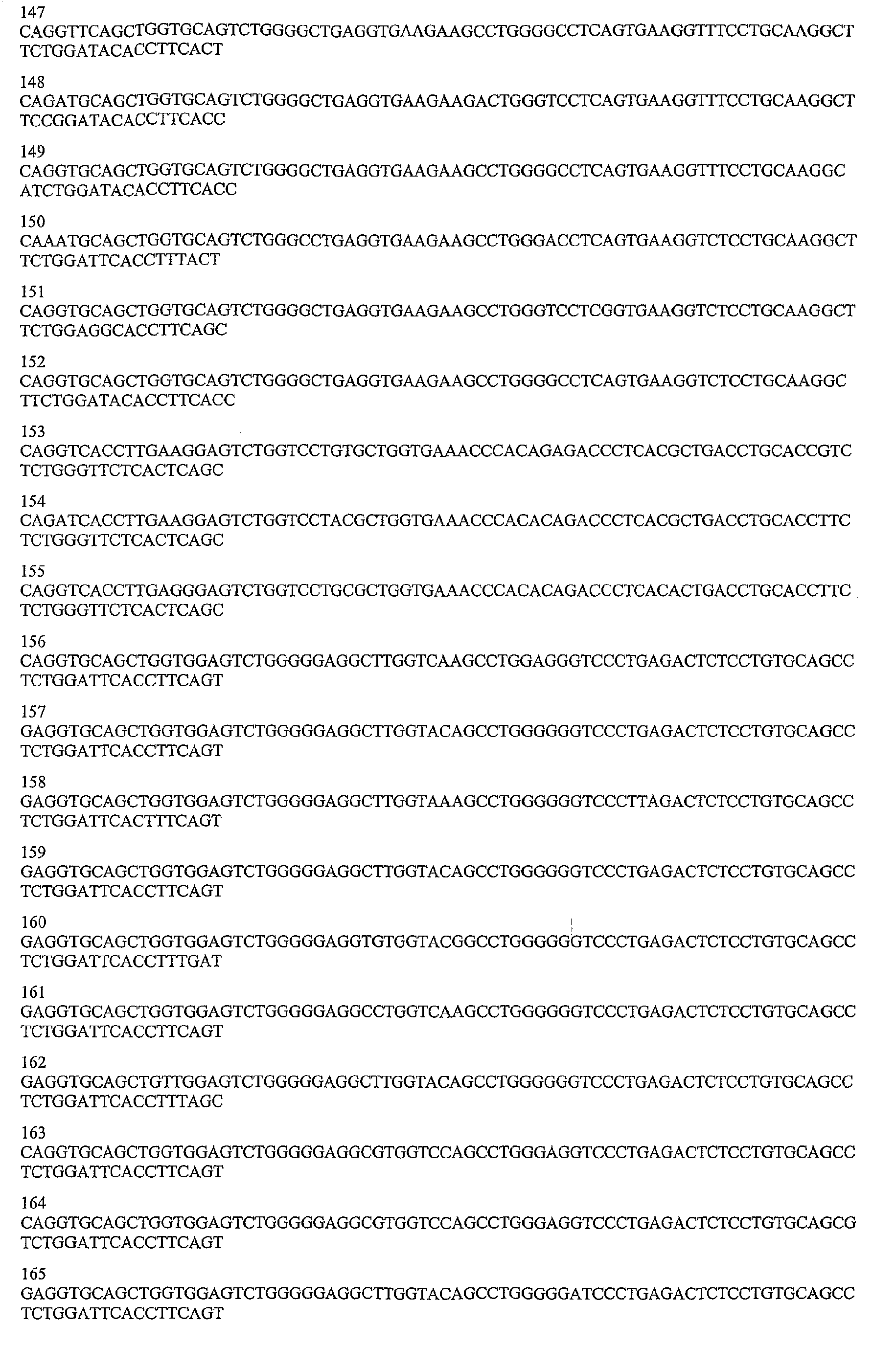

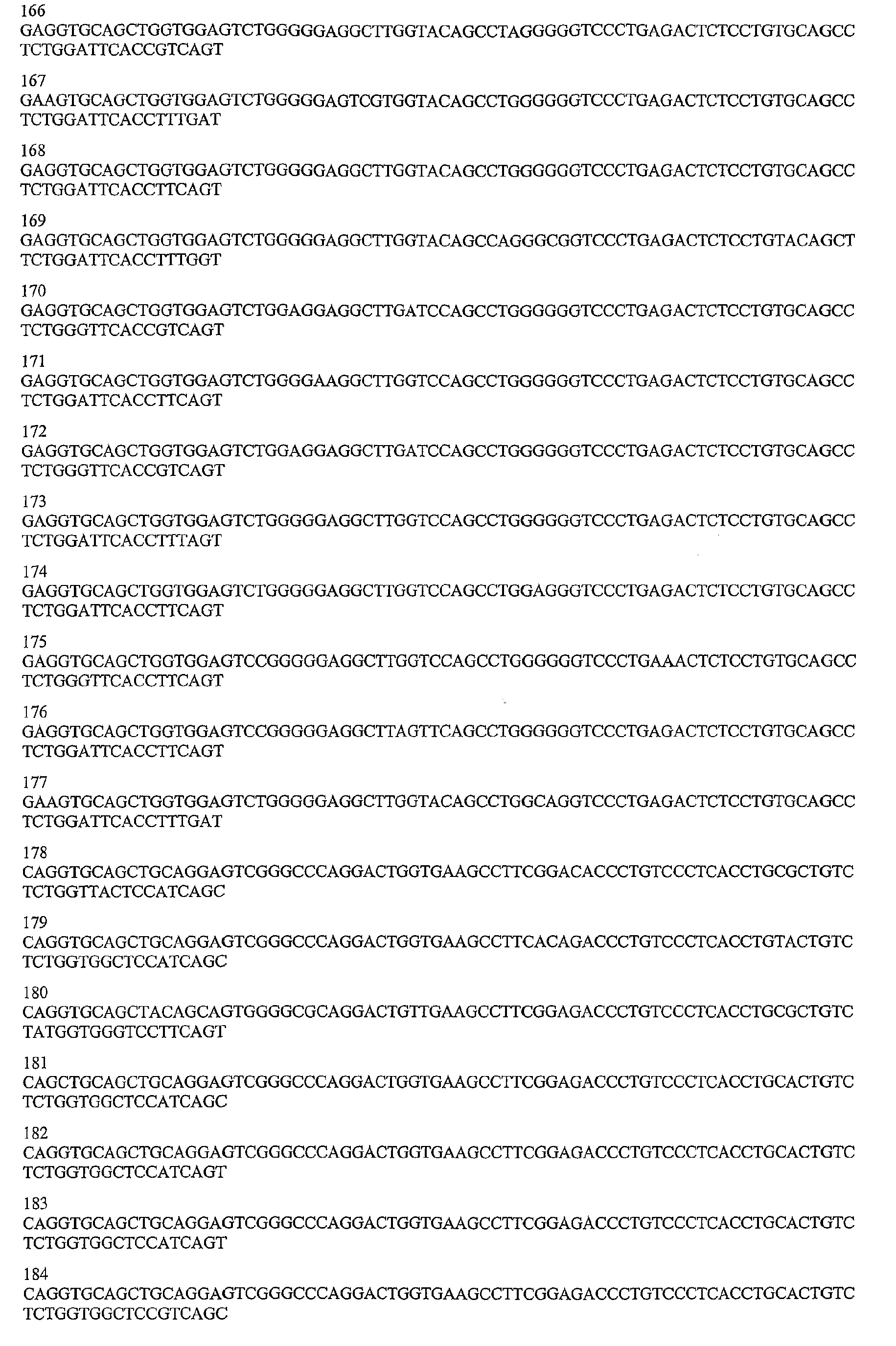

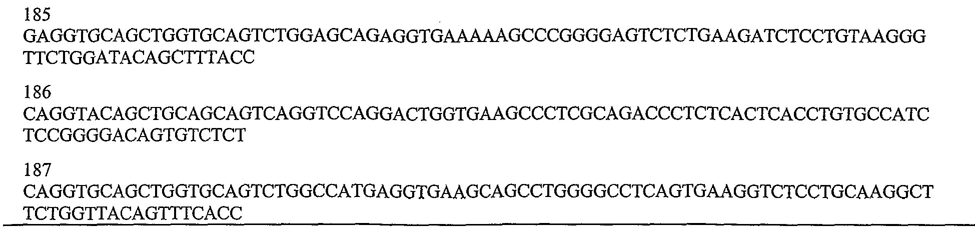

**Table 6. FR2 of Heavy Chains (Kabat definition)**

| | |
|---|---|
| 188 | TGGGTGCGACAGGCCCCTGGACAAGGGCTTGAGTGGATGGGA |
| 189 | TGGGTGCGACAGGCCCCTGGACAAGGGCTTGAGTGGATGGGA |
| 190 | TGGGTGCGACAGGCTCCTGGAAAAGGGCTTGAGTGGATGGGA |
| 191 | TGGGTGCGCCAGGCCCCCGGACAAAGGCTTGAGTGGATGGGA |
| 192 | TGGGTGCGACAGGCCCCCGGACAAGCGCTTGAGTGGATGGGA |
| 193 | TGGGTGCGACAGGCCCCTGGACAAGGGCTTGAGTGGATGGGA |
| 194 | TGGGTGCGACAGGCTCGTGGACAACGCCTTGAGTGGATAGGA |
| 195 | TGGGTGCGACAGGCCCCTGGACAAGGGCTTGAGTGGATGGGA |
| 196 | TGGGTGCGACAGGCCACTGGACAAGGGCTTGAGTGGATGGGA |
| 197 | TGGATCCGTCAGCCCCCAGGGAAGGCCCTGGAGTGGCTTGCA |
| 198 | TGGATCCGTCAGCCCCCAGGAAAGGCCCTGGAGTGGCTTGCA |
| 199 | TGGATCCGTCAGCCCCCAGGGAAGGCCCTGGAGTGGCTTGCA |
| 200 | TGGATCCGCCAGGCTCCAGGGAAGGGGCTGGAGTGGGTTTCA |
| 201 | TGGGTCCGCCAAGCTACAGGAAAAGGTCTGGAGTGGGTCTCA |
| 202 | TGGGTCCGCCAGGCTCCAGGGAAGGGGCTGGAGTGGGTTGGC |
| 203 | TGGGCCCGCAAGGCTCCAGGAAAGGGGCTGGAGTGGGTATCG |
| 204 | TGGGTCCGCCAAGCTCCAGGGAAGGGGCTGGAGTGGGTCTCT |
| 205 | TGGGTCCGCCAGGCTCCAGGGAAGGGGCTGGAGTGGGTCTCA |
| 206 | TGGGTCCGCCAGGCTCCAGGGAAGGGGCTGGAGTGGGTCTCA |
| 207 | TGGGTCCGCCAGGCTCCAGGCAAGGGGCTGGAGTGGGTGGCA |
| 208 | TGGGTCCGCCAGGCTCCAGGCAAGGGGCTGGAGTGGGTGGCA |
| 209 | TGGGTCCATCAGGCTCCAGGAAAGGGGCTGGAGTGGGTATCG |
| 210 | TGGATCCGCCAGGCTCCAGGGAAGGGGCTGGAGTGGGTCTCA |
| 211 | TGGGTCCGTCAAGCTCCGGGGAAGGGTCTGGAGTGGGTCTCT |
| 212 | TGGGTCCGCCAGGCTCCAGGGAAGGGGCTGGAGTGGGTTTCA |
| 213 | TGGTTCCGCCAGGCTCCAGGGAAGGGGCTGGAGTGGGTAGGT |
| 214 | TGGGTCCGCCAGGCTCCAGGGAAGGGGCTGGAGTGGGTCTCA |
| 215 | TGGGTCCGCCAGGCTCCAGGGAAGGGACTGGAATATGTTTCA |
| 216 | TGGGTCCGCCAGGCTCCAGGGAAGGGGCTGGAGTGGGTCTCA |
| 217 | TGGGTCCGCCAGGCTCCAGGGAAGGGGCTGGAGTGGGTGGCC |
| 218 | TGGGTCCGCCAGGCTCCAGGGAAGGGGCTGGAGTGGGTTGGC |
| 219 | TGGGTCCGCCAGGCTTCCGGGAAAGGGCTGGAGTGGGTTGGC |
| 220 | TGGGTCCGCCAAGCTCCAGGGAAGGGGCTGGTGTGGGTCTCA |
| 221 | TGGGTCCGGCAAGCTCCAGGGAAGGGCCTGGAGTGGGTCTCA |
| 222 | TGGATCCGGCAGCCCCCAGGGAAGGGACTGGAGTGGATTGGG |
| 223 | TGGATCCGCCAGCACCCAGGGAAGGGCCTGGAGTGGATTGGG |
| 224 | TGGATCCGCCAGCCCCCAGGGAAGGGGCTGGAGTGGATTGGG |
| 225 | TGGATCCGCCAGCCCCCAGGGAAGGGGCTGGAGTGGATTGGG |
| 226 | TGGATCCGGCAGCCCGCCGGGAAGGGACTGGAGTGGATTGGG |
| 227 | TGGATCCGGCAGCCCCCAGGGAAGGGACTGGAGTGGATTGGG |
| 228 | TGGATCCGGCAGCCCCCAGGGAAGGGACTGGAGTGGATTGGG |
| 229 | TGGGTGCGCCAGATGCCCGGGAAAGGCCTGGAGTGGATGGGG |
| 230 | TGGATCAGGCAGTCCCCATCGAGAGGCCTTGAGTGGCTGGGA |
| 231 | TGGGTGCCACAGGCCCCTGGACAAGGGCTTGAGTGGATGGGA |

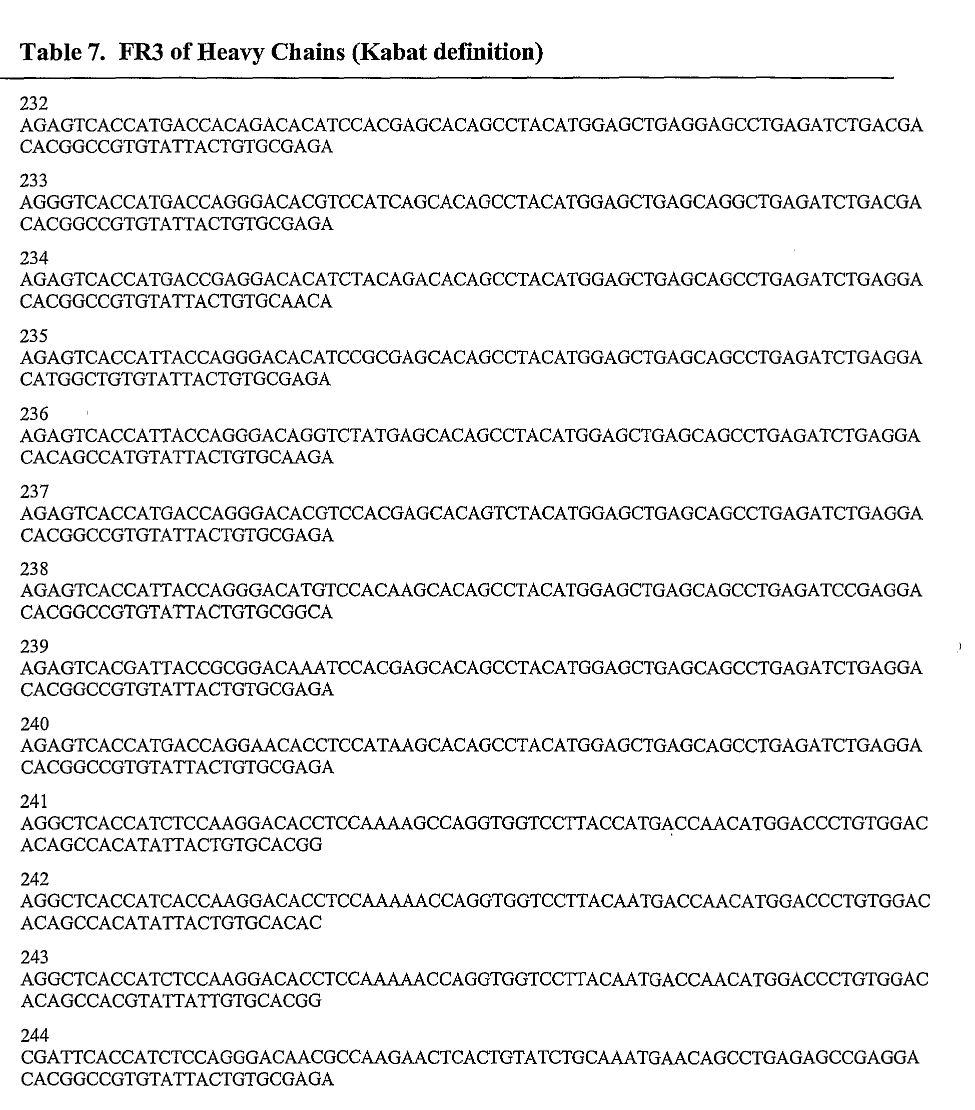

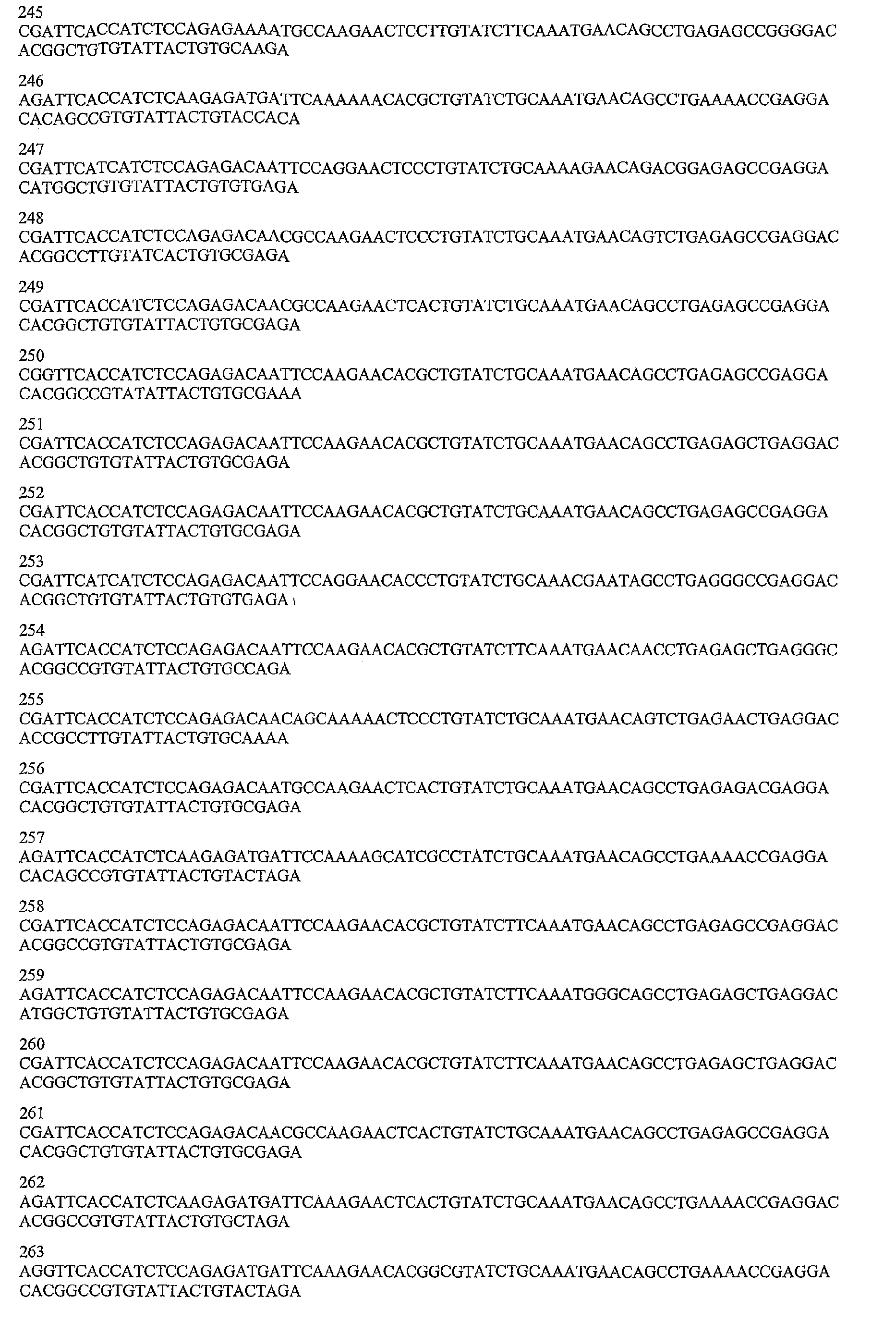

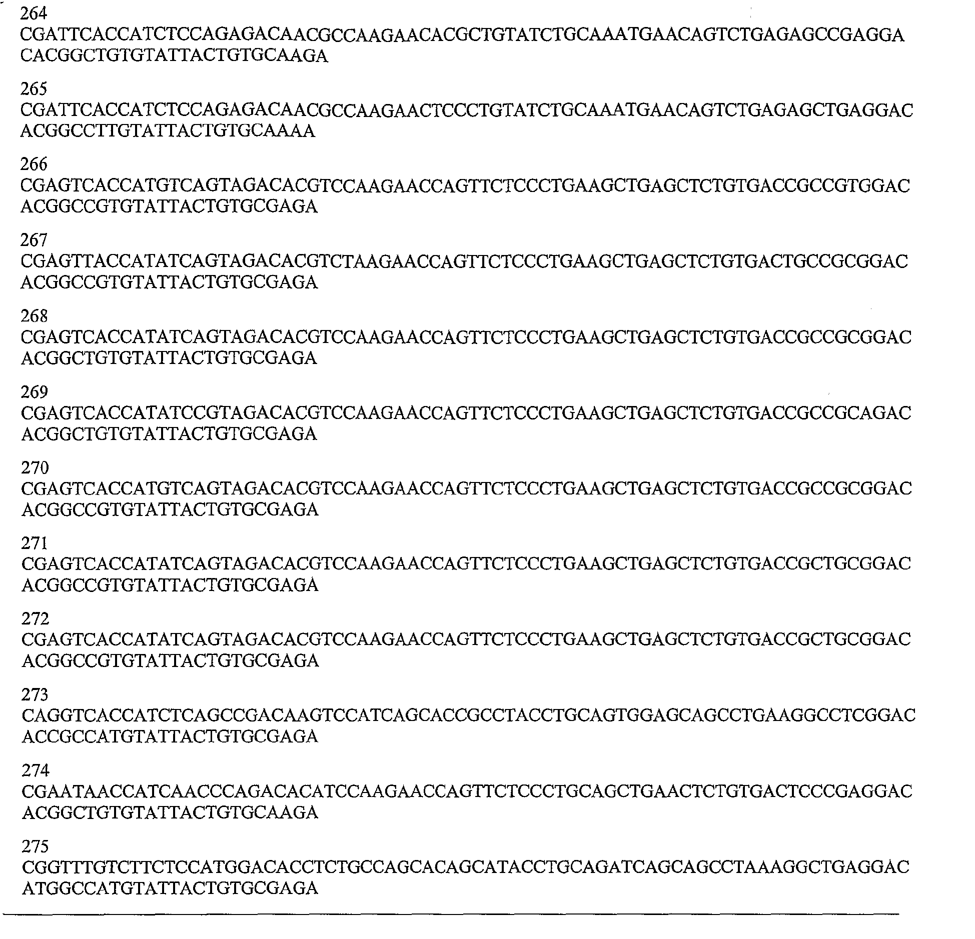

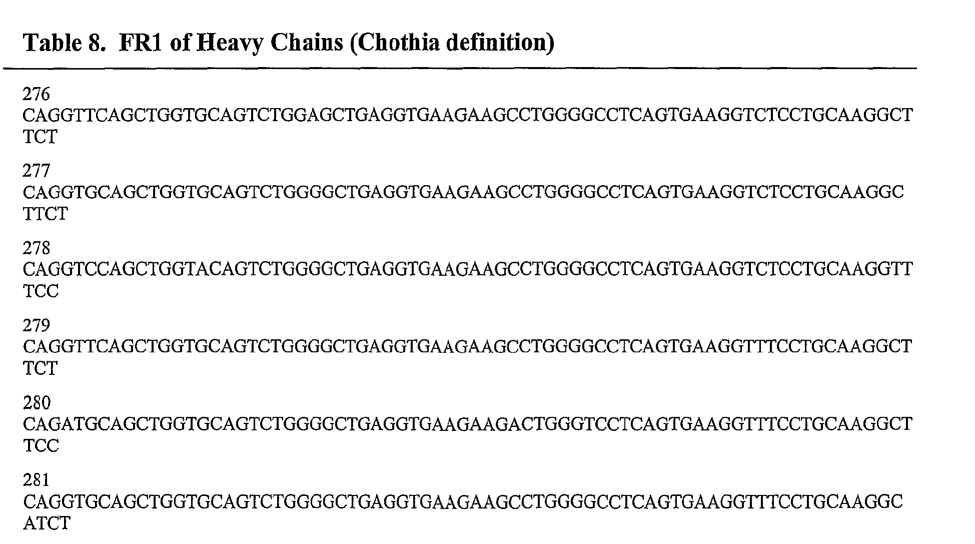

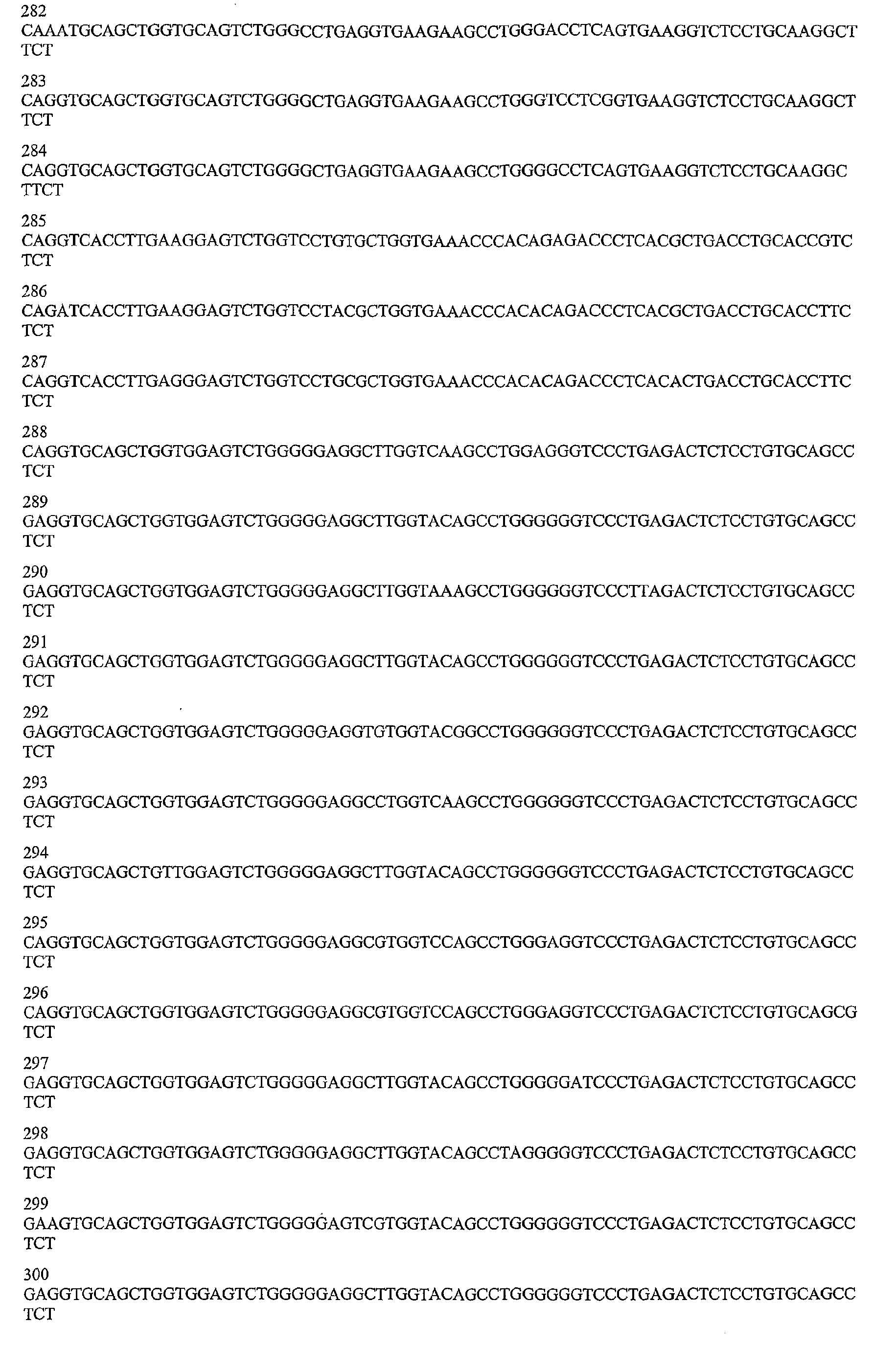

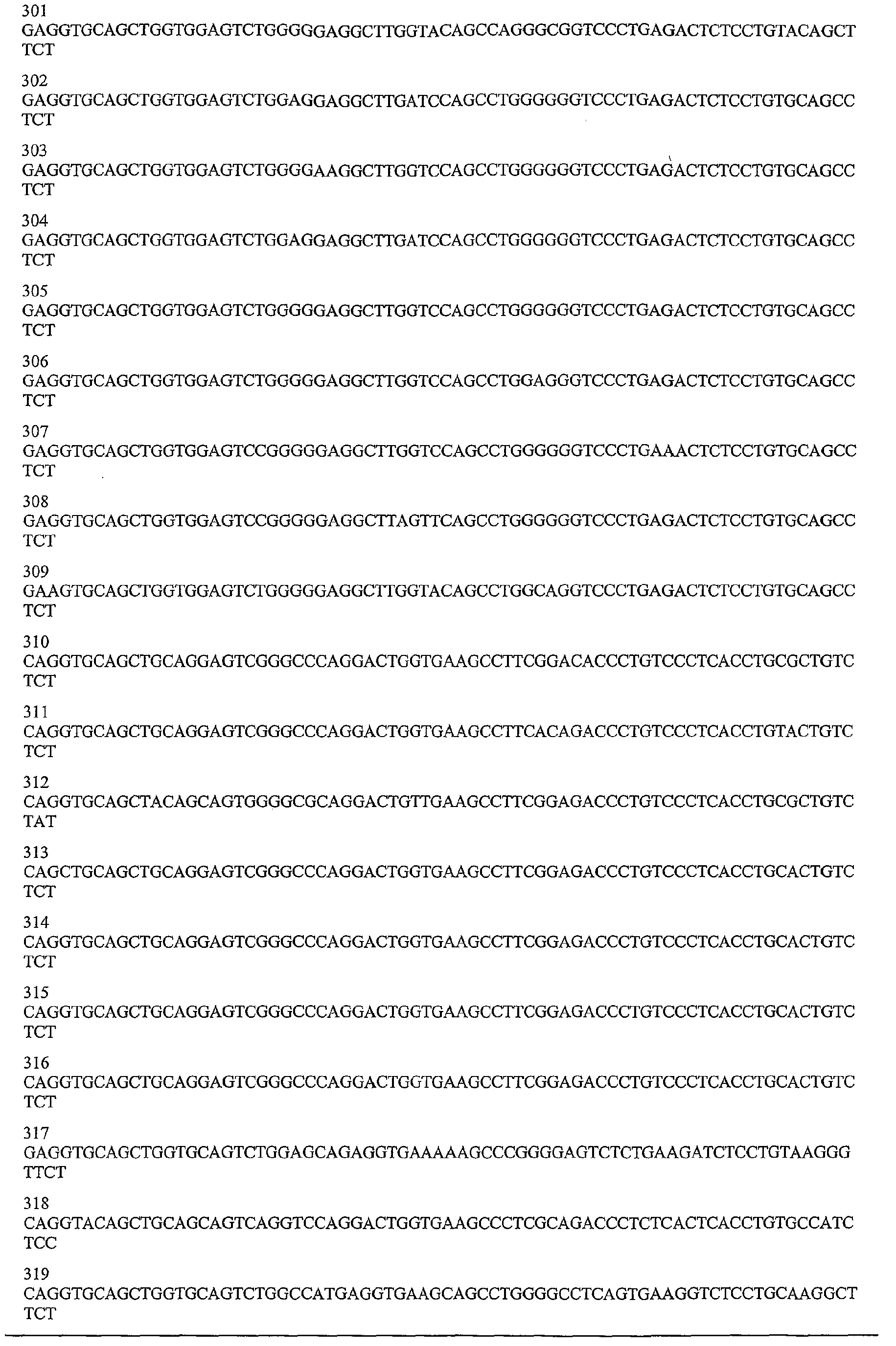

**Table 9. FR2 of Heavy Chains (Chothia definition)**

| | |
|---|---|
| 320 | TATGGTATCAGCTGGGTGCGACAGGCCCCTGGACAAGGGCTTGAGTGGATGGGATGGATC |
| 321 | TACTATATGCACTGGGTGCGACAGGCCCCTGGACAAGGGCTTGAGTGGATGGGATGGATC |
| 322 | TTATCCATGCACTGGGTGCGACAGGCTCCTGGAAAAGGGCTTGAGTGGATGGGAGGTTTT |
| 323 | TATGCTATGCATTGGGTGCGCCAGGCCCCCGGACAAAGGCTTGAGTGGATGGGATGGAGC |
| 324 | CGCTACCTGCACTGGGTGCGACAGGCCCCCGGACAAGCGCTTGAGTGGATGGGATGGATC |
| 325 | TACTATATGCACTGGGTGCGACAGGCCCCTGGACAAGGGCTTGAGTGGATGGGAATAATC |
| 326 | TCTGCTATGCAGTGGGTGCGACAGGCTCGTGGACAACGCCTTGAGTGGATAGGATGGATC |
| 327 | TATGCTATCAGCTGGGTGCGACAGGCCCCTGGACAAGGGCTTGAGTGGATGGGAGGGATC |
| 328 | TATGATATCAACTGGGTGCGACAGGCCACTGGACAAGGGCTTGAGTGGATGGGATGGATG |
| 329 | ATGGGTGTGAGCTGGATCCGTCAGCCCCCAGGGAAGGCCCTGGAGTGGCTTGCACACATT |
| 330 | GTGGGTGTGGGCTGGATCCGTCAGCCCCCAGGAAAGGCCCTGGAGTGGCTTGCACTCATT |
| 331 | ATGTGTGTGAGCTGGATCCGTCAGCCCCCAGGGAAGGCCCTGGAGTGGCTTGCACTCATT |
| 332 | TACTACATGAGCTGGATCCGCCAGGCTCCAGGGAAGGGGCTGGAGTGGGTTTCATACATT |
| 333 | TACGACATGCACTGGGTCCGCCAAGCTACAGGAAAAGGTCTGGAGTGGGTCTCAGCTATT |
| 334 | GCCTGGATGAGCTGGGTCCGCCAGGCTCCAGGGAAGGGGCTGGAGTGGGTTGGCCGTATT |
| 335 | AGTGACATGAACTGGGCCCGCAAGGCTCCAGGAAAGGGGCTGGAGTGGGTATCGGGTGTT |
| 336 | TATGGCATGAGCTGGGTCCGCCAAGCTCCAGGGAAGGGGCTGGAGTGGGTCTCTGGTATT |
| 337 | TATAGCATGAACTGGGTCCGCCAGGCTCCAGGGAAGGGGCTGGAGTGGGTCTCATCCATT |
| 338 | TATGCCATGAGCTGGGTCCGCCAGGCTCCAGGGAAGGGGCTGGAGTGGGTCTCAGCTATT |
| 339 | TATGGCATGCACTGGGTCCGCCAGGCTCCAGGCAAGGGGCTGGAGTGGGTGGCAGTTATA |
| 340 | TATGGCATGCACTGGGTCCGCCAGGCTCCAGGCAAGGGGCTGGAGTGGGTGGCAGTTATA |
| 341 | AGTGACATGAACTGGGTCCATCAGGCTCCAGGAAAGGGGCTGGAGTGGGTATCGGGTGTT |
| 342 | AATGAGATGAGCTGGATCCGCCAGGCTCCAGGGAAGGGGCTGGAGTGGGTCTCATCCATT |
| 343 | TATACCATGCACTGGGTCCGTCAAGCTCCGGGGAAGGGTCTGGAGTGGGTCTCTCTTATT |
| 344 | TATAGCATGAACTGGGTCCGCCAGGCTCCAGGGAAGGGGCTGGAGTGGGTTTCATACATT |
| 345 | TATGCTATGAGCTGGTTCCGCCAGGCTCCAGGGAAGGGGCTGGAGTGGGTAGGTTTCATT |
| 346 | AACTACATGAGCTGGGTCCGCCAGGCTCCAGGGAAGGGGCTGGAGTGGGTCTCAGTTATT |
| 347 | TATGCTATGCACTGGGTCCGCCAGGCTCCAGGGAAGGGACTGGAATATGTTTCAGCTATT |
| 348 | AACTACATGAGCTGGGTCCGCCAGGCTCCAGGGAAGGGGCTGGAGTGGGTCTCAGTTATT |
| 349 | TATTGGATGAGCTGGGTCCGCCAGGCTCCAGGGAAGGGGCTGGAGTGGGTGGCCAACATA |
| 350 | CACTACATGGACTGGGTCCGCCAGGCTCCAGGGAAGGGGCTGGAGTGGGTTGGCCGTACT |
| 351 | TCTGCTATGCACTGGGTCCGCCAGGCTTCCGGGAAAGGGCTGGAGTGGGTTGGCCGTATT |
| 352 | TACTGGATGCACTGGGTCCGCCAAGCTCCAGGGAAGGGGCTGGTGTGGGTCTCACGTATT |
| 353 | TATGCCATGCACTGGGTCCGGCAAGCTCCAGGGAAGGGCCTGGAGTGGGTCTCAGGTATT |
| 354 | AACTGGTGGGGCTGGATCCGGCAGCCCCCAGGGAAGGGACTGGAGTGGATTGGGTACATC |
| 355 | TACTACTGGAGCTGGATCCGCCAGCACCCAGGGAAGGGCCTGGAGTGGATTGGGTACATC |
| 356 | TACTACTGGAGCTGGATCCGCCAGCCCCCAGGGAAGGGGCTGGAGTGGATTGGGGAAATC |
| 357 | TACTACTGGGGCTGGATCCGCCAGCCCCCAGGGAAGGGGCTGGAGTGGATTGGGAGTATC |
| 358 | TACTACTGGAGCTGGATCCGGCAGCCCGCCGGGAAGGGACTGGAGTGGATTGGGCGTATC |
| 359 | TACTACTGGAGCTGGATCCGGCAGCCCCCAGGGAAGGGACTGGAGTGGATTGGGTATATC |
| 360 | TACTACTGGAGCTGGATCCGGCAGCCCCCAGGGAAGGGACTGGAGTGGATTGGGTATATC |
| 361 | TACTGGATCGGCTGGGTGCGCCAGATGCCCGGGAAAGGCCTGGAGTGGATGGGGATCATC |
| 362 | GCTGCTTGGAACTGGATCAGGCAGTCCCCATCGAGAGGCCTTGAGTGGCTGGGAAGGACA |
| 363 | TATGGTATGAATTGGGTGCCACAGGCCCCTGGACAAGGGCTTGAGTGGATGGGATGGTTC |

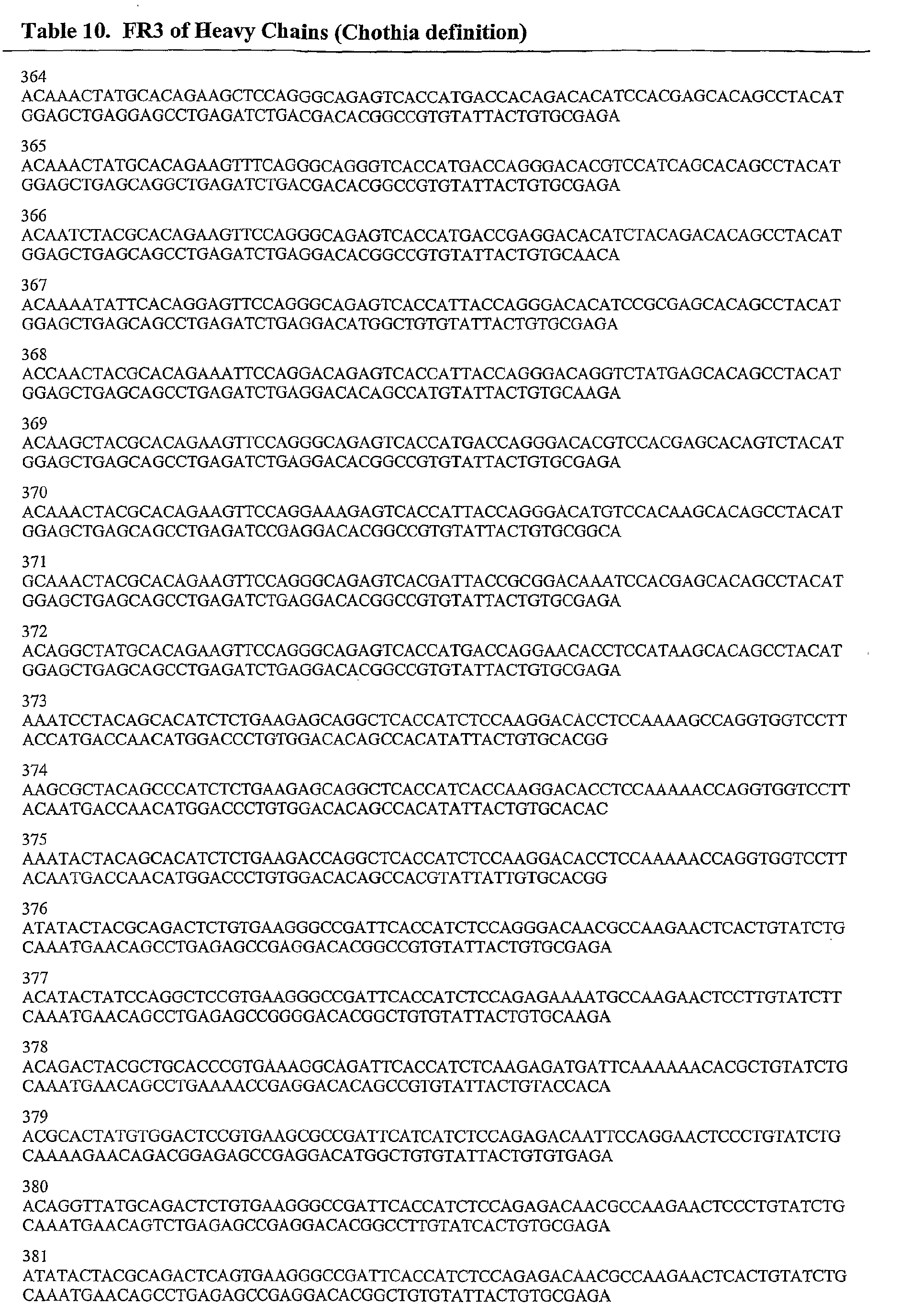

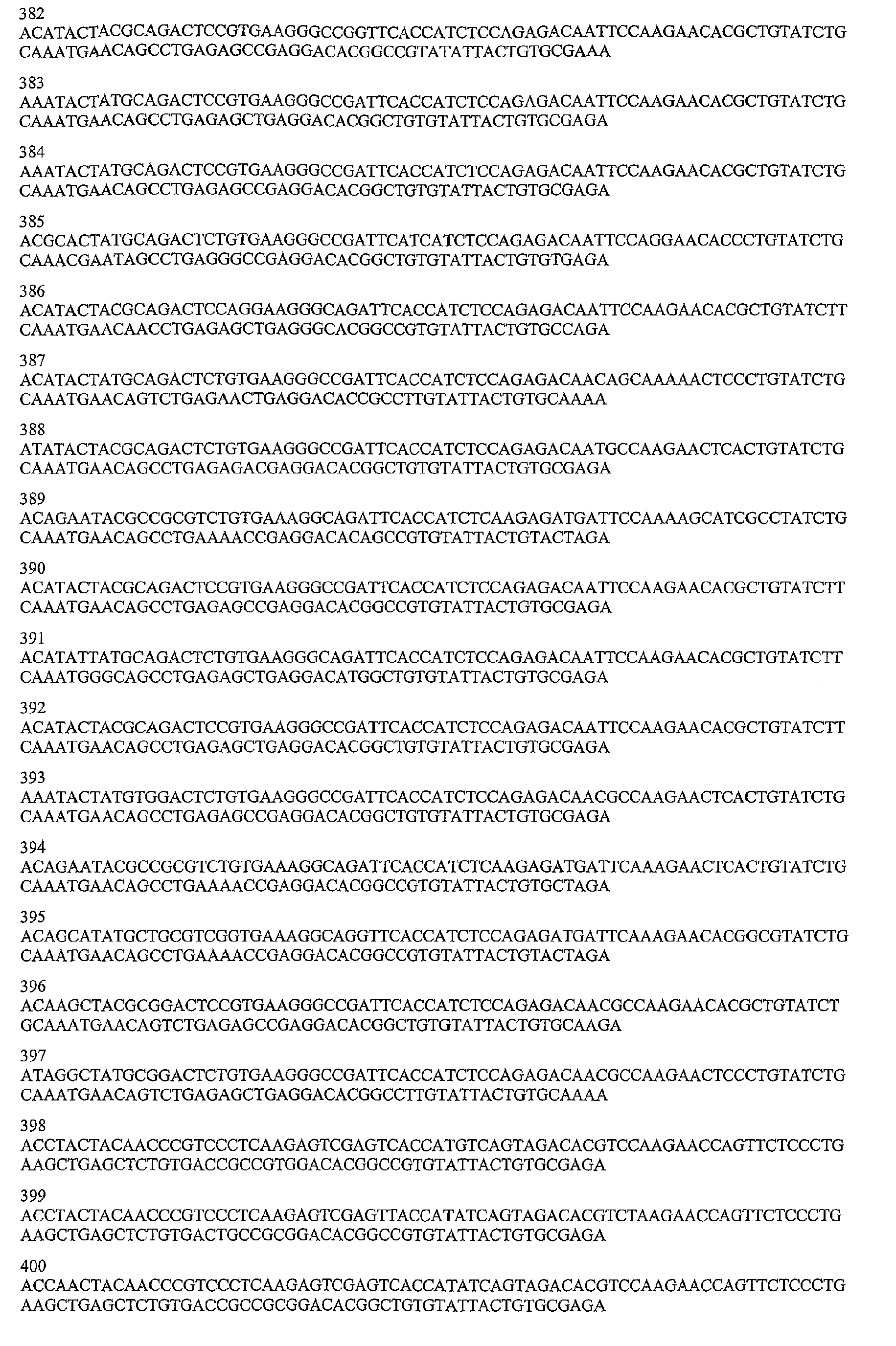

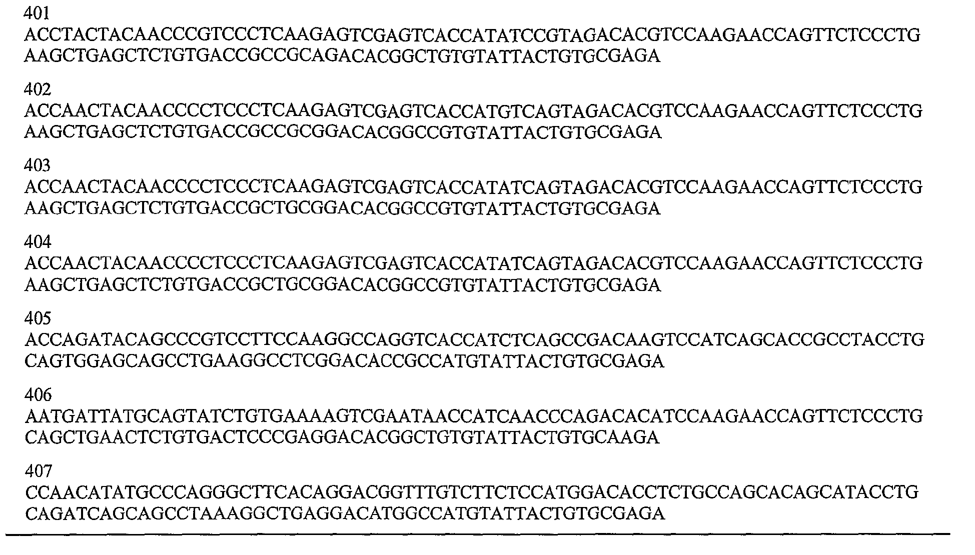

**Table 11. FR4 of Heavy Chain**

| | |
|---|---|
| 408 | TGGGGCCAGGGCACCCTGGTCACCGTCTCCTCA |
| 409 | TGGGGCCGTGGCACCCTGGTCACTGTCTCCTCA |
| 410 | TGGGGCCAAGGGACAATGGTCACCGTCTCTTCA |
| 411 | TGGGGCCAAGGAACCCTGGTCACCGTCTCCTCA |
| 412 | TGGGGCCAAGGAACCCTGGTCACCGTCTCCTCA |
| 413 | TGGGGGCAAGGGACCACGGTCACCGTCTCCTCA |

As used herein, the term "germline antibody gene" or "gene fragment" refers to an immunoglobulin sequence encoded by non-lymphoid cells that have not undergone the maturation process that leads to genetic rearrangement and mutation for expression of a particular immunoglobulin. (*See, e.g.,* Shapiro et al., Crit. Rev. Immunol. 22(3):183-200 (2002); Marchalonis et al., Adv Exp Med Biol. 484:13-30 (2001)). One of the advantages provided by various embodiments of the present invention stems from the recognition that germline antibody genes are more likely than mature antibody genes to conserve essential amino acid sequence structures characteristic of individuals in the species, hence less likely to be recognized as from a foreign source when used therapeutically in that species.

As used herein, the term "humanized antibody" is an antibody or a variant, derivative, analog or fragment thereof which immunospecifically binds to an antigen of interest and which comprises a framework (FR) region having substantially the amino acid sequence of a human antibody and a complementarity determining region (CDR) having substantially the amino acid sequence of a non-human antibody. As used herein, the term "substantially" in the context of a CDR refers to a CDR having an amino acid sequence at least 80%, preferably at least 85%, at least 90%, at least 95%, at least 98% or at least 99% identical to the amino acid sequence of a non-human antibody CDR. A humanized antibody comprises substantially all of at least one, and typically two, variable domains (Fab, Fab', F(ab')₂, FabC, Fv) in which all or substantially all of the CDR regions correspond to those of a non-human immunoglobulin (*i.e*., donor antibody) and all or substantially all of the framework regions are those of a human immunoglobulin sequence. Preferably, a humanized antibody also comprises at least a portion of an immunoglobulin constant region (Fc), typically that of a human immunoglobulin. In some embodiments, a humanized antibody contains both the light chain as well as at least the variable domain of a heavy chain. The antibody also may include the CH1, hinge, CH2, CH3, and CH4 regions of the heavy chain. In some embodiments, a humanized antibody only contains a humanized light chain. In some embodiments, a humanized antibody only contains a humanized heavy chain. In specific embodiments, a humanized antibody only contains a humanized variable domain of a light chain and/or humanized heavy chain.

The humanized antibody can be selected from any class of immunoglobulins, including IgM, IgG, IgD, IgA and IgE, and any isotype, including without limitation IgG₁, IgG₂, IgG₃ and IgG₄. The humanized antibody may comprise sequences from more than one class or isotype, and particular constant domains may be selected to optimize desired effector functions using techniques well-known in the art.

The framework and CDR regions of a humanized antibody need not correspond precisely to the parental sequences, *e.g*., the donor antibody CDR or the acceptor framework may be mutagenized by substitution, insertion and/or deletion of at least one amino acid residue so that the CDR or framework residue at that site does not correspond to either the donor antibody or the acceptor framework. Such mutations, however, will not be extensive. Usually, at least 80%, preferably at least 85%, more preferably at least 90%, and most preferably at least 95% of the humanized antibody residues will correspond to those of the parental FR and CDR sequences.

As used herein, the term "host cell" includes a to the particular subject cell transfected or transformed with a nucleic acid molecule and the progeny or potential progeny of such a cell. Progeny of such a cell may not be identical to the parent cell transfected with the nucleic acid molecule due to mutations or environmental influences that may occur in succeeding generations or integration of the nucleic acid molecule into the host cell genome.

As used herein, the term "immunospecifically binds to an antigen" and analogous terms refer to peptides, polypeptides, proteins (including, but not limited to fusion proteins and antibodies) or fragments thereof that specifically bind to an antigen or a fragment and do not specifically bind to other antigens. A peptide, polypeptide, or protein that immunospecifically binds to an antigen may bind to other antigens with lower affinity as determined by, *e.g*., immunoassays, BIAcore, or other assays known in the art. Antibodies or fragments that immunospecifically bind to an antigen may be cross-reactive with related antigens. Preferably, antibodies or fragments that immunospecifically bind to an antigen do not cross-react with other antigens.

As used herein, the term "isolated" in the context of a proteinaceous agent (*e.g*., a peptide, polypeptide or protein (such as fusion protein or antibody)) refers to a proteinaceous agent which is substantially free of cellular material or contaminating proteins, polypeptides, peptides and antibodies from the cell or tissue source from which it is derived, or substantially free of chemical precursors or other chemicals when chemically synthesized. The language "substantially free of cellular material" includes preparations of a proteinaceous agent in which the proteinaceous agent is separated from cellular components of the cells from which it is isolated or recombinantly produced. Thus, a proteinaceous agent that is substantially free of cellular material includes preparations of a proteinaceous agent having less than about 30%, 20%, 10%, or 5% (by dry weight) of heterologous protein, polypeptide or peptide (also referred to as a "contaminating protein"). When the proteinaceous agent is recombinantly produced, it is also preferably substantially free of culture medium, *i*.*e*., culture medium represents less than about 20%, 10%, or 5% of the volume of the proteinaceous agent preparation. When the proteinaceous agent is produced by chemical synthesis, it is preferably substantially free of chemical precursors or other chemicals, *i.e.,* it is separated from chemical precursors or other chemicals which are involved in the synthesis of the proteinaceous agent. Accordingly, such preparations of a proteinaceous agent have less than about 30%, 20%, 10%, 5% (by dry weight) of chemical precursors or compounds other than the proteinaceous agent of interest. In a specific embodiment, proteinaceous agents disclosed herein are isolated. In a preferred embodiment, an antibody of the invention is isolated.

As used herein, the term "isolated" in the context of nucleic acid molecules refers to a nucleic acid molecule which is separated from other nucleic acid molecules which are present in the natural source of the nucleic acid molecule. Moreover, an "isolated" nucleic acid molecule, such as a cDNA molecule, is preferably substantially free of other cellular material, or culture medium when produced by recombinant techniques, or substantially free of chemical precursors or other chemicals when chemically synthesized. In a specific embodiment, nucleic acid molecules are isolated. In a preferred embodiment, a nucleic acid molecule encoding an antibody of the invention is isolated. As used herein, the term "substantially free" refers to the preparation of the "isolated" nucleic acid having less than about 30%, 20%, 10%, or 5% (by dry weight) of heterologous nucleic acids, and preferably other cellular material, culture medium, chemical precursors, or other chemicals.

As used herein, the term "in combination" refers to the use of more than one therapies (*e.g*., more than one prophylactic agent and/or therapeutic agent). The use of the term "in combination" does not restrict the order in which therapies (*e.g*., prophylactic and/or therapeutic agents) are administered to a subject. A first therapy (*e.g*., a first prophylactic or therapeutic agent) can be administered prior to (*e.g*., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (*e.g*., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second therapy (*e.g*., a second prophylactic or therapeutic agent) to a subject.

As used herein, the terms "manage," "managing," and "management" refer to the beneficial effects that a subject derives from a therapy (*e.g*., a prophylactic or therapeutic agent), which does not result in a cure of the disease. In certain embodiments, a subject is administered one or more therapies (*e.g*., one or more prophylactic or therapeutic agents) to "manage" a disease so as to prevent the progression or worsening of the disease.

As used herein, the term "mature antibody gene" refers to a genetic sequence encoding an immunoglobulin that is expressed, for example, in a lymphocyte such as a B cell, in a hybridoma or in any antibody producing cell that has undergone a maturation process so that the particular immunoglobulin is expressed. The term includes mature genomic DNA, cDNA and other nucleic acid sequences that encode such mature genes, which have been isolated and/or recombinantly engineered for expression in other cell types. Mature antibody genes have undergone various mutations and rearrangements that structurally distinguish them from antibody genes encoded in all cells other than lymphocytes. Mature antibody genes in humans, rodents, and many other mammals are formed by fusion of V and J gene segments in the case of antibody light chains and fusion of V, D, and J gene segments in the case of antibody heavy chains. Many mature antibody genes acquire point mutations subsequent to fusion, some of which increase the affinity of the antibody protein for a specific antigen.

As used herein, the term "pharmaceutically acceptable" refers approved by a regulatory agency of the federal or a state government, or listed in the U.S. Pharmacopeia, European Pharmacopeia, or other generally recognized pharmacopeia for use in animals, and more particularly, in humans.

As used herein, the terms "prevent," "preventing," and "prevention" refer to the inhibition of the development or onset of a disorder or the prevention of the recurrence, onset, or development of one or more symptoms of a disorder in a subject resulting from the administration of a therapy (*e.g*., a prophylactic or therapeutic agent), or the administration of a combination of therapies (*e.g*., a combination of prophylactic or therapeutic agents).

As used herein, the terms "prophylactic agent" and "prophylactic agents" refer to any agent(s) which can be used in the prevention of a disorder or one or more of the symptoms thereof. In certain embodiments, the term "prophylactic agent" refers to an antibody of the invention. In certain other embodiments, the term "prophylactic agent" refers to an agent other than an antibody of the invention. Preferably, a prophylactic agent is an agent which is known to be useful to or has been or is currently being used to the prevent or impede the onset, development, progression and/or severity of a disorder or one or more symptoms thereof.

As used herein, the term "prophylactically effective amount" refers to the amount of a therapy (*e.g*., prophylactic agent) which is sufficient to result in the prevention of the development, recurrence, or onset of a disorder or one or more symptoms thereof, or to enhance or improve the prophylactic effect(s) of another therapy (*e.g*., a prophylactic agent).

As used herein, the phrase "protocol" refers to a regimen for dosing and timing the administration of one or more therapies (*e.g*., therapeutic agents) that has a therapeutic effective.

As used herein, the phrase "side effects" encompasses unwanted and adverse effects of a prophylactic or therapeutic agent. Side effects are always unwanted, but unwanted effects are not necessarily adverse. An adverse effect from a therapy (*e*.*g*., a prophylactic or therapeutic agent) might be harmful, uncomfortable, or risky.

As used herein, the term "small molecules" and analogous terms include, but are not limited to, peptides, peptidomimetics, amino acids, amino acid analogs, polynucleotides, polynucleotide analogs, nucleotides, nucleotide analogs, organic or inorganic compounds (*i*.*e*., including heteroorganic and organometallic compounds) having a molecular weight less than about 10,000 grams per mole, organic or inorganic compounds having a molecular weight less than about 5,000 grams per mole, organic or inorganic compounds having a molecular weight less than about 1,000 grams per mole, organic or inorganic compounds having a molecular weight less than about 500 grams per mole, and salts, esters, and other pharmaceutically acceptable forms of such agents.

As used herein, the terms "subject" and "patient" are used interchangeably. As used herein, the terms "subject" and "subjects" refer to an animal, preferably a mammal including a non-primate (*e.g*., a cow, pig, horse, cat, dog, rat, and mouse) and a primate (*e.g*., a monkey, such as a cynomolgous monkey, a chimpanzee, and a human), and most preferably a human. In one embodiment, the subject is a non-human animal such as a bird (*e.g.,* a quail, chicken, or turkey), a farm animal (*e.g*., a cow, horse, pig, or sheep), a pet (*e.g.,* a cat, dog, or guinea pig), or laboratory animal (*e.g*., an animal model for a disorder). In a preferred embodiment, the subject is a human (*e.g*., an infant, child, adult, or senior citizen).

As used herein, the term "synergistic" refers to a combination of therapies (*e.g*., prophylactic or therapeutic agents) which is more effective than the additive effects of any two or more single therapies (*e.g*., one or more prophylactic or therapeutic agents). A synergistic effect of a combination of therapies (*e.g*., a combination of prophylactic or therapeutic agents) permits the use of lower dosages of one or more of therapies (*e.g*., one or more prophylactic or therapeutic agents) and/or less frequent administration of said therapies to a subject with a disorder. The ability to utilize lower dosages of therapies (*e.g*., prophylactic or therapeutic agents) and/or to administer said therapies less frequently reduces the toxicity associated with the administration of said therapies to a subject without reducing the efficacy of said therapies in the prevention or treatment of a disorder. In addition, a synergistic effect can result in improved efficacy of therapies (*e.g*., prophylactic or therapeutic agents) in the prevention or treatment of a disorder. Finally, synergistic effect of a combination of therapies (*e.g*., prophylactic or therapeutic agents) may avoid or reduce adverse or unwanted side effects associated with the use of any single therapy.

As used herein, the terms "therapeutic agent" and "therapeutic agents" refer to any agent(s) which can be used in the prevention, treatment, management, or amelioration of a disorder or one or more symptoms thereof. In certain embodiments, the term "therapeutic agent" refers to an antibody of the invention. In certain other embodiments, the term "therapeutic agent" refers an agent other than an antibody of the invention. Preferably, a therapeutic agent is an agent which is known to be useful for, or has been or is currently being used for the prevention, treatment, management, or amelioration of a disorder or one or more symptoms thereof.

As used herein, the term "therapeutically effective amount" refers to the amount of a therapy (*e.g*., an antibody of the invention), which is sufficient to reduce the severity of a disorder, reduce the duration of a disorder, ameliorate one or more symptoms of a disorder, prevent the advancement of a disorder, cause regression of a disorder, or enhance or improve the therapeutic effect(s) of another therapy.

As used herein, the terms "therapies" and "therapy" can refer to any protocol(s), method(s), and/or agent(s) that can be used in the prevention, treatment, management, and/or amelioration of a disorder or one or more symptoms thereof. In certain embodiments, the terms "therapy" and "therapy" refer to anti-viral therapy, anti-bacterial therapy, anti-fungal therapy, anti-cancer agent, biological therapy, supportive therapy, and/or other therapies useful in treatment, management, prevention, or amelioration of a disorder or one or more symptoms thereof known to one skilled in the art, for example, a medical professional such as a physician.

As used herein, the terms "treat," "treatment," and "treating" refer to the reduction or amelioration of the progression, severity, and/or duration of a disorder or amelioration of one or more symptoms thereof resulting from the administration of one or more therapies (including, but not limited to, the administration of one or more prophylactic or therapeutic agents).

### 3.2 BRIEF DESCRIPTION OF THE FIGURES

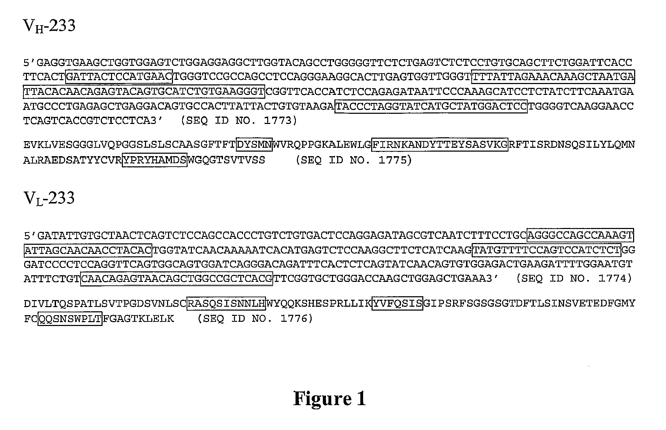

Figure 1. Nucleic acid and protein sequences of the heavy and light chains of the mouse anti-human EphA2 monoclonal antibody B233. CDR1, 2 and 3 regions as defined by Kabat are boxed. The full amino acid sequences of the variable heavy (V_{H}) and light (V_{L}) chains are given using the standard one letter code.

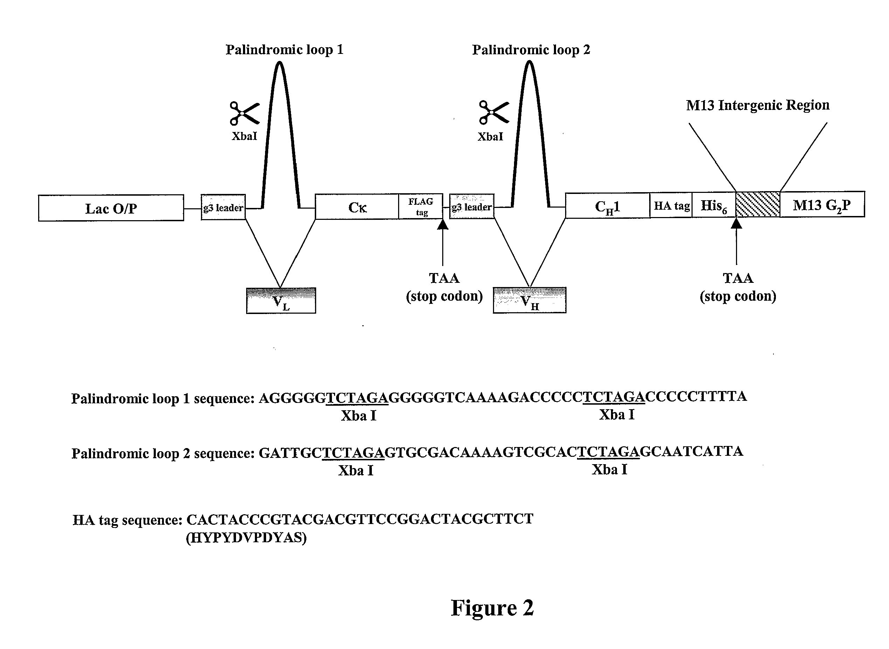

Figure 2. Phage vector used for screening of the framework shuffling libraries and expression of the corresponding Fab fragments. Streptavidin purified, single-stranded DNA of each of the V_{L} and V_{H} genes is annealed to the vector by hybridization mutagenesis using homology in the gene 3 leader/Cκ and gene 3 leader/Cγ1 regions, respectively. The unique Xba1 site in the palindromic loops allows elimination of the parental vector. V_{H} and V_{L} genes are then expressed in frame with the first constant domain of the human κ1 heavy chain and the constant domain of the human kappa (κ) light chain, respectively.

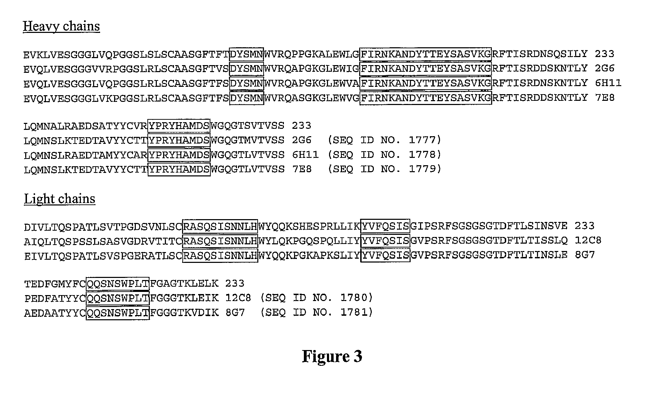

Figure 3. Protein sequences of framework-shuffled, humanized clones of the anti-human EphA2 monoclonal antibody B233 isolated after screening of libraries A and B. CDR1, 2 and 3 regions as defined by Kabat are boxed. The full amino acid sequences of the variable heavy (V_{H}) and light (V_{L}) chains are given using the standard one letter code.

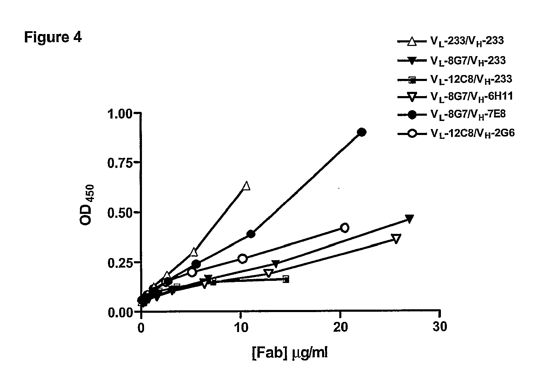

Figure 4. ELISA titration using Fab extracts on immobilized human EphA2-Fc.

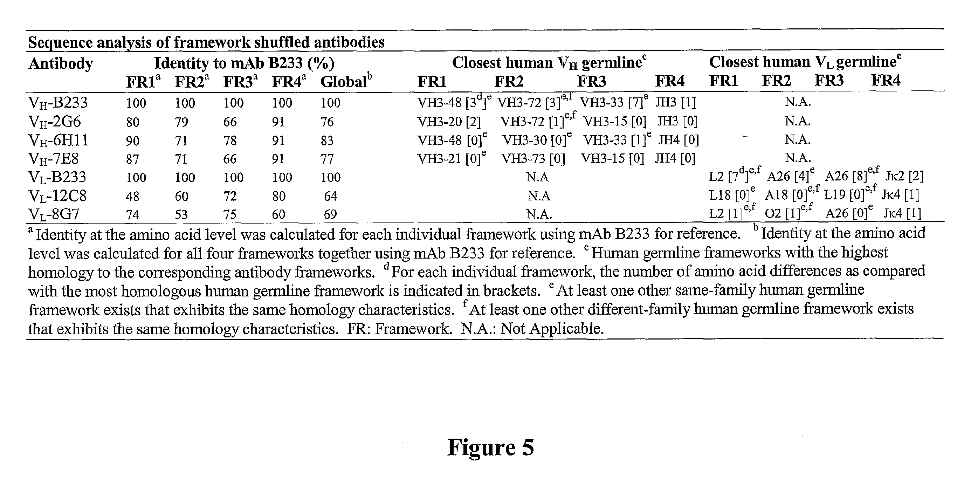

Figure 5. Sequence analysis of framework shuffled antibodies. ^{a}Percent identity at the amino acid level was calculated for each individual antibody framework using mAb B233 for reference

### DETAILED DESCRIPTION OF THE INVENTION

The present invention provides methods of re-engineering or re-shaping an antibody from a first species, wherein the re-engineered or re-shaped antibody does not elicit undesired immune response in a second species, and the re-engineered or re-shaped antibody retains substantially the same antigen binding-ability of the antibody from the first species. In accordance with the present invention, a combinatorial library comprising the CDRs of the antibody from the first species fused in frame with framework regions from a bank of framework regions derived from a second species can be constructed and screened for the desired modified antibody.

The present invention also provides cells comprising, containing or engineered to express the nucleic acid sequences described herein. The present invention provides a method of producing a heavy chain variable region (preferably, a humanized heavy chain variable region), said method comprising expressing the nucleotide sequence encoding a heavy chain variable region (preferably, a humanized heavy chain variable region) in a cell described herein. The present invention provides a method of producing an light chain variable region (preferably, a humanized light chain variable region), said method comprising expressing the nucleotide sequence encoding a light chain variable region (preferably, a humanized light chain variable region) in a cell described herein. The present invention also provides a method of producing an antibody (preferably, a humanized antibody) that immunospecifically binds to an antigen, said method comprising expressing the nucleic acid sequence(s) encoding the humanized antibody contained in the cell described herein.

The present invention provides antibodies produced by the methods described herein. In a preferred embodiment, the invention provides humanized antibodies produced by the methods described herein. The present invention also provides a composition comprising an antibody produced by the methods described herein and a carrier, diluent or excipient. In a preferred embodiment, the invention provides a composition comprising a humanized antibody produced by the methods described herein and a carrier, diluent or excipient. Preferably, the compositions of the invention are pharmaceutical compositions in a form for its intended use.

For clarity of disclosure, and not by way of limitation, the detailed description of the invention is divided into the following subsections:
(i) construction of a global bank of acceptor framework regions
(ii) selection of CDRs
(iii) construction of combinatorial sub-libraries
(iv) construction of combinatorial libraries
(v) expression of the combinatorial libraries
(vi) selection of humanized antibodies
(vii) production and characterization of humanized antibodies
(viii) antibody conjugates
(ix) uses of the compositions of the invention
(x) administration and formulations
(xi) dosage and frequency of administration
(xii) biological assays
(xiii) kits
(xiv) article of manufacture

### 2.2 Construction of a Global Bank of Acceptor Framework Regions

According to the present invention, a variable light chain region and/or variable heavy chain region of a donor antibody (*e.g*., a non-human antibody) can be modified (*e.g*., humanized) by fusing together nucleic acid sequences encoding framework regions (FR1, FR2, FR3, FR4 of the light chain, and FR1, FR2, FR3, FR4 of the heavy chain) of an acceptor antibody(ies) (*e.g*., a human antibody) and nucleic acid sequences encoding complementarity-determining regions (CDR1, CDR2, CDR3 of the light chain, and CDR1, CDR2, CDR3 of the heavy chain) of the donor antibody. Preferably, the modified (*e.g*., humanized) antibody light chain comprises FR1, CDR1, FR2, CDR2, FR3, CDR3, and FR4. A modified (*e.g*., humanized) antibody heavy chain comprises FR1, CDR1, FR2, CDR2, FR3, CDR3, and FR4. Each acceptor (*e.g*., human) framework region (FR1, 2, 3, 4 of light chain, and FR1, 2, 3, 4 of heavy chain) can be generated from FR sub-banks for the light chain and FR sub-banks for the heavy chain, respectively. A global bank of acceptor (*e.g*., human) framework regions comprises two or more FR sub-banks.

### 2.2.1 Generation of Sub-banks for the Light Chain Frameworks

Light chain sub-banks 1, 2, 3 and 4 are constructed, wherein sub-bank 1 comprises plurality of nucleic acid sequences comprising nucleotide sequences, each nucleotide sequence encoding a light chain FR1; sub-bank 2 comprises a plurality of nucleic acid sequences comprising nucleotide sequences, each nucleotide sequence encoding a light chain FR2; sub-bank 3 comprises a plurality of nucleic acid sequences comprising nucleotide sequences, each nucleotide sequence encoding a light chain FR3; and sub-bank 4 comprises a plurality of nucleic acid sequences comprising nucleotide sequences, each nucleotide sequence encoding a light chain FR4. In some embodiments, the FR sequences are obtained or derived from functional human antibody sequences (*e.g*., an antibody known in the art and/or commercially available). In some embodiments, the FR sequences are derived from human germline light chain sequences. In one embodiment, the sub-bank FR sequences are derived from a human germline kappa chain sequences. In another embodiment, the sub-bank FR sequences are derived from a human germline lambda chain sequences.

By way of example but not limitation, the following describes a method of generating 4 light chain FR sub-banks using Polymerase Chain Reaction (PCR), wherein human germline kappa chain sequences are used as templates. Light chain FR sub-banks 1, 2 and 3 (encoding FR1, 2, 3 respectively) encompass 46 human germline kappa chain sequences (A1, A10, A11, A14, A17, A18, A19, A2, A20, A23, A26, A27, A3, A30, A5, A7, B2, B3, L1, L10, L11, L12, L14, L15, L16, L18, L19, L2, L20, L22, L23, L24, L25, L4/18a, L5, L6, L8, L9, O1, O11, 012, 014, 018, 02, 04 and 08). See Kawasaki et al., 2001, Eur. J. Immunol., 31:1017-1028; Schable and Zachau, 1993, Biol. Chem. Hoppe Seyler 374:1001-1022; and Brensing-Kuppers et al., 1997, Gene 191:173-181. The sequences are summarized at the NCBI website:
www.ncbi.nlm.nih.gov/igblast/showGermline.cgi?organism=human&chainType=VK&seqT ype=nucleotide; each of which is incorporated herein by reference in its entirety. Light chain FR sub-bank 4 (encoding FR4) encompasses 5 human germline kappa chain sequences (Jκ1, Jκ2, Jκ3, Jκ4 and Jκ5). See Hieter et al., 1982, J. Biol. Chem. 257:1516-1522; which is incorporated herein by reference in its entirety. The sequences are summarized at the NCBI website:
www.ncbi.nlm.nih.gov/igblast/showGermline.cgi?organism=human&chainType=JK&seqT ype=nucleotide; which is incorporated herein by reference in its entirety.

By way of example but not limitation, the construction of light chain FR1 sub-bank is carried out using the Polymerase Chain Reaction by overlap extension using the oligonucleotides listed in Table 12 and Table 13 (all shown in the 5' to 3' orientation, name followed by sequence):

**Table 12. Light Chain FR1 Forward Primers (for Sub-Bank 1)**

| | | |
|---|---|---|
| 414 | FR1L1 | GATGTTGTGATGACTCAGTCTCCACTCTCCCTGCCCGTCACCC |
| 415 | FR1L2 | GATGTTGTGATGACTCAGTCTCCACTCTCCCTGCCCGTCACCC |
| 416 | FR1L3 | GATATTGTGATGACCCAGACTCCACTCTCTCTGTCCGTCACCC |
| 417 | FR1L4 | GATATTGTGATGACTCAGTCTCCACTCTCCCTGCCCGTCACCC |
| 418 | FR1L5 | GATATTGTGATGACCCAGACTCCACTCTCTCTGTCCGTCACCC |
| 419 | FR1L6 | GATATTGTGATGACCCAGACTCCACTCTCCTCACCTGTCACCC |
| 420 | FR1L7 | GATATTGTGATGACTCAGTCTCCACTCTCCCTGCCCGTCACCC |
| 421 | FR1L8 | GAGATTGTGATGACCCAGACTCCACTCTCCTTGTCTATCACCC |
| 422 | FR1L9 | GATATTGTGATGACCCAGACTCCACTCTCCTCGCCTGTCACCC |
| 423 | FR1L10 | GAAATTGTGCTGACTCAGTCTCCAGACTTTCAGTCTGTGACTC |
| 424 | FR1L11 | GATGTTGTGATGACACAGTCTCCAGCTTTCCTCTCTGTGACTC |
| 425 | FR1L12 | GACATCCAGATGACCCAGTCTCCATCCTCCCTGTCTGCATCTG |
| 426 | FR1L13 | GAAATTGTGCTGACTCAGTCTCCAGACTTTCAGTCTGTGACTC |
| 427 | FR1L14 | GACATCCAGATGACCCAGTCTCCATCCTCCCTGTCTGCATCTG |
| 428 | FR1L15 | GAAACGACACTCACGCAGTCTCCAGCATTCATGTCAGCGACTC |
| 429 | FR1L16 | GACATCCAGATGACCCAGTCTCCATCCTCACTGTCTGCATCTG |
| 430 | FR1L17 | GCCATCCAGATGACCCAGTCTCCATCCTCCCTGTCTGCATCTG |
| 431 | FR1L18 | GACATCCAGATGACCCAGTCTCCTTCCACCCTGTCTGCATCTG |
| 432 | FR1L19 | AACATCCAGATGACCCAGTCTCCATCTGCCATGTCTGCATCTG |
| 433 | FR1L20 | GACATCCAGATGACCCAGTCTCCATCCTCACTGTCTGCATCTG |
| 434 | FR1L21 | GAAATAGTGATGATGCAGTCTCCAGCCACCCTGTCTGTGTCTC |
| 435 | FR1L22 | GCCATCCAGTTGACCCAGTCTCCATCCTCCCTGTCTGCATCTG |
| 436 | FR1L23 | GACATCCAGATGACCCAGTCTCCATCTTCTGTGTCTGCATCTG |
| 437 | FR1L24 | GAAATAGTGATGACGCAGTCTCCAGCCACCCTGTCTGTGTCTC |
| 438 | FR1L25 | GAAATTGTGTTGACACAGTCTCCAGCCACCCTGTCTTTGTCTC |
| 439 | FR1L26 | GACATCCAGATGATCCAGTCTCCATCTTTCCTGTCTGCATCTG |
| 440 | FR1L27 | GCCATCCGGATGACCCAGTCTCCATTCTCCCTGTCTGCATCTG |
| 441 | FR1L28 | GTCATCTGGATGACCCAGTCTCCATCCTTACTCTCTGCATCTA |
| 442 | FR1L29 | GCCATCCAGTTGACCCAGTCTCCATCCTCCCTGTCTGCATCTG |
| 443 | FR1L30 | GACATCCAGATGACCCAGTCTCCATCTTCCGTGTCTGCATCTG |
| 444 | FR1L31 | GAAATTGTGTTGACACAGTCTCCAGCCACCCTGTCTTTGTCTC |
| 445 | FR1L32 | GACATCCAGTTGACCCAGTCTCCATCCTTCCTGTCTGCATCTG |
| 446 | FR1L33 | GCCATCCGGATGACCCAGTCTCCATCCTCATTCTCTGCATCTA |
| 447 | FR1L34 | GACATCCAGATGACCCAGTCTCCATCCTCCCTGTCTGCATCTG |
| 448 | FR1L35 | GACATCCAGTTGACCCAGTCTCCATCCTCCCTGTCTGCATCTG |
| 449 | FR1L36 | GACATCCAGATGACCCAGTCTCCATCCTCCCTGTCTGCATCTG |
| 450 | FR1L37 | GACATCCAGATGACCCAGTCTCCATCCTCCCTGTCTGCATCTG |
| 451 | FR1L38 | GACATCCAGTTGACCCAGTCTCCATCCTCCCTGTCTGCATCTG |
| 452 | FR1L39 | GACATCCAGATGACCCAGTCTCCATCCTCCCTGTCTGCATCTG |
| 453 | FR1L40 | GAAATTGTAATGACACAGTCTCCACCCACCCTGTCTTTGTCTC |
| 454 | FR1L41 | GAAATTGTAATGACACAGTCTCCAGCCACCCTGTCTTTGTCTC |
| 455 | FR1L42 | GAAATTGTGTTGACGCAGTCTCCAGCCACCCTGTCTTTGTCTC |
| 456 | FR1L43 | GAAATTGTGTTGACGCAGTCTCCAGGCACCCTGTCTTTGTCTC |
| 457 | FR1L44 | GACATCGTGATGACCCAGTCTCCAGACTCCCTGGCTGTGTCTC |
| 458 | FR1L45 | GATATTGTGATGACCCAGACTCCACTCTCCCTGCCCGTCACCC |
| 459 | 4FR1L46 | GATATTGTGATGACCCAGACTCCACTCTCCCTGCCCGTCACCC |

**Table 13. Light Chain FR1 Reverse Primers (for Sub-Bank 1)**

| | | |
|---|---|---|
| 460 | FR1L1' | GCAGGAGATGGAGGCCGGCTGTCCAAGGGTGACGGGCAGGGAGAGTG |
| 461 | FR1L2' | GCAGGAGATGGAGGCCGGCTGTCCAAGGGTGACGGGCAGGGAGAGTG |
| 462 | FR1L3' | GCAGGAGATGGAGGCCGGCTGTCCAGGGGTGACGGACAGAGAGAGTG |
| 463 | FR1L4' | GCAGGAGATGGAGGCCGGCTCTCCAGGGGTGACGGGCAGGGAGAGTG |
| 464 | FR1L5' | GCAGGAGATGGAGGCCGGCTGTCCAGGGGTGACGGACAGAGAGAGTG |
| 465 | FR1L6' | GCAGGAGATGGAGGCCGGCTGTCCAAGGGTGACAGGTGAGGAGAGTG |
| 466 | FR1L7' | GCAGGAGATGGAGGCCGGCTCTCCAGGGGTGACGGGCAGGGAGAGTG |
| 467 | FR1L8' | GCAGGAGATGGAGGCCTGCTCTCCAGGGGTGATAGACAAGGAGAGTG |
| 468 | FR1L9' | GAAGGAGATGGAGGCCGGCTGTCCAAGGGTGACAGGCGAGGAGAGTG |
| 469 | FR1L10' | GCAGGTGATGGTGACTTTCTCCTTTGGAGTCACAGACTGAAAGTCTG |
| 470 | FR1L11' | GCAGGTGATGGTGACTTTCTCCCCTGGAGTCACAGAGAGGAAAGCTG |
| 471 | FR1L12' | GCAAGTGATGGTGACTCTGTCTCCTACAGATGCAGACAGGGAGGATG |
| 472 | FR1L13' | GCAGGTGATGGTGACTTTCTCCTTTGGAGTCACAGACTGAAAGTCTG |
| 473 | FR1L14' | GCAAGTGATGGTGACTCTGTCTCCTACAGATGCAGACAGGGAGGATG |
| 474 | FR1L15' | GCAGGAGATGTTGACTTTGTCTCCTGGAGTCGCTGACATGAATGCTG |
| 475 | FR1L16' | ACAAGTGATGGTGACTCTGTCTCCTACAGATGCAGACAGTGAGGATG |
| 476 | FR1L17' | GCAAGTGATGGTGACTCTGTCTCCTACAGATGCAGACAGGGAGGATG |
| 477 | FR1L18' | GCAAGTGATGGTGACTCTGTCTCCTACAGATGCAGACAGGGTGGAAG |
| 478 | FR1L19' | ACAAGTGATGGTGACTCTGTCTCCTACAGATGCAGACATGGCAGATG |
| 479 | FR1L20' | ACAAGTGATGGTGACTCTGTCTCCTACAGATGCAGACAGTGAGGATG |
| 480 | FR1L21' | GCAGGAGAGGGTGGCTCTTTCCCCTGGAGACACAGACAGGGTGGCTG |
| 481 | FR1L22' | GCAAGTGATGGTGACTCTGTCTCCTACAGATGCAGACAGGGAGGATG |
| 482 | FR1L23' | ACAAGTGATGGTGACTCTGTCTCCTACAGATGCAGACACAGAAGATG |
| 483 | FR1L24' | GCAGGAGAGGGTGGCTCTTTCCCCTGGAGACACAGACAGGGTGGCTG |
| 484 | FR1L25' | GCAGGAGAGGGTGGCTCTTTCCCCTGGAGACAAAGACAGGGTGGCTG |
| 485 | FR1L26' | GCAAATGATACTGACTCTGTCTCCTACAGATGCAGACAGGAAAGATG |
| 486 | FR1L27' | GCAAGTGATGGTGACTCTGTCTCCTACAGATGCAGACAGGGAGAATG |
| 487 | FR1L28' | ACAACTGATGGTGACTCTGTCTCCTGTAGATGCAGAGAGTAAGGATG |
| 488 | FR1L29' | GCAAGTGATGGTGACTCTGTCTCCTACAGATGCAGACAGGGAGGATG |
| 489 | FR1L30' | ACAAGTGATGGTGACTCTGTCTCCTACAGATGCAGACACGGAAGATG |
| 490 | FR1L31' | GCAGGAGAGGGTGGCTCTTTCCCCTGGAGACAAAGACAGGGTGGCTG |
| 491 | FR1L32' | GCAAGTGATGGTGACTCTGTCTCCTACAGATGCAGACAGGAAGGATG |
| 492 | FR1L33' | ACAAGTGATGGTGACTCTGTCTCCTGTAGATGCAGAGAATGAGGATG |
| 493 | FR1L34' | GCAAGTGATGGTGACTCTGTCTCCTACAGATGCAGACAGGGAGGATG |
| 494 | FR1L35' | GCAAGTGATGGTGACTCTGTCTCCTACAGATGCAGACAGGGAGGATG |
| 495 | FR1L36' | GCAAGTGATGGTGACTCTGTCTCCTACAGATGCAGACAGGGAGGATG |
| 496 | FR1L37' | GCAAGTGATGGTGACTCTGTCTCCTACAGATGCAGACAGGGAGGATG |
| 497 | FR1L38' | GCAAGTGATGGTGACTCTGTCTCCTACAGATGCAGACAGGGAGGATG |
| 498 | FR1L39' | GCAAGTGATGGTGACTCTGTCTCCTACAGATGCAGACAGGGAGGATG |
| 499 | FR1L40' | GCAGGAGAGGGTGACTCTTTCGCCTGGAGACAAAGACAGGGTGGGTG |
| 500 | FR1L41' | GCAGGAGAGGGTGGCTCTTTCCCCTGGAGACAAAGACAGGGTGGCTG |
| 501 | FR1L42' | GCAGGAGAGGGTGGCTCTTTCCCCTGGAGACAAAGACAGGGTGGCTG |
| 502 | FR1L43' | GCAGGAGAGGGTGGCTCTTTCCCCTGGAGACAAAGACAGGGTGCCTG |
| 503 | FR1L44' | GCAGTTGATGGTGGCCCTCTCGCCCAGAGACACAGCCAGGGAGTCTG |
| 504 | FR1L45' | GCAGGAGATGGAGGCCGGCTCTCCAGGGGTGACGGGCAGGGAGAGTG |
| 505 | FR1L46' | GCAGGAGATGGAGGCCGGCTCTCCAGGGGTGACGGGCAGGGAGAGTG |

PCR is carried out using the following oligonucleotide combinations (46 in total): FR1L1/FR1L1', FR1L2/FR1L2', FR1L3/FR1L3', FR1L4/FR1L4', FR1L5/FR1L5', FR1L6/FR1L6', FR1L7/FR1L7', FR1L8/FR1L8', FR1L9/FR1L9', FR1L10/FR1L10', FR1L11/FR1L11', FR1L12/FR1L12', FR1L13/FR1L13', FR1L14/FR1L14', FR1L15/FR1L15', FR1L16/FR1L16', FR1L17/FR1L17', FR1L18/FR1L18', FR1L19/FR1L19', FR1L20/FR1L20', FR1L21/FR1L21', FR1L22/FR1L22', FR1L23/FR1L23', FR1L24/FR1L24', FR1L25/FR1L25', FR1L26/FR1L26', FR1L27/FR1L27', FR1L28/FR1L28', FR1L29/FR1L29', FR1L30/FR1L30', FR1L31/FR1L31', FR1L32/FR1L32', FR1L33/FR1L33', FR1L34/FR1L34', FR1L35/FR1L35', FR1L36/FR1L36', FR1L37/FR1L37', FR1L38/FR1L38', FR1L39/FR1L39', FR1L40/FR1L40', FR1L41/FR1L41', FR1L42/FR1L42', FR1L43/FR1L43', FR1L44/FR1L44', FR1L45/FR1L45', and FR1L46/FR1L46'. The pooling of the PCR products generates sub-bank 1.

By way of example but not limitation, the construction of light chain FR2 sub-bank is carried out using the Polymerase Chain Reaction by overlap extension using the oligonucleotides listed in Table 14 and Table 15 (all shown in the 5' to 3' orientation, name followed by sequence):

**Table 14. Light Chain FR2 Forward Primers (for Sub-Bank 2)**

| | | |
|---|---|---|
| 506 | FR2L1 | TGGTTTCAGCAGAGGCCAGGCCAATCTCCAA |
| 507 | FR2L2 | TGGTTTCAGCAGAGGCCAGGCCAATCTCCAA |
| 508 | FR2L3 | TGGTACCTGCAGAAGCCAGGCCAGTCTCCAC |
| 509 | FR2L4 | TGGTACCTGCAGAAGCCAGGGCAGTCTCCAC |
| 510 | FR2L5 | TGGTACCTGCAGAAGCCAGGCCAGCCTCCAC |
| 511 | FR2L6 | TGGCTTCAGCAGAGGCCAGGCCAGCCTCCAA |
| 512 | FR2L7 | TGGTACCTGCAGAAGCCAGGGCAGTCTCCAC |
| 513 | FR2L8 | TGGTTTCTGCAGAAAGCCAGGCCAGTCTCCA |
| 514 | FR2L9 | TGGCTTCAGCAGAGGCCAGGCCAGCCTCCAA |
| 515 | FR2L10 | TGGTACCAGCAGAAACCAGATCAGTCTCCAA |
| 516 | FR2L11 | TGGTACCAGCAGAAACCAGATCAAGCCCCAA |
| 517 | FR2L12 | TGGTATCAGCAGAAACCAGGGAAAGTTCCTA |
| 518 | FR2L13 | TGGTACCAGCAGAAACCAGATCAGTCTCCAA |
| 519 | FR2L14 | TGGTATCAGCAGAAACCAGGGAAAGCCCCTA |
| 520 | FR2L15 | TGGTACCAACAGAAACCAGGAGAAGCTGCTA |
| 521 | FR2L16 | TGGTTTCAGCAGAAACCAGGGAAAGCCCCTA |
| 522 | FR2L17 | TGGTATCAGCAGAAACCAGGGAAAGCCCCTA |
| 523 | FR2L18 | TGGTATCAGCAGAAACCAGGGAAAGCCCCTA |
| 524 | FR2L19 | TGGTTTCAGCAGAAACCAGGGAAAGTCCCTA |
| 525 | FR2L20 | TGGTATCAGCAGAAACCAGAGAAAGCCCCTA |
| 526 | FR2L21 | TGGTACCAGCAGAAACCTGGCCAGGCTCCCA |
| 527 | FR2L22 | TGGTATCAGCAGAAACCAGGGAAAGCTCCTA |
| 528 | FR2L23 | TGGTATCAGCAGAAACCAGGGAAAGCCCCTA |
| 529 | FR2L24 | TGGTACCAGCAGAAACCTGGCCAGGCTCCCA |
| 530 | FR2L25 | TGGTACCAGCAGAAACCTGGCCAGGCTCCCA |
| 531 | FR2L26 | TGGTATCTGCAGAAACCAGGGAAATCCCCTA |
| 532 | FR2L27 | TGGTATCAGCAAAAACCAGCAAAAGCCCCTA |
| 533 | FR2L28 | TGGTATCAGCAAAAACCAGGGAAAGCCCCTG |
| 534 | FR2L29 | TGGTATCAGCAGAAACCAGGGAAAGCTCCTA |
| 535 | FR2L30 | TGGTATCAGCAGAAACCAGGGAAAGCCCCTA |
| 536 | FR2L31 | TGGTACCAACAGAAACCTGGCCAGGCTCCCA |
| 537 | FR2L32 | TGGTATCAGCAAAAACCAGGGAAAGCCCCTA |
| 538 | FR2L33 | TGGTATCAGCAAAAACCAGGGAAAGCCCCTA |
| 539 | FR2L34 | TGGTATCAGCAGAAACCAGGGAAAGCCCCTA |
| 540 | FR2L35 | TGGTATCGGCAGAAACCAGGGAAAGTTCCTA |
| 541 | FR2L36 | TGGTATCAGCAGAAACCAGGGAAAGCCCCTA |
| 542 | FR2L37 | TGGTATCAGCAGAAACCAGGGAAAGCCCCTA |
| 543 | FR2L38 | TGGTATCGGCAGAAACCAGGGAAAGTTCCTA |
| 544 | FR2L39 | TGGTATCAGCAGAAACCAGGGAAAGCCCCTA |
| 545 | FR2L40 | TGGTATCAGCAGAAACCTGGCCAGGCGCCCA |
| 546 | FR2L41 | TGGTACCAGCAGAAACCTGGGCAGGCTCCCA |
| 547 | FR2L42 | TGGTACCAGCAGAAACCTGGCCTGGCGCCCA |
| 548 | FR2L43 | TGGTACCAGCAGAAACCTGGCCAGGCTCCCA |
| 549 | FR2L44 | TGGTACCAGCAGAAACCAGGACAGCCTCCTA |
| 550 | FR2L45 | TGGTACCTGCAGAAGCCAGGGCAGTCTCCAC |
| 551 | FR2L46 | TGGTACCTGCAGAAGCCAGGGCAGTCTCCAC |

**Table 15. Light Chain FR2 Reverse Primers (for Sub-Bank 2)**

| | | |
|---|---|---|
| 552 | FR2L1' | ATAAATTAGGCGCCTTGGAGATTGGCCTGGCCTCT |
| 553 | FR2L2' | ATAAATTAGGCGCCTTGGAGATTGGCCTGGCCTCT |
| 554 | FR2L3' | ATAGATCAGGAGCTGTGGAGACTGGCCTGGCTTCT |
| 555 | FR2L4' | ATAGATCAGGAGCTGTGGAGACTGCCCTGGCTTCT |
| 556 | FR2L5' | ATAGATCAGGAGCTGTGGAGGCTGGCCTGGCTTCT |
| 557 | FR2L6' | ATAAATTAGGAGTCTTGGAGGCTGGCCTGGCCTCT |
| 558 | FR2L7' | ATAGATCAGGAGCTGTGGAGACTGCCCTGGCTTCT |
| 559 | FR2L8' | ATAGATCAGGAGTGTGGAGACTGGCCTGGCTTTCT |
| 560 | FR2L9' | ATAAATTAGGAGTCTTGGAGGCTGGCCTGGCCTCT |
| 561 | FR2L10' | CTTGATGAGGAGCTTTGGAGACTGATCTGGTTTCT |
| 562 | FR2L11' | CTTGATGAGGAGCTTTGGGGCTTGATCTGGTTTCT |
| 563 | FR2L12' | ATAGATCAGGAGCTTAGGAACTTTCCCTGGTTTCT |
| 564 | FR2L13' | CTTGATGAGGAGCTTTGGAGACTGATCTGGTTTCT |
| 565 | FR2L14' | ATAGATCAGGCGCTTAGGGGCTTTCCCTGGTTTCT |
| 566 | FR2L15' | TTGAATAATGAAAATAGCAGCTTCTCCTGGTTTCT |
| 567 | FR2L16' | ATAGATCAGGGACTTAGGGGCTTTCCCTGGTTTCT |
| 568 | FR2L17' | ATAGATCAGGAGCTTAGGGGCTTTCCCTGGTTTCT |
| 569 | FR2L18' | ATAGATCAGGAGCTTAGGGGCTTTCCCTGGTTTCT |
| 570 | FR2L19' | ATAGATCAGGTGCTTAGGGACTTTCCCTGGTTTCT |
| 571 | FR2L20' | ATAGATCAGGGACTTAGGGGCTTTCTCTGGTTTCT |
| 572 | FR2L21' | ATAGATGAGGAGCCTGGGAGCCTGGCCAGGTTTCT |
| 573 | FR2L22' | ATAGATCAGGAGCTTAGGAGCTTTCCCTGGTTTCT |
| 574 | FR2L23' | ATAGATCAGGAGCTTAGGGGCTTTCCCTGGTTTCT |
| 575 | FR2L24' | ATAGATGAGGAGCCTGGGAGCCTGGCCAGGTTTCT |
| 576 | FR2L25' | ATAGATGAGGAGCCTGGGAGCCTGGCCAGGTTTCT |
| 577 | FR2L26' | ATAGAGGAAGAGCTTAGGGGATTTCCCTGGTTTCT |
| 578 | FR2L27' | ATAGATGAAGAGCTTAGGGGCTTTTGCTGGTTTTT |
| 579 | FR2L28' | ATAGATCAGGAGCTCAGGGGCTTTCCCTGGTTTTT |
| 580 | FR2L29' | ATAGATCAGGAGCTTAGGAGCTTTCCCTGGTTTCT |
| 581 | FR2L30' | ATAGATCAGGAGCTTAGGGGCTTTCCCTGGTTTCT |
| 582 | FR2L31' | ATAGATGAGGAGCCTGGGAGCCTGGCCAGGTTTCT |
| 583 | FR2L32' | ATAGATCAGGAGCTTAGGGGCTTTCCCTGGTTTTT |
| 584 | FR2L33' | ATAGATCAGGAGCTTAGGGGCTTTCCCTGGTTTIT |
| 585 | FR2L34' | ATAGATCAGGAGCTTAGGGGCTTTCCCTGGTTTCT |
| 586 | FR2L35' | ATAGATCAGGAGCTTAGGAACTTTCCCTGGTTTCT |
| 587 | FR2L36' | GTAGATCAGGAGCTTAGGGGCTTTCCCTGGTTTCT |
| 588 | FR2L37' | ATAGATCAGGAGCTTAGGGGCTTTCCCTGGTTTCT |
| 589 | FR2L38' | ATAGATCAGGAGCTTAGGAACTTTCCCTGGTTTCT |
| 590 | FR2L39' | GTAGATCAGGAGCTTAGGGGCTTTCCCTGGTTTCT |
| 591 | FR2L40' | ATAGATGAGGAGCCTGGGCGCCTGGCCAGGTTTCT |
| 592 | FR2L41' | ATAGATGAGGAGCCTGGGAGCCTGCCCAGGTTTCT |
| 593 | FR2L42' | ATAGATGAGGAGCCTGGGCGCCAGGCCAGGTTTCT |
| 594 | FR2L43' | ATAGATGAGGAGCCTGGGAGCCTGGCCAGGTTTCT |
| 595 | FR2L44' | GTAAATGAGCAGCTTAGGAGGCTGTCCTGGTTTCT |
| 596 | FR2L45' | ATAGATCAGGAGCTGTGGAGACTGCCCTGGCTTCT |
| 597 | FR2L46' | ATAGATCAGGAGCTGTGGAGACTGCCCTGGCTTCT |

PCR is carried out using the following oligonucleotide combinations (46 in total): FR2L1/FR2L1', FR2L2/FR2L2', FR2L3/FR2L3', FR2L4/FR2L4', FR2L5/FR2L5', FR2L6/FR2L6', FR2L7/FR2L7', FR2L8/FR2L8', FR2L9/FR2L9', FR2L10/FR2L10', FR2L11/FR2L11', FR2L12/FR2L12', FR2L13/FR2L13', FR2L14/FR2L14', FR2L15/FR2L15', FR2L16/FR2L16', FR2L17/FR2L17', FR2L18/FR2L18', FR2L19/FR2L19', FR2L20/FR2L20', FR2L21/FR2L21', FR2L22/FR2L22', FR2L23/FR2L23', FR2L24/FR2L24', FR2L25/FR2L25', FR2L26/FR2L26', FR2L27/FR2L27', FR2L28/FR2L28', FR2L29/FR2L29', FR2L30/FR2L30', FR2L31/FR2L31', FR2L32/FR2L32', FR2L33/FR2L33', FR2L34/FR2L34', FR2L35/FR2L35', FR2L36/FR2L36', FR2L37/FR2L37', FR2L38/FR2L38', FR2L39/FR2L39', FR2L40/FR2L40', FR2L41/FR2L41', FR2L42/FR2L42', FR2L43/FR2L43', FR2L44/FR2L44', FR2L45/FR2L45', and FR2L46/FR2L46'. The pooling of the PCR products generates sub-bank 2.

By way of example but not limitation, the construction of light chain FR3 sub-bank is carried out using the Polymerase Chain Reaction by overlap extension using the oligonucleotides listed in Table 16 and Table 17 (all shown in the 5' to 3' orientation, name followed by sequence):

**Table 16. Light Chain FR3 Forward Primers (for Sub-Bank 3)**

| | |
|---|---|
| 598 | FR3L1 GGGGTCCCAGACAGATTCAGCGGCAGTGGGTCAGGCACTGATTTCACACTGAAAATCAG |
| 599 | FR3L2 GGGGTCCCAGACAGATTCAGCGGCAGTGGGTCAGGCACTGATTTCACACTGAAAATCAG |
| 600 | FR3L3 GGAGTGCCAGATAGGTTCAGTGGCAGCGGGTCAGGGACAGATTTCACACTGAAAATCAG |
| 601 | FR3L4 GGGGTCCCTGACAGGTTCAGTGGCAGTGGATCAGGCACAGATTTTACACTGAAAATCAG |
| 602 | FR3L5 GGAGTGCCAGATAGGTTCAGTGGCAGCGGGTCAGGGACAGATTTCACACTGAAAATCAG |
| 603 | FR3L6 GGGGTCCCAGACAGATTCAGTGGCAGTGGGGCAGGGACAGATTTCACACTGAAAATCAG |
| 604 | FR3L7 GGGGTCCCTGACAGGTTCAGTGGCAGTGGATCAGGCACAGATTTTACACTGAAAATCAG |
| 605 | FR3L8 GGAGTGCCAGATAGGTTCAGTGGCAGCGGGTCAGGGACAGATTTCACACTGAAAATCAG |
| 606 | FR3L9 GGGGTCCCAGACAGATTCAGTGGCAGTGGGGCAGGGACAGATTTCACACTGAAAATCAG |
| 607 | FR3L10 GGGGTCCCCTCGAGGTTCAGTGGCAGTGGATCTGGGACAGATTTCACCCTCACCATCAA |
| 608 | FR3L11 GGGGTCCCCTCGAGGTTCAGTGGCAGTGGATCTGGGACAGATTTCACCTTTACCATCAG |
| 609 | FR3L12 GGGGTCCCATCTCGGTTCAGTGGCAGTGGATCTGGGACAGATTTCACTCTCACCATCAG |
| 610 | FR3L13 GGGGTCCCCTCGAGGTTCAGTGGCAGTGGATCTGGGACAGATTTCACCCTCACCATCAA |
| 611 | FR3L14 GGGGTCCCATCAAGGTTCAGCGGCAGTGGATCTGGGACAGAATTCACTCTCACAATCAG |
| 612 | FR3L15 GGAATCCCACCTCGATTCAGTGGCAGCGGGTATGGAACAGATTTTACCCTCACAATTAA |
| 613 | FR3L16 GGGGTCCCATCAAGGTTCAGCGGCAGTGGATCTGGGACAGATTTCACTCTCACCATCAG |
| 614 | FR3L17 GGGGTCCCATCAAGGTTCAGCGGCAGTGGATCTGGCACAGATTTCACTCTCACCATCAG |
| 615 | FR3L18 GGGGTCCCATCAAGGTTCAGCGGCAGTGGATCTGGGACAGAATTCACTCTCACCATCAG |
| 616 | FR3L19 GGGGTCCCATCAAGGTTCAGCGGCAGTGGATCTGGGACAGAATTCACTCTCACAATCAG |
| 617 | FR3L20 GGGGTCCCATCAAGGTTCAGCGGCAGTGGATCTGGGACAGATTTCACTCTCACCATCAG |
| 618 | FR3L21 GGCATCCCAGCCAGGTTCAGTGGCAGTGGGTCTGGGACAGAGTTCACTCTCACCATCAG |
| 619 | FR3L22 GGGGTCCCATCAAGGTTCAGCGGCAGTGGATCTGGGACAGATTTCACTCTCACCATCAG |
| 620 | FR3L23 GGGGTCCCATCAAGGTTCAGCGGCAGTGGATCTGGGACAGATTTCACTCTCACTATCAG |
| 621 | FR3L24 GGTATCCCAGCCAGGTTCAGTGGCAGTGGGTCTGGGACAGAGTTCACTCTCACCATCAG |
| 622 | FR3L25 GGCATCCCAGCCAGGTTCAGTGGCAGTGGGCCTGGGACAGACTTCACTCTCACCATCAG |
| 623 | FR3L26 GGGGTCTCATCGAGGTTCAGTGGCAGGGGATCTGGGACGGATTTCACTCTCACCATCAT |
| 624 | FR3L27 GGGGTCCCATCAAGGTTCAGCGGCAGTGGATCTGGGACGGATTACACTCTCACCATCAG |
| 625 | FR3L28 GGGGTCCCATCAAGGTTCAGTGGCAGTGGATCTGGGACAGATTTCACTCTCACCATCAG |
| 626 | FR3L29 GGGGTCCCATCAAGGTTCAGCGGCAGTGGATCTGGGACAGATTTCACTCTCACCATCAG |
| 627 | FR3L30 GGGGTCCCATCAAGGTTCAGCGGCAGTGGATCTGGGACAGATTTCACTCTCACCATCAG |
| 628 | FR3L31 GGCATCCCAGCCAGGTTCAGTGGCAGTGGGTCTGGGACAGACTTCACTCTCACCATCAG |
| 629 | FR3L32 GGGGTCCCATCAAGGTTCAGCGGCAGTGGATCTGGGACAGAATTCACTCTCACAATCAG |
| 630 | FR3L33 GGGGTCCCATCAAGGTTCAGCGGCAGTGGATCTGGGACAGATTTCACTCTCACCATCAG |
| 631 | FR3L34 GGGGTCCCATCAAGGTTCAGTGGCAGTGGATCTGGGACAGATTTCACTCTCACCATCAG |
| 632 | FR3L35 GGAGTCCCATCTCGGTTCAGTGGCAGTGGATCTGGGACAGATTTCACTCTCACTATCAG |
| 633 | FR3L36 GGGGTCCCATCAAGGTTCAGTGGAAGTGGATCTGGGACAGATTTTACTTTCACCATCAG |
| 634 | FR3L37 GGGGTCCCATCAAGGTTCAGTGGCAGTGGATCTGGGACAGATTTCACTCTCACCATCAG |
| 635 | FR3L38 GGAGTCCCATCTCGGTTCAGTGGCAGTGGATCTGGGACAGATTTCACTCTCACTATCAG |
| 636 | FR3L39 GGGGTCCCATCAAGGTTCAGTGGAAGTGGATCTGGGACAGATTTTACTTTCACCATCAG |
| 637 | FR3L40 AGCATCCCAGCCAGGTTCAGTGGCAGTGGGTCTGGGACAGACTTCACTCTCACCATCAG |
| 638 | FR3L41 GGCATCCCAGCCAGGTTCAGTGGCAGTGGGTCTGGGACAGACTTCACTCTCACCATCAG |
| 639 | FR3L42 GGCATCCCAGACAGGTTCAGTGGCAGTGGGTCTGGGACAGACTTCACTCTCACCATCAG |
| 640 | FR3L43 GGCATCCCAGACAGGTTCAGTGGCAGTGGGTCTGGGACAGACTTCACTCTCACCATCAG |
| 641 | FR3L44 GGGGTCCCTGACCGATTCAGTGGCAGCGGGTCTGGGACAGATTTCACTCTCACCATCAG |
| 642 | FR3L45 GGAGTCCCAGACAGGTTCAGTGGCAGTGGGTCAGGCACTGATTTCACACTGAAAATCAG |
| 643 | FR3L46 GGAGTCCCAGACAGGTTCAGTGGCAGTGGGTCAGGCACTGATTTCACACTGAAAATCAG |

**Table 17. Light Chain FR3 Reverse Primers (for Sub-Bank 3)**

| | | |
|---|---|---|
| 644 | FR3L1' | GCAGTAATAAACCCCAACATCCTCAGCCTCCACCCTGCTGATTTTCAGTGTGAAA |
| 645 | FR3L2' | GCAGTAATAAACCCCAACATCCTCAGCCTCCACCCTGCTGATTTTCAGTGTGAAA |
| 646 | FR3L3 | TCAGTAATAAACCCCAACATCCTCAGCCTCCACCCGGCTGATTTTCAGTGTGAAA |
| 647 | FR3L4' | GCAGTAATAAACCCCAACATCCTCAGCCTCCACTCTGCTGATTTTCAGTGTAAAA |
| 648 | FR3L5' | GCAGTAATAAACCCCAACATCCTCAGCCTCCACCCGGCTGATTTTCAGTGTGAAA |
| 649 | FR3L6' | GCAGTAATAAACCCCGACATCCTCAGCTTCCACCCTGCTGATTTTCAGTGTGAAA |
| 650 | FR3L7' | GCAGTAATAAACCCCAACATCCTCAGCCTCCACTCTGCTGATTTTCAGTGTAAAA |
| 651 | FR3L8' | GCAGTAATAAACTCCAAAATCCTCAGCCTCCACCCGGCTGATTTTCAGTGTGAAA |
| 652 | FR3L9' | GCAGTAATAAACCCCGACATCCTCAGCTTCCACCCTGCTGATTTTCAGTGTGAAA |
| 653 | FR3L10' | ACAGTAATACGTTGCAGCATCTTCAGCTTCCAGGCTATTGATGGTGAGGGTGAAA |
| 654 | FR3L11' | ACAGTAATATGTTGCAGCATCTTCAGCTTCCAGGCTACTGATGGTAAAGGTGAAA |
| 655 | FR3L12' | ACAGTAATAAGTTGCAACATCTTCAGGCTGCAGGCTGCTGATGGTGAGAGTGAAA |
| 656 | FR3L13' | ACAGTAATACGTTGCAGCATCTTCAGCTTCCAGGCTATTGATGGTGAGGGTGAAA |
| 657 | FR3L14' | ACAGTAATAAGTTGCAAAATCTTCAGGCTGCAGGCTGCTGATTGTGAGAGTGAAT |
| 658 | FR3L15' | ACAGAAGTAATATGCAGCATCCTCAGATTCTATGTTATTAATTGTGAGGGTAAAA |
| 659 | FR3L16' | GCAGTAATAAGTTGCAAAATCTTCAGGCTGCAGGCTGCTGATGGTGAGAGTGAAA |
| 660 | FR3L17' | ACAGTAATAAGTTGCAAAATCTTCAGGCTGCAGGCTGCTGATGGTGAGAGTGAAA |
| 661 | FR3L18' | GCAGTAATAAGTTGCAAAATCATCAGGCTGCAGGCTGCTGATGGTGAGAGTGAAT |
| 662 | FR3L19' | ACAGTAATAAGTTGCAAAATCTTCAGGCTGCAGGCTGCTGATTGTGAGAGTGAAT |
| 663 | FR3L20' | GCAGTAATAAGTTGCAAAATCTTCAGGCTGCAGGCTGCTGATGGTGAGAGTGAAA |
| 664 | FR3L21' | ACAGTAATAAACTGCAAAATCTTCAGACTGCAGGCTGCTGATGGTGAGAGTGAAC |
| 665 | FR3L22' | ACAGTAATAAGTTGCAAAATCTTCAGGCTGCAGGCTGCTGATGGTGAGAGTGAAA |
| 666 | FR3L23' | ACAATAGTAAGTTGCAAAATCTTCAGGCTGCAGGCTGCTGATAGTGAGAGTGAAA |
| 667 | FR3L24' | ACAGTAATAAACTGCAAAATCTTCAGACTGCAGGCTGCTGATGGTGAGAGTGAAC |
| 668 | FR3L25' | ACAGTAATAAACTGCAAAATCTTCAGGCTCTAGGCTGCTGATGGTGAGAGTGAAG |
| 669 | FR3L26' | ACAGTAATAAGCTGCAAAATCTTCAGGCTTCAGGCTGATGATGGTGAGAGTGAAA |
| 670 | FR3L27' | ACAGTAATAAGTTGCAAAATCTTCAGGCTGCAGGCTGCTGATGGTGAGAGTGTAA |
| 671 | FR3L28' | ACAGTAATAAGTTGCAAAATCTTCAGACTGCAGGCAACTGATGGTGAGAGTGAAA |
| 672 | FR3L29' | ACAGTAATAAGTTGCAAAATCTTCAGGCTGCAGGCTGCTGATGGTGAGAGTGAAA |
| 673 | FR3L30' | ACAATAGTAAGTTGCAAAATCTTCAGGCTGCAGGCTGCTGATGGTGAGAGTGAAA |
| 674 | FR3L31' | ACAGTAATAAACTGCAAAATCTTCAGGCTCTAGGCTGCTGATGGTGAGAGTGAAG |
| 675 | FR3L32' | ACAGTAATAAGTTGCAAAATCTTCAGGCTGCAGGCTGCTGATTGTGAGAGTGAAT |
| 676 | FR3L33' | ACAGTAATAAGTTGCAAAATCTTCAGACTGCAGGCAGCTGATGGTGAGAGTGAAA |
| 677 | FR3L34' | ACAGTAGTAAGTTGCAAAATCTTCAGGTTGCAGACTGCTGATGGTGAGAGTGAAA |
| 678 | FR3L35' | ACCGTAATAAGTTGCAACATCTTCAGGCTGCAGGCTGCTGATAGTGAGAGTGAAA |
| 679 | FR3L36' | ACAGTAATATGTTGCAATATCTTCAGGCTGCAGGCTGCTGATGGTGAAAGTAAAA |
| 680 | FR3L37' | ACAGTAGTAAGTTGCAAAATCTTCAGGTTGCAGACTGCTGATGGTGAGAGTGAAA |
| 681 | FR3L38' | ACCGTAATAAGTTGCAACATCTTCAGGCTGCAGGCTGCTGATAGTGAGAGTGAAA |
| 682 | FR3L39' | ACAGTAATATGTTGCAATATCTTCAGGCTGCAGGCTGCTGATGGTGAAAGTAAAA |
| 683 | FR3L40' | ACAGTAATAAACTGCAAAATCTTCAGGCTGCAGGCTGCTGATGGTGAGAGTGAAG |
| 684 | FR3L41' | ACAGTAATAAACTGCAAAATCTTCAGGCTGCAGGCTGCTGATGGTGAGAGTGAAG |
| 685 | FR3L42' | ACAGTAATACACTGCAAAATCTTCAGGCTCCAGTCTGCTGATGGTGAGAGTGAAG |
| 686 | FR3L43' | ACAGTAATACACTGCAAAATCTTCAGGCTCCAGTCTGCTGATGGTGAGAGTGAAG |
| 687 | FR3L44' | ACAGTAATAAACTGCCACATCTTCAGCCTGCAGGCTGCTGATGGTGAGAGTGAAA |
| 688 | FR3L45' | GCAGTAATAAACTCCAACATCCTCAGCCTCCACCCTGCTGATTTTCAGTGTGAAA |
| 689 | FR3L46' | GCAGTAATAAACTCCAACATCCTCAGCCTCCACCCTGCTGATTTTCAGTGTGAAA |

PCR is carried out using the following oligonucleotide combinations (46 in total): FR3L1/FR3LF, FR3L2/FR3L2', FR3L3/FR3L3', FR3L4/FR3L4', FR3L5/FR3L5', FR3L6/FR3L6', FR3L7/FR3L7', FR3L8/FR3L8', FR3L9/FR3L9', FR3L10/FR3L10', FR3L11/FR3L11', FR3L12/FR3L12', FR3L13/FR3L13', FR3L14/FR3L14', FR3L15/FR3L15', FR3L16/FR3L16', FR3L17/FR3L17', FR3L18/FR3L18', FR3L19/FR3L19', FR3L20/FR3L20', FR3L21/FR3L21', FR3L22/FR3L22', FR3L23/FR3L23', FR3L24/FR3L24', FR3L25/FR3L25', FR3L26/FR3L26', FR3L27/FR3L27', FR3L28/FR3L28', FR3L29/FR3L29', FR3L30/FR3L30', FR3L31/FR3L31', FR3L32/FR3L32', FR3L33/FR3L33', FR3L34/FR3L34', FR3L35/FR3L35', FR3L36/FR3L36', FR3L37/FR3L37', FR3L38/FR3L38', FR3L39/FR3L39', FR3L40/FR3L40', FR3L41/FR3L41', FR3L42/FR3L42', FR3L43/FR3L43', FR3L44/FR3L44', FR3L45/FR3L45', and FR3L46/FR3L46'. The pooling of the PCR products generates sub-bank 3.

By way of example but not limitation, the construction of light chain FR4 sub-bank is carried out using the Polymerase Chain Reaction by overlap extension using the oligonucleotides listed in Table 18 and Table 19 (all shown in the 5' to 3' orientation, name followed by sequence):

**Table 18. Light Chain FR4 Forward Primers (for Sub-Bank 4)**

| | | |
|---|---|---|
| 690 | FR4L1 | TTCGGCCAAGGGACCAAGGTGGAAATCAAA |
| 691 | FR4L2 | TTTGGCCAGGGGACCAAGCTGGAGATCAAA |
| 692 | FR4L3 | TTCGGCCCTGGGACCAAAGTGGATATCAAA |
| 693 | FR4L4 | TTCGGCGGAGGGACCAAGGTGGAGATCAAA |
| 694 | FR4L5 | TTCGGCCAAGGGACACGACTGGAGATTAAA |

**Table 19. Light Chain FR4 Reverse Primers (for Sub-Bank 4)**

| | | |
|---|---|---|
| 695 | FR4L1' | TTTGATTTCCACCTTGGTCCCTTGGCCGAA |
| 696 | FR4L2' | TTTGATCTCCAGCTTGGTCCCCTGGCCAAA |
| 697 | FR4L3' | TTTGATATCCACTTTGGTCCCAGGGCCGAA |
| 698 | FR4L4' | TTTGATCTCCACCTTGGTCCCTCCGCCGAA |
| 699 | FR4L5' | TTTAATCTCCAGTCGTGTCCCTTGGCCGAA |

PCR is carried out using the following oligonucleotide combinations (5 in total): FR4L1/FR4L1', FR4L2/FR4L2', FR4L3/FR4L3', FR4L4/FR4L4', or FR4L5/FR4L5'. The pooling of the PCR products generates sub-bank 4.

### 2.2.2 Generation of Sub-banks for the Heavy Chain Frameworks

In some embodiments, heavy chain FR sub-banks 5, 6, 7 and 11 are constructed wherein sub-bank 5 comprises a plurality of nucleic acid sequences comprising nucleotide sequences, each nucleotide sequence encoding a heavy chain FR1; sub-bank 6 comprises a plurality of nucleic acid sequences comprising nucleotide sequences, each nucleotide sequence encoding a heavy chain FR2; sub-bank 7 comprises a plurality of nucleic acid sequences comprising nucleotide sequences, each nucleotide sequence encoding a heavy chain FR3; and sub-bank 11 comprises a plurality of nucleic acid sequences comprising nucleotide sequences, each nucleotide sequence encoding a heavy chain FR4, respectively; wherein the heavy chain FR1, FR2, and FR3 are defined according to Kabat definition for CDR H1 and H2. In some embodiments, the FR sequences are derived form functional human antibody sequences. In other embodiments, the FR sequences are derived from human germline heavy chain sequences.

By way of example but not limitation, the following describes a method of generating 4 heavy chain FR sub-banks using Polymerase Chain Reaction (PCR), wherein human germline heavy chain sequences are used as templates. Heavy chain FR sub-banks 5, 6 and 7 (encoding FR1, 2, 3 respectively) encompass 44 human germline heavy chain sequences (VH1-18, VH1-2, VH1-24, VH1-3, VH1-45, VH1-46, VH1-58, VH-69, VH1-8, VH2-26, VH2-5, VH2-70, VH3-11, VH3-13, VH3-15, VH3-16, VH3-20, VH3-21, VH3-23, VH3-30, VH3-33, VH3-35, VH3-38, VH3-43, VH3-48, VH3-49, VH3-53, VH3-64, VH3-66, VH3-7, VH3-72, VH3-73, VH3-74, VH3-9, VH4-28, VH4-31, VH4-34, VH4-39, VH4-4, VH4-59, VH4-61, VH5-51, VH6-1 and VH7-81). See Matsuda et al., 1998, J. Exp. Med., 188:1973-1975. The sequences are summarized at the NCBI website: www.ncbi.nlm.nih.gov/igblast/showGermline.cgi?organism=human&chainType=VH&seqT ype=nucleotide. Heavy chain FR sub-bank 11 (encoding FR4) encompasses 6 human germline heavy chain sequences (JH1, JH2, JH3, JH4, JH5 and JH6). See Ravetch et al., 1981, Cell 27(3 Pt 2):583-591. The sequences are summarized at the NCBI website: www.ncbi.nlm.nih.gov/igblast/showGermline.cgi?organism=human&chainType=JH&seqT ype=nucleotide.

By way of example but not limitation, the construction of heavy chain FR1 sub-bank (according to Kabat definition) is carried out using the Polymerase Chain Reaction by overlap extension using the oligonucleotides listed in Table 20 and Table 21 (all shown in the 5' to 3' orientation, name followed by sequence):

**Table 20. Heavy Chain FR1 (Kabat Definition) Forward Primers (for Sub-Bank 5):**

| | |
|---|---|
| 700 | FR1HK1 CAGGTTCAGCTGGTGCAGTCTGGAGCTGAGGTGAAGAAGCCTGGGGCCTCAGTGAAGGT |
| 701 | FR1HK2 CAGGTGCAGCTGGTGCAGTCTGGGGCTGAGGTGAAGAAGCCTGGGGCCTCAGTGAAGGT |
| 702 | FR1HK3 CAGGTCCAGCTGGTACAGTCTGGGGCTGAGGTGAAGAAGCCTGGGGCCTCAGTGAAGGT |
| 703 | FR1HK4 CAGGTTCAGCTGGTGCAGTCTGGGGCTGAGGTGAAGAAGCCTGGGGCCTCAGTGAAGGT |
| 704 | FR1HK5 CAGATGCAGCTGGTGCAGTCTGGGGCTGAGGTGAAGAAGACTGGGTCCTCAGTGAAGGT |
| 705 | FR1HK6 CAGGTGCAGCTGGTGCAGTCTGGGGCTGAGGTGAAGAAGCCTGGGGCCTCAGTGAAGGT |
| 706 | FR1HK7 CAAATGCAGCTGGTGCAGTCTGGGCCTGAGGTGAAGAAGCCTGGGACCTCAGTGAAGGT |
| 707 | FR1HK8 CAGGTGCAGCTGGTGCAGTCTGGGGCTGAGGTGAAGAAGCCTGGGTCCTCGGTGAAGGT |
| 708 | FR1HK9 CAGGTGCAGCTGGTGCAGTCTGGGGCTGAGGTGAAGAAGCCTGGGGCCTCAGTGAAGGT |
| 709 | FR1HK10 CAGGTCACCTTGAAGGAGTCTGGTCCTGTGCTGGTGAAACCCACAGAGACCCTCACGCT |
| 710 | FR1HK11 CAGATCACCTTGAAGGAGTCTGGTCCTACGCTGGTGAAACCCACACAGACCCTCACGCT |
| 711 | FR1HK12 CAGGTCACCTTGAGGGAGTCTGGTCCTGCGCTGGTGAAACCCACACAGACCCTCACACT |
| 712 | FR1HK13 CAGGTGCAGCTGGTGGAGTCTGGGGGAGGCTTGGTCAAGCCTGGAGGGTCCCTGAGACT |
| 713 | FR1HK14 GAGGTGCAGCTGGTGGAGTCTGGGGGAGGCTTGGTACAGCCTGGGGGGTCCCTGAGACT |
| 714 | FR1HK15 GAGGTGCAGCTGGTGGAGTCTGGGGGAGGCTTGGTAAAGCCTGGGGGGTCCCTTAGACT |
| 715 | FR1HK16 GAGGTGCAGCTGGTGGAGTCTGGGGGAGGCTTGGTACAGCCTGGGGGGTCCCTGAGACT |
| 716 | FR1HK17 GAGGTGCAGCTGGTGGAGTCTGGGGGAGGTGTGGTACGGCCTGGGGGGTCCCTGAGACT |
| 717 | FR1HK18 GAGGTGCAGCTGGTGGAGTCTGGGGGAGGCCTGGTCAAGCCTGGGGGGTCCCTGAGACT |
| 718 | FR1HK19 GAGGTGCAGCTGTTGGAGTCTGGGGGAGGCTTGGTACAGCCTGGGGGGTCCCTGAGACT |
| 719 | FR1HK20 CAGGTGCAGCTGGTGGAGTCTGGGGGAGGCGTGGTCCAGCCTGGGAGGTCCCTGAGACT |
| 720 | FR1HK21 CAGGTGCAGCTGGTGGAGTCTGGGGGAGGCGTGGTCCAGCCTGGGAGGTCCCTGAGACT |
| 721 | FR1HK22 GAGGTGCAGCTGGTGGAGTCTGGGGGAGGCTTGGTACAGCCTGGGGGATCCCTGAGACT |
| 722 | FR1HK23 GAGGTGCAGCTGGTGGAGTCTGGGGGAGGCTTGGTACAGCCTAGGGGGTCCCTGAGACT |
| 723 | FR1HK24 GAAGTGCAGCTGGTGGAGTCTGGGGGAGTCGTGGTACAGCCTGGGGGGTCCCTGAGACT |
| 724 | FR1HK25 GAGGTGCAGCTGGTGGAGTCTGGGGGAGGCTTGGTACAGCCTGGGGGGTCCCTGAGACT |
| 725 | FR1HK26 GAGGTGCAGCTGGTGGAGTCTGGGGGAGGCTTGGTACAGCCAGGGCGGTCCCTGAGACT |
| 726 | FR1HK27 GAGGTGCAGCTGGTGGAGTCTGGAGGAGGCTTGATCCAGCCTGGGGGGTCCCTGAGACT |
| 727 | FR1HK28 GAGGTGCAGCTGGTGGAGTCTGGGGAAGGCTTGGTCCAGCCTGGGGGGTCCCTGAGACT |
| 728 | FR1HK29 GAGGTGCAGCTGGTGGAGTCTGGAGGAGGCTTGATCCAGCCTGGGGGGTCCCTGAGACT |
| 729 | FR1HK30 GAGGTGCAGCTGGTGGAGTCTGGGGGAGGCTTGGTCCAGCCTGGGGGGTCCCTGAGACT |
| 730 | FR1HK31 GAGGTGCAGCTGGTGGAGTCTGGGGGAGGCTTGGTCCAGCCTGGAGGGTCCCTGAGACT |
| 731 | FR1HK32 GAGGTGCAGCTGGTGGAGTCCGGGGGAGGCTTGGTCCAGCCTGGGGGGTCCCTGAAACT |
| 732 | FR1HK33 GAGGTGCAGCTGGTGGAGTCCGGGGGAGGCTTAGTTCAGCCTGGGGGGTCCCTGAGACT |
| 733 | FR1HK34 GAAGTGCAGCTGGTGGAGTCTGGGGGAGGCTTGGTACAGCCTGGCAGGTCCCTGAGACT |
| 734 | FR1HK35 CAGGTGCAGCTGCAGGAGTCGGGCCCAGGACTGGTGAAGCCTTCGGACACCCTGTCCCT |
| 735 | FR1HK36 CAGGTGCAGCTGCAGGAGTCGGGCCCAGGACTGGTGAAGCCTTCACAGACCCTGTCCCT |
| 736 | FR1HK37 CAGGTGCAGCTACAGCAGTGGGGCGCAGGACTGTTGAAGCCTTCGGAGACCCTGTCCCT |
| 737 | FR1HK38 CAGCTGCAGCTGCAGGAGTCGGGCCCAGGACTGGTGAAGCCTTCGGAGACCCTGTCCCT |
| 738 | FR1HK39 CAGGTGCAGCTGCAGGAGTCGGGCCCAGGACTGGTGAAGCCTTCGGAGACCCTGTCCCT |
| 739 | FR1HK40 CAGGTGCAGCTGCAGGAGTCGGGCCCAGGACTGGTGAAGCCTTCGGAGACCCTGTCCCT |
| 740 | FR1HK41 CAGGTGCAGCTGCAGGAGTCGGGCCCAGGACTGGTGAAGCCTTCGGAGACCCTGTCCCT |
| 741 | FR1HK42 GAGGTGCAGCTGGTGCAGTCTGGAGCAGAGGTGAAAAAGCCCGGGGAGTCTCTGAAGAT |
| 742 | FR1HK43 CAGGTACAGCTGCAGCAGTCAGGTCCAGGACTGGTGAAGCCCTCGCAGACCCTCTCACT |
| 743 | FR1HK44 CAGGTGCAGCTGGTGCAGTCTGGCCATGAGGTGAAGCAGCCTGGGGCCTCAGTGAAGGT |

**Table 21. Heavy Chain FR1 (Kabat Definition) Reverse Primers (for Sub-Bank 5):**

| | | |
|---|---|---|
| 744 | FR1HK1' | GGTAAAGGTGTAACCAGAAGCCTTGCAGGAGACCTTCACTGAGGCCCCAGGC |
| 745 | FR1HK2' | GGTGAAGGTGTATCCAGAAGCCTTGCAGGAGACCTTCACTGAGGCCCCAGGC |
| 746 | FR1HK3' | AGTGAGGGTGTATCCGGAAACCTTGCAGGAGACCTTCACTGAGGCCCCAGGC |
| 747 | FR1HK4' | AGTGAAGGTGTATCCAGAAGCCTTGCAGGAAACCTTCACTGAGGCCCCAGGC |
| 748 | FR1HK5' | GGTGAAGGTGTATCCGGAAGCCTTGCAGGAAACCTTCACTGAGGACCCAGTC |
| 749 | FR1HK6' | GGTGAAGGTGTATCCAGATGCCTTGCAGGAAACCTTCACTGAGGCCCCAGGC |
| 750 | FR1HK7' | AGTAAAGGTGAATCCAGAAGCCTTGCAGGAGACCTTCACTGAGGTCCCAGGC |
| 751 | FR1HK8' | GCTGAAGGTGCCTCCAGAAGCCTTGCAGGAGACCTTCACCGAGGACCCAGGC |
| 752 | FR1HK9' | GGTGAAGGTGTATCCAGAAGCCTTGCAGGAGACCTTCACTGAGGCCCCAGGC |
| 753 | FR1HK10' | GCTGAGTGAGAACCCAGAGACGGTGCAGGTCAGCGTGAGGGTCTCTGTGGGT |
| 754 | FR1HK11' | GCTGAGTGAGAACCCAGAGAAGGTGCAGGTCAGCGTGAGGGTCTGTGTGGGT |
| 755 | FR1HK12' | GCTGAGTGAGAACCCAGAGAAGGTGCAGGTCAGTGTGAGGGTCTGTGTGGGT |
| 756 | FR1HK13' | ACTGAAGGTGAATCCAGAGGCTGCACAGGAGAGTCTCAGGGACCCTCCAGGC |
| 757 | FR1HK14' | ACTGAAGGTGAATCCAGAGGCTGCACAGGAGAGTCTCAGGGACCCCCCAGGC |
| 758 | FR1HK15' | ACTGAAAGTGAATCCAGAGGCTGCACAGGAGAGTCTAAGGGACCCCCCAGGC |
| 759 | FR1HK16' | ACTGAAGGTGAATCCAGAGGCTGCACAGGAGAGTCTCAGGGACCCCCCAGGC |
| 760 | FR1HK17' | ATCAAAGGTGAATCCAGAGGCTGCACAGGAGAGTCTCAGGGACCCCCCAGGC |
| 761 | FR1HK18' | ACTGAAGGTGAATCCAGAGGCTGCACAGGAGAGTCTCAGGGACCCCCCAGGC |
| 762 | FR1HK19' | GCTAAAGGTGAATCCAGAGGCTGCACAGGAGAGTCTCAGGGACCCCCCAGGC |
| 763 | FR1HK20' | ACTGAAGGTGAATCCAGAGGCTGCACAGGAGAGTCTCAGGGACCTCCCAGGC |
| 764 | FR1HK21' | ACTGAAGGTGAATCCAGACGCTGCACAGGAGAGTCTCAGGGACCTCCCAGGC |
| 765 | FR1HK22' | ACTGAAGGTGAATCCAGAGGCTGCACAGGAGAGTCTCAGGGATCCCCCAGGC |
| 766 | FR1HK23' | ACTGACGGTGAATCCAGAGGCTGCACAGGAGAGTCTCAGGGACCCCCTAGGC |
| 767 | FR1HK24' | ATCAAAGGTGAATCCAGAGGCTGCACAGGAGAGTCTCAGGGACCCCCCAGGC |
| 768 | FR1HK25' | ACTGAAGGTGAATCCAGAGGCTGCACAGGAGAGTCTCAGGGACCCCCCAGGC |
| 769 | FR1HK26' | ACCAAAGGTGAATCCAGAAGCTGTACAGGAGAGTCTCAGGGACCGCCCTGGC |
| 770 | FR1HK27' | ACTGACGGTGAACCCAGAGGCTGCACAGGAGAGTCTCAGGGACCCCCCAGGC |
| 771 | FR1HK28' | ACTGAAGGTGAATCCAGAGGCTGCACAGGAGAGTCTCAGGGACCCCCCAGGC |
| 772 | FR1HK29' | ACTGACGGTGAACCCAGAGGCTGCACAGGAGAGTCTCAGGGACCCCCCAGGC |
| 773 | FR1HK30' | ACTAAAGGTGAATCCAGAGGCTGCACAGGAGAGTCTCAGGGACCCCCCAGGC |
| 774 | FR1HK31' | ACTGAAGGTGAATCCAGAGGCTGCACAGGAGAGTCTCAGGGACCCTCCAGGC |
| 775 | FR1HK32' | ACTGAAGGTGAACCCAGAGGCTGCACAGGAGAGTTTCAGGGACCCCCCAGGC |
| 776 | FR1HK33' | ACTGAAGGTGAATCCAGAGGCTGCACAGGAGAGTCTCAGGGACCCCCCAGGC |
| 777 | FR1HK34' | ATCAAAGGTGAATCCAGAGGCTGCACAGGAGAGTCTCAGGGACCTGCCAGGC |
| 778 | FR1HK35' | GCTGATGGAGTAACCAGAGACAGCGCAGGTGAGGGACAGGGTGTCCGAAGGC |
| 779 | FR1HK36' | GCTGATGGAGCCACCAGAGACAGTACAGGTGAGGGACAGGGTCTGTGAAGGC |
| 780 | FR1HK37' | ACTGAAGGACCCACCATAGACAGCGCAGGTGAGGGACAGGGTCTCCGAAGGC |
| 781 | FR1HK38' | GCTGATGGAGCCACCAGAGACAGTGCAGGTGAGGGACAGGGTCTCCGAAGGC |
| 782 | FR1HK39' | ACTGATGGAGCCACCAGAGACAGTGCAGGTGAGGGACAGGGTCTCCGAAGGC |
| 783 | FR1HK40' | ACTGATGGAGCCACCAGAGACAGTGCAGGTGAGGGACAGGGTCTCCGAAGGC |
| 784 | FR1HK41' | GCTGACGGAGCCACCAGAGACAGTGCAGGTGAGGGACAGGGTCTCCGAAGGC |
| 785 | FR1HK42' | GGTAAAGCTGTATCCAGAACCCTTACAGGAGATCTTCAGAGACTCCCCGGGC |
| 786 | FR1HK43' | AGAGACACTGTCCCCGGAGATGGCACAGGTGAGTGAGAGGGTCTGCGAGGGC |
| 787 | FR1HK44' | GGTGAAACTGTAACCAGAAGCCTTGCAGGAGACCTTCACTGAGGCCCCAGGC |

PCR is carried out using the following oligonucleotide combinations (44 in total): FR1HK1/FR1HK1', FR1HK2/FR1HK2', FR1HK3/FR1HK3', FR1HK4/FR1HK4', FR1HK5/FR1HK5', FR1HK6/FR1HK6', FR1HK7/FR1HK7', FR1HK8/FR1HK8', FR1HK9/FR1HK9', FR1HK10/FR1HK10', FR1HK11/FR1HK11', FR1HK12/FR1HK12', FR1HK13/FR1HK13', FR1HK14/FR1HK14', FR1HK15/FR1HK15', FR1HK16/FR1HK16', FR1HK17/FR1HK17', FR1HK18/FR1HK18', FR1HK19/FR1HK19', FR1HK20/FR1HK20', FR1HK21/FR1HK21', FR1HK22/FR1HK22', FR1HK23/FR1HK23', FR1HK24/FR1HK24', FR1HK25/FR1HK25', FR1HK26/FR1HK26', FR1HK27/FR1HK27', FR1HK28/FR1HK28', FR1HK29/FR1HK29', FR1HK30/FR1HK31', FR1HK32/FR1HK32', FR1HK33/FR1HK33', FR1HK34/FR1HK34', FR1HK35/FR1HK35', FR1HK36/FR1HK36', FR1HK37/FR1HK37', FR1HK38/FR1HK38', FR1HK39/FR1HK39', FR1HK40/FR1HK40', FR1HK41/FR1HK41', FR1HK42/FR1HK42', FR1HK43/FR1HK43', or FR1HK44/FR1HK44'. The pooling of the PCR products generates sub-bank 5.

By way of example but not limitation, the construction of heavy chain FR2 sub-bank (according to Kabat definition) is carried out using the Polymerase Chain Reaction by overlap extension using the oligonucleotides listed in Table 22 and Table 23 (all shown in the 5' to 3' orientation, name followed by sequence):

**Table 22. Heavy Chain FR2 (Kabat Definition) Forward Primers (for Sub-Bank 6):**

| | | |
|---|---|---|
| 788 | FR2HK1 | TGGGTGCGACAGGCCCCTGGACAAGGGCTTG |
| 789 | FR2HK2 | TGGGTGCGACAGGCCCCTGGACAAGGGCTTG |
| 790 | FR2HK3 | TGGGTGCGACAGGCTCCTGGAAAAGGGCTTG |
| 791 | FR2HK4 | TGGGTGCGCCAGGCCCCCGGACAAAGGCTTG |
| 792 | FR2HK5 | TGGGTGCGACAGGCCCCCGGACAAGCGCTTG |
| 793 | FR2HK6 | TGGGTGCGACAGGCCCCTGGACAAGGGCTTG |
| 794 | FR2HK7 | TGGGTGCGACAGGCTCGTGGACAACGCCTTG |
| 795 | FR2HK8 | TGGGTGCGACAGGCCCCTGGACAAGGGCTTG |
| 796 | FR2HK9 | TGGGTGCGACAGGCCACTGGACAAGGGCTTG |
| 797 | FR2HK10 | TGGATCCGTCAGCCCCCAGGGAAGGCCCTGG |
| 798 | FR2HK11 | TGGATCCGTCAGCCCCCAGGAAAGGCCCTGG |
| 799 | FR2HK12 | TGGATCCGTCAGCCCCCAGGGAAGGCCCTGG |
| 800 | FR2HK13 | TGGATCCGCCAGGCTCCAGGGAAGGGGCTGG |
| 801 | FR2HK14 | TGGGTCCGCCAAGCTACAGGAAAAGGTCTGG |
| 802 | FR2HK15 | TGGGTCCGCCAGGCTCCAGGGAAGGGGCTGG |
| 803 | FR2HK16 | TGGGCCCGCAAGGCTCCAGGAAAGGGGCTGG |
| 804 | FR2HK17 | TGGGTCCGCCAAGCTCCAGGGAAGGGGCTGG |
| 805 | FR2HK18 | TGGGTCCGCCAGGCTCCAGGGAAGGGGCTGG |
| 806 | FR2HK19 | TGGGTCCGCCAGGCTCCAGGGAAGGGGCTGG |
| 807 | FR2HK20 | TGGGTCCGCCAGGCTCCAGGCAAGGGGCTGG |
| 808 | FR2HK21 | TGGGTCCGCCAGGCTCCAGGCAAGGGGCTGG |
| 809 | FR2HK22 | TGGGTCCATCAGGCTCCAGGAAAGGGGCTGG |
| 810 | FR2HK23 | TGGATCCGCCAGGCTCCAGGGAAGGGGCTGG |
| 811 | FR2HK24 | TGGGTCCGTCAAGCTCCGGGGAAGGGTCTGG |
| 812 | FR2HK25 | TGGGTCCGCCAGGCTCCAGGGAAGGGGCTGG |
| 813 | FR2HK26 | TGGTTCCGCCAGGCTCCAGGGAAGGGGCTGG |
| 814 | FR2HK27 | TGGGTCCGCCAGGCTCCAGGGAAGGGGCTGG |
| 815 | FR2HK28 | TGGGTCCGCCAGGCTCCAGGGAAGGGACTGG |
| 816 | FR2HK29 | TGGGTCCGCCAGGCTCCAGGGAAGGGGCTGG |
| 817 | FR2HK30 | TGGGTCCGCCAGGCTCCAGGGAAGGGGCTGG |
| 818 | FR2HK31 | TGGGTCCGCCAGGCTCCAGGGAAGGGGCTGG |
| 819 | FR2HK32 | TGGGTCCGCCAGGCTTCCGGGAAAGGGCTGG |
| 820 | FR2HK33 | TGGGTCCGCCAAGCTCCAGGGAAGGGGCTGG |
| 821 | FR2HK34 | TGGGTCCGGCAAGCTCCAGGGAAGGGCCTGG |
| 522 | FR2HK35 | TGGATCCGGCAGCCCCCAGGGAAGGGACTGG |
| 823 | FR2HK36 | TGGATCCGCCAGCACCCAGGGAAGGGCCTGG |
| 824 | FR2HK37 | TGGATCCGCCAGCCCCCAGGGAAGGGGCTGG |
| 825 | FR2HK38 | TGGATCCGCCAGCCCCCAGGGAAGGGGCTGG |
| 826 | FR2HK39 | TGGATCCGGCAGCCCGCCGGGAAGGGACTGG |
| 827 | FR2HK40 | TGGATCCGGCAGCCCCCAGGGAAGGGACTGG |
| 828 | FR2HK41 | TGGATCCGGCAGCCCCCAGGGAAGGGACTGG |
| 829 | FR2HK42 | TGGGTGCGCCAGATGCCCGGGAAAGGCCTGG |
| 830 | FR2HK43 | TGGATCAGGCAGTCCCCATCGAGAGGCCTTG |
| 831 | FR2HK44 | TGGGTGCCACAGGCCCCTGGACAAGGGCTTG |

**Table 23. Heavy Chain FR2 (Kabat Definition) Reverse Primers (for Sub-Bank 6):**

| | | |
|---|---|---|
| 832 | FR2HK1' | TCCCATCCACTCAAGCCCTTGTCCAGGGGCCT |
| 833 | FR2HK2' | TCCCATCCACTCAAGCCCTTGTCCAGGGGCCT |
| 834 | FR2HK3' | TCCCATCCACTCAAGCCCTTTTCCAGGAGCCT |
| 835 | FR2HK4' | TCCCATCCACTCAAGCCTTTGTCCGGGGGCCT |
| 836 | FR2HK5' | TCCCATCCACTCAAGCGCTTGTCCGGGGGCCT |
| 837 | FR2HK6' | TCCCATCCACTCAAGCCCTTGTCCAGGGGCCT |
| 838 | FR2HK7' | TCCTATCCACTCAAGGCGTTGTCCACGAGCCT |
| 839 | FR2HK8' | TCCCATCCACTCAAGCCCTTGTCCAGGGGCCT |
| 840 | FR2HK9' | TCCCATCCACTCAAGCCCTTGTCCAGTGGCCT |
| 841 | FR2HK10' | TGCAAGCCACTCCAGGGCCTTCCCTGGGGGCT |
| 842 | FR2HK11' | TGCAAGCCACTCCAGGGCCTTTCCTGGGGGCT |
| 843 | FR2HK12' | TGCAAGCCACTCCAGGGCCTTCCCTGGGGGCT |
| 844 | FR2HK13' | TGAAACCCACTCCAGCCCCTTCCCTGGAGCCT |
| 845 | FR2HK14' | TGAGACCCACTCCAGACCTTTTCCTGTAGCTT |
| 846 | FR2HK15' | GCCAACCCACTCCAGCCCCTTCCCTGGAGCCT |
| 847 | FR2HK16' | CGATACCCACTCCAGCCCCTTTCCTGGAGCCT |
| 848 | FR2HK17' | AGAGACCCACTCCAGCCCCTTCCCTGGAGCTT |
| 849 | FR2HK18' | TGAGACCCACTCCAGCCCCTTCCCTGGAGCCT |
| 850 | FR2HK19' | TGAGACCCACTCCAGCCCCTTCCCTGGAGCCT |
| 851 | FR2HK20' | TGCCACCCACTCCAGCCCCTTGCCTGGAGCCT |
| 852 | FR2HK21' | TGCCACCCACTCCAGCCCCTTGCCTGGAGCCT |
| 853 | FR2HK22' | CGATACCCACTCCAGCCCCTTTCCTGGAGCCT |
| 854 | FR2HK23' | TGAGACCCACTCCAGCCCCTTCCCTGGAGCCT |
| 855 | FR2HK24' | AGAGACCCACTCCAGACCCTTCCCCGGAGCTT |
| 856 | FR2HK25' | TGAAACCCACTCCAGCCCCTTCCCTGGAGCCT |
| 857 | FR2HK26' | ACCTACCCACTCCAGCCCCTTCCCTGGAGCCT |
| 858 | FR2HK27' | TGAGACCCACTCCAGCCCCTTCCCTGGAGCCT |
| 859 | FR2HK28' | TGAAACATATTCCAGTCCCTTCCCTGGAGCCT |
| 860 | FR2HK29' | TGAGACCCACTCCAGCCCCTTCCCTGGAGCCT |
| 861 | FR2HK30 | GGCCACCCACTCCAGCCCCTTCCCTGGAGCCT |
| 862 | FR2HK31' | GCCAACCCACTCCAGCCCCTTCCCTGGAGCCT |
| 863 | FR2HK32' | GCCAACCCACTCCAGCCCTTTCCCGGAAGCCT |
| 864 | FR2HK33' | TGAGACCCACACCAGCCCCTTCCCTGGAGCTT |
| 865 | FR2HK34' | TGAGACCCACTCCAGGCCCTTCCCTGGAGCTT |
| 866 | FR2HK35' | CCCAATCCACTCCAGTCCCTTCCCTGGGGGCT |
| 867 | FR2HK36' | CCCAATCCACTCCAGGCCCTTCCCTGGGTGCT |
| 868 | FR2HK37' | CCCAATCCACTCCAGCCCCTTCCCTGGGGGCT |
| 869 | FR2HK38' | CCCAATCCACTCCAGCCCCTTCCCTGGGGGCT |
| 870 | FR2HK39' | CCCAATCCACTCCAGTCCCTTCCCGGCGGGCT |
| 871 | FR2HK40' | CCCAATCCACTCCAGTCCCTTCCCTGGGGGCT |
| 872 | FR2HK41' | CCCAATCCACTCCAGTCCCTTCCCTGGGGGCT |
| 873 | FR2HK42' | CCCCATCCACTCCAGGCCTTTCCCGGGCATCT |
| 874 | FR2HK43' | TCCCAGCCACTCAAGGCCTCTCGATGGGGACT |
| 875 | FR2HK44' | TCCCATCCACTCAAGCCCTTGTCCAGGGGCCT |

PCR is carried out using the following oligonucleotide combinations (44 in total): FR2HK1/FR2HK1', FR2HK2/FR2HK2', FR2HK3/FR2HK3', FR2HK4/FR2HK4', FR2HK5/FR2HK5', FR2HK6/FR2HK6', FR2HK7/FR2HK7', FR2HK8/FR2HK8', FR2HK9/FR2HK9', FR2HK10/FR2HK10', FR2HK11/FR2HK11', FR2HK12/FR2HK12', FR2HK13/FR2HK13', FR2HK14/FR2HK14', FR2HK15/FR2HK15', FR2HK16/FR2HK16', FR2HK17/FR2HK17', FR2HK18/FR2HK18', FR2HK19/FR2HK19', FR2HK20/FR2HK20', FR2HK21/FR2HK21', FR2HK22/FR2HK22', FR2HK23/FR2HK23', FR2HK24/FR2HK24', FR2HK25/FR2HK25', FR2HK26/FR2HK26', FR2HK27/FR2HK27', FR2HK28/FR2HK28', FR2HK29/FR2HK29', FR2HK30/FR2HK31', FR2HK32/FR2HK32', FR2HK33/FR2HK33', FR2HK34/FR2HK34', FR2HK35/FR2HK35', FR2HK36/FR2HK36', FR2HK37/FR2HK37', FR2HK38/FR2HK38', FR2HK39/FR2HK39', FR2HK40/FR2HK40', FR2HK41/FR2HK41', FR2HK42/FR2HK42', FR2HK43/FR2HK43', or FR2HK44/FR2HK44'. The pooling of the PCR products generates sub-bank 6.

By way of example but not limitation, the construction of heavy chain FR3 sub-bank (according to Kabat definition) is carried out using the Polymerase Chain Reaction by overlap extension using the oligonucleotides listed in Table 24 and Table 25 (all shown in the 5' to 3' orientation, name followed by sequence):

**Table 24. Heavy Chain FR3 (Kabat Definition) Forward Primers (for Sub-Bank 7):**

| | | |
|---|---|---|
| 876 | FR3HK1 | AGAGTCACCATGACCACAGACACATCCACGAGCACAGCCTACATGGAGCTGAGGAGCCTGAGATCTG |
| 877 | FR3HK2 | AGGGTCACCATGACCAGGGACACGTCCATCAGCACAGCCTACATGGAGCTGAGCAGGCTGAGATCTG |
| 878 | FR3HK3 | AGAGTCACCATGACCGAGGACACATCTACAGACACAGCCTACATGGAGCTGAGCAGCCTGAGATCTG |
| 879 | FR3HK4 | AGAGTCACCATTACCAGGGACACATCCGCGAGCACAGCCTACATGGAGCTGAGCAGCCTGAGATCTG |
| 880 | FR3HK5 | AGAGTCACCATTACCAGGGACAGGTCTATGAGCACAGCCTACATGGAGCTGAGCAGCCTGAGATCTG |
| 881 | FR3HK6 | AGAGTCACCATGACCAGGGACACGTCCACGAGCACAGTCTACATGGAGCTGAGCAGCCTGAGATCTG |
| 882 | FR3HK7 | AGAGTCACCATTACCAGGGACATGTCCACAAGCACAGCCTACATGGAGCTGAGCAGCCTGAGATCCG |
| 883 | FR3HK8 | AGAGTCACGATTACCGCGGACAAATCCACGAGCACAGCCTACATGGAGCTGAGCAGCCTGAGATCTG |
| 884 | FR3HK9 | AGAGTCACCATGACCAGGAACACCTCCATAAGCACAGCCTACATGGAGCTGAGCAGCCTGAGATCTG |
| 885 | FR3HK10 | AGGCTCACCATCTCCAAGGACACCTCCAAAAGCCAGGTGGTCCTTACCATGACCAACATGGACCCTG |
| 886 | FR3HK11 | AGGCTCACCATCACCAAGGACACCTCCAAAAACCAGGTGGTCCTTACAATGACCAACATGGACCCTG |
| 887 | FR3HK12 | AGGCTCACCATCTCCAAGGACACCTCCAAAAACCAGGTGGTCCTTACAATGACCAACATGGACCCTG |
| 888 | FR3HK13 | CGATTCACCATCTCCAGGGACAACGCCAAGAACTCACTGTATCTGCAAATGAACAGCCTGAGAGCCG |
| 889 | FR3HK14 | CGATTCACCATCTCCAGAGAAAATGCCAAGAACTCCTTGTATCTTCAAATGAACAGCCTGAGAGCCG |
| 890 | FR3HK15 | AGATTCACCATCTCAAGAGATGATTCAAAAAACACGCTGTATCTGCAAATGAACAGCCTGAAAACCG |
| 891 | FR3HK16 | CGATTCATCATCTCCAGAGACAATTCCAGGAACTCCCTGTATCTGCAAAAGAACAGACGGAGAGCCG |
| 892 | FR3HK17 | CGATTCACCATCTCCAGAGACAACGCCAAGAACTCCCTGTATCTGCAAATGAACAGTCTGAGAGCCG |
| 893 | FR3HK18 | CGATTCACCATCTCCAGAGACAACGCCAAGAACTCACTGTATCTGCAAATGAACAGCCTGAGAGCCG |
| 894 | FR3HK19 | CGGTTCACCATCTCCAGAGACAATTCCAAGAACACGCTGTATCTGCAAATGAACAGCCTGAGAGCCG |
| 895 | FR3HK20 | CGATTCACCATCTCCAGAGACAATTCCAAGAACACGCTGTATCTGCAAATGAACAGCCTGAGAGCTG |
| 896 | FR3HK21 | CGATTCACCATCTCCAGAGACAATTCCAAGAACACGCTGTATCTGCAAATGAACAGCCTGAGAGCCG |
| 897 | FR3HK22 | CGATTCATCATCTCCAGAGACAATTCCAGGAACACCCTGTATCTGCAAACGAATAGCCTGAGGGCCG |
| 898 | FR3HK23 | AGATTCACCATCTCCAGAGACAATTCCAAGAACACGCTGTATCTTCAAATGAACAACCTGAGAGCTG |
| 899 | FR3HK24 | CGATTCACCATCTCCAGAGACAACAGCAAAAACTCCCTGTATCTGCAAATGAACAGTCTGAGAACTG |
| 900 | FR3HK25 | CGATTCACCATCTCCAGAGACAATGCCAAGAACTCACTGTATCTGCAAATGAACAGCCTGAGAGACG |
| 901 | FR3HK26 | AGATTCACCATCTCAAGAGATGATTCCAAAAGCATCGCCTATCTGCAAATGAACAGCCTGAAAACCG |
| 902 | FR3HK27 | CGATTCACCATCTCCAGAGACAATTCCAAGAACACGCTGTATCTTCAAATGAACAGCCTGAGAGCCG |
| 903 | FR3HK28 | AGATTCACCATCTCCAGAGACAATTCCAAGAACACGCTGTATCTTCAAATGGGCAGCCTGAGAGCTG |
| 904 | FR3HK29 | CGATTCACCATCTCCAGAGACAATTCCAAGAACACGCTGTATCTTCAAATGAACAGCCTGAGAGCTG |
| 905 | FR3HK30 | CGATTCACCATCTCCAGAGACAACGCCAAGAACTCACTGTATCTGCAAATGAACAGCCTGAGAGCCG |
| 906 | FR3HK31 | AGATTCACCATCTCAAGAGATGATTCAAAGAACTCACTGTATCTGCAAATGAACAGCCTGAAAACCG |
| 907 | FR3HK32 | AGGTTCACCATCTCCAGAGATGATTCAAAGAACACGGCGTATCTGCAAATGAACAGCCTGAAAACCG |
| 908 | FR3HK33 | CGATTCACCATCTCCAGAGACAACGCCAAGAACACGCTGTATCTGCAAATGAACAGTCTGAGAGCCG |
| 909 | FR3HK34 | CGATTCACCATCTCCAGAGACAACGCCAAGAACTCCCTGTATCTGCAAATGAACAGTCTGAGAGCTG |
| 910 | FR3HK35 | CGAGTCACCATGTCAGTAGACACGTCCAAGAACCAGTTCTCCCTGAAGCTGAGCTCTGTGACCGCCG |
| 911 | FR3HK36 | CGAGTTACCATATCAGTAGACACGTCTAAGAACCAGTTCTCCCTGAAGCTGAGCTCTGTGACTGCCG |
| 912 | FR3HK37 | CGAGTCACCATATCAGTAGACACGTCCAAGAACCAGTTCTCCCTGAAGCTGAGCTCTGTGACCGCCG |
| 913 | FR3HK38 | CGAGTCACCATATCCGTAGACACGTCCAAGAACCAGTTCTCCCTGAAGCTGAGCTCTGTGACCGCCG |
| 914 | FR3HK39 | CGAGTCACCATGTCAGTAGACACGTCCAAGAACCAGTTCTCCCTGAAGCTGAGCTCTGTGACCGCCG |
| 915 | FR3HK40 | CGAGTCACCATATCAGTAGACACGTCCAAGAACCAGTTCTCCCTGAAGCTGAGCTCTGTGACCGCTG |
| 916 | FR3HK41 | CGAGTCACCATATCAGTAGACACGTCCAAGAACCAGTTCTCCCTGAAGCTGAGCTCTGTGACCGCTG |
| 917 | FR3HK42 | CAGGTCACCATCTCAGCCGACAAGTCCATCAGCACCGCCTACCTGCAGTGGAGCAGCCTGAAGGCCT |
| 918 | FR3HK43 | CGAATAACCATCAACCCAGACACATCCAAGAACCAGTTCTCCCTGCAGCTGAACTCTGTGACTCCCG |
| 919 | FR3HK44 | CGGTTTGTCTTCTCCATGGACACCTCTGCCAGCACAGCATACCTGCAGATCAGCAGCCTAAAGGCTG |

**Table 25. Heavy Chain FR3 (Kabat Definition) Reverse Primers (for Sub-Bank 7):**

| | | |
|---|---|---|
| 920 | FR3HK1' | TCTCGCACAGTAATACACGGCCGTGTCGTCAGATCTCAGGCTCCTCAGCT |
| 921 | FR3HK2' | TCTCGCACAGTAATACACGGCCGTGTCGTCAGATCTCAGCCTGCTCAGCT |
| 922 | FR3HK3' | TGTTGCACAGTAATACACGGCCGTGTCCTCAGATCTCAGGCTGCTCAGCT |
| 923 | FR3HK4' | TCTCGCACAGTAATACACAGCCATGTCCTCAGATCTCAGGCTGCTCAGCT |
| 924 | FR3HK5' | TCTTGCACAGTAATACATGGCTGTGTCCTCAGATCTCAGGCTGCTCAGCT |
| 925 | FR3HK6' | TCTCGCACAGTAATACACGGCCGTGTCCTCAGATCTCAGGCTGCTCAGCT |
| 926 | FR3HK7' | TGCCGCACAGTAATACACGGCCGTGTCCTCGGATCTCAGGCTGCTCAGCT |
| 927 | FR3HK8' | TCTCGCACAGTAATACACGGCCGTGTCCTCAGATCTCAGGCTGCTCAGCT |
| 928 | FR3HK9' | TCTCGCACAGTAATACACGGCCGTGTCCTCAGATCTCAGGCTGCTCAGCT |
| 929 | FR3HK10' | CCGTGCACAGTAATATGTGGCTGTGTCCACAGGGTCCATGTTGGTCATGG |
| 930 | FR3HK11' | GTGTGCACAGTAATATGTGGCTGTGTCCACAGGGTCCATGTTGGTCATTG |
| 931 | FR3HK12' | CCGTGCACAATAATACGTGGCTGTGTCCACAGGGTCCATGTTGGTCATTG |
| 932 | FR3HK13' | TCTCGCACAGTAATACACGGCCGTGTCCTCGGCTCTCAGGCTGTTCATTT |
| 933 | FR3HK14' | TCTTGCACAGTAATACACAGCCGTGTCCCCGGCTCTCAGGCTGTTCATTT |
| 934 | FR3HK15' | TGTGGTACAGTAATACACGGCTGTGTCCTCGGTTTTCAGGCTGTTCATTT |
| 935 | FR3HK16' | TCTCACACAGTAATACACAGCCATGTCCTCGGCTCTCCGTCTGTTCTTIT |
| 936 | FR3HK17' | TCTCGCACAGTGATACAAGGCCGTGTCCTCGGCTCTCAGACTGTTCATTT |
| 937 | FR3HK18' | TCTCGCACAGTAATACACAGCCGTGTCCTCGGCTCTCAGGCTGTTCATTT |
| 938 | FR3HK19' | TTTCGCACAGTAATATACGGCCGTGTCCTCGGCTCTCAGGCTGTTCATTT |
| 939 | FR3HK20' | TCTCGCACAGTAATACACAGCCGTGTCCTCAGCTCTCAGGCTGTTCATTT |
| 940 | FR3HK21' | CTCGCACAGTAATACAGAGCGGTGTCCTCGGCTCTCAGGCTGTTCATTT |
| 941 | FR3HK22' | TCTCACACAGTAATACACAGCCGTGTCCTCGGCCCTCAGGCTATTCGTTT |
| 942 | FR3HK23' | TCTGGCACAGTAATACACGGCCGTGCCCTCAGCTCTCAGGTTGTTCATTT |
| 943 | FR3HK24' | TTTTGCACAGTAATACAAGGCGGTGTCCTCAGTTCTCAGACTGTTCATTT |
| 944 | FR3HK25' | TCTCGCACAGTAATACACAGCCGTGTCCTCGTCTCTCAGGCTGTTCATTT |
| 945 | FR3HK26' | TCTAGTACAGTAATACACGGCTGTGTCCTCGGTTTTCAGGCTGTTCATTT |
| 946 | FR3HK27' | TCTCGCACAGTAATACACGGCCGTGTCCTCGGCTCTCAGGCTGTTCATTT |
| 947 | FR3HK28' | TCTCGCACAGTAATACACAGCCATGTCCTCAGCTCTCAGGCTGCCCATTT |
| 948 | FR3HK29' | TCTCGCACAGTAATACACAGCCGTGTCCTCAGCTCTCAGGCTGTTCATTT |
| 949 | FR3HK30' | TCTCGCACAGTAATACACAGCCGTGTCCTCGGCTCTCAGGCTGTTCATTT |
| 950 | FR3HK31' | TCTAGCACAGTAATACACGGCCGTGTCCTCGGTTTTCAGGCTGTTCATTT |
| 951 | FR3HK32' | TCTAGTACAGTAATACACGGCCGTGTCCTCGGTTTTCAGGCTGTTCATTT |
| 952 | FR3HK33' | TCTTGCACAGTAATACACAGCCGTGTCCTCGGCTGTCAGACTGTTGATTT |
| 953 | FR3HK34' | TTTTGCACAGTAATACAAGGCCGTGTCCTCAGCTCTCAGACTGTTCATTT |
| 954 | FR3HK35' | TCTCGCACAGTAATACACGGCCGTGTCCACGGCGGTCACAGAGCTCAGCT |
| 955 | FR3HK36' | TCTCGCACAGTAATACACGGCCGTGTCCGCGGCAGTCACAGAGCTCAGCT |
| 956 | FR3HK37' | TCTCGCACAGTAATACACAGCCGTGTCCGCGGCGGTCACAGAGCTCAGCT |
| 957 | FR3HK38' | TCTCGCACAGTAATACACAGCCGTGTCTGCGGCGGTCACAGAGCTCAGCT |
| 958 | FR3HK39' | TCTCGCACAGTAATACACGGCCGTGTCCGCGGCGGTCACAGAGCTCAGCT |
| 959 | FR3HK40' | TCTCGCACAGTAATACACGGCCGTGTCCGCAGCGGTCACAGAGCTCAGCT |
| 960 | FR3HK41' | TCTCGCACAGTAATACACGGCCGTGTCCGCAGCGGTCACAGAGCTCAGCT |
| 961 | FR3HK42' | TCTCGCACAGTAATACATGGCGGTGTCCGAGGCCTTCAGGCTGCTCCACT |
| 962 | FR3HK43' | TCTTGCACAGTAATACACAGCCGTGTCCTCGGGAGTCACAGAGTTCAGCT |
| 963 | FR3HK44' | TCTCGCACAGTAATACATGGCCATGTCCTCAGCCTTTAGGCTGCTGATCT |

PCR is carried out using the following oligonucleotide combinations (44 in total): FR3HK1/FR3HK1', FR3HK2/FR3HK2', FR3HK3/FR3HK3', FR3HK4/FR3HK4', FR3HK5/FR3HK5', FR3HK6/FR3HK6', FR3HK7/FR3HK7', FR3HK8/FR3HK8', FR3HK9/FR3HK9', FR3HK10/FR3HK10', FR3HK11/FR3HK11', FR3HK12/FR3HK12', FR3HK13/FR3HK13', FR3HK14/FR3HK14', FR3HK15/FR3HK15', FR3HK16/FR3HK16', FR3HK17/FR3HK17', FR3HK18/FR3HK18', FR3HK19/FR3HK19', FR3HK20/FR3HK20', FR3HK21/FR3HK21', FR3HK22/FR3HK22', FR3HK23/FR3HK23', FR3HK24/FR3HK24', FR3HK25/FR3HK25', FR3HK26/FR3HK26', FR3HK27/FR3HK27', FR3HK28/FR3HK28', FR3HK29/FR3HK29', FR3HK30/FR3HK31', FR3HK32/FR3HK32', FR3HK33/FR3HK33', FR3HK34/FR3HK34', FR3HK35/FR3HK35', FR3HK36/FR3HK36', FR3HK37/FR3HK37', FR3HK38/FR3HK38', FR3HK39/FR3HK39', FR3HK40/FR3HK40', FR3HK41/FR3HK41', FR3HK42/FR3HK42', FR3HK43/FR3HK43', or FR3HK44/FR3HK44'. The pooling of the PCR products generates sub-bank 7.

By way of example but not limitation, the construction of heavy chain FR4 sub-bank is carried out using the Polymerase Chain Reaction by overlap extension using the oligonucleotides listed in Table 26 and Table 27 (all shown in the 5' to 3' orientation, name followed by sequence):

**Table 26. Heavy Chain FR4 Forward Primers (for Sub-Bank 11):**

| | | |
|---|---|---|
| 964 | FR4H1 | TGGGGCCAGGGCACCCTGGTCACCGTCTCCTCA |
| 965 | FR4H2 | TGGGGCCGTGGCACCCTGGTCACTGTCTCCTCA |
| 966 | FR4H3 | TGGGGCCAAGGGACAATGGTCACCGTCTCTTCA |
| 967 | FR4H4 | TGGGGCCAAGGAACCCTGGTCACCGTCTCCTCA |
| 968 | FR4H5 | TGGGGCCAAGGAACCCTGGTCACCGTCTCCTCA |
| 969 | FR4H6 | TGGGGGCAAGGGACCACGGTCACCGTCTCCTCA |

**Table 27. Heavy Chain FR4 Reverse Primers (for Sub-Bank 11):**

| | | |
|---|---|---|
| 970 | FR4H1' | TGAGGAGACGGTGACCAGGGTGCCCTGGCCCCA |
| 971 | FR4H2' | TGAGGAGACAGTGACCAGGGTGCCACGGCCCCA |
| 972 | FR4H3' | TGAAGAGACGGTGACCATTGTCCCTTGGCCCCA |
| 973 | FR4H4' | TGAGGAGACGGTGACCAGGGTTCCTTGGCCCCA |
| 974 | FR4H5' | TGAGGAGACGGTGACCAGGGTTCCTTGGCCCCA |
| 975 | FR4H6' | TGAGGAGACGGTGACCGTGGTCCCTTGCCCCCA |

PCR is carried out using the following oligonucleotide combinations (6 in total): FR4H1/FR4H1', FR4H2/FR4H2', FR4H3/FR4H3', FR4H4/FR4H4', FR4H5/FR4H5', or FR4H6/FR4H6'. The pooling of the PCR products generates sub-bank 11.

In some embodiments, heavy chain FR sub-banks 8, 9, 10 and 11 are constructed wherein sub-bank 8 comprises nucleic acids, each of which encodes a heavy chain FR1; sub-bank 9 comprises nucleic acids, each of which encodes a heavy chain FR2; sub-bank 10 comprises nucleic acids, each of which encodes a heavy chain FR3; and sub-bank 11 comprises nucleic acids, each of which encodes a heavy chain FR4, respectively, and wherein the heavy chain FR1, FR2, and FR3 are defmed according to Chothia definition for CDR H1 and H2. In some embodiments, the FR sequences are derived form functional human anitbody sequences. In other embodiments, the FR sequences are derived from human germline heavy chain sequences.

By way of example but not limitation, the following describes a method of generating 4 heavy chain FR sub-banks using Polymerase Chain Reaction (PCR), wherein human germline heavy chain sequences are used as templates. Heavy chain FR sub-banks 7, 8 and 9 (encoding FR1, 2, 3 respectively) encompass 44 human germline heavy chain sequences (VH1-18, VH1-2, VH1-24, VH1-3, VH1-45, VH1-46, VH1-58, VH1-69, VH1-8, VH2-26, VH2-5, VH2-70, VH3-11, VH3-13, VH3-15, VH3-16, VH3-20, VH3-21, VH3-23, VH3-30, VH3-33, VH3-35, VH3-38, VH3-43, VH3-48, VH3-49, VH3-53, VH3-64, VH3-66, VH3-7, VH3-72, VH3-73, VH3-74, VH3-9, VH4-28, VH4-31, VH4-34, VH4-39, VH4-4, VH4-59, VH4-61, VH5-51, VH6-1 and VH7-81). See Matsuda et al., 1998, J. Exp. Med., 188:1973-1975. The sequences are summarized at the NCBI website: www.ncbi.nlm.nih.gov/igblast/showGermline.cgi?organism=human&chainType=VH&seqT ype=nucleotide. Sub-bank 11 (encodes FR4) is the same sub-bank 11 as described above.

By way of example but not limitation, the construction of heavy chain FR1 sub-bank (according to Chothia definition) is carried out using the Polymerase Chain Reaction by overlap extension using the oligonucleotides listed in Table 28 and Table 29 (all shown in the 5' to 3' orientation, name followed by sequence):

**Table 28. Heavy Chain FR1 (Chothia Definition) Forward Primers (for Sub-Bank 8):**

| | | |
|---|---|---|
| 976 | FR1HC1 | CAGGTTCAGCTGGTGCAGTCTGGAGCTGAGGTGAAGAAGCCTGGGGCCTCA |
| 977 | FR1HC2 | CAGGTGCAGCTGGTGCAGTCTGGGGCTGAGGTGAAGAAGCCTGGGGCCTCA |
| 978 | FR1HC3 | CAGGTCCAGCTGGTACAGTCTGGGGCTGAGGTGAAGAAGCCTGGGGCCTCA |
| 979 | FR1HC4 | CAGGTTCAGCTGGTGCAGTCTGGGGCTGAGGTGAAGAAGCCTGGGGCCTCA |
| 980 | FR1HC5 | CAGATGCAGCTGGTGCAGTCTGGGGCTGAGGTGAAGAAGACTGGGTCCTCA |
| 981 | FR1HC6 | CAGGTGCAGCTGGTGCAGTCTGGGGCTGAGGTGAAGAAGCCTGGGGCCTCA |
| 982 | FR1HC7 | CAAATGCAGCTGGTGCAGTCTGGGCCTGAGGTGAAGAAGCCTGGGACCTCA |
| 983 | FR1HC8 | CAGGTGCAGCTGGTGCAGTCTGGGGCTGAGGTGAAGAAGCCTGGGTCCTCG |
| 984 | FR1HC9 | CAGGTGCAGCTGGTGCAGTCTGGGGCTGAGGTGAAGAAGCCTGGGGCCTCA |
| 985 | FR1HC10 | CAGGTCACCTTGAAGGAGTCTGGTCCTGTGCTGGTGAAACCCACAGAGACC |
| 986 | FR1HC11 | CAGATCACCTTGAAGGAGTCTGGTCCTACGCTGGTGAAACCCACACAGACC |
| 987 | FR1HC12 | CAGGTCACCTTGAGGGAGTCTGGTCCTGCGCTGGTGAAACCCACACAGACC |
| 988 | FR1HC13 | CAGGTGCAGCTGGTGGAGTCTGGGGGAGGCTTGGTCAAGCCTGGAGGGTCC |
| 989 | FR1HC14 | GAGGTGCAGCTGGTGGAGTCTGGGGGAGGCTTGGTACAGCCTGGGGGGTCC |
| 990 | FR1HC15 | GAGGTGCAGCTGGTGGAGTCTGGGGGAGGCTTGGTAAAGCCTGGGGGGTCC |
| 991 | FR1HC16 | GAGGTGCAGCTGGTGGAGTCTGGGGGAGGCTTGGTACAGCCTGGGGGGTCC |
| 992 | FR1HC17 | GAGGTGCAGCTGGTGGAGTCTGGGGGAGGTGTGGTACGGCCTGGGGGGTCC |
| 993 | FR1HC18 | GAGGTGCAGCTGGTGGAGTCTGGGGGAGGCCTGGTCAAGCCTGGGGGGTCC |
| 994 | FR1HC19 | GAGGTGCAGCTGTTGGAGTCTGGGGGAGGCTTGGTACAGCCTGGGGGGTCC |
| 995 | FR1HC20 | CAGGTGCAGCTGGTGGAGTCTGGGGGAGGCGTGGTCCAGCCTGGGAGGTCC |
| 996 | FR1HC21 | CAGGTGCAGCTGGTGGAGTCTGGGGGAGGCGTGGTCCAGCCTGGGAGGTCC |
| 997 | FR1HC22 | GAGGTGCAGCTGGTGGAGTCTGGGGGAGGCTTGGTACAGCCTGGGGGATCC |
| 998 | FR1HC23 | GAGGTGCAGCTGGTGGAGTCTGGGGGAGGCTTGGTACAGCCTAGGGGGTCC |
| 999 | FR1HC24 | GAAGTGCAGCTGGTGGAGTCTGGGGGAGTCGTGGTACAGCCTGGGGGGTCC |
| 1000 | FR1HC25 | GAGGTGCAGCTGGTGGAGTCTGGGGGAGGCTTGGTACAGCCTGGGGGGTCC |
| 1001 | FR1HC26 | GAGGTGCAGCTGGTGGAGTCTGGGGGAGGCTTGGTACAGCCAGGGCGGTCC |
| 1002 | FR1HC27 | GAGGTGCAGCTGGTGGAGTCTGGAGGAGGCTTGATCCAGCCTGGGGGGTCC |
| 1003 | FR1HC28 | GAGGTGCAGCTGGTGGAGTCTGGGGAAGGCTTGGTCCAGCCTGGGGGGTCC |
| 1004 | FR1HC29 | GAGGTGCAGCTGGTGGAGTCTGGAGGAGGCTTGATCCAGCCTGGGGGGTCC |
| 1005 | FR1HC30 | GAGGTGCAGCTGGTGGAGTCTGGGGGAGGCTTGGTCCAGCCTGGGGGGTCC |
| 1006 | FR1HC31 | GAGGTGCAGCTGGTGGAGTCTGGGGGAGGCTTGGTCCAGCCTGGAGGGTCC |
| 1007 | FR1HC32 | GAGGTGCAGCTGGTGGAGTCCGGGGGAGGCTTGGTCCAGCCTGGGGGGTCC |
| 1008 | FR1HC33 | GAGGTGCAGCTGGTGGAGTCCGGGGGAGGCTTAGTTCAGCCTGGGGGGTCC |
| 1009 | FR1HC34 | GAAGTGCAGCTGGTGGAGTCTGGGGGAGGCTTGGTACAGCCTGGCAGGTCC |
| 1010 | FR1HC35 | CAGGTGCAGCTGCAGGAGTCGGGCCCAGGACTGGTGAAGCCTTCGGACACC |
| 1011 | FR1HC36 | CAGGTGCAGCTGCAGGAGTCGGGCCCAGGACTGGTGAAGCCTTCACAGACC |
| 1012 | FR1HC37 | CAGGTGCAGCTACAGCAGTGGGGCGCAGGACTGTTGAAGCCTTCGGAGACC |
| 1013 | FR1HC38 | CAGCTGCAGCTGCAGGAGTCGGGCCCAGGACTGGTGAAGCCTTCGGAGACC |
| 1014 | FR1HC39 | CAGGTGCAGCTGCAGGAGTCGGGCCCAGGACTGGTGAAGCCTTCGGAGACC |
| 1015 | FR1HC40 | CAGGTGCAGCTGCAGGAGTCGGGCCCAGGACTGGTGAAGCCTTCGGAGACC |
| 1016 | FR1HC41 | CAGGTGCAGCTGCAGGAGTCGGGCCCAGGACTGGTGAAGCCTTCGGAGACC |
| 1017 | FR1HC42 | GAGGTGCAGCTGGTGCAGTCTGGAGCAGAGGTGAAAAAGCCCGGGGAGTCT |
| 1018 | FR1HC43 | CAGGTACAGCTGCAGCAGTCAGGTCCAGGACTGGTGAAGCCCTCGCAGACC |
| 1019 | FR1HC44 | CAGGTGCAGCTGGTGCAGTCTGGCCATGAGGTGAAGCAGCCTGGGGCCTCA |

**Table 29. Heavy Chain FR1 (Chothia Definition) Reverse Primers (for Sub-Bank 8):**

| | | |
|---|---|---|
| 1020 | FR1HC1' | AGAAGCCTTGCAGGAGACCTTCACTGAGGCCCCAGGCTTCTTCAC |
| 1021 | FR1HC2' | AGAAGCCTTGCAGGAGACCTTCACTGAGGCCCCAGGCTTCTTCAC |
| 1022 | FR1HC3' | GGAAACCTTGCAGGAGACCTTCACTGAGGCCCCAGGCTTCTTCAC |
| 1023 | FR1HC4' | AGAAGCCTTGCAGGAAACCTTCACTGAGGCCCCAGGCTTCTTCAC |
| 1024 | FR1HC5' | GGAAGCCTTGCAGGAAACCTTCACTGAGGACCCAGTCTTCTTCAC |
| 1025 | FR1HC6' | AGATGCCTTGCAGGAAACCTTCACTGAGGCCCCAGGCTTCTTCAC |
| 1026 | FR1HC7' | AGAAGCCTTGCAGGAGACCTTCACTGAGGTCCCAGGCTTCTTCAC |
| 1027 | FR1HC8' | AGAAGCCTTGCAGGAGACCTTCACCGAGGACCCAGGCTTCTTCAC |
| 1028 | FR1HC9' | AGAAGCCTTGCAGGAGACCTTCACTGAGGCCCCAGGCTTCTTCAC |
| 1029 | FR1HC10' | AGAGACGGTGCAGGTCAGCGTGAGGGTCTCTGTGGGTTTCACCAG |
| 1030 | FR1HC11' | AGAGAAGGTGCAGGTCAGCGTGAGGGTCTGTGTGGGTTTCACCAG |
| 1031 | FR1HC12' | AGAGAAGGTGCAGGTCAGTGTGAGGGTCTGTGTGGGTTTCACCAG |
| 1032 | FR1HC13' | AGAGGCTGCACAGGAGAGTCTCAGGGACCCTCCAGGCTTGACCAA |
| 1033 | FR1HC14' | AGAGGCTGCACAGGAGAGTCTCAGGGACCCCCCAGGCTGTACCAA |
| 1034 | FR1HC15' | AGAGGCTGCACAGGAGAGTCTAAGGGACCCCCCAGGCTTTACCAA |
| 1035 | FR1HC16' | AGAGGCTGCACAGGAGAGTCTCAGGGACCCCCCAGGCTGTACCAA |
| 1036 | FR1HC17' | AGAGGCTGCACAGGAGAGTCTCAGGGACCCCCCAGGCCGTACCAC |
| 1037 | FR1HC18' | AGAGGCTGCACAGGAGAGTCTCAGGGACCCCCCAGGCTTGACCAG |
| 1038 | FR1HC19' | AGAGGCTGCACAGGAGAGTCTCAGGGACCCCCCAGGCTGTACCAA |
| 1039 | FR1HC20' | AGAGGCTGCACAGGAGAGTCTCAGGGACCTCCCAGGCTGGACCAC |
| 1040 | FR1HC21' | AGACGCTGCACAGGAGAGTCTCAGGGACCTCCCAGGCTGGACCAC |
| 1041 | FR1HC22' | AGAGGCTGCACAGGAGAGTCTCAGGGATCCCCCAGGCTGTACCAA |
| 1042 | FR1HC23' | AGAGGCTGCACAGGAGAGTCTCAGGGACCCCCTAGGCTGTACCAA |
| 1043 | FR1HC24' | AGAGGCTGCACAGGAGAGTCTCAGGGACCCCCCAGGCTGTACCAC |
| 1044 | FR1HC25' | AGAGGCTGCACAGGAGAGTCTCAGGGACCCCCCAGGCTGTACCAA |
| 1045 | FR1HC26' | AGAAGCTGTACAGGAGAGTCTCAGGGACCGCCCTGGCTGTACCAA |
| 1046 | FR1HC27' | AGAGGCTGCACAGGAGAGTCTCAGGGACCCCCCAGGCTGGATCAA |
| 1047 | FR1HC28' | AGAGGCTGCACAGGAGAGTCTCAGGGACCCCCCAGGCTGGACCAA |
| 1048 | FR1HC29' | AGAGGCTGCACAGGAGAGTCTCAGGGACCCCCCAGGCTGGATCAA |
| 1049 | FR1HC30' | AGAGGCTGCACAGGAGAGTCTCAGGGACCCCCCAGGCTGGACCAA |
| 1050 | FR1HC31' | AGAGGCTGCACAGGAGAGTCTCAGGGACCCTCCAGGCTGGACCAA |
| 1051 | FR1HC32' | AGAGGCTGCACAGGAGAGTTTCAGGGACCCCCCAGGCTGGACCAA |
| 1052 | FR1HC33' | AGAGGCTGCACAGGAGAGTCTCAGGGACCCCCCAGGCTGAACTAA |
| 1053 | FR1HC34' | AGAGGCTGCACAGGAGAGTCTCAGGGACCTGCCAGGCTGTACCAA |
| 1054 | FR1HC35' | AGAGACAGCGCAGGTGAGGGACAGGGTGTCCGAAGGCTTCACCAG |
| 1055 | FR1HC36' | AGAGACAGTACAGGTGAGGGACAGGGTCTGTGAAGGCTTCACCAG |
| 1056 | FR1HC37' | ATAGACAGCGCAGGTGAGGGACAGGGTCTCCGAAGGCTTCAACAG |
| 1057 | FR1HC38' | AGAGACAGTGCAGGTGAGGGACAGGGTCTCCGAAGGCTTCACCAG |
| 1058 | FR1HC39' | AGAGACAGTGCAGGTGAGGGACAGGGTCTCCGAAGGCTTCACCAG |
| 1059 | FR1HC40' | AGAGACAGTGCAGGTGAGGGACAGGGTCTCCGAAGGCTTCACCAG |
| 1060 | FR1HC41' | AGAGACAGTGCAGGTGAGGGACAGGGTCTCCGAAGGCTTCACCAG |
| 1061 | FR1HC42' | AGAACCCTTACAGGAGATCTTCAGAGACTCCCCGGGCTTTTTCAC |
| 1062 | FR1HC43' | GGAGATGGCACAGGTGAGTGAGAGGGTCTGCGAGGGCTTCACCAG |
| 1063 | FR1HC44' | AGAAGCCTTGCAGGAGACCTTCACTGAGGCCCCAGGCTGCTTCAC |

PCR is carried out using the following oligonucleotide combinations (44 in total): FR1HC1/FR1HC1', FR1HC2/FR1HC2', FR1HC3/FR1HC3', FR1HC4/FR1HC4', FR1HC5/FR1HC5', FR1HC6/FR1HC6', FR1HC7/FR1HC7', FR1HC8/FR1HC8', FR1HC9/FR1HC9', FR1HC10/FR1HC10', FR1HC11/FR1HC11', FR1HC12/FR1HC12', FR1HC13/FR1HC13', FR1HC14/FR1HC14', FR1HC15/FR1HC15', FR1HC16/FR1HC16', FR1HC17/FR1HC17', FR1HC18/FR1HC18', FR1HC19/FR1HC19', FR1HC20/FR1HC20', FR1HC21/FR1HC21', FR1HC22/FR1HC22', FR1HC23/FRlHC23', FR1HC24/FR1HC24', FR1HC25/FR1HC25', FR1HC26/FR1HC26', FR1HC27/FR1HC27', FR1HC28/FR1HC28', FR1HC29/FR1HC29', FR1HC30/FR1HC30', FR1HC31/FR1HC31', FR1HC32/FR1HC32', FR1HC33/FR1HC33', FR1HC34/FR1HC34', FR1HC35/FR1HC35', FR1HC36/FR1HC36', FR1HC37/FR1HC37', FR1HC38/FR1HC38', FR1HC39/FR1HC39', FR1HC40/FR1HC40', FR1HC41/FR1HC41', FR1HC42/FR1HC42', FR1HC43/FR1HC43', or FR1HC44/FR1HC44'. The pooling of the PCR products generates sub-bank 8.

By way of example but not limitation, the construction of heavy chain FR2 sub-bank (according to Chothia definition) is carried out using the Polymerase Chain Reaction by overlap extension using the oligonucleotides listed in Table 30 and Table 31 (all shown in the 5' to 3' orientation, name followed by sequence):

**Table 30. Heavy Chain FR2 (Chothia Definition) Forward Primers (for Sub-Bank 9):**

| | | |
|---|---|---|
| 1064 | FR2HC1 | TATGGTATCAGCTGGGTGCGACAGGCCCCTGGACAAGGGCTT |
| 1065 | FR2HC2 | TACTATATGCACTGGGTGCGACAGGCCCCTGGACAAGGGCTT |
| 1066 | FR2HC3 | TTATCCATGCACTGGGTGCGACAGGCTCCTGGAAAAGGGCTT |
| 1067 | FR2HC4 | TATGCTATGCATTGGGTGCGCCAGGCCCCCGGACAAAGGCTT |
| 1068 | FR2HC5 | CGCTACCTGCACTGGGTGCGACAGGCCCCCGGACAAGCGCTT |
| 1069 | FR2HC6 | TACTATATGCACTGGGTGCGACAGGCCCCTGGACAAGGGCTT |
| 1070 | FR2HC7 | TCTGCTATGCAGTGGGTGCGACAGGCTCGTGGACAACGCCTT |
| 1071 | FR2HC8 | TATGCTATCAGCTGGGTGCGACAGGCCCCTGGACAAGGGCTT |
| 1072 | FR2HC9 | TATGATATCAACTGGGTGCGACAGGCCACTGGACAAGGGCTT |
| 1073 | FR2HC10 | ATGGGTGTGAGCTGGATCCGTCAGCCCCCAGGGAAGGCCCTG |
| 1074 | FR2HC11 | GTGGGTGTGGGCTGGATCCGTCAGCCCCCAGGAAAGGCCCTG |
| 1075 | FR2HC12 | ATGTGTGTGAGCTGGATCCGTCAGCCCCCAGGGAAGGCCCTG |
| 1076 | FR2HC13 | TACTACATGAGCTGGATCCGCCAGGCTCCAGGGAAGGGGCTG |
| 1077 | FR2HC14 | TACGACATGCACTGGGTCCGCCAAGCTACAGGAAAAGGTCTG |
| 1078 | FR2HC15 | GCCTGGATGAGCTGGGTCCGCCAGGCTCCAGGGAAGGGGCTG |
| 1079 | FR2HC16 | AGTGACATGAACTGGGCCCGCAAGGCTCCAGGAAAGGGGCTG |
| 1080 | FR2HC17 | TATGGCATGAGCTGGGTCCGCCAAGCTCCAGGGAAGGGGCTG |
| 1081 | FR2HC18 | TATAGCATGAACTGGGTCCGCCAGGCTCCAGGGAAGGGGCTG |
| 1082 | FR2HC19 | TATGCCATGAGCTGGGTCCGCCAGGCTCCAGGGAAGGGGCTG |
| 1083 | FR2HC20 | TATGGCATGCACTGGGTCCGCCAGGCTCCAGGCAAGGGGCTG |
| 1084 | FR2HC21 | TATGGCATGCACTGGGTCCGCCAGGCTCCAGGCAAGGGGCTG |
| 1085 | FR2HC22 | AGTGACATGAACTGGGTCCATCAGGCTCCAGGAAAGGGGCTG |
| 1086 | FR2HC23 | AATGAGATGAGCTGGATCCGCCAGGCTCCAGGGAAGGGGCTG |
| 1087 | FR2HC24 | TATACCATGCACTGGGTCCGTCAAGCTCCGGGGAAGGGTCTG |
| 1088 | FR2HC25 | TATAGCATGAACTGGGTCCGCCAGGCTCCAGGGAAGGGGCTG |
| 1089 | FR2HC26 | TATGCTATGAGCTGGTTCCGCCAGGCTCCAGGGAAGGGGCTG |
| 1090 | FR2HC27 | AACTACATGAGCTGGGTCCGCCAGGCTCCAGGGAAGGGGCTG |
| 1091 | FR2HC28 | TATGCTATGCACTGGGTCCGCCAGGCTCCAGGGAAGGGACTG |
| 1092 | FR2HC29 | AACTACATGAGCTGGGTCCGCCAGGCTCCAGGGAAGGGGCTG |
| 1093 | FR2HC30 | TATTGGATGAGCTGGGTCCGCCAGGCTCCAGGGAAGGGGCTG |
| 1094 | FR2HC31 | CACTACATGGACTGGGTCCGCCAGGCTCCAGGGAAGGGGCTG |
| 1095 | FR2HC32 | TCTGCTATGCACTGGGTCCGCCAGGCTTCCGGGAAAGGGCTG |
| 1096 | FR2HC33 | TACTGGATGCACTGGGTCCGCCAAGCTCCAGGGAAGGGGCTG |
| 1097 | FR2HC34 | TATGCCATGCACTGGGTCCGGCAAGCTCCAGGGAAGGGCCTG |
| 1098 | FR2HC35 | AACTGGTGGGGCTGGATCCGGCAGCCCCCAGGGAAGGGACTG |
| 1099 | FR2HC36 | TACTACTGGAGCTGGATCCGCCAGCACCCAGGGAAGGGCCTG |
| 1100 | FR2HC37 | TACTACTGGAGCTGGATCCGCCAGCCCCCAGGGAAGGGGCTG |
| 1101 | FR2HC38 | TACTACTGGGGCTGGATCCGCCAGCCCCCAGGGAAGGGGCTG |
| 1102 | FR2HC39 | TACTACTGGAGCTGGATCCGGCAGCCCGCCGGGAAGGGACTG |
| 1103 | FR2HC40 | TACTACTGGAGCTGGATCCGGCAGCCCCCAGGGAAGGGACTG |
| 1104 | FR2HC41 | TACTACTGGAGCTGGATCCGGCAGCCCCCAGGGAAGGGACTG |
| 1105 | FR2HC42 | TACTGGATCGGCTGGGTGCGCCAGATGCCCGGGAAAGGCCTG |
| 1106 | FR2HC43 | GCTGCTTGGAACTGGATCAGGCAGTCCCCATCGAGAGGCCTT |
| 1107 | FR2HC44 | TATGGTATGAATTGGGTGCCACAGGCCCCTGGACAAGGGCTT |

**Table 31. Heavy Chain FR2 (Chothia Definition) Reverse Primers (for Sub-Bank 9):**

| | | |
|---|---|---|
| 1108 | FR2HC1' | GATCCATCCCATCCACTCAAGCCCTTGTCCAGGGGCCTG |
| 1109 | FR2HC2' | GATCCATCCCATCCACTCAAGCCCTTGTCCAGGGGCCTG |
| 1110 | FR2HC3' | AAAACCTCCCATCCACTCAAGCCCTTTTCCAGGAGCCTG |
| 1111 | FR2HC4' | GCTCCATCCCATCCACTCAAGCCTTTGTCCGGGGGCCTG |
| 1112 | FR2HC5' | GATCCATCCCATCCACTCAAGCGCTTGTCCGGGGGCCTG |
| 1113 | FR2HC6' | GATTATTCCCATCCACTCAAGCCCTTGTCCAGGGGCCTG |
| 1114 | FR2HC7' | GATCCATCCTATCCACTCAAGGCGTTGTCCACGAGCCTG |
| 1115 | FR2HC8' | GATCCCTCCCATCCACTCAAGCCCTTGTCCAGGGGCCTG |
| 1116 | FR2HC9' | CATCCATCCCATCCACTCAAGCCCTTGTCCAGTGGCCTG |
| 1117 | FR2HC10' | AATGTGTGCAAGCCACTCCAGGGCCTTCCCTGGGGGCTG |
| 1118 | FR2HC11' | AATGAGTGCAAGCCACTCCAGGGCCTTTCCTGGGGGCTG |
| 1119 | FR2HC12' | AATGAGTGCAAGCCACTCCAGGGCCTTCCCTGGGGGCTG |
| 1120 | FR2HC13' | AATGTATGAAACCCACTCCAGCCCCTTCCCTGGAGCCTG |
| 1121 | FR2HC14' | AATAGCTGAGACCCACTCCAGACCTTTTCCTGTAGCTTG |
| 1122 | FR2HC15' | AATACGGCCAACCCACTCCAGCCCCTTCCCTGGAGCCTG |
| 1123 | FR2HC16' | AACACCCGATACCCACTCCAGCCCCTITCCTGGAGCCTT |
| 1124 | FR2HC17' | AATACCAGAGACCCACTCCAGCCCCTTCCCTGGAGCTTG |
| 1125 | FR2HC18' | AATGGATGAGACCCACTCCAGCCCCTTCCCTGGAGCCTG |
| 1126 | FR2HC19' | AATAGCTGAGACCCACTCCAGCCCCTTCCCTGGAGCCTG |
| 1127 | FR2HC20' | TATAACTGCCACCCACTCCAGCCCCTTGCCTGGAGCCTG |
| 1128 | FR2HC21' | TATAACTGCCACCCACTCCAGCCCCTTGCCTGGAGCCTG |
| 1129 | FR2HC22' | AACACCCGATACCCACTCCAGCCCCTTTCCTGGAGCCTG |
| 1130 | FR2HC23' | AATGGATGAGACCCACTCCAGCCCCTTCCCTGGAGCCTG |
| 1131 | FR2HC24' | ATAAGAGAGACCCACTCCAGACCCTTCCCCGGAGCTTG |
| 1132 | FR2HC25' | AATGTATGAAACCCACTCCAGCCCCTTCCCTGGAGCCTG |
| 1133 | FR2HC26' | AATGAAACCTACCCACTCCAGCCCCTTCCCTGGAGCCTG |
| 1134 | FR2HC27' | AATAACTGAGACCCACTCCAGCCCCTTCCCTGGAGCCTG |
| 1135 | FR2HC28' | AATAGCTGAAACATATTCCAGTCCCTTCCCTGGAGCCTG |
| 1136 | FR2HC29' | AATAACTGAGACCCACTCCAGCCCCTTCCCTGGAGCCTG |
| 1137 | FR2HC30' | TATGTTGGCCACCCACTCCAGCCCCTTCCCTGGAGCCTG |
| 1138 | FR2HC31' | AGTACGGCCAACCCACTCCAGCCCCTTCCCTGGAGCCTG |
| 1139 | FR2HC32' | AATACGGCCAACCCACTCCAGCCCTTTCCCGGAAGCCTG |
| 1140 | FR2HC33' | AATACGTGAGACCCACACCAGCCCCTTCCCTGGAGCTTG |
| 1141 | FR2HC34' | AATACCTGAGACCCACTCCAGGCCCTTCCCTGGAGCTTG |
| 1142 | FR2HC35' | GATGTACCCAATCCACTCCAGTCCCTTCCCTGGGGGCTG |
| 1143 | FR2HC36' | GATGTACCCAATCCACTCCAGGCCCTTCCCTGGGTGCTG |
| 1144 | FR2HC37' | GATTTCCCCAATCCACTCCAGCCCCTTCCCTGGGGGCTG |
| 1145 | FR2HC38' | GATACTCCCAATCCACTCCAGCCCCTTCCCTGGGGGCTG |
| 1146 | FR2HC39' | GATACGCCCAATCCACTCCAGTCCCTTCCCGGCGGGCTG |
| 1147 | FR2HC40' | GATATACCCAATCCACTCCAGTCCCTTCCCTGGGGGCTG |
| 1148 | FR2HC41' | GATATACCCAATCCACTCCAGTCCCTTCCCTGGGGGCTG |
| 1149 | FR2HC42' | GATGATCCCCATCCACTCCAGGCCTTTCCCGGGCATCTG |
| 1150 | FR2HC43' | TGTCCTTCCCAGCCACTCAAGGCCTCTCGATGGGGACTG |
| 1151 | FR2HC44' | GAACCATCCCATCCACTCAAGCCCTTGTCCAGGGGCCTG |

PCR is carried out using the following oligonucleotide combinations (44 in total): FR2HC1/FR2HC1', FR2HC2/FR2HC2', FR2HC3/FR2HC3', FR2HC4/FR2HC4', FR2HC5/FR2HC5', FR2HC6/FR2HC6', FR2HC7/FR2HC7', FR2HC8/FR2HC8', FR2HC9/FR2HC9', FR2HC 10/FR2HC 10', FR2HC 11 /FR2HC 11', FR2HC12/FR2HC12', FR2HC13/FR2HC13', FR2HC14/FR2HC14', FR2HC15/FR2HC15', FR2HC16/FR2HC16', FR2HC17/FR2HC17', FR2HC18/FR2HC18', FR2HC19/FR2HC19', FR2HC20/FR2HC20', FR2HC21/FR2HC21', FR2HC22/FR2HC22', FR2HC23/FR2HC23', FR2HC24/FR2HC24', FR2HC25/FR2HC25', FR2HC26/FR2HC26', FR2HC27/FR2HC27', FR2HC28/FR2HC28', FR2HC29/FR2HC29', FR2HC30/FR2HC30', FR2HC31/FR2HC31', FR2HC32/FR2HC32', FR2HC33/FR2HC33', FR2HC34/FR2HC34', FR2HC35/FR2HC35', FR2HC36/FR2HC36', FR2HC37/FR2HC37', FR2HC38/FR2HC38', FR2HC39/FR2HC39', FR2HC40/FR2HC40', FR2HC41/FR2HC41', FR2HC42/FR2HC42', FR2HC43/FR2HC43', or FR2HC44/FR2HC44'. The pooling of the PCR products generates sub-bank 9.

By way of example but not limitation, the construction of heavy chain FR3 sub-bank (according to Chothia definition) is carried out using the Polymerase Chain Reaction by overlap extension using the oligonucleotides listed in Table 32 and Table 33 (all shown in the 5' to 3' orientation, name followed by sequence):

**Table 32. Heavy Chain FR3 (Chothia Definition) Forward Primers (for Sub-Bank 10):**

| | |
|---|---|
| 1152 | FR3HC1 ACAAACTATGCACAGAAGCTCCAGGGCAGAGTCACCATGACCACAGACACATCCACGAGCACAGCCTACATGG |
| 1153 | FR3HC2 ACAAACTATGCACAGAAGTTTCAGGGCAGGGTCACCATGACCAGGGACACGTCCATCAGCACAGCCTACATGG |
| 1154 | FR3HC3 ACAATCTACGCACAGAAGTTCCAGGGCAGAGTCACCATGACCGAGGACACATCTACAGACACAGCCTACATGG |
| 1155 | FR3HC4 ACAAAATATTCACAGGAGTTCCAGGGCAGAGTCACCATTACCAGGGACACATCCGCGAGCACAGCCTACATGG |
| 1156 | FR3HC5 ACCAACTACGCACAGAAATTCCAGGACAGAGTCACCATTACCAGGGACAGGTCTATGAGCACAGCCTACATGG |
| 1157 | FR3HC6 ACAAGCTACGCACAGAAGTTCCAGGGCAGAGTCACCATGACCAGGGACACGTCCACGAGCACAGTCTACATGG |
| 1158 | FR3HC7 ACAAACTACGCACAGAAGTTCCAGGAAAGAGTCACCATTACCAGGGACATGTCCACAAGCACAGCCTACATGG |
| 1159 | FR3HC8 GCAAACTACGCACAGAAGTTCCAGGGCAGAGTCACGATTACCGCGGACAAATCCACGAGCACAGCCTACATGG |
| 1160 | FR3HC9 ACAGGCTATGCACAGAAGTTCCAGGGCAGAGTCACCATGACCAGGAACACCTCCATAAGCACAGCCTACATGG |
| 1161 | FR3HC10 AAATCCTACAGCACATCTCTGAAGAGCAGGCTCACCATCTCCAAGGACACCTCCAAAAGCCAGGTGGTCCTTA |
| 1162 | FR3HC11 AAGCGCTACAGCCCATCTCTGAAGAGCAGGCTCACCATCACCAAGGACACCTCCAAAAACCAGGTGGTCCTTA |
| 1163 | FR3HC12 AAATACTACAGCACATCTCTGAAGACCAGGCTCACCATCTCCAAGGACACCTCCAAAAACCAGGTGGTCCTTA |
| 1164 | FR3HC13 ATATACTACGCAGACTCTGTGAAGGGCCGATTCACCATCTCCAGGGACAACGCCAAGAACTCACTGTATCTGC |
| 1165 | FR3HC14 ACATACTATCCAGGCTCCGTGAAGGGCCGATTCACCATCTCCAGAGAAAATGCCAAGAACTCCTTGTATCTTC |
| 1166 | FR3HC15 ACAGACTACGCTGCACCCGTGAAAGGCAGATTCACCATCTCAAGAGATGATTCAAAAAACACGCTGTATCTGC |
| 1167 | FR3HC16 ACGCACTATGTGGACTCCGTGAAGCGCCGATTCATCATCTCCAGAGACAATTCCAGGAACTCCCTGTATCTGC |
| 1168 | FR3HC17 ACAGGTTATGCAGACTCTGTGAAGGGCCGATTCACCATCTCCAGAGACAACGCCAAGAACTCCCTGTATCTGC |
| 1169 | FR3HC18 ATATACTACGCAGACTCAGTGAAGGGCCGATTCACCATCTCCAGAGACAACGCCAAGAACTCACTGTATCTGC |
| 1170 | FR3HC19 ACATACTACGCAGACTCCGTGAAGGGCCGGTTCACCATCTCCAGAGACAATTCCAAGAACACGCTGTATCTGC |
| 1171 | FR3HC20 AAATACTATGCAGACTCCGTGAAGGGCCGATTCACCATCTCCAGAGACAATTCCAAGAACACGCTGTATCTGC |
| 1172 | FR3HC21 AAATACTATGCAGACTCCGTGAAGGGCCGATTCACCATCTCCAGAGACAATTCCAAGAACACGCTGTATCTGC |
| 1173 | FR3HC22 ACGCACTATGCAGACTCTGTGAAGGGCCGATTCATCATCTCCAGAGACAATTCCAGGAACACCCTGTATCTGC |
| 1174 | FR3HC23 ACATACTACGCAGACTCCAGGAAGGGCAGATTCACCATCTCCAGAGACAATTCCAAGAACACGCTGTATCTTC |
| 1175 | FR3HC24 ACATACTATGCAGACTCTGTGAAGGGCCGATTCACCATCTCCAGAGACAACAGCAAAAACTCCCTGTATCTGC |
| 1176 | FR3HC25 ATATACTACGCAGACTCTGTGAAGGGCCGATTCACCATCTCCAGAGACAATGCCAAGAACTCACTGTATCTGC |
| 1177 | FR3HC26 ACAGAATACGCCGCGTCTGTGAAAGGCAGATTCACCATCTCAAGAGATGATTCCAAAAGCATCGCCTATCTGC |
| 1178 | FR3HC27 ACATACTACGCAGACTCCGTGAAGGGCCGATTCACCATCTCCAGAGACAATTCCAAGAACACGCTGTATCTTC |
| 1179 | FR3HC28 ACATATTATGCAGACTCTGTGAAGGGCAGATTCACCATCTCCAGAGACAATTCCAAGAACACGCTGTATCTTC |
| 1180 | FR3HC29 ACATACTACGCAGACTCCGTGAAGGGCCGATTCACCATCTCCAGAGACAATTCCAAGAACACGCTGTATCTTC |
| 1181 | FR3HC30 AAATACTATGTGGACTCTGTGAAGGGCCGATTCACCATCTCCAGAGACAACGCCAAGAACTCACTGTATCTGC |
| 1182 | FR3HC31 ACAGAATACGCCGCGTCTGTGAAAGGCAGATTCACCATCTCAAGAGATGATTCAAAGAACTCACTGTATCTGC |
| 1183 | FR3HC32 ACAGCATATGCTGCGTCGGTGAAAGGCAGGTTCACCATCTCCAGAGATGATTCAAAGAACACGGCGTATCTGC |
| 1184 | FR3HC33 ACAAGCTACGCGGACTCCGTGAAGGGCCGATTCACCATCTCCAGAGACAACGCCAAGAACACGCTGTATCTGC |
| 1185 | FR3HC34 ATAGGCTATGCGGACTCTGTGAAGGGCCGATTCACCATCTCCAGAGACAACGCCAAGAACTCCCTGTATCTGC |
| 1186 | FR3HC35 ACCTACTACAACCCGTCCCTCAAGAGTCGAGTCACCATGTCAGTAGACACGTCCAAGAACCAGTTCTCCCTGA |
| 1187 | FR3HC36 ACCTACTACAACCCGTCCCTCAAGAGTCGAGTTACCATATCAGTAGACACGTCTAAGAACCAGTTCTCCCTGA |
| 1188 | FR3HC37 ACCAACTACAACCCGTCCCTCAAGAGTCGAGTCACCATATCAGTAGACACGTCCAAGAACCAGTTCTCCCTGA |
| 1189 | FR3HC38 ACCTACTACAACCCGTCCCTCAAGAGTCGAGTCACCATATCCGTAGACACGTCCAAGAACCAGTTCTCCCTGA |
| 1190 | FR3HC39 ACCAACTACAACCCCTCCCTCAAGAGTCGAGTCACCATGTCAGTAGACACGTCCAAGAACCAGTTCTCCCTGA |
| 1191 | FR3HC40 ACCAACTACAACCCCTCCCTCAAGAGTCGAGTCACCATATCAGTAGACACGTCCAAGAACCAGTTCTCCCTGA |
| 1192 | FR3HC41 ACCAACTACAACCCCTCCCTCAAGAGTCGAGTCACCATATCAGTAGACACGTCCAAGAACCAGTTCTCCCTGA |
| 1193 | FR3HC42 ACCAGATACAGCCCGTCCTTCCAAGGCCAGGTCACCATCTCAGCCGACAAGTCCATCAGCACCGCCTACCTGC |
| 1194 | FR3HC43 AATGATTATGCAGTATCTGTGAAAAGTCGAATAACCATCAACCCAGACACATCCAAGAACCAGTTCTCCCTGC |
| 1195 | FR3HC44 CCAACATATGCCCAGGGCTTCACAGGACGGTTTGTCTTCTCCATGGACACCTCTGCCAGCACAGCATACCTGC |

**Table 33. Heavy Chain FR3 (Chothia Definition) Reverse Primers (for Sub-Bank 10):**

| | |
|---|---|
| 1196 | FR3HC1' TCTCGCACAGTAATACACGGCCGTGTCGTCAGATCTCAGGCTCCTCAGCTCCATGTAGGCTGTGCTCGTGG |
| 1197 | FR3HC2' TCTCGCACAGTAATACACGGCCGTGTCGTCAGATCTCAGCCTGCTCAGCTCCATGTAGGCTGTGCTGATGG |
| 1198 | FR3HC3' TGTTGCACAGTAATACACGGCCGTGTCCTCAGATCTCAGGCTGCTCAGCTCCATGTAGGCTGTGTCTGTAG |
| 1199 | FR3HC4' TCTCGCACAGTAATACACAGCCATGTCCTCAGATCTCAGGCTGCTCAGCTCCATGTAGGCTGTGCTCGCGG |
| 1200 | FR3HC5' TCTTGCACAGTAATACATGGCTGTGTCCTCAGATCTCAGGCTGCTCAGCTCCATGTAGGCTGTGCTCATAG |
| 1201 | FR3HC6' TCTCGCACAGTAATACACGGCCGTGTCCTCAGATCTCAGGCTGCTCAGCTCCATGTAGACTGTGCTCGTGG |
| 1202 | FR3HC7' TGCCGCACAGTAATACACGGCCGTGTCCTCGGATCTCAGGCTGCTCAGCTCCATGTAGGCTGTGCTTGTGG |
| 1203 | FR3HC8' TCTCGCACAGTAATACACGGCCGTGTCCTCAGATCTCAGGCTGCTCAGCTCCATGTAGGCTGTGCTCGTGG |
| 1204 | FR3HC9' TCTCGCACAGTAATACACGGCCGTGTCCTCAGATCTCAGGCTGCTCAGCTCCATGTAGGCTGTGCTTATGG |
| 1205 | FR3HC10' CCGTGCACAGTAATATGTGGCTGTGTCCACAGGGTCCATGTTGGTCATGGTAAGGACCACCTGGCTTTTGG |
| 1206 | FR3HC11' GTGTGCACAGTAATATGTGGCTGTGTCCACAGGGTCCATGTTGGTCATTGTAAGGACCACCTGGTTTTTGG |
| 1207 | FR3HC12' CCGTGCACAATAATACGTGGCTGTGTCCACAGGGTCCATGTTGGTCATTGTAAGGACCACCTGGTTTTTGG |
| 1208 | FR3HC13' TCTCGCACAGTAATACACGGCCGTGTCCTCGGCTCTCAGGCTGTTCATTTGCAGATACAGTGAGTTCTTGG |
| 1209 | FR3HC14' TCTTGCACAGTAATACACAGCCGTGTCCCCGGCTCTCAGGCTGTTCATTTGAAGATACAAGGAGTTCTTGG |
| 1210 | FR3HC15' TGTGGTACAGTAATACACGGCTGTGTCCTCGGTTTTCAGGCTGTTCATTTGCAGATACAGCGTGTTTTTG |
| 1211 | FR3HC16' TCTCACACAGTAATACACAGCCATGTCCTCGGCTCTCCGTCTGTTCTTTTGCAGATACAGGGAGTTCCTGG |
| 1212 | FR3HC17' TCTCGCACAGTGATACAAGGCCGTGTCCTCGGCTCTCAGACTGTTCATTTGCAGATACAGGGAGTTCTTGG |
| 1213 | FR3HC18' TCTCGCACAGTAATACACAGCCGTGTCCTCGGCTCTCAGGCTGTTCATTTGCAGATACAGTGAGTTCTTGG |
| 1214 | FR3HC19' TTTCGCACAGTAATATACGGCCGTGTCCTCGGCTCTCAGGCTGTTCATTTGCAGATACAGCGTGTTCTTGG |
| 1215 | FR3HC20' TCTCGCACAGTAATACACAGCCGTGTCCTCAGCTCTCAGGCTGTTCATTTGCAGATACAGCGTGTTCTTGG |
| 1216 | FR3HC21' TCTCGCACAGTAATACACAGCCGTGTCCTCGGCTCTCAGGCTGTTCATTTGCAGATACAGCGTGTTCTTGG |
| 1217 | FR3HC22' TCTCACACAGTAATACACAGCCGTGTCCTCGGCCCTCAGGCTATTCGTTTGCAGATACAGGGTGTTCCTGG |
| 1218 | FR3HC23' TCTGGCACAGTAATACACGGCCGTGCCCTCAGCTCTCAGGTTGTTCATTTGAAGATACAGCGTGTTCTTGG |
| 1219 | FR3HC24' TTTTGCACAGTAATACAAGGCGGTGTCCTCAGTTCTCAGACTGTTCATTTGCAGATACAGGGAGTTTTTGC |
| 1220 | FR3HC25' TCTCGCACAGTAATACACAGCCGTGTCCTCGTCTCTCAGGCTGTTCATTTGCAGATACAGTGAGTTCTTGG |
| 1221 | FR3HC26' TCTAGTACAGTAATACACGGCTGTGTCCTCGGTTTTCAGGCTGTTCATTTGCAGATAGGCGATGCTTTTGG |
| 1222 | FR3HC27' TCTCGCACAGTAATACACGGCCGTGTCCTCGGCTCTCAGGCTGTTCATTTGAAGATACAGCGTGTTCTTGG |
| 1223 | FR3HC28' TCTCGCACAGTAATACACAGCCATGTCCTCAGCTCTCAGGCTGCCCATTTGAAGATACAGCGTGTTCTTGG |
| 1224 | FR3HC29' TCTCGCACAGTAATACACAGCCGTGTCCTCAGCTCTCAGGCTGTTCATTTGAAGATACAGCGTGTTCTTGG |
| 1225 | FR3HC30' TCTCGCACAGTAATACACAGCCGTGTCCTCGGCTCTCAGGCTGTTCATTTGCAGATACAGTGAGTTCTTGG |
| 1226 | FR3HC31' TCTAGCACAGTAATACACGGCCGTGTCCTCGGTTTTCAGGCTGTTCATTTGCAGATACAGTGAGTTCTTTG |
| 1227 | FR3HC32' TCTAGTACAGTAATACACGGCCGTGTCCTCGGTTTTCAGGCTGTTCATTTGCAGATACGCCGTGTTCTTTG |
| 1228 | FR3HC33' TCTTGCACAGTAATACACAGCCGTGTCCTCGGCTCTCAGACTGTTCATTTGCAGATACAGCGTGTTCTTGG |
| 1229 | FR3HC34' TTTTGCACAGTAATACAAGGCCGTGTCCTCAGCTCTCAGACTGTTCATTTGCAGATACAGGGAGTTCTTGG |
| 1230 | FR3HC35' TCTCGCACAGTAATACACGGCCGTGTCCACGGCGGTCACAGAGCTCAGCTTCAGGGAGAACTGGTTCTTGG |
| 1231 | FR3HC36' TCTCGCACAGTAATACACGGCCGTGTCCGCGGCAGTCACAGAGCTCAGCTTCAGGGAGAACTGGTTCTTAG |
| 1232 | FR3HC37' TCTCGCACAGTAATACACAGCCGTGTCCGCGGCGGTCACAGAGCTCAGCTTCAGGGAGAACTGGTTCTTGG |
| 1233 | FR3HC38' TCTCGCACAGTAATACACAGCCGTGTCTGCGGCGGTCACAGAGCTCAGCTTCAGGGAGAACTGGTTCTTGG |
| 1234 | FR3HC39' TCTCGCACAGTAATACACGGCCGTGTCCGCGGCGGTCACAGAGCTCAGCTTCAGGGAGAACTGGTTCTTGG |
| 1235 | FR3HC40' TCTCGCACAGTAATACACGGCCGTGTCCGCAGCGGTCACAGAGCTCAGCTTCAGGGAGAACTGGTTCTTGG |
| 1236 | FR3HC41' TCTCGCACAGTAATACACGGCCGTGTCCGCAGCGGTCACAGAGCTCAGCTTCAGGGAGAACTGGTTCTTGG |
| 1237 | FR3HC42' TCTCGCACAGTAATACATGGCGGTGTCCGAGGCCTTCAGGCTGCTCCACTGCAGGTAGGCGGTGCTGATGG |
| 1238 | FR3HC43' TCTTGCACAGTAATACACAGCCGTGTCCTCGGGAGTCACAGAGTTCAGCTGCAGGGAGAACTGGTTCTTGG |
| 1239 | FR3HC44' TCTCGCACAGTAATACATGGCCATGTCCTCAGCCTTTAGGCTGCTGATCTGCAGGTATGCTGTGCTGGCAG |

PCR is carried out using the following oligonucleotide combinations (44 in total): FR3HC1/FR3HC1', FR3HC2/FR3HC2', FR3HC3/FR3HC3', FR3HC4/FR3HC4', FR3HC5/FR3HC5', FR3HC6/FR3HC6', FR3HC7/FR3HC7', FR3HC8/FR3HC8', FR3HC9/FR3HC9', FR3HC10/FR3HC10', FR3HC11/FR3HC11', FR3HC12/FR3HC12', FR3HC13/FR3HC13', FR3HC14/FR3HC14', FR3HC15/FR3HC15', FR3HC16/FR3HC16', FR3HC17/FR3HC17', FR3HC18/FR3HC18', FR3HC19/FR3HC19', FR3HC20/FR3HC20', FR3HC21/FR3HC21', FR3HC22/FR3HC22', FR3HC23/FR3HC23', FR3HC24/FR3HC24', FR3HC25/FR3HC25', FR3HC26/FR3HC26', FR3HC27/FR3HC27', FR3HC28/FR3HC28', FR3HC29/FR3HC29', FR3HC30/FR3HC30', FR3HC31/FR3HC31', FR3HC32/FR3HC32', FR3HC33/FR3HC33', FR3HC34/FR3HC34', FR3HC35/FR3HC35', FR3HC36/FR3HC36', FR3HC37/FR3HC37', FR3HC38/FR3HC38', FR3HC39/FR3HC39', FR3HC40/FR3HC40', FR3HC41/FR3HC41', FR3HC42/FR3HC42', FR3HC43/FR3HC43', or FR3HC44/FR3HC44'. The pooling of the PCR products generates sub-bank 10.

### 2.3 Selection of CDRs

In addition to the synthesis of framework region sub-banks, sub-banks of CDRs can be generated and randomly fused in frame with framework regions from framework region sub-banks to produced combinatorial libraries of antibodies (with or without constant regions) that can be screened for their immunospecificity for an antigen of interest, as well as their immunogenicity in an organism of interest. The combinatorial library methodology of the invention is exemplified herein for the production of humanized antibodies for use in human beings. However, the combinatorial library methodology of the invention can readily be applied to the production of antibodies for use in any organism of interest.

The present invention provides for a CDR sub-bank for each CDR of the variable light chain and variable heavy chain. Accordingly, the invention provides a CDR region sub-bank for variable light chain CDR1, variable light chain CDR2, and variable light CDR3 for each species of interest and for each definition of a CDR (*e.g.*, Kabat and Chothia). The invention also provides a CDR sub-bank for variable heavy chain CDR1, variable heavy CDR2, and variable heavy chain CDR3 for each species of interest and for each definition of a CDR (*e.g.*, Kabat and Chothia). A CDR sub-banks may comprise CDRs that have been identified as part of an antibody that immunospecifically to an antigen of interest. The CDR sub-banks can be readily used to synthesize a combinatorial library of antibodies which can be screened for their immunospecificity for an antigen of interest, as well as their immunogencity in an organism of interest.

For example, light chain CDR sub-banks 12, 13 and 14 can be constructed, wherein CDR sub-bank 12 comprises a plurality of nucleic acid sequences comprising nucleotide sequences, each nucleotide sequence encoding light chain CDR1 according to Kabat system; CDR sub-bank 13 comprises a plurality of nucleic acid sequences comprising nucleotide sequences, each nucleotide sequence encoding light chain CDR2 according to Kabat system; and CDR sub-bank 14 comprises a plurality of nucleic acid sequences comprising nucleotide sequences, each nucleotide sequence encoding light chain CDR3 according to Kabat system. Light chain CDR sub-banks 15, 16 and 17 can be constructed, wherein CDR sub-bank 15 comprises a plurality of nucleic acid sequences comprising nucleotide sequences, each nucleotide sequence encoding light chain CDR1 according to Chothia system; CDR sub-bank 16 comprises a plurality of nucleic acid sequences comprising nucleotide sequences, each nucleotide sequence encoding light chain CDR2 according to Chothia system; and CDR sub-bank 17 comprises a plurality of nucleic acid sequences comprising nucleotide sequences, each nucleotide sequence encoding light chain CDR3 according to Chothia system

Heavy chain CDR sub-bank 18, 19 and 20 can be constructed, wherein CDR sub-bank 18 comprises a plurality of nucleic acid sequences comprising nucleotide sequences, each nucleotide sequence encoding heavy chain CDR1 according to Kabat system; CDR sub-bank 19 comprises a plurality of nucleic acid sequences comprising nucleotide sequences, each nucleotide sequence encoding heavy chain CDR2 according to Kabat system; and CDR sub-bank 20 comprises a plurality of nucleic acid sequences comprising nucleotide sequences, each nucleotide sequence encoding heavy chain CDR3 according to Kabat system. Heavy chain CDR sub-bank 21, 22 and 23 can be constructed, wherein CDR sub-bank 21 comprises a plurality of nucleic acid sequences comprising nucleotide sequences, each nucleotide sequence encoding heavy chain CDR1 according to Chothia system; CDR sub-bank 22 comprises a plurality of nucleic acid sequences comprising nucleotide sequences, each nucleotide sequence encoding heavy chain CDR2 according to Chothia system; and CDR sub-bank 23 comprises a plurality of nucleic acid sequences comprising nucleotide sequences, each nucleotide sequence encoding heavy chain CDR3 according to Chothia system.

In some embodiments, the CDR sequences are derived from functional antibody sequences. In some embodiments, the CDR sequences are random sequences, which comprises at least 5, at least 6, at least 7, at least 8, at least 9, or at least 10 contiguous nucleotide sequence, synthesized by any methods known in the art. The CDR sub-banks can be used for construction of combinatorial sub-libraries. Alternatively, a CDR of particular interest can be selected and then used for the construction of combinatorial sub-libraries (see Section 5.3).

### 2.4 Construction of Combinatorial Sub-libraries

Combinatorial sub-libraries are constructed by fusing in frame non-human CDRs with corresponding human framework regions of the FR sub-banks. For example, combinatorial sub-library 1 is constructed by fusing in frame non-human CDR with corresponding kappa light chain human framework regions using sub-banks 1; combinatorial sub-library 2 is constructed by fusing in frame non-human CDR with corresponding kappa light chain human framework regions using sub-banks 2; combinatorial sub-library 3 is constructed by fusing in frame non-human CDR with corresponding kappa light chain human framework regions using sub-banks 3; combinatorial sub-library 4 is constructed by fusing in frame non-human CDR with corresponding kappa light chain human framework regions using sub-banks 4; combinatorial sub-libraries 5, 6, and 7 are constructed by fusing in frame non-human CDRs (Kabat definition for CDR H1 and H2) with the corresponding heavy chain human framework regions using sub-banks 5, 6 and 7, respectively; combinatorial sub-libraries 8, 9 and 10 are constructed by fusing in frame non-human CDRs (Chothia definition for CDR H1 and H2) with the corresponding heavy chain human framework regions using sub-banks 8, 9 and 10, respectively; combinatorial sub-library 11 is constructed by fusing in frame non-human CDR H3 (Kabat and Chothia definition) with the corresponding human heavy chain framework regions using sub-bank 11. In some embodiments, the non-human CDRs may also be selected from a CDR library.

The construction of combinatorial sub-libraries can be carried out using any method known in the art. By way of example but not limitation, the combinatorial sub-library 1 is constructed using the Polymerase Chain Reaction (PCR) by overlap extension using the oligonucleotides in Table 34 and Table 35 (all shown in the 5' to 3' orientation, name followed by sequence) where K= G or T, M= A or C, R= A or G, S= C or G, W= A or T and Y= C or T.

**Table 34. Light Chain FR1 Antibody-Specific Forward Primers (for Sub-Library 1)**

| | | |
|---|---|---|
| 1240 | AL1 | GATGTTGTGATGACWCAGTCT |
| 1241 | AL2 | GACATCCAGATGAYCCAGTCT |
| 1242 | AL3 | GCCATCCAGWTGACCCAGTCT |
| 1243 | AL4 | GAAATAGTGATGAYGCAGTCT |
| 1244 | AL5 | GAAATTGTGTTGACRCAGTCT |
| 1245 | AL6 | GAKATTGTGATGACCCAGACT |
| 1246 | AL7 | GAAATTGTRMTGACWCAGTCT |
| 1247 | AL8 | GAYATYGTGATGACYCAGTCT |
| 1248 | AL9 | GAAACGACACTCACGCAGTCT |
| 1249 | AL10 | GACATCCAGTTGACCCAGTCT |
| 1250 | AL11 | AACATCCAGATGACCCAGTCT |
| 1251 | AL12 | GCCATCCGGATGACCCAGTCT |
| 1252 | AL13 | GTCATCTGGATGACCCAGTCT |

**Table 35. Light Chain FR1 Antibody-Specific Reverse Primers (for Sub-Library 1)**

| | | |
|---|---|---|
| 1253 | AL1' | [first 70% of CDR L1]-GCAGGAGATGGAGGCCGGCTS |
| 1254 | AL2' | [first 70% of CDR L1]-GCAGGAGAGGGTGRCTCTTTC |
| 1255 | AL3' | [first 70% of CDR L1]-ACAASTGATGGTGACTCTGTC |
| 1256 | AL4' | [first 70% of CDR L1]-GAAGGAGATGGAGGCCGGCTG |
| 1257 | AL5' | [first 70% of CDR L1]-GCAGGAGATGGAGGCCTGCTC |
| 1258 | AL6' | [first 70% of CDR L1]-GCAGGAGATGTTGACTTTGTC |
| 1259 | AL7' | [first 70% of CDR L1]-GCAGGTGATGGTGACTTTCTC |
| 1260 | AL8' | [first 70% of CDR L1]-GCAGTTGATGGTGGCCCTCTC |
| 1261 | AL9' | [first 70% of CDR L1]-GCAAGTGATGGTGACTCTGTC |
| 1262 | AL10' | [first 70% of CDR L1]-GCAAATGATACTGACTCTGTC |

PCR is carried out with AL1 to AL13 in combination with AL1' to AL10' using sub-bank 1 as a template. This generates combinatorial sub-library 1 or a pool of oligonucleotides corresponding to sequences described in Table 1.

By way of example but not limitation, the combinatorial sub-library 2 is constructed using the Polymerase Chain Reaction (PCR) by overlap extension using the oligonucleotides in Table 36 and Table 37 (all shown in the 5' to 3' orientation, name followed by sequence) where K= G or T, M= A or C, R= A or G, S= C or G, W= A or T and Y = C or T.

**Table 36. Light Chain FR2 Antibody-Specific Forward Primers (for Sub-Library 2):**

| | | |
|---|---|---|
| 1263 | BL1 | [last 70% of CDR L1]-TGGYTTCAGCAGAGGCCAGGC |
| 1264 | BL2 | [last 70% of CDR L1]-TGGTACCTGCAGAAGCCAGGS |
| 1265 | BL3 | [last 70% of CDR L1]-TGGTATCRGCAGAAACCAGGG |
| 1266 | BL4 | [last 70% of CDR L1]-TGGTACCARCAGAAACCAGGA |
| 1267 | BL5 | [last 70% of CDR L1]-TGGTACCARCAGAAACCTGGC |
| 1268 | BL6 | [last 70% of CDR L1]-TGGTAYCWGCAGAAACCWGGG |
| 1269 | BL7 | [last 70% of CDR L1]-TGGTATCAGCARAAACCWGGS |
| 1270 | BL8 | [last 70% of CDR L1]-TGGTAYCAGCARAAACCAG |
| 1271 | BL9 | [last 70% of CDR L1]-TGGTTTCTGCAGAAAGCCAGG |
| 1272 | BL10 | [last 70% of CDR L1]-TGGTTTCAGCAGAAACCAGGG |

**Table 37. Light Chain FR2 Antibody-Specific Reverse Primers (for Sub-Library 2)**

| | | |
|---|---|---|
| 1273 | BL1' | [first 70% of CDR L2]-ATAGATCAGGAGCTGTGGAGR |
| 1274 | BL2' | [first 70% of CDR L2]-ATAGATCAGGAGCTTAGGRGC |
| 1275 | BL3' | [first 70% of CDR L2]-ATAGATGAGGAGCCTGGGMGC |
| 1276 | BL4' | [first 70% of CDR L2]-RTAGATCAGGMGCTTAGGGGC |
| 1277 | BL5' | [first 70% of CDR L2]-ATAGATCAGGWGCTTAGGRAC |
| 1278 | BL6' | [first 70% of CDR L2]-ATAGATGAAGAGCTTAGGGGC |
| 1279 | BL7' | [first 70% of CDR L2]-ATAAATTAGGAGTCTTGGAGG |
| 1280 | BL8' | [first 70% of CDR L2]-GTAAATGAGCAGCTTAGGAGG |
| 1281 | BL9' | [first 70% of CDR L2]-ATAGATCAGGAGTGTGGAGAC |
| 1281 | BL10' | [first 70% of CDR L2]-ATAGATCAGGAGCTCAGGGGC |
| 1283 | BL11' | [first 70% of CDR L2]-ATAGATCAGGGACTTAGGGGC |
| 1284 | BL12' | [first 70% of CDR L2]-ATAGAGGAAGAGCTTAGGGGA |
| 1285 | BL13' | [first 70% of CDR L2]-CTTGATGAGGAGCTTTGGAGA |
| 1286 | BL14' | [first 70% of CDR L2]-ATAAATTAGGCGCCTTGGAGA |
| 1287 | BL15' | [first 70% of CDR L2]-CTTGATGAGGAGCTTTGGGGC |
| 1288 | BL16' | [first 70% of CDR L2]-TTGAATAATGAAAATAGCAGC |

PCR is carried out with BL1 to BL10 in combination with BL1' to BL16' using sub-bank 2 as a template. This generates combinatorial sub-library 2 or a pool of oligonucleotides corresponding to sequences described in Table 2.

By way of example but not limitation, the combinatorial sub-library 3 is constructed using the Polymerase Chain Reaction (PCR) by overlap extension using the oligonucleotides in Table 38 and Table 39 (all shown in the 5' to 3' orientation, name followed by sequence) where K= G or T, M= A or C, R= A or G, S= C or G, W= A or T and Y= C or T.

**Table 38. Light Chain FR3 Antibody-Specific Forward Primers (for Sub-Library 3):**

| | |
|---|---|
| 1289 | CL1 [Last 70% of CDR L2]-GGGGTCCCAGACAGATTCAGY |
| 1290 | CL2 [Last 70% of CDR L2]-GGGGTCCCATCAAGGTTCAGY |
| 1291 | CL3 [Last 70% of CDR L2]-GGYATCCCAGCCAGGTTCAGT |
| 1292 | CL4 [Last 70% of CDR L2]-GGRGTCCCWGACAGGTTCAGT |
| 1293 | CL5 [Last 70% of CDR L2]-AGCATCCCAGCCAGGTTCAGT |
| 1294 | CL6 [Last 70% of CDR L2]-GGGGTCCCCTCGAGGTTCAGT |
| 1295 | CL7 [Last 70% of CDR L2]-GGAATCCCACCTCGATTCAGT |
| 1296 | CL8 [Last 70% of CDR L2]-GGGGTCCCTGACCGATTCAGT |
| 1297 | CL9 [Last 70% of CDR L2]-GGCATCCCAGACAGGTTCAGT |
| 1298 | CL10 [Last 70% of CDR L2]-GGGGTCTCATCGAGGTTCAGT |
| 1299 | CL11 [Last 70% of CDR L2]-GGAGTGCCAGATAGGTTCAGT |

**Table 39. Light Chain FR3 Antibody-Specific Reverse Primers (for Sub-Library 3)**

| | |
|---|---|
| 1300 | CL1' [First 70% of CDR L3]-KCAGTAATAAACCCCAACATC |
| 1301 | CL2' [First 70% of CDR L3]-ACAGTAATAYGTTGCAGCATC |
| 1302 | CL3' [First 70% of CDR L3]-ACMGTAATAAGTTGCAACATC |
| 1303 | CL4' [First 70% of CDR L3]-RCAGTAATAAGTTGCAAAATC |
| 1304 | CL5' [First 70% of CDR L3]-ACAGTAATAARCTGCAAAATC |
| 1305 | CL6' [First 70% of CDR L3]-ACARTAGTAAGTTGCAAAATC |
| 1306 | CL7' [First 70% of CDR L3]-GCAGTAATAAACTCCAAMATC |
| 1307 | CL8' [First 70% of CDR L3]-GCAGTAATAAACCCCGACATC |
| 1308 | CL9' [First 70% of CDR L3]-ACAGAAGTAATATGCAGCATC |
| 1309 | CL10' [First 70% of CDR L3]-ACAGTAATATGTTGCAATATC |
| 1310 | CL11' [First 70% of CDR L3]-ACAGTAATACACTGCAAAATC |
| 1311 | CL12' [First 70% of CDR L3]-ACAGTAATAAACTGCCACATC |

PCR is carried out with CL1 to CL11 in combination with CL1' to CL12' using sub-bank 3 as a template. This generates combinatorial sub-library 3 or a pool of oligonucleotides corresponding to sequences described in Table 3.

By way of example but not limitation, the combinatorial sub-library 4 is constructed using the Polymerase Chain Reaction (PCR) by overlap extension using the oligonucleotides in Table 40 and Table 41 (all shown in the 5' to 3' orientation, name followed by sequence) where K= G or T, M= A or C, R= A or G, S= C or G, W= A or T and Y= C or T.

**Table 40. Light Chain FR4 Antibody-Specific Forward Primers (for Sub-Library 4):**

| | |
|---|---|
| 1312 | DL1 [Last 70% of CDR L3]-TTYGGCCARGGGACCAAGSTG |
| 1313 | DL2 [Last 70% of CDR L3]-TTCGGCCAAGGGACACGACTG |
| 1314 | DL3 [Last 70% of CDR L3]-TTCGGCCCTGGGACCAAAGTG |
| 1315 | DL4 [Last 70% of CDR L3]-TTCGGCGGAGGGACCAAGGTG |

**Table 41. Light Chain FR4 Antibody-Specific Reverse Primers (for Sub-Library 4)**

| | | |
|---|---|---|
| 1316 | DL1' | TTTGATYTCCACCTTGGTCCC |
| 1317 | DL2' | TTTGATCTCCAGCTTGGTCCC |
| 1318 | DL3' | TTTGATATCCACTTTGGTCCC |
| 1319 | DL4' | TTTAATCTCCAGTCGTGTCCC |

PCR is carried out with DL1 to DL4 in combination with DL1' to DL14' using sub-bank 4 as a template. This generates combinatorial sub-library 4 or a pool of oligonucleotides corresponding to sequences described in Table 4.

By way of example but not limitation, the combinatorial sub-library 5 is constructed using the Polymerase Chain Reaction (PCR) by overlap extension using the oligonucleotides in Table 42 and Table 43 (all shown in the 5' to 3' orientation, name followed by sequence) where K= G or T, M= A or C, R= A or G, S= C or G, W= A or T and Y= C or T.

**Table 42. Heavy Chain FR1 (Kabat Definition) Antibody-Specific Forward Primers (for Sub-Library 5):**

| | | |
|---|---|---|
| 1320 | AH1 | CAGGTKCAGCTGGTGCAGTCT |
| 1321 | AH2 | GAGGTGCAGCTGKTGGAGTCT |
| 1322 | AH3 | CAGSTGCAGCTGCAGGAGTCG |
| 1323 | AH4 | CAGGTCACCTTGARGGAGTCT |
| 1324 | AH5 | CARATGCAGCTGGTGCAGTCT |
| 1325 | AH6 | GARGTGCAGCTGGTGSAGTC |
| 1326 | AH7 | CAGATCACCTTGAAGGAGTCT |
| 1327 | AH8 | CAGGTSCAGCTGGTRSAGTCT |
| 1328 | AH9 | CAGGTACAGCTGCAGCAGTCA |
| 1329 | AH10 | CAGGTGCAGCTACAGCAGTGG |

**Table 43. Heavy Chain FR1 (Kabat Definition) Antibody-Specific Reverse Primers (for Sub-Library 5):**

| | |
|---|---|
| 1330 | AHK1' [First 70% of CDR H1]-RGTGAAGGTGTATCCAGAAGC |
| 1331 | AHK2' [First 70% of CDR H1]-GCTGAGTGAGAACCCAGAGAM |
| 1332 | AHK3' [First 70% of CDR H1]-ACTGAARGTGAATCCAGAGGC |
| 1333 | AHK4' [First 70% of CDR H1]-ACTGACGGTGAAYCCAGAGGC |
| 1334 | AHK5' [First 70% of CDR H1]-GCTGAYGGAGCCACCAGAGAC |
| 1335 | AHK6' [First 70% of CDR H1]-RGTAAAGGTGWAWCCAGAAGC |
| 1336 | AHK7' [First 70% of CDR H1]-ACTRAAGGTGAAYCCAGAGGC |
| 1337 | AHK8' [First 70% of CDR H1]-GGTRAARCTGTAWCCAGAASC |
| 1338 | AHK9' [First 70% of CDR H1]-AYCAAAGGTGAATCCAGARGC |
| 1339 | AHK10'[First 70% of CDR H1]-RCTRAAGGTGAATCCAGASGC |
| 1340 | AHK12 [First 70% of CDR H1]-GGTGAAGGTGTATCCRGAWGC |
| 1341 | AHK13'[First 70% of CDR H1]-ACTGAAGGACCCACCATAGAC |
| 1342 | AHK14'[First 70% of CDR H1]-ACTGATGGAGCCACCAGAGAC |
| 1343 | AHK15'[First 70% of CDR H1]-GCTGATGGAGTAACCAGAGAC |
| 1344 | AHK16 [First 70% of CDR H1]-AGTGAGGGTGTATCCGGAAAC |
| 1345 | ARK17' [First 70% of CDR H1]-GCTGAAGGTGCCTCCAGAAGC |
| 1346 | AHK18'[First 70% of CDR H1]-AGAGACACTGTCCCCGGAGAT |

PCR is carried out with AH1 to AH10 in combination with AHK1' to AHK18' using sub-bank 5 as a template. This generates combinatorial sub-library 5 or a pool of oligonucleotides corresponding to sequences described in Table 5.

By way of example but not limitation, the combinatorial sub-library 6 is constructed using the Polymerase Chain Reaction (PCR) by overlap extension using the oligonucleotides in Table 44 and Table 45 (all shown in the 5' to 3' orientation, name followed by sequence) where K= G or T, M= A or C, R= A or G, S= C or G, W= A or T and Y= C or T.

**Table 44. Heavy Chain FR2 (Kabat Definition) Antibody-Specific Forward Primers (for Sub-Library 6):**

| | |
|---|---|
| 1347 | BHK1 [Last 70% of CDR H1]-TGGGTGCGACAGGCYCCTGGA |
| 1348 | BHK2 [Last 70% of CDR H1]-TGGGTGCGMCAGGCCCCCGGA |
| 1349 | BHK3 [Last 70% of CDR H1]-TGGATCCGTCAGCCCCCAGGR |
| 1350 | BHK4 [Last 70% of CDR H1]-TGGRTCCGCCAGGCTCCAGGG |
| 1351 | BHK5 [Last 70% of CDR H1]-TGGATCCGSCAGCCCCCAGGG |
| 1352 | BHK6 [Last 70% of CDR H1]-TGGGTCCGSCAAGCTCCAGGG |
| 1353 | BHK7 [Last 70% of CDR H1]-TGGGTCCRTCARGCTCCRGGR |
| 1354 | BHK8 [Last 70% of CDR H1]-TGGGTSCGMCARGCYACWGGA |
| 1355 | BHK9 [Last 70% of CDR H1]-TGGKTCCGCCAGGCTCCAGGS |
| 1356 | BHK10 [Last 70% of CDR H1]-TGGATCAGGCAGTCCCCATCG |
| 1357 | BHK11 [Last 70% of CDR H1]-TGGGCCCGCAAGGCTCCAGGA |
| 1358 | BHK12 [Last 70% of CDR H1]-TGGATCCGCCAGCACCCAGGG |
| 1359 | BHK13 [Last 70% of CDR H1]-TGGGTCCGCCAGGCTTCCGGG |
| 1360 | BHK14 [Last 70% of CDR H1]-TGGGTGCGCCAGATGCCCGGG |
| 1361 | BHK15 [Last 70% of CDR H1]-TGGGTGCGACAGGCTCGTGGA |
| 1362 | BHK16 [Last 70% of CDR H1]-TGGATCCGGCAGCCCGCCGGG |
| 1363 | BHK17 [Last 70% of CDR H1]-TGGGTGCCACAGGCCCCTGGA |

**Table 45. Heavy Chain FR2 (Kabat Definition) Antibody-Specific Reverse Primers (for Sub-Library 6):**

| | |
|---|---|
| 1364 | BHK1' [First 70% of CDR H2]-TCCCATCCACTCAAGCCYTTG |
| 1365 | BHK2' [First 70% of CDR H2]-TCCCATCCACTCAAGCSCTT |
| 1366 | BHK3' [First 70% of CDR H2]-WGAGACCCACTCCAGCCCCTT |
| 1367 | BHK4' [First 70% of CDR H2]-CCCAATCCACTCCAGKCCCTT |
| 1368 | BHK5' [First 70% of CDR H2]-TGAGACCCACTCCAGRCCCTT |
| 1369 | BHK6' [First 70% of CDR H2]-GCCAACCCACTCCAGCCCYTT |
| 1370 | BHK7' [First 70% of CDR H2]-KGCCACCCACTCCAGCCCCTT |
| 1371 | BHK8' [First 70% of CDR H2]-TCCCAGCCACTCAAGGCCTC |
| 1372 | BHK9' [First 70% of CDR H2]-CCCCATCCACTCCAGGCCTT |
| 1373 | BHK10' [First 70% of CDR H2]-TGARACCCACWCCAGCCCCTT |
| 1374 | BHK12' [First 70% of CDR H2]-MGAKACCCACTCCAGMCCCTT |
| 1375 | BHK13' [First 70% of CDR H2]-YCCMATCCACTCMAGCCCYTT |
| 1376 | BHK14' [First 70% of CDR H2]-TCCTATCCACTCAAGGCGTTG |
| 1377 | BHK15' [First 70% of CDR H2]-TGCAAGCCACTCCAGGGCCTT |
| 1378 | BHK16' [First 70% of CDR H2]-TGAAACATATTCCAGTCCCTT |
| 1379 | BHK17' [First 70% of CDR H2]-CGATACCCACTCCAGCCCCTT |

PCR is carried out with BHK1 to BHK17 in combination with BHK1' to BHK17' using sub-bank 6 as a template. This generates combinatorial sub-library 6 or a pool of oligonucleotides corresponding to sequences described in Table 6.

By way of example but not limitation, the combinatorial sub-library 7 is constructed using the Polymerase Chain Reaction (PCR) by overlap extension using the oligonucleotides in Table 46 and Table 47 (all shown in the 5' to 3' orientation, name followed by sequence) where K= G or T, M= A or C, R= A or G, S= C or G, W= A or T and Y= C or T.

**Table 46. Heavy Chain FR3 (Kabat Definition) Antibody-Specific Forward Primers (for Sub-Library 7):**

| | |
|---|---|
| 1380 | CHK1 [Last 70% of CDR H2]-AGAGTCACCATGACCAGGRAC |
| 1381 | CHK2 [Last 70% of CDR H2]-AGGCTCACCATCWCCAAGGAC |
| 1382 | CHK3 [Last 70% of CDR H2]-CGAGTYACCATATCAGTAGAC |
| 1383 | CHK4 [Last 70% of CDR H2]-CGATTCACCATCTCCAGRGAC |
| 1384 | CHK5 [Last 70% of CDR H2]-AGATTCACCATCTCMAGAGA |
| 1385 | CHK6 [Last 70% of CDR H2]-MGGTTCACCATCTCCAGAGA |
| 1386 | CHK7 [Last 70% of CDR H2]-CGATTCAYCATCTCCAGAGA |
| 1387 | CHK8 [Last 70% of CDR H2]-CGAGTCACCATRTCMGTAGAC |
| 1388 | CHK9 [Last 70% of CDR H2]-AGRGTCACCATKACCAGGGAC |
| 1389 | CHK10 [Last 70% of CDR H2]-CAGGTCACCATCTCAGCCGAC |
| 1390 | CHK11 [Last 70% of CDR H2]-CGAATAACCATCAACCCAGAC |
| 1391 | CHK12 [Last 70% of CDR H2]-CGGTTTGTCTTCTCCATGGAC |
| 1392 | CHK13 [Last 70% of CDR H2]-AGAGTCACCATGACCGAGGAC |
| 1393 | CHK14 [Last 70% of CDR H2]-AGAGTCACGATTACCGCGGAC |
| 1394 | CHK15 [Last 70% of CDR H2]-AGAGTCACCATGACCACAGAC |

**Table 47. Heavy Chain FR3 (Kabat Definition) Antibody-Specific Reverse Primers (for Sub-Library 7)**

| | |
|---|---|
| 1395 | CHK1' [First 70% of CDR H3]-TCTAGYACAGTAATACACGGC |
| 1396 | CHK2' [First 70% of CDR H3]-TCTCGCACAGTAATACAYGGC |
| 1397 | CHK3' [First 70% of CDR H3]-TCTYGCACAGTAATACACAGC |
| 1398 | CHK4' [First 70% of CDR H3]-TGYYGCACAGTAATACACGGC |
| 1399 | CHK5' [First 70% of CDR H3]-CCGTGCACARTAATAYGTGGC |
| 1400 | CHK6' [First 70% of CDR H3]-TCTGGGACAGTAATACACGGC |
| 1401 | CHK7' [First 70% of CDR H3]-TGTGGTACAGTAATACACGGC |
| 1402 | CHK8' [First 70% of CDR H3]-TCTCGCACAGTGATACAAGGC |
| 1403 | CHK9' [First 70% of CDR H3]-TTTTGCACAGTAATACAAGGC |
| 1404 | CHK10' [First 70% of CDR H3]-TCTTGCACAGTAATACATGGC |
| 1405 | CHK11' [First 70% of CDR H3]-GTGTGCACAGTAATATGTGGC |
| 1406 | CHK12' [First 70% of CDR H3]-TTTCGCACAGTAATATACGGC |
| 1407 | CHK13' [First 70% of CDR H3]-TCTCACACAGTAATACACAGC |

PCR is carried out with CHK1 to CHK15 in combination with CHK1' to CHK13' using sub-bank 7 as a template. This generates combinatorial sub-library 7 or a pool of oligonucleotides corresponding to sequences described in Table 7.

By way of example but not limitation, the combinatorial sub-library 8 is constructed using the Polymerase Chain Reaction (PCR) by overlap extension using the oligonucleotides in Table 48 and Table 49 (all shown in the 5' to 3' orientation, name followed by sequence) where K= G or T, M= A or C, R= A or G, S= C or G, W= A or T and Y= C or T.

**Table 48. Heavy Chain FR1 (Chothia Definition) Antibody-Specific Forward Primers (for Sub-Library 8):**

| | | |
|---|---|---|
| 1408 | AH1 | CAGGTKCAGCTGGTGCAGTCT |
| 1409 | AH2 | GAGGTGCAGCTGKTGGAGTCT |
| 1410 | AH3 | CAGSTGCAGCTGCAGGAGTCG |
| 1411 | AH4 | CAGGTCACCTTGARGGAGTCT |
| 1412 | AH5 | CARATGCAGCTGGTGCAGTCT |
| 1413 | AH6 | GARGTGCAGCTGGTGSAGTC |
| 1414 | AH7 | CAGATCACCTTGAAGGAGTCT |
| 1415 | AH8 | CAGGTSCAGCTGGTRSAGTCT |
| 1416 | AH9 | CAGGTACAGCTGCAGCAGTCA |
| 1417 | AH10 | CAGGTGCAGCTACAGCAGTGG |

**Table 49. Heavy Chain FR1 (Chothia Definition) Antibody-Specific Reverse Primers (for Sub-Library 8)**

| | | |
|---|---|---|
| 1418 | AHC1' | [First 70% of CDR H1]- RGAARCCTTGCAGGAGACCTT |
| 1419 | AHC2' | [First 70% of CDR H1]- RGAAGCCTTGCAGGAAACCTT |
| 1420 | AHC3' | [First 70% of CDR H1]- AGATGCCTTGCAGGAAACCTT |
| 1421 | AHC4' | [First 70% of CDR H1]- AGAGAMGGTGCAGGTCAGCGT |
| 1422 | AHC5' | [First 70% of CDR H1]- AGASGCTGCACAGGAGAGTCT |
| 1423 | AHC6' | [First 70% of CDR H1]- AGAGACAGTRCAGGTGAGGGA |
| 1424 | AHC7' | [First 70% of CDR H1]- AKAGACAGCGCAGGTGAGGGA |
| 1425 | AHC8' | [First 70% of CDR H1]- AGAGAAGGTGCAGGTCAGTGT |
| 1426 | AHC9' | [First 70% of CDR H1]- AGAAGCTGTACAGGAGAGTCT |
| 1427 | AHC10' | [First 70% of CDR H1]- AGAGGCTGCACAGGAGAGTTT |
| 1428 | AHC12' | [First 70% of CDR H1]- AGAACCCTTACAGGAGATCTT |
| 1429 | AHC13' | [First 70% of CDR H1]- GGAGATGGCACAGGTGAGTGA |

PCR is carried out with AH1 to AH10 in combination with AHC1' to AHC13' using sub-bank 8 as a template. This generates combinatorial sub-library 8 or a pool of oligonucleotides corresponding to sequences described in Table 8.

By way of example but not limitation, the combinatorial sub-library 9 is constructed using the Polymerase Chain Reaction (PCR) by overlap extension using the oligonucleotides in Table 50 and Table 51 (all shown in the 5' to 3' orientation, name followed by sequence) where K= G or T, M= A or C, R= A or G, S= C or G, W= A or T and Y= C or T.

**Table 50. Heavy Chain FR2 (Chothia Definition) Antibody-Specific Forward Primers (for Sub-Library 9):**

| | | |
|---|---|---|
| 1430 | BHC1 | [Last 70% of CDR H1]-TATGGYATSAGCTGGGTGCGM |
| 1431 | BHC2 | [Last 70% of CDR H1]-ATGKGTGTGAGCTGGATCCGT |
| 1432 | BHC3 | [Last 70% of CDR H1]-TACTACTGGRGCTGGATCCGS |
| 1433 | BHC4 | [Last 70% of CDR H1]-TATGCYATSAGCTGGGTSCGM |
| 1434 | BHC5 | [Last 70% of CDR H1]-TCTGCTATGCASTGGGTSCGM |
| 1435 | BHC6 | [Last 70% of CDR H1]-TATGCYATGCAYTGGGTSCGS |
| 1436 | BHC7 | [Last 70% of CDR H1]-CGCTACCTGCACTGGGTGCGA |
| 1437 | BHC8 | [Last 70% of CDR H1]-TTATCCATGCACTGGGTGCGA |
| 1438 | BHC9 | [Last 70% of CDR H1]-GCCTGGATGAGCTGGGTCCGC |
| 1439 | BHC10 | [Last 70% of CDR H1]-GCTGCTTGGAACTGGATCAGG |
| 1440 | BHC11 | [Last 70% of CDR H1]-AATGAGATGAGCTGGATCCGC |
| 1441 | BHC12 | [Last 70% of CDR H1]-AACTACATGAGCTGGGTCCGC |
| 1442 | BHC13 | [Last 70% of CDR H1]-AACTGGTGGGGCTGGATCCGG |
| 1443 | BHC14 | [Last 70% of CDR H1]-GTGGGTGTGGGCTGGATCCGT |
| 1444 | BHC15 | [Last 70% of CDR H1]-CACTACATGGACTGGGTCCGC |
| 1445 | BHC16 | [Last 70% of CDR H1]-AGTGACATGAACTGGGCCCGC |
| 1446 | BHC17 | [Last 70% of CDR H1]-AGTGACATGAACTGGGTCCAT |
| 1447 | BHC18 | [Last 70% of CDR H1]-TATACCATGCACTGGGTCCGT |
| 1448 | BHC19 | [Last 70% of CDR H1]-TATGCTATGCACTGGGTCCGC |
| 1449 | BHC20 | [Last 70% of CDR H1]-TATGCTATGAGCTGGTTCCGC |
| 1450 | BHC21 | [Last 70% of CDR H1]-TATAGCATGAACTGGGTCCGC |
| 1451 | BHC22 | [Last 70% of CDR H1]-TATGGCATGCACTGGGTCCGC |
| 1452 | BHC23 | [Last 70% of CDR H1]-TATTGGATGAGCTGGGTCCGC |
| 1453 | BHC24 | [Last 70% of CDR H1]-TACGACATGCACTGGGTCCGC |
| 1454 | BHC25 | [Last 70% of CDR H1]-TACTACATGAGCTGGATCCGC |
| 1455 | BHC26 | [Last 70% of CDR H1]-TACTGGATGCACTGGGTCCGC |
| 1456 | BHC27 | [Last 70% of CDR H1]-TACTGGATCGGCTGGGTGCGC |
| 1457 | BHC28 | [Last 70% of CDR H1]-TACTATATGCACTGGGTGCGA |
| 1458 | BHC29 | [Last 70% of CDR H1]-TATGATATCAACTGGGTGCGA |
| 1459 | BHC30 | [Last 70% of CDR H1]-TATGGTATGAATTGGGTGCCA |

**Table 51. Heavy Chain FR2 (Chothia Definition) Antibody-Specific Reverse Primers (for Sub-Library 9)**

| | |
|---|---|
| 1460 | BHC1' [First 70% of CDR H2]-AATASCWGAGACCCACTCCAG |
| 1461 | BHC2' [First 70% of CDR H2]-AATAASWGAGACCCACTCCAG |
| 1462 | BHC3' [First 70% of CDR H2]-GMTCCATCCCATCCACTCAAG |
| 1463 | BHC4' [First 70% of CDR H2]-GATACKCCCAATCCACTCCAG |
| 1464 | BHC5' [First 70% of CDR H2]-GATRTACCCAATCCACTCCAG |
| 1465 | BHC6' [First 70% of CDR H2]-AATGWGTGCAAGCCACTCCAG |
| 1466 | BHC7' [First 70% of CDR H2]-AAYACCYGAKACCCACTCCAG |
| 1467 | BHC8' [First 70% of CDR H2]-AATGKATGARACCCACTCCAG |
| 1468 | BHC9' [First 70% of CDR H2]-ARTACGGCCAACCCACTCCAG |
| 1469 | BHC10' [First 70% of CDR H2]-AAAACCTCCCATCCACTCAAG |
| 1470 | BHC12' [First 70% of CDR H2]-GATTATTCCCATCCACTCAAG |
| 1471 | BHC13' [First 70% of CDR H2]-GATCCATCCTATCCACTCAAG |
| 1472 | BHC14' [First 70% of CDR H2]-GAACCATCCCATCCACTCAAG |
| 1473 | BHC15' [First 70% of CDR H2]-GATCCCTCCCATCCACTCAAG |
| 1474 | BHC16' [First 70% of CDR H2]-CATCCATCCCATCCACTCAAG |
| 1475 | BHC17' [First 70% of CDR H2]-TGTCCTTCCCAGCCACTCAAG |
| 1476 | BHC18' [First 70% of CDR H2]-AATACGTGAGACCCACACCAG |
| 1477 | BHC19' [First 70% of CDR H2]-AATAGCTGAAACATATTCCAG |
| 1478 | BHC20' [First 70% of CDR H2]-GATTTCCCCAATCCACTCCAG |
| 1479 | BHC21' [First 70% of CDR H2]-GATGATCCCCATCCACTCCAG |
| 1480 | BHC22' [First 70% of CDR H2]-TATAACTGCCACCCACTCCAG |
| 1481 | BHC23' [First 70% of CDR H2]-AATGAAACCTACCCACTCCAG |
| 1482 | BHC24' [First 70% of CDR H2]-TATGTTGGCCACCCACTCCAG |

PCR is carried out with BHC1 to BHC30 in combination with BHC1' to BHC24' using sub-bank 9 as a template. This generates combinatorial sub-library 9 or a pool of oligonucleotides corresponding to sequences described in Table 9.

By way of example but not limitation, the combinatorial sub-library 10 is constructed using the Polymerase Chain Reaction (PCR) by overlap extension using the oligonucleotides in Table 52 and Table 53 (all shown in the 5' to 3' orientation, name followed by sequence) where K= G or T, M= A or C, R= A or G, S= C or G, W= A or T and Y= C or T.

**Table 52. Heavy Chain FR3 (Chothia Definition) Antibody-Specific Forward Primers (for Sub-Library 10):**

| | | |
|---|---|---|
| 1483 | CHC1 | [Last 70% of CDR H2]-ACCAACTACAACCCSTCCCTC |
| 1484 | CHC2 | [Last 70% of CDR H2]-ATATACTACGCAGACTCWGTG |
| 1485 | CHC3 | [Last 70% of CDR H2]-ACATACTAYGCAGACTCYGTG |
| 1486 | CHC4 | [Last 70% of CDR H2]-ACMAACTACGCACAGAARTTC |
| 1487 | CHC5 | [Last 70% of CDR H2]-ACAAACTATGCACAGAAGYT |
| 1488 | CHC6 | [Last 70% of CDR H2]-ACARGCTAYGCACAGAAGTTC |
| 1489 | CHC7 | [Last 70% of CDR H2]-AYAGGYTATGCRGACTCTGTG |
| 1490 | CHC8 | [Last 70% of CDR H2]-AAATMCTACAGCACATCTCTG |
| 1491 | CHC9 | [Last 70% of CDR H2]-AAATACTATGTGGACTCTGTG |
| 1492 | CHC10 | [Last 70% of CDR H2]-CCAACATATGCCCAGGGCTTC |
| 1493 | CHC11 | [Last 70% of CDR H2]-GCAAACTACGCACAGAAGTTC |
| 1494 | CHC12 | [Last 70% of CDR H2]-AAATACTATGCAGACTCCGTG |
| 1495 | CHC13 | [Last 70% of CDR H2]-AAGCGCTACAGCCCATCTCTG |
| 1496 | CHC14 | [Last 70% of CDR H2]-AATGATTATGCAGTATCTGTG |
| 1497 | CHC15 | [Last 70% of CDR H2]-ACCAGATACAGCCCGTCCTTC |
| 1498 | CHC16 | [Last 70% of CDR H2]-ACAGAATACGCCGCGTCTGTG |
| 1499 | CHC17 | [Last 70% of CDR H2]-ACGCACTATGCAGACTCTGTG |
| 1500 | CHC18 | [Last 70% of CDR H2]-ACGCACTATGTGGACTCCGTG |
| 1501 | CHC19 | [Last 70% of CDR H2]-ACAATCTACGCACAGAAGTTC |
| 1502 | CHC20 | [Last 70% of CDR H2]-ACAAAATATTCACAGGAGTTC |
| 1503 | CHC21 | [Last 70% of CDR H2]-ACATACTACGCAGACTCCAGG |
| 1504 | CHC22 | [Last 70% of CDR H2]-ACAAGCTACGCGGACTCCGTG |
| 1505 | CHC23 | [Last 70% of CDR H2]-ACATATTATGCAGACTCTGTG |
| 1506 | CHC24 | [Last 70% of CDR H2]-ACAGACTACGCTGCACCCGTG |
| 1507 | CHC25 | [Last 70% of CDR H2]-ACAGCATATGCTGCGTCGGTG |
| 1508 | CHC26 | [Last 70% of CDR H2]-ACATACTATCCAGGCTCCGTG |
| 1509 | CHC27 | [Last 70% of CDR H2]-ACCTACTACAACCCGTCCCTC |

**Table 53. Heavy Chain FR3 (Chothia Definition) Antibody-Specific Reverse Primers (for Sub-Library 10):**

| | | |
|---|---|---|
| 1510 | CHC1' | [First 70% of CDR H3]- TSTYGCACAGTAATACACGGC |
| 1511 | CHC2' | [First 70% of CDR H3]- TCTYGCACAGTAATACATGGC |
| 1512 | CHC3' | [First 70% of CDR H3]- TCTAGYACAGTAATACACGGC |
| 1513 | CHC4' | [First 70% of CDR H3]- CCGTGCACARTAATAYGTGGC |
| 1514 | CHC5' | [First 70% of CDR H3]- TCTYGCACAGTAATACACAGC |
| 1515 | CHC6' | [First 70% of CDR H3]- GTGTGCACAGTAATATGTGGC |
| 1516 | CHC7' | [First 70% of CDR H3]- TGCCGCACAGTAATACACGGC |
| 1517 | CHC8' | [First 70% of CDR H3]- TGTGGTACAGTAATACACGGC |
| 1518 | CHC9' | [First 70% of CDR H3]- TCTCACACAGTAATACACAGC |
| 1519 | CHC10' | [First 70% of CDR H3]- TCTCGCACAGTGATACAAGGC |
| 1520 | CHC11' | [First 70% of CDR H3]- TTTCGCACAGTAATATACGGC |
| 1521 | CHC12' | [First 70% of CDR H3]- TCTGGCACAGTAATACACGGC |
| 1522 | CHC13' | [First 70% of CDR H3]- TTTTGCACAGTAATACAAGGC |

PCR is carried out with CHC1 to CHC27 in combination with CHC1' to CHC13' using sub-bank 10 as a template. This generates combinatorial sub-library 10 or a pool of oligonucleotides corresponding to sequences described in Table 10.

By way of example but not limitation, the combinatorial sub-library 11 is constructed using the Polymerase Chain Reaction (PCR) by overlap extension using the oligonucleotides in Table 54 and Table 55 (all shown in the 5' to 3' orientation, name followed by sequence) where K= G or T, M= A or C, R= A or G, S= C or G, W= A or T and Y= C or T.

**Table 54. Heavy Chain FR4 (Kabat and Chothia Definition) Antibody-Specific Forward Primers (for Sub-Library 11):**

| | | |
|---|---|---|
| 1523 | DH1 | [Last 70% of CDR H3]-TGGGGCCARGGMACCCTGGTC |
| 1524 | DH2 | [Last 70% of CDR H3]-TGGGGSCAAGGGACMAYGGTC |
| 1525 | DH3 | [Last 70% of CDR H3]-TGGGGCCGTGGCACCCTGGTC |

**Table 55. Heavy Chain FR4 (Kabat and Chothia Definition) Antibody-Specific Reverse Primers (for Sub-Library 11)**

| | | |
|---|---|---|
| 1526 | DH1' | TGAGGAGACRGTGACCAGGGT |
| 1527 | DH2' | TGARGAGACGGTGACCRTKGT |
| 1528 | DH3' | TGAGGAGACGGTGACCAGGGT |

PCR is carried out with DH1 to DHC3 in combination with DH1' to DH3' using sub-bank 11 as a template. This generates combinatorial sub-library 11 or a pool of oligonucleotides corresponding to sequences described in Table 11.

In some embodiments, nine combinatorial sub-libraries can be constructed using direct ligation of non-human CDRs and the human frameworks of the sub-banks. For example, combinatorial sub-libraries 1', 2' and 3' are built separately by direct ligation of the non-human CDRs L1, L2 and L3 (in a single stranded or double stranded form) to sub-banks 1, 2 and 3, respectively. In one embodiment, the non-human CDRs (L1, L2 and L3) are single strand nucleic acids. In another embodiment, the non-human CDRs (L1, L2 and L3) are double strand nucleic acids. Alternatively, combinatorial sub-libraries 1', 2' and 3' can be obtained by direct ligation of the non-human CDRs (L1, L2 and L3) in a single stranded (+) form to the nucleic acid 1-46 listed in Table 1, nucleic acid 47-92 listed in Table 2, and nucleic acid 93-138 listed in Table 3, respectively.

In some embodiments, combinatorial sub-libraries 5' and 6' are built separately by direct ligation of the non-human CDRs H1 and H2 (in a single stranded or double stranded form and according to Kabat definition) to sub-banks 5 and 6, respectively. Alternatively, sub-libraries 5' and 6' can be obtained by direct ligation of the non-human CDRs H1 and H2 (according to Kabat definition and in a single stranded (+) form) to nucleic acid 1 to 44 listed in Table 5 and 45 to 88 listed in Table 6, respectively.

In some embodiments, combinatorial sub-libraries 8' and 9'are built separately by direct ligation of the non-human CDRs H1 and H2 (in a single stranded or double stranded form and according to Chothia definition) to sub-banks 8 and 9, respectively. Alternatively, sub-libraries 8' and 9' can be obtained by direct ligation of the non-human CDRs H1 and H2 (according to Chothia definition and in a single stranded (+) form) to nucleic acid 133 to 176 listed in Table 8 and 177 to 220 of Table 9, respectively.

Combinatorial sub-libraries 11' and 12' are built separately by direct ligation of the non-human CDR H3 (in a single stranded or double stranded form) to sub-bank 7 (Kabat definition) and 10 (Chothia definition), respectively. Alternatively, sub-libraries 11' and 12' can be obtained by direct ligation of non-human CDR H3 (in a single stranded (+) form) to nucleic acid 89 to 132 listed in Table 7 and 221 to 264 of Table 10, respectively.

Direct ligation of DNA fragments can be carried out according to standard protocols. It can be followed by purification/separation of the ligated products from the unligated ones.

### 2.5 Construction of Combinatorial Libraries

Combinatorial libraries are constructed by assembling together combinatorial sub-libraries of corresponding variable light chain region or variable heavy chain region. For example, combinatorial library of human kappa light chain germline frameworks (combination library 1) can be built by assembling together sub-libraries 1, 2, 3 and 4 through overlapping regions in the CDRs as described below; two combinatorial libraries of human heavy chain germline frameworks (one for Kabat definition of the CDRs, combination library 2, and one for Chothia definition of the CDRs, combination library 3) can be built by assembling together sub-libraries 5, 6, 7, 11 (Kabat definition) or sub-libraries 8, 9, 10, 11 (Chothia definition) through overlapping regions in the CDRs as described below.

In one embodiment, the construction of combinatorial library 1 is carried out using the Polymerase Chain Reaction (PCR) by overlap extension using the oligonucleotides listed in Table 56 and Table 57 (all shown in the 5' to 3' orientation, the name of the primer followed by the sequence):

**Table 56. Light Chain Forward Primers (for Combinatorial Library 1):**

| | | |
|---|---|---|
| 1529 | AL1 | GATGTTGTGATGACWCAGTCT |
| 1530 | AL2 | GACATCCAGATGAYCCAGTCT |
| 1531 | AL3 | GCCATCCAGWTGACCCAGTCT |
| 1532 | AL4 | GAAATAGTGATGAYGCAGTCT |
| 1533 | AL5 | GAAATTGTGTTGACRCAGTCT |
| 1534 | AL6 | GAKATTGTGATGACCCAGACT |
| 1535 | AL7 | GAAATTGTRMTGACWCAGTCT |
| 1536 | AL8 | GAYATYGTGATGACYCAGTCT |
| 1537 | AL9 | GAAACGACACTCACGCAGTCT |
| 1538 | AL10 | GACATCCAGTTGACCCAGTCT |
| 1539 | AL11 | AACATCCAGATGACCCAGTCT |
| 1540 | AL12 | GCCATCCGGATGACCCAGTCT |
| 1541 | AL13 | GTCATCTGGATGACCCAGTCT |

**Table 57. Light Chain Reverse Primers (for Combinatorial Library 1):**

| | | |
|---|---|---|
| 1542 | DL1' | TTTGATYTCCACCTTGGTCCC |
| 1543 | DL2' | TTTGATCTCCAGCTTGGTCCC |
| 1544 | DL3' | TTTGATATCCACTTTGGTCCC |
| 1545 | DL4' | TTTAATCTCCAGTCGTGTCCC |

PCR is carried out with AL1 to AL13 in combination with DL1' to DL4' using sub-libraries 1, 2, 3 and 4 together or a pool of oligonucleotides corresponding to sequences described in Table 1, 2, 3 and 4 as a template. This generates combinatorial library 1.

In one embodiment, the construction of combinatorial library 2 and 3 is carried out using the Polymerase Chain Reaction (PCR) by overlap extension using the oligonucleotides listed in Table 58 and Table 59 (all shown in the 5' to 3' orientation, name followed by sequence):

**Table 58. Heavy Chain Forward Primers (for Combinatorial Library 2 and 3, Kabat and Chothia Definition):**

| | | |
|---|---|---|
| 1546 | AH1 | CAGGTKCAGCTGGTGCAGTCT |
| 1547 | AH2 | GAGGTGCAGCTGKTGGAGTCT |
| 1548 | AH3 | CAGSTGCAGCTGCAGGAGTCG |
| 1549 | AH4 | CAGGTCACCTTGARGGAGTCT |
| 1550 | AH5 | CARATGCAGCTGGTGCAGTCT |
| 1551 | AH6 | GARGTGCAGCTGGTGSAGTC |
| 1552 | AH7 | CAGATCACCTTGAAGGAGTCT |
| 1553 | AH8 | CAGGTSCAGCTGGTRSAGTCT |
| 1554 | AH9 | CAGGTACAGCTGCAGCAGTCA |
| 1555 | AH10 | CAGGTGCAGCTACAGCAGTGG |

**Table 59. Heavy Chain Reverse Primers (for Combinatorial Library 2 and 3, Kabat and Chothia Definition):**

| | | |
|---|---|---|
| 1556 | DH1' | TGAGGAGACRGTGACCAGGGT |
| 1557 | DH2' | TGARGAGACGGTGACCRTKGT |
| 1558 | DH3' | TGAGGAGACGGTGACCAGGGT |

PCR is carried out with AH1 to AH10 in combination with DH1' to DH3' using sub-libraries 5, 6, 7, 11 together, or a pool of oligonucleotides corresponding to sequences described in Table 5, 6, 7 and 11, or sub-libraries 8, 9, 10, 11, or a pool of oligonucleotides corresponding to sequences described in Table 8, 9, 10 and 11, together as a template. This generates combinatorial library 2 or 3, respectively.

In another embodiment, combinatorial libraries are constructed by direct ligation. For example, combinatorial library of human kappa light chain germline frameworks (combination library 1') is built by direct sequential ligation of sub-libraries 1', 2', 3' and sub-bank 4 (or nucleic acids 139 to 143, see Table 4) together. This is followed by a Polymerase Chain Reaction step using the oligonucleotides described in Table 60 and Table 61. Two combinatorial libraries of human heavy chain germline framework regions (one for Kabat definition of the CDRs, combination library 2'; and one for Chothia definition of the CDRs, combination library 3') are built by direct sequential ligation of sub-libraries 5', 6', 11' and sub-bank 11 (Kabat definition) or of sub-libraries 8', 9', 12' and sub-bank 11 (Chothia definition) together. Alternatively, sub-bank 11 can be substituted with nucleic acids 265 to 270 (see Table 11) in the ligation reactions. This is followed by a Polymerase Chain Reaction step using the oligonucleotides described in Table 62 and Table 63.

**Table 60. Light Chain Forward Primers (for Combinatorial Library 1'):**

| | | |
|---|---|---|
| 1559 | AL1 | GATGTTGTGATGACWCAGTCT |
| 1560 | AL2 | GACATCCAGATGAYCCAGTCT |
| 1561 | AL3 | GCCATCCAGWTGACCCAGTCT |
| 1562 | AL4 | GAAATAGTGATGAYGCAGTCT |
| 1563 | AL5 | GAAATTGTGTTGACRCAGTCT |
| 1564 | AL6 | GAKATTGTGATGACCCAGACT |
| 1565 | AL7 | GAAATTGTRMTGACWCAGTCT |
| 1566 | AL8 | GAYATYGTGATGACYCAGTCT |
| 1567 | AL9 | GAAACGACACTCACGCAGTCT |
| 1568 | AL10 | GACATCCAGTTGACCCAGTCT |
| 1569 | AL11 | AACATCCAGATGACCCAGTCT |
| 1570 | AL12 | GCCATCCGGATGACCCAGTCT |
| 1571 | AL13 | GTCATCTGGATGACCCAGTCT |

**Table 61. Light Chain Reverse Primers (for Combinatorial Library 1'):**

| | | |
|---|---|---|
| 1572 | DL1' | TTTGATYTCCACCTTGGTCCC |
| 1573 | DL2' | TTTGATCTCCAGCTTGGTCCC |
| 1574 | DL3' | TTTGATATCCACTTTGGTCCC |
| 1575 | DL4' | TTTAATCTCCAGTCGTGTCCC |

PCR is carried out with AL1 to AL13 in combination with DL1' to DL4' using sub-libraries 1', 2', 3' and sub-bank 4 (or nucleic acids 139 to 143, see Table 4) previously ligated together as a template. This generates combinatorial library 1'.

**Table 62. Heavy Chain Forward Primers (for Combinatorial Library 2' and 3', Kabat and Chothia Definition):**

| | | |
|---|---|---|
| 1576 | AH1 | CAGGTKCAGCTGGTGCAGTCT |
| 1577 | AH2 | GAGGTGCAGCTGKTGGAGTCT |
| 1578 | AH3 | CAGSTGCAGCTGCAGGAGTCG |
| 1579 | AH4 | CAGGTCACCTTGARGGAGTCT |
| 1580 | AH5 | CARATGCAGCTGGTGCAGTCT |
| 1581 | AH6 | GARGTGCAGCTGGTGSAGTC |
| 1582 | AH7 | CAGATCACCTTGAAGGAGTCT |
| 1583 | AH8 | CAGGTSCAGCTGGTRSAGTCT |
| 1584 | AH9 | CAGGTACAGCTGCAGCAGTCA |
| 1585 | AH10 | CAGGTGCAGCTACAGCAGTGG |

**Table 63. Heavy Chain Reverse Primers (for Combinatorial Library 2' and 3', Kabat and Chothia Definition):**

| | | |
|---|---|---|
| 1586 | DH1' | TGAGGAGACRGTGACCAGGGT |
| 1587 | DH2' | TGARGAGACGGTGACCRTKGT |
| 1588 | DH3' | TGAGGAGACGGTGACCAGGGT |

PCR is carried out with AH1 to AH10 in combination with DH1' to DH3' using sub-libraries 5', 6', 11' and sub-bank 11 (or nucleic acids 265 to 270, see Table 11) previously ligated together or sub-libraries 8', 9', 12' and sub-bank 11 (or nucleic acids 265 to 270, see Table 11) previously ligated together as a template. This generates combinatorial library 2' or 3', respectively.

The sub-banks of framework regions, sub-banks of CDRs, combinatorial sub-libraries, and combinatorial libraries constructed in accordance with the present invention can be stored for a later use. The nucleic acids can be stored in a solution, as a dry sterilized lyophilized powder, or a water free concentrate in a hermetically sealed container. In cases where the nucleic acids are not stored in a solution, the nucleic acids can be reconstituted (*e*.*g*., with water or saline) to the appropriate concentration for a later use. The sub-banks, combinatorial sub-libraries and combinatorial libraries of the invention are preferably stored at between 2°C and 8°C in a container indicating the quantity and concentration of the nucleic acids.

### 2.6 Expression of the Combinatorial Libraries

The combinatorial libraries constructed in accordance with the present invention can be expressed using any methods know in the art, including but not limited to, bacterial expression system, mammalian expression system, and *in vitro* ribosomal display system.

In preferred embodiments, the present invention encompasses the use of phage vectors to express the combinatorial libraries. Phage vectors have particular advantages of providing a means for screening a very large population of expressed display proteins and thereby locate one or more specific clones that code for a desired binding activity.

The use of phage display vectors to express a large population of antibody molecules are well known in the art and will not be reviewed in detail herein. The method generally involves the use of a filamentous phage (phagemid) surface expression vector system for cloning and expressing antibody species of a library. See, *e.g.*, Kang et al., Proc. Natl. Acad. Sci., USA, 88:4363-4366 (1991); Barbas et al., Proc. Natl. Acad. Sci., USA, 88:7978-7982 (1991); Zebedee et al., Proc. Natl. Acad. Sci., USA, 89:3175-3179 (1992); Kang et al., Proc. Natl. Acad. Sci., USA, 88:11120-11123 (1991); Barbas et al., Proc. Natl. Acad. Sci., USA, 89:4457-4461 (1992); Gram et al., Proc. Natl. Acad. Sci., USA, 89:3576-3580 (1992); Brinkman et al., J. Immunol. Methods 182:41-50 (1995); Ames et al., J. Immunol. Methods 184:177-186 (1995); Kettleborough et al., Eur. J. Immunol. 24:952-958 (1994); Persic et al., Gene 187 9-18 (1997); Burton et al., Advances in Immunology 57:191-280 (1994); PCT application No. PCT/GB91/01134; PCT publication Nos. WO 90/02809; WO 91/10737; WO 92/01047; WO 92/18619; WO 93/11236; WO 95/15982; WO 95/20401; and U.S. Patent Nos. 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108, all of which are incorporated herein by reference in their entireties.

A preferred phagemid vector of the present invention is a recombinant DNA molecule containing a nucleotide sequence that codes for and is capable of expressing a fusion polypeptide containing, in the direction of amino- to carboxy-terminus, (1) a prokaryotic secretion signal domain, (2) a heterologous polypeptide defining an immunoglobulin heavy or light chain variable region, and (3) a filamentous phage membrane anchor domain. The vector includes DNA expression control sequences for expressing the fusion polypeptide, preferably prokaryotic control sequences.

The filamentous phage membrane anchor is preferably a domain of the cpIII or cpVIII coat protein capable of associating with the matrix of a filamentous phage particle, thereby incorporating the fusion polypeptide onto the phage surface.

Preferred membrane anchors for the vector are obtainable from filamentous phage M13, f1, fd, and equivalent filamentous phage. Preferred membrane anchor domains are found in the coat proteins encoded by gene III and gene VIII. (See Ohkawa et al., J. Biol. Chem., 256:9951-9958, 1981). The membrane anchor domain of a filamentous phage coat protein is a portion of the carboxy terminal region of the coat protein and includes a region of hydrophobic amino acid residues for spanning a lipid bilayer membrane, and a region of charged amino acid residues normally found at the cytoplasmic face of the membrane and extending away from the membrane. For detailed descriptions of the structure of filamentous phage particles, their coat proteins and particle assembly, see the reviews by Rached et al., Microbiol. Rev., 50:401-427 (1986); and Model et al., in "The Bacteriophages: Vol. 2", R. Calendar, ed. Plenum Publishing Co., pp. 375-456 (1988).

The secretion signal is a leader peptide domain of a protein that targets the protein to the periplasmic membrane of gram negative bacteria. A preferred secretion signal is a pelB secretion signal. (Better et al., Science, 240:1041-1043 (1988); Sastry et al., Proc. Natl. Acad. Sci., USA, 86:5728-5732 (1989); and Mullinax et al., Proc. Natl. Acad. Sci., USA, 87:8095-8099 (1990)). The predicted amino acid residue sequences of the secretion signal domain from two pelB gene product variants from Erwinia carotova are described in Lei et al., Nature, 331:543-546 (1988). Amino acid residue sequences for other secretion signal polypeptide domains from *E. coli* useful in this invention as described in Oliver, Escherichia coli and Salmonella Typhimurium, Neidhard, F. C. (ed.), American Society for Microbiology, Washington, D.C., 1:56-69 (1987).

DNA expression control sequences comprise a set of DNA expression signals for expressing a structural gene product and include both 5' and 3' elements, as is well known, operatively linked to the gene. The 5' control sequences define a promoter for initiating transcription and a ribosome binding site operatively linked at the 5' terminus of the upstream translatable DNA sequence. The 3' control sequences define at least one termination (stop) codon in frame with and operatively linked to the heterologous fusion polypeptide.

In preferred embodiments, the vector used in this invention includes a prokaryotic origin of replication or replicon, *i.e*., a DNA sequence having the ability to direct autonomous replication and maintenance of the recombinant DNA molecule extrachromosomally in a prokaryotic host cell, such as a bacterial host cell, transformed therewith. Such origins of replication are well known in the art. Preferred origins of replication are those that are efficient in the host organism. A preferred host cell is *E. coli.* See Sambrook et al., in "Molecular Cloning: a Laboratory Manual", 2nd edition, Cold Spring Harbor Laboratory Press, New York (1989).

In addition, those embodiments that include a prokaryotic replicon can also include a nucleic acid whose expression confers a selective advantage, such as drug resistance, to a bacterial host transformed therewith. Typical bacterial drug resistance genes are those that confer resistance to ampicillin, tetracycline, neomycin/kanamycin or chloramphenicol. Vectors typically also contain convenient restriction sites for insertion of translatable DNA sequences.

In some embodiments, the vector is capable of co-expression of two cistrons contained therein, such as a nucleotide sequence encoding a variable heavy chain region and a nucleotide sequence encoding a variable light chain region. Co-expression has been accomplished in a variety of systems and therefore need not be limited to any particular design, so long as sufficient relative amounts of the two gene products are produced to allow assembly and expression of functional heterodimer.

In some embodiments, a DNA expression vector is designed for convenient manipulation in the form of a filamentous phage particle encapsulating a genome. In this embodiment, a DNA expression vector further contains a nucleotide sequence that defines a filamentous phage origin of replication such that the vector, upon presentation of the appropriate genetic complementation, can replicate as a filamentous phage in single stranded replicative form and be packaged into filamentous phage particles. This feature provides the ability of the DNA expression vector to be packaged into phage particles for subsequent segregation of the particle, and vector contained therein, away from other particles that comprise a population of phage particles.

A filamentous phage origin of replication is a region of the phage genome, as is well known, that defines sites for initiation of replication, termination of replication and packaging of the replicative form produced by replication (see for example, Rasched et al., Microbiol. Rev., 50:401-427, 1986; and Horiuchi, J. Mol. Biol., 188:215-223, 1986). A preferred filamentous phage origin of replication for use in the present invention is an M13, f1 or fd phage origin of replication (Short et al., Nucl. Acids Res., 16:7583-7600, 1988).

The method for producing a heterodimeric immunoglobulin molecule generally involves (1) introducing a large population of display vectors each capable of expressing different putative binding sites displayed on a phagemid surface display protein to a filamentous phage particle, (3) expressing the display protein and binding site on the surface of a filamentous phage particle, and (3) isolating (screening) the surface-expressed phage particle using affinity techniques such as panning of phage particles against a preselected antigen, thereby isolating one or more species of phagemid containing a display protein containing a binding site that binds a preselected antigen.

The isolation of a particular vector capable of expressing an antibody binding site of interest involves the introduction of the dicistronic expression vector able to express the phagemid display protein into a host cell permissive for expression of filamentous phage genes and the assembly of phage particles. Typically, the host is *E. coli.* Thereafter, a helper phage genome is introduced into the host cell containing the phagemid expression vector to provide the genetic complementation necessary to allow phage particles to be assembled.

The resulting host cell is cultured to allow the introduced phage genes and display protein genes to be expressed, and for phage particles to be assembled and shed from the host cell. The shed phage particles are then harvested (collected) from the host cell culture media and screened for desirable antibody binding properties. Typically, the harvested particles are "panned" for binding with a preselected antigen. The strongly binding particles are then collected, and individual species of particles are clonally isolated and further screened for binding to the antigen. Phages which produce a binding site of desired antigen binding specificity are selected.

After phage selection, the antibody coding regions from the phage can be isolated and used to generate whole antibodies or any other desired antigen binding fragment, and expressed in any desired host, including mammalian cells, insect cells, plant cells, yeast, and bacteria, *e.g*., as described in detail below. For example, techniques to recombinantly produce Fab, Fab' and F(ab')₂ fragments can also be employed using methods known in the art such as those disclosed in International Publication No. WO 92/22324; Mullinax et al., BioTechniques 12(6):864-869 (1992); and Sawai et al., AJRI 34:26-34 (1995); and Better et al., Science 240:1041-1043 (1988) (said references incorporated by reference in their entireties). Examples of techniques which can be used to produce single-chain Fvs and antibodies include those described in U.S. Patent Nos. 4,946,778 and 5,258,498; Huston et al., Methods in Enzymology 203:46-88 (1991); Shu et al., PNAS 90:7995-7999 (1993); and Skerra et al., Science 240:1038-1040 (1988).

The invention also encompasses a host cell containing a vector or nucleotide sequence of this invention. In a specific embodiment, the host cell is *E. coli.*

In a preferred embodiment, a combinatorial library of the invention is cloned into a M13-based phage vector. This vector allows the expression of Fab fragments that contain the first constant domain of the human γ1 heavy chain and the constant domain of the human kappa (κ) light chain under the control of the lacZ promoter. This can be carried out by hybridization mutagenesis as described in Wu & An, 2003, Methods Mol. Biol., 207, 213-233; Wu, 2003, Methods Mol. Biol., 207, 197-212; and Kunkel et al., 1987, Methods Enzymol. 154, 367-382; all of which are incorporated herein by reference in their entireties. Briefly, purified minus strands corresponding to the heavy and light chains to be cloned are annealed to two regions containing each one palindromic loop. Those loops contain a unique XbaI site which allows for the selection of the vectors that contain both V_{L} and V_{H} chains fused in frame with the human kappa (κ) constant and first human γ1 constant regions, respectively (Wu & An, 2003, Methods Mol. Biol., 207, 213-233, Wu, 2003, Methods Mol. Biol., 207, 197-212). Synthesized DNA is then electroporated into XL1-blue for plaque formation on XL1-blue bacterial lawn or production of Fab fragments as described in Wu, 2003, Methods Mol. Biol., 207, 197-212.

In addition to bacterial/phage expression systems, other host-vector systems may be utilized in the present invention to express the combinatorial libraries of the present invention. These include, but are not limited to, mammalian cell systems transfected with a vector or infected with virus (*e.g*., vaccinia virus, adenovirus, etc.); insect cell systems transfected with a vector or infected with virus (*e.g*., baculovirus); microorganisms such as yeast containing yeast vectors; or bacteria transformed with DNA, plasmid DNA, or cosmid DNA. *See e.g.,* Verma et al., J Immunol Methods. 216(1-2):165-81 (1998), which is incorporated herein by reference.

The expression elements of vectors vary in their strengths and specificities. Depending on the host-vector system utilized, any one of a number of suitable transcription and translation elements may be used. In a preferred aspect, each nucleic acid of a combinatorial library of the invention is part of an expression vector that expresses the humanized heavy and/or light chain or humanized heavy and/or light variable regions in a suitable host. In particular, such nucleic acids have promoters, preferably heterologous promoters, operably linked to the antibody coding region, said promoter being inducible or constitutive, and, optionally, tissue-specific. (See Section 5.7 for more detail.) In another particular embodiment, nucleic acid molecules are used in which the antibody coding sequences and any other desired sequences are flanked by regions that promote homologous recombination at a desired site in the genome, thus providing for intrachromosomal expression of the antibody encoding nucleic acids (Koller and Smithies, 1989, Proc. Natl. Acad. Sci. USA 86:8932-8935; Zijlstra et al., 1989, Nature 342:435-438).

The combinatorial libraries can also be expressed using *in vitro* systems, such as the ribosomal display systems (see Section 5.6 for detail).

### 2.7 Selection of Humanized Antibodies

The expressed combinatorial libraries can be screened for binding to the antigen recognized by the donor antibody using any methods known in the art. In preferred embodiments, a phage display library constructed and expressed as described in section 5.4. and 5.6, respectively, is screened for binding to the antigen recognized by the donor antibody, and the phage expressing V_{H} and/or V_{L} domain with significant binding to the antigen can be isolated from a library using the conventional screening techniques (*e.g*. as described in Harlow, E., and Lane, D., 1988, supra Gherardi, E et al. 1990. J. Immunol. meth. 126 p61-68). The shed phage particles from host cells are harvested (collected) from the host cell culture media and screened for desirable antibody binding properties. Typically, the harvested particles are "panned" for binding with a preselected antigen. The strongly binding particles are then collected, and individual species of particles are clonally isolated and further screened for binding to the antigen. Phages which produce a binding site of desired antigen binding specificity are selected. Preferably, a humanized antibody of the invention has affinity of at least 1×10⁶ M⁻¹, preferably at least 1×10⁷ M⁻¹, at least 1×10⁸ M⁻¹, or at least 1×10⁹ M⁻¹ for an antigen of interest.

In a preferred embodiment, a phage library is first screened using a modified plaque lifting assay, termed capture lift. See Watkins et al., 1997, Anal. Biochem., 253:37-45. Briefly, phage infected bacteria are plated on solid agar lawns and subsequently, are overlaid with nitrocellulose filters that have been coated with a Fab-specific reagent (*e.g*., an anti-Fab antibody). Following the capture of nearly uniform quantities of phage-expressed Fab, the filters are probed with desired antigen-Ig fusion protein at a concentration substantially below the Kd value of the Fab.

In another embodiment, the combinatorial libraries are expressed and screened using *in vitro* systems, such as the ribosomal display systems *(see, e.g.,* Graddis et al., Curr Pharm Biotechnol. 3(4):285-97 (2002); Hanes and Plucthau PNAS USA 94:4937-4942 (1997); He, 1999, J. Immunol. Methods, 231:105; Jermutus et al. (1998) Current Opinion in Biotechnology, 9:534-548; each of which is incorporated herein by reference). The ribosomal display system works by translating a library of antibody or fragment thereof *in vitro* without allowing the release of either antibody (or fragment thereof) or the mRNA from the translating ribosome. This is made possible by deleting the stop codon and utilizing a ribosome stabilizing buffer system. The translated antibody (or fragment thereof) also contains a C-terminal tether polypeptide extension in order to facilitate the newly synthesized antibody or fragment thereof to emerge from the ribosomal tunnel and fold independently. The folded antibody or fragment thereof can be screened or captured with a cognate antigen. This allows the capture of the mRNA, which is subsequently enriched *in vitro.* The *E. coli* and rabbit reticulocute systems are commonly used for the ribosomal display.

Other methods know in the art, *e.g*., PROfusion™ (U.S. Patent No. 6,281,344, Phylos Inc., Lexington, MA), Covalent Display (International Publication No. WO 9837186, Actinova Ltd., Cambridge, U.K.), can also be used in accordance with the present invention.

In another embodiment, an antigen can be bound to a solid support(s), which can be provided by a petri dish, chromatography beads, magnetic beads and the like. As used herein, the term "solid support" is not limited to a specific type of solid support. Rather a large number of supports are available and are known to one skilled in the art. Solid supports include silica gels, resins, derivatized plastic films, glass beads, cotton, plastic beads, polystyrene beads, alumina gels, and polysaccharides. A suitable solid support may be selected on the basis of desired end use and suitability for various synthetic protocols. For example, for peptide synthesis, a solid support can be a resin such as p-methylbenzhydrylamine (pMBHA) resin (Peptides International, Louisville, KY), polystyrenes (*e.g*., PAM-resin obtained from Bachem Inc., Peninsula Laboratories, etc.), including chloromethylpolystyrene, hydroxymethylpolystyrene and aminomethylpolystyrene, poly (dimethylacrylamide)-grafted styrene co-divinyl-benzene (*e.g*., POLYHIPE resin, obtained from Aminotech, Canada), polyamide resin (obtained from Peninsula Laboratories), polystyrene resin grafted with polyethylene glycol (*e.g.,* TENTAGEL or ARGOGEL, Bayer, Tubingen, Germany) polydimethylacrylamide resin (obtained from Milligen/Biosearch, Califomia), or Sepharose (Pharmacia, Sweden).

The combinatorial library is then passed over the antigen, and those individual antibodies that bind are retained after washing, and optionally detected with a detection system. If samples of bound population are removed under increasingly stringent conditions, the binding affinity represented in each sample will increase. Conditions of increased stringency can be obtained, for example, by increasing the time of soaking or changing the pH of the soak solution, etc.

In another embodiment, enzyme linked immunosorbent assay (ELISA) is used to screen for an antibody with desired binding activity. ELISAs comprise preparing antigen, coating the wells of a microtiter plate with the antigen, washing away antigen that did not bind the wells, adding the antibody of interest conjugated to a detectable compound such as an enzymatic substrate (*e.g*., horseradish peroxidase or alkaline phosphatase) to the wells and incubating for a period of time, washing away unbound antibodies or nonspecifically bound antibodies, and detecting the presence of the antibodies specifically bound to the antigen coating the well. In ELISAs, the antibody of interest does not have to be conjugated to a detectable compound; instead, a second antibody (which recognizes the antibody of interest) conjugated to a detectable compound may be added to the well. Further, instead of coating the well with the antigen, the antibody may be coated to the well. In this case, the detectable molecule could be the antigen conjugated to a detectable compound such as an enzymatic substrate (*e.g*., horseradish peroxidase or alkaline phosphatase). One of skill in the art would be knowledgeable as to the parameters that can be modified to increase the signal detected as well as other variations of ELISAs known in the art. For further discussion regarding ELISAs see, *e.g*., Ausubel et al., eds, 1994, Current Protocols in Molecular Biology, Vol. I, John Wiley & Sons, Inc., New York at 11.2.1.

In another embodiment, BIAcore kinetic analysis is used to determine the binding on and off rates (Kd) of antibodies of the invention to a specific antigen. BIAcore kinetic analysis comprises analyzing the binding and dissociation of an antigen from chips with immobilized antibodies of the invention on their surface. *See* Wu et al., 1999, J. Mol. Biol., 294:151-162, which is incorporated herein by reference in its entirety. Briefly, antigen-Ig fusion protein is immobilized to a (1-ethyl-3-[3-dimethylaminopropyl]-carbodiimide hydrochloride) and N-hydroxy-succinimide-activated sensor chip CM5 by injecting antigen-Ig in sodium acetate. Antigen-Ig is immobilized at a low density to prevent rebinding of Fabs during the dissociation phase. To obtain association rate constant (Kon), the binding rate at six different Fab concentrations is determined at certain flow rate. Dissociation rate constant (Koff) are the average of six measurements obtained by analyzing the dissociation phase. Sensorgrams are analyzed with the BIAevaluation 3.0 program. Kd is calculated from Kd = Koff/Kon. Residual Fab is removed after each measurement by prolonged dissociation. In a more preferred embodiment, positive plaques are picked, replated at a lower density, and screened again.

In another embodiment, the binding affinity of an antibody (including a scFv or other molecule comprising, or alternatively consisting of, antibody fragments or variants thereof) to an antigen and the off-rate of an antibody-antigen interaction can be determined by competitive binding assays. One example of a competitive binding assay is a radioimmunoassay comprising the incubation of labeled antigen (*e.g*., ³H or ¹²¹I) with the antibody of interest in the presence of increasing amounts of unlabeled antigen, and the detection of the antibody bound to the labeled antigen. The affinity of the antibody of the present invention and the binding off-rates can be determined from the data by Scatchard plot analysis. Competition with a second antibody can also be determined using radioimmunoassays. In this case, an antigen is incubated with an antibody of the present invention conjugated to a labeled compound (*e.g*., ³H or ¹²¹I) in the presence of increasing amounts of an unlabeled second antibody.

Other assays, such as immunoassays, including but not limited to, competitive and non-competitive assay systems using techniques such as western blots, radioimmunoassays, ELISA (enzyme linked immunosorbent assay), sandwich immunoassays, immunoprecipitation assays, precipitin reactions, gel diffusion precipitin reactions, immunodiffusion assays, agglutination assays, complement-fixation assays, fluorescent immunoassays, and protein A immunoassays, can also be used to screen or further characterization of the binding specificity of a humanized antibody. Such assays are routine and well known in the art (see, *e.g*., Ausubel et al., eds, 1994, Current Protocols in Molecular Biology, Vol. 1, John Wiley & Sons, Inc., New York, which is incorporated by reference herein in its entirety). Exemplary immunoassays are described briefly below (which are not intended by way of limitation).

In a preferred embodiment, ELISA is used as a secondary screening on supernatant prepared from bacterial culture expressing Fab fragments in order to confirm the clones identified by the capture lift assay. Two ELISAs can be carried out: (1) Quantification ELISA: this can be carried out essentially as described in Wu, 2003, Methods Mol. Biol., 207, 197-212, which is incorporated herein by reference in its entirety. Briefly, concentrations can be determined by an anti-human Fab ELISA: individual wells of a 96-well Maxisorp Immunoplate are coated with 50 ng of a goat anti-human Fab antibody and then incubated with samples (supernatant-expressed Fabs) or standard (human IgG Fab). Incubation with a goat anti-human kappa horseradish peroxydase (HRP) conjugate then followed. HRP activity can be detected with TMB substrate and the reaction quenched with 0.2 M H2SO4. Plates are read at 450 nm. Clones that express detactable amount of Fab are then selected for the next part of the secondary screening. (2) Functional ELISA: briefly, a particular antigen binding activity is determined by the antigen-based ELISA: individual wells of a 96-well Maxisorp Immunoplate are coated with 50 ng of the antigen of interest, blocked with 1%BSA/0.1%Tween 20 and then incubated with samples (supernatant-expressed Fabs). Incubation with a goat anti-human kappa horseradish peroxydase (HRP) conjugate then followed. HRP activity is detected with TMB substrate and the reaction quenched with 0.2 M H2SO4. Plates are read at 450 nm.

Immunoprecipitation protocols generally comprise lysing a population of cells in a lysis buffer such as RIPA buffer (I % NP-40 or Triton X- 100, 1 % sodium deoxycholate, 0. 1 % SDS, 0. 15 M NaCl, 0.0 1 M sodium phosphate at pH 7. 2, 1% Trasylol) supplemented with protein phosphatase and/or protease inhibitors (*e.g*., EDTA, PMSF, 159 aprotinin, sodium vanadate), adding the antibody of interest to the cell lysate, incubating for a period of time (*e.g*., to 4 hours) at 40 degrees C, adding protein A and/or protein G sepharose beads to the cell lysate, incubating for about an hour or more at 40 degrees C, washing the beads in lysis buffer and re-suspending the beads in SDS/sample buffer. The ability of the antibody of interest to immunoprecipitate a particular antigen can be assessed by, *e.g*., western blot analysis. One of skill in the art would be knowledgeable as to the parameters that can be modified to increase the binding of the antibody to an antigen and decrease the background (*e.g*., pre-clearing the cell lysate with sepharose beads). For further discussion regarding immunoprecipitation protocols see, *e.g*., Ausubel et al.,.eds, 1994, Current Protocols in Molecular Biology, Vol. 1, John Wiley & Sons, Inc., New York, at 10. 16. 1.

Western blot analysis generally comprises preparing protein samples, electrophoresis of the protein samples in a polyacrylamide get (*e.g*., 8%- 20% SDS-PAGE depending on the molecular weight of the antigen), transferring the protein sample from the polyacrylamide get to a membrane such as nitrocellulose, PVDF or nylon, blocking the membrane in blocking solution (*e.g*., PBS with 3% BSA or non-fat milk), washing the membrane in washing buffer (*e.g*., PBSTween 20), blocking the membrane with primary antibody (the antibody of interest) diluted in blocking buffer, washing the membrane in washing buffer, blocking the membrane with a secondary antibody (which recognizes the primary antibody, *e.g*., an anti-human antibody) conjugated to an enzymatic substrate (*e.g*., horseradish peroxidase or alkaline phosphatase) or radioactive molecule (*e.g*., 12P or 121I) diluted in blocking buffer, washing the membrane in wash buffer, and detecting the presence of the antigen. One of skill in the art would be knowledgeable as to the parameters that can be modified to increase the signal detected and to reduce the background noise. For further discussion regarding western blot protocols see, *e.g*., Ausubel et al., eds, 1994, GinTent Protocols in Molecular Biology, Vol. 1, John Wiley & Sons, Inc., New York at 10.8.1.

A nucleic acid encoding a modified (*e.g*., humanized) antibody or fragment thereof with desired antigen binding activity can be characterized by sequencing, such as dideoxynucleotide sequencing using a ABI300 genomic analyzer. Other immunoassays, such as the two-part secondary ELISA screen described above, can be used to compare the modified (*e.g*., humanized) antibodies to each other and to the donor antibody in terms of binding to a particular antigen of interest.

### 2.8 Production and Characterization of Humanized Antibodies

Once one or more nucleic acids encoding a humanized antibody or fragment thereof with desired binding activity are selected, the nucleic acid can be recovered by standard techniques known in the art. In a preferred embodiment, the selected phage particles are recovered and used to infect fresh bacteria before recovering the desired nucleic acids.

A phage displaying a protein comprising a humanized variable region with a desired specificity or affinity can be elution from an affinity matrix by any method known in the art. In one embodiment, a ligand with better affinity to the matrix is used. In a specific embodiment, the corresponding non-humanized antibody is used. In another embodiment, an elution method which is not specific to the antigen-antibody complex is used.

The method of mild elution uses binding of the phage antibody population to biotinylated antigen and binding to streptavidin magnetic beads. Following washing to remove non-binding phage, the phage antibody is eluted and used to infect cells to give a selected phage antibody population. A disulfide bond between the biotin and the antigen molecule allows mild elution with dithiothreitol. In one embodiment, biotinylated antigen can be used in excess but at or below a concentration equivalent to the desired dissociation constant for the antigen-antibody binding. This method is advantageous for the selection of high affinity antibodies (R. E. Hawkins, S. J. Russell and G. Winter J. Mol. Biol. 226 889-896, 1992). Antibodies may also be selected for slower off rates for antigen selection as described in Hawkins et al, 1992, *supra.* The concentration of biotinylated antigen may gradually be reduced to select higher affinity phage antibodies. As an alternative, the phage antibody may be in excess over biotinylated antigen in order that phage antibodies compete for binding, in an analogous way to the competition of peptide phage to biotinylated antibody described by J. K. Scott & G. P. Smith (Science 249 386-390, 1990).

In another embodiment, a nucleotide sequence encoding amino acids constituting a recognition site for cleavage by a highly specific protease can be introduced between the foreign nucleic acid inserted, *e.g*., between a nucleic acid encoding an antibody fragment, and the sequence of the remainder of gene III. Non-limiting examples of such highly specific proteases are Factor X and thrombin. After binding of the phage to an affinity matrix and elution to remove non-specific binding phage and weak binding phage, the strongly bound phage would be removed by washing the column with protease under conditions suitable for digestion at the cleavage site. This would cleave the antibody fragment from the phage particle eluting the phage. These phage would be expected to be infective, since the only protease site should be the one specifically introduced. Strongly binding phage could then be recovered by infecting, *e.g., E. coli* TG1 cells.

An alternative procedure to the above is to take the affinity matrix which has retained the strongly bound pAb and extract the DNA, for example by boiling in SDS solution. Extracted DNA can then be used to directly transform *E. coli* host cells or alternatively the antibody encoding sequences can be amplified, for example using PCR with suitable primers, and then inserted into a vector for expression as a soluble antibody for further study or a pAb for further rounds of selection.

In another embodiment, a population of phage is bound to an affinity matrix which contains a low amount of antigen. There is competition between phage, displaying high affinity and low affinity proteins, for binding to the antigen on the matrix. Phage displaying high affinity protein is preferentially bound and low affinity protein is washed away. The high affinity protein is then recovered by elution with the ligand or by other procedures which elute the phage from the affinity matrix (International Publication No. WO92/01047 demonstrates this procedure).

The recovered nucleic acid encoding donor CDRs and humanized framework can be used by itself or can be used to construct nucleic acid for a complete antibody molecule by joining them to the constant region of the respective human template. When the nucleic acids encoding antibodies are introduced into a suitable host cell line, the transfected cells can secrete antibodies with all the desirable characteristics of monoclonal antibodies.

Once a nucleic acid encoding an antibody molecule or a heavy or light chain of an antibody, or fragment thereof (preferably, containing the heavy or light chain variable region) of the invention has been obtained, the vector for the production of the antibody molecule may be produced by recombinant DNA technology using techniques well known in the art. Thus, methods for preparing a protein by expressing a nucleic acid encoding an antibody are described herein. Methods which are well known to those skilled in the art can be used to construct expression vectors containing antibody coding sequences and appropriate transcriptional and translational control signals. These methods include, for example, *in vitro* recombinant DNA techniques, synthetic techniques, and *in vivo* genetic recombination. The invention, thus, provides replicable vectors comprising a nucleotide sequence encoding an antibody molecule of the invention, a heavy or light chain of an antibody, a heavy or light chain variable domain of an antibody or a fragment thereof, or a heavy or light chain CDR, operably linked to a promoter. In a specific embodiment, the expression of an antibody molecule of the invention, a heavy or light chain of an antibody, a heavy or light chain variable domain of an antibody or a fragment thereof, or a heavy or light chain CDR is regulated by a constitutive promoter. In another embodiment, the expression of an antibody molecule of the invention, a heavy or light chain of an antibody, a heavy or light chain variable domain of an antibody or a fragment thereof, or a heavy or light chain CDR is regulated by an inducible promoter. In another embodiment, the expression of an antibody molecule of the invention, a heavy or light chain of an antibody, a heavy or light chain variable domain of an antibody or a fragment thereof, or a heavy or light chain CDR is regulated by a tissue specific promoter. Such vectors may also include the nucleotide sequence encoding the constant region of the antibody molecule (*see, e.g.,* International Publication No. WO 86/05807; International Publication No. WO 89/01036; and U.S. Patent No. 5,122,464) and the variable domain of the antibody may be cloned into such a vector for expression of the entire heavy, the entire light chain, or both the entire heavy and light chains.

The expression vector is transferred to a host cell by conventional techniques and the transfected cells are then cultured by conventional techniques to produce an antibody of the invention. Thus, the invention includes host cells containing a polynucleotide encoding an antibody of the invention or fragments thereof, or a heavy or light chain thereof, or portion thereof, or a single chain antibody of the invention, operably linked to a heterologous promoter. In preferred embodiments for the expression of double-chained antibodies, vectors encoding both the heavy and light chains may be co-expressed in the host cell for expression of the entire immunoglobulin molecule, as detailed below.

Preferably, the cell line which is transformed to produce the altered antibody is an immortalized mammalian cell line of lymphoid origin, including but not limited to, a myeloma, hybridoma, trioma or quadroma cell line. The cell line may also comprise a normal lymphoid cell, such as a B cell, which has been immortalized by transformation with a virus, such as the Epstein Barr virus. Most preferably, the immortalized cell line is a myeloma cell line or a derivative thereof.

It is known that some immortalized lymphoid cell lines, such as myeloma cell lines, in their normal state, secrete isolated immunoglobulin light or heavy chains. If such a cell line is transformed with the recovered nucleic acid from phage library, it will not be necessary to reconstruct the recovered fragment to a constant region, provided that the normally secreted chain is complementarity to the variable domain of the immunoglobulin chain encoded by the recovered nucleic acid from the phage library.

Although the cell line used to produce the antibodies of the invention is preferably a mammalian cell line, any other suitable cell line may alternatively be used. These include, but are not limited to, microorganisms such as bacteria (*e.g., E. coli* and *B. subtilis*) transformed with recombinant bacteriophage DNA, plasmid DNA or cosmid DNA expression vectors containing antibody coding sequences; yeast (*e.g., Saccharomyces Pichia*) transformed with recombinant yeast expression vectors containing antibody coding sequences; insect cell systems infected with recombinant virus expression vectors (*e.g*., baculovirus) containing antibody coding sequences; plant cell systems infected with recombinant virus expression vectors (*e.g*., cauliflower mosaic virus, CaMV; tobacco mosaic virus, TMV) or transformed with recombinant plasmid expression vectors (*e.g*., Ti plasmid) containing antibody coding sequences; or mammalian cell systems (*e.g*., COS, CHO, BHK, 293, NS0, and 3T3 cells) harboring recombinant expression constructs containing promoters derived from the genome of mammalian cells (*e.g*., metallothionein promoter) or from mammalian viruses (*e.g*., the adenovirus late promoter; the vaccinia virus 7.5K promoter). Preferably, bacterial cells such as *Escherichia coli,* and more preferably, eukaryotic cells, especially for the expression of whole recombinant antibody molecule, are used for the expression of a recombinant antibody molecule. For example, mammalian cells such as Chinese hamster ovary cells (CHO), in conjunction with a vector such as the major intermediate early gene promoter element from human cytomegalovirus is an effective expression system for antibodies (Foecking et al., 1986, Gene 45:101; and Cockett et al., 1990, Bio/Technology 8:2).

In bacterial systems, a number of expression vectors may be advantageously selected depending upon the use intended for the antibody molecule being expressed. For example, when a large quantity of such a protein is to be produced, for the generation of pharmaceutical compositions of an antibody molecule, vectors which direct the expression of high levels of fusion protein products that are readily purified may be desirable. Such vectors include, but are not limited to, the *E. coli* expression vector pUR278 (Ruther et al., 1983, EMBO 12:1791), in which the antibody coding sequence maybe ligated individually into the vector in frame with the lac Z coding region so that a fusion protein is produced; pIN vectors (Inouye & Inouye, 1985, Nucleic Acids Res. 13:3101-3109; Van Heeke & Schuster, 1989, J. Biol. Chem. 24:5503-5509); and the like. pGEX vectors may also be used to express foreign polypeptides as fusion proteins with glutathione 5-transferase (GST). In general, such fusion proteins are soluble and can easily be purified from lysed cells by adsorption and binding to matrix glutathione agarose beads followed by elution in the presence of free glutathione. The pGEX vectors are designed to include thrombin or factor Xa protease cleavage sites so that the cloned target can be released from the GST moiety.

In an insect system, Autographa californica nuclear polyhedrosis virus (AcNPV) is used as a vector to express foreign genes. The virus grows in *Spodoptera frugiperda* cells. The antibody coding sequence may be cloned individually into non-essential regions (for example the polyhedrin gene) of the virus and placed under control of an AcNPV promoter (for example the polyhedrin promoter).

In mammalian host cells, a number of viral-based expression systems may be utilized. In cases where an adenovirus is used as an expression vector, the antibody coding sequence of interest may be ligated to an adenovirus transcription/translation control complex, e.g., the late promoter and tripartite leader sequence. This chimeric gene may then be inserted in the adenovirus genome by *in vitro* or *in vivo* recombination. Insertion in a non-essential region of the viral genome (*e.g*., region El or E3) will result in a recombinant virus that is viable and capable of expressing the antibody molecule in infected hosts (*e.g.,* see Logan & Shenk, 1984, Proc. Natl. Acad. Sci. USA 8 1:355-359). Specific initiation signals may also be required for efficient translation of inserted antibody coding sequences. These signals include the ATG initiation codon and adjacent sequences. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (*see, e.g.,* Bittner et al., 1987, Methods in Enzymol. 153:516-544).

In addition, a host cell strain may be chosen which modulates the expression of the inserted sequences, or modifies and processes the nucleic acid in a specific fashion desired. Such modifications (*e.g*., glycosylation) and processing (*e.g*., cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include but are not limited to CHO, VERY, BHK, Hela, COS, MDCK, 293, 3T3, W138, BT483, Hs578T, HTB2, BT2O and T47D, NS0 (a murine myeloma cell line that does not endogenously produce any immunoglobulin chains), CRL7O3O and HsS78Bst cells.

For long-term, high-yield production of recombinant proteins, stable expression is preferred. For example, cell lines which stably express the antibody molecule may be engineered. Rather than using expression vectors which contain viral origins of replication, host cells can be transformed with DNA controlled by appropriate expression control elements (*e.g*., promoter, enhancer, sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the antibody molecule. Such engineered cell lines may be particularly useful in screening and evaluation of compositions that interact directly or indirectly with the antibody molecule.

A number of selection systems may be used, including but not limited to, the herpes simplex virus thymidine kinase (Wigler et al., 1977, Cell 11:223), hypoxanthineguanine phosphoribosyltransferase (Szybalska & Szybalski, 1992, Proc. Natl. Acad. Sci. USA 48:202), and adenine phosphoribosyltransferase (Lowy et al., 1980, Cell 22:8-17) genes can be employed in tk-, hgprt- or aprt- cells, respectively. Also, antimetabolite resistance can be used as the basis of selection for the following genes: *dhfr,* which confers resistance to methotrexate (Wigler et al., 1980, Natl. Acad. Sci. USA 77:357; O'Hare et al., 1981, Proc. Natl. Acad. Sci. USA 78:1527); *gpt,* which confers resistance to mycophenolic acid (Mulligan & Berg, 1981, Proc. Natl. Acad. Sci. USA 78:2072); neo, which confers resistance to the aminoglycoside G-418 (Wu and Wu, 1991, Biotherapy 3:87-95; Tolstoshev, 1993, Ann. Rev. Pharmacol. Toxicol. 32:573-596; Mulligan, 1993, Science 260:926-932; and Morgan and Anderson, 1993, Ann. Rev. Biochem. 62: 191-217; May, 1993, TIB TECH 11(5):155-2 15); and *hygro,* which confers resistance to hygromycin (Santerre et al., 1984, Gene 30:147). Methods commonly known in the art of recombinant DNA technology may be routinely applied to select the desired recombinant clone, and such methods are described, for example, in Ausubel et al. (eds.), Current Protocols in Molecular Biology, John Wiley & Sons, NY (1993); Kriegler, Gene Transfer and Expression, A Laboratory Manual, Stockton Press, NY (1990); and in Chapters 12 and 13, Dracopoli et al. (eds), Current Protocols in Human Genetics, John Wiley & Sons, NY (1994); Colberre-Garapin et al., 1981, J. Mol. Biol. 150:1, which are incorporated by reference herein in their entireties.

The expression levels of an antibody molecule can be increased by vector amplification (for a review, see Bebbington and Hentschel, The use of vectors based on gene amplification for the expression of cloned genes in mammalian cells in DNA cloning, Vol.3. (Academic Press, New York, 1987)). When a marker in the vector system expressing antibody is amplifiable, increase in the level of inhibitor present in culture of host cell will increase the number of copies of the marker gene. Since the amplified region is associated with the antibody gene, production of the antibody will also increase (Crouse et al., 1983, Mol. Cell. Biol. 3:257).

The host cell may be co-transfected with two expression vectors of the invention, the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes, and is capable of expressing, both heavy and light chain polypeptides. In such situations, the light chain should be placed before the heavy chain to avoid an excess of toxic free heavy chain (Proudfoot, 1986, Nature 322:52; and Kohler, 1980, Proc. Natl. Acad. Sci. USA 77:2 197). The coding sequences for the heavy and light chains may comprise cDNA or genomic DNA.

The antibodies of the invention can also be introduced into a transgenic animal (*e.g*., transgenic mouse). *See, e.g.,* Bruggemann, Arch. Immunol. Ther. Exp. (Warsz). 49(3):203-8 (2001); Bruggemann and Neuberger, Immunol. Today 8:391-7 (1996), each of which is incorporated herein by reference. Transgene constructs or transloci can be obtained by, *e.g*., plasmid assembly, cloning in yeast artificial chromosomes, and the use of chromosome fragments. Translocus integration and maintenance in transgenic animal strains can be achieved by pronuclear DNA injection into oocytes and various transfection methods using embryonic stem cells.

For example, nucleic acids encoding humanized heavy and/or light chain or humanized heavy and/or light variable regions may be introduced randomly or by homologous recombination into mouse embryonic stem cells. The mouse heavy and light chain immunoglobulin genes may be rendered non-functional separately or simultaneously with the introduction of nucleic acids encoding humanized antibodies by homologous recombination. In particular, homozygous deletion of the JH region prevents endogenous antibody production. The modified embryonic stem cells are expanded and microinjected into blastocysts to produce chimeric mice. The chimeric mice are then be bred to produce homozygous offspring which express humanized antibodies.

Once an antibody molecule of the invention has been produced by recombinant expression, it may be purified by any method known in the art for purification of an immunoglobulin molecule, for example, by chromatography (*e.g*., ion exchange, affinity, particularly by affinity for the specific antigen after Protein A, and sizing column chromatography), centrifugation, differential solubility, or by any other standard technique for the purification of proteins. Further, the antibodies of the present invention or fragments thereof may be fused to heterologous polypeptide sequences described herein or otherwise known in the art to facilitate purification.

### 2.9 Antibody Conjugates

The present invention encompasses antibodies or fragments thereof that are conjugated or fused to one or more moieties, including but not limited to, peptides, polypeptides, proteins, fusion proteins, nucleic acid molecules, small molecules, mimetic agents, synthetic drugs, inorganic molecules, and organic molecules.

The present invention encompasses antibodies or fragments thereof that are recombinantly fused or chemically conjugated (including both covalent and non-covalent conjugations) to a heterologous protein or polypeptide (or fragment thereof, preferably to a polypepetide of at least 10, at least 20, at least 30, at least 40, at least 50, at least 60, at least 70, at least 80, at least 90 or at least 100 amino acids) to generate fusion proteins. The fusion does not necessarily need to be direct, but may occur through linker sequences. For example, antibodies may be used to target heterologous polypeptides to particular cell types, either *in vitro* or *in vivo,* by fusing or conjugating the antibodies to antibodies specific for particular cell surface receptors. Antibodies fused or conjugated to heterologous polypeptides may also be used in *in vitro* immunoassays and purification methods using methods known in the art. See *e.g*., International publication No. WO 93/21232; European Patent No. EP 439,095; Naramura et al., 1994, Immunol. Lett. 39:91-99; U.S. Patent No. 5,474,981; Gillies et al., 1992, PNAS 89:1428-1432; and Fell et al., 1991, J. Immunol. 146:2446-2452, which are incorporated by reference in their entireties.

The present invention further includes compositions comprising heterologous proteins, peptides or polypeptides fused or conjugated to antibody fragments. For example, the heterologous polypeptides may be fused or conjugated to a Fab fragment, Fd fragment, Fv fragment, F(ab)₂ fragment, a VH domain, a VL domain, a VH CDR, a VL CDR, or fragment thereof. Methods for fusing or conjugating polypeptides to antibody portions are well-known in the art. See, *e.g*., U.S. Patent Nos. 5,336,603, 5,622,929, 5,359,046, 5,349,053, 5,447,851, and 5,112,946; European Patent No.s EP 307,434 and EP 367,166; International publication Nos. WO 96/04388 and WO 91/06570; Ashkenazi et al., 1991, Proc. Natl. Acad. Sci. USA 88: 10535-10539; Zheng et al., 1995, J. Immunol. 154:5590-5600; and Vil et al., 1992, Proc. Natl. Acad. Sci. USA 89:11337- 11341 (said references incorporated by reference in their entireties).

Additional fusion proteins may be generated through the techniques of geneshuffling, motif-shuffling, exon-shuffling, and/or codon-shuffling (collectively referred to as "DNA shuffling"). DNA shuffling may be employed to alter the activities of antibodies of the invention or fragments thereof (*e.g*., antibodies or fragments thereof with higher affinities and lower dissociation rates). *See,* generally, U.S. Patent Nos. 5,605,793; 5,811,238; 5,830,721; 5,834,252; and 5,837,458, and Patten et al., 1997, Curr. Opinion Biotechnol. 8:724-33 ; Harayama, 1998, Trends Biotechnol. 16(2):76-82; Hansson, et al., 1999, J. Mol. Biol. 287:265-76; and Lorenzo and Blasco, 1998, Biotechniques 24(2):308-313 (each of these patents and publications are hereby incorporated by reference in its entirety). Antibodies or fragments thereof, or the encoded antibodies or fragments thereof, may be altered by being subj ected to random mutagenesis by error-prone PCR, random nucleotide insertion or other methods prior to recombination. One or more portions of a polynucleotide encoding an antibody or antibody fragment may be recombined with one or more components, motifs, sections, parts, domains, fragments, etc. of one or more heterologous molecules.

Moreover, the antibodies or fragments thereof can be fused to marker sequences, such as a peptide to facilitate purification. In preferred embodiments, the marker amino acid sequence is a hexa-histidine peptide, such as the tag provided in a pQE vector (QIAGEN, Inc., 9259 Eton Avenue, Chatsworth, CA, 91311), among others, many of which are commercially available. As described in Gentz et al., 1989, Proc. Natl. Acad. Sci. USA 86:821-824, for instance, hexa-histidine provides for convenient purification of the fusion protein. Other peptide tags useful for purification include, but are not limited to, the hemagglutinin "HA" tag, which corresponds to an epitope derived from the influenza hemagglutinin protein (Wilson et al., 1984, Cell 37:767) and the "flag" tag.

In other embodiments, antibodies of the present invention or fragments, analogs or derivatives thereof can be conjugated to a diagnostic or detectable agent. Such antibodies can be useful for monitoring or prognosing the development or progression of a disorder as part of a clinical testing procedure, such as determining the efficacy of a particular therapy. Such diagnosis and detection can be accomplished by coupling the antibody to detectable substances including, but not limited to various enzymes, such as but not limited to horseradish peroxidase, alkaline phosphatase, beta-galactosidase, or acetylcholinesterase; prosthetic groups, such as but not limited to streptavidinlbiotin and avidin/biotin; fluorescent materials, such as but not limited to, umbelliferone, fluorescein, fluorescein isothiocynate, rhodamine, dichlorotriazinylamine fluorescein, dansyl chloride or phycoerythrin; luminescent materials, such as but not limited to, luminol; bioluminescent materials, such as but not limited to, luciferase, luciferin, and aequorin; radioactive materials, such as but not limited to iodine (¹³¹I, ¹²⁵I, ¹²³I, ¹²¹I_{,}), carbon (¹⁴C), sulfur (³⁵S), tritium (³H), indium (¹¹⁵In, ¹¹³In, ¹¹²In, ¹¹¹In,), and technetium (⁹⁹Tc), thallium (²⁰¹Ti), gallium (⁶⁸Ga, ⁶⁷Ga), palladium (¹⁰³Pd), molybdenum (⁹⁹Mo), xenon (¹³³Xe), fluorine (¹⁸F), ¹⁵³Sm, ¹⁷⁷Lu, ¹⁵⁹Gd, ¹⁴⁹Pm, ¹⁴⁰La, ¹⁷⁵Yb, ¹⁶⁶Ho, ⁹⁰Y, ⁴⁷Sc, ¹⁸⁶Re, ¹⁸⁸Re, ¹⁴² Pr, ¹⁰⁵Rh, ⁹⁷Ru, ⁶⁸Ge, ⁵⁷Co, ⁶⁵Zn, ⁸⁵Sr, ³²P, ¹⁵³Gd, ¹⁶⁹Yb, ⁵¹Cr, ⁵⁴Mn, ⁷⁵Se, ¹¹³Sn, and ¹¹⁷Tin; positron emitting metals using various positron emission tomographies, noradioactive paramagnetic metal ions, and molecules that are radiolabelled or conjugated to specific radioisotopes.

The present invention further encompasses antibodies or fragments thereof that are conjugated to a therapeutic moiety. An antibody or fragment thereof may be conjugated to a therapeutic moiety such as a cytotoxin, *e.g*., a cytostatic or cytocidal agent, a therapeutic agent or a radioactive metal ion, *e.g*., alpha-emitters. A cytotoxin or cytotoxic agent includes any agent that is detrimental to cells. Therapeutic moieties include, but are not limited to, antimetabolites (*e.g*., methotrexate, 6-mercaptopurine, 6-thioguanine, cytarabine, 5-fluorouracil decarbazine), alkylating agents (*e.g*., mechlorethamine, thioepa chlorambucil, melphalan, carmustine (BCNU) and lomustine (CCNU), cyclothosphamide, busulfan, dibromomannitol, streptozotocin, mitomycin C, and cisdichlorodiamine platinum (II) (DDP) cisplatin), anthracyclines (*e.g*., daunorubicin (formerly daunomycin) and doxorubicin), antibiotics (*e.g*., dactinomycin (formerly actinomycin), bleomycin, mithramycin, and anthramycin (AMC)), Auristatin molecules (*e.g*., auristatin PHE, bryostatin 1, and solastatin 10; *see* Woyke et al., Antimicrob. Agents Chemother. 46:3802-8 (2002), Woyke et al., Antimicrob. Agents Chemother. 45:3580-4 (2001), Mohammad et al., Anticancer Drugs 12:735-40 (2001), Wall et al., Biochem. Biophys. Res. Commun. 266:76-80 (1999), Mohammad et al., Int. J. Oncol. 15:367-72 (1999), all of which are incorporated herein by reference), hormones (*e.g*., glucocorticoids, progestins, androgens, and estrogens), DNA-repair enzyme inhibitors (*e.g*., etoposide or topotecan), kinase inhibitors (*e.g.*, compound ST1571, imatinib mesylate (Kantarjian et al., Clin Cancer Res. 8(7):2167-76 (2002)), cytotoxic agents (*e.g*., paclitaxel, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, procaine, tetracaine, lidocaine, propranolol, and puromycin and analogs or homologs thereof) and those compounds disclosed in U.S. Pat. Nos. 6,245,759, 6,399,633, 6,383,790, 6,335,156, 6,271,242, 6,242,196, 6,218,410, 6,218,372, 6,057,300, 6,034,053, 5,985,877, 5,958,769, 5,925,376, 5,922,844, 5,911,995, 5,872,223, 5,863,904, 5,840,745, 5,728,868, 5,648,239, 5,587,459), farnesyl transferase inhibitors (*e.g*., R115777, BMS-214662, and those disclosed by, for example, U.S. Patent Nos: 6,458,935, 6,451,812, 6,440,974, 6,436,960, 6,432,959, 6,420,387, 6,414,145, 6,410,541, 6,410,539, 6,403,581, 6,399,615, 6,387,905, 6,372,747, 6,369,034, 6,362,188, 6,342,765, 6,342,487, 6,300,501, 6,268,363, 6,265,422, 6,248,756, 6,239,140, 6,232,338, 6,228,865, 6,228,856, 6,225,322, 6,218,406, 6,211,193, 6,187,786, 6,169,096, 6,159,984, 6,143,766, 6,133,303, 6,127,366, 6,124,465, 6,124,295, 6,103,723, 6,093,737, 6,090,948, 6,080,870, 6,077,853, 6,071,935, 6,066,738, 6,063,930, 6,054,466, 6,051,582, 6,051,574, and 6,040,305), topoisomerase inhibitors (*e.g*., camptothecin; irinotecan; SN-38; topotecan; 9-aminocamptothecin; GG-211 (GI 147211); DX-8951f; IST-622; rubitecan; pyrazoloacridine; XR-5000; saintopin; UCE6; UCE1022; TAN-1518A; TAN-1518B; KT6006; KT6528; ED-110; NB-506; ED-110; NB-506; and rebeccamycin); bulgarein; DNA minor groove binders such as Hoescht dye 33342 and Hoechst dye 33258; nitidine; fagaronine; epiberberine; coralyne; beta-lapachone; BC-4-1; bisphosphonates (*e.g*., alendronate, cimadronte, clodronate, tiludronate, etidronate, ibandronate, neridronate, olpandronate, risedronate, piridronate, pamidronate, zolendronate) HMG-CoA reductase inhibitors, (*e.g*., lovastatin, simvastatin, atorvastatin, pravastatin, fluvastatin, statin, cerivastatin, lescol, lupitor, rosuvastatin and atorvastatin) and pharmaceutically acceptable salts, solvates, clathrates, and prodrugs thereof. See, *e.g*., Rothenberg, M.L., Annals of Oncology 8:837-855(1997); and Moreau, P., et al., J. Med. Chem. 41:1631-1640(1998)), antisense oligonucleotides (*e.g*., those disclosed in the U.S. Pat. Nos. 6,277,832, 5,998,596, 5,885,834, 5,734,033, and 5,618,709), immunomodulators (*e.g*., antibodies and cytokines), antibodies, and adenosine deaminase inhibitors (*e.g*., Fludarabine phosphate and 2-Chlorodeoxyadenosine).

Further, an antibody or fragment thereof may be conjugated to a therapeutic moiety or drug moiety that modifies a given biological response. Therapeutic moieties or drug moieties are not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, cholera toxin, or diphtheria toxin; a protein such as tumor necrosis factor, α-interferon, β-interferon, nerve growth factor, platelet derived growth factor, tissue plasminogen activator, an apoptotic agent, *e.g*., TNF-α, TNF-β, AIM I (see, International publication No. WO 97/33899), AIM II (see, International Publication No. WO 97/34911), Fas Ligand (Takahashi et al., 1994, J. Immunol., 6:1567-1574), and VEGI (see, International publication No. WO 99/23105), a thrombotic agent or an anti-angiogenic agent, *e.g*., angiostatin, endostatin or a component of the coagulation pathway (*e.g*., tissue factor); or, a biological response modifier such as, for example, a lymphokine (*e.g*., interleukin-1 ("IL-1"), interleukin-2 ("IL-2"), interleukin-6 ("IL-6"), granulocyte macrophage colony stimulating factor ("GM-CSF"), and granulocyte colony stimulating factor ("G-CSF")), a growth factor (*e.g*., growth hormone ("GH")), or a coagulation agent (e.g., calcium, vitamin K, tissue factors, such as but not limited to, Hageman factor (factor XII), high-molecular-weight kininogen (HMWK), prekallikrein (PK), coagulation proteins-factors II (prothrombin), factor V, XIIa, VIII, XIIIa, XI, XIa,, IX, IXa, X, phospholipid. fibrinopeptides A and B from the α and β chains of fibrinogen, fibrin monomer).

Moreover, an antibody can be conjugated to therapeutic moieties such as a radioactive metal ion, such as alph-emiters such as ²¹³Bi or macrocyclic chelators useful for conjugating radiometal ions, including but not limited to, ¹³¹In, ¹³¹LU, ¹³¹Y, ¹³¹Ho, ¹³¹Sm, to polypeptides. In certain embodiments, the macrocyclic chelator is 1,4,7,10-tetraazacyclododecane-N,N',N",N"'-tetraacetic acid (DOTA) which can be attached to the antibody via a linker molecule. Such linker molecules are commonly known in the art and described in Denardo et al., 1998, Clin Cancer Res. 4(10):2483-90; Peterson et al., 1999, Bioconjug. Chem. 10(4):553-7; and Zimmerman et al., 1999, Nucl. Med. Biol. 26(8):943-50, each incorporated by reference in their entireties.

Techniques for conjugating therapeutic moieties to antibodies are well known, *see, e.g.,* Arnon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies 84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., 1982, Immunol. Rev. 62:119-58.

Alternatively, an antibody can be conjugated to a second antibody to form an antibody heteroconjugate as described by Segal in U.S. Patent No. 4,676,980, which is incorporated herein by reference in its entirety.

The therapeutic moiety or drug conjugated to an antibody or fragment thereof should be chosen to achieve the desired prophylactic or therapeutic effect(s) for a particular disorder in a subject. A clinician or other medical personnel should consider the following when deciding on which therapeutic moiety or drug to conjugate to an antibody or fragment thereof: the nature of the disease, the severity of the disease, and the condition of the subj ect.

Antibodies may also be attached to solid supports, which are particularly useful for immunoassays or purification of the target antigen. Such solid supports include, but are not limited to, glass, cellulose, polyacrylamide, nylon, polystyrene, polyvinyl chloride or polypropylene.

### 2.10 Uses of the Antibodies of the Invention

The present invention provides methods of efficiently humanizing an antibody of interest. The humanized antibodies of the present invention can be used alone or in combination with other prophylactic or therapeutic agents for treating, managing, preventing or ameliorating a disorder or one or more symptoms thereof.

The present invention provides methods for preventing, managing, treating, or ameliorating a disorder comprising administering to a subject in need thereof one or more antibodies of the invention alone or in combination with one or more therapies (*e.g*., one or more prophylactic or therapeutic agents) other than an antibody of the invention. The present invention also provides compositions comprising one or more antibodies of the invention and one or more prophylactic or therapeutic agents other than antibodies of the invention and methods of preventing, managing, treating, or ameliorating a disorder or one or more symptoms thereof utilizing said compositions. Therapeutic or prophylactic agents include, but are not limited to, small molecules, synthetic drugs, peptides, polypeptides, proteins, nucleic acids (*e.g*., DNA and RNA nucleotides including, but not limited to, antisense nucleotide sequences, triple helices, RNAi, and nucleotide sequences encoding biologically active proteins, polypeptides or peptides) antibodies, synthetic or natural inorganic molecules, mimetic agents, and synthetic or natural organic molecules.

Any therapy which is known to be useful, or which has been used or is currently being used for the prevention, management, treatment, or amelioration of a disorder or one or more symptoms thereof can be used in combination with an antibody of the invention in accordance with the invention described herein. See, *e.g.,* Gilman et al., Goodman and Gilman's: The Pharmacological Basis of Therapeutics, 10th ed., McGraw-Hill, New York, 2001; The Merck Manual of Diagnosis and Therapy, Berkow, M.D. et al. (eds.), 17th Ed., Merck Sharp & Dohme Research Laboratories, Rahway, NJ, 1999; Cecil Textbook of Medicine, 20th Ed., Bennett and Plum (eds.), W.B. Saunders, Philadelphia, 1996 for information regarding therapies (*e.g*., prophylactic or therapeutic agents) which have been or are currently being used for preventing, treating, managing, or ameliorating a disorder or one or more symptoms thereof. Examples of such agents include, but are not limited to, immunomodulatory agents, anti-inflammatory agents (*e.g*., adrenocorticoids, corticosteroids (*e.g*., beclomethasone, budesonide, flunisolide, fluticasone, triamcinolone, methlyprednisolone, prednisolone, prednisone, hydrocortisone), glucocorticoids, steroids, non-steriodal anti-inflammatory drugs (*e.g*., aspirin, ibuprofen, diclofenac, and COX-2 inhibitors), pain relievers, leukotreine antagonists (*e.g*., montelukast, methyl xanthines, zafirlukast, and zileuton), beta2-agonists (*e.g*., albuterol, biterol, fenoterol, isoetharie, metaproterenol, pirbuterol, salbutamol, terbutalin formoterol, salmeterol, and salbutamol terbutaline), anticholinergic agents (*e.g*., ipratropium bromide and oxitropium bromide), sulphasalazine, penicillamine, dapsone, antihistamines, anti-malarial agents (*e.g*., hydroxychloroquine), anti-viral agents, and antibiotics (*e.g*., dactinomycin (formerly actinomycin), bleomycin, erythomycin, penicillin, mithramycin, and anthramycin (AMC)).

The humanized antibodies of the invention can be used directly against a particular antigen. In some embodiments, antibodies of the invention belong to a subclass or isotype that is capable of mediating the lysis of cells to which the antibody binds. In a specific embodiment, the antibodies of the invention belong to a subclass or isotype that, upon complexing with cell surface proteins, activates serum complement and/or mediates antibody dependent cellular cytotoxicity (ADCC) by activating effector cells such as natural killer cells or macrophages.

The biological activities of antibodies are known to be determined, to a large extent, by the constant domains or Fc region of the antibody molecule (Uananue and Benacerraf, Textbook of Immunology, 2nd Edition, Williams & Wilkins, p. 218 (1984)). This includes their ability to activate complement and to mediate antibody-dependent cellular cytotoxicity (ADCC) as effected by leukocytes. Antibodies of different classes and subclasses differ in this respect, as do antibodies from the same subclass but different species; according to the present invention, antibodies of those classes having the desired biological activity are prepared. Preparation of these antibodies involves the selection of antibody constant domains and their incorporation in the humanized antibody by known technique. For example, mouse immunoglobulins of the IgG3 and lgG2a class are capable of activating serum complement upon binding to the target cells which express the cognate antigen, and therefore humanized antibodies which incorporate IgG3 and IgG2a effector functions are desirable for certain therapeutic applications.

In general, mouse antibodies of the IgG₂ₐ and IgG₃ subclass and occasionally IgG₁ can mediate ADCC, and antibodies of the IgG₃, IgG₂ₐ, and IgM subclasses bind and activate serum complement. Complement activation generally requires the binding of at least two IgG molecules in close proximity on the target cell. However, the binding of only one IgM molecule activates serum complement.

The ability of any particular antibody to mediate lysis of the target cell by complement activation and/or ADCC can be assayed. The cells of interest are grown and labeled in vitro; the antibody is added to the cell culture in combination with either serum complement or immune cells which may be activated by the antigen antibody complexes. Cytolysis of the target cells is detected by the release of label from the lysed cells. In fact, antibodies can be screened using the patient's own serum as a source of complement and/or immune cells. The antibody that is capable of activating complement or mediating ADCC in the *in vitro* test can then be used therapeutically in that particular patient.

Use of IgM antibodies may be preferred for certain applications, however IgG molecules by being smaller may be more able than IgM molecules to localize to certain types of infected cells.

In some embodiments, the antibodies of this invention are useful in passively immunizing patients.

The antibodies of the invention can also be used in diagnostic assays either in *vivo* or *in vitro* for detection/identification of the expression of an antigen in a subject or a biological sample (*e.g*., cells or tissues). Non-limiting examples of using an antibody, a fragment thereof, or a composition comprising an antibody or a fragment thereof in a diagnostic assay are given in U.S. Patent Nos. 6,392,020; 6,156,498; 6,136,526; 6,048,528; 6,015,555; 5,833,988; 5,811,310; 8 5,652,114; 5,604,126; 5,484,704; 5,346,687; 5,318,892; 5,273,743; 5,182,107; 5,122,447; 5,080,883; 5,057,313; 4,910,133; 4,816,402; 4,742,000; 4,724,213; 4,724,212; 4,624,846; 4,623,627; 4,618,486; 4,176,174 (all of which are incorporated herein by reference). Suitable diagnostic assays for the antigen and its antibodies depend on the particular antibody used. Non-limiting examples are an ELISA, sandwich assay, and steric inhibition assays. For *in vivo* diagnostic assays using the antibodies of the invention, the antibodies may be conjugated to a label that can be detected by imaging techniques, such as X-ray, computed tomography (CT), ultrasound, or magnetic resonance imaging (MRI). The antibodies of the invention can also be used for the affinity purification of the antigen from recombinant cell culture or natural sources.

### 2.11 Administration and Formulations

The invention provides for compositions comprising antibodies of the invention for use in diagnosing, detecting, or monitoring a disorder, in preventing, treating, managing, or ameliorating of a disorder or one or more symptoms thereof, and/or in research. In a specific embodiment, a composition comprises one or more antibodies of the invention. In another embodiment, a composition comprises one or more antibodies of the invention and one or more prophylactic or therapeutic agents other than antibodies of the invention. Preferably, the prophylactic or therapeutic agents known to be useful for or having been or currently being used in the prevention, treatment, management, or amelioration of a disorder or one or more symptoms thereof. In accordance with these embodiments, the composition may further comprise of a carrier, diluent or excipient.

The compositions of the invention include, but are not limited to, bulk drug compositions useful in the manufacture of pharmaceutical compositions (*e.g*., impure or non-sterile compositions) and pharmaceutical compositions (*i.e*., compositions that are suitable for administration to a subject or patient) which can be used in the preparation of unit dosage forms. Such compositions comprise a prophylactically or therapeutically effective amount of a prophylactic and/or therapeutic agent disclosed herein or a combination of those agents and a pharmaceutically acceptable carrier. Preferably, compositions of the invention are pharmaceutical compositions and comprise an effective amount of one or more antibodies of the invention, a pharmaceutically acceptable carrier, and, optionally, an effective amount of another prophylactic or therapeutic agent.

The pharmaceutical composition can be formulated as an oral or non-oral dosage form, for immediate or extended release. The composition can comprise inactive ingredients ordinarily used in pharmaceutical preparation such as diluents, fillers, disintegrants, sweeteners, lubricants and flavors. The pharmaceutical composition is preferably formulated for intravenous administration, either by bolus injection or sustained drip, or for release from an implanted capsule. A typical formulation for intravenous administration utilizes physiological saline as a diluent.

Fab or Fab' portions of the antibodies of the invention can also be utilized as the therapeutic active ingredient. Preparation of these antibody fragments is well-known in the art.

The composition of the present invention can also include printed matter that describes clinical indications for which the antibodies can be administered as a therapeutic agent, dosage amounts and schedules, and/or contraindications for administration of the antibodies of the invention to a patient.

The compositions of the invention include, but are not limited to, bulk drug compositions useful in the manufacture of pharmaceutical compositions (*e.g*., impure or non-sterile compositions) and pharmaceutical compositions (*i.e*., compositions that are suitable for administration to a subject or patient) which can be used in the preparation of unit dosage forms. Such compositions comprise a prophylactically or therapeutically effective amount of a prophylactic and/or therapeutic agent disclosed herein or a combination of those agents and a pharmaceutically acceptable carrier. Preferably, compositions of the invention are pharmaceutical compositions and comprise an effective amount of one or more antibodies of the invention, a pharmaceutically acceptable carrier, and, optionally, an effective amount of another prophylactic or therapeutic agent.

In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant (*e.g*., Freund's adjuvant (complete and incomplete)), excipient, or vehicle with which the therapeutic is contained in or administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like.

Generally, the ingredients of compositions of the invention are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The compositions of the invention can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

Various delivery systems are known and can be used to administer one or more antibodies of the invention or the combination of one or more antibodies of the invention and a prophylactic agent or therapeutic agent useful for preventing, managing, treating, or ameliorating a disorder or one or more symptoms thereof, *e.g*., encapsulation in liposomes, microparticles, microcapsules, recombinant cells capable of expressing the antibody or antibody fragment, receptor-mediated endocytosis (see, *e.g*., Wu and Wu, J. Biol. Chem. 262:4429-4432 (1987)), construction of a nucleic acid as part of a retroviral or other vector, etc. Methods of administering a prophylactic or therapeutic agent of the invention include, but are not limited to, parenteral administration (*e.g*., intradermal, intramuscular, intraperitoneal, intravenous and subcutaneous), epidurala administration, intratumoral administration, and mucosal adminsitration (*e.g*., intranasal and oral routes). In addition, pulmonary administration can be employed, *e.g*., by use of an inhaler or nebulizer, and formulation with an aerosolizing agent. See, *e.g*., U.S. Patent Nos. 6,019,968, 5,985, 320, 5,985,309, 5,934,272, 5,874,064, 5,855,913, 5,290,540, and 4,880,078; and PCT Publication Nos. WO 92/19244, WO 97/32572, WO 97/44013, WO 98/31346, and WO 99/66903, each of which is incorporated herein by reference their entireties. In one embodiment, an antibody of the invention, combination therapy, or a composition of the invention is administered using Alkermes AIR™ pulmonary drug delivery technology (Alkermes, Inc., Cambridge, MA). In a specific embodiment, prophylactic or therapeutic agents of the invention are administered intramuscularly, intravenously, intratumorally, orally, intranasally, pulmonary, or subcutaneously. The prophylactic or therapeutic agents may be administered by any convenient route, for example by infusion or bolus injection, by absorption through epithelial or mucocutaneous linings (*e.g*., oral mucosa, rectal and intestinal mucosa, etc.) and may be administered together with other biologically active agents. Administration can be systemic or local.

In a specific embodiment, it may be desirable to administer the prophylactic or therapeutic agents of the invention locally to the area in need of treatment; this may be achieved by, for example, and not by way of limitation, local infusion, by injection, or by means of an implant, said implant being of a porous or non-porous material, including membranes and matrices, such as sialastic membranes, polymers, fibrous matrices (*e.g*., Tissuel^{®}), or collagen matrices. In one embodiment, an effective amount of one or more antibodies of the invention antagonists is administered locally to the affected area to a subject to prevent, treat, manage, and/or ameliorate a disorder or a symptom thereof. In another embodiment, an effective amount of one or more antibodies of the invention is administered locally to the affected area in combination with an effective amount of one or more therapies (*e.g*., one or more prophylactic or therapeutic agents) other than an antibody of the invention of a subject to prevent, treat, manage, and/or ameliorate a disorder or one or more symptoms thereof.

In another embodiment, the prophylactic or therapeutic agent can be delivered in a controlled release or sustained release system. In one embodiment, a pump may be used to achieve controlled or sustained release (see Langer, *supra*; Sefton, 1987, CRC Crit. Ref. Biomed. Eng. 14:20; Buchwald et al., 1980, Surgery 88:507; Saudek et al., 1989, N. Engl. J. Med. 321:574). In another embodiment, polymeric materials can be used to achieve controlled or sustained release of the therapies of the invention (see *e.g*., Medical Applications of Controlled Release, Langer and Wise (eds.), CRC Pres., Boca Raton, Florida (1974); Controlled Drug Bioavailability, Drug Product Design and Performance, Smolen and Ball (eds.), Wiley, New York (1984); Ranger and Peppas, 1983, J., Macromol. Sci. Rev. Macromol. Chem. 23:61; see also Levy et al., 1985, Science 228:190; During et al., 1989, Ann. Neurol. 25:351; Howard et al., 1989, J. Neurosurg. 7 1:105); U.S. Patent No. 5,679,377; U.S. Patent No. 5,916,597; U.S. Patent No. 5,912,015; U.S. Patent No. 5,989,463; U.S. Patent No. 5,128,326; PCT Publication No. WO 99/15154; and PCT Publication No. WO 99/20253. Examples of polymers used in sustained release formulations include, but are not limited to, poly(2-hydroxy ethyl methacrylate), poly(methyl methacrylate), poly(acrylic acid), poly(ethylene-co-vinyl acetate), poly(methacrylic acid), polyglycolides (PLG), polyanhydrides, poly(N-vinyl pyrrolidone), poly(vinyl alcohol), polyacrylamide, poly(ethylene glycol), polylactides (PLA), poly(lactide-co-glycolides) (PLGA), and polyorthoesters. In a preferred embodiment, the polymer used in a sustained release formulation is inert, free of leachable impurities, stable on storage, sterile, and biodegradable. In yet another embodiment, a controlled or sustained release system can be placed in proximity of the prophylactic or therapeutic target, thus requiring only a fraction of the systemic dose (see, *e.g*., Goodson, in Medical Applications of Controlled Release, supra, vol. 2, pp. 115-138 (1984)).

Controlled release systems are discussed in the review by Langer (1990, Science 249:1527-1533). Any technique known to one of skill in the art can be used to produce sustained release formulations comprising one or more therapeutic agents of the invention. See, *e.g*., U.S. Patent No. 4,526,938, PCT publication WO 91/05548, PCT publication WO 96/20698,.Ning et al., 1996, "Intratumoral Radioimmunotheraphy of a Human Colon Cancer Xenograft Using a Sustained-Release Gel," Radiotherapy & Oncology 39:179-189, Song et al., 1995, "Antibody Mediated Lung Targeting of Long-Circulating Emulsions," PDA Journal of Pharmaceutical Science & Technology 50:372-397, Cleek et al., 1997, "Biodegradable Polymeric Carriers for a bFGF Antibody for Cardiovascular Application," Pro. Int'l. Symp. Control. Rel. Bioact. Mater. 24:853-854, and Lam et al., 1997, "Microencapsulation of Recombinant Humanized Monoclonal Antibody for Local Delivery," Proc. Int'l. Symp. Control Rel. Bioact. Mater. 24:759-760, each of which is incorporated herein by reference in their entireties.

In a specific embodiment, where the composition of the invention is a nucleic acid encoding a prophylactic or therapeutic agent, the nucleic acid can be administered *in vivo* to promote expression of its encoded prophylactic or therapeutic agent, by constructing it as part of an appropriate nucleic acid expression vector and administering it so that it becomes intracellular, *e.g*., by use of a retroviral vector (see U.S. Patent No. 4,980,286), or by direct injection, or by use of microparticle bombardment (*e.g*., a gene gun; Biolistic, Dupont), or coating with lipids or cell-surface receptors or transfecting agents, or by administering it in linkage to a homeobox-like peptide which is known to enter the nucleus (see, *e.g.,* Joliot et al., 1991, Proc. Natl. Acad. Sci. USA 88:1864-1868). Alternatively, a nucleic acid can be introduced intracellularly and incorporated within host cell DNA for expression by homologous recombination.

A pharmaceutical composition of the invention is formulated to be compatible with its intended route of administration. Examples of routes of administration include, but are not limited to, parenteral, *e.g*., intravenous, intradermal, subcutaneous, oral, intranasal (*e.g*., inhalation), transdermal (*e.g*., topical), transmucosal, and rectal administration. In a specific embodiment, the composition is formulated in accordance with routine procedures as a pharmaceutical composition adapted for intravenous, subcutaneous, intramuscular, oral, intranasal, or topical administration to human beings. Typically, compositions for intravenous administration are solutions in sterile isotonic aqueous buffer. Where necessary, the composition may also include a solubilizing agent and a local anesthetic such as lignocamne to ease pain at the site of the injection.

If the compositions of the invention are to be administered topically, the compositions can be formulated in the form of an ointment, cream, transdermal patch, lotion, gel, shampoo, spray, aerosol, solution, emulsion, or other form well-known to one of skill in the art. See, *e.g*., Remington's Pharmaceutical Sciences and Introduction to Pharmaceutical Dosage Forms, 19th ed., Mack Pub. Co., Easton, PA (1995). For non-sprayable topical dosage forms, viscous to semi-solid or solid forms comprising a carrier or one or more excipients compatible with topical application and having a dynamic viscosity preferably greater than water are typically employed. Suitable formulations include, without limitation, solutions, suspensions, emulsions, creams, ointments, powders, liniments, salves, and the like, which are, if desired, sterilized or mixed with auxiliary agents (*e.g*., preservatives, stabilizers, wetting agents, buffers, or salts) for influencing various properties, such as, for example, osmotic pressure. Other suitable topical dosage forms include sprayable aerosol preparations wherein the active ingredient, preferably in combination with a solid or liquid inert carrier, is packaged in a mixture with a pressurized volatile (*e.g*., a gaseous propellant, such as freon) or in a squeeze bottle. Moisturizers or humectants can also be added to pharmaceutical compositions and dosage forms if desired. Examples of such additional ingredients are well-known in the art.

If the method of the invention comprises intranasal administration of a composition, the composition can be formulated in an aerosol form, spray, mist or in the form of drops. In particular, prophylactic or therapeutic agents for use according to the present invention can be conveniently delivered in the form of an aerosol spray presentation from pressurized packs or a nebuliser, with the use of a suitable propellant (*e.g*., dichlorodifluoromethane, trichlorofluoromethane, dichlorotetrafluoroethane, carbon dioxide or other suitable gas). In the case of a pressurized aerosol the dosage unit may be determined by providing a valve to deliver a metered amount. Capsules and cartridges (composed of, *e.g*., gelatin) for use in an inhaler or insufflator may be formulated containing a powder mix of the compound and a suitable powder base such as lactose or starch.

If the method of the invention comprises oral administration, compositions can be formulated orally in the form of tablets, capsules, cachets, gelcaps, solutions, suspensions, and the like. Tablets or capsules can be prepared by conventional means with pharmaceutically acceptable excipients such as binding agents (*e.g*., pregelatinised maize starch, polyvinylpyrrolidone, or hydroxypropyl methylcellulose); fillers (*e.g*., lactose, microcrystalline cellulose, or calcium hydrogen phosphate); lubricants (*e.g*., magnesium stearate, talc, or silica); disintegrants (*e.g*., potato starch or sodium starch glycolate); or wetting agents (*e.g*., sodium lauryl sulphate). The tablets may be coated by methods well-known in the art. Liquid preparations for oral administration may take the form of, but not limited to, solutions, syrups or suspensions, or they may be presented as a dry product for constitution with water or other suitable vehicle before use. Such liquid preparations may be prepared by conventional means with pharmaceutically acceptable additives such as suspending agents (*e.g*., sorbitol syrup, cellulose derivatives, or hydrogenated edible fats); emulsifying agents (*e.g*., lecithin or acacia); non-aqueous vehicles (*e.g*., almond oil, oily esters, ethyl alcohol, or fractionated vegetable oils); and preservatives (*e.g*., methyl or propyl-p-hydroxybenzoates or sorbic acid). The preparations may also contain buffer salts, flavoring, coloring, and sweetening agents as appropriate. Preparations for oral administration may be suitably formulated for slow release, controlled release, or sustained release of a prophylactic or therapeutic agent(s).

The method of the invention may comprise pulmonary administration, *e.g*., by use of an inhaler or nebulizer, of a composition formulated with an aerosolizing agent. See, *e.g*., U.S. Patent Nos. 6,019,968, 5,985, 320, 5,985,309, 5,934,272, 5,874,064, 5,855,913, 5,290,540, and 4,880,078; and PCT Publication Nos. WO 92/19244, WO 97/32572, WO 97/44013, WO 98/31346, and WO 99/66903, each of which is incorporated herein by reference their entireties. In a specific embodiment, an antibody of the invention, combination therapy, and/or composition of the invention is administered using Alkermes AIR™ pulmonary drug delivery technology (Alkermes, Inc., Cambridge, MA).

The method of the invention may comprise administration of a composition formulated for parenteral administration by injection (*e.g*., by bolus injection or continuous infusion). Formulations for injection may be presented in unit dosage form (*e.g*., in ampoules or in multi-dose containers) with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle (*e.g*., sterile pyrogen-free water) before use.

The methods of the invention may additionally comprise of administration of compositions formulated as depot preparations. Such long acting formulations may be administered by implantation (*e.g*., subcutaneously or intramuscularly) or by intramuscular injection. Thus, for example, the compositions may be formulated with suitable polymeric or hydrophobic materials (*e.g*., as an emulsion in an acceptable oil) or ion exchange resins, or as sparingly soluble derivatives (*e.g*., as a sparingly soluble salt).

The methods of the invention encompasses administration of compositions formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

Generally, the ingredients of compositions are supplied either separately or mixed together in unit dosage form, for example, as a dry lyophilized powder or water free concentrate in a hermetically sealed container such as an ampoule or sachette indicating the quantity of active agent. Where the mode of administration is infusion, composition can be dispensed with an infusion bottle containing sterile pharmaceutical grade water or saline. Where the mode of administration is by injection, an ampoule of sterile water for injection or saline can be provided so that the ingredients may be mixed prior to administration.

In particular, the invention also provides that one or more of the prophylactic or therapeutic agents, or pharmaceutical compositions of the invention is packaged in a hermetically sealed container such as an ampoule or sachette indicating the quantity of the agent. In one embodiment, one or more of the prophylactic or therapeutic agents, or pharmaceutical compositions of the invention is supplied as a dry sterilized lyophilized powder or water free concentrate in a hermetically sealed container and can be reconstituted (*e.g*., with water or saline) to the appropriate concentration for administration to a subject. Preferably, one or more of the prophylactic or therapeutic agents or pharmaceutical compositions of the invention is supplied as a dry sterile lyophilized powder in a hermetically sealed container at a unit dosage of at least 5 mg, more preferably at least 10 mg, at least 15 mg, at least 25 mg, at least 35 mg, at least 45 mg, at least 50 mg, at least 75 mg, or at least 100 mg. The lyophilized prophylactic or therapeutic agents or pharmaceutical compositions of the invention should be stored at between 2°C and 8°C in its original container and the prophylactic or therapeutic agents, or pharmaceutical compositions of the invention should be administered within 1 week, preferably within 5 days, within 72 hours, within 48 hours, within 24 hours, within 12 hours, within 6 hours, within 5 hours, within 3 hours, or within 1 hour after being reconstituted. In an alternative embodiment, one or more of the prophylactic or therapeutic agents or pharmaceutical compositions of the invention is supplied in liquid form in a hermetically sealed container indicating the quantity and concentration of the agent. Preferably, the liquid form of the administered composition is supplied in a hermetically sealed container at least 0.25 mg/ml, more preferably at least 0.5 mg/ml, at least 1 mg/ml, at least 2.5 mg/ml, at least 5 mg/ml, at least 8 mg/ml, at least 10 mg/ml, at least 15 mg/kg, at least 25 mg/ml, at least 50 mg/ml, at least 75 mg/ml or at least 100 mg/ml. The liquid form should be stored at between 2°C and 8°C in its original container.

Generally, the ingredients of the compositions of the invention are derived from a subject that is the same species origin or species reactivity as recipient of such compositions. Thus, in a preferred embodiment, human or humanized antibodies are administered to a human patient for therapy or prophylaxis.

### 2.11.1 Gene Therapy

In a specific embodiment, nucleic acid sequences comprising nucleotide sequences encoding an antibody of the invention or another prophylactic or therapeutic agent of the invention are administered to treat, prevent, manage, or ameliorate a disorder or one or more symptoms thereof by way of gene therapy. Gene therapy refers to therapy performed by the administration to a subject of an expressed or expressible nucleic acid. In this embodiment of the invention, the nucleic acids produce their encoded antibody or prophylactic or therapeutic agent of the invention that mediates a prophylactic or therapeutic effect.

Any of the methods for gene therapy available in the art can be used according to the present invention. For general reviews of the methods of gene therapy, see Goldspiel et al., 1993, Clinical Pharmacy 12:488-505; Wu and Wu, 1991, Biotherapy 3:87-95; Tolstoshev, 1993, Ann. Rev. Pharmacol. Toxicol. 32:573-596; Mulligan, Science 260:926-932 (1993); and Morgan and Anderson, 1993, Ann. Rev. Biochem. 62:191-217; May, 1993, TIBTECH 11 (5):155-215. Methods commonly known in the art of recombinant DNA technology which can be used are described in Ausubel et al. (eds.), Current Protocols in Molecular Biology, John Wiley & Sons, NY (1993); and Kriegler, Gene Transfer and Expression, A Laboratory Manual, Stockton Press, NY (1990).

In one embodiment, the method of the invention comprises administration of a composition comprising nucleic acids encoding antibodies or another prophylactic or therapeutic agent of the invention, said nucleic acids being part of an expression vector that expresses the antibody, another prophylactic or therapeutic agent of the invention, or fragments or chimeric proteins or heavy or light chains thereof in a suitable host. In particular, such nucleic acids have promoters, preferably heterologous promoters, operably linked to the antibody coding region, said promoter being inducible or constitutive, and, optionally, tissue- specific. In another embodiment, nucleic acid molecules are used in which the coding sequences of an antibody or another prophylactic or therapeutic agent of the invention and any other desired sequences are flanked by regions that promote homologous recombination at a desired site in the genome, thus providing for intrachromosomal expression of the antibody encoding nucleic acids (Koller and Smithies, 1989, Proc. Natl. Acad. Sci. USA 86:8932-8935; Zijlstra et al., 1989, Nature 342:435-438). In specific embodiments, the expressed antibody or other prophylactic or therapeutic agent is a single chain antibody; alternatively, the nucleic acid sequences include sequences encoding both the heavy and light chains, or fragments thereof, of the antibody or another prophylactic or therapeutic agent of the invention.

Delivery of the nucleic acids into a subject may be either direct, in which case the subject is directly exposed to the nucleic acid or nucleic acid-carrying vectors, or indirect, in which case, cells are first transformed with the nucleic acids *in vitro,* then transplanted into the subject. These two approaches are known, respectively, as *in vivo* or *ex vivo* gene therapy.

In a specific embodiment, the nucleic acid sequences are directly administered *in vivo,* where it is expressed to produce the encoded product. This can be accomplished by any of numerous methods known in the art, *e.g*., by constructing them as part of an appropriate nucleic acid expression vector and administering it so that they become intracellular, *e.g*., by infection using defective or attenuated retrovirals or other viral vectors (see U.S. Patent No. 4,980,286), or by direct injection of naked DNA, or by use of microparticle bombardment (*e.g*., a gene gun; Biolistic, Dupont), or coating with lipids or cell-surface receptors or transfecting agents, encapsulation in liposomes, microparticles, or microcapsules, or by administering them in linkage to a peptide which is known to enter the nucleus, by administering it in linkage to a ligand subject to receptor-mediated endocytosis (see, *e.g*., Wu and Wu, 1987, J. Biol. Chem. 262:4429-4432) (which can be used to target cell types specifically expressing the receptors). In another embodiment, nucleic acid-ligand complexes can be formed in which the ligand comprises a fusogenic viral peptide to disrupt endosomes, allowing the nucleic acid to avoid lysosomal degradation. In yet another embodiment, the nucleic acid can be targeted *in vivo* for cell specific uptake and expression, by targeting a specific receptor (see, *e.g*., International Publication Nos. WO 92/06180; WO 92/22635; W092/20316; W093/14188; and WO 93/20221). Alternatively, the nucleic acid can be introduced intracellularly and incorporated within host cell DNA for expression, by homologous recombination (Koller and Smithies, 1989, Proc. Natl. Acad. Sci. USA 86:8932-8935; and Zijlstra et al., 1989, Nature 342:435-438).

In a specific embodiment, viral vectors that contains nucleic acid sequences encoding an antibody, another prophylactic or therapeutic agent of the invention, or fragments thereof are used. For example, a retroviral vector can be used (see Miller et al., 1993, Meth. Enzymol. 217:581-599). These retroviral vectors contain the components necessary for the correct packaging of the viral genome and integration into the host cell DNA. The nucleic acid sequences encoding the antibody or another prophylactic or therapeutic agent of the invention to be used in gene therapy are cloned into one or more vectors, which facilitates delivery of the gene into a subject. More detail about retroviral vectors can be found in Boesen et al., 1994, Biotherapy 6:291-302, which describes the use of a retroviral vector to deliver the mdr 1 gene to hematopoietic stem cells in order to make the stem cells more resistant to chemotherapy. Other references illustrating the use of retroviral vectors in gene therapy are: Clowes et al., 1994, J. Clin. Invest. 93:644-651; Klein et al., 1994, Blood 83:1467-1473; Salmons and Gunzberg, 1993, Human Gene Therapy 4:129-141; and Grossman and Wilson, 1993, Curr. Opin. in Genetics and Devel. 3:110-114.

Adenoviruses are other viral vectors that can be used in gene therapy. Adenoviruses are especially attractive vehicles for delivering genes to respiratory epithelia. Adenoviruses naturally infect respiratory epithelia where they cause a mild disease. Other targets for adenovirus-based delivery systems are liver, the central nervous system, endothelial cells, and muscle. Adenoviruses have the advantage of being capable of infecting non-dividing cells. Kozarsky and Wilson, 1993, Current Opinion in Genetics and Development 3:499-503 present a review of adenovirus-based gene therapy. Bout et al., 1994, Human Gene Therapy 5:3-10 demonstrated the use of adenovirus vectors to transfer genes to the respiratory epithelia of rhesus monkeys. Other instances of the use of adenoviruses in gene therapy can be found in Rosenfeld et al., 1991, Science 252:431-434; Rosenfeld et al., 1992, Cell 68:143-155; Mastrangeli et al., 1993, J. Clin. Invest. 91:225-234; PCT Publication W094/12649; and Wang et al., 1995, Gene Therapy 2:775-783. In a preferred embodiment, adenovirus vectors are used.

Adeno-associated virus (AAV) has also been proposed for use in gene therapy (Walsh et al., 1993, Proc. Soc. Exp. Biol. Med. 204:289-300; and U.S. Patent No. 5,436,146).

Another approach to gene therapy involves transferring a gene to cells in tissue culture by such methods as electroporation, lipofection, calcium phosphate mediated transfection, or viral infection. Usually, the method of transfer includes the transfer of a selectable marker to the cells. The cells are then placed under selection to isolate those cells that have taken up and are expressing the transferred gene. Those cells are then delivered to a subject.

In this embodiment, the nucleic acid is introduced into a cell prior to administration *in vivo* of the resulting recombinant cell. Such introduction can be carried out by any method known in the art, including but not limited to transfection, electroporation, microinjection, infection with a viral or bacteriophage vector containing the nucleic acid sequences, cell fusion, chromosome-mediated gene transfer, microcellmediated gene transfer, spheroplast fusion, etc. Numerous techniques are known in the art for the introduction of foreign genes into cells (see, *e.g*., Loeffler and Behr, 1993, Meth. Enzymol. 217:599-618; Cohen et al., 1993, Meth. Enzymol. 217:618-644; Clin. Pharma. Ther. 29:69-92 (1985)) and may be used in accordance with the present invention, provided that the necessary developmental and physiological functions of the recipient cells are not disrupted. The technique should provide for the stable transfer of the nucleic acid to the cell, so that the nucleic acid is expressible by the cell and preferably heritable and expressible by its cell progeny.

The resulting recombinant cells can be delivered to a subject by various methods known in the art. Recombinant blood cells (*e.g*., hematopoietic stem or progenitor cells) are preferably administered intravenously. The amount of cells envisioned for use depends on the several factors including, but not limited to, the desired effects and the patient state, and can be determined by one skilled in the art.

Cells into which a nucleic acid can be introduced for purposes of gene therapy encompass any desired, available cell type, and include but are not limited to epithelial cells, endothelial cells, keratinocytes, fibroblasts, muscle cells, hepatocytes; blood cells such as T lymphocytes, B lymphocytes, monocytes, macrophages, neutrophils, eosinophils, mast cells, megakaryocytes, granulocytes; various stem or progenitor cells, in particular hematopoietic stem or progenitor cells (*e.g*., as obtained from bone marrow, umbilical cord blood, peripheral blood, fetal liver, etc.). In a preferred embodiment, the cell used for gene therapy is autologous to the subject.

In an embodiment in which recombinant cells are used in gene therapy, nucleic acid sequences encoding an antibody or fragment thereof are introduced into the cells such that they are expressible by the cells or their progeny, and the recombinant cells are then administered *in vivo* for therapeutic effect. In a specific embodiment, stem or progenitor cells are used. Any stem and/or progenitor cells which can be isolated and maintained *in vitro* can potentially be used in accordance with this embodiment of the present invention (see *e.g*., PCT Publication WO 94/08598; Stemple and Anderson, 1992, Cell 7 1:973-985; Rheinwald, 1980, Meth. Cell Bio. 21A:229; and Pittelkow and Scott, 1986, Mayo Clinic Proc. 61:771).

In a specific embodiment, the nucleic acid to be introduced for purposes of gene therapy comprises an inducible promoter operably linked to the coding region, such that expression of the nucleic acid is controllable by controlling the presence or absence of the appropriate inducer of transcription.

### 2.12 Dosage and Frequency of Administration

The amount of a prophylactic or therapeutic agent or a composition of the present invention which will be effective in the treatment, management, prevention, or amelioration of a disorder or one or more symptoms thereof can be determined by standard clinical. The frequency and dosage will vary according to factors specific for each patient depending on the specific therapy or therapies (*e.g*., the specific therapeutic or prophylactic agent or agents) administered, the severity of the disorder, disease, or condition, the route of administration, as well as age, body, weight, response, the patient's immune status, and the past medical history of the patient. For example, the dosage of a prophylactic or therapeutic agent or a composition of the invention which will be effective in the treatment, prevention, management, or amelioration of a disorder or one or more symptoms thereof can be determined by administering the composition to an animal model such as, *e.g*., the animal models disclosed herein or known to those skilled in the art. In addition, *in vitro* assays may optionally be employed to help identify optimal dosage ranges. Suitable regimens can be selected by one skilled in the art by considering such factors and by following, for example, dosages reported in the literature and recommended in the *Physician's Desk Reference* (57th ed., 2003).

The toxicity and/or efficacy of the prophylactic and/or therapeutic protocols of the instant invention can be determined by standard pharmaceutical procedures in cell cultures or experimental animals, *e.g*., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Therapies that exhibit large therapeutic indices are preferred. While therapies that exhibit toxic side effects may be used, care should be taken to design a delivery system that targets such agents to the site of affected tissue in order to minimize potential damage to uninfected cells and, thereby, reduce side effects.

The data obtained from the cell culture assays and animal studies can be used in formulating a range of dosage of the prophylactic and/or therapeutic agents for use in humans. The dosage of such agents lies preferably within a range of circulating concentrations that include the ED₅₀ with little or no toxicity. The dosage may vary within this range depending upon the dosage form employed and the route of administration utilized. For any therapy used in the method of the invention, the therapeutically effective dose can be estimated initially from cell culture assays. A dose may be formulated in animal models to achieve a circulating plasma concentration range that includes the IC₅₀ (*i.e.,* the concentration of the test compound that achieves a half-maximal inhibition of symptoms) as determined in cell culture. Such information can be used to more accurately determine useful doses in humans. Levels in plasma may be measured, for example, by high performance liquid chromatography.

For peptides, polypeptides, proteins, fusion proteins, and antibodies, the dosage administered to a patient is typically 0.01 mg/kg to 100 mg/kg of the patient's body weight. Preferably, the dosage administered to a patient is between 0.1 mg/kg and 20 mg/kg of the patient's body weight, more preferably 1 mg/kg to 10 mg/kg of the patient's body weight. Generally, human and humanized antibodies have a longer half-life within the human body than antibodies from other species due to the immune response to the foreign polypeptides. Thus, lower dosages of human antibodies and less frequent administration is often possible.

Exemplary doses of a small molecule include milligram or microgram amounts of the small molecule per kilogram of subject or sample weight (*e.g*., about 1 microgram per kilogram to about 500 milligrams per kilogram, about 100 micrograms per kilogram to about 5 milligrams per kilogram, or about 1 microgram per kilogram to about 50 micrograms per kilogram).

The dosages of prophylactic or therapeutically agents are described in the Physicians' Desk Reference (56th ed., 2002).

### 2.13 Biological Assays

Antibodies of the present invention or fragments thereof may be characterized in a variety of ways well-known to one of skill in the art. In particular, antibodies of the invention or fragments thereof may be assayed for the ability to immunospecifically bind to an antigen. Such an assay may be performed in solution (*e.g*., Houghten, 1992, Bio/Techniques 13:412 421), on beads (Lam, 1991, Nature 354:82 84), on chips (Fodor, 1993, Nature 364:555 556), on bacteria (U.S. Patent No. 5,223,409), on spores (U.S. Patent Nos. 5,571,698; 5,403,484; and 5,223,409), on plasmids (Cull et al., 1992, Proc. Natl. Acad. Sci. USA 89:1865 1869) or on phage (Scott and Smith, 1990, Science 249:386 390; Cwirla et al., 1990, Proc. Natl. Acad. Sci. USA 87:6378 6382; and Felici, 1991, J. Mol. Biol. 222:301 310) (each of these references is incorporated herein in its entirety by reference). Antibodies or fragments thereof that have been identified can then be assayed for specificity and affinity.

The antibodies of the invention or fragments thereof may be assayed for immunospecific binding to a specific antigen and cross-reactivity with other antigens by any method known in the art. Immunoassays which can be used to analyze immunospecific binding and cross-reactivity include, but are not limited to, competitive and non-competitive assay systems using techniques such as western blots, radioimmunoassays, ELISA (enzyme linked immunosorbent assay), "sandwich" immmoassays, immunoprecipitation assays, precipitin reactions, gel diffusion precipitin reactions, immunodiffusion assays, agglutination assays, complement-fixation assays, immunoradiometric assays, fluorescent immunoassays, protein A immunoassays, to name but a few. Such assays are routine and well-known in the art (see, *e.g*., Ausubel et al., eds., 1994, Current Protocols in Molecular Biology, Vol. 1, John Wiley & Sons, Inc., New York, which is incorporated by reference herein in its entirety). Exemplary immunoassays are described briefly in Section 5.6.

The antibodies of the invention or fragments thereof can also be assayed for their ability to inhibit the binding of an antigen to its host cell receptor using techniques known to those of skill in the art. For example, cells expressing a receptor can be contacted with a ligand for that receptor in the presence or absence of an antibody or fragment thereof that is an antagonist of the ligand and the ability of the antibody or fragment thereof to inhibit the ligand's binding can measured by, for example, flow cytometry or a scintillation assay. The ligand or the antibody or antibody fragment can be labeled with a detectable compound such as a radioactive label (*e.g*., ³²P, ³²S, and ¹²⁵I) or a fluorescent label (*e.g.,* fluorescein isothiocyanate, rhodamine, phycoerythrin, phycocyanin, allophycocyanin, o-phthaldehyde and fluorescamine) to enable detection of an interaction between the ligand and its receptor. Alternatively, the ability of antibodies or fragments thereof to inhibit a ligand from binding to its receptor can be determined in cell-free assays. For example, a ligand can be contacted with an antibody or fragment thereof that is an antagonist of the ligand and the ability of the antibody or antibody fragment to inhibit the ligand from binding to its receptor can be determined. Preferably, the antibody or the antibody fragment that is an antagonist of the ligand is immobilized on a solid support and the ligand is labeled with a detectable compound. Alternatively, the ligand is immobilized on a solid support and the antibody or fragment thereof is labeled with a detectable compound. A ligand may be partially or completely purified (*e.g*., partially or completely free of other polypeptides) or part of a cell lysate. Alternatively, a ligand can be biotinylated using techniques well known to those of skill in the art (*e.g*., biotinylation kit, Pierce Chemicals; Rockford, IL).

An antibody or a fragment thereof constructed and/or identified in accordance with the present invention can be tested *in vitro* and/or *in vivo* for its ability to modulate the biological activity of cells. Such ability can be assessed by, *e.g*., detecting the expression of antigens and genes; detecting the proliferation of cells; detecting the activation of signaling molecules (*e.g*., signal transduction factors and kinases); detecting the effector function of cells; or detecting the differentiation of cells. Techniques known to those of skill in the art can be used for measuring these activities. For example, cellular proliferation can be assayed by ³H-thymidine incorporation assays and trypan blue cell counts. Antigen expression can be assayed, for example, by immunoassays including, but are not limited to, competitive and non-competitive assay systems using techniques such as western blots, immunohistochemistry radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, protein A immunoassays, and FACS analysis. The activation of signaling molecules can be assayed, for example, by kinase assays and electrophoretic shift assays (EMSAs).

The antibodies, fragments thereof, or compositions of the invention are preferably tested *in vitro* and then *in vivo* for the desired therapeutic or prophylactic activity prior to use in humans. For example, assays which can be used to determine whether administration of a specific pharmaceutical composition is indicated include cell culture assays in which a patient tissue sample is grown in culture and exposed to, or otherwise contacted with, a pharmaceutical composition, and the effect of such composition upon the tissue sample is observed. The tissue sample can be obtained by biopsy from the patient. This test allows the identification of the therapeutically most effective therapy (*e.g*., prophylactic or therapeutic agent) for each individual patient. In various specific embodiments, *in vitro* assays can be carried out with representative cells of cell types involved a particular disorder to determine if a pharmaceutical composition of the invention has a desired effect upon such cell types. For example, *in vitro* asssay can be carried out with cell lines.

The effect of an antibody, a fragment thereof, or a composition of the invention on peripheral blood lymphocyte counts can be monitored/assessed using standard techniques known to one of skill in the art. Peripheral blood lymphocytes counts in a subject can be determined by, *e.g*., obtaining a sample of peripheral blood from said subject, separating the lymphocytes from other components of peripheral blood such as plasma using,*e.g*., Ficoll-Hypaque (Pharmacia) gradient centrifugation, and counting the lymphocytes using trypan blue. Peripheral blood T-cell counts in subject can be determined by, *e.g*., separating the lymphocytes from other components of peripheral blood such as plasma using, *e.g*., a use of Ficoll-Hypaque (Pharmacia) gradient centrifugation, labeling the T-cells with an antibody directed to a T-cell antigen which is conjugated to FITC or phycoerythrin, and measuring the number of T-cells by FACS.

The antibodies, fragments, or compositions of the invention used to treat, manage, prevent, or ameliorate a viral infection or one or more symptoms thereof can be tested for their ability to inhibit viral replication or reduce viral load in *in vitro* assays. For example, viral replication can be assayed by a plaque assay such as described, *e.g*., by Johnson et al., 1997, Journal of Infectious Diseases 176:1215-1224 176:1215-1224. The antibodies or fragments thereof administered according to the methods of the invention can also be assayed for their ability to inhibit or downregulate the expression of viral polypeptides. Techniques known to those of skill in the art, including, but not limited to, western blot analysis, northern blot analysis, and RT-PCR can be used to measure the expression of viral polypeptides.

The antibodies, fragments, or compositions of the invention used to treat, manage, prevent, or ameliorate a bacterial infection or one or more symptoms thereof can be tested in *in vitro* assays that are well-known in the art. *In vitro* assays known in the art can also be used to test the existence or development of resistance of bacteria to a therapy. Such *in vitro* assays are described in Gales et al., 2002, Diag. Nicrobiol. Infect. Dis. 44(3):301-311; Hicks et al., 2002, Clin. Microbiol. Infect. 8(11): 753-757; and Nicholson et al., 2002, Diagn. Microbiol. Infect. Dis. 44(1): 101-107.

The antibodies, fragments, or compositions of the invention used to treat, manage, prevent, or ameliorate a fungal infection or one or more symptoms thereof can be tested for anti-fungal activity against different species of fungus. Any of the standard antifungal assays well-known in the art can be used to assess the anti-fungal activity of a therapy. The anti-fungal effect on different species of fungus can be tested. The tests recommended by the National Committee for Clinical Laboratories (NCCLS) (See National Committee for Clinical Laboratories Standards. 1995, Proposed Standard M27T. Villanova, Pa., all of which is incorporated herein by reference in its entirety) and other methods known to those skilled in the art (Pfaller et al., 1993, Infectious Dis. Clin. N. Am. 7: 435-444) can be used to assess the anti-fungal effect of a therapy. The antifungal properties of a therapy may also be determined from a fungal lysis assay, as well as by other methods, including, inter alia, growth inhibition assays, fluorescence-based fungal viability assays, flow cytometry analyses, and other standard assays known to those skilled in the art.

For example, the anti-fungal activity of a therapy can be tested using macrodilution methods and/or microdilution methods using protocols well-known to those skilled in the art (see, e.g., Clancy et al., 1997 Journal of Clinical Microbiology, 35(11): 2878-82; Ryder et al., 1998, Antimicrobial Agents and Chemotherapy, 42(5): 1057-61; U.S. 5,521,153; U.S. 5,883,120, U.S. 5,521,169, all of which are incorporated by reference in their entirety). Briefly, a fungal strain is cultured in an appropriate liquid media, and grown at an appropriate temperature, depending on the particular fungal strain used for a determined amount of time, which is also depends on the particular fungal strain used. An innoculum is then prepared photometrically and the turbidity of the suspension is matched to that of a standard, e.g., a McFarland standard. The effect of a therapy on the turbidity of the inoculum is determined visually or spectrophotometrically. The minimal inhibitory concentration ("MIC") of the therapy is determined, which is defined as the lowest concentration of the lead compound which prevents visible growth of an inoculum as measured by determining the culture turbidity.

The anti-fungal activity of a therapy can also be determined utilizing colorimetric based assays well-known to one of skill in the art. One exemplary colorimetric assay that can be used to assess the anti-fungal activity of a therapy is described by Pfaller et al. (1994, Journal of Clinical Microbiology, 32(8): 1993-6, which is incorporated herein by reference in its entirety; also see Tiballi et al., 1995, Journal of Clinical Microbiology, 33(4): 915-7). This assay employs a colorimetric endpoint using an oxidation-reduction indicator (Alamar Biosciences, Inc., Sacramento CA).

The anti-fungal activity of a therapy can also be determined utilizing photometric assays well-known to one of skill in the art (see, *e.g*., Clancy et al., 1997 Journal of Clinical Microbiology, 35(11): 2878-82; Jahn et al., 1995, Journal of Clinical Microbiology, 33(3): 661-667, each of which is incorporated herein by reference in its entirety). This photometric assay is based on quantifying mitochondrial respiration by viable fungi through the reduction of 3-(4,5-dimethyl-2thiazolyl)-2,5,-diphenyl-2H-tetrazolium bromide (MTT) to formazan. MIC's determined by this assay are defined as the highest concentration of the test therapy associated with the first precipitous drop in optical density. In some embodiments, the therapy is assayed for anti-fungal activity using macrodilution, microdilution and MTT assays in parallel.

Further, any *in vitro* assays known to those skilled in the art can be used to evaluate the prophylactic and/or therapeutic utility of an antibody therapy disclosed herein for a particular disorder or one or more symptoms thereof.

The antibodies, compositions, or combination therapies of the invention can be tested in suitable animal model systems prior to use in humans. Such animal model systems include, but are not limited to, rats, mice, chicken, cows, monkeys, pigs, dogs, rabbits, etc. Any animal system well-known in the art may be used. Several aspects of the procedure may vary; said aspects include, but are not limited to, the temporal regime of administering the therapies (*e.g*., prophylactic and/or therapeutic agents) whether such therapies are administered separately or as an admixture, and the frequency of administration of the therapies.

Animal models can be used to assess the efficacy of the antibodies, fragments thereof, or compositions of the invention for treating, managing, preventing, or ameliorating a particular disorder or one or more symptom thereof.

The administration of antibodies, compositions, or combination therapies according to the methods of the invention can be tested for their ability to decrease the time course of a particular disorder by at least 25%, preferably at least 50%, at least 60%, at least 75%, at least 85%, at least 95%, or at least 99%. The antibodies, compositions, or combination therapies of the invention can also be tested for their ability to increase the survival period of humans suffering from a particular disorder by at least 25%, preferably at least 50%, at least 60%, at least 75%, at least 85%, at least 95%, or at least 99%. Further, antibodies, compositions, or combination therapies of the invention can be tested for their ability reduce the hospitalization period of humans suffering from viral respiratory infection by at least 60%, preferably at least 75%, at least 85%, at least 95%, or at least 99%. Techniques known to those of skill in the art can be used to analyze the function of the antibodies, compositions, or combination therapies of the invention *in vivo.*

Further, any *in vivo* assays known to those skilled in the art can be used to evaluate the prophylactic and/or therapeutic utility of an antibody, a fragment thereof, a composition, a combination therapy disclosed herein for a particular disorder or one or more symptoms thereof.

The toxicity and/or efficacy of the prophylactic and/or therapeutic protocols of the instant invention can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Therapies that exhibit large therapeutic indices are preferred. While therapies that exhibit toxic side effects may be used, care should be taken to design a delivery system that targets such agents to the site of affected tissue in order to minimize potential damage to uninfected cells and, thereby, reduce side effects.

The data obtained from the cell culture assays and animal studies can be used in formulating a range of dosage of the prophylactic and/or therapeutic agents for use in humans. The dosage of such agents lies preferably within a range of circulating concentrations that include the ED50 with little or no toxicity. The dosage may vary within this range depending upon the dosage form employed and the route of administration utilized. For any therapy used in the method of the invention, the therapeutically effective dose can be estimated initially from cell culture assays. A dose may be formulated in animal models to achieve a circulating plasma concentration range that includes the IC50 (i.e., the concentration of the test compound that achieves a half-maximal inhibition of symptoms) as determined in cell culture. Such information can be used to more accurately determine useful doses in humans. Levels in plasma may be measured, for example, by high performance liquid chromatography.

### 2.14 Kits

The invention provides kits comprising sub-banks of antibody framework regions of a species of interest. The invention also provides kits comprising sub-banks of CDRs of a species of interest. The invention also provides kits comprising combinatorial sub-libraries that comprises plurality of nucleic acid sequences comprising nucleotide sequences, each nucleotide sequence encoding one framework region (*e.g*., FR1) in frame fused to one corresponding CDR (*e.g*., CDR1). The invention further provides kits comprising combinatorial libraries that comprises plurality of nucleic acid sequences comprising nucleotide sequences, each nucleotide sequence encoding the framework regions and CDRs fused in-frame (*e.g*., FR1+CDR1+FR2+CDR2+FR3+CDR3+FR4).

In some preferred embodiments, the invention provides kits comprising sub-banks of human immunoglobulin framework regions, sub-banks of CDRs, combinatorial sub-libraries, and/or combinatorial libraries. In one embodiment, the invention provides a kit comprising a framework region sub-bank for variable light chain framework region 1, 2, 3, and/or 4, wherein the framework region is defined according to the Kabat system. In another embodiment, the invention provides a kit comprising a framework region sub-bank for variable light chain framework region 1, 2, 3, and/or 4, wherein the framework region is defined according to the Chothia system. In another embodiment, the invention provides a kit comprising a framework region sub-bank for variable heavy chain framework region 1, 2, 3, and/or 4, wherein the framework region is defined according to the Kabat system. In another embodiment, the invention provides a kit comprising a framework region sub-bank for variable heavy chain framework region 1, 2, 3, and/or 4, wherein the framework region is defined according to the Chothia system. In yet another embodiment, the invention provides a kit comprising sub-banks of both the light chain and the heavy chain frameworks.

The invention also provides a pharmaceutical pack or kit comprising one or more containers filled with a humanized antibody of the invention. The pharmaceutical pack or kit may further comprises one or more other prophylactic or therapeutic agents useful for the treatment of a particular disease. The invention also provides a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects approval by the agency of manufacture, use or sale for human administration.

### 2.15 Article of Manufacture

The present invention also encompasses a finished packaged and labeled pharmaceutical product. This article of manufacture includes the appropriate unit dosage form in an appropriate vessel or container such as a glass vial or other container that is hermetically sealed. In the case of dosage forms suitable for parenteral administration the active ingredient is sterile and suitable for administration as a particulate free solution. In other words, the invention encompasses both parenteral solutions and lyophilized powders, each being sterile, and the latter being suitable for reconstitution prior to injection. Alternatively, the unit dosage form may be a solid suitable for oral, transdermal, topical or mucosal delivery.

In a preferred embodiment, the unit dosage form is suitable for intravenous, intramuscular or subcutaneous delivery. Thus, the invention encompasses solutions, preferably sterile, suitable for each delivery route.

As with any pharmaceutical product, the packaging material and container are designed to protect the stability of the product during storage and shipment. Further, the products of the invention include instructions for use or other informational material that advise the physician, technician or patient on how to appropriately prevent or treat the disease or disorder in question. In other words, the article of manufacture includes instruction means indicating or suggesting a dosing regimen including, but not limited to, actual doses, monitoring procedures (such as methods for monitoring mean absolute lymphocyte counts, tumor cell counts, and tumor size) and other monitoring information.

More specifically, the invention provides an article of manufacture comprising packaging material, such as a box, bottle, tube, vial, container, sprayer, insufflator, intravenous (i.v.) bag, envelope and the like; and at least one unit dosage form of a pharmaceutical agent contained within said packaging material. The invention further provides an article of manufacture comprising packaging material, such as a box, bottle, tube, vial, container, sprayer, insufflator, intravenous (i.v.) bag, envelope and the like; and at least one unit dosage form of each pharmaceutical agent contained within said packaging material.

In a specific embodiment, an article of manufacture comprises packaging material and a pharmaceutical agent and instructions contained within said packaging material, wherein said pharmaceutical agent is a humanized antibody and a pharmaceutically acceptable carrier, and said instructions indicate a dosing regimen for preventing, treating or managing a subject with a particular disease. In another embodiment, an article of manufacture comprises packaging material and a pharmaceutical agent and instructions contained within said packaging material, wherein said pharmaceutical agent is a humanized antibody, a prophylactic or therapeutic agent other than the humanized antibody and a pharmaceutically acceptable carrier, and said instructions indicate a dosing regimen for preventing, treating or managing a subject with a particular disease. In another embodiment, an article of manufacture comprises packaging material and two pharmaceutical agents and instructions contained within said packaging material, wherein said first pharmaceutical agent is a humanized antibody and a pharmaceutically acceptable carrier and said second pharmaceutical agent is a prophylactic or therapeutic agent other than the humanized antibody, and said instructions indicate a dosing regimen for preventing, treating or managing a subject with a particular disease.

The present invention provides that the adverse effects that may be reduced or avoided by the methods of the invention are indicated in informational material enclosed in an article of manufacture for use in preventing, treating or ameliorating one or more symptoms associated with a disease. Adverse effects that may be reduced or avoided by the methods of the invention include but are not limited to vital sign abnormalities (*e.g*., fever, tachycardia, bardycardia, hypertension, hypotension), hematological events (*e.g*., anemia, lymphopenia, leukopenia, thrombocytopenia), headache, chills, dizziness, nausea, asthenia, back pain, chest pain (*e.g*., chest pressure), diarrhea, myalgia, pain, pruritus, psoriasis, rhinitis, sweating, injection site reaction, and vasodilatation. Since some of the therapies may be immunosuppressive, prolonged immunosuppression may increase the risk of infection, including opportunistic infections. Prolonged and sustained immunosuppression may also result in an increased risk of developing certain types of cancer.

Further, the information material enclosed in an article of manufacture can indicate that foreign proteins may also result in allergic reactions, including anaphylaxis, or cytosine release syndrome. The information material should indicate that allergic reactions may exhibit only as mild pruritic rashes or they may be severe such as erythroderma, Stevens Johnson syndrome, vasculitis, or anaphylaxis. The information material should also indicate that anaphylactic reactions (anaphylaxis) are serious and occasionally fatal hypersensitivity reactions. Allergic reactions including anaphylaxis may occur when any foreign protein is injected into the body. They may range from mild manifestations such as urticaria or rash to lethal systemic reactions. Anaphylactic reactions occur soon after exposure, usually within 10 minutes. Patients may experience paresthesia, hypotension, laryngeal edema, mental status changes, facial or pharyngeal angioedema, airway obstruction, bronchospasm, urticaria and pruritus, serum sickness, arthritis, allergic nephritis, glomerulonephritis, temporal arthritis, or eosinophilia.

The information material can also indicate that cytokine release syndrome is an acute clinical syndrome, temporally associated with the administration of certain activating anti T cell antibodies. Cytokine release syndrome has been attributed to the release of cytokines by activated lymphocytes or monocytes. The clinical manifestations for cytokine release syndrome have ranged from a more frequently reported mild, self limited, "flu like" illness to a less frequently reported severe, life threatening, shock like reaction, which may include serious cardiovascular, pulmonary and central nervous system manifestations. The syndrome typically begins approximately 30 to 60 minutes after administration (but may occur later) and may persist for several hours. The frequency and severity of this symptom complex is usually greatest with the first dose. With each successive dose, both the incidence and severity of the syndrome tend to diminish. Increasing the amount of a dose or resuming treatment after a hiatus may result in a reappearance of the syndrome. As mentioned above, the invention encompasses methods of treatment and prevention that avoid or reduce one or more of the adverse effects discussed herein.

### 2.16 Exemplary Embodiments

1. A nucleic acid sequence comprising a first nucleotide sequence encoding a humanized heavy chain variable region, said first nucleotide sequence encoding the humanized heavy chain variable region produced by fusing together a nucleic acid sequence encoding a heavy chain framework region 1, a nucleic acid sequence encoding a heavy chain complementarity determining region (CDR) 1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, wherein the CDRs are derived from a donor antibody heavy chain variable region and each heavy chain framework region is from a sub-bank of human heavy chain framework regions.
2. A nucleic acid sequence comprising a first nucleotide sequence encoding a humanized light chain variable region, said first nucleotide sequence encoding the humanized light chain variable region produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the CDRs are derived from a donor antibody light chain variable region and each light chain framework region is from a sub-bank of human light chain framework regions.
3. The nucleic acid sequence of embodiment 1 further comprising a second nucleotide sequence encoding a donor light chain variable region.
4. The nucleic acid sequence of embodiment 1 further comprising a second nucleotide sequence encoding a humanized light chain variable region, said second nucleotide sequence encoding the humanized light chain variable region produced by fusing together a nucleic acid sequence encoding a light chain framework region 1, a nucleic acid sequence encoding a light chain CDR1, a nucleic acid sequence encoding a light chain framework region 2, a nucleic acid sequenced encoding a light chain CDR2, a nucleic acid sequence encoding a light chain framework region 3, a nucleic acid sequence encoding a light chain CDR3, and a nucleic acid sequence encoding a light chain framework region 4, wherein the CDRs are derived from a donor antibody light chain variable region and each light chain framework region is from a sub-bank of human light chain framework regions.
5. The nucleic acid sequence of embodiment 2 further comprising a second nucleotide sequence encoding a donor heavy chain variable region.
6. The nucleic acid sequence of embodiment 1, wherein one or more of the CDRs derived from the donor antibody heavy chain variable region contains one or more mutations relative to the nucleic acid sequence encoding the corresponding CDR in the donor antibody.
7. The nucleic acid sequence of embodiment 2, wherein one or more of the CDRs derived from the donor antibody light chain variable region contains one or more mutations relative to the nucleic acid sequence encoding the corresponding CDR in the donor antibody.
8. The nucleic acid sequence of embodiment 4, wherein one or more of the CDRs derived from the donor antibody light chain variable region contains one or more mutations relative to the nucleic acid sequence encoding the corresponding CDR in the donor antibody.
9. A nucleic acid sequence comprising a first nucleotide sequence encoding a humanized heavy chain variable region, said first nucleotide acid sequence encoding the humanized heavy chain variable region produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, wherein at least one CDR is from a sub-bank of heavy chain CDRs derived from donor antibodies that immunospecifically bind to an antigen and at least one heavy chain framework region is from a sub-bank of human heavy chain framework regions.
10. A nucleic acid sequence comprising a first nucleotide sequence encoding a humanized light chain variable region, said first nucleotide sequence encoding the humanized light chain variable region produced by fusing together a nucleic acid sequence encoding a light chain framework region 1, a nucleic acid sequence encoding a light chain CDR1, a nucleic acid sequence encoding a light chain framework region 2, a nucleic acid Sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one CDR is from a sub-bank of light chain CDRs derived from donor antibodies that immunospecifically bind to an antigen and at least one light chain framework region is from a sub-bank of human light chain framework regions.
11. The nucleic acid of embodiment 9 further comprising a second nucleotide sequence encoding a donor light chain variable region.
12. The nucleic acid sequence of embodiment 9 further comprising a second nucleotide sequence encoding a humanized light chain variable region, said second nucleotide sequence encoding the humanized light chain variable region produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the CDRs are derived from a donor antibody light chain variable region and at least one light chain framework region is from a sub-bank of human light chain framework regions.
13. The nucleic acid sequence of embodiment 9 further comprising a second nucleotide sequence encoding a humanized light chain variable region, said second nucleotide sequence encoding the humanized light chain variable region produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one CDR is from a sub-bank of light chain CDRs derived from donor antibodies that immunospecifically bind to an antigen and at least one light chain framework region is from a sub-bank of human light chain framework regions.
14. The nucleic acid sequence of embodiment 10 further comprising a second nucleotide sequence encoding a donor heavy chain variable region.
15. The nucleic acid sequence of embodiment 10 further comprising a second nucleotide sequence encoding a humanized heavy chain variable region, said second nucleotide sequence encoding the humanized heavy chain variable region produced by fusing together a nucleic acid sequence encoding a heavy chain framework region 1, a nucleic acid sequence encoding a heavy chain complementarity determining region (CDR) 1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, wherein the CDRs are derived from a donor antibody heavy chain variable region and at least one heavy chain framework region is from a sub-bank of human heavy chain framework regions.
16. A cell engineered to contain the nucleic acid sequence of embodiment 1.
17. A cell engineered to contain the nucleic acid sequence of embodiment 2.
18. The cell of embodiment 16 further engineered to contain the nucleic acid sequence of embodiment 2.
19. A cell engineered to contain the nucleic acid of embodiment 3.
20. A cell engineered to contain the nucleic acid of embodiment 4.
21. A cell engineered to contain the nucleic acid of embodiment 5.
22. A cell engineered to contain the nucleic acid sequence of embodiment 9.
23. A cell engineered to contain the nucleic acid sequence of embodiment 10.
24. The cell of embodiment 22 further engineered to contain the nucleic acid sequence of embodiment 10.
25. A cell engineered to contain the nucleic acid sequence of embodiment 11.
26. A cell engineered to contain the nucleic acid sequence of embodiment 12.
27. A cell engineered to contain the nucleic acid sequence of embodiment 13.
28. A cell engineered to contain the nucleic acid sequence of embodiment 14.
29. A cell engineered to contain the nucleic acid sequence of embodiment 15.
30. A cell comprising a first nucleic acid sequence comprising a first nucleotide sequence encoding a humanized heavy chain variable region, said cell produced by the process comprising introducing into a cell a nucleic acid sequence comprising a nucleotide sequence encoding a humanized heavy chain variable region synthesized by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, wherein the CDRs are derived from a donor antibody heavy chain variable region and at least one heavy chain framework region is from a sub-bank of human heavy chain framework regions.
31. A cell comprising a first nucleic acid sequence comprising a first nucleotide sequence encoding a humanized light chain variable region, said cell produced by the process comprising introducing into a cell a nucleic acid sequence comprising a nucleotide sequence encoding a humanized light chain variable region synthesized by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the CDRs are derived from a donor antibody light chain variable region and at least one light chain framework region is from a sub-bank of human light chain framework regions.
32. A cell comprising a nucleic acid sequence comprising a first nucleotide sequence encoding a humanized heavy chain variable region and a second nucleotide sequence encoding a humanized light chain variable region, said cell produced by the process comprising introducing into a cell a nucleic acid sequence comprising: (i) a first nucleotide sequence encoding a humanized heavy chain variable region synthesized by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4; and (ii) a second nucleotide sequence encoding a humanized light chain variable region synthesized by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the CDRs of the heavy chain variable region are derived from a donor antibody heavy chain variable region, the CDRs of the light chain variable region are derived from a donor light chain variable region, at least one heavy chain framework region is from a sub-bank of human heavy chain framework regions, and at least one light chain framework region is from a sub-bank of human light chain framework regions.
33. A cell comprising a first nucleic acid sequence comprising a first nucleotide sequence encoding a humanized heavy chain variable region, said cell produced by the process comprising introducing into a cell a nucleic acid sequence comprising a nucleotide sequence encoding a humanized heavy chain variable region synthesized by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, wherein at least one CDR is from a sub-bank of heavy chain CDRs derived from donor antibodies that immunospecifically bind to an antigen and at least one heavy chain framework region is from a sub-bank of human heavy chain framework regions.
34. A cell comprising a first nucleic acid sequence comprising a first nucleotide sequence encoding a humanized light chain variable region, said cell produced by the process comprising introducing into a cell a nucleic acid sequence comprising a nucleotide sequence encoding a humanized light chain variable region synthesized by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one CDR is from a sub-bank of light chain CDRs derived from donor antibodies that immunospecifically bind to an antigen and at least one light chain framework region is from a sub-bank of human light chain framework regions.
35. A cell comprising a nucleic acid sequence comprising a first nucleotide sequence encoding a humanized heavy chain variable region and a second nucleotide sequence encoding a humanized light chain variable region, said cell produced by the process comprising introducing into a cell a nucleic acid sequence comprising: (i) a first nucleotide sequence encoding a humanized heavy chain variable region synthesized by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4; and (ii) a second nucleotide sequence encoding a humanized light chain variable region synthesized by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one heavy chain variable region CDR is from a sub-bank of heavy chain CDRs derived from donor antibodies that immunospecifically bind to an antigen, at least one light chain variable region CDR is from a sub-bank of light chain CDRs derived from donor antibodies that immunospecifically bind to an antigen; at least one heavy chain framework region is from a sub-bank of human heavy chain framework regions, and at least one light chain framework region is from a sub-bank of human light chain framework regions.
36. A cell comprising a nucleic acid sequence comprising a first nucleotide sequence encoding a humanized heavy chain variable region and a second nucleotide sequence encoding a humanize light chain variable region, said cell produced by the process comprising introducing into a cell a nucleic acid sequence comprising: (i) a first nucleotide sequence encoding a humanized heavy chain variable region synthesized by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4; and (ii) a second nucleotide sequence encoding a humanized light chain variable region synthesized by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the heavy chain variable region CDRs are derived from a donor antibody heavy chain variable region, at least one light chain variable region CDR is from a sub-bank of light chain CDRs derived from donor antibodies that immunospecifically bind to an antigen, at least one heavy chain framework region is from a sub-bank of human heavy chain framework regions, and at least one light chain framework region is from a sub-bank of human light chain framework regions.
37. A cell comprising a nucleic acid sequence comprising a first nucleotide sequence encoding a humanized heavy chain variable region and a second nucleotide sequence encoding a humanized light chain variable region, said cell produced by the process comprising introducing into a cell a nucleic acid sequence comprising: (i) a first nucleotide sequence encoding a humanized heavy chain variable region synthesized by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4; and (ii) a second nucleotide sequence encoding a humanized light chain variable region synthesized by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one heavy chain variable region CDR is from a sub-bank of heavy chain CDRs derived from donor antibodies that immunospecifically bind to an antigen, the light chain variable region CDRs are derived from a donor antibody light chain variable region, at least one heavy chain framework region is from a sub-bank of human heavy chain framework regions, and at least one light chain framework region is from a sub-bank of human light chain framework regions.
38. The cell of embodiment 30 further comprising a second nucleic acid sequence comprising a second nucleotide sequence encoding a humanized light chain variable region.
39. The cell of embodiment 30 further comprising a second nucleic acid sequence comprising a second nucleotide sequence encoding a light chain variable region.
40. The cell of embodiment 31 further comprising a second nucleic acid sequence comprising a second nucleotide sequence encoding a heavy chain variable region.
41. The cell of embodiment 33 further comprising a second nucleic acid sequence comprising a second nucleotide sequence encoding a humanized light chain variable region.
42. The cell of embodiment 33 further comprising a second nucleic acid sequence comprising a second nucleotide sequence encoding a light chain variable region.
43. The cell of embodiment 34 further comprising a second nucleic acid sequence comprising a second nucleotide sequence encoding a heavy chain variable region.
44. A cell containing nucleic acid sequences encoding a humanized antibody that immunospecifically binds to an antigen, said cell produced by the process comprising:
   (a) introducing into a cell a nucleic acid sequence comprising a nucleotide sequence encoding a humanized heavy chain variable region, said first nucleotide sequence synthesized by fusing together a nucleic acid sequence encoding a heavy chain framework region 1, a nucleic acid sequence encoding a heavy chain complementarity determining region (CDR) 1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, wherein the CDRs are derived from a donor antibody heavy chain variable region and at least one heavy chain framework region is from a sub-bank of human heavy chain framework regions; and
   (b) introducing into a cell a nucleic acid sequence comprising a nucleotide sequence encoding a humanized light chain variable region, said nucleotide sequence synthesized by fusing together a nucleic acid sequence encoding a light chain framework region 1, a nucleic acid sequence encoding a light chain complementarity determining region (CDR) 1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the CDRs are derived from a donor antibody light chain variable region and at least one light chain framework region is from a sub-bank of human light chain framework region.
45. A cell containing nucleic acid sequences encoding a humanized antibody that immunospecifically binds to an antigen, said cell produced by the process comprising:
   (a) introducing into a cell a nucleic acid sequence comprising a nucleotide sequence encoding a heavy chain variable region, said nucleotide sequence synthesized by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, wherein at least one CDR is from a sub-bank of heavy chain CDRs derived from donor antibodies that immunospecifically bind to an antigen and at least one heavy chain framework region is from a sub-bank of human heavy chain framework regions; and
   (b) introducing into a cell a nucleic acid sequence comprising a nucleotide sequence encoding a humanized light chain variable region, said nucleotide sequence synthesized by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the CDRs are derived from a donor antibody light chain variable region and at least one light chain framework region is from a sub-bank of human light chain framework region.
46. A cell containing nucleic acid sequences encoding a humanized antibody that immunospecifically binds to an antigen, said cell produced by the process comprising:
   (a) introducing into a cell a nucleic acid sequence comprising a nucleotide acid sequence encoding a heavy chain variable region, said nucleotide sequence synthesized by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, wherein at least one CDR is from a sub-bank of heavy chain CDRs derived from donor antibodies that immunospecifically bind to an antigen and at least one heavy chain framework region is from a sub-bank of human heavy chain framework regions; and
   (b) introducing into a cell a nucleic acid sequence comprising a nucleotide sequence encoding a humanized light chain variable region, said nucleotide sequence synthesized by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one CDR is from a sub-bank of light chain CDRs derived from donor antibodies that immunospecifically bind to an antigen and at least one light chain framework region is from a sub-bank of human light chain framework regions.
47. A cell containing nucleic acid sequences encoding a humanized antibody that immunospecifically binds to an antigen, said cell produced by the process comprising:
   (a) introducing into a cell a nucleic acid sequence comprising a nucleotide sequence encoding a heavy chain variable region, said nucleotide sequence synthesized by fusing together a nucleic acid sequence encoding a heavy chain framework region 1, a nucleic acid sequence encoding a heavy chain complementarity determining region (CDR) 1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, wherein the CDRs are derived from a donor antibody heavy chain variable region and at least one heavy chain framework region is from a sub-bank of human heavy chain framework regions; and
   (b) introducing into a cell a nucleic acid sequence comprising a nucleotide sequence encoding a humanized light chain variable region, said nucleotide sequence synthesized by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one CDR is from a sub-bank of light chain CDRs derived from donor antibodies that immunospecifically bind to an antigen and at least one light chain framework region is from a sub-bank of human light chain framework regions.
48. A method of producing a humanized heavy chain variable region, said method comprising expressing the nucleotide sequence encoding the humanized heavy chain variable region in the cell of embodiment 30 or 33.
49. A method of producing a humanized light chain variable region, said method comprising expressing the nucleotide sequence encoding the humanized light chain variable region in the cell of embodiment 31 or 34.
50. A method of producing a humanized antibody, said method comprising expressing the nucleic acid sequence comprising the first nucleotide sequence encoding the humanized heavy chain variable region and the second nucleotide sequence encoding the humanized light chain variable region in the cell of embodiment 32, 35, 36 or 37.
51. A method of producing a humanized antibody that immunospecifically binds to an antigen, said method comprising expressing the nucleic acid sequences encoding the humanized antibody contained in the cell of embodiment 44, 45, 46 or 47.
52. A method of producing a humanized antibody that immunospecifically binds to an antigen, said method comprising:
   (a) generating sub-banks of heavy chain framework regions;
   (b) synthesizing a nucleic acid sequence comprising a nucleotide sequence encoding a humanized heavy chain variable region, said nucleotide sequence produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding heavy chain CDR2, a nucleic acid sequence encoding a heavy chain framework region 3, a nucleic acid sequence encoding a heavy chain CDR3, and a nucleic acid sequence encoding a heavy chain framework region 4, wherein the CDRs are derived from a donor antibody heavy chain variable region and at least one heavy chain framework region is from a sub-bank of human heavy chain framework regions;
   (c) introducing the nucleic acid sequence into a cell containing a nucleic acid sequence comprising a nucleotide sequence encoding a humanized variable light chain variable region; and
   (d) expressing the nucleotide sequences encoding the humanized heavy chain variable region and the humanized light chain variable region.
53. A method of producing a humanized antibody that immunospecifically binds to an antigen, said method comprising:
   (a) generating sub-banks of heavy chain framework regions;
   (b) synthesizing a nucleic acid sequence comprising a nucleotide sequence encoding a humanized heavy chain variable region, said nucleotide sequence produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding heavy chain CDR2, a nucleic acid sequence encoding a heavy chain framework region 3, a nucleic acid sequence encoding a heavy chain CDR3, and a nucleic acid sequence encoding a heavy chain framework region 4, wherein at least one CDR is from a sub-bank of heavy chain CDRs derived from donor antibodies that immunospecifically bind to an antigen and at least one heavy chain framework region is from a sub-bank of human heavy chain framework regions;
   (c) introducing the nucleic acid sequence into a cell containing a nucleic acid sequence comprising a nucleotide sequence encoding a humanized variable light chain variable region; and
   (d) expressing the nucleotide sequences encoding the humanized heavy chain variable region and the humanized light chain variable region.
54. A method of producing a humanized antibody that immunospecifically binds to an antigen, said method comprising:
   (a) generating sub-banks of light chain framework regions;
   (b) synthesizing a nucleic acid sequence comprising a nucleotide sequence encoding a humanized light chain variable region, said nucleotide sequence produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the CDRs are derived from a donor antibody light chain variable region and at least one light chain framework region is from a sub-bank of human light chain framework regions;
   (c) introducing the nucleic acid sequence into a cell containing a nucleic acid sequence comprising a nucleotide sequence encoding a humanized variable heavy chain variable region; and
   (d) expressing the nucleotide sequences encoding the humanized heavy chain variable region and the humanized light chain variable region.
55. A method of producing a humanized antibody that immunospecifically binds to an antigen, said method comprising:
   (a) generating sub-banks of light chain framework regions;
   (b) synthesizing a nucleic acid sequence comprising a nucleotide sequence encoding a humanized light chain variable region, said nucleotide sequence produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one CDR is from a sub-bank of light chain CDRs derived from donor antibodies that immunospecifically bind to an antigen and at least one light chain framework region is from a sub-bank of human light chain framework regions;
   (c) introducing the nucleic acid sequence into a cell containing a nucleic acid sequence comprising a nucleotide sequence encoding a humanized variable heavy chain variable region; and
   (d) expressing the nucleotide sequences encoding the humanized heavy chain variable region and the humanized light chain variable region.
56. A method of producing a humanized antibody that immunospecifically binds to an antigen, said method comprising:
   (a) generating sub-banks of light chain framework regions;
   (b) generating sub-banks of heavy chain framework regions;
   (c) synthesizing a nucleic acid sequence comprising a nucleotide sequence encoding a humanized heavy chain variable region, said nucleotide sequence produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding heavy chain CDR2, a nucleic acid sequence encoding a heavy chain framework region 3, a nucleic acid sequence encoding a heavy chain CDR3, and a nucleic acid sequence encoding a heavy chain framework region 4, wherein the CDRs are derived from a donor antibody heavy chain variable region and at least one heavy chain framework region is from a sub-bank of human heavy chain framework regions;
   (d) synthesizing a nucleic acid sequence comprising a nucleotide sequence encoding a humanized light chain variable region, said nucleotide sequence produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the CDRs are derived from a donor antibody light chain variable region and at least one light chain framework region is from a sub-bank of human light chain framework regions;
   (e) introducing the nucleic acid sequences into a cell; and
   (f) expressing the nucleotide sequences encoding the humanized heavy chain variable region and the humanized light chain variable region.
57. A method of producing a humanized antibody that immunospecifically binds to an antigen, said method comprising:
   (a) generating sub-banks of light chain framework regions;
   (b) generating sub-banks of heavy chain framework regions;
   (c) synthesizing a nucleic acid sequence comprising a nucleotide sequence encoding a humanized heavy chain variable region, said nucleotide sequence produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding heavy chain CDR2, a nucleic acid sequence encoding a heavy chain framework region 3, a nucleic acid sequence encoding a heavy chain CDR3, and a nucleic acid sequence encoding a heavy chain framework region 4, wherein at least one CDR is from a sub-bank of heavy chain CDRs derived from donor antibodies that immunospecifically bind to an antigen and at least one heavy chain framework region is from a sub-bank of human heavy chain framework regions;
   (d) synthesizing a nucleic acid sequence comprising a nucleotide sequence encoding a humanized light chain variable region, said nucleotide sequence produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the CDRs are derived from a donor antibody light chain variable region and at least one light chain framework region is from a sub-bank of human light chain framework regions;
   (e) introducing the nucleic acid sequences into a cell; and
   (f) expressing the nucleotide sequences encoding the humanized heavy chain variable region and the humanized light chain variable region.
58. A method of producing a humanized antibody that immunospecifically binds to an antigen, said method comprising:
   (a) generating sub-banks of light chain framework regions;
   (b) generating sub-banks of heavy chain framework regions;
   (c) synthesizing a nucleic acid sequence comprising a nucleotide sequence encoding a humanized heavy chain variable region, said nucleotide sequence produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding heavy chain CDR2, a nucleic acid sequence encoding a heavy chain framework region 3, a nucleic acid sequence encoding a heavy chain CDR3, and a nucleic acid sequence encoding a heavy chain framework region 4, wherein the CDRs are derived from a donor antibody heavy chain variable region and at least one heavy chain framework region is from a sub-bank of human heavy chain framework regions;
   (d) synthesizing a nucleic acid sequence comprising a nucleotide sequence encoding a humanized light chain variable region, said nucleotide sequence produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one CDR is from a sub-bank of light chain CDRs derived from donor antibodies that immunospecifically bind to an antigen and at least one light chain framework region is from a sub-bank of human light chain framework regions;
   (e) introducing the nucleic acid sequences into a cell; and
   (f) expressing the nucleotide sequences encoding the humanized heavy chain variable region and the humanized light chain variable region.
59. A method of producing a humanized antibody that immunospecifically binds to an antigen, said method comprising:
   (a) generating sub-banks of light chain framework regions;
   (b) generating sub-banks of heavy chain framework regions;
   (c) synthesizing a nucleic acid sequence comprising a nucleotide sequence encoding a humanized heavy chain variable region, said nucleotide sequence produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding heavy chain CDR2, a nucleic acid sequence encoding a heavy chain framework region 3, a nucleic acid sequence encoding a heavy chain CDR3, and a nucleic acid sequence encoding a heavy chain framework region 4, wherein at least one CDR is from a sub-bank of heavy chain CDRs derived from donor antibodies that immunospecifically bind to an antigen and at least one heavy chain framework region is from a sub-bank of human heavy chain framework regions;
   (d) synthesizing a nucleic acid sequence comprising a nucleotide sequence encoding a humanized light chain variable region, said nucleotide sequence produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one CDR is from a sub-bank of light chain CDRs derived from donor antibodies that immunospecifically bind to an antigen and at least one light chain framework region is from a sub-bank of human light chain framework regions;
   (e) introducing the nucleic acid sequences into a cell; and
   (f) expressing the nucleotide sequences encoding the humanized heavy chain variable region and the humanized light chain variable region.
60. A method of producing a humanized antibody that immunospecifically binds to an antigen, said method comprising:
   (a) generating sub-banks of light chain framework regions;
   (b) generating sub-banks of heavy chain framework regions;
   (c) synthesizing a nucleic acid sequence comprising: (i) a first nucleotide sequence encoding a humanized heavy chain variable region, said first nucleotide sequence produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding heavy chain CDR2, a nucleic acid sequence encoding a heavy chain framework region 3, a nucleic acid sequence encoding a heavy chain CDR3, and a nucleic acid sequence encoding a heavy chain framework region 4, and (ii) a second nucleotide sequence encoding a humanized light chain variable region, said second nucleotide sequence produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the heavy chain variable region CDRs are derived from a donor antibody heavy chain variable region, the light chain variable region CDRs are derived from a donor antibody light chain variable region, at least one heavy chain framework region is from a sub-bank of human heavy chain framework regions and at least one light chain framework region is from a sub-bank of human light chain framework regions;
   (d) introducing the nucleic acid sequence into a cell; and
   (e) expressing the nucleotide sequences encoding the humanized heavy chain variable region and the humanized light chain variable region.
61. A method of producing a humanized antibody that immunospecifically binds to an antigen, said method comprising:
   (a) generating sub-banks of light chain framework regions;
   (b) generating sub-banks of heavy chain framework regions;
   (c) synthesizing a nucleic acid sequence comprising: (i) a first nucleotide sequence encoding a humanized heavy chain variable region, said first nucleotide sequence produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding heavy chain CDR2, a nucleic acid sequence encoding a heavy chain framework region 3, a nucleic acid sequence encoding a heavy chain CDR3, and a nucleic acid sequence encoding a heavy chain framework region 4, and (ii) a second nucleotide sequence encoding a humanized light chain variable region, said second nucleotide sequence produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one heavy chain variable region CDR is from a sub-bank of heavy chain CDRs derived from donor antibodies that immunospecifically bind to an antigen, the light chain variable region CDRs are derived from a donor antibody light chain variable region, at least one heavy chain framework region is from a sub-bank of human heavy chain framework regions and at least one light chain framework region is from a sub-bank of human light chain framework regions;
   (d) introducing the nucleic acid sequence into a cell; and
   (e) expressing the nucleotide sequences encoding the humanized heavy chain variable region and the humanized light chain variable region.
62. A method of producing a humanized antibody that immunospecifically binds to an antigen, said method comprising:
   (a) generating sub-banks of light chain framework regions;
   (b) generating sub-banks of heavy chain framework regions;
   (c) synthesizing a nucleic acid sequence comprising: (i) a first nucleotide sequence encoding a humanized heavy chain variable region, said first nucleotide sequence produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding heavy chain CDR2, a nucleic acid sequence encoding a heavy chain framework region 3, a nucleic acid sequence encoding a heavy chain CDR3, and a nucleic acid sequence encoding a heavy chain framework region 4, and (ii) a second nucleotide sequence encoding a humanized light chain variable region, said second nucleotide sequence produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the heavy chain variable region CDRs are derived from a donor antibody heavy chain variable region, at least one light chain variable region CDR is from a sub-bank of light chain CDRs derived from donor antibodies that immunospecifically bind to an antigen, at least one heavy chain framework region is from a sub-bank of human heavy chain framework regions and at least one light chain framework region is from a sub-bank of human light chain framework regions;
   (d) introducing the nucleic acid sequence into a cell; and
   (e) expressing the nucleotide sequences encoding the humanized heavy chain variable region and the humanized light chain variable region.
63. A method of producing a humanized antibody that immunospecifically binds to an antigen, said method comprising:
   (a) generating sub-banks of light chain framework regions;
   (b) generating sub-banks of heavy chain framework regions;
   (c) synthesizing a nucleic acid sequence comprising: (i) a first nucleotide sequence encoding a humanized heavy chain variable region, said first nucleotide sequence produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding heavy chain CDR2, a nucleic acid sequence encoding a heavy chain framework region 3, a nucleic acid sequence encoding a heavy chain CDR3, and a nucleic acid sequence encoding a heavy chain framework region 4, and (ii) a second nucleotide sequence encoding a humanized light chain variable region, said second nucleotide sequence produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one heavy chain variable region CDR is from a sub-bank of heavy chain CDRs derived from donor antibodies that immunospecifically bind to an antigen, at least one light chain variable region CDR is from a sub-bank of light chain CDRs derived from donor antibodies that immunospecifically bind to an antigen, at least one heavy chain framework region is from a sub-bank of human heavy chain framework regions and at least one light chain framework region is from a sub-bank of human light chain framework regions;
   (d) introducing the nucleic acid sequence into a cell; and
   (e) expressing the nucleotide sequences encoding the humanized heavy chain variable region and the humanized light chain variable region.
64. The method of embodiment 52, 53, 54 or 55 further comprising (e) screening for a humanized antibody that immunospecifically binds to the antigen.
65. The method of embodiment 56, 57, 58 or 59 further comprising (g) screening for a humanized antibody that immunospecifically binds to the antigen.
66. The method of embodiment 60, 61, 62 or 63 further comprising (f) screening for a humanized antibody that immunospecifically binds to the antigen.
67. A humanized antibody produced by the method of embodiment 48.
68. A humanized antibody produced by the method of embodiment 49.
69. A humanized antibody produced by the method of embodiment 50.
70. A humanized antibody produced by the method of embodiment 51.
71. A humanized antibody produced by the method of any one of embodiments 52-63.
72. A humanized antibody produced by the method of embodiment 64.
73. A humanized antibody produced by the method of embodiment 65.
74. A humanized antibody produced by the method of embodiment 66.
75. A composition comprising the humanized antibody of embodiment 67, and a carrier, diluent or excipient.
76. A composition comprising the humanized antibody of embodiment 68, and a carrier, diluent or excipient.
77. A composition comprising the humanized antibody of embodiment 69, and a carrier, diluent or excipient.
78. A composition comprising the humanized antibody of embodiment 70, and a carrier, diluent or excipient.
79. A composition comprising the humanized antibody of embodiment 71, and a carrier, diluent or excipient.
80. A composition comprising the humanized antibody of embodiment 72, and a carrier, diluent or excipient.
81. A composition comprising the humanized antibody of embodiment 73, and a carrier, diluent or excipient.
82. A plurality of nucleic acid sequences comprising nucleotide sequences encoding humanized heavy chain variable regions, said nucleotide sequences encoding the humanized heavy chain variable regions each produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, wherein the CDRs are derived from a donor antibody heavy chain variable region and at least one heavy chain framework region is from a sub-bank of human heavy chain framework regions.
83. A plurality of nucleic acid sequences comprising nucleotide sequences encoding humanized heavy chain variable regions, said nucleotide sequences encoding the humanized heavy chain variable regions each produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, wherein at least one CDR is from a sub-bank of heavy chain CDRs derived from donor antibodies that immunospecifically bind to an antigen and at least one heavy chain framework region is from a sub-bank of human heavy chain framework regions.
84. A plurality of nucleic acid sequences comprising nucleotide sequences encoding humanized light chain variable regions, said nucleotide sequences encoding the humanized light chain variable regions each produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the CDRs are derived from a donor antibody light chain variable region and at least one light chain framework region is from a sub-bank of human light chain framework regions.
85. A plurality of nucleic acid sequences comprising nucleotide sequences encoding humanized light chain variable regions, said nucleotide sequences encoding the humanized light chain variable regions each produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one CDR is from a sub-bank of light chain CDRs derived from donor antibodies that immunospecifically bind to an antigen and at least one light chain framework region is from a sub-bank of human light chain framework regions.
86. A plurality of nucleic acid sequences comprising: (i) a first set of nucleotide sequences encoding humanized heavy chain variable regions, said first set of nucleotide sequences encoding the humanized heavy chain variable regions each produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, and (ii) a second set of nucleotide sequences encoding humanized light chain variable regions, said second set of nucleotide sequences encoding the humanized light chain variable regions each produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the heavy chain variable region CDRs are derived from a donor antibody heavy chain variable region, the light chain variable region CDRs are derived from a donor antibody light chain variable region, at least one heavy chain framework region is from a sub-bank of human heavy chain framework regions and at least one light chain framework region is from a sub-bank of human light chain framework regions.
87. A plurality of nucleic acid sequences comprising: (i) a first set of nucleotide sequences encoding humanized heavy chain variable regions, said first set of nucleotide sequences encoding the humanized heavy chain variable regions each produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, and (ii) a second set of nucleotide sequences encoding humanized light chain variable regions, said second set of nucleotide sequences encoding the humanized light chain variable regions each produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one heavy chain variable region CDR is from a sub-bank of heavy chain CDRs derived from donor antibodies that immunospecifically bind to an antigen, the light chain variable region CDRs are derived from a donor antibody light chain variable region, at least one heavy chain framework region is from a sub-bank of human heavy chain framework regions and at least one light chain framework region is from a sub-bank of human light chain framework regions.
88. A plurality of nucleic acid sequences comprising: (i) a first set of nucleotide sequences encoding humanized heavy chain variable regions, said first set of nucleotide sequences encoding the humanized heavy chain variable regions each produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, and (ii) a second set of nucleotide sequences encoding humanized light chain variable regions, said second set of nucleotide sequences encoding the humanized light chain variable regions each produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the heavy chain variable region CDRs are derived from a donor antibody heavy chain variable region, at least one light chain variable region CDR is from a sub-bank of light chain CDRs derived from donor antibodies that immunospecifically bind to an antigen, at least one heavy chain framework region is from a sub-bank of human heavy chain framework regions and at least one light chain framework region is from a sub-bank of human light chain framework regions.
89. A plurality of nucleic acid sequences comprising: (i) a first set of nucleotide sequences encoding humanized heavy chain variable regions, said first set of nucleotide sequences encoding the humanized heavy chain variable regions each produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, and (ii) a second set of nucleotide sequences encoding humanized light chain variable regions, said second set of nucleotide sequences encoding the humanized light chain variable regions each produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one heavy chain variable region CDR is from a sub-bank of heavy chain CDRs derived from donor antibodies that immunospecifically bind to an antigen, at least one light chain variable region CDR is from a sub-bank of light chain CDRs derived from donor antibodies that immunospecifically bind to an antigen, at least one heavy chain framework region is from a sub-bank of human heavy chain framework regions and at least one light chain framework region is from a sub-bank of human light chain framework regions.
90. A population of cells comprising nucleic acid sequences comprising nucleotide sequences encoding a plurality of humanized heavy chain variable regions, said cells produced by the process comprising introducing into cells nucleic acid sequences comprising nucleotide sequences encoding humanized heavy chain variable regions each synthesized by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, wherein the CDRs are derived from a donor antibody heavy chain variable region and at least one heavy chain framework region is from a sub-bank of human heavy chain framework regions.
91. A population of cells comprising nucleic acid sequences comprising nucleotide acid sequences encoding a plurality of humanized heavy chain variable regions, said cells produced by the process comprising introducing into cells nucleic acid sequences comprising nucleotide sequences encoding humanized heavy chain variable regions each synthesized by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, wherein at least one CDR is from a sub-bank of heavy chain CDRs derived from donor antibodies that immunospecifically bind to an antigen and at least one heavy chain framework region is from a sub-bank of human heavy chain framework regions.
92. A population of cells comprising nucleic sequences comprising nucleotide sequences encoding a plurality of humanized light chain variable regions, said cells produced by the process comprising introducing into cells nucleic acid sequences comprising nucleotide sequences encoding humanized light chain variable regions each synthesized by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the CDRs are derived from a donor antibody light chain variable region and at least one light chain framework region is from a sub-bank of human light chain framework regions.
93. A population of cells comprising nucleic acid sequences comprising nucleotide sequences encoding a plurality of humanized light chain variable regions, said cells produced by the process comprising introducing into cells nucleic acid sequences comprising nucleotide sequences encoding humanized light chain variable regions each synthesized by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one CDR is from a sub-bank of light chain CDRs derived from donor antibodies that immunospecifically bind to an antigen and at least one light chain framework region is from a sub-bank of human light chain framework regions.
94. The cells of embodiment 90, wherein the cells further comprise a nucleic acid sequence comprising a nucleotide sequence encoding a light chain variable region.
95. The cells of embodiment 91, wherein the cells further comprise a nucleic acid sequence comprising a nucleotide sequence encoding a light chain variable region.
96. The cells of embodiment 92, wherein the cells further comprise a nucleic acid sequence comprising a nucleotide sequence encoding a light chain variable region.
97. The cells of embodiment 93, wherein the cells further comprise a nucleic acid sequence comprising a nucleotide sequence encoding a humanized light chain variable region.
98. A population of cells comprising nucleic acid sequences comprising nucleotide sequences encoding a plurality of humanized heavy chain variable regions and a plurality of humanized light chain variable regions, said cells each produced by the process comprising introducing into cells nucleic acid sequences comprising: (i) a first set of nucleotide sequences encoding humanized heavy chain variable regions each synthesized by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, and (ii) a second set of nucleotide sequences encoding humanized light chain variable regions each synthesized by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the heavy chain variable region CDRs are derived from a donor antibody heavy chain variable region, the light chain variable region CDRs are derived from a donor antibody light chain variable region, at least one heavy chain framework region is from a sub-bank of human heavy chain framework regions and at least one light chain framework region is from a sub-bank of human light chain framework regions.
99. A population of cells comprising nucleic acid sequences comprising nucleotide sequences encoding a plurality of humanized heavy chain variable regions and a plurality of humanized light chain variable regions, said cells each produced by the process comprising introducing into cells nucleic acid sequences comprising: (i) a first set of nucleotide sequences encoding humanized heavy chain variable regions each synthesized by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, and (ii) a second set of nucleotide sequences encoding humanized light chain variable regions each synthesized by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one heavy chain variable region CDR is from a sub-bank of heavy chain CDRs derived from donor antibodies that immunospecifically bind to an antigen, the light chain variable region CDRs are derived from a donor antibody light chain variable region, at least one heavy chain framework region is from a sub-bank of human heavy chain framework regions and at least one light chain framework region is from a sub-bank of human light chain framework regions.
100. A population of cells comprising nucleic acid sequences comprising nucleotide sequences encoding a plurality of humanized heavy chain variable regions and a plurality of humanized light chain variable regions, said cells each produced by the process comprising introducing into cells nucleic acid sequences comprising: (i) a first set of nucleotide sequences encoding humanized heavy chain variable regions each synthesized by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, and (ii) a second set of nucleotide sequences encoding humanized light chain variable regions each synthesized by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the heavy chain variable region CDRs are derived from a donor antibody heavy chain variable region, at least one light chain variable region CDR is from a sub-bank of light chain CDRs derived from donor antibodies that immunospecifically bind to an antigen, at least one heavy chain framework region is from a sub-bank of human heavy chain framework regions and at least one light chain framework region is from a sub-bank of human light chain framework regions.
101. A population of cells comprising nucleic acid sequences comprising nucleotide sequences encoding a plurality of humanized heavy chain variable regions and a plurality of humanized light chain variable regions, said cells each produced by the process comprising introducing into cells nucleic acid sequences comprising: (i) a first set of nucleotide sequences encoding humanized heavy chain variable regions each synthesized by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, and (ii) a second set of nucleotide sequences encoding humanized light chain variable regions each synthesized by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein at least one heavy chain variable region CDR is from a sub-bank of heavy chain CDRs derived from donor antibodies that immunospecifically bind to an antigen, at least one light chain variable region CDR is from a sub-bank of light chain CDRs derived from donor antibodies that immunospecifically bind to an antigen, at least one heavy chain framework region is from a sub-bank of human heavy chain framework regions and at least one light chain framework region is from a sub-bank of human light chain framework regions.
102. A method of identifying a humanized antibody that immunospecifically binds to an antigen, said method comprising expressing the nucleic acid sequences in the cells of embodiment 94, 95, 96 or 97 and screening for a humanized antibody that has an affinity of 1 x 10⁶ M⁻¹ or above for said antigen.
103. A method of identifying a humanized antibody that immunospecifically binds to an antigen, said method comprising expressing the nucleic acid sequences in the cells of embodiment 98, 99, 100 or 101 and screening for a humanized antibody that has an affinity of 1 x 10⁶ M⁻¹ or above for said antigen.
104. A humanized antibody identified by the method of embodiment 102.
105. A humanized antibody identified by the method of embodiment 103.
106. A composition comprising the humanized antibody of embodiment 104, and a carrier, diluent or excipient.
107. A composition comprising the humanized antibody of embodiment 105, and a carrier, diluent or excipient.

### 5.0 EXAMPLE

### Reagents

All chemicals were of analytical grade. Restriction enzymes and DNA-modifying enzymes were purchased from New England Biolabs, Inc. (Beverly, MA). *pfu* DNA polymerase and oligonucleotides were purchased from Invitrogen (Carlsbad, CA). Human EphA2-Fc fusion protein (consisting of human EphA2 fused with the Fc portion of a human IgG1 (Carles-Kinch et al. Cancer Res. 62: 2840-2847 (2002)) was expressed in human embryonic kidney (HEK) 293 cells and purified by protein G affinity chromatography using standard protocols. Streptavidin magnetic beads were purchased from Dynal (Lake Success, NY). Human EphA2-Fc biotinylation was carried out using an EZ-Link Sulfo-NHS-LC-Biotinylation Kit according to the manufacturer's instructions (Pierce, Rockford, IL).

### 5.1- Cloning and sequencing of the parental monoclonal antibody

A murine hybridoma cell line (B233) secreting a monoclonal antibody (mAb) raised against the human receptor tyrosine kinase EphA2 (Kinch et al. Clin. Exp. Metastasis. 20:59-68 (2003)) was acquired by MedImmune, Inc. This mouse mAb is referred to as mAb B233 thereafter. Cloning and sequencing of the variable heavy (V_{H}) and light (V_{L}) genes of mAb B233 were carried out after isolation and purification of the messenger RNA from B233 using a Straight A's mRNA Purification kit (Novagen, Madison, WI) according to the manufacturer's instructions. cDNA was synthesized with a First Strand cDNA synthesis kit (Novagen, Madison, WI) as recommended by the manufacturer. Amplification of both V_{H} and V_{L} genes was carried out using the IgGV_{H} and IgxV_{L} oligonucleotides from the Mouse Ig-Primer Set (Novagen, Madison, WI) as suggested by the manufacturer. DNA fragments resulting from productive amplifications were cloned into pSTBlue-1 using the Perfectly Blunt Cloning Kit (Novagen, Madison, WI). Multiple V_{H} and V_{L} clones were then sequenced by the dideoxy chain termination method (Sanger et al., Proc. Natl. Acad. Sci. USA. 74: 5463-5467 (1977)) using a ABI3000 sequencer (Applied Biosystems, Foster City, CA). The consensus sequences of mAb B233 V_{L} (V_{L}-233) and V_{H} (V_{H}-233) genes are shown in Figure 1.

### 5.2- Selection of the human frameworks

Human framework genes were selected from the publicly available pool of antibody germline genes. More precisely, this included 46 human germline kappa chain genes (A1, A10, A11, A14, A17, A18, A19, A2, A20, A23, A26, A27, A3, A30, A5, A7, B2, B3, L1, L10, L11, L12, L14, L15, L16, L18, L19, L2, L20, L22, L23, L24, L25, L4/18a, L5, L6, L8, L9, O1, O11, O12, O14, O18, 02, 04 and 08; K.F. Schable, et al., Biol. Chem. Hoppe Seyler 374:1001-1022, (1993); J. Brensing-Kuppers, et al., Gene 191:173-181(1997)) for the 1^{st}, 2^{nd} and 3^{rd} frameworks and 5 human germline J sequences for the 4^{th} framework (Jκ1, Jκ2, Jκ3, Jκ4 and Jκ5; P.A. Hieter, et al., J. Biol. Chem. 257:1516-1522 (1982)). The heavy chain portion of the library included 44 human germline heavy chain sequences (VH1-18, VH1-2, VH1-24, VH1-3, VH1-45, VH1-46, VH1-58, VH1-69, VH1-8, VH2-26, VH2-5, VH2-70, VH3-11, VH3-13, VH3-15, VH3-16, VH3-20, VH3-21, VH3-23, VH3-30, VH3-33, VH3-35, VH3-38, VH3-43, VH3-48, VH3-49, VH3-53, VH3-64, VH3-66, VH3-7, VH3-72, VH3-73, VH3-74, VH3-9, VH4-28, VH4-31, VH4-34, VH4-39, VH4-4, VH4-59, VH4-61, VH5-51, VH6-1 and VH7-8; F. Matsuda, et al., J. Exp. Med. 188:1973-1975 (1998)) for the 1^{st}, 2^{nd} and 3^{rd} frameworks and 6 human germline J sequences for the 4^{th} framework (JH1, JH2, JH3, JH4, JH5 and JH6; J.V. Ravetch, et al., Cell 27(3 Pt 2): 583-591 (1981)).

### 5.3- Construction of the framework-shuffled libraries

### 5.3.1- Description of the libraries

Three main framework-shuffled libraries (library A, B and C) were constructed. Library A included a light chain framework shuffled sub-library (V_{L} sub1) paired with the heavy chain of mAb B233 (V_{H}-233). Library B included a heavy chain framework shuffled sub-library (V_{H} sub 1) paired with the fixed framework shuffled light chains V_{L}-12C8 and V_{L}-8G7 (see §5.4.1.1, §5.4.1.1.2 and §5.4.1.3). Library C included a light chain framework shuffled sub-library (V_{L} sub2) paired with a heavy chain framework shuffled sub-library (V_{H} sub2).

The construction of the framework shuffled V_{H} and V_{L} sub-libraries was carried out using the oligonucleotides shown in Tables 1-7 and 11. More precisely, the oligonucleotides described in Tables 1-7 and 11 encode the complete sequences of all known human framework germline genes for the light (κ) and heavy chains, Kabat definition. The oligonucleotides described in Tables 64 and 65 encode part of the CDRs of mAb B233 and are overlapping with the corresponding human germline frameworks. With respect to Table 64, with the exception of AL1-13 and DL1Ü-4Ü, each oligonucleotide encodes portions of one CDR of mAb B233 (underlined) and of one human germline light chain framework (Kabat definition; Kabat et al., Sequences of Proteins of Immunological Interest, U.S. Public Health Service, National Institutes of Health, Washington, DC, 1991). CDRL1, L2 and L3 are encoded by AL1Ü-10Ü/BL1-10, BL1Ü-16Ü/CL1-11 and CL1Ü-12Ü/DL1-4, respectively. Oligonucleotides AL1-13 contain a M13 gene 3 leader overlapping sequence (bold) and oligonucleotides DL1Ü-4Ü contain a Cκ overlapping sequence (bold). With respect to table 65, with the exception of AHl-10 and DH1Ü-3Ü, each oligonucleotide encodes portions of one CDR of mAb B233 (underlined) and of one human germline heavy chain framework (Kabat definition). CDRH1, H2 and H3 are encoded by AH1Ü-17Ü/BH1-17, BH1Ü-16Ü/CH1-15 and CH1Ü-13Ü/DH1-3, respectively. Oligonucleotides AH1-10 contain a M13 gene 3 leader overlapping sequence (bold) whereas oligonucleotides DH1Ü-3Ü contain a Cκ₁ overlapping sequence (bold). (K=G or T, M=A or C, R=A or G, S=C or G, W=A or T and Y=C or T).

**Table 64. Oligonucleotides used for the fusion of mAb B233 light chain CDRs with human germline light chain frameworks.**

| | | |
|---|---|---|
| 1589 | AL1 | 5'-**GGTCGTTCCATTTTACTCCCACTCC**GATGTTGTGATGACWCAGTCT-3' |
| 1590 | AL2 | 5'-**GGTCGTTCCATTTTACTCCCACTCC**GACATCCAGATGAYCCAGTCT-3' |
| 1591 | AL3 | 5'-**GGTCGTTCCATTTTACTCCCACTCC**GCCATCCAGWTGACCCAGTCT-3' |
| 1592 | AL4 | 5'**-GGTCGTTCCATTTTACTCCCACTCC**GAAATAGTGATGAYGCAGTCT-3' |
| 1593 | AL5 | 5'-**GGTCGTTCCATTTTACTCCCACTCC**GAAATTGTGTTGACRCAGTCT-3' |
| 1594 | AL6 | 5'-**GGTCGTTCCATTTTACTCCCACTCC**GAKATTGTGATGACCCAGACT-3' |
| 1595 | AL7 | 5'-**GGTCGTTCCATTTTACTCCCACTCC**GAAATTGTRMTGACWCAGTCT-3' |
| 1596 | AL8 | 5'-**GGTCGTTCCATTTTACTCCCACTCC**GAYATYGTGATGACYCAGTCT-3' |
| 1597 | AL9 | 5'-**GGTCGTTCCATTTTACTCCCACTCC**GAAACGACACTCACGCAGTCT-3' |
| 1598 | AL10 | 5'-**GGTCGTTCCATTTTACTCCCACTCC**GACATCCAGTTGACCCAGTCT-3' |
| 1599 | AL11 | 5'-**GGTCGTTCCATTTTACTCCCACTCC**AACATCCAGATGACCCAGTCT-3' |
| 1600 | AL12 | 5'-**GGTCGTTCCATTTTACTCCCACTCC**GCCATCCGGATGACCCAGTCT-3' |
| 1601 | AL13 | 5'-**GGTCGTTCCATTTTACTCCCACTCC**GTCATCTGGATGACCCAGTCT-3' |
| 1602 | AL1Ü | 5'-TAATACTTTGGCTGGCCCTGCAGGAGATGGAGGCCGGC-3' |
| 1603 | AL2Ü | 5'-TAATACTTTGGCTGGCCCTGCAGGAGAGGGTGRCTCTTTC-3' |
| 1604 | AL3Ü | 5'-TAATACTTTGGCTGGCCCTACAASTGATGGTGACTCTGTC-3' |
| 1605 | AL4Ü | 5'-TAATACTTTGGCTGGCCCTGAAGGAGATGGAGGCCGGCTG-3' |
| 1606 | AL5Ü | 5'-TAATACTTTGGCTGGCCCTGCAGGAGATGGAGGCCTGCTC-3' |
| 1607 | AL6Ü | 5'-TAATACTTTGGCTGGCCCTGCAGGAGATGTTGACTTTGTC-3' |
| 1608 | AL7Ü | 5'-TAATACTTTGGCTGGCCCTGCAGGTGATGGTGACTTTCTC-3' |
| 1609 | AL8Ü | 5'-TAATACTTTGGCTGGCCCTGCAGTTGATGGTGGCCCTCTC-3' |
| 1610 | AL9Ü | 5'-TAATACTTTGGCTGGCCCTGCAAGTGATGGTGACTCTGTC-3' |
| 1611 | AL10Ü | 5'-TAATACTTTGGCTGGCCCTGCAAATGATACTGACTCTGTC-3' |
| 1612 | BL1 | 5'-CCAGCCAAAGTATTAGCAACAACCTACACTGGYTTCAGCAGAGGCCAGGC-3' |
| 1613 | BL2 | 5'-CCAGCCAAAGTATTAGCAACAACCTACACTGGTACCTGCAGAAGCCAGGS-3' |
| 1614 | BL3 | 5'-CCAGCCAAAGTATTAGCAACAACCTACACTGGTATCRGCAGAAACCAGGG-3' |
| 1615 | BL4 | 5'-CCAGCCAAAGTATTAGCAACAACCTACACTGGTACCARCAGAAACCAGGA- 3 ' |
| 1616 | BL5 | 5'-CCAGCCAAAGTATTAGCAACAACCTACACTGGTACCARCAGAAACCTGGC-3' |
| 1617 | BL6 | 5'-CCAGCCAAAGTATTAGCAACAACCTACACTGGTAYCWGCAGAAACCWGGG-3' |
| 1618 | BL7 | 5'-CCAGCCAAAGTATTAGCAACAACCTACACTGGTATCAGCARAAACCWGGS-3' |
| 1619 | BL8 | 5'-CCAGCCAAAGTATTAGCAACAACCTACACTGGTAYCAGCARAAACCAG-3' |
| 1620 | BL9 | 5'-CCAGCCAAAGTATTAGCAACAACCTACACTGGTTTCTGCAGAAAGCCAGG-3' |
| 1621 | BL10 | 5'-CCAGCCAAAGTATTAGCAACAACCTACACTGGTTTCAGCAGAAACCAGGG-3' |
| 1622 | BL1Ü | 5'-GATGGACTGGAAAACATAATAGATCAGGAGCTGTGGAG-3' |
| 1623 | BL2Ü | 5'-GATGGACTGGAAAACATAATAGATCAGGAGCTTAGGRGC-3' |
| 1624 | BL3Ü | 5'-GATGGACTGGAAAACATAATAGATGAGGAGCCTGGGMGC-3' |
| 1625 | BL4Ü | 5'-GATGGACTGGAAAACATARTAGATCAGGMGCTTAGGGGC-3' |
| 1626 | BL5Ü | 5'-GATGGACTGGAAAACATAATAGATCAGGWGCTTAGGRAC-3' |
| 1627 | BL6Ü | 5'-GATGGACTGGAAAACATAATAGATGAAGAGCTTAGGGGC-3' |
| 1628 | BL7Ü | 5'-GATGGACTGGAAAACATAATAAATTAGGAGTCTTGGAGG-3' |
| 1629 | BL8Ü | 5'-GATGGACTGGAAAACATAGTAAATGAGCAGCTTAGGAGG-3' |
| 1630 | BL9Ü | 5'-GATGGACTGGAAAACATAATAGATCAGGAGTGTGGAGAC-3' |
| 1631 | BL10Ü | 5'-GATGGACTGGAAAACATAATAGATCAGGAGCTCAGGGGC-3' |
| 1632 | BL11Ü | 5'-GATGGACTGGAAAACATAATAGATCAGGGACTTAGGGGC-3' |
| 1633 | BL12Ü | 5'-GATGGACTGGAAAACATAATAGAGGAAGAGCTTAGGGGA-3' |
| 1634 | BL13Ü | 5'-GATGGACTGGAAAACATACTTGATGAGGAGC'T'TTGGAGA-3' |
| 1635 | BL14Ü | 5'-GATGGACTGGAAAACATAATAAATTAGGCGCCTTGGAGA-3' |
| 1636 | BL15Ü | 5'-GATGGACTGGAAAACATACTTGATGAGGAGCTTTGGGGC-3' |
| 1637 | BL16Ü | 5'-GATGGACTGGAAAACATATTGAATAATGAAAATAGCAGC-3' |
| 1638 | CL1 | 5'-GTTTTCCAGTCCATCTCTGGGGTCCCAGACAGATTCAGY-3' |
| 1639 | CL2 | 5'-GTTTTCCAGTCCATCTCTGGGGTCCCATCAAGGTTCAGY-3' |
| 1744 | CL3 | 5'-GTTTTCCAGTCCATCTCTGGYATCCCAGCCAGGTTCAGT-3' |
| 1745 | CL4 | 5'-GTTTTCCAGTCCATCTCTGGRGTCCCWGACAGGTTCAGT-3' |
| 1746 | CL5 | 5'-GTTTTCCAGTCCATCTCTAGCATCCCAGCCAGGTTCAGT-3' |
| 1747 | CL6 | 5'-GTTTTCCAGTCCATCTCTGGGGTCCCCTCGAGGTTCAGT-3' |
| 1748 | CL7 | 5'-GTTTTCCAGTCCATCTCTGGAATCCCACCTCGATTCAGT-3' |
| 1749 | CL8 | 5'-GTTTTCCAGTCCATCTCTGGGGTCCCTGACCGATTCAGT-3' |
| 1750 | CL9 | 5'-GTTTTCCAGTCCATCTCTGGCATCCCAGACAGGTTCAGT-3' |
| 1751 | CL10 | 5'-GTTTTCCAGTCCATCTCTGGGGTCTCATCGAGGTTCAGT-3' |
| 1752 | CL11 | 5'-GTTTTCCAGTCCATCTCTGGAGTGCCAGATAGGTTCAGT-3' |
| 1753 | CL1Ü | 5'-CCAGCTGTTACTCTGTTGKCAGTAATAAACCCCAACATC-3' |
| 1754 | CL2Ü | 5'-CCAGCTGTTACTCTGTTGACAGTAATAYGTTGCAGCATC-3' |
| 1755 | CL3Ü | 5'-CCAGCTGTTACTCTGTTGACMGTAATAAGTTGCAACATC-3' |
| 1756 | CL4Ü | 5'-CCAGCTGTTACTCTGTTGRCAGTAATAAGTTGCAAAATC-3' |
| 1757 | CL5Ü | 5'-CCAGCTGTTACTCTGTTGACAGTAATAARCTGCAAAATC-3' |
| 1758 | CL6Ü | 5°-CCAGCTGTTACTCTGTTGACARTAGTAAGTTGCAAAATC-3' |
| 1759 | CL7Ü | 5'-CCAGCTGTTACTCTGTTGGCAGTAATAAACTCCAAMATC-3' |
| 2760 | CL8Ü | 5'-CCAGCTGTTACTCTGTTGGCAGTAATAAACCCCGACATC-3' |
| 1761 | CL9Ü | 5'-CCAGCTGTTACTCTGTTGACAGAAGTAATATGCAGCATC-3' |
| 1762 | CL10Ü | 5'-CCAGCTGTTACTCTGTTGACAGTAATATGTTGCAATATC-3' |
| 1763 | CL11Ü | 5'-CCAGCTGTTACTCTGTTGACAGTAATACACTGCAAAATC-3' |
| 1764 | CL12Ü | 5'-CCAGCTGTTACTCTGTTGACAGTAATAAACTGCCACATC-3' |
| 1765 | DL1 | 5'-CAGAGTAACAGCTGGCCGCTCACGTTYGGCCARGGGACCAAGSTG-3' |
| 1766 | DL2 | 5'-CAGAGTAACAGCTGGCCGCTCACGTTCGGCCAAGGGACACGACTG-3' |
| 1767 | DL3 | 5'-CAGAGTAACAGCTGGCCGCTCACGTTCGGCCCTGGGACCAAAGTG-3' |
| 1768 | DL4 | 5'-CAGAGTAACAGCTGGCCGCTCACGTTCGGCGGAGGGACCAAGGTG-3' |
| 1769 | DL1Ü | 5'-**GATGAAGACAGATGGTGCAGCCACAGTACG**TTTGATYTCCACCTTGG-3' |
| 1770 | DL2Ü | 5'-**GATGAAGACAGATGGTGCAGCCACAGTACG**TTTGATCTCCAGCTTGG-3' |
| 1771 | DL3Ü | 5'-**GATGAAGACAGATGGTGCAGCCACAGTACG**TTTGATATCCACTTTGG-3' |
| 1772 | DL4Ü | 5'-**GATGAAGACAGATGGTGCAGCCACAGTACG**TTTAATCTCCAGTCGTG-3' |

**Table 65. Oligonuleotides used for the fusion of mAb B233 heavy chain CDRs with human germline light chain frameworks.**

| | | |
|---|---|---|
| 1640 | AH1 | 5'-**GCTGGTGGTGCCGTTCTATAGC**CATAGCCAGGTKCAGCTGGTGCAGTCT-3' |
| 1641 | AH2 | 5'-**GCTGGTGGTGCCGTTCTATAGC**CATAGCGAGGTGCAGCTGKTGGAGTCT-3' |
| 1642 | AH3 | 5'-**GCTGGTGGTGCCGTTCTATAGC**CATAGCCAGSTGCAGCTGCAGGAGTCG-3' |
| 1643 | AH4 | 5'-**GCTGGTGGTGCCGTTCTATAGC**CATAGCCAGGTCACCTTGARGGAGTCT-3' |
| 1644 | AH5 | 5'-**GCTGGTGGTGCCGTTCTATAGC**CATAGCCARATGCAGCTGGTGCAGTCT-3' |
| 1645 | AH6 | 5'-**GCTGGTGGTGCCGTTCTATAGCCATAGC**GARGTGCAGCTGGTGSAGTC-3' |
| 1646 | AH7 | 5'-**GCTGGTGGTGCCGTTCTATAGCCATAGC**CAGATCACCTTGAAGGAGTCT-3' |
| 1647 | AH8 | 5'-**GCTGGTGGTGCCGTTCTATAGC**CATAGCCAGGTSCAGCTGGTRSAGTCT-3' |
| 1648 | AH9 | 5'-**GCTGGTGGTGCCGTTCTATAGC**CATAGCCAGGTACAGCTGCAGCAGTCA-3' |
| 1649 | AH10 | 5'-**GCTGGTGGTGCCGTTCTATAGC**CATAGCCAGGTGCAGCTACAGCAGTGG-3' |
| 1650 | AH1Ü | 5'-GTTCATGGAGTAATCRGTGAAGGTGTATCCAGAAGC-3' |
| 1651 | AH2Ü | 5'-GTTCATGGAGTAATCGCTGAGTGAGAACCCAGAGAM-3' |
| 1652 | AH3Ü | 5'-GTTCATGGAGTAATCACTGAARGTGAATCCAGAGGC-3' |
| 1653 | AH4Ü | 5'-GTTCATGGAGTAATCACTGACGGTGAAYCCAGAGGC-3' |
| 1654 | AH5Ü | 5'-GTTCATGGAGTAATCGCTGAYGGAGCCACCAGAGAC-3' |
| 1655 | AH6Ü | 5'-GTTCATGGAGTAATCRGTAAAGGTGWAWCCAGAAGC-3' |
| 1656 | AH7Ü | 5'-GTTCATGGAGTAATCACTRAAGGTGAAYCCAGAGGC-3' |
| 1657 | AH8Ü | 5'-GTTCATGGAGTAATCGGTRAARCTGTAWCCAGAASC-3' |
| 1658 | AH9Ü | 5'-GTTCATGGAGTAATCAYCAAAGGTGAATCCAGARGC-3' |
| 1659 | AH10Ü | 5'-GTTCATGGAGTAATCRCTRAAGGTGAATCCAGASGC-3' |
| 1660 | AH11Ü | 5'-GTTCATGGAGTAATCGGTGAAGGTGTATCCRGAWGC-3' |
| 1661 | AH12Ü | 5'-GTTCATGGAGTAATCACTGAAGGACCCACCATAGAC-3' |
| 1662 | AH13Ü | 5'-GTTCATGGAGTAATCACTGATGGAGCCACCAGAGAC-3' |
| 1663 | AH14Ü | 5'-GTTCATGGAGTAATCGCTGATGGAGTAACCAGAGAC-3' |
| 1664 | AH15Ü | 5'-GTTCATGGAGTAATCAGTGAGGGTGTATCCGGAAAC-3' |
| 1665 | AH16Ü | 5'-GTTCATGGAGTAATCGCTGAAGGTGCCTCCAGAAGC-3' |
| 1666 | AH17Ü | 5'-GTTCATGGAGTAATCAGAGACACTGTCCCCGGAGAT-3' |
| 1667 | BH1 | 5'-GATTACTCCATGAACTGGGTGCGACAGGCYCCTGGA-3' |
| 1668 | BH2 | 5'-GATTACTCCATGAACTGGGTGCGMCAGGCCCCCGGA-3° |
| 1669 | BH3 | 5'-GATTACTCCATGAACTGGATCCGTCAGCCCCCAGGR-3' |
| 1670 | BH4 | 5'-GATTACTCCATGAACTGGRTCCGCCAGGCTCCAGGG-3' |
| 1671 | BH5 | 5'-GATTACTCCATGAACTGGATCCGSCAGCCCCCAGGG-3' |
| 1672 | BH6 | 5'-GATTACTCCATGAACTGGGTCCGSCAAGCTCCAGGG-3' |
| 1673 | BH7 | 5'-GATTACTCCATGAACTGGGTCCRTCARGCTCCRGGR-3' |
| 1674 | BH8 | 5'-GATTACTCCATGAACTGGGTSCGMCARGCYACWGGA-3' |
| 1675 | BH9 | 5'-GATTACTCCATGAACTGGKTCCGCCAGGCTCCAGGS-3' |
| 1676 | BH10 | 5'-GATTACTCCATGAACTGGATCAGGCAGTCCCCATCG-3' |
| 1677 | BH11 | 5'-GATTACTCCATGAACTGGGCCCGCAAGGCTCCAGGA-3' |
| 1678 | BH12 | 5'-GATTACTCCATGAACTGGATCCGCCAGCACCCAGGG-3' |
| 1679 | BH13 | 5'-GATTACTCCATGAACTGGGTCCGCCAGGCTTCCGGG-3' |
| 1680 | BH14 | 5'-GATTACTCCATGAACTGGGTGCGCCAGATGCCCGGG-3' |
| 1681 | BH15 | 5'-GATTACTCCATGAACTGGGTGCGACAGGCTCGTGGA-3' |
| 1682 | BH16 | 5'-GATTACTCCATGAACTGGATCCGGCAGCCCGCCGGG-3' |
| 1683 | BH17 | 5'-GATTACTCCATGAACTGGGTGCCACAGGCCCCTGGA-3' |
| 1684 | BH1U | 5'-TGTGTAATCATTAGCTTTGTTTCTAATAAATCCCATCCACTCAAGCCYTTG-3' |
| 1685 | BH2Ü | 5'-TGTTGTGTAATCATTAGCTTTGTTTCTAATAAATCCCATCCACTCAAGCSCTT-3' |
| 1686 | BH3Ü | 5'-TGTTGTGTAATCATTAGCTTTGTTTCTAATAAAWGAGACCCACTCCAGCCCCTT-3' |
| 1687 | BH4Ü | 5'-TGTTGTGTAATCATTAGCTTTGTTTCTAATAAACCCAATCCACTCCAGKCCCTT-3' |
| 1688 | BH5Ü | 5'-TGTTGTGTAATCATTAGCTTTGTTTCTAATAAATGAGACCCACTCCAGRCCCTT-3' |
| 1689 | BH6Ü | 5'-TGTTGTGTAATCATTAGCTTTGTTTCTAATAAAGCCAACCCACTCCAGCCCYTT-3' |
| 1690 | BH7Ü | 5'-TGTTGTGTAATCATTAGCTTTGTTTCTAATAAAKGCCACCCACTCCAGCCCCTT-3' |
| 1691 | BH8Ü | 5'-TGTTGTGTAATCATTAGCTTTGTTTCTAATAAATCCCAGCCACTCAAGGCCTC-3' |
| 1692 | BH9Ü | 5'-TGTTGTGTAATCATTAGCTTTGTTTCTAATAAACCCCATCCACTCCAGGCCTT-3' |
| 1693 | BH10Ü | 5'-TGTTGTGTAATCATTAGCTTTGTTTCTAATAAATGARACCCACWCCAGCCCCTT-3' |
| 1694 | BH11Ü | 5'-TGTTGTGTAATCATTAGCTTTGTTTCTAATAAAMGAKACCCACTCCAGMCCCTT-3' |
| 1695 | BH12Ü | 5'-TGTTGTGTAATCATTAGCTTTGTTTCTAATAAAYCCMATCCACTCMAGCCCYTT-3' |
| 1696 | 1BH13Ü | 5'-TGTTGTGTAATCATTAGCTTTGTTTCTAATAAATCCTATCCACTCAAGGCGTTG-3' |
| 1697 | BH14Ü | 5'-TGTTGTGTAATCATTAGCTTTGTTTCTAATAAATGCAAGCCACTCCAGGGCCTT-3' |
| 1698 | BH15Ü | 5'-TGTTGTGTAATCATTAGCTTTGTTTCTAATAAATGAAACATATTCCAGTCCCTT-3' |
| 1699 | BH16Ü | 5'-TGTTGTGTAATCATTAGCTTTGTTTCTAATAAACGATACCCACTCCAGCCCCTT-3' |
| 1700 | CH1 | 5'-GCTAATGATTACACAACAGAGTACAGTGCATCTGTGAAGGGTAGAGTCACCATGACCAGGRAC-3' |
| 1701 | CH2 | 5'-GCTAATGATTACACAACAGAGTACAGTGCATCTGTGAAGGGTAGGCTCACCATCWCCAAGGAC-3' |
| 1702 | CH3 | 5'-GCTAATGATTACACAACAGAGTACAGTGCATCTGTGAAGGGTCGAGTYACCATATCAGTAGAC-3' |
| 1703 | CH4 | 5'-GCTAATGATTACACAACAGAGTACAGTGCATCTGTGAAGGGTCGATTCACCATCTCCAGRGAC-3' |
| 1704 | CH5 | 5'-GCTAATGATTACACAACAGAGTACAGTGCATCTGTGAAGGGTAGATTCACCATCTCMAGAGA-3' |
| 1705 | CH6 | 5'-GCTAATGATTACACAACAGAGTACAGTGCATCTGTGAAGGGTMGGTTCACCATCTCCAGAGA-3' |
| 1706 | CH7 | 5'-GCTAATGATTACACAACAGAGTACAGTGCATCTGTGAAGGGTCGATTCAYCATCTCCAGAGA-3' |
| 1707 | CH8 | 5'-GCTAATGATTACACAACAGAGTACAGTGCATCTGTGAAGGGTCGAGTCACCATRTCMGTAGAC-3' |
| 1708 | CH9 | 5'-GCTAATGATTACACAACAGAGTACAGTGCATCTGTGAAGGGTAGRGTCACCATKACCAGGGAC-3' |
| 1709 | CH10 | 5'-GCTAATGATTACACAACAGAGTACAGTGCATCTGTGAAGGGTCAGGTCACCATCTCAGCCGAC-3' |
| 1710 | CH11 | 5'-GCTAATGATTACACAACAGAGTACAGTGCATCTGTGAAGGGTCGAATAACCATCAACCCAGAC-3' |
| 1711 | CH12 | 5'-CTAATGATTACACAACAGAGTACAGTGCATCTGTGAAGGGTCGGTTTGTCTTCTCCATGGAC-3' |
| 1712 | CH13 | 5'-GCTAATGATTACACAACAGAGTACAGTGCATCTGTGAAGGGTAGAGTCACCATGACCGAGGAC-3' |
| 1713 | CH14 | 5'-GCTAATGATTACACAACAGAGTACAGTGCATCTGTGAAGGGTAGAGTCACGATTACCGCGGAC-3' |
| 1714 | CH15 | 5'-GCTAATGATTACACAACAGAGTACAGTGCATCTGTGAAGGGTAGAGTCACCATGACCACAGAC-3' |
| 1715 | CH1U | 5'-GTCCATAGCATGATACCTAGGGTATCTAGYACAGTAATACACGGC-3' |
| 1716 | CH2Ü | 5'-GTCCATAGCATGATACCTAGGGTATCTCGCACAGTAATACAYGGC-3' |
| 1717 | CH3Ü | 5'-GTCCATAGCATGATACCTAGGGTATCTYGCACAGTAATACACAGC-3' |
| 1718 | CH4Ü | 5'-GTCCATAGCATGATACCTAGGGTATGYYGCACAGTAATACACGGC-3' |
| 1719 | CH5Ü | 5'-GTCCATAGCATGATACCTAGGGTACCGTGCACARTAATAYGTGGC-3' |
| 1720 | CH6Ü | 5'-GTCCATAGCATGATACCTAGGGTATCTGGCACAGTAATACACGGC-3' |
| 1721 | CH7Ü | 5'-GTCCATAGCATGATACCTAGGGTATGTGGTACAGTAATACACGGC-3' |
| 1722 | CH8Ü | 5'-GTCCATAGCATGATACCTAGGGTATCTCGCACAGTGATACAAGGC-3' |
| 1723 | CH9Ü | 5'-GTCCATAGCATGATACCTAGGGTATTTTGCACAGTAATACAAGGC-3' |
| 1724 | CH10Ü | 5'-GTCCATAGCATGATACCTAGGGTATCTTGCACAGTAATACATGGC-3' |
| 1725 | CH11Ü | 5'-GTCCATAGCATGATACCTAGGGTAGTGTGCACAGTAATATGTGGC-3' |
| 1726 | CH12Ü | 5'-GTCCATAGCATGATACCTAGGGTATTTCGCACAGTAATATACGGC-3' |
| 1727 | CH13Ü | 5'-GTCCATAGCATGATACCTAGGGTATCTCACACAGTAATACACAGC-3' |
| 1728 | DH1 | 5'-CCTAGGTATCATGCTATGGACTCCTGGGGCCARGGMACCCTGGTC-3' |
| 1729 | DH2 | 5'-CCTAGGTATCATGCTATGGACTCCTGGGGSCAAGGGACMAYGGTC-3' |
| 1730 | DH3 | 5'-CCTAGGTATCATGCTATGGACTCCTGGGGCCGTGGCACCCTGGTC-3' |
| 1731 | DH1Ü | 5'-**GGAAGACCGATGGGCCCTTGGTGGAGGC**TGAGGAGACRGTGACCAGGGT-3' |
| 1732 | DH2Ü | 5'-**GGAAGACCGATGGGCCCTTGGTGGAGGC**TGARGAGACGGTGACCRTKGT-3' |
| 1733 | DH3Ü | 5'-**GGAAGACCGATGGGCCCTTGGTGGAGGC**TGAGGAGACGGTGACCAGGGT-3' |

### 5.3.2- Construction of the V_{H} and V_{L} sub-libraries

V_{L} sub 1 sub-library was assembled sequentially using the polymerase chain reaction (PCR) by overlap extension. Ho et al., Gene 77:51-59 (1989). More precisely, so-called "intermediate" PCRs were carried out to synthesize each individual human germline framework fused in frame with a portion of the corresponding donor CDRs using the following oligonucleotide combinations: AL1-13/AL1Ü-10Ü/1-46, BL1-10/BL1Ü-16Ü/47-92, CL1-11/CL1Ü-12Ü/93-138 and DL1-4/DL1Ü-4Ü/139-143 for the 1^{st}, 2^{nd}, 3^{rd} and 4^{th} frameworks, respectively. This was carried out using *pfu* DNA polymerase (PCR SuperMix, Invitrogen) in 100 µl volume and approximately 5 pmol of oligonucleotides AL1-13, AL1Ü-10Ü, BL1-10, BL1Ü-16Ü, CL1-11, CL1Ü-12Ü, DL1-4 and DL1Ü-4Ü and approximately 100 pmol of oligonucleotides 1-143. The PCR program consisted of 5 min at 95°C; 1 min at 94°C, 1 min at 45°C, 1 min at 72°C for 30 cycles then 8 min at 72°C. A second PCR ("assembly PCR") was then carried out using *pfu* DNA polymerase (PCR SuperMix, Invitrogen), 0.5-2 µl of each of the "intermediate" PCRs, 25 pmol of each of the oligonucleotides DL1Ü, DL2Ü, DL3Ü, DL4Ü (see Table 64) and 100 pmol of the biotinylated oligonucleotide 5'-GGTCGTTCCATTTTACTCCCAC-3' (SEQ ID NO. 1734) in a 100 µl reaction volume. The assembly PCR program consisted of 5 min at 95°C; 30 s at 94°C, 30 s at 50°C, 45 s at 72°C for 30 cycles then 8 min at 72°C.

V_{H} sub1, V_{H} sub2 and V_{L} sub2 framework-shuffled sub-libraries were also synthesized using the PCR by overlap extension. Ho et al., Gene 77:51-59 (1989). This total *in vitro* synthesis of the framework shuffled V_{H} and V_{L} genes was done essentially as described H. Wu et al., Methods Mol. Biol. 207: 213-233 (2003). Briefly, a first so-called "fusion PCR" was carried out using *pfu* DNA polymerase (PCR SuperMix, Invitrogen). Construction of V_{H} sub1 was carried out using approximately 3-10 pmol of each of the oligonucleotides described in Tables 5, 6, 7, 11 and 65 in a 100 µl reaction volume. Construction of V_{H} sub2 was carried out using approximately 0.5 pmol of each of the oligonucleotides described in Tables 5, 6, 7, 11 and 65 in a 100 µl reaction volume. Construction of V_{L} sub2 was carried out using approximately 0.5 pmol of each of the oligonucleotides described in Tables 1, 2, 3, 4, and 64 in a 100 µl reaction volume. For each V_{H} sub1, V_{H} sub2 and V_{L} sub2 sub-library, the fusion PCR program consisted of 1 min at 95°C; 20 s at , 94°C, 30 s at 50°C, 30 s at 72°C for 5 cycles; 20 s at 94°C, 30 s at 55°C, 30 s at 72°C for 25 cycles then 7 min at 72°C. A second so-called "synthesis PCR" then followed. More precisely, V_{H} sub1 and V_{H} sub2 sub-libraries were synthesized using *pfu* DNA polymerase (PCR SuperMix, Invitrogen), 2-3 µl of the corresponding "fusion PCR", 30 pmol of each of the oligonucleotides DH1Ü, DH2Ü, DH3Ü (see Table 65) and 100 pmol of the biotinylated oligonucleotide 5'-GCTGGTGGTGCCGTTCTATAGCC-3' (SEQ ID NO. 1735) in a 100 µl reaction volume. V_{L} sub2 sub-library was synthesized using *pfu* DNA polymerase (PCR SuperMix, Invitrogen), 3 µl of the corresponding "fusion PCR", 25 pmol of each of the oligonucleotides DL1Ü, DL2Ü, DL3Ü, DL4Ü (see Table 64) and 100 pmol of the biotinylated oligonucleotide 5'-GGTCGTTCCATTTTACTCCCAC-3' (SEQ ID NO. 1734) in a 100 µl reaction volume. For each V_{H} sub1, V_{H} sub2 and V_{L} sub2 sub-library, the synthesis PCR program consisted of 5 min at 94°c; 1 min at 94°C, 1 min at 45°C, 1 min at 72°C for 30 cycles then 8 min at 72°C.

### 5.3.3- Synthesis of the V_{L} -12C8 and V_{L}-8G7 genes

V_{L}-12C8 and V_{L}-8G7 light chain genes, used in the context of library B (V_{L}-12C8+VL-8G7+V_{H} sub1), were synthesized by PCR from the corresponding V region-encoding M13 phage vector (see §§ 5.4.1.1, 5.4.1.2, 5.4.1.3) using the 12C8for/12C8back and 8G7for/8G7back oligonucleotide combinations, respectively (see below).

12C8for 5’-GGTCGTTCCATTTTACTCCCACTCCGCCATCCAGTTGACTCAGTCTCC-
3’(biotinylated) (SEQ ID NO. 1736)

12C8back 5'-
GATGAAGACAGATGGTGCAGCCACAGTACGTTTGATCTCCAGCTTGGTCCCTCC-3'
(SEQ ID NO. 1737)

8G7for 5'-GGTCGTTCCATTTTACTCCCACTCCGAAATTGTGTTGACACAGTCTCCAG-
3' (biotinylated) (SEQ ID NO. 1738)

8G7back 5'-
GATGAAGACAGATGGTGCAGCCACAGTACGTTTGATATCCACTTTGGTCCCTC-3' (SEQ
ID NO. 1739).

Oligonucleotides 12C8 for and 8G7 for contain a M13 gene 3 leader overlapping sequence (bold). Oligonucleotides 8G7back and 12C8back contain a Cκ overlapping sequence (underlined).

### 5.3.4- Synthesis of the V_{H}-233 and V_{L}-233 genes

V_{H}-233 and V_{L}-233 heavy and light chain genes, used in the context of a chimaeric Fab positive control (V_{H}-233+V_{L}-233) or of library A (V_{L} sub1+V_{H}-233), were synthesized by PCR from the corresponding pSTBlue-1 (see §5.1)vector using the 233Hfor/233Hback and 233Lfor/233Lback oligonucleotide combinations, respectively (see below).

233Hfor 5'-
GCTGGTGGTGCCGTTCTATAGCCATAGCGAGGTGAAGCTGGTGGAGTCTGGAGGAG-
3' (biotinylated) (SEQ ID NO. 1740)

233Hback 5'-
GGAAGACCGATGGGCCCTTGGTGGAGGCTGAGGAGACGGTGACTGAGGTTCCTTG-3'
(SEQ ID NO. 1741)

233Lfor 5'-
GGTCGTTCCATTTTACTCCCACTCCGATATTGTGCTAACTCAGTCTCCAGCCACCCTG
-3' (biotinylated) (SEQ ID NO. 1742)

233Lback 5'-
GATGAAGACAGATGGTGCAGCCACAGTACGTTTCAGCTCCAGCTTGGTCCCAGCACCG
AACG-3' (SEQ ID NO. 1743)

Oligonucleotides 233Hfor and 233Lfor contain a M13 gene 3 leader overlapping sequence (bold). Oligonucleotide 233Hback contains a Cκ₁ overlapping sequence (underlined). Oligonucleotide 233Lback contains a Cκ overlapping sequence (underlined).

### 5.3.5- Cloning of the various V regions into a phage expression vector

Libraries A, B and C as well as the chimaeric Fab version of mAb B233 were cloned into a M13-based phage expression vector. This vector allows the expression of Fab fragments that contain the first constant domain of a human γ1 heavy chain and the constant domain of a human kappa (κ) light chain under the control of the *lacZ* promoter (Figure 2). The cloning was carried out by hybridization mutagenesis, Kunkel et al., Methods Enzymol. 154:367-382 (1987), as described Wu et al., Methods Mol. Biol. 207: 213-233 (2003). Briefly, minus single-stranded DNA corresponding to the various V regions of interest (see §5.3.2, §5.3.3 and §5.3.4) was purified from the final PCR products by ethanol precipitation after dissociation of the double-stranded PCR product using sodium hydroxide and elimination of the biotinylated strand by streptavidin-coated magnetic beads as described (H. Wu, et al., Methods Mol. Biol. 207: 213-233(2003); H. Wu, Methods Mol. Biol. 207: 197-212 (2003)). Equimolar amounts of different minus strands were mixed as follows: V_{H}-233/V_{L}sub1,V_{H} subl/V_{L}-8G7/V_{L}-12C8, V_{H} sub2/V_{L} sub2 and V_{H}-233 /V_{L}-233 to construct library A, library B, library C and chimaeric Fab 233, respectively. These different mixes were then individually annealed to two regions of the vector containing each one palindromic loop. Those loops contained a unique XbaI site which allows for the selection of the vectors that contain both V_{L} and V_{H} chains fused in frame with the human kappa (κ) constant and first human γ constant regions, respectively. Synthesized DNA was then electroporated into XL1-Blue for plaque formation on XLl-Blue bacterial lawn or production of Fab fragments as described Wu et al., Methods Mol. Biol. 207: 213-233 (2003).

### 5.4- Screening of the libraries

### 5.4.1- Primary screen

### 5.4.1.1- Description

The primary screen consisted of a single point ELISA (SPE) which was carried out using periplasmic extracts prepared from 1 ml-bacterial culture grown in 96 deep-well plates and infected with individual recombinant M13 clones (see §5.3.5) essentially as described in Wu et al., Methods Mol. Biol. 207: 213-233 (2003). Briefly, individual wells of a 96-well Maxisorp Immunoplate were coated with 20-500 ng of a goat anti-human Fab antibody, blocked with 3% BSA/PBS for 2h at 37°C and incubated with samples (periplasm-expressed Fabs) for 1h at room temperature. 300 ng/well of biotinylated human EphA2-Fc was then added for 1h at room temperature. This was followed by incubation with neutravidin-horseradish peroxydase (HRP) conjugate for 40 min at room temperature. HRP activity was detected with tetra methyl benzidine (TMB) substrate and the reaction quenched with 0.2 M H₂SO₄. Plates were read at 450 nm.

### 5.4.1.2- Results of the primary screen

Out of ∼ 500 clones from library A that were screened using 100 ng of the goat anti-human Fab capture reagent, 14 exhibited a significant signal (OD₄₅₀ ranging from 0.2-0.6). This typically corresponded to a signal at least 1.3-fold above the corresponding background signal (OD₄₅₀ ranged from 0.1-0.4) of an irrelevant antibody (MEDI-493; S. Johnson et al., J. Infect. Dis. 176: 1215-1224 (1997)). Under these conditions, Fab 233 exhibited an OD₄₅₀ ranging from 0.4-0.6.

Out of ∼ 200 clones from library A that were screened using 20 ng of the goat anti-human Fab capture reagent, 4 exhibited a significant signal (OD₄₅₀ ranging from 0.2-0.4). This typically corresponded to a signal at least 2-fold above the corresponding background signal of an irrelevant antibody (OD₄₅₀ of 0.1). Under these conditions, Fab 233 exhibited an OD₄₅₀ ranging from 0.2-0.3.

Out of ∼ 750 clones from library A that were screened using 500 ng of the goat anti-human Fab capture reagent, 16 exhibited a significant signal (OD₄₅₀ ranging from 0.1-0.7). This typically corresponded to a signal at least 1.3-fold above the corresponding background signal of an irrelevant antibody (OD₄₅₀ ranged from 0.06-0.2). Under these conditions, Fab 233 exhibited an OD₄₅₀ ranging from 0.1-0.6. Clones V_{H}-233/V_{L}-12C8 and V_{H}-233/V_{L}-8G7 were isolated from this round of screening and both exhibited an OD₄₅₀ of 0.4 (same plate background OD₄₅₀ values were 0.1 and 0.2, respectively; same plate Fab 233 OD₄₅₀ values were 0.2 and 0.5, respectively).

Out of ∼ 750 clones from library B that were screened using 500 ng of the goat anti-human Fab capture reagent, 27 exhibited a significant signal (OD₄₅₀ ranging from 0.3-2.8). This typically corresponded to a signal at least 1.3-fold above the corresponding background signal of an irrelevant antibody (OD₄₅₀ ranged from 0.2-0.3). Under these conditions, both V_{H}-233/V_{L}-12C8 and V_{H}-233/V_{L}-8G7 exhibited OD₄₅₀ values ranging from 0.2-0.4. Clones V_{H}-2G6/V_{L}-12C8, V_{H}-6H11/V_{L}-8G7 and V_{H}-7E8/V_{L}-8G7 were isolated from this round of screening and exhibited an OD₄₅₀ of 2.8, 2.5 and 1.6, respectively (same plate background OD₄₅₀ values were 0.3, 0.2 and 0.2, respectively; same plate V_{H}-233/V_{L}-12C8 OD₄₅₀ values were 0.4, 0.3 and 0.3, respectively; same plate V_{H}-233/V_{L}-8G7 OD₄₅₀ values were 0.4, 0.3 and 0.3, respectively).

Out of ∼ 1150 clones from library C that were screened using 500 ng of the goat anti-human Fab capture reagent, 36 exhibited a significant signal (OD₄₅₀ ranging from 0.1-0.3). This typically corresponded to a signal at least 1.3-fold above the corresponding background signal of an irrelevant antibody (OD₄₅₀ ranged from 0.07-0.1). Under these conditions, Fab 233 exhibited an OD₄₅₀ ranging from 0.1-0.2.

### 5.4.1.3- Validation of the positive clones

Altogether, 9 clones from library A, 7 clones from library B and 0 clone from library C were re-confirmed in a second, independent, single point ELISA using periplasmic extracts prepared from 15 ml-bacterial culture and 500 ng of the goat anti-human Fab capture reagent. Specifically, two clones from library A (V_{H}-233/V_{L}-12C8 and V_{H}-233/V_{L}-8G7) that exhibited amongst the highest [specific OD₄₅₀/background OD_{450]} ratio (ranging from approximately 15-50) were further characterized by dideoxynucleotide sequencing using a ABI3000 genomic analyzer. DNA sequence analysis of clone V_{H}-233/V_{L}-12C8 revealed that its heavy chain contained a single base substitution at base 104 resulting in a substitution (N to S) at position H35 (Kabat numbering). This mutation was corrected using the QuickChange XL site-directed mutagenesis Kit (Stratagene, La Jolla, CA) according to the manufacturer's instructions. Corrected clone V_{H}-233/V_{L}-12C8 exhibited a [specific OD₄₅₀/background OD₄₅₀] ratio up to approximately 50 (similar to mutated V_{H}-233/V_{L}-12C8) which indicated retention of binding to EphA2-Fc. Partially humanized clones V_{H}-233/V_{L}-12C8 and V_{H}-233/V_{L}-8G7 were then selected for further characterization by a secondary screen (see §5.4.2). The sequences of V_{L}-12C8 and V_{L}-8G7 are indicated in Figure 3. As mentioned above, these two humanized light chains were then included in the design of Library B. Three clones from this library that exhibited amongst the highest [specific OD₄₅₀/background OD₄₅₀] ratio (approximately 40) were further characterized by dideoxynucleotide sequencing. This lead to the identification of three different humanized heavy chains (V_{H}-2G6, V_{H}-6H11 and 7E8; see Figure 3). V_{H}-2G6, V_{H}-6H11 and V_{H}-7E8 were found to be paired with V_{L}-12C8, V_{L}-8G7 and V_{L}-8G7, respectively. These three fully humanized clones were then selected for further characterization by a secondary screen (see §5.4.2).

### 5.4.2- Secondary screen

### 5.4.2.1- Description

In order to further characterize the previously identified humanized clones (see §5.4.1.3), a secondary screen using Fab fragments expressed in periplasmic extracts prepared from 15 ml-bacterial culture was carried out. More precisely, two ELISAs were used: (i) a functional ELISA in which individual wells of a 96-well Maxisorp Immunoplate were coated with 500 ng of human EphA2-Fc and blocked with 3%BSA/PBS for 2h at 37°C. 2-fold serially diluted samples were then added and incubated for 1h at room temperature. Incubation with a goat anti-human kappa horseradish peroxydase (HRP) conjugate then followed. HRP activity was detected with TMB substrate and the reaction quenched with 0.2 M H₂SO₄. Plates were read at 450 nm; (ii) an anti-human Fab quantification ELISA which was carried out essentially as described. Wu et al., Methods Mol. Biol. 207: 213-233 (2003). Briefly, individual wells of a 96-well Immulon Immunoplate were coated with 100 ng of a goat anti-human Fab antibody and then incubated with 2-fold serially diluted samples (starting at a 1/25 dilution) or standard (human IgG Fab, 500-3.91 ng/ml). Incubation with a goat anti-human kappa horseradish peroxydase (HRP) conjugate then followed. HRP activity was detected with TMB substrate and the reaction quenched with 0.2 M H₂SO₄. Plates were read at 450 nm.

### 5.4.2.2- Results of the secondary screen

The two-part secondary ELISA screen allowed us to compare Fab clones V_{H}-233/V_{L}-12C8, V_{H}-233/V_{L}-8G7, V_{H}-2G6/V_{L}-12C8, V_{H}-6H11/V_{L}-8G7 and V_{H}-7E8/V_{L}-8G7 to each other and to the chimaeric Fab of mAb B233 (V_{H}-233/V_{L}-233) in terms of binding to human EphA2. As shown in Figure 4, all framework shuffled Fabs retain binding to human EphA2 as compared with the chimaeric Fab of mAb B233. Interestingly, some clones whose heavy and light chains are both humanized (V_{H}-2G6/V_{L}-12C8 and V_{H}-7E8/V_{L}-8G7) exhibit better apparent binding to human EphA2-Fc than clones in which only the same light chains are humanized (V_{H}-233/V_{L}-12C8 and V_{H}-233/V_{L}-8G7). This indicates the existence of a process whereby humanized heavy chains are specifically selected for optimal binding to the antigen in the context of a given humanized light chain. In order to further characterize the different fully humanized molecules, clones V_{H}-2G6/V_{L}-12C8, V_{H}-6H11/V_{L}-8G7 and V_{H}-7E8/V_{L}-8G7 as well as the chimaeric form of mAb B233 (V_{H}-233/V_{L}-233) were then cloned and expressed as a full length human IgG1 (see § 5.5).

### 5.5 Cloning, expression and purification of the various humanized versions of mAb B233 in a human IgG1 format

The variable regions of framework shuffled clones V_{H}-2G6, V_{H}-6H11, V_{H}-7E8, V_{L}-12C8 and V_{L}-8G7 and of V_{H}-233 and V_{L}-233 were PCR-amplified from the corresponding V region-encoding M13 phage vectors using *pfu* DNA polymerase. They were then individually cloned into mammalian expression vectors encoding a human cytomegalovirus major immediate early (hCMVie) enhancer, promoter and 5'-untranslated region. M. Boshart, et al., Cell 41:521-530 (1985). In this system, a human γ chain is secreted along with a human κ chain. S. Johnson, et al., Infect. Dis. 176:1215-1224 (1997). The different constructs were expressed transiently in human embryonic kidney (HEK) 293 cells and harvested 72 hours post-transfection. The secreted, soluble human IgG1s were purified from the conditioned media directly on 1 ml HiTrap protein A or protein G columns according to the manufacturer's instructions (APBiotech, Inc., Piscataway, NJ). Purified human IgG1s (typically >95%homogeneity, as judged by SDS-PAGE) were recovered in yields varying from 2-13 µg/ml conditioned media, dialyzed against phosphate buffered saline (PBS), flash frozen and stored at -70°C.

### 5.6 BIAcore analysis of the binding of framework-shuffled, chimaeric and mAb B233 IgGs to EphA2-Fc

The interaction of soluble V_{H}-2G6/V_{L}-12C8, V_{H}-6H11/V_{L}-8G7, V_{H}-7E8/V_{L}-8G7 and V_{H}-233/V_{L}-233 IgGs as well as of mAb B233 with immobilized EphA2-Fc was monitored by surface plasmon resonance detection using a BIAcore 3000 instrument (Pharmacia Biosensor, Uppsala, Sweden). EphA2-Fc was coupled to the dextran matrix of a CM5 sensor chip (Pharmacia Biosensor) using an Amine Coupling Kit as described (B. Johnsson et al., Anal. Biochem. 198: 268-277 (1991)) at a surface density of between 105 and 160 RU. IgGs were diluted in 0.01 M HEPES pH 7.4 containing 0.15 M NaCl, 3 mM EDTA and 0.005% P20. All subsequent dilutions were made in the same buffer. All binding experiments were performed at 25°C with IgG concentrations typically ranging from 100 nM to 0.2 nM at a flow rate of 75 µL/min; data were collected for approximately 25 min and one 1-min pulse of 1M NaCl, 50 mM NaOH was used to regenerate the surfaces. IgGs were also flowed over an uncoated cell and the sensorgrams from these blank runs subtracted from those obtained with EphA2-Fc-coupled chips. Data were fitted to a 1:1 Langmuir binding model. This algorithm calculates both the *kₒₙ* and the *k_{off},* from which the apparent equilibrium dissociation constant, K_{D}, is deduced as the ratio of the two rate constants (*k_{off}*/ *kₒₙ*). The values obtained are indicated in Table 66.

**Table 66. Affinity measurements for the binding of different IgGs to human EphA2-Fc^{a}**

| **Antibody** | Association rate (*kₒₙ*)^{b} | Dissociation rate (*k_{off}*)^{b} | Dissociation |
|---|---|---|---|
| Constant (*K_{D}*)^{c} | (M⁻¹.s⁻¹) | (s⁻¹) | (nM) |
| B233 (murine) | 2.8 x 10⁵ | 1.1 x 10⁻⁴ | 0.4V_{H}- |
| B233N_{L}-B233 (chimaeric) | 2.4 x 10⁵ | 8.0 x 10⁻⁵ | 0.3 |
| V_{H}-2G6/V_{L}-12C8 (humanized) | 6.4 x 10⁴ | 1.9 x 10⁻⁴ | 3.0 |
| V_{H}-6H11/V_{L}-8G7 (humanized) | 9.6 x 10⁴ | 1.8 x 10⁻⁴ | 1.9 |
| Vₙ-7E8/V_{L}-8G7 (humanized) | 9.3 x 10³ | 4.5 x 10⁻⁴ | 48 |
| ^{a}Affinity measurements were carried out by BIAcore as reported in Description of Method. ^{b}Kinetic parameters represent the average of 5-18 individual measurements. ^{c}*K_{D}* was calculated as a ration of the rate constants (*k_{off}*/*kₒₙ*). | | | |

### 5.7- Analysis of the framework-shuffled variants

### 5.7.1- Sequence analysis

Overall, two unique humanized light chains (V_{L}-12C8 and V_{L}-8G7) and three unique humanized heavy chains (V_{H}-2G6, V_{H}-6H11 and V_{H}-7E8) were found that supported efficient binding to human EphA2-Fc. The promiscuous nature of humanized light chain V_{L}-8G7 is highlighted by its ability to mediate productive binding in the context of two different heavy chains (V_{H}-7E8 and V_{H}-6H11). All of these humanized variants exhibited a high level of global amino acid identity to mAb B233 in the corresponding framework regions, ranging from 76-83% for the heavy chains and from 64-69% for the light chains (Figure 5). This can be explained by the fact that high-homology human frameworks are more likely to retain parental key residues. Analysis of individual frameworks revealed a wider range of differences, ranging from 48% for the first framework of V_{L}-12C8 to 91% for the fourth framework of V_{H}-2G6, V_{H}-6H11 and V_{H}-7E8.

Interestingly, humanized heavy chain V_{H}-7E8 consisted exclusively of human frameworks that were a perfect match with human framework germline sequences (Figure 5). Humanized heavy chains V_{H}-6H11 and V_{H}-2G6 contained one and two human frameworks, respectively, that exhibited a near-perfect match with the most related human framework germline sequences (Figure 5). The differences amounted to a maximum of three residues per chain (V_{H}-2G6) and two residues per framework (first framework of V_{H}-2G6). In no cases did these differences encode amino acids not found in other most distant human framework germline sequences. Thus, arguably, these clones may also be referred to as "fully humanized". Humanized light chains V_{L}-12C8 and V_{L}-8G7 contained one and three human frameworks, respectively, that exhibited a near-perfect match with the most related human framework germline sequences (Figure 5). The number of differences amounted to a maximum of three residues per chain (V_{L}-8G7) and one residue per framework (first, second and fourth framework of V_{L}-8G7; fourth framework of V_{L}-12C8). However, here again, the residues at these positions were also found in other, less homologous human framework sequences; therefore these variants may also be referred to as fully humanized. Since these differences were not built-in within our libraries, we attribute their origin to a combination of factors such as PCR fidelity and/or oligonucleotides quality.

### 5.7.2- Binding analysis

It is worth nothing that only a two-step humanization process in which the light and heavy chains of mAb B233 were successively humanized (Library A and B) allowed us to isolate humanized clones retaining binding to human EphA2-Fc. Indeed, screening of a library in which both the light and heavy chains were simultaneously humanized (Library C) did not allow us to recover molecules exhibiting detectable binding to this antigen. This probably reflects factors such as sub-optimal library quality, incomplete library sampling and/or inefficient prokaryotic expression of a portion of the library. We anticipate that screening a larger number of clones would have resulted in the identification of humanized antibody fragments retaining binding to human EphA2.

As expected in light of their identical heavy and light chains variable regions, parental mAb B233 and its chimaeric IgG version exhibited virtually identical dissociation constant (K_{D} = 0.4 and 0.3 nM, respectively; Table 66). Humanized clones V_{H}-6H11/V_{L}-8G7 and V_{H}-2G6/V_{L}-12C8, when formatted as a human IgG1, exhibited avidities towards human EphA2 which were similar to the parental and chimaeric version of mAb B233 (K_{D} = 1.9 and 3.0 nM, respectively; Table 66). This corresponded to a small avidity decrease of 6 and 10-fold, respectively, when compared with parental mAb B233. Humanized clone V_{H}-7E8/V_{L}-8G7 exhibited the lowest avidity (K_{D} = 48 nM), which corresponded to a larger decrease of 160-fold when compared with parental mAb B233. It is worth noting that in terms of strength of binding to EphA2-Fc, the BIAcore-based ranking of humanized IgG clones V_{H}-6H11/V_{L}-8G7, V_{H}-2G6/V_{L}-12C8 and V_{H}-7E8/V_{L}-8G7 (Table 66) was different from the ELISA-based ranking that utilized their Fab counterparts (Figure 4). This is particularly striking in the case of clone V_{H}-7E8/V_{L}-8G7 which showed the lowest avidity (Table 66), yet consistently exhibited the highest signal by ELISA titration (Figure 4). We do not know what accounts for this difference but think that it is likely attributable to the format of the assays and/or imprecision in the quantification ELISA. Alternatively, it is possible that this discrepancy reflects unique, clone-specific correlations between affinity (as measured in Figure 4) and avidity (as measured in Table 66). Indeed, individual bivalent binding measurements depend on various factors such as the particular spatial arrangements of the corresponding antigen binding sites or the local antigen surface distribution (D.M. Crothers, et al. Immunochemistry 9: 341-357(1972); K.M. Müller, et al., Anal. Biochem. 261: 49-158(1998)).

This application claims the benefit of United States provisional application serial number 60/496,367, filed on August 18, 2003, which is incorporated by reference in its entirety.

### References Cited and Equivalents:

All references cited herein are incorporated herein by reference in their entireties and for all purposes to the same extent as if each individual publication or patent or patent application was specifically and individually indicated to be incorporated by reference in its entirety for all purposes. Many modifications and variations of this invention can be made without departing from its spirit and scope, as will be apparent to those skilled in the art.

## Claims

1. A method of generating a combinatorial library comprising a plurality of nucleotide sequences encoding heavy chain variable regions, said nucleotide sequences encoding the heavy chain variable regions each produced by fusing together a nucleic acid sequence encoding a heavy chain framework region 1, a nucleic acid sequence encoding a heavy chain complementarity determining region (CDR) 1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, wherein the CDRs are derived from a donor antibody heavy chain variable region and at least one nucleic acid sequence encoding a heavy chain framework region is from a sub-bank comprising a plurality of nucleic acid sequences encoding said heavy chain framework regions.

2. A method of generating a combinatorial library comprising a plurality of nucleotide sequences encoding light chain variable regions, said nucleotide sequences encoding the light chain variable regions each produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4, wherein the CDRs are derived from a donor antibody light chain variable region and at least one nucleic acid sequence encoding a light chain framework region is from a sub-bank comprising a plurality of nucleic acid sequences encoding said light chain framework regions.

3. A method of generating a combinatorial library comprising: (i) a first set of nucleotide sequences encoding heavy chain variable regions, said nucleotide sequences encoding the heavy chain variable regions each produced by fusing together a nucleic acid sequence encoding a heavy chain framework region 1, a nucleic acid sequence encoding CDR 1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, and (ii) a second set of nucleotide sequences encoding light chain variable regions, said nucleotide sequences encoding the light chain variable regions each produced by fusing together a nucleic acid sequence encoding a light chain framework region 1, a nucleic acid sequence encoding a light chain CDR1, a nucleic acid sequence encoding a light chain framework region 2, a nucleic acid sequence encoding a light chain CDR2, a nucleic acid sequence encoding a light chain framework region 3, a nucleic acid sequence encoding a light chain CDR3, and a nucleic acid sequence encoding a light chain framework region 4,wherein the heavy chain CDRs are derived from a donor antibody heavy chain variable region, the light chain variable region CDRs are derived from a donor antibody light chain variable region, at least one nucleic acid sequence encoding a heavy chain framework region is from a sub-bank comprising a plurality of nucleic acid sequences encoding said heavy chain framework regions, and at least one nucleic acid sequence encoding a light chain framework region is from a sub-bank comprising a plurality of nucleic acid sequences encoding said light chain framework regions.

4. The method of claim 1, 2, or 3, further comprising introducing the combinatorial library of nucleotide sequence into a population of cells.

5. A method of producing an antibody that immunospecifically binds to an antigen, said method comprising: (a) synthesizing a plurality of polynucleotide sequences each comprising a nucleotide sequence encoding a heavy chain variable region, said nucleotide sequence produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, wherein the CDRs are derived from a donor antibody heavy chain variable region that immunospecifically binds said antigen and at least one heavy chain framework region nucleic acid sequence is from a sub-bank comprising a plurality of nucleic acid sequences encoding said heavy chain framework region; (b) introducing the polynucleotide sequences into a population of cells each containing a polynucleotide sequence encoding a light chain variable region; and (c) expressing the polynucleotide sequences encoding the heavy chain variable region and the light chain variable region.

6. A method of producing an antibody that immunospecifically binds to an antigen, said method comprising: (a) synthesizing a plurality of polynucleotide sequences each comprising a nucleotide sequence encoding a light chain variable region, said nucleotide sequence produced by fusing together a nucleic acid sequence encoding a light chain framework region 1, a nucleic acid sequence encoding a light chain CDR1, a nucleic acid sequence encoding a light chain framework region 2, a nucleic acid sequence encoding light chain CDR2, a nucleic acid sequence encoding a light chain framework region 3, a nucleic acid sequence encoding a light chain CDR3, and a nucleic acid sequence encoding a light chain framework region 4, wherein the CDRs are derived from a donor antibody light chain variable region that immunospecifically binds said antigen and at least one light chain framework region nucleic acid sequence is from a sub-bank comprising a plurality of nucleic acid sequences encoding said light chain framework region; (b) introducing the polynucleotide sequences into a population of cells each containing a polynucleotide sequence encoding a heavy chain variable region and (c) expressing the polynucleotide sequences encoding the heavy chain variable region and the light chain variable region.

7. A method of producing an antibody that immunospecifically binds to an antigen, said method comprising: (a) synthesizing a plurality of first polynucleotide sequences each comprising a nucleotide sequence encoding a heavy chain variable region, said nucleotide sequence produced by fusing together a nucleic acid sequence encoding a heavy chain framework region 1, a nucleic acid sequence encoding a heavy chain CDR1, a nucleic acid sequence encoding a heavy chain framework region 2, a nucleic acid sequence encoding a heavy chain CDR2, a nucleic acid sequence encoding a heavy chain framework region 3, a nucleic acid sequence encoding a heavy chain CDR3, and a nucleic acid sequence encoding a heavy chain framework region 4, wherein the CDRs are derived from a donor antibody heavy chain variable region that immunospecifically binds said antigen and at least one heavy chain framework region nucleic acid sequence is from a sub-bank comprising a plurality of nucleic acid sequences encoding said heavy chain framework region; (b) synthesizing a plurality of second polynucleotide sequences each comprising a nucleotide sequence encoding a light chain variable region, said nucleotide sequence produced by fusing together a nucleic acid sequence encoding a light chain framework region 1, a nucleic acid sequence encoding a light chain CDR1, a nucleic acid sequence encoding a light chain framework region 2, a nucleic acid sequence encoding light chain CDR2, a nucleic acid sequence encoding a light chain framework region 3, a nucleic acid sequence encoding a light chain CDR3, and a nucleic acid sequence encoding a light chain framework region 4, wherein the CDRs are derived from a donor antibody light chain variable region that immunospecifically binds said antigen and at least one light chain framework region nucleic acid sequence is from a sub-bank comprising a plurality of nucleic acid sequences encoding said human light chain framework region; (c) introducing the nucleic acid sequences generated in steps (a) and (b) into a population of cells; and (d) expressing the nucleotide sequences encoding the heavy chain variable region and the light chain variable region.

8. The method of claim 5, 6 or 7, further comprising, after the step of expressing the nucleotide sequences, the step of screening for an antibody that immunospecifically binds to the antigen.

9. The method of claim 5, 6, 7 or 8, further comprising, prior to step (a), the step of generating the sub-banks of framework region sequences.

10. The method of any of the preceding claims, wherein the framework region sub-bank(s) comprise framework regions from germline framework sequences and/or framework regions from functional antibody sequences.

11. The method of any of the preceding claims, wherein the framework region sub-bank(s) comprise framework regions from germline framework sequences and/or framework regions from functional antibody sequences into which one or mutations have been introduced.

12. The method of any of the preceding claims wherein the framework region sub-banks comprise framework regions from human germline framework sequences and/or framework regions from functional human antibody sequences.

13. The method of any of the preceding claims, wherein the framework region sub-bank(s) comprise framework regions from human germline framework sequences and/or framework regions from functional human antibody sequences into which one or mutations have been introduced.

14. The method of any of the preceding claims, wherein one or more of the CDRs derived from the donor antibody heavy chain variable region contains one or more mutations relative to the nucleic acid sequence encoding the corresponding CDR in the donor antibody.


SEQUENCE LISTING

<110> MEDIMMUNE LLC

<120> HUMANIZATION OF ANTIBODIES

<130> MED0018.3EP

<140>
<141>

<150> US 60/496,367
<151> 2003-08-18

<160> 1781

<170> FastSEQ for Windows Version 4.0

<210> 1
<211> 69
<212> DNA
<213> Homo sapiens

<400> 1
gatgttgtga tgactcagtc tccactctcc ctgcccgtca cccttggaca gccggcctcc 60
atctcctgc 69

<210> 2
<211> 69
<212> DNA
<213> Homo sapiens

<400> 2
gatgttgtga tgactcagtc tccactctcc ctgcccgtca cccttggaca gccggcctcc 60
atctcctgc 69

<210> 3
<211> 69
<212> DNA
<213> Homo sapiens

<400> 3
gatattgtga tgacccagac tccactctct ctgtccgtca cccctggaca gccggcctcc 60
atctcctgc 69

<210> 4
<211> 69
<212> DNA
<213> Homo sapiens

<400> 4
gatattgtga tgactcagtc tccactctcc ctgcccgtca cccctggaga gccggcctcc 60
atctcctgc 69

<210> 5
<211> 69
<212> DNA
<213> Homo sapiens

<400> 5
gatattgtga tgacccagac tccactctct ctgtccgtca cccctggaca gccggcctcc 60
atctcctgc 69

<210> 6
<211> 69
<212> DNA

<213> Homo sapiens

<400> 6
gatattgtga tgacccagac tccactctcc tcacctgtca cccttggaca gccggcctcc 60
atctcctgc 69

<210> 7
<211> 69
<212> DNA
<213> Homo sapiens

<400> 7
gatattgtga tgactcagtc tccactctcc ctgcccgtca cccctggaga gccggcctcc 60
atctcctgc 69

<210> 8
<211> 69
<212> DNA
<213> Homo sapiens

<400> 8
gagattgtga tgacccagac tccactctcc ttgtctatca cccctggaga gcaggcctcc 60
atctcctgc 69

<210> 9
<211> 69
<212> DNA
<213> Homo sapiens

<400> 9
gatattgtga tgacccagac tccactctcc tcgcctgtca cccttggaca gccggcctcc 60
atctccttc 69

<210> 10
<211> 69
<212> DNA
<213> Homo sapiens

<400> 10
gaaattgtgc tgactcagtc tccagacttt cagtctgtga ctccaaagga gaaagtcacc 60
atcacctgc 69

<210> 11
<211> 69
<212> DNA
<213> Homo sapiens

<400> 11
gatgttgtga tgacacagtc tccagctttc ctctctgtga ctccagggga gaaagtcacc 60
atcacctgc 69

<210> 12
<211> 69
<212> DNA
<213> Homo sapiens

<400> 12
gacatccaga tgacccagtc tccatcctcc ctgtctgcat ctgtaggaga cagagtcacc 60
atcacttgc 69

<210> 13
<211> 69
<212> DNA
<213> Homo sapiens

<400> 13
gaaattgtgc tgactcagtc tccagacttt cagtctgtga ctccaaagga gaaagtcacc 60
atcacctgc 69

<210> 14
<211> 69
<212> DNA
<213> Homo sapiens

<400> 14
gacatccaga tgacccagtc tccatcctcc ctgtctgcat ctgtaggaga cagagtcacc 60
atcacttgc 69

<210> 15
<211> 69
<212> DNA
<213> Homo sapiens

<400> 15
gaaacgacac tcacgcagtc tccagcattc atgtcagcga ctccaggaga caaagtcaac 60
atctcctgc 69

<210> 16
<211> 69
<212> DNA
<213> Homo sapiens

<400> 16
gacatccaga tgacccagtc tccatcctca ctgtctgcat ctgtaggaga cagagtcacc 60
atcacttgt 69

<210> 17
<211> 69
<212> DNA
<213> Homo sapiens

<400> 17
gccatccaga tgacccagtc tccatcctcc ctgtctgcat ctgtaggaga cagagtcacc 60
atcacttgc 69

<210> 18
<211> 69
<212> DNA
<213> Homo sapiens

<400> 18
gacatccaga tgacccagtc tccttccacc ctgtctgcat ctgtaggaga cagagtcacc 60
atcacttgc 69

<210> 19
<211> 69
<212> DNA
<213> Homo sapiens

<400> 19
aacatccaga tgacccagtc tccatctgcc atgtctgcat ctgtaggaga cagagtcacc 60
atcacttgt 69

<210> 20
<211> 69
<212> DNA
<213> Homo sapiens

<400> 20
gacatccaga tgacccagtc tccatcctca ctgtctgcat ctgtaggaga cagagtcacc 60
atcacttgt 69

<210> 21
<211> 69
<212> DNA
<213> Homo sapiens

<400> 21
gaaatagtga tgatgcagtc tccagccacc ctgtctgtgt ctccagggga aagagccacc 60
ctctcctgc 69

<210> 22
<211> 69
<212> DNA
<213> Homo sapiens

<400> 22
gccatccagt tgacccagtc tccatcctcc ctgtctgcat ctgtaggaga cagagtcacc 60
atcacttgc 69

<210> 23
<211> 69
<212> DNA
<213> Homo sapiens

<400> 23
gacatccaga tgacccagtc tccatcttct gtgtctgcat ctgtaggaga cagagtcacc 60
atcacttgt 69

<210> 24
<211> 69
<212> DNA
<213> Homo sapiens

<400> 24
gaaatagtga tgacgcagtc tccagccacc ctgtctgtgt ctccagggga aagagccacc 60
ctctcctgc 69

<210> 25
<211> 69
<212> DNA
<213> Homo sapiens

<400> 25
gaaattgtgt tgacacagtc tccagccacc ctgtctttgt ctccagggga aagagccacc 60
ctctcctgc 69

<210> 26
<211> 69
<212> DNA
<213> Homo sapiens

<400> 26
gacatccaga tgatccagtc tccatctttc ctgtctgcat ctgtaggaga cagagtcagt 60
atcatttgc 69

<210> 27
<211> 69
<212> DNA
<213> Homo sapiens

<400> 27
gccatccgga tgacccagtc tccattctcc ctgtctgcat ctgtaggaga cagagtcacc 60
atcacttgc 69

<210> 28
<211> 69
<212> DNA
<213> Homo sapiens

<400> 28
gtcatctgga tgacccagtc tccatcctta ctctctgcat ctacaggaga cagagtcacc 60
atcagttgt 69

<210> 29
<211> 69
<212> DNA
<213> Homo sapiens

<400> 29
gccatccagt tgacccagtc tccatcctcc ctgtctgcat ctgtaggaga cagagtcacc 60
atcacttgc 69

<210> 30
<211> 69
<212> DNA
<213> Homo sapiens

<400> 30
gacatccaga tgacccagtc tccatcttcc gtgtctgcat ctgtaggaga cagagtcacc 60
atcacttgt 69

<210> 31
<211> 69
<212> DNA
<213> Homo sapiens

<400> 31
gaaattgtgt tgacacagtc tccagccacc ctgtctttgt ctccagggga aagagccacc 60
ctctcctgc 69

<210> 32
<211> 69
<212> DNA
<213> Homo sapiens

<400> 32
gacatccagt tgacccagtc tccatccttc ctgtctgcat ctgtaggaga cagagtcacc 60
atcacttgc 69

<210> 33
<211> 69
<212> DNA
<213> Homo sapiens

<400> 33
gccatccgga tgacccagtc tccatcctca ttctctgcat ctacaggaga cagagtcacc 60
atcacttgt 69

<210> 34
<211> 69
<212> DNA
<213> Homo sapiens

<400> 34
gacatccaga tgacccagtc tccatcctcc ctgtctgcat ctgtaggaga cagagtcacc 60
atcacttgc 69

<210> 35
<211> 69
<212> DNA
<213> Homo sapiens

<400> 35
gacatccagt tgacccagtc tccatcctcc ctgtctgcat ctgtaggaga cagagtcacc 60
atcacttgc 69

<210> 36
<211> 69
<212> DNA
<213> Homo sapiens

<400> 36
gacatccaga tgacccagtc tccatcctcc ctgtctgcat ctgtaggaga cagagtcacc 60
atcacttgc 69

<210> 37
<211> 69
<212> DNA
<213> Homo sapiens

<400> 37
gacatccaga tgacccagtc tccatcctcc ctgtctgcat ctgtaggaga cagagtcacc 60
atcacttgc 69

<210> 38
<211> 69
<212> DNA
<213> Homo sapiens

<400> 38
gacatccagt tgacccagtc tccatcctcc ctgtctgcat ctgtaggaga cagagtcacc 60
atcacttgc 69

<210> 39
<211> 69
<212> DNA
<213> Homo sapiens

<400> 39
gacatccaga tgacccagtc tccatcctcc ctgtctgcat ctgtaggaga cagagtcacc 60
atcacttgc 69

<210> 40
<211> 69
<212> DNA
<213> Homo sapiens

<400> 40
gaaattgtaa tgacacagtc tccacccacc ctgtctttgt ctccagggga aagagtcacc 60
ctctcctgc 69

<210> 41
<211> 69
<212> DNA
<213> Homo sapiens

<400> 41
gaaattgtaa tgacacagtc tccagccacc ctgtctttgt ctccagggga aagagccacc 60
ctctcctgc 69

<210> 42
<211> 69
<212> DNA
<213> Homo sapiens

<400> 42
gaaattgtgt tgacgcagtc tccagccacc ctgtctttgt ctccagggga aagagccacc 60
ctctcctgc 69

<210> 43
<211> 69
<212> DNA
<213> Homo sapiens

<400> 43
gaaattgtgt tgacgcagtc tccaggcacc ctgtctttgt ctccagggga aagagccacc 60
ctctcctgc 69

<210> 44

<211> 69
<212> DNA
<213> Homo sapiens

<400> 44
gacatcgtga tgacccagtc tccagactcc ctggctgtgt ctctgggcga gagggccacc 60
atcaactgc 69

<210> 45
<211> 69
<212> DNA
<213> Homo sapiens

<400> 45
gatattgtga tgacccagac tccactctcc ctgcccgtca cccctggaga gccggcctcc 60
atctcctgc 69

<210> 46
<211> 69
<212> DNA
<213> Homo sapiens

<400> 46
gatattgtga tgacccagac tccactctcc ctgcccgtca cccctggaga gccggcctcc 60
atctcctgc 69

<210> 47
<211> 45
<212> DNA
<213> Homo sapiens

<400> 47
tggtttcagc agaggccagg ccaatctcca aggcgcctaa tttat 45

<210> 48
<211> 45
<212> DNA
<213> Homo sapiens

<400> 48
tggtttcagc agaggccagg ccaatctcca aggcgcctaa tttat 45

<210> 49
<211> 45
<212> DNA
<213> Homo sapiens

<400> 49
tggtacctgc agaagccagg ccagtctcca cagctcctga tctat 45

<210> 50
<211> 45
<212> DNA
<213> Homo sapiens

<400> 50
tggtacctgc agaagccagg gcagtctcca cagctcctga tctat 45

<210> 51
<211> 45
<212> DNA
<213> Homo sapiens

<400> 51
tggtacctgc agaagccagg ccagcctcca cagctcctga tctat 45

<210> 52
<211> 45

<212> DNA
<213> Homo sapiens

<400> 52
tggcttcagc agaggccagg ccagcctcca agactcctaa tttat 45

<210> 53
<211> 45
<212> DNA
<213> Homo sapiens

<400> 53
tggtacctgc agaagccagg gcagtctcca cagctcctga tctat 45

<210> 54
<211> 45
<212> DNA
<213> Homo sapiens

<400> 54
tggtttctgc agaaagccag gccagtctcc acactcctga tctat 45

<210> 55
<211> 45
<212> DNA
<213> Homo sapiens

<400> 55
tggcttcagc agaggccagg ccagcctcca agactcctaa tttat 45

<210> 56
<211> 45
<212> DNA
<213> Homo sapiens

<400> 56
tggtaccagc agaaaccaga tcagtctcca aagctcctca tcaag 45

<210> 57
<211> 45
<212> DNA
<213> Homo sapiens

<400> 57
tggtaccagc agaaaccaga tcaagcccca aagctcctca tcaag 45

<210> 58
<211> 45
<212> DNA
<213> Homo sapiens

<400> 58
tggtatcagc agaaaccagg gaaagttcct aagctcctga tctat 45

<210> 59
<211> 45
<212> DNA
<213> Homo sapiens

<400> 59
tggtaccagc agaaaccaga tcagtctcca aagctcctca tcaag 45

<210> 60
<211> 45
<212> DNA
<213> Homo sapiens

<400> 60

tggtatcagc agaaaccagg gaaagcccct aagcgcctga tctat 45

<210> 61
<211> 45
<212> DNA
<213> Homo sapiens

<400> 61
tggtaccaac agaaaccagg agaagctgct attttcatta ttcaa 45

<210> 62
<211> 45
<212> DNA
<213> Homo sapiens

<400> 62
tggtttcagc agaaaccagg gaaagcccct aagtccctga tctat 45

<210> 63
<211> 45
<212> DNA
<213> Homo sapiens

<400> 63
tggtatcagc agaaaccagg gaaagcccct aagctcctga tctat 45

<210> 64
<211> 45
<212> DNA
<213> Homo sapiens

<400> 64
tggtatcagc agaaaccagg gaaagcccct aagctcctga tctat 45

<210> 65
<211> 45
<212> DNA
<213> Homo sapiens

<400> 65
tggtttcagc agaaaccagg gaaagtccct aagcacctga tctat 45

<210> 66
<211> 45
<212> DNA
<213> Homo sapiens

<400> 66
tggtatcagc agaaaccaga gaaagcccct aagtccctga tctat 45

<210> 67
<211> 45
<212> DNA
<213> Homo sapiens

<400> 67
tggtaccagc agaaacctgg ccaggctccc aggctcctca tctat 45

<210> 68
<211> 45
<212> DNA
<213> Homo sapiens

<400> 68
tggtatcagc agaaaccagg gaaagctcct aagctcctga tctat 45

<210> 69
<211> 45

<212> DNA
<213> Homo sapiens

<400> 69
tggtatcagc agaaaccagg gaaagcccct aagctcctga tctat 45

<210> 70
<211> 45
<212> DNA
<213> Homo sapiens

<400> 70
tggtaccagc agaaacctgg ccaggctccc aggctcctca tctat 45

<210> 71
<211> 45
<212> DNA
<213> Homo sapiens

<400> 71
tggtaccagc agaaacctgg ccaggctccc aggctcctca tctat 45

<210> 72
<211> 45
<212> DNA
<213> Homo sapiens

<400> 72
tggtatctgc agaaaccagg gaaatcccct aagctcttcc tctat 45

<210> 73
<211> 45
<212> DNA
<213> Homo sapiens

<400> 73
tggtatcagc aaaaaccagc aaaagcccct aagctcttca tctat 45

<210> 74
<211> 45
<212> DNA
<213> Homo sapiens

<400> 74
tggtatcagc aaaaaccagg gaaagcccct gagctcctga tctat 45

<210> 75
<211> 45
<212> DNA
<213> Homo sapiens

<400> 75
tggtatcagc agaaaccagg gaaagctcct aagctcctga tctat 45

<210> 76
<211> 45
<212> DNA
<213> Homo sapiens

<400> 76
tggtatcagc agaaaccagg gaaagcccct aagctcctga tctat 45

<210> 77
<211> 45
<212> DNA
<213> Homo sapiens

<400> 77

tggtaccaac agaaacctgg ccaggctccc aggctcctca tctat 45

<210> 78
<211> 45
<212> DNA
<213> Homo sapiens

<400> 78
tggtatcagc aaaaaccagg gaaagcccct aagctcctga tctat 45

<210> 79
<211> 45
<212> DNA
<213> Homo sapiens

<400> 79
tggtatcagc aaaaaccagg gaaagcccct aagctcctga tctat 45

<210> 80
<211> 45
<212> DNA
<213> Homo sapiens

<400> 80
tggtatcagc agaaaccagg gaaagcccct aagctcctga tctat 45

<210> 81
<211> 45
<212> DNA
<213> Homo sapiens

<400> 81
tggtatcggc agaaaccagg gaaagttcct aagctcctga tctat 45

<210> 82
<211> 45
<212> DNA
<213> Homo sapiens

<400> 82
tggtatcagc agaaaccagg gaaagcccct aagctcctga tctac 45

<210> 83
<211> 45
<212> DNA
<213> Homo sapiens

<400> 83
tggtatcagc agaaaccagg gaaagcccct aagctcctga tctat 45

<210> 84
<211> 45
<212> DNA
<213> Homo sapiens

<400> 84
tggtatcggc agaaaccagg gaaagttcct aagctcctga tctat 45

<210> 85
<211> 45
<212> DNA
<213> Homo sapiens

<400> 85
tggtatcagc agaaaccagg gaaagcccct aagctcctga tctac 45

<210> 86
<211> 45

<212> DNA
<213> Homo sapiens

<400> 86
tggtatcagc agaaacctgg ccaggcgccc aggctcctca tctat 45

<210> 87
<211> 45
<212> DNA
<213> Homo sapiens

<400> 87
tggtaccagc agaaacctgg gcaggctccc aggctcctca tctat 45

<210> 88
<211> 45
<212> DNA
<213> Homo sapiens

<400> 88
tggtaccagc agaaacctgg cctggcgccc aggctcctca tctat 45

<210> 89
<211> 45
<212> DNA
<213> Homo sapiens

<400> 89
tggtaccagc agaaacctgg ccaggctccc aggctcctca tctat 45

<210> 90
<211> 45
<212> DNA
<213> Homo sapiens

<400> 90
tggtaccagc agaaaccagg acagcctcct aagctgctca tttac 45

<210> 91
<211> 45
<212> DNA
<213> Homo sapiens

<400> 91
tggtacctgc agaagccagg gcagtctcca cagctcctga tctat 45

<210> 92
<211> 45
<212> DNA
<213> Homo sapiens

<400> 92
tggtacctgc agaagccagg gcagtctcca cagctcctga tctat 45

<210> 93
<211> 96
<212> DNA
<213> Homo sapiens

<400> 93
ggggtcccag acagattcag cggcagtggg tcaggcactg atttcacact gaaaatcagc 60
agggtggagg ctgaggatgt tggggtttat tactgc 96

<210> 94
<211> 96
<212> DNA
<213> Homo sapiens

<400> 94
ggggtcccag acagattcag cggcagtggg tcaggcactg atttcacact gaaaatcagc 60
agggtggagg ctgaggatgt tggggtttat tactgc 96

<210> 95
<211> 96
<212> DNA
<213> Homo sapiens

<400> 95
ggagtgccag ataggttcag tggcagcggg tcagggacag atttcacact gaaaatcagc 60
cgggtggagg ctgaggatgt tggggtttat tactga 96

<210> 96
<211> 96
<212> DNA
<213> Homo sapiens

<400> 96
ggggtccctg acaggttcag tggcagtgga tcaggcacag attttacact gaaaatcagc 60
agagtggagg ctgaggatgt tggggtttat tactgc 96

<210> 97
<211> 96
<212> DNA
<213> Homo sapiens

<400> 97
ggagtgccag ataggttcag tggcagcggg tcagggacag atttcacact gaaaatcagc 60
cgggtggagg ctgaggatgt tggggtttat tactgc 96

<210> 98
<211> 96
<212> DNA
<213> Homo sapiens

<400> 98
ggggtcccag acagattcag tggcagtggg gcagggacag atttcacact gaaaatcagc 60
agggtggaag ctgaggatgt cggggtttat tactgc 96

<210> 99
<211> 96
<212> DNA
<213> Homo sapiens

<400> 99
ggggtccctg acaggttcag tggcagtgga tcaggcacag attttacact gaaaatcagc 60
agagtggagg ctgaggatgt tggggtttat tactgc 96

<210> 100
<211> 96
<212> DNA
<213> Homo sapiens

<400> 100
ggagtgccag ataggttcag tggcagcggg tcagggacag atttcacact gaaaatcagc 60
cgggtggagg ctgaggattt tggagtttat tactgc 96

<210> 101
<211> 96
<212> DNA
<213> Homo sapiens

<400> 101
ggggtcccag acagattcag tggcagtggg gcagggacag atttcacact gaaaatcagc 60
agggtggaag ctgaggatgt cggggtttat tactgc 96

<210> 102

<211> 96
<212> DNA
<213> Homo sapiens

<400> 102
ggggtcccct cgaggttcag tggcagtgga tctgggacag atttcaccct caccatcaat 60
agcctggaag ctgaagatgc tgcaacgtat tactgt 96

<210> 103
<211> 96
<212> DNA
<213> Homo sapiens

<400> 103
ggggtcccct cgaggttcag tggcagtgga tctgggacag atttcacctt taccatcagt 60
agcctggaag ctgaagatgc tgcaacatat tactgt 96

<210> 104
<211> 96
<212> DNA
<213> Homo sapiens

<400> 104
ggggtcccat ctcggttcag tggcagtgga tctgggacag atttcactct caccatcagc 60
agcctgcagc ctgaagatgt tgcaacttat tactgt 96

<210> 105
<211> 96
<212> DNA
<213> Homo sapiens

<400> 105
ggggtcccct cgaggttcag tggcagtgga tctgggacag atttcaccct caccatcaat 60
agcctggaag ctgaagatgc tgcaacgtat tactgt 96

<210> 106
<211> 96
<212> DNA
<213> Homo sapiens

<400> 106
ggggtcccat caaggttcag cggcagtgga tctgggacag aattcactct cacaatcagc 60
agcctgcagc ctgaagattt tgcaacttat tactgt 96

<210> 107
<211> 96
<212> DNA
<213> Homo sapiens

<400> 107
ggaatcccac ctcgattcag tggcagcggg tatggaacag attttaccct cacaattaat 60
aacatagaat ctgaggatgc tgcatattac ttctgt 96

<210> 108
<211> 96
<212> DNA
<213> Homo sapiens

<400> 108
ggggtcccat caaggttcag cggcagtgga tctgggacag atttcactct caccatcagc 60
agcctgcagc ctgaagattt tgcaacttat tactgc 96

<210> 109
<211> 96
<212> DNA
<213> Homo sapiens

<400> 109

ggggtcccat caaggttcag cggcagtgga tctggcacag atttcactct caccatcagc 60
agcctgcagc ctgaagattt tgcaacttat tactgt 96

<210> 110
<211> 96
<212> DNA
<213> Homo sapiens

<400> 110
ggggtcccat caaggttcag cggcagtgga tctgggacag aattcactct caccatcagc 60
agcctgcagc ctgatgattt tgcaacttat tactgc 96

<210> 111
<211> 96
<212> DNA
<213> Homo sapiens

<400> 111
ggggtcccat caaggttcag cggcagtgga tctgggacag aattcactct cacaatcagc 60
agcctgcagc ctgaagattt tgcaacttat tactgt 96

<210> 112
<211> 96
<212> DNA
<213> Homo sapiens

<400> 112
ggggtcccat caaggttcag cggcagtgga tctgggacag atttcactct caccatcagc 60
agcctgcagc ctgaagattt tgcaacttat tactgc 96

<210> 113
<211> 96
<212> DNA
<213> Homo sapiens

<400> 113
ggcatcccag ccaggttcag tggcagtggg tctgggacag agttcactct caccatcagc 60
agcctgcagt ctgaagattt tgcagtttat tactgt 96

<210> 114
<211> 96
<212> DNA
<213> Homo sapiens

<400> 114
ggggtcccat caaggttcag cggcagtgga tctgggacag atttcactct caccatcagc 60
agcctgcagc ctgaagattt tgcaacttat tactgt 96

<210> 115
<211> 96
<212> DNA
<213> Homo sapiens

<400> 115
ggggtcccat caaggttcag cggcagtgga tctgggacag atttcactct cactatcagc 60
agcctgcagc ctgaagattt tgcaacttac tattgt 96

<210> 116
<211> 96
<212> DNA
<213> Homo sapiens

<400> 116
ggtatcccag ccaggttcag tggcagtggg tctgggacag agttcactct caccatcagc 60
agcctgcagt ctgaagattt tgcagtttat tactgt 96

<210> 117
<211> 96

<212> DNA
<213> Homo sapiens

<400> 117
ggcatcccag ccaggttcag tggcagtggg cctgggacag acttcactct caccatcagc 60
agcctagagc ctgaagattt tgcagtttat tactgt 96

<210> 118
<211> 96
<212> DNA
<213> Homo sapiens

<400> 118
ggggtctcat cgaggttcag tggcagggga tctgggacgg atttcactct caccatcatc 60
agcctgaagc ctgaagattt tgcagcttat tactgt 96

<210> 119
<211> 96
<212> DNA
<213> Homo sapiens

<400> 119
ggggtcccat caaggttcag cggcagtgga tctgggacgg attacactct caccatcagc 60
agcctgcagc ctgaagattt tgcaacttat tactgt 96

<210> 120
<211> 96
<212> DNA
<213> Homo sapiens

<400> 120
ggggtcccat caaggttcag tggcagtgga tctgggacag atttcactct caccatcagt 60
tgcctgcagt ctgaagattt tgcaacttat tactgt 96

<210> 121
<211> 96
<212> DNA
<213> Homo sapiens

<400> 121
ggggtcccat caaggttcag cggcagtgga tctgggacag atttcactct caccatcagc 60
agcctgcagc ctgaagattt tgcaacttat tactgt 96

<210> 122
<211> 96
<212> DNA
<213> Homo sapiens

<400> 122
ggggtcccat caaggttcag cggcagtgga tctgggacag atttcactct caccatcagc 60
agcctgcagc ctgaagattt tgcaacttac tattgt 96

<210> 123
<211> 96
<212> DNA
<213> Homo sapiens

<400> 123
ggcatcccag ccaggttcag tggcagtggg tctgggacag acttcactct caccatcagc 60
agcctagagc ctgaagattt tgcagtttat tactgt 96

<210> 124
<211> 96
<212> DNA
<213> Homo sapiens

<400> 124
ggggtcccat caaggttcag cggcagtgga tctgggacag aattcactct cacaatcagc 60

agcctgcagc ctgaagattt tgcaacttat tactgt 96

<210> 125
<211> 96
<212> DNA
<213> Homo sapiens

<400> 125
ggggtcccat caaggttcag cggcagtgga tctgggacag atttcactct caccatcagc 60
tgcctgcagt ctgaagattt tgcaacttat tactgt 96

<210> 126
<211> 96
<212> DNA
<213> Homo sapiens

<400> 126
ggggtcccat caaggttcag tggcagtgga tctgggacag atttcactct caccatcagc 60
agtctgcaac ctgaagattt tgcaacttac tactgt 96

<210> 127
<211> 96
<212> DNA
<213> Homo sapiens

<400> 127
ggagtcccat ctcggttcag tggcagtgga tctgggacag atttcactct cactatcagc 60
agcctgcagc ctgaagatgt tgcaacttat tacggt 96

<210> 128
<211> 96
<212> DNA
<213> Homo sapiens

<400> 128
ggggtcccat caaggttcag tggaagtgga tctgggacag attttacttt caccatcagc 60
agcctgcagc ctgaagatat tgcaacatat tactgt 96

<210> 129
<211> 96
<212> DNA
<213> Homo sapiens

<400> 129
ggggtcccat caaggttcag tggcagtgga tctgggacag atttcactct caccatcagc 60
agtctgcaac ctgaagattt tgcaacttac tactgt 96

<210> 130
<211> 96
<212> DNA
<213> Homo sapiens

<400> 130
ggagtcccat ctcggttcag tggcagtgga tctgggacag atttcactct cactatcagc 60
agcctgcagc ctgaagatgt tgcaacttat tacggt 96

<210> 131
<211> 96
<212> DNA
<213> Homo sapiens

<400> 131
ggggtcccat caaggttcag tggaagtgga tctgggacag attttacttt caccatcagc 60
agcctgcagc ctgaagatat tgcaacatat tactgt 96

<210> 132
<211> 96
<212> DNA

<213> Homo sapiens

<400> 132
agcatcccag ccaggttcag tggcagtggg tctgggacag acttcactct caccatcagc 60
agcctgcagc ctgaagattt tgcagtttat tactgt 96

<210> 133
<211> 96
<212> DNA
<213> Homo sapiens

<400> 133
ggcatcccag ccaggttcag tggcagtggg tctgggacag acttcactct caccatcagc 60
agcctgcagc ctgaagattt tgcagtttat tactgt 96

<210> 134
<211> 96
<212> DNA
<213> Homo sapiens

<400> 134
ggcatcccag acaggttcag tggcagtggg tctgggacag acttcactct caccatcagc 60
agactggagc ctgaagattt tgcagtgtat tactgt 96

<210> 135
<211> 96
<212> DNA
<213> Homo sapiens

<400> 135
ggcatcccag acaggttcag tggcagtggg tctgggacag acttcactct caccatcagc 60
agactggagc ctgaagattt tgcagtgtat tactgt 96

<210> 136
<211> 96
<212> DNA
<213> Homo sapiens

<400> 136
ggggtccctg accgattcag tggcagcggg tctgggacag atttcactct caccatcagc 60
agcctgcagg ctgaagatgt ggcagtttat tactgt 96

<210> 137
<211> 96
<212> DNA
<213> Homo sapiens

<400> 137
ggagtcccag acaggttcag tggcagtggg tcaggcactg atttcacact gaaaatcagc 60
agggtggagg ctgaggatgt tggagtttat tactgc 96

<210> 138
<211> 96
<212> DNA
<213> Homo sapiens

<400> 138
ggagtcccag acaggttcag tggcagtggg tcaggcactg atttcacact gaaaatcagc 60
agggtggagg ctgaggatgt tggagtttat tactgc 96

<210> 139
<211> 30
<212> DNA
<213> Homo sapiens

<400> 139
ttcggccaag ggaccaaggt ggaaatcaaa 30

<210> 140
<211> 30
<212> DNA
<213> Homo sapiens

<400> 140
tttggccagg ggaccaagct ggagatcaaa 30

<210> 141
<211> 30
<212> DNA
<213> Homo sapiens

<400> 141
ttcggccctg ggaccaaagt ggatatcaaa 30

<210> 142
<211> 30
<212> DNA
<213> Homo sapiens

<400> 142
ttcggcggag ggaccaaggt ggagatcaaa 30

<210> 143
<211> 30
<212> DNA
<213> Homo sapiens

<400> 143
ttcggccaag ggacacgact ggagattaaa 30

<210> 144
<211> 90
<212> DNA
<213> Homo sapiens

<400> 144
caggttcagc tggtgcagtc tggagctgag gtgaagaagc ctggggcctc agtgaaggtc 60
tcctgcaagg cttctggtta cacctttacc 90

<210> 145
<211> 90
<212> DNA
<213> Homo sapiens

<400> 145
caggtgcagc tggtgcagtc tggggctgag gtgaagaagc ctggggcctc agtgaaggtc 60
tcctgcaagg cttctggata caccttcacc 90

<210> 146
<211> 90
<212> DNA
<213> Homo sapiens

<400> 146
caggtccagc tggtacagtc tggggctgag gtgaagaagc ctggggcctc agtgaaggtc 60
tcctgcaagg tttccggata caccctcact 90

<210> 147
<211> 90
<212> DNA
<213> Homo sapiens

<400> 147
caggttcagc tggtgcagtc tggggctgag gtgaagaagc ctggggcctc agtgaaggtt 60
tcctgcaagg cttctggata caccttcact 90

<210> 148
<211> 90
<212> DNA
<213> Homo sapiens

<400> 148
cagatgcagc tggtgcagtc tggggctgag gtgaagaaga ctgggtcctc agtgaaggtt 60
tcctgcaagg cttccggata caccttcacc 90

<210> 149
<211> 90
<212> DNA
<213> Homo sapiens

<400> 149
caggtgcagc tggtgcagtc tggggctgag gtgaagaagc ctggggcctc agtgaaggtt 60
tcctgcaagg catctggata caccttcacc 90

<210> 150
<211> 90
<212> DNA
<213> Homo sapiens

<400> 150
caaatgcagc tggtgcagtc tgggcctgag gtgaagaagc ctgggacctc agtgaaggtc 60
tcctgcaagg cttctggatt cacctttact 90

<210> 151
<211> 90
<212> DNA
<213> Homo sapiens

<400> 151
caggtgcagc tggtgcagtc tggggctgag gtgaagaagc ctgggtcctc ggtgaaggtc 60
tcctgcaagg cttctggagg caccttcagc 90

<210> 152
<211> 90
<212> DNA
<213> Homo sapiens

<400> 152
caggtgcagc tggtgcagtc tggggctgag gtgaagaagc ctggggcctc agtgaaggtc 60
tcctgcaagg cttctggata caccttcacc 90

<210> 153
<211> 90
<212> DNA
<213> Homo sapiens

<400> 153
caggtcacct tgaaggagtc tggtcctgtg ctggtgaaac ccacagagac cctcacgctg 60
acctgcaccg tctctgggtt ctcactcagc 90

<210> 154
<211> 90
<212> DNA
<213> Homo sapiens

<400> 154
cagatcacct tgaaggagtc tggtcctacg ctggtgaaac ccacacagac cctcacgctg 60
acctgcacct tctctgggtt ctcactcagc 90

<210> 155
<211> 90
<212> DNA
<213> Homo sapiens

<400> 155
caggtcacct tgagggagtc tggtcctgcg ctggtgaaac ccacacagac cctcacactg 60
acctgcacct tctctgggtt ctcactcagc 90

<210> 156
<211> 90
<212> DNA
<213> Homo sapiens

<400> 156
caggtgcagc tggtggagtc tgggggaggc ttggtcaagc ctggagggtc cctgagactc 60
tcctgtgcag cctctggatt caccttcagt 90

<210> 157
<211> 90
<212> DNA
<213> Homo sapiens

<400> 157
gaggtgcagc tggtggagtc tgggggaggc ttggtacagc ctggggggtc cctgagactc 60
tcctgtgcag cctctggatt caccttcagt 90

<210> 158
<211> 90
<212> DNA
<213> Homo sapiens

<400> 158
gaggtgcagc tggtggagtc tgggggaggc ttggtaaagc ctggggggtc ccttagactc 60
tcctgtgcag cctctggatt cactttcagt 90

<210> 159
<211> 90
<212> DNA
<213> Homo sapiens

<400> 159
gaggtgcagc tggtggagtc tgggggaggc ttggtacagc ctggggggtc cctgagactc 60
tcctgtgcag cctctggatt caccttcagt 90

<210> 160
<211> 90
<212> DNA
<213> Homo sapiens

<400> 160
gaggtgcagc tggtggagtc tgggggaggt gtggtacggc ctggggggtc cctgagactc 60
tcctgtgcag cctctggatt cacctttgat 90

<210> 161
<211> 90
<212> DNA
<213> Homo sapiens

<400> 161
gaggtgcagc tggtggagtc tgggggaggc ctggtcaagc ctggggggtc cctgagactc 60
tcctgtgcag cctctggatt caccttcagt 90

<210> 162
<211> 90
<212> DNA
<213> Homo sapiens

<400> 162
gaggtgcagc tgttggagtc tgggggaggc ttggtacagc ctggggggtc cctgagactc 60
tcctgtgcag cctctggatt cacctttagc 90

<210> 163

<211> 90
<212> DNA
<213> Homo sapiens

<400> 163
caggtgcagc tggtggagtc tgggggaggc gtggtccagc ctgggaggtc cctgagactc 60
tcctgtgcag cctctggatt caccttcagt 90

<210> 164
<211> 90
<212> DNA
<213> Homo sapiens

<400> 164
caggtgcagc tggtggagtc tgggggaggc gtggtccagc ctgggaggtc cctgagactc 60
tcctgtgcag cgtctggatt caccttcagt 90

<210> 165
<211> 90
<212> DNA
<213> Homo sapiens

<400> 165
gaggtgcagc tggtggagtc tgggggaggc ttggtacagc ctggggggtc cctgagactc 60
tcctgtgcag cctctggatt caccttcagt 90

<210> 166
<211> 90
<212> DNA
<213> Homo sapiens

<400> 166
gaggtgcagc tggtggagtc tgggggaggc ttggtacagc ctagggggtc cctgagactc 60
tcctgtgcag cctctggatt caccgtcagt 90

<210> 167
<211> 90
<212> DNA
<213> Homo sapiens

<400> 167
gaagtgcagc tggtggagtc tgggggagtc gtggtacagc ctggggggtc cctgagactc 60
tcctgtgcag cctctggatt cacctttgat 90

<210> 168
<211> 90
<212> DNA
<213> Homo sapiens

<400> 168
gaggtgcagc tggtggagtc tgggggaggc ttggtacagc ctggggggtc cctgagactc 60
tcctgtgcag cctctggatt caccttcagt 90

<210> 169
<211> 90
<212> DNA
<213> Homo sapiens

<400> 169
gaggtgcagc tggtggagtc tgggggaggc ttggtacagc cagggcggtc cctgagactc 60
tcctgtacag cttctggatt cacctttggt 90

<210> 170
<211> 90
<212> DNA
<213> Homo sapiens

<400> 170

gaggtgcagc tggtggagtc tggaggaggc ttgatccagc ctggggggtc cctgagactc 60
tcctgtgcag cctctgggtt caccgtcagt 90

<210> 171
<211> 90
<212> DNA
<213> Homo sapiens

<400> 171
gaggtgcagc tggtggagtc tggggaaggc ttggtccagc ctggggggtc cctgagactc 60
tcctgtgcag cctctggatt caccttcagt 90

<210> 172
<211> 90
<212> DNA
<213> Homo sapiens

<400> 172
gaggtgcagc tggtggagtc tggaggaggc ttgatccagc ctggggggtc cctgagactc 60
tcctgtgcag cctctgggtt caccgtcagt 90

<210> 173
<211> 90
<212> DNA
<213> Homo sapiens

<400> 173
gaggtgcagc tggtggagtc tgggggaggc ttggtccagc ctggggggtc cctgagactc 60
tcctgtgcag cctctggatt cacctttagt 90

<210> 174
<211> 90
<212> DNA
<213> Homo sapiens

<400> 174
gaggtgcagc tggtggagtc tgggggaggc ttggtccagc ctggagggtc cctgagactc 60
tcctgtgcag cctctggatt caccttcagt 90

<210> 175
<211> 90
<212> DNA
<213> Homo sapiens

<400> 175
gaggtgcagc tggtggagtc cgggggaggc ttggtccagc ctggggggtc cctgaaactc 60
tcctgtgcag cctctgggtt caccttcagt 90

<210> 176
<211> 90
<212> DNA
<213> Homo sapiens

<400> 176
gaggtgcagc tggtggagtc cgggggaggc ttagttcagc ctggggggtc cctgagactc 60
tcctgtgcag cctctggatt caccttcagt 90

<210> 177
<211> 90
<212> DNA
<213> Homo sapiens

<400> 177
gaagtgcagc tggtggagtc tgggggaggc ttggtacagc ctggcaggtc cctgagactc 60
tcctgtgcag cctctggatt cacctttgat 90

<210> 178
<211> 90

<212> DNA
<213> Homo sapiens

<400> 178
caggtgcagc tgcaggagtc gggcccagga ctggtgaagc cttcggacac cctgtccctc 60
acctgcgctg tctctggtta ctccatcagc 90

<210> 179
<211> 90
<212> DNA
<213> Homo sapiens

<400> 179
caggtgcagc tgcaggagtc gggcccagga ctggtgaagc cttcacagac cctgtccctc 60
acctgtactg tctctggtgg ctccatcagc 90

<210> 180
<211> 90
<212> DNA
<213> Homo sapiens

<400> 180
caggtgcagc tacagcagtg gggcgcagga ctgttgaagc cttcggagac cctgtccctc 60
acctgcgctg tctatggtgg gtccttcagt 90

<210> 181
<211> 90
<212> DNA
<213> Homo sapiens

<400> 181
cagctgcagc tgcaggagtc gggcccagga ctggtgaagc cttcggagac cctgtccctc 60
acctgcactg tctctggtgg ctccatcagc 90

<210> 182
<211> 90
<212> DNA
<213> Homo sapiens

<400> 182
caggtgcagc tgcaggagtc gggcccagga ctggtgaagc cttcggagac cctgtccctc 60
acctgcactg tctctggtgg ctccatcagt 90

<210> 183
<211> 90
<212> DNA
<213> Homo sapiens

<400> 183
caggtgcagc tgcaggagtc gggcccagga ctggtgaagc cttcggagac cctgtccctc 60
acctgcactg tctctggtgg ctccatcagt 90

<210> 184
<211> 90
<212> DNA
<213> Homo sapiens

<400> 184
caggtgcagc tgcaggagtc gggcccagga ctggtgaagc cttcggagac cctgtccctc 60
acctgcactg tctctggtgg ctccgtcagc 90

<210> 185
<211> 90
<212> DNA
<213> Homo sapiens

<400> 185
gaggtgcagc tggtgcagtc tggagcagag gtgaaaaagc ccggggagtc tctgaagatc 60

tcctgtaagg gttctggata cagctttacc 90

<210> 186
<211> 90
<212> DNA
<213> Homo sapiens

<400> 186
caggtacagc tgcagcagtc aggtccagga ctggtgaagc cctcgcagac cctctcactc 60
acctgtgcca tctccgggga cagtgtctct 90

<210> 187
<211> 90
<212> DNA
<213> Homo sapiens

<400> 187
caggtgcagc tggtgcagtc tggccatgag gtgaagcagc ctggggcctc agtgaaggtc 60
tcctgcaagg cttctggtta cagtttcacc 90

<210> 188
<211> 42
<212> DNA
<213> Homo sapiens

<400> 188
tgggtgcgac aggcccctgg acaagggctt gagtggatgg ga 42

<210> 189
<211> 42
<212> DNA
<213> Homo sapiens

<400> 189
tgggtgcgac aggcccctgg acaagggctt gagtggatgg ga 42

<210> 190
<211> 42
<212> DNA
<213> Homo sapiens

<400> 190
tgggtgcgac aggctcctgg aaaagggctt gagtggatgg ga 42

<210> 191
<211> 42
<212> DNA
<213> Homo sapiens

<400> 191
tgggtgcgcc aggcccccgg acaaaggctt gagtggatgg ga 42

<210> 192
<211> 42
<212> DNA
<213> Homo sapiens

<400> 192
tgggtgcgac aggcccccgg acaagcgctt gagtggatgg ga 42

<210> 193
<211> 42
<212> DNA
<213> Homo sapiens

<400> 193
tgggtgcgac aggcccctgg acaagggctt gagtggatgg ga 42

<210> 194
<211> 42
<212> DNA
<213> Homo sapiens

<400> 194
tgggtgcgac aggctcgtgg acaacgcctt gagtggatag ga 42

<210> 195
<211> 42
<212> DNA
<213> Homo sapiens

<400> 195
tgggtgcgac aggcccctgg acaagggctt gagtggatgg ga 42

<210> 196
<211> 42
<212> DNA
<213> Homo sapiens

<400> 196
tgggtgcgac aggccactgg acaagggctt gagtggatgg ga 42

<210> 197
<211> 42
<212> DNA
<213> Homo sapiens

<400> 197
tggatccgtc agcccccagg gaaggccctg gagtggcttg ca 42

<210> 198
<211> 42
<212> DNA
<213> Homo sapiens

<400> 198
tggatccgtc agcccccagg aaaggccctg gagtggcttg ca 42

<210> 199
<211> 42
<212> DNA
<213> Homo sapiens

<400> 199
tggatccgtc agcccccagg gaaggccctg gagtggcttg ca 42

<210> 200
<211> 42
<212> DNA
<213> Homo sapiens

<400> 200
tggatccgcc aggctccagg gaaggggctg gagtgggttt ca 42

<210> 201
<211> 42
<212> DNA
<213> Homo sapiens

<400> 201
tgggtccgcc aagctacagg aaaaggtctg gagtgggtct ca 42

<210> 202
<211> 42
<212> DNA
<213> Homo sapiens

<400> 202
tgggtccgcc aggctccagg gaaggggctg gagtgggttg gc 42

<210> 203
<211> 42
<212> DNA
<213> Homo sapiens

<400> 203
tgggcccgca aggctccagg aaaggggctg gagtgggtat cg 42

<210> 204
<211> 42
<212> DNA
<213> Homo sapiens

<400> 204
tgggtccgcc aagctccagg gaaggggctg gagtgggtct ct 42

<210> 205
<211> 42
<212> DNA
<213> Homo sapiens

<400> 205
tgggtccgcc aggctccagg gaaggggctg gagtgggtct ca 42

<210> 206
<211> 42
<212> DNA
<213> Homo sapiens

<400> 206
tgggtccgcc aggctccagg gaaggggctg gagtgggtct ca 42

<210> 207
<211> 42
<212> DNA
<213> Homo sapiens

<400> 207
tgggtccgcc aggctccagg caaggggctg gagtgggtgg ca 42

<210> 208
<211> 42
<212> DNA
<213> Homo sapiens

<400> 208
tgggtccgcc aggctccagg caaggggctg gagtgggtgg ca 42

<210> 209
<211> 42
<212> DNA
<213> Homo sapiens

<400> 209
tgggtccatc aggctccagg aaaggggctg gagtgggtat cg 42

<210> 210
<211> 42
<212> DNA
<213> Homo sapiens

<400> 210
tggatccgcc aggctccagg gaaggggctg gagtgggtct ca 42

<210> 211
<211> 42
<212> DNA
<213> Homo sapiens

<400> 211
tgggtccgtc aagctccggg gaagggtctg gagtgggtct ct                42

<210> 212
<211> 42
<212> DNA
<213> Homo sapiens

<400> 212
tgggtccgcc aggctccagg gaaggggctg gagtgggttt ca                42

<210> 213
<211> 42
<212> DNA
<213> Homo sapiens

<400> 213
tggttccgcc aggctccagg gaaggggctg gagtgggtag gt                42

<210> 214
<211> 42
<212> DNA
<213> Homo sapiens

<400> 214
tgggtccgcc aggctccagg gaaggggctg gagtgggtct ca                42

<210> 215
<211> 42
<212> DNA
<213> Homo sapiens

<400> 215
tgggtccgcc aggctccagg gaagggactg gaatatgttt ca                42

<210> 216
<211> 42
<212> DNA
<213> Homo sapiens

<400> 216
tgggtccgcc aggctccagg gaaggggctg gagtgggtct ca                42

<210> 217
<211> 42
<212> DNA
<213> Homo sapiens

<400> 217
tgggtccgcc aggctccagg gaaggggctg gagtgggtgg cc                42

<210> 218
<211> 42
<212> DNA
<213> Homo sapiens

<400> 218
tgggtccgcc aggctccagg gaaggggctg gagtgggttg gc                42

<210> 219
<211> 42
<212> DNA
<213> Homo sapiens

<400> 219
tgggtccgcc aggcttccgg gaaagggctg gagtgggttg gc 42

<210> 220
<211> 42
<212> DNA
<213> Homo sapiens

<400> 220
tgggtccgcc aagctccagg gaaggggctg gtgtgggtct ca 42

<210> 221
<211> 42
<212> DNA
<213> Homo sapiens

<400> 221
tgggtccggc aagctccagg gaagggcctg gagtgggtct ca 42

<210> 222
<211> 42
<212> DNA
<213> Homo sapiens

<400> 222
tggatccggc agcccccagg gaagggactg gagtggattg gg 42

<210> 223
<211> 42
<212> DNA
<213> Homo sapiens

<400> 223
tggatccgcc agcacccagg gaagggcctg gagtggattg gg 42

<210> 224
<211> 42
<212> DNA
<213> Homo sapiens

<400> 224
tggatccgcc agcccccagg gaaggggctg gagtggattg gg 42

<210> 225
<211> 42
<212> DNA
<213> Homo sapiens

<400> 225
tggatccgcc agcccccagg gaaggggctg gagtggattg gg 42

<210> 226
<211> 42
<212> DNA
<213> Homo sapiens

<400> 226
tggatccggc agcccgccgg gaagggactg gagtggattg gg 42

<210> 227
<211> 42
<212> DNA
<213> Homo sapiens

<400> 227
tggatccggc agcccccagg gaagggactg gagtggattg gg 42

<210> 228
<211> 42
<212> DNA
<213> Homo sapiens

<400> 228
tggatccggc agcccccagg gaagggactg gagtggattg gg 42

<210> 229
<211> 42
<212> DNA
<213> Homo sapiens

<400> 229
tgggtgcgcc agatgcccgg gaaaggcctg gagtggatgg gg 42

<210> 230
<211> 42
<212> DNA
<213> Homo sapiens

<400> 230
tggatcaggc agtccccatc gagaggcctt gagtggctgg ga 42

<210> 231
<211> 42
<212> DNA
<213> Homo sapiens

<400> 231
tgggtgccac aggcccctgg acaagggctt gagtggatgg ga 42

<210> 232
<211> 96
<212> DNA
<213> Homo sapiens

<400> 232
agagtcacca tgaccacaga cacatccacg agcacagcct acatggagct gaggagcctg 60
agatctgacg acacggccgt gtattactgt gcgaga 96

<210> 233
<211> 96
<212> DNA
<213> Homo sapiens

<400> 233
agggtcacca tgaccaggga cacgtccatc agcacagcct acatggagct gagcaggctg 60
agatctgacg acacggccgt gtattactgt gcgaga 96

<210> 234
<211> 96
<212> DNA
<213> Homo sapiens

<400> 234
agagtcacca tgaccgagga cacatctaca gacacagcct acatggagct gagcagcctg 60
agatctgagg acacggccgt gtattactgt gcaaca 96

<210> 235
<211> 96
<212> DNA
<213> Homo sapiens

<400> 235
agagtcacca ttaccaggga cacatccgcg agcacagcct acatggagct gagcagcctg 60
agatctgagg acatggctgt gtattactgt gcgaga 96

<210> 236
<211> 96
<212> DNA
<213> Homo sapiens

<400> 236
agagtcacca ttaccaggga caggtctatg agcacagcct acatggagct gagcagcctg 60
agatctgagg acacagccat gtattactgt gcaaga 96

<210> 237
<211> 96
<212> DNA
<213> Homo sapiens

<400> 237
agagtcacca tgaccaggga cacgtccacg agcacagtct acatggagct gagcagcctg 60
agatctgagg acacggccgt gtattactgt gcgaga 96

<210> 238
<211> 96
<212> DNA
<213> Homo sapiens

<400> 238
agagtcacca ttaccaggga catgtccaca agcacagcct acatggagct gagcagcctg 60
agatccgagg acacggccgt gtattactgt gcggca 96

<210> 239
<211> 96
<212> DNA
<213> Homo sapiens

<400> 239
agagtcacga ttaccgcgga caaatccacg agcacagcct acatggagct gagcagcctg 60
agatctgagg acacggccgt gtattactgt gcgaga 96

<210> 240
<211> 96
<212> DNA
<213> Homo sapiens

<400> 240
agagtcacca tgaccaggaa cacctccata agcacagcct acatggagct gagcagcctg 60
agatctgagg acacggccgt gtattactgt gcgaga 96

<210> 241
<211> 96
<212> DNA
<213> Homo sapiens

<400> 241
aggctcacca tctccaagga cacctccaaa agccaggtgg tccttaccat gaccaacatg 60
gaccctgtgg acacagccac atattactgt gcacgg 96

<210> 242
<211> 96
<212> DNA
<213> Homo sapiens

<400> 242
aggctcacca tcaccaagga cacctccaaa aaccaggtgg tccttacaat gaccaacatg 60
gaccctgtgg acacagccac atattactgt gcacac 96

<210> 243
<211> 96
<212> DNA
<213> Homo sapiens

<400> 243
aggctcacca tctccaagga cacctccaaa aaccaggtgg tccttacaat gaccaacatg 60
gaccctgtgg acacagccac gtattattgt gcacgg 96

<210> 244
<211> 96
<212> DNA
<213> Homo sapiens

<400> 244
cgattcacca tctccaggga caacgccaag aactcactgt atctgcaaat gaacagcctg 60
agagccgagg acacggccgt gtattactgt gcgaga 96

<210> 245
<211> 96
<212> DNA
<213> Homo sapiens

<400> 245
cgattcacca tctccagaga aaatgccaag aactccttgt atcttcaaat gaacagcctg 60
agagccgggg acacggctgt gtattactgt gcaaga 96

<210> 246
<211> 96
<212> DNA
<213> Homo sapiens

<400> 246
agattcacca tctcaagaga tgattcaaaa aacacgctgt atctgcaaat gaacagcctg 60
aaaaccgagg acacagccgt gtattactgt accaca 96

<210> 247
<211> 96
<212> DNA
<213> Homo sapiens

<400> 247
cgattcatca tctccagaga caattccagg aactccctgt atctgcaaaa gaacagacgg 60
agagccgagg acatggctgt gtattactgt gtgaga 96

<210> 248
<211> 96
<212> DNA
<213> Homo sapiens

<400> 248
cgattcacca tctccagaga caacgccaag aactccctgt atctgcaaat gaacagtctg 60
agagccgagg acacggcctt gtatcactgt gcgaga 96

<210> 249
<211> 96
<212> DNA
<213> Homo sapiens

<400> 249
cgattcacca tctccagaga caacgccaag aactcactgt atctgcaaat gaacagcctg 60
agagccgagg acacggctgt gtattactgt gcgaga 96

<210> 250
<211> 96
<212> DNA
<213> Homo sapiens

<400> 250
cggttcacca tctccagaga caattccaag aacacgctgt atctgcaaat gaacagcctg 60
agagccgagg acacggccgt atattactgt gcgaaa 96

<210> 251

<211> 96
<212> DNA
<213> Homo sapiens

<400> 251
cgattcacca tctccagaga caattccaag aacacgctgt atctgcaaat gaacagcctg 60
agagctgagg acacggctgt gtattactgt gcgaga 96

<210> 252
<211> 96
<212> DNA
<213> Homo sapiens

<400> 252
cgattcacca tctccagaga caattccaag aacacgctgt atctgcaaat gaacagcctg 60
agagccgagg acacggctgt gtattactgt gcgaga 96

<210> 253
<211> 96
<212> DNA
<213> Homo sapiens

<400> 253
cgattcatca tctccagaga caattccagg aacaccctgt atctgcaaac gaatagcctg 60
agggccgagg acacggctgt gtattactgt gtgaga 96

<210> 254
<211> 96
<212> DNA
<213> Homo sapiens

<400> 254
agattcacca tctccagaga caattccaag aacacgctgt atcttcaaat gaacaacctg 60
agagctgagg gcacggccgt gtattactgt gccaga 96

<210> 255
<211> 96
<212> DNA
<213> Homo sapiens

<400> 255
cgattcacca tctccagaga caacagcaaa aactccctgt atctgcaaat gaacagtctg 60
agaactgagg acaccgcctt gtattactgt gcaaaa 96

<210> 256
<211> 96
<212> DNA
<213> Homo sapiens

<400> 256
cgattcacca tctccagaga caatgccaag aactcactgt atctgcaaat gaacagcctg 60
agagacgagg acacggctgt gtattactgt gcgaga 96

<210> 257
<211> 96
<212> DNA
<213> Homo sapiens

<400> 257
agattcacca tctcaagaga tgattccaaa agcatcgcct atctgcaaat gaacagcctg 60
aaaaccgagg acacagccgt gtattactgt actaga 96

<210> 258
<211> 96
<212> DNA
<213> Homo sapiens

<400> 258

cgattcacca tctccagaga caattccaag aacacgctgt atcttcaaat gaacagcctg 60
agagccgagg acacggccgt gtattactgt gcgaga 96

<210> 259
<211> 96
<212> DNA
<213> Homo sapiens

<400> 259
agattcacca tctccagaga caattccaag aacacgctgt atcttcaaat gggcagcctg 60
agagctgagg acatggctgt gtattactgt gcgaga 96

<210> 260
<211> 96
<212> DNA
<213> Homo sapiens

<400> 260
cgattcacca tctccagaga caattccaag aacacgctgt atcttcaaat gaacagcctg 60
agagctgagg acacggctgt gtattactgt gcgaga 96

<210> 261
<211> 96
<212> DNA
<213> Homo sapiens

<400> 261
cgattcacca tctccagaga caacgccaag aactcactgt atctgcaaat gaacagcctg 60
agagccgagg acacggctgt gtattactgt gcgaga 96

<210> 262
<211> 96
<212> DNA
<213> Homo sapiens

<400> 262
agattcacca tctcaagaga tgattcaaag aactcactgt atctgcaaat gaacagcctg 60
aaaaccgagg acacggccgt gtattactgt gctaga 96

<210> 263
<211> 96
<212> DNA
<213> Homo sapiens

<400> 263
aggttcacca tctccagaga tgattcaaag aacacggcgt atctgcaaat gaacagcctg 60
aaaaccgagg acacggccgt gtattactgt actaga 96

<210> 264
<211> 96
<212> DNA
<213> Homo sapiens

<400> 264
cgattcacca tctccagaga caacgccaag aacacgctgt atctgcaaat gaacagtctg 60
agagccgagg acacggctgt gtattactgt gcaaga 96

<210> 265
<211> 96
<212> DNA
<213> Homo sapiens

<400> 265
cgattcacca tctccagaga caacgccaag aactccctgt atctgcaaat gaacagtctg 60
agagctgagg acacggcctt gtattactgt gcaaaa 96

<210> 266
<211> 96

<212> DNA
<213> Homo sapiens

<400> 266
cgagtcacca tgtcagtaga cacgtccaag aaccagttct ccctgaagct gagctctgtg 60
accgccgtgg acacggccgt gtattactgt gcgaga 96

<210> 267
<211> 96
<212> DNA
<213> Homo sapiens

<400> 267
cgagttacca tatcagtaga cacgtctaag aaccagttct ccctgaagct gagctctgtg 60
actgccgcgg acacggccgt gtattactgt gcgaga 96

<210> 268
<211> 96
<212> DNA
<213> Homo sapiens

<400> 268
cgagtcacca tatcagtaga cacgtccaag aaccagttct ccctgaagct gagctctgtg 60
accgccgcgg acacggctgt gtattactgt gcgaga 96

<210> 269
<211> 96
<212> DNA
<213> Homo sapiens

<400> 269
cgagtcacca tatccgtaga cacgtccaag aaccagttct ccctgaagct gagctctgtg 60
accgccgcag acacggctgt gtattactgt gcgaga 96

<210> 270
<211> 96
<212> DNA
<213> Homo sapiens

<400> 270
cgagtcacca tgtcagtaga cacgtccaag aaccagttct ccctgaagct gagctctgtg 60
accgccgcgg acacggccgt gtattactgt gcgaga 96

<210> 271
<211> 96
<212> DNA
<213> Homo sapiens

<400> 271
cgagtcacca tatcagtaga cacgtccaag aaccagttct ccctgaagct gagctctgtg 60
accgctgcgg acacggccgt gtattactgt gcgaga 96

<210> 272
<211> 96
<212> DNA
<213> Homo sapiens

<400> 272
cgagtcacca tatcagtaga cacgtccaag aaccagttct ccctgaagct gagctctgtg 60
accgctgcgg acacggccgt gtattactgt gcgaga 96

<210> 273
<211> 96
<212> DNA
<213> Homo sapiens

<400> 273
caggtcacca tctcagccga caagtccatc agcaccgcct acctgcagtg gagcagcctg 60

aaggcctcgg acaccgccat gtattactgt gcgaga 96

<210> 274
<211> 96
<212> DNA
<213> Homo sapiens

<400> 274
cgaataacca tcaacccaga cacatccaag aaccagttct ccctgcagct gaactctgtg 60
actcccgagg acacggctgt gtattactgt gcaaga 96

<210> 275
<211> 96
<212> DNA
<213> Homo sapiens

<400> 275
cggtttgtct tctccatgga cacctctgcc agcacagcat acctgcagat cagcagccta 60
aaggctgagg acatggccat gtattactgt gcgaga 96

<210> 276
<211> 75
<212> DNA
<213> Homo sapiens

<400> 276
caggttcagc tggtgcagtc tggagctgag gtgaagaagc ctggggcctc agtgaaggtc 60
tcctgcaagg cttct 75

<210> 277
<211> 75
<212> DNA
<213> Homo sapiens

<400> 277
caggtgcagc tggtgcagtc tggggctgag gtgaagaagc ctggggcctc agtgaaggtc 60
tcctgcaagg cttct 75

<210> 278
<211> 75
<212> DNA
<213> Homo sapiens

<400> 278
caggtccagc tggtacagtc tggggctgag gtgaagaagc ctggggcctc agtgaaggtc 60
tcctgcaagg tttcc 75

<210> 279
<211> 75
<212> DNA
<213> Homo sapiens

<400> 279
caggttcagc tggtgcagtc tggggctgag gtgaagaagc ctggggcctc agtgaaggtt 60
tcctgcaagg cttct 75

<210> 280
<211> 75
<212> DNA
<213> Homo sapiens

<400> 280
cagatgcagc tggtgcagtc tggggctgag gtgaagaaga ctgggtcctc agtgaaggtt 60
tcctgcaagg cttcc 75

<210> 281
<211> 75
<212> DNA

<213> Homo sapiens

<400> 281
caggtgcagc tggtgcagtc tggggctgag gtgaagaagc ctggggcctc agtgaaggtt 60
tcctgcaagg catct 75

<210> 282
<211> 75
<212> DNA
<213> Homo sapiens

<400> 282
caaatgcagc tggtgcagtc tgggcctgag gtgaagaagc ctgggacctc agtgaaggtc 60
tcctgcaagg cttct 75

<210> 283
<211> 75
<212> DNA
<213> Homo sapiens

<400> 283
caggtgcagc tggtgcagtc tggggctgag gtgaagaagc ctgggtcctc ggtgaaggtc 60
tcctgcaagg cttct 75

<210> 284
<211> 75
<212> DNA
<213> Homo sapiens

<400> 284
caggtgcagc tggtgcagtc tggggctgag gtgaagaagc ctggggcctc agtgaaggtc 60
tcctgcaagg cttct 75

<210> 285
<211> 75
<212> DNA
<213> Homo sapiens

<400> 285
caggtcacct tgaaggagtc tggtcctgtg ctggtgaaac ccacagagac cctcacgctg 60
acctgcaccg tctct 75

<210> 286
<211> 75
<212> DNA
<213> Homo sapiens

<400> 286
cagatcacct tgaaggagtc tggtcctacg ctggtgaaac ccacacagac cctcacgctg 60
acctgcacct tctct 75

<210> 287
<211> 75
<212> DNA
<213> Homo sapiens

<400> 287
caggtcacct tgagggagtc tggtcctgcg ctggtgaaac ccacacagac cctcacactg 60
acctgcacct tctct 75

<210> 288
<211> 75
<212> DNA
<213> Homo sapiens

<400> 288
caggtgcagc tggtggagtc tgggggaggc ttggtcaagc ctggagggtc cctgagactc 60
tcctgtgcag cctct 75

<210> 289
<211> 75
<212> DNA
<213> Homo sapiens

<400> 289
gaggtgcagc tggtggagtc tgggggaggc ttggtacagc ctggggggtc cctgagactc 60
tcctgtgcag cctct 75

<210> 290
<211> 75
<212> DNA
<213> Homo sapiens

<400> 290
gaggtgcagc tggtggagtc tgggggaggc ttggtaaagc ctggggggtc ccttagactc 60
tcctgtgcag cctct 75

<210> 291
<211> 75
<212> DNA
<213> Homo sapiens

<400> 291
gaggtgcagc tggtggagtc tgggggaggc ttggtacagc ctggggggtc cctgagactc 60
tcctgtgcag cctct 75

<210> 292
<211> 75
<212> DNA
<213> Homo sapiens

<400> 292
gaggtgcagc tggtggagtc tgggggaggt gtggtacggc ctggggggtc cctgagactc 60
tcctgtgcag cctct 75

<210> 293
<211> 75
<212> DNA
<213> Homo sapiens

<400> 293
gaggtgcagc tggtggagtc tgggggaggc ctggtcaagc ctggggggtc cctgagactc 60
tcctgtgcag cctct 75

<210> 294
<211> 75
<212> DNA
<213> Homo sapiens

<400> 294
gaggtgcagc tgttggagtc tgggggaggc ttggtacagc ctggggggtc cctgagactc 60
tcctgtgcag cctct 75

<210> 295
<211> 75
<212> DNA
<213> Homo sapiens

<400> 295
caggtgcagc tggtggagtc tgggggaggc gtggtccagc ctgggaggtc cctgagactc 60
tcctgtgcag cctct 75

<210> 296
<211> 75
<212> DNA
<213> Homo sapiens

<400> 296
caggtgcagc tggtggagtc tgggggaggc gtggtccagc ctgggaggtc cctgagactc 60
tcctgtgcag cgtct 75

<210> 297
<211> 75
<212> DNA
<213> Homo sapiens

<400> 297
gaggtgcagc tggtggagtc tgggggaggc ttggtacagc ctgggggatc cctgagactc 60
tcctgtgcag cctct 75

<210> 298
<211> 75
<212> DNA
<213> Homo sapiens

<400> 298
gaggtgcagc tggtggagtc tgggggaggc ttggtacagc ctagggggtc cctgagactc 60
tcctgtgcag cctct 75

<210> 299
<211> 75
<212> DNA
<213> Homo sapiens

<400> 299
gaagtgcagc tggtggagtc tgggggagtc gtggtacagc ctggggggtc cctgagactc 60
tcctgtgcag cctct 75

<210> 300
<211> 75
<212> DNA
<213> Homo sapiens

<400> 300
gaggtgcagc tggtggagtc tgggggaggc ttggtacagc ctggggggtc cctgagactc 60
tcctgtgcag cctct 75

<210> 301
<211> 75
<212> DNA
<213> Homo sapiens

<400> 301
gaggtgcagc tggtggagtc tgggggaggc ttggtacagc cagggcggtc cctgagactc 60
tcctgtacag cttct 75

<210> 302
<211> 75
<212> DNA
<213> Homo sapiens

<400> 302
gaggtgcagc tggtggagtc tggaggaggc ttgatccagc ctggggggtc cctgagactc 60
tcctgtgcag cctct 75

<210> 303
<211> 75
<212> DNA
<213> Homo sapiens

<400> 303
gaggtgcagc tggtggagtc tggggaaggc ttggtccagc ctggggggtc cctgagactc 60
tcctgtgcag cctct 75

<210> 304
<211> 75
<212> DNA
<213> Homo sapiens

<400> 304
gaggtgcagc tggtggagtc tggaggaggc ttgatccagc ctggggggtc cctgagactc 60
tcctgtgcag cctct 75

<210> 305
<211> 75
<212> DNA
<213> Homo sapiens

<400> 305
gaggtgcagc tggtggagtc tgggggaggc ttggtccagc ctggggggtc cctgagactc 60
tcctgtgcag cctct 75

<210> 306
<211> 75
<212> DNA
<213> Homo sapiens

<400> 306
gaggtgcagc tggtggagtc tgggggaggc ttggtccagc ctggagggtc cctgagactc 60
tcctgtgcag cctct 75

<210> 307
<211> 75
<212> DNA
<213> Homo sapiens

<400> 307
gaggtgcagc tggtggagtc cgggggaggc ttggtccagc ctggggggtc cctgaaactc 60
tcctgtgcag cctct 75

<210> 308
<211> 75
<212> DNA
<213> Homo sapiens

<400> 308
gaggtgcagc tggtggagtc cgggggaggc ttagttcagc ctggggggtc cctgagactc 60
tcctgtgcag cctct 75

<210> 309
<211> 75
<212> DNA
<213> Homo sapiens

<400> 309
gaagtgcagc tggtggagtc tgggggaggc ttggtacagc ctggcaggtc cctgagactc 60
tcctgtgcag cctct 75

<210> 310
<211> 75
<212> DNA
<213> Homo sapiens

<400> 310
caggtgcagc tgcaggagtc gggcccagga ctggtgaagc cttcggacac cctgtccctc 60
acctgcgctg tctct 75

<210> 311
<211> 75
<212> DNA
<213> Homo sapiens

<400> 311
caggtgcagc tgcaggagtc gggcccagga ctggtgaagc cttcacagac cctgtccctc 60
acctgtactg tctct 75

<210> 312
<211> 75
<212> DNA
<213> Homo sapiens

<400> 312
caggtgcagc tacagcagtg gggcgcagga ctgttgaagc cttcggagac cctgtccctc 60
acctgcgctg tctat 75

<210> 313
<211> 75
<212> DNA
<213> Homo sapiens

<400> 313
cagctgcagc tgcaggagtc gggcccagga ctggtgaagc cttcggagac cctgtccctc 60
acctgcactg tctct 75

<210> 314
<211> 75
<212> DNA
<213> Homo sapiens

<400> 314
caggtgcagc tgcaggagtc gggcccagga ctggtgaagc cttcggagac cctgtccctc 60
acctgcactg tctct 75

<210> 315
<211> 75
<212> DNA
<213> Homo sapiens

<400> 315
caggtgcagc tgcaggagtc gggcccagga ctggtgaagc cttcggagac cctgtccctc 60
acctgcactg tctct 75

<210> 316
<211> 75
<212> DNA
<213> Homo sapiens

<400> 316
caggtgcagc tgcaggagtc gggcccagga ctggtgaagc cttcggagac cctgtccctc 60
acctgcactg tctct 75

<210> 317
<211> 75
<212> DNA
<213> Homo sapiens

<400> 317
gaggtgcagc tggtgcagtc tggagcagag gtgaaaaagc ccggggagtc tctgaagatc 60
tcctgtaagg gttct 75

<210> 318
<211> 75
<212> DNA
<213> Homo sapiens

<400> 318
caggtacagc tgcagcagtc aggtccagga ctggtgaagc cctcgcagac cctctcactc 60
acctgtgcca tctcc 75

<210> 319

<211> 75
<212> DNA
<213> Homo sapiens

<400> 319
caggtgcagc tggtgcagtc tggccatgag gtgaagcagc ctggggcctc agtgaaggtc 60
tcctgcaagg cttct 75

<210> 320
<211> 60
<212> DNA
<213> Homo sapiens

<400> 320
tatggtatca gctgggtgcg acaggcccct ggacaagggc ttgagtggat gggatggatc 60

<210> 321
<211> 60
<212> DNA
<213> Homo sapiens

<400> 321
tactatatgc actgggtgcg acaggcccct ggacaagggc ttgagtggat gggatggatc 60

<210> 322
<211> 60
<212> DNA
<213> Homo sapiens

<400> 322
ttatccatgc actgggtgcg acaggctcct ggaaaagggc ttgagtggat gggaggtttt 60

<210> 323
<211> 60
<212> DNA
<213> Homo sapiens

<400> 323
tatgctatgc attgggtgcg ccaggccccc ggacaaaggc ttgagtggat gggatggagc 60

<210> 324
<211> 60
<212> DNA
<213> Homo sapiens

<400> 324
cgctacctgc actgggtgcg acaggccccc ggacaagcgc ttgagtggat gggatggatc 60

<210> 325
<211> 60
<212> DNA
<213> Homo sapiens

<400> 325
tactatatgc actgggtgcg acaggcccct ggacaagggc ttgagtggat gggaataatc 60

<210> 326
<211> 60
<212> DNA
<213> Homo sapiens

<400> 326

tctgctatgc agtgggtgcg acaggctcgt ggacaacgcc ttgagtggat aggatggatc 60

<210> 327
<211> 60
<212> DNA
<213> Homo sapiens

<400> 327
tatgctatca gctgggtgcg acaggcccct ggacaagggc ttgagtggat gggagggatc 60

<210> 328
<211> 60
<212> DNA
<213> Homo sapiens

<400> 328
tatgatatca actgggtgcg acaggccact ggacaagggc ttgagtggat gggatggatg 60

<210> 329
<211> 60
<212> DNA
<213> Homo sapiens

<400> 329
atgggtgtga gctggatccg tcagccccca gggaaggccc tggagtggct tgcacacatt 60

<210> 330
<211> 60
<212> DNA
<213> Homo sapiens

<400> 330
gtgggtgtgg gctggatccg tcagccccca ggaaaggccc tggagtggct tgcactcatt 60

<210> 331
<211> 60
<212> DNA
<213> Homo sapiens

<400> 331
atgtgtgtga gctggatccg tcagccccca gggaaggccc tggagtggct tgcactcatt 60

<210> 332
<211> 60
<212> DNA
<213> Homo sapiens

<400> 332
tactacatga gctggatccg ccaggctcca gggaaggggc tggagtgggt ttcatacatt 60

<210> 333
<211> 60
<212> DNA
<213> Homo sapiens

<400> 333
tacgacatgc actgggtccg ccaagctaca ggaaaaggtc tggagtgggt ctcagctatt 60

<210> 334
<211> 60

<212> DNA
<213> Homo sapiens

<400> 334
gcctggatga gctgggtccg ccaggctcca gggaaggggc tggagtgggt tggccgtatt 60


<210> 335
<211> 60
<212> DNA
<213> Homo sapiens

<400> 335
agtgacatga actgggcccg caaggctcca ggaaaggggc tggagtgggt atcgggtgtt 60


<210> 336
<211> 60
<212> DNA
<213> Homo sapiens

<400> 336
tatggcatga gctgggtccg ccaagctcca gggaaggggc tggagtgggt ctctggtatt 60


<210> 337
<211> 60
<212> DNA
<213> Homo sapiens

<400> 337
tatagcatga actgggtccg ccaggctcca gggaaggggc tggagtgggt ctcatccatt 60


<210> 338
<211> 60
<212> DNA
<213> Homo sapiens

<400> 338
tatgccatga gctgggtccg ccaggctcca gggaaggggc tggagtgggt ctcagctatt 60


<210> 339
<211> 60
<212> DNA
<213> Homo sapiens

<400> 339
tatggcatgc actgggtccg ccaggctcca ggcaaggggc tggagtgggt ggcagttata 60


<210> 340
<211> 60
<212> DNA
<213> Homo sapiens

<400> 340
tatggcatgc actgggtccg ccaggctcca ggcaaggggc tggagtgggt ggcagttata 60


<210> 341
<211> 60
<212> DNA
<213> Homo sapiens

<400> 341
agtgacatga actgggtcca tcaggctcca ggaaaggggc tggagtgggt atcgggtgtt 60

<210> 342
<211> 60
<212> DNA
<213> Homo sapiens

<400> 342
aatgagatga gctggatccg ccaggctcca gggaaggggc tggagtgggt ctcatccatt 60


<210> 343
<211> 60
<212> DNA
<213> Homo sapiens

<400> 343
tataccatgc actgggtccg tcaagctccg gggaagggtc tggagtgggt ctctcttatt 60


<210> 344
<211> 60
<212> DNA
<213> Homo sapiens

<400> 344
tatagcatga actgggtccg ccaggctcca gggaaggggc tggagtgggt ttcatacatt 60


<210> 345
<211> 60
<212> DNA
<213> Homo sapiens

<400> 345
tatgctatga gctggttccg ccaggctcca gggaaggggc tggagtgggt aggtttcatt 60


<210> 346
<211> 60
<212> DNA
<213> Homo sapiens

<400> 346
aactacatga gctgggtccg ccaggctcca gggaaggggc tggagtgggt ctcagttatt 60


<210> 347
<211> 60
<212> DNA
<213> Homo sapiens

<400> 347
tatgctatgc actgggtccg ccaggctcca gggaagggac tggaatatgt ttcagctatt 60


<210> 348
<211> 60
<212> DNA
<213> Homo sapiens

<400> 348
aactacatga gctgggtccg ccaggctcca gggaaggggc tggagtgggt ctcagttatt 60


<210> 349
<211> 60
<212> DNA

<213> Homo sapiens

<400> 349
tattggatga gctgggtccg ccaggctcca gggaaggggc tggagtgggt ggccaacata 60


<210> 350
<211> 60
<212> DNA
<213> Homo sapiens

<400> 350
cactacatgg actgggtccg ccaggctcca gggaaggggc tggagtgggt tggccgtact 60


<210> 351
<211> 60
<212> DNA
<213> Homo sapiens

<400> 351
tctgctatgc actgggtccg ccaggcttcc gggaaagggc tggagtgggt tggccgtatt 60


<210> 352
<211> 60
<212> DNA
<213> Homo sapiens

<400> 352
tactggatgc actgggtccg ccaagctcca gggaaggggc tggtgtgggt ctcacgtatt 60


<210> 353
<211> 60
<212> DNA
<213> Homo sapiens

<400> 353
tatgccatgc actgggtccg gcaagctcca gggaagggcc tggagtgggt ctcaggtatt 60


<210> 354
<211> 60
<212> DNA
<213> Homo sapiens

<400> 354
aactggtggg gctggatccg gcagccccca gggaagggac tggagtggat tgggtacatc 60


<210> 355
<211> 60
<212> DNA
<213> Homo sapiens

<400> 355
tactactgga gctggatccg ccagcaccca gggaagggcc tggagtggat tgggtacatc 60


<210> 356
<211> 60
<212> DNA
<213> Homo sapiens

<400> 356
tactactgga gctggatccg ccagccccca gggaaggggc tggagtggat tggggaaatc 60

<210> 357
<211> 60
<212> DNA
<213> Homo sapiens

<400> 357
tactactggg gctggatccg ccagccccca gggaaggggc tggagtggat tgggagtatc 60


<210> 358
<211> 60
<212> DNA
<213> Homo sapiens

<400> 358
tactactgga gctggatccg gcagcccgcc gggaagggac tggagtggat tgggcgtatc 60


<210> 359
<211> 60
<212> DNA
<213> Homo sapiens

<400> 359
tactactgga gctggatccg gcagccccca gggaagggac tggagtggat tgggtatatc 60


<210> 360
<211> 60
<212> DNA
<213> Homo sapiens

<400> 360
tactactgga gctggatccg gcagccccca gggaagggac tggagtggat tgggtatatc 60


<210> 361
<211> 60
<212> DNA
<213> Homo sapiens

<400> 361
tactggatcg gctgggtgcg ccagatgccc gggaaaggcc tggagtggat ggggatcatc 60


<210> 362
<211> 60
<212> DNA
<213> Homo sapiens

<400> 362
gctgcttgga actggatcag gcagtcccca tcgagaggcc ttgagtggct gggaaggaca 60


<210> 363
<211> 60
<212> DNA
<213> Homo sapiens

<400> 363
tatggtatga attgggtgcc acaggcccct ggacaagggc ttgagtggat gggatggttc 60


<210> 364
<211> 123
<212> DNA
<213> Homo sapiens

<400> 364
acaaactatg cacagaagct ccagggcaga gtcaccatga ccacagacac atccacgagc 60
acagcctaca tggagctgag gagcctgaga tctgacgaca cggccgtgta ttactgtgcg 120
aga 123

<210> 365
<211> 123
<212> DNA
<213> Homo sapiens

<400> 365
acaaactatg cacagaagtt tcagggcagg gtcaccatga ccagggacac gtccatcagc 60
acagcctaca tggagctgag caggctgaga tctgacgaca cggccgtgta ttactgtgcg 120
aga 123

<210> 366
<211> 123
<212> DNA
<213> Homo sapiens

<400> 366
acaatctacg cacagaagtt ccagggcaga gtcaccatga ccgaggacac atctacagac 60
acagcctaca tggagctgag cagcctgaga tctgaggaca cggccgtgta ttactgtgca 120
aca 123

<210> 367
<211> 123
<212> DNA
<213> Homo sapiens

<400> 367
acaaaatatt cacaggagtt ccagggcaga gtcaccatta ccagggacac atccgcgagc 60
acagcctaca tggagctgag cagcctgaga tctgaggaca tggctgtgta ttactgtgcg 120
aga 123

<210> 368
<211> 123
<212> DNA
<213> Homo sapiens

<400> 368
accaactacg cacagaaatt ccaggacaga gtcaccatta ccagggacag gtctatgagc 60
acagcctaca tggagctgag cagcctgaga tctgaggaca cagccatgta ttactgtgca 120
aga 123

<210> 369
<211> 123
<212> DNA
<213> Homo sapiens

<400> 369
acaagctacg cacagaagtt ccagggcaga gtcaccatga ccagggacac gtccacgagc 60
acagtctaca tggagctgag cagcctgaga tctgaggaca cggccgtgta ttactgtgcg 120
aga 123

<210> 370
<211> 123
<212> DNA
<213> Homo sapiens

<400> 370
acaaactacg cacagaagtt ccaggaaaga gtcaccatta ccagggacat gtccacaagc 60
acagcctaca tggagctgag cagcctgaga tccgaggaca cggccgtgta ttactgtgcg 120
gca 123

<210> 371
<211> 123

<212> DNA
<213> Homo sapiens

<400> 371
gcaaactacg cacagaagtt ccagggcaga gtcacgatta ccgcggacaa atccacgagc 60
acagcctaca tggagctgag cagcctgaga tctgaggaca cggccgtgta ttactgtgcg 120
aga 123

<210> 372
<211> 123
<212> DNA
<213> Homo sapiens

<400> 372
acaggctatg cacagaagtt ccagggcaga gtcaccatga ccaggaacac ctccataagc 60
acagcctaca tggagctgag cagcctgaga tctgaggaca cggccgtgta ttactgtgcg 120
aga 123

<210> 373
<211> 123
<212> DNA
<213> Homo sapiens

<400> 373
aaatcctaca gcacatctct gaagagcagg ctcaccatct ccaaggacac ctccaaaagc 60
caggtggtcc ttaccatgac caacatggac cctgtggaca cagccacata ttactgtgca 120
cgg 123

<210> 374
<211> 123
<212> DNA
<213> Homo sapiens

<400> 374
aagcgctaca gcccatctct gaagagcagg ctcaccatca ccaaggacac ctccaaaaac 60
caggtggtcc ttacaatgac caacatggac cctgtggaca cagccacata ttactgtgca 120
cac 123

<210> 375
<211> 123
<212> DNA
<213> Homo sapiens

<400> 375
aaatactaca gcacatctct gaagaccagg ctcaccatct ccaaggacac ctccaaaaac 60
caggtggtcc ttacaatgac caacatggac cctgtggaca cagccacgta ttattgtgca 120
cgg 123

<210> 376
<211> 123
<212> DNA
<213> Homo sapiens

<400> 376
atatactacg cagactctgt gaagggccga ttcaccatct ccagggacaa cgccaagaac 60
tcactgtatc tgcaaatgaa cagcctgaga gccgaggaca cggccgtgta ttactgtgcg 120
aga 123

<210> 377
<211> 123
<212> DNA
<213> Homo sapiens

<400> 377
acatactatc caggctccgt gaagggccga ttcaccatct ccagagaaaa tgccaagaac 60
tccttgtatc ttcaaatgaa cagcctgaga gccggggaca cggctgtgta ttactgtgca 120
aga 123

<210> 378
<211> 123
<212> DNA
<213> Homo sapiens

<400> 378
acagactacg ctgcacccgt gaaaggcaga ttcaccatct caagagatga ttcaaaaaac 60
acgctgtatc tgcaaatgaa cagcctgaaa accgaggaca cagccgtgta ttactgtacc 120
aca 123

<210> 379
<211> 123
<212> DNA
<213> Homo sapiens

<400> 379
acgcactatg tggactccgt gaagcgccga ttcatcatct ccagagacaa ttccaggaac 60
tccctgtatc tgcaaaagaa cagacggaga gccgaggaca tggctgtgta ttactgtgtg 120
aga 123

<210> 380
<211> 123
<212> DNA
<213> Homo sapiens

<400> 380
acaggttatg cagactctgt gaagggccga ttcaccatct ccagagacaa cgccaagaac 60
tccctgtatc tgcaaatgaa cagtctgaga gccgaggaca cggccttgta tcactgtgcg 120
aga 123

<210> 381
<211> 123
<212> DNA
<213> Homo sapiens

<400> 381
atatactacg cagactcagt gaagggccga ttcaccatct ccagagacaa cgccaagaac 60
tcactgtatc tgcaaatgaa cagcctgaga gccgaggaca cggctgtgta ttactgtgcg 120
aga 123

<210> 382
<211> 123
<212> DNA
<213> Homo sapiens

<400> 382
acatactacg cagactccgt gaagggccgg ttcaccatct ccagagacaa ttccaagaac 60
acgctgtatc tgcaaatgaa cagcctgaga gccgaggaca cggccgtata ttactgtgcg 120
aaa 123

<210> 383
<211> 123
<212> DNA
<213> Homo sapiens

<400> 383
aaatactatg cagactccgt gaagggccga ttcaccatct ccagagacaa ttccaagaac 60
acgctgtatc tgcaaatgaa cagcctgaga gctgaggaca cggctgtgta ttactgtgcg 120
aga 123

<210> 384
<211> 123
<212> DNA
<213> Homo sapiens

<400> 384
aaatactatg cagactccgt gaagggccga ttcaccatct ccagagacaa ttccaagaac 60
acgctgtatc tgcaaatgaa cagcctgaga gccgaggaca cggctgtgta ttactgtgcg 120

aga                                                                 123

<210> 385
<211> 123
<212> DNA
<213> Homo sapiens

<400> 385
acgcactatg cagactctgt gaagggccga ttcatcatct ccagagacaa ttccaggaac 60
accctgtatc tgcaaacgaa tagcctgagg gccgaggaca cggctgtgta ttactgtgtg 120
aga                                                                 123

<210> 386
<211> 123
<212> DNA
<213> Homo sapiens

<400> 386
acatactacg cagactccag gaagggcaga ttcaccatct ccagagacaa ttccaagaac 60
acgctgtatc ttcaaatgaa caacctgaga gctgagggca cggccgtgta ttactgtgcc 120
aga                                                                 123

<210> 387
<211> 123
<212> DNA
<213> Homo sapiens

<400> 387
acatactatg cagactctgt gaagggccga ttcaccatct ccagagacaa cagcaaaaac 60
tccctgtatc tgcaaatgaa cagtctgaga actgaggaca ccgccttgta ttactgtgca 120
aaa                                                                 123

<210> 388
<211> 123
<212> DNA
<213> Homo sapiens

<400> 388
atatactacg cagactctgt gaagggccga ttcaccatct ccagagacaa tgccaagaac 60
tcactgtatc tgcaaatgaa cagcctgaga gacgaggaca cggctgtgta ttactgtgcg 120
aga                                                                 123

<210> 389
<211> 123
<212> DNA
<213> Homo sapiens

<400> 389
acagaatacg ccgcgtctgt gaaaggcaga ttcaccatct caagagatga ttccaaaagc 60
atcgcctatc tgcaaatgaa cagcctgaaa accgaggaca cagccgtgta ttactgtact 120
aga                                                                 123

<210> 390
<211> 123
<212> DNA
<213> Homo sapiens

<400> 390
acatactacg cagactccgt gaagggccga ttcaccatct ccagagacaa ttccaagaac 60
acgctgtatc ttcaaatgaa cagcctgaga gccgaggaca cggccgtgta ttactgtgcg 120
aga                                                                 123

<210> 391
<211> 123
<212> DNA
<213> Homo sapiens

<400> 391

acatattatg cagactctgt gaagggcaga ttcaccatct ccagagacaa ttccaagaac 60
acgctgtatc ttcaaatggg cagcctgaga gctgaggaca tggctgtgta ttactgtgcg 120
aga 123

<210> 392
<211> 123
<212> DNA
<213> Homo sapiens

<400> 392
acatactacg cagactccgt gaagggccga ttcaccatct ccagagacaa ttccaagaac 60
acgctgtatc ttcaaatgaa cagcctgaga gctgaggaca cggctgtgta ttactgtgcg 120
aga 123

<210> 393
<211> 123
<212> DNA
<213> Homo sapiens

<400> 393
aaatactatg tggactctgt gaagggccga ttcaccatct ccagagacaa cgccaagaac 60
tcactgtatc tgcaaatgaa cagcctgaga gccgaggaca cggctgtgta ttactgtgcg 120
aga 123

<210> 394
<211> 123
<212> DNA
<213> Homo sapiens

<400> 394
acagaatacg ccgcgtctgt gaaaggcaga ttcaccatct caagagatga ttcaaagaac 60
tcactgtatc tgcaaatgaa cagcctgaaa accgaggaca cggccgtgta ttactgtgct 120
aga 123

<210> 395
<211> 123
<212> DNA
<213> Homo sapiens

<400> 395
acagcatatg ctgcgtcggt gaaaggcagg ttcaccatct ccagagatga ttcaaagaac 60
acggcgtatc tgcaaatgaa cagcctgaaa accgaggaca cggccgtgta ttactgtact 120
aga 123

<210> 396
<211> 123
<212> DNA
<213> Homo sapiens

<400> 396
acaagctacg cggactccgt gaagggccga ttcaccatct ccagagacaa cgccaagaac 60
acgctgtatc tgcaaatgaa cagtctgaga gccgaggaca cggctgtgta ttactgtgca 120
aga 123

<210> 397
<211> 123
<212> DNA
<213> Homo sapiens

<400> 397
ataggctatg cggactctgt gaagggccga ttcaccatct ccagagacaa cgccaagaac 60
tccctgtatc tgcaaatgaa cagtctgaga gctgaggaca cggccttgta ttactgtgca 120
aaa 123

<210> 398
<211> 123
<212> DNA
<213> Homo sapiens

<400> 398
acctactaca acccgtccct caagagtcga gtcaccatgt cagtagacac gtccaagaac 60
cagttctccc tgaagctgag ctctgtgacc gccgtggaca cggccgtgta ttactgtgcg 120
aga 123

<210> 399
<211> 123
<212> DNA
<213> Homo sapiens

<400> 399
acctactaca acccgtccct caagagtcga gttaccatat cagtagacac gtctaagaac 60
cagttctccc tgaagctgag ctctgtgact gccgcggaca cggccgtgta ttactgtgcg 120
aga 123

<210> 400
<211> 123
<212> DNA
<213> Homo sapiens

<400> 400
accaactaca acccgtccct caagagtcga gtcaccatat cagtagacac gtccaagaac 60
cagttctccc tgaagctgag ctctgtgacc gccgcggaca cggctgtgta ttactgtgcg 120
aga 123

<210> 401
<211> 123
<212> DNA
<213> Homo sapiens

<400> 401
acctactaca acccgtccct caagagtcga gtcaccatat ccgtagacac gtccaagaac 60
cagttctccc tgaagctgag ctctgtgacc gccgcagaca cggctgtgta ttactgtgcg 120
aga 123

<210> 402
<211> 123
<212> DNA
<213> Homo sapiens

<400> 402
accaactaca acccctccct caagagtcga gtcaccatgt cagtagacac gtccaagaac 60
cagttctccc tgaagctgag ctctgtgacc gccgcggaca cggccgtgta ttactgtgcg 120
aga 123

<210> 403
<211> 123
<212> DNA
<213> Homo sapiens

<400> 403
accaactaca acccctccct caagagtcga gtcaccatat cagtagacac gtccaagaac 60
cagttctccc tgaagctgag ctctgtgacc gctgcggaca cggccgtgta ttactgtgcg 120
aga 123

<210> 404
<211> 123
<212> DNA
<213> Homo sapiens

<400> 404
accaactaca acccctccct caagagtcga gtcaccatat cagtagacac gtccaagaac 60
cagttctccc tgaagctgag ctctgtgacc gctgcggaca cggccgtgta ttactgtgcg 120
aga 123

<210> 405
<211> 123

<212> DNA
<213> Homo sapiens

<400> 405
accagataca gcccgtcctt ccaaggccag gtcaccatct cagccgacaa gtccatcagc 60
accgcctacc tgcagtggag cagcctgaag gcctcggaca ccgccatgta ttactgtgcg 120
aga 123

<210> 406
<211> 123
<212> DNA
<213> Homo sapiens

<400> 406
aatgattatg cagtatctgt gaaaagtcga ataaccatca acccagacac atccaagaac 60
cagttctccc tgcagctgaa ctctgtgact cccgaggaca cggctgtgta ttactgtgca 120
aga 123

<210> 407
<211> 123
<212> DNA
<213> Homo sapiens

<400> 407
ccaacatatg cccagggctt cacaggacgg tttgtcttct ccatggacac ctctgccagc 60
acagcatacc tgcagatcag cagcctaaag gctgaggaca tggccatgta ttactgtgcg 120
aga 123

<210> 408
<211> 33
<212> DNA
<213> Homo sapiens

<400> 408
tggggccagg gcaccctggt caccgtctcc tca 33

<210> 409
<211> 33
<212> DNA
<213> Homo sapiens

<400> 409
tggggccgtg gcaccctggt cactgtctcc tca 33

<210> 410
<211> 33
<212> DNA
<213> Homo sapiens

<400> 410
tggggccaag ggacaatggt caccgtctct tca 33

<210> 411
<211> 33
<212> DNA
<213> Homo sapiens

<400> 411
tggggccaag gaaccctggt caccgtctcc tca 33

<210> 412
<211> 33
<212> DNA
<213> Homo sapiens

<400> 412
tggggccaag gaaccctggt caccgtctcc tca 33

<210> 413
<211> 33
<212> DNA
<213> Homo sapiens

<400> 413
tgggggcaag ggaccacggt caccgtctcc tca                                    33

<210> 414
<211> 43
<212> DNA
<213> Artificial Sequence

<220>
<223> Primers

<400> 414
gatgttgtga tgactcagtc tccactctcc ctgcccgtca ccc                         43

<210> 415
<211> 43
<212> DNA
<213> Artificial Sequence

<220>
<223> Primers

<400> 415
gatgttgtga tgactcagtc tccactctcc ctgcccgtca ccc                         43

<210> 416
<211> 43
<212> DNA
<213> Artificial Sequence

<220>
<223> Primers

<400> 416
gatattgtga tgacccagac tccactctct ctgtccgtca ccc                         43

<210> 417
<211> 43
<212> DNA
<213> Artificial Sequence

<220>
<223> Primers

<400> 417
gatattgtga tgactcagtc tccactctcc ctgcccgtca ccc                         43

<210> 418
<211> 43
<212> DNA
<213> Artificial Sequence

<220>
<223> Primers

<400> 418
gatattgtga tgacccagac tccactctct ctgtccgtca ccc                         43

<210> 419
<211> 43
<212> DNA
<213> Artificial Sequence

<220>
<223> Primers

<400> 419
gatattgtga tgacccagac tccactctcc tcacctgtca ccc                43

<210> 420
<211> 43
<212> DNA
<213> Artificial Sequence

<220>
<223> Primers

<400> 420
gatattgtga tgactcagtc tccactctcc ctgcccgtca ccc                43

<210> 421
<211> 43
<212> DNA
<213> Artificial Sequence

<220>
<223> Primers

<400> 421
gagattgtga tgacccagac tccactctcc ttgtctatca ccc                43

<210> 422
<211> 43
<212> DNA
<213> Artificial Sequence

<220>
<223> Primers

<400> 422
gatattgtga tgacccagac tccactctcc tcgcctgtca ccc                43

<210> 423
<211> 43
<212> DNA
<213> Artificial Sequence

<220>
<223> Primers

<400> 423
gaaattgtgc tgactcagtc tccagacttt cagtctgtga ctc                43

<210> 424
<211> 43
<212> DNA
<213> Artificial Sequence

<220>
<223> Primers

<400> 424
gatgttgtga tgacacagtc tccagctttc ctctctgtga ctc                43

<210> 425
<211> 43
<212> DNA
<213> Artificial Sequence

<220>
<223> Primers

<400> 425
gacatccaga tgacccagtc tccatcctcc ctgtctgcat ctg                43

<210> 426
<211> 43
<212> DNA
<213> Artificial Sequence

<220>
<223> Primers

<400> 426
gaaattgtgc tgactcagtc tccagacttt cagtctgtga ctc                43

<210> 427
<211> 43
<212> DNA
<213> Artificial Sequence

<220>
<223> Primers

<400> 427
gacatccaga tgacccagtc tccatcctcc ctgtctgcat ctg                43

<210> 428
<211> 43
<212> DNA
<213> Artificial Sequence

<220>
<223> Primers

<400> 428
gaaacgacac tcacgcagtc tccagcattc atgtcagcga ctc                43

<210> 429
<211> 43
<212> DNA
<213> Artificial Sequence

<220>
<223> Primers

<400> 429
gacatccaga tgacccagtc tccatcctca ctgtctgcat ctg                43

<210> 430
<211> 43
<212> DNA
<213> Artificial Sequence

<220>
<223> Primers

<400> 430
gccatccaga tgacccagtc tccatcctcc ctgtctgcat ctg                43

<210> 431
<211> 43
<212> DNA
<213> Artificial Sequence

<220>
<223> Primers

<400> 431

gacatccaga tgacccagtc tccttccacc ctgtctgcat ctg 43

<210> 432
<211> 43
<212> DNA
<213> Artificial Sequence

<220>
<223> Primers

<400> 432
aacatccaga tgacccagtc tccatctgcc atgtctgcat ctg 43

<210> 433
<211> 43
<212> DNA
<213> Artificial Sequence

<220>
<223> Primers

<400> 433
gacatccaga tgacccagtc tccatcctca ctgtctgcat ctg 43

<210> 434
<211> 43
<212> DNA
<213> Artificial Sequence

<220>
<223> Primers

<400> 434
gaaatagtga tgatgcagtc tccagccacc ctgtctgtgt ctc 43

<210> 435
<211> 43
<212> DNA
<213> Artificial Sequence

<220>
<223> Primers

<400> 435
gccatccagt tgacccagtc tccatcctcc ctgtctgcat ctg 43

<210> 436
<211> 43
<212> DNA
<213> Artificial Sequence

<220>
<223> Primers

<400> 436
gacatccaga tgacccagtc tccatcttct gtgtctgcat ctg 43

<210> 437
<211> 43
<212> DNA
<213> Artificial Sequence

<220>
<223> Primers

<400> 437
gaaatagtga tgacgcagtc tccagccacc ctgtctgtgt ctc 43

<210> 438
<211> 43
<212> DNA
<213> Artificial Sequence

<220>
<223> Primers

<400> 438
gaaattgtgt tgacacagtc tccagccacc ctgtctttgt ctc                43

<210> 439
<211> 43
<212> DNA
<213> Artificial Sequence

<220>
<223> Primers

<400> 439
gacatccaga tgatccagtc tccatctttc ctgtctgcat ctg                43

<210> 440
<211> 43
<212> DNA
<213> Artificial Sequence

<220>
<223> Primers

<400> 440
gccatccgga tgacccagtc tccattctcc ctgtctgcat ctg                43

<210> 441
<211> 43
<212> DNA
<213> Artificial Sequence

<220>
<223> Primers

<400> 441
gtcatctgga tgacccagtc tccatcctta ctctctgcat cta                43

<210> 442
<211> 43
<212> DNA
<213> Artificial Sequence

<220>
<223> Primers

<400> 442
gccatccagt tgacccagtc tccatcctcc ctgtctgcat ctg                43

<210> 443
<211> 43
<212> DNA
<213> Artificial Sequence

<220>
<223> Primers

<400> 443
gacatccaga tgacccagtc tccatcttcc gtgtctgcat ctg                43

<210> 444
<211> 43

<212> DNA
<213> Artificial Sequence

<220>
<223> Primers

<400> 444
gaaattgtgt tgacacagtc tccagccacc ctgtctttgt ctc                43

<210> 445
<211> 43
<212> DNA
<213> Artificial Sequence

<220>
<223> Primers

<400> 445
gacatccagt tgacccagtc tccatccttc ctgtctgcat ctg                43

<210> 446
<211> 43
<212> DNA
<213> Artificial Sequence

<220>
<223> Primers

<400> 446
gccatccgga tgacccagtc tccatcctca ttctctgcat cta                43

<210> 447
<211> 43
<212> DNA
<213> Artificial Sequence

<220>
<223> Primers

<400> 447
gacatccaga tgacccagtc tccatcctcc ctgtctgcat ctg                43

<210> 448
<211> 43
<212> DNA
<213> Artificial Sequence

<220>
<223> Primers

<400> 448
gacatccagt tgacccagtc tccatcctcc ctgtctgcat ctg                43

<210> 449
<211> 43
<212> DNA
<213> Artificial Sequence

<220>
<223> Primers

<400> 449
gacatccaga tgacccagtc tccatcctcc ctgtctgcat ctg                43

<210> 450
<211> 43
<212> DNA
<213> Artificial Sequence

<220>
<223> Primers

<400> 450
gacatccaga tgacccagtc tccatcctcc ctgtctgcat ctg 43

<210> 451
<211> 43
<212> DNA
<213> Artificial Sequence

<220>
<223> Primers

<400> 451
gacatccagt tgacccagtc tccatcctcc ctgtctgcat ctg 43

<210> 452
<211> 43
<212> DNA
<213> Artificial Sequence

<220>
<223> Primers

<400> 452
gacatccaga tgacccagtc tccatcctcc ctgtctgcat ctg 43

<210> 453
<211> 43
<212> DNA
<213> Artificial Sequence

<220>
<223> Primers

<400> 453
gaaattgtaa tgacacagtc tccacccacc ctgtctttgt ctc 43

<210> 454
<211> 43
<212> DNA
<213> Artificial Sequence

<220>
<223> Primers

<400> 454
gaaattgtaa tgacacagtc tccagccacc ctgtctttgt ctc 43

<210> 455
<211> 43
<212> DNA
<213> Artificial Sequence

<220>
<223> Primers

<400> 455
gaaattgtgt tgacgcagtc tccagccacc ctgtctttgt ctc 43

<210> 456
<211> 43
<212> DNA
<213> Artificial Sequence

<220>

<223> Primers

<400> 456
gaaattgtgt tgacgcagtc tccaggcacc ctgtctttgt ctc 43

<210> 457
<211> 43
<212> DNA
<213> Artificial Sequence

<220>
<223> Primers

<400> 457
gacatcgtga tgacccagtc tccagactcc ctggctgtgt ctc 43

<210> 458
<211> 43
<212> DNA
<213> Artificial Sequence

<220>
<223> Primers

<400> 458
gatattgtga tgacccagac tccactctcc ctgcccgtca ccc 43

<210> 459
<211> 43
<212> DNA
<213> Artificial Sequence

<220>
<223> Primers

<400> 459
gatattgtga tgacccagac tccactctcc ctgcccgtca ccc 43

<210> 460
<211> 47
<212> DNA
<213> Artificial Sequence

<220>
<223> Primers

<400> 460
gcaggagatg gaggccggct gtccaagggt gacgggcagg gagagtg 47

<210> 461
<211> 47
<212> DNA
<213> Artificial Sequence

<220>
<223> Primers

<400> 461
gcaggagatg gaggccggct gtccaagggt gacgggcagg gagagtg 47

<210> 462
<211> 47
<212> DNA
<213> Artificial Sequence

<220>
<223> Primers

<400> 462
gcaggagatg gaggccggct gtccaggggt gacggacaga gagagtg 47

<210> 463
<211> 47
<212> DNA
<213> Artificial Sequence

<220>
<223> Primers

<400> 463
gcaggagatg gaggccggct ctccaggggt gacgggcagg gagagtg 47

<210> 464
<211> 47
<212> DNA
<213> Artificial Sequence

<220>
<223> Primers

<400> 464
gcaggagatg gaggccggct gtccaggggt gacggacaga gagagtg 47

<210> 465
<211> 47
<212> DNA
<213> Artificial Sequence

<220>
<223> Primers

<400> 465
gcaggagatg gaggccggct gtccaagggt gacaggtgag gagagtg 47

<210> 466
<211> 47
<212> DNA
<213> Artificial Sequence

<220>
<223> Primers

<400> 466
gcaggagatg gaggccggct ctccaggggt gacgggcagg gagagtg 47

<210> 467
<211> 47
<212> DNA
<213> Artificial Sequence

<220>
<223> Primers

<400> 467
gcaggagatg gaggcctgct ctccaggggt gatagacaag gagagtg 47

<210> 468
<211> 47
<212> DNA
<213> Artificial Sequence

<220>
<223> Primers

<400> 468
gaaggagatg gaggccggct gtccaagggt gacaggcgag gagagtg 47

<210> 469
<211> 47
<212> DNA
<213> Artificial Sequence

<220>
<223> Primers

<400> 469
gcaggtgatg gtgactttct cctttggagt cacagactga aagtctg 47

<210> 470
<211> 47
<212> DNA
<213> Artificial Sequence

<220>
<223> Primers

<400> 470
gcaggtgatg gtgactttct cccctggagt cacagagagg aaagctg 47

<210> 471
<211> 47
<212> DNA
<213> Artificial Sequence

<220>
<223> Primers

<400> 471
gcaagtgatg gtgactctgt ctcctacaga tgcagacagg gaggatg 47

<210> 472
<211> 47
<212> DNA
<213> Artificial Sequence

<220>
<223> Primers

<400> 472
gcaggtgatg gtgactttct cctttggagt cacagactga aagtctg 47

<210> 473
<211> 47
<212> DNA
<213> Artificial Sequence

<220>
<223> Primers

<400> 473
gcaagtgatg gtgactctgt ctcctacaga tgcagacagg gaggatg 47

<210> 474
<211> 47
<212> DNA
<213> Artificial Sequence

<220>
<223> Primers

<400> 474
gcaggagatg ttgactttgt ctcctggagt cgctgacatg aatgctg 47

<210> 475

<211> 47
<212> DNA
<213> Artificial Sequence

<220>
<223> Primers

<400> 475
acaagtgatg gtgactctgt ctcctacaga tgcagacagt gaggatg 47

<210> 476
<211> 47
<212> DNA
<213> Artificial Sequence

<220>
<223> Primers

<400> 476
gcaagtgatg gtgactctgt ctcctacaga tgcagacagg gaggatg 47

<210> 477
<211> 47
<212> DNA
<213> Artificial Sequence

<220>
<223> Primers

<400> 477
gcaagtgatg gtgactctgt ctcctacaga tgcagacagg gtggaag 47

<210> 478
<211> 47
<212> DNA
<213> Artificial Sequence

<220>
<223> Primers

<400> 478
acaagtgatg gtgactctgt ctcctacaga tgcagacatg gcagatg 47

<210> 479
<211> 47
<212> DNA
<213> Artificial Sequence

<220>
<223> Primers

<400> 479
acaagtgatg gtgactctgt ctcctacaga tgcagacagt gaggatg 47

<210> 480
<211> 47
<212> DNA
<213> Artificial Sequence

<220>
<223> Primers

<400> 480
gcaggagagg gtggctcttt cccctggaga cacagacagg gtggctg 47

<210> 481
<211> 47
<212> DNA

<213> Artificial Sequence

<220>
<223> Primers

<400> 481
gcaagtgatg gtgactctgt ctcctacaga tgcagacagg gaggatg                47

<210> 482
<211> 47
<212> DNA
<213> Artificial Sequence

<220>
<223> Primers

<400> 482
acaagtgatg gtgactctgt ctcctacaga tgcagacaca gaagatg                47

<210> 483
<211> 47
<212> DNA
<213> Artificial Sequence

<220>
<223> Primers

<400> 483
gcaggagagg gtggctcttt cccctggaga cacagacagg gtggctg                47

<210> 484
<211> 47
<212> DNA
<213> Artificial Sequence

<220>
<223> Primers

<400> 484
gcaggagagg gtggctcttt cccctggaga caaagacagg gtggctg                47

<210> 485
<211> 47
<212> DNA
<213> Artificial Sequence

<220>
<223> Primers

<400> 485
gcaaatgata ctgactctgt ctcctacaga tgcagacagg aaagatg                47

<210> 486
<211> 47
<212> DNA
<213> Artificial Sequence

<220>
<223> Primers

<400> 486
gcaagtgatg gtgactctgt ctcctacaga tgcagacagg gagaatg                47

<210> 487
<211> 47
<212> DNA
<213> Artificial Sequence

<220>
<223> Primers

<400> 487
acaactgatg gtgactctgt ctcctgtaga tgcagagagt aaggatg 47

<210> 488
<211> 47
<212> DNA
<213> Artificial Sequence

<220>
<223> Primers

<400> 488
gcaagtgatg gtgactctgt ctcctacaga tgcagacagg gaggatg 47

<210> 489
<211> 47
<212> DNA
<213> Artificial Sequence

<220>
<223> Primers

<400> 489
acaagtgatg gtgactctgt ctcctacaga tgcagacacg gaagatg 47

<210> 490
<211> 47
<212> DNA
<213> Artificial Sequence

<220>
<223> Primers

<400> 490
gcaggagagg gtggctcttt cccctggaga caaagacagg gtggctg 47

<210> 491
<211> 47
<212> DNA
<213> Artificial Sequence

<220>
<223> Primers

<400> 491
gcaagtgatg gtgactctgt ctcctacaga tgcagacagg aaggatg 47

<210> 492
<211> 47
<212> DNA
<213> Artificial Sequence

<220>
<223> Primers

<400> 492
acaagtgatg gtgactctgt ctcctgtaga tgcagagaat gaggatg 47

<210> 493
<211> 47
<212> DNA
<213> Artificial Sequence

<220>
<223> Primers

<400> 493
gcaagtgatg gtgactctgt ctcctacaga tgcagacagg gaggatg 47

<210> 494
<211> 47
<212> DNA
<213> Artificial Sequence

<220>
<223> Primers

<400> 494
gcaagtgatg gtgactctgt ctcctacaga tgcagacagg gaggatg 47

<210> 495
<211> 47
<212> DNA
<213> Artificial Sequence

<220>
<223> Primers

<400> 495
gcaagtgatg gtgactctgt ctcctacaga tgcagacagg gaggatg 47

<210> 496
<211> 47
<212> DNA
<213> Artificial Sequence

<220>
<223> Primers

<400> 496
gcaagtgatg gtgactctgt ctcctacaga tgcagacagg gaggatg 47

<210> 497
<211> 47
<212> DNA
<213> Artificial Sequence

<220>
<223> Primers

<400> 497
gcaagtgatg gtgactctgt ctcctacaga tgcagacagg gaggatg 47

<210> 498
<211> 47
<212> DNA
<213> Artificial Sequence

<220>
<223> Primers

<400> 498
gcaagtgatg gtgactctgt ctcctacaga tgcagacagg gaggatg 47

<210> 499
<211> 47
<212> DNA
<213> Artificial Sequence

<220>
<223> Primers

<400> 499

gcaggagagg gtgactcttt cccctggaga caaagacagg gtgggtg 47

<210> 500
<211> 47
<212> DNA
<213> Artificial Sequence

<220>
<223> Primers

<400> 500
gcaggagagg gtggctcttt cccctggaga caaagacagg gtggctg 47

<210> 501
<211> 47
<212> DNA
<213> Artificial Sequence

<220>
<223> Primers

<400> 501
gcaggagagg gtggctcttt cccctggaga caaagacagg gtggctg 47

<210> 502
<211> 47
<212> DNA
<213> Artificial Sequence

<220>
<223> Primers

<400> 502
gcaggagagg gtggctcttt cccctggaga caaagacagg gtgcctg 47

<210> 503
<211> 47
<212> DNA
<213> Artificial Sequence

<220>
<223> Primers

<400> 503
gcagttgatg gtggccctct cgcccagaga cacagccagg gagtctg 47

<210> 504
<211> 47
<212> DNA
<213> Artificial Sequence

<220>
<223> Primers

<400> 504
gcaggagatg gaggccggct ctccaggggt gacgggcagg gagagtg 47

<210> 505
<211> 47
<212> DNA
<213> Artificial Sequence

<220>
<223> Primers

<400> 505
gcaggagatg gaggccggct ctccaggggt gacgggcagg gagagtg 47

<210> 506
<211> 31
<212> DNA
<213> Artificial Sequence

<220>
<223> Primers

<400> 506
tggtttcagc agaggccagg ccaatctcca a                                      31

<210> 507
<211> 31
<212> DNA
<213> Artificial Sequence

<220>
<223> Primers

<400> 507
tggtttcagc agaggccagg ccaatctcca a                                      31

<210> 508
<211> 31
<212> DNA
<213> Artificial Sequence

<220>
<223> Primers

<400> 508
tggtacctgc agaagccagg ccagtctcca c                                      31

<210> 509
<211> 31
<212> DNA
<213> Artificial Sequence

<220>
<223> Primers

<400> 509
tggtacctgc agaagccagg gcagtctcca c                                      31

<210> 510
<211> 31
<212> DNA
<213> Artificial Sequence

<220>
<223> Primers

<400> 510
tggtacctgc agaagccagg ccagcctcca c                                      31

<210> 511
<211> 31
<212> DNA
<213> Artificial Sequence

<220>
<223> Primers

<400> 511
tggcttcagc agaggccagg ccagcctcca a                                      31

<210> 512
<211> 31

<212> DNA
<213> Artificial Sequence

<220>
<223> Primers

<400> 512
tggtacctgc agaagccagg gcagtctcca c                                    31

<210> 513
<211> 31
<212> DNA
<213> Artificial Sequence

<220>
<223> Primers

<400> 513
tggtttctgc agaaagccag gccagtctcc a                                    31

<210> 514
<211> 31
<212> DNA
<213> Artificial Sequence

<220>
<223> Primers

<400> 514
tggcttcagc agaggccagg ccagcctcca a                                    31

<210> 515
<211> 31
<212> DNA
<213> Artificial Sequence

<220>
<223> Primers

<400> 515
tggtaccagc agaaaccaga tcagtctcca a                                    31

<210> 516
<211> 31
<212> DNA
<213> Artificial Sequence

<220>
<223> Primers

<400> 516
tggtaccagc agaaaccaga tcaagcccca a                                    31

<210> 517
<211> 31
<212> DNA
<213> Artificial Sequence

<220>
<223> Primers

<400> 517
tggtatcagc agaaaccagg gaaagttcct a                                    31

<210> 518
<211> 31
<212> DNA
<213> Artificial Sequence

<220>
<223> Primers

<400> 518
tggtaccagc agaaaccaga tcagtctcca a                                    31

<210> 519
<211> 31
<212> DNA
<213> Artificial Sequence

<220>
<223> Primers

<400> 519
tggtatcagc agaaaccagg gaaagcccct a                                    31

<210> 520
<211> 31
<212> DNA
<213> Artificial Sequence

<220>
<223> Primers

<400> 520
tggtaccaac agaaaccagg agaagctgct a                                    31

<210> 521
<211> 31
<212> DNA
<213> Artificial Sequence

<220>
<223> Primers

<400> 521
tggtttcagc agaaaccagg gaaagcccct a                                    31

<210> 522
<211> 31
<212> DNA
<213> Artificial Sequence

<220>
<223> Primers

<400> 522
tggtatcagc agaaaccagg gaaagcccct a                                    31

<210> 523
<211> 31
<212> DNA
<213> Artificial Sequence

<220>
<223> Primers

<400> 523
tggtatcagc agaaaccagg gaaagcccct a                                    31

<210> 524
<211> 31
<212> DNA
<213> Artificial Sequence

<220>

<223> Primers

<400> 524
tggtttcagc agaaaccagg gaaagtccct a                                    31

<210> 525
<211> 31
<212> DNA
<213> Artificial Sequence

<220>
<223> Primers

<400> 525
tggtatcagc agaaaccaga gaaagcccct a                                    31

<210> 526
<211> 31
<212> DNA
<213> Artificial Sequence

<220>
<223> Primers

<400> 526
tggtaccagc agaaacctgg ccaggctccc a                                    31

<210> 527
<211> 31
<212> DNA
<213> Artificial Sequence

<220>
<223> Primers

<400> 527
tggtatcagc agaaaccagg gaaagctcct a                                    31

<210> 528
<211> 31
<212> DNA
<213> Artificial Sequence

<220>
<223> Primers

<400> 528
tggtatcagc agaaaccagg gaaagcccct a                                    31

<210> 529
<211> 31
<212> DNA
<213> Artificial Sequence

<220>
<223> Primers

<400> 529
tggtaccagc agaaacctgg ccaggctccc a                                    31

<210> 530
<211> 31
<212> DNA
<213> Artificial Sequence

<220>
<223> Primers

<400> 530
tggtaccagc agaaacctgg ccaggctccc a                                    31

<210> 531
<211> 31
<212> DNA
<213> Artificial Sequence

<220>
<223> Primers

<400> 531
tggtatctgc agaaaccagg gaaatcccct a                                    31

<210> 532
<211> 31
<212> DNA
<213> Artificial Sequence

<220>
<223> Primers

<400> 532
tggtatcagc aaaaaccagc aaaagcccct a                                    31

<210> 533
<211> 31
<212> DNA
<213> Artificial Sequence

<220>
<223> Primers

<400> 533
tggtatcagc aaaaaccagg gaaagcccct g                                    31

<210> 534
<211> 31
<212> DNA
<213> Artificial Sequence

<220>
<223> Primers

<400> 534
tggtatcagc agaaaccagg gaaagctcct a                                    31

<210> 535
<211> 31
<212> DNA
<213> Artificial Sequence

<220>
<223> Primers

<400> 535
tggtatcagc agaaaccagg gaaagcccct a                                    31

<210> 536
<211> 31
<212> DNA
<213> Artificial Sequence

<220>
<223> Primers

<400> 536
tggtaccaac agaaacctgg ccaggctccc a                                    31

<210> 537
<211> 31
<212> DNA
<213> Artificial Sequence

<220>
<223> Primers

<400> 537
tggtatcagc aaaaaccagg gaaagcccct a                                    31

<210> 538
<211> 31
<212> DNA
<213> Artificial Sequence

<220>
<223> Primers

<400> 538
tggtatcagc aaaaaccagg gaaagcccct a                                    31

<210> 539
<211> 31
<212> DNA
<213> Artificial Sequence

<220>
<223> Primers

<400> 539
tggtatcagc agaaaccagg gaaagcccct a                                    31

<210> 540
<211> 31
<212> DNA
<213> Artificial Sequence

<220>
<223> Primers

<400> 540
tggtatcggc agaaaccagg gaaagttcct a                                    31

<210> 541
<211> 31
<212> DNA
<213> Artificial Sequence

<220>
<223> Primers

<400> 541
tggtatcagc agaaaccagg gaaagcccct a                                    31

<210> 542
<211> 31
<212> DNA
<213> Artificial Sequence

<220>
<223> Primers

<400> 542
tggtatcagc agaaaccagg gaaagcccct a                                    31

<210> 543

<211> 31
<212> DNA
<213> Artificial Sequence

<220>
<223> Primers

<400> 543
tggtatcggc agaaaccagg gaaagttcct a                                    31

<210> 544
<211> 31
<212> DNA
<213> Artificial Sequence

<220>
<223> Primers

<400> 544
tggtatcagc agaaaccagg gaaagcccct a                                    31

<210> 545
<211> 31
<212> DNA
<213> Artificial Sequence

<220>
<223> Primers

<400> 545
tggtatcagc agaaacctgg ccaggcgccc a                                    31

<210> 546
<211> 31
<212> DNA
<213> Artificial Sequence

<220>
<223> Primers

<400> 546
tggtaccagc agaaacctgg gcaggctccc a                                    31

<210> 547
<211> 31
<212> DNA
<213> Artificial Sequence

<220>
<223> Primers

<400> 547
tggtaccagc agaaacctgg cctggcgccc a                                    31

<210> 548
<211> 31
<212> DNA
<213> Artificial Sequence

<220>
<223> Primers

<400> 548
tggtaccagc agaaacctgg ccaggctccc a                                    31

<210> 549
<211> 31
<212> DNA

<213> Artificial Sequence

<220>
<223> Primers

<400> 549
tggtaccagc agaaaccagg acagcctcct a                                    31

<210> 550
<211> 31
<212> DNA
<213> Artificial Sequence

<220>
<223> Primers

<400> 550
tggtacctgc agaagccagg gcagtctcca c                                    31

<210> 551
<211> 31
<212> DNA
<213> Artificial Sequence

<220>
<223> Primers

<400> 551
tggtacctgc agaagccagg gcagtctcca c                                    31

<210> 552
<211> 35
<212> DNA
<213> Artificial Sequence

<220>
<223> Primers

<400> 552
ataaattagg cgccttggag attggcctgg cctct                                35

<210> 553
<211> 35
<212> DNA
<213> Artificial Sequence

<220>
<223> Primers

<400> 553
ataaattagg cgccttggag attggcctgg cctct                                35

<210> 554
<211> 35
<212> DNA
<213> Artificial Sequence

<220>
<223> Primers

<400> 554
atagatcagg agctgtggag actggcctgg cttct                                35

<210> 555
<211> 35
<212> DNA
<213> Artificial Sequence

<220>
<223> Primers

<400> 555
atagatcagg agctgtggag actgccctgg cttct                                35

<210> 556
<211> 35
<212> DNA
<213> Artificial Sequence

<220>
<223> Primers

<400> 556
atagatcagg agctgtggag gctggcctgg cttct                                35

<210> 557
<211> 35
<212> DNA
<213> Artificial Sequence

<220>
<223> Primers

<400> 557
ataaattagg agtcttggag gctggcctgg cctct                                35

<210> 558
<211> 35
<212> DNA
<213> Artificial Sequence

<220>
<223> Primers

<400> 558
atagatcagg agctgtggag actgccctgg cttct                                35

<210> 559
<211> 35
<212> DNA
<213> Artificial Sequence

<220>
<223> Primers

<400> 559
atagatcagg agtgtggaga ctggcctggc tttct                                35

<210> 560
<211> 35
<212> DNA
<213> Artificial Sequence

<220>
<223> Primers

<400> 560
ataaattagg agtcttggag gctggcctgg cctct                                35

<210> 561
<211> 35
<212> DNA
<213> Artificial Sequence

<220>
<223> Primers

<400> 561
cttgatgagg agctttggag actgatctgg tttct                                35

<210> 562
<211> 35
<212> DNA
<213> Artificial Sequence

<220>
<223> Primers

<400> 562
cttgatgagg agctttgggg cttgatctgg tttct                                35

<210> 563
<211> 35
<212> DNA
<213> Artificial Sequence

<220>
<223> Primers

<400> 563
atagatcagg agcttaggaa ctttccctgg tttct                                35

<210> 564
<211> 35
<212> DNA
<213> Artificial Sequence

<220>
<223> Primers

<400> 564
cttgatgagg agctttggag actgatctgg tttct                                35

<210> 565
<211> 35
<212> DNA
<213> Artificial Sequence

<220>
<223> Primers

<400> 565
atagatcagg cgcttagggg ctttccctgg tttct                                35

<210> 566
<211> 35
<212> DNA
<213> Artificial Sequence

<220>
<223> Primers

<400> 566
ttgaataatg aaaatagcag cttctcctgg tttct                                35

<210> 567
<211> 35
<212> DNA
<213> Artificial Sequence

<220>
<223> Primers

<400> 567

atagatcagg gacttagggg ctttccctgg tttct                                35

<210> 568
<211> 35
<212> DNA
<213> Artificial Sequence

<220>
<223> Primers

<400> 568
atagatcagg agcttagggg ctttccctgg tttct                                35

<210> 569
<211> 35
<212> DNA
<213> Artificial Sequence

<220>
<223> Primers

<400> 569
atagatcagg agcttagggg ctttccctgg tttct                                35

<210> 570
<211> 35
<212> DNA
<213> Artificial Sequence

<220>
<223> Primers

<400> 570
atagatcagg tgcttaggga ctttccctgg tttct                                35

<210> 571
<211> 35
<212> DNA
<213> Artificial Sequence

<220>
<223> Primers

<400> 571
atagatcagg gacttagggg ctttctctgg tttct                                35

<210> 572
<211> 35
<212> DNA
<213> Artificial Sequence

<220>
<223> Primers

<400> 572
atagatgagg agcctgggag cctggccagg tttct                                35

<210> 573
<211> 35
<212> DNA
<213> Artificial Sequence

<220>
<223> Primers

<400> 573
atagatcagg agcttaggag ctttccctgg tttct                                35

<210> 574
<211> 35
<212> DNA
<213> Artificial Sequence

<220>
<223> Primers

<400> 574
atagatcagg agcttagggg ctttccctgg tttct                                35

<210> 575
<211> 35
<212> DNA
<213> Artificial Sequence

<220>
<223> Primers

<400> 575
atagatgagg agcctgggag cctggccagg tttct                                35

<210> 576
<211> 35
<212> DNA
<213> Artificial Sequence

<220>
<223> Primers

<400> 576
atagatgagg agcctgggag cctggccagg tttct                                35

<210> 577
<211> 35
<212> DNA
<213> Artificial Sequence

<220>
<223> Primers

<400> 577
atagaggaag agcttagggg atttccctgg tttct                                35

<210> 578
<211> 35
<212> DNA
<213> Artificial Sequence

<220>
<223> Primers

<400> 578
atagatgaag agcttagggg cttttgctgg ttttt                                35

<210> 579
<211> 35
<212> DNA
<213> Artificial Sequence

<220>
<223> Primers

<400> 579
atagatcagg agctcagggg ctttccctgg ttttt                                35

<210> 580
<211> 35

<212> DNA
<213> Artificial Sequence

<220>
<223> Primers

<400> 580
atagatcagg agcttaggag ctttccctgg tttct                                35

<210> 581
<211> 35
<212> DNA
<213> Artificial Sequence

<220>
<223> Primers

<400> 581
atagatcagg agcttagggg ctttccctgg tttct                                35

<210> 582
<211> 35
<212> DNA
<213> Artificial Sequence

<220>
<223> Primers

<400> 582
atagatgagg agcctgggag cctggccagg tttct                                35

<210> 583
<211> 35
<212> DNA
<213> Artificial Sequence

<220>
<223> Primers

<400> 583
atagatcagg agcttagggg ctttccctgg ttttt                                35

<210> 584
<211> 35
<212> DNA
<213> Artificial Sequence

<220>
<223> Primers

<400> 584
atagatcagg agcttagggg ctttccctgg ttttt                                35

<210> 585
<211> 35
<212> DNA
<213> Artificial Sequence

<220>
<223> Primers

<400> 585
atagatcagg agcttagggg ctttccctgg tttct                                35

<210> 586
<211> 35
<212> DNA
<213> Artificial Sequence

<220>
<223> Primers

<400> 586
atagatcagg agcttaggaa ctttccctgg tttct                                35

<210> 587
<211> 35
<212> DNA
<213> Artificial Sequence

<220>
<223> Primers

<400> 587
gtagatcagg agcttagggg ctttccctgg tttct                                35

<210> 588
<211> 35
<212> DNA
<213> Artificial Sequence

<220>
<223> Primers

<400> 588
atagatcagg agcttagggg ctttccctgg tttct                                35

<210> 589
<211> 35
<212> DNA
<213> Artificial Sequence

<220>
<223> Primers

<400> 589
atagatcagg agcttaggaa ctttccctgg tttct                                35

<210> 590
<211> 35
<212> DNA
<213> Artificial Sequence

<220>
<223> Primers

<400> 590
gtagatcagg agcttagggg ctttccctgg tttct                                35

<210> 591
<211> 35
<212> DNA
<213> Artificial Sequence

<220>
<223> Primers

<400> 591
atagatgagg agcctgggcg cctggccagg tttct                                35

<210> 592
<211> 35
<212> DNA
<213> Artificial Sequence

<220>

<223> Primers

<400> 592
atagatgagg agcctgggag cctgcccagg tttct                                35

<210> 593
<211> 35
<212> DNA
<213> Artificial Sequence

<220>
<223> Primers

<400> 593
atagatgagg agcctgggcg ccaggccagg tttct                                35

<210> 594
<211> 35
<212> DNA
<213> Artificial Sequence

<220>
<223> Primers

<400> 594
atagatgagg agcctgggag cctggccagg tttct                                35

<210> 595
<211> 35
<212> DNA
<213> Artificial Sequence

<220>
<223> Primers

<400> 595
gtaaatgagc agcttaggag gctgtcctgg tttct                                35

<210> 596
<211> 35
<212> DNA
<213> Artificial Sequence

<220>
<223> Primers

<400> 596
atagatcagg agctgtggag actgccctgg cttct                                35

<210> 597
<211> 35
<212> DNA
<213> Artificial Sequence

<220>
<223> Primers

<400> 597
atagatcagg agctgtggag actgccctgg cttct                                35

<210> 598
<211> 59
<212> DNA
<213> Artificial Sequence

<220>
<223> Primers

<400> 598
ggggtcccag acagattcag cggcagtggg tcaggcactg atttcacact gaaaatcag 59

<210> 599
<211> 59
<212> DNA
<213> Artificial Sequence

<220>
<223> Primers

<400> 599
ggggtcccag acagattcag cggcagtggg tcaggcactg atttcacact gaaaatcag 59

<210> 600
<211> 59
<212> DNA
<213> Artificial Sequence

<220>
<223> Primers

<400> 600
ggagtgccag ataggttcag tggcagcggg tcagggacag atttcacact gaaaatcag 59

<210> 601
<211> 59
<212> DNA
<213> Artificial Sequence

<220>
<223> Primers

<400> 601
ggggtccctg acaggttcag tggcagtgga tcaggcacag attttacact gaaaatcag 59

<210> 602
<211> 59
<212> DNA
<213> Artificial Sequence

<220>
<223> Primers

<400> 602
ggagtgccag ataggttcag tggcagcggg tcagggacag atttcacact gaaaatcag 59

<210> 603
<211> 59
<212> DNA
<213> Artificial Sequence

<220>
<223> Primers

<400> 603
ggggtcccag acagattcag tggcagtggg gcagggacag atttcacact gaaaatcag 59

<210> 604
<211> 59
<212> DNA
<213> Artificial Sequence

<220>
<223> Primers

<400> 604
ggggtccctg acaggttcag tggcagtgga tcaggcacag attttacact gaaaatcag 59

<210> 605
<211> 59
<212> DNA
<213> Artificial Sequence

<220>
<223> Primers

<400> 605
ggagtgccag ataggttcag tggcagcggg tcagggacag atttcacact gaaaatcag 59

<210> 606
<211> 59
<212> DNA
<213> Artificial Sequence

<220>
<223> Primers

<400> 606
ggggtcccag acagattcag tggcagtggg gcagggacag atttcacact gaaaatcag 59

<210> 607
<211> 59
<212> DNA
<213> Artificial Sequence

<220>
<223> Primers

<400> 607
ggggtcccct cgaggttcag tggcagtgga tctgggacag atttcaccct caccatcaa 59

<210> 608
<211> 59
<212> DNA
<213> Artificial Sequence

<220>
<223> Primers

<400> 608
ggggtcccct cgaggttcag tggcagtgga tctgggacag atttcacctt taccatcag 59

<210> 609
<211> 59
<212> DNA
<213> Artificial Sequence

<220>
<223> Primers

<400> 609
ggggtcccat ctcggttcag tggcagtgga tctgggacag atttcactct caccatcag 59

<210> 610
<211> 59
<212> DNA
<213> Artificial Sequence

<220>
<223> Primers

<400> 610
ggggtcccct cgaggttcag tggcagtgga tctgggacag atttcaccct caccatcaa 59

<210> 611

<211> 59
<212> DNA
<213> Artificial Sequence

<220>
<223> Primers

<400> 611
ggggtcccat caaggttcag cggcagtgga tctgggacag aattcactct cacaatcag 59

<210> 612
<211> 59
<212> DNA
<213> Artificial Sequence

<220>
<223> Primers

<400> 612
ggaatcccac ctcgattcag tggcagcggg tatggaacag attttaccct cacaattaa 59

<210> 613
<211> 59
<212> DNA
<213> Artificial Sequence

<220>
<223> Primers

<400> 613
ggggtcccat caaggttcag cggcagtgga tctgggacag atttcactct caccatcag 59

<210> 614
<211> 59
<212> DNA
<213> Artificial Sequence

<220>
<223> Primers

<400> 614
ggggtcccat caaggttcag cggcagtgga tctggcacag atttcactct caccatcag 59

<210> 615
<211> 59
<212> DNA
<213> Artificial Sequence

<220>
<223> Primers

<400> 615
ggggtcccat caaggttcag cggcagtgga tctgggacag aattcactct caccatcag 59

<210> 616
<211> 59
<212> DNA
<213> Artificial Sequence

<220>
<223> Primers

<400> 616
ggggtcccat caaggttcag cggcagtgga tctgggacag aattcactct cacaatcag 59

<210> 617
<211> 59
<212> DNA

<213> Artificial Sequence

<220>
<223> Primers

<400> 617
ggggtcccat caaggttcag cggcagtgga tctgggacag atttcactct caccatcag 59

<210> 618
<211> 59
<212> DNA
<213> Artificial Sequence

<220>
<223> Primers

<400> 618
ggcatcccag ccaggttcag tggcagtggg tctgggacag agttcactct caccatcag 59

<210> 619
<211> 59
<212> DNA
<213> Artificial Sequence

<220>
<223> Primers

<400> 619
ggggtcccat caaggttcag cggcagtgga tctgggacag atttcactct caccatcag 59

<210> 620
<211> 59
<212> DNA
<213> Artificial Sequence

<220>
<223> Primers

<400> 620
ggggtcccat caaggttcag cggcagtgga tctgggacag atttcactct cactatcag 59

<210> 621
<211> 59
<212> DNA
<213> Artificial Sequence

<220>
<223> Primers

<400> 621
ggtatcccag ccaggttcag tggcagtggg tctgggacag agttcactct caccatcag 59

<210> 622
<211> 59
<212> DNA
<213> Artificial Sequence

<220>
<223> Primers

<400> 622
ggcatcccag ccaggttcag tggcagtggg cctgggacag acttcactct caccatcag 59

<210> 623
<211> 59
<212> DNA
<213> Artificial Sequence

<220>
<223> Primers

<400> 623
ggggtctcat cgaggttcag tggcagggga tctgggacgg atttcactct caccatcat 59

<210> 624
<211> 59
<212> DNA
<213> Artificial Sequence

<220>
<223> Primers

<400> 624
ggggtcccat caaggttcag cggcagtgga tctgggacgg attacactct caccatcag 59

<210> 625
<211> 59
<212> DNA
<213> Artificial Sequence

<220>
<223> Primers

<400> 625
ggggtcccat caaggttcag tggcagtgga tctgggacag atttcactct caccatcag 59

<210> 626
<211> 59
<212> DNA
<213> Artificial Sequence

<220>
<223> Primers

<400> 626
ggggtcccat caaggttcag cggcagtgga tctgggacag atttcactct caccatcag 59

<210> 627
<211> 59
<212> DNA
<213> Artificial Sequence

<220>
<223> Primers

<400> 627
ggggtcccat caaggttcag cggcagtgga tctgggacag atttcactct caccatcag 59

<210> 628
<211> 59
<212> DNA
<213> Artificial Sequence

<220>
<223> Primers

<400> 628
ggcatcccag ccaggttcag tggcagtggg tctgggacag acttcactct caccatcag 59

<210> 629
<211> 59
<212> DNA
<213> Artificial Sequence

<220>
<223> Primers

<400> 629
ggggtcccat caaggttcag cggcagtgga tctgggacag aattcactct cacaatcag 59

<210> 630
<211> 59
<212> DNA
<213> Artificial Sequence

<220>
<223> Primers

<400> 630
ggggtcccat caaggttcag cggcagtgga tctgggacag atttcactct caccatcag 59

<210> 631
<211> 59
<212> DNA
<213> Artificial Sequence

<220>
<223> Primers

<400> 631
ggggtcccat caaggttcag tggcagtgga tctgggacag atttcactct caccatcag 59

<210> 632
<211> 59
<212> DNA
<213> Artificial Sequence

<220>
<223> Primers

<400> 632
ggagtcccat ctcggttcag tggcagtgga tctgggacag atttcactct cactatcag 59

<210> 633
<211> 59
<212> DNA
<213> Artificial Sequence

<220>
<223> Primers

<400> 633
ggggtcccat caaggttcag tggaagtgga tctgggacag attttacttt caccatcag 59

<210> 634
<211> 59
<212> DNA
<213> Artificial Sequence

<220>
<223> Primers

<400> 634
ggggtcccat caaggttcag tggcagtgga tctgggacag atttcactct caccatcag 59

<210> 635
<211> 59
<212> DNA
<213> Artificial Sequence

<220>
<223> Primers

<400> 635

ggagtcccat ctcggttcag tggcagtgga tctgggacag atttcactct cactatcag 59

<210> 636
<211> 59
<212> DNA
<213> Artificial Sequence

<220>
<223> Primers

<400> 636
ggggtcccat caaggttcag tggaagtgga tctgggacag attttacttt caccatcag 59

<210> 637
<211> 59
<212> DNA
<213> Artificial Sequence

<220>
<223> Primers

<400> 637
agcatcccag ccaggttcag tggcagtggg tctgggacag acttcactct caccatcag 59

<210> 638
<211> 59
<212> DNA
<213> Artificial Sequence

<220>
<223> Primers

<400> 638
ggcatcccag ccaggttcag tggcagtggg tctgggacag acttcactct caccatcag 59

<210> 639
<211> 59
<212> DNA
<213> Artificial Sequence

<220>
<223> Primers

<400> 639
ggcatcccag acaggttcag tggcagtggg tctgggacag acttcactct caccatcag 59

<210> 640
<211> 59
<212> DNA
<213> Artificial Sequence

<220>
<223> Primers

<400> 640
ggcatcccag acaggttcag tggcagtggg tctgggacag acttcactct caccatcag 59

<210> 641
<211> 59
<212> DNA
<213> Artificial Sequence

<220>
<223> Primers

<400> 641
ggggtccctg accgattcag tggcagcggg tctgggacag atttcactct caccatcag 59

<210> 642
<211> 59
<212> DNA
<213> Artificial Sequence

<220>
<223> Primers

<400> 642
ggagtcccag acaggttcag tggcagtggg tcaggcactg atttcacact gaaaatcag 59

<210> 643
<211> 59
<212> DNA
<213> Artificial Sequence

<220>
<223> Primers

<400> 643
ggagtcccag acaggttcag tggcagtggg tcaggcactg atttcacact gaaaatcag 59

<210> 644
<211> 55
<212> DNA
<213> Artificial Sequence

<220>
<223> Primers

<400> 644
gcagtaataa accccaacat cctcagcctc caccctgctg attttcagtg tgaaa 55

<210> 645
<211> 55
<212> DNA
<213> Artificial Sequence

<220>
<223> Primers

<400> 645
gcagtaataa accccaacat cctcagcctc caccctgctg attttcagtg tgaaa 55

<210> 646
<211> 55
<212> DNA
<213> Artificial Sequence

<220>
<223> Primers

<400> 646
tcagtaataa accccaacat cctcagcctc cacccggctg attttcagtg tgaaa 55

<210> 647
<211> 55
<212> DNA
<213> Artificial Sequence

<220>
<223> Primers

<400> 647
gcagtaataa accccaacat cctcagcctc cactctgctg attttcagtg taaaa 55

<210> 648
<211> 55

<212> DNA
<213> Artificial Sequence

<220>
<223> Primers

<400> 648
gcagtaataa accccaacat cctcagcctc cacccggctg attttcagtg tgaaa 55

<210> 649
<211> 55
<212> DNA
<213> Artificial Sequence

<220>
<223> Primers

<400> 649
gcagtaataa accccgacat cctcagcttc caccctgctg attttcagtg tgaaa 55

<210> 650
<211> 55
<212> DNA
<213> Artificial Sequence

<220>
<223> Primers

<400> 650
gcagtaataa accccaacat cctcagcctc cactctgctg attttcagtg taaaa 55

<210> 651
<211> 55
<212> DNA
<213> Artificial Sequence

<220>
<223> Primers

<400> 651
gcagtaataa actccaaaat cctcagcctc cacccggctg attttcagtg tgaaa 55

<210> 652
<211> 55
<212> DNA
<213> Artificial Sequence

<220>
<223> Primers

<400> 652
gcagtaataa accccgacat cctcagcttc caccctgctg attttcagtg tgaaa 55

<210> 653
<211> 55
<212> DNA
<213> Artificial Sequence

<220>
<223> Primers

<400> 653
acagtaatac gttgcagcat cttcagcttc caggctattg atggtgaggg tgaaa 55

<210> 654
<211> 55
<212> DNA
<213> Artificial Sequence

<220>
<223> Primers

<400> 654
acagtaatat gttgcagcat cttcagcttc caggctactg atggtaaagg tgaaa 55

<210> 655
<211> 55
<212> DNA
<213> Artificial Sequence

<220>
<223> Primers

<400> 655
acagtaataa gttgcaacat cttcaggctg caggctgctg atggtgagag tgaaa 55

<210> 656
<211> 55
<212> DNA
<213> Artificial Sequence

<220>
<223> Primers

<400> 656
acagtaatac gttgcagcat cttcagcttc caggctattg atggtgaggg tgaaa 55

<210> 657
<211> 55
<212> DNA
<213> Artificial Sequence

<220>
<223> Primers

<400> 657
acagtaataa gttgcaaaat cttcaggctg caggctgctg attgtgagag tgaat 55

<210> 658
<211> 55
<212> DNA
<213> Artificial Sequence

<220>
<223> Primers

<400> 658
acagaagtaa tatgcagcat cctcagattc tatgttatta attgtgaggg taaaa 55

<210> 659
<211> 55
<212> DNA
<213> Artificial Sequence

<220>
<223> Primers

<400> 659
gcagtaataa gttgcaaaat cttcaggctg caggctgctg atggtgagag tgaaa 55

<210> 660
<211> 55
<212> DNA
<213> Artificial Sequence

<220>

<223> Primers

<400> 660
acagtaataa gttgcaaaat cttcaggctg caggctgctg atggtgagag tgaaa        55

<210> 661
<211> 55
<212> DNA
<213> Artificial Sequence

<220>
<223> Primers

<400> 661
gcagtaataa gttgcaaaat catcaggctg caggctgctg atggtgagag tgaat        55

<210> 662
<211> 55
<212> DNA
<213> Artificial Sequence

<220>
<223> Primers

<400> 662
acagtaataa gttgcaaaat cttcaggctg caggctgctg attgtgagag tgaat        55

<210> 663
<211> 55
<212> DNA
<213> Artificial Sequence

<220>
<223> Primers

<400> 663
gcagtaataa gttgcaaaat cttcaggctg caggctgctg atggtgagag tgaaa        55

<210> 664
<211> 55
<212> DNA
<213> Artificial Sequence

<220>
<223> Primers

<400> 664
acagtaataa actgcaaaat cttcagactg caggctgctg atggtgagag tgaac        55

<210> 665
<211> 55
<212> DNA
<213> Artificial Sequence

<220>
<223> Primers

<400> 665
acagtaataa gttgcaaaat cttcaggctg caggctgctg atggtgagag tgaaa        55

<210> 666
<211> 55
<212> DNA
<213> Artificial Sequence

<220>
<223> Primers

<400> 666
acaatagtaa gttgcaaaat cttcaggctg caggctgctg atagtgagag tgaaa        55

<210> 667
<211> 55
<212> DNA
<213> Artificial Sequence

<220>
<223> Primers

<400> 667
acagtaataa actgcaaaat cttcagactg caggctgctg atggtgagag tgaac        55

<210> 668
<211> 55
<212> DNA
<213> Artificial Sequence

<220>
<223> Primers

<400> 668
acagtaataa actgcaaaat cttcaggctc taggctgctg atggtgagag tgaag        55

<210> 669
<211> 55
<212> DNA
<213> Artificial Sequence

<220>
<223> Primers

<400> 669
acagtaataa gctgcaaaat cttcaggctt caggctgatg atggtgagag tgaaa        55

<210> 670
<211> 55
<212> DNA
<213> Artificial Sequence

<220>
<223> Primers

<400> 670
acagtaataa gttgcaaaat cttcaggctg caggctgctg atggtgagag tgtaa        55

<210> 671
<211> 55
<212> DNA
<213> Artificial Sequence

<220>
<223> Primers

<400> 671
acagtaataa gttgcaaaat cttcagactg caggcaactg atggtgagag tgaaa        55

<210> 672
<211> 55
<212> DNA
<213> Artificial Sequence

<220>
<223> Primers

<400> 672
acagtaataa gttgcaaaat cttcaggctg caggctgctg atggtgagag tgaaa        55

<210> 673
<211> 55
<212> DNA
<213> Artificial Sequence

<220>
<223> Primers

<400> 673
acaatagtaa gttgcaaaat cttcaggctg caggctgctg atggtgagag tgaaa 55

<210> 674
<211> 55
<212> DNA
<213> Artificial Sequence

<220>
<223> Primers

<400> 674
acagtaataa actgcaaaat cttcaggctc taggctgctg atggtgagag tgaag 55

<210> 675
<211> 55
<212> DNA
<213> Artificial Sequence

<220>
<223> Primers

<400> 675
acagtaataa gttgcaaaat cttcaggctg caggctgctg attgtgagag tgaat 55

<210> 676
<211> 55
<212> DNA
<213> Artificial Sequence

<220>
<223> Primers

<400> 676
acagtaataa gttgcaaaat cttcagactg caggcagctg atggtgagag tgaaa 55

<210> 677
<211> 55
<212> DNA
<213> Artificial Sequence

<220>
<223> Primers

<400> 677
acagtagtaa gttgcaaaat cttcaggttg cagactgctg atggtgagag tgaaa 55

<210> 678
<211> 55
<212> DNA
<213> Artificial Sequence

<220>
<223> Primers

<400> 678
accgtaataa gttgcaacat cttcaggctg caggctgctg atagtgagag tgaaa 55

<210> 679

<211> 55
<212> DNA
<213> Artificial Sequence

<220>
<223> Primers

<400> 679
acagtaatat gttgcaatat cttcaggctg caggctgctg atggtgaaag taaaa 55

<210> 680
<211> 55
<212> DNA
<213> Artificial Sequence

<220>
<223> Primers

<400> 680
acagtagtaa gttgcaaaat cttcaggttg cagactgctg atggtgagag tgaaa 55

<210> 681
<211> 55
<212> DNA
<213> Artificial Sequence

<220>
<223> Primers

<400> 681
accgtaataa gttgcaacat cttcaggctg caggctgctg atagtgagag tgaaa 55

<210> 682
<211> 55
<212> DNA
<213> Artificial Sequence

<220>
<223> Primers

<400> 682
acagtaatat gttgcaatat cttcaggctg caggctgctg atggtgaaag taaaa 55

<210> 683
<211> 55
<212> DNA
<213> Artificial Sequence

<220>
<223> Primers

<400> 683
acagtaataa actgcaaaat cttcaggctg caggctgctg atggtgagag tgaag 55

<210> 684
<211> 55
<212> DNA
<213> Artificial Sequence

<220>
<223> Primers

<400> 684
acagtaataa actgcaaaat cttcaggctg caggctgctg atggtgagag tgaag 55

<210> 685
<211> 55
<212> DNA

<213> Artificial Sequence

<220>
<223> Primers

<400> 685
acagtaatac actgcaaaat cttcaggctc cagtctgctg atggtgagag tgaag        55

<210> 686
<211> 55
<212> DNA
<213> Artificial Sequence

<220>
<223> Primers

<400> 686
acagtaatac actgcaaaat cttcaggctc cagtctgctg atggtgagag tgaag        55

<210> 687
<211> 55
<212> DNA
<213> Artificial Sequence

<220>
<223> Primers

<400> 687
acagtaataa actgccacat cttcagcctg caggctgctg atggtgagag tgaaa        55

<210> 688
<211> 55
<212> DNA
<213> Artificial Sequence

<220>
<223> Primers

<400> 688
gcagtaataa actccaacat cctcagcctc caccctgctg attttcagtg tgaaa        55

<210> 689
<211> 55
<212> DNA
<213> Artificial Sequence

<220>
<223> Primers

<400> 689
gcagtaataa actccaacat cctcagcctc caccctgctg attttcagtg tgaaa        55

<210> 690
<211> 30
<212> DNA
<213> Artificial Sequence

<220>
<223> Primers

<400> 690
ttcggccaag ggaccaaggt ggaaatcaaa                                    30

<210> 691
<211> 30
<212> DNA
<213> Artificial Sequence

<220>
<223> Primers

<400> 691
tttggccagg ggaccaagct ggagatcaaa                                        30

<210> 692
<211> 30
<212> DNA
<213> Artificial Sequence

<220>
<223> Primers

<400> 692
ttcggccctg ggaccaaagt ggatatcaaa                                        30

<210> 693
<211> 30
<212> DNA
<213> Artificial Sequence

<220>
<223> Primers

<400> 693
ttcggcggag ggaccaaggt ggagatcaaa                                        30

<210> 694
<211> 30
<212> DNA
<213> Artificial Sequence

<220>
<223> Primers

<400> 694
ttcggccaag ggacacgact ggagattaaa                                        30

<210> 695
<211> 30
<212> DNA
<213> Artificial Sequence

<220>
<223> Primers

<400> 695
tttgatttcc accttggtcc cttggccgaa                                        30

<210> 696
<211> 30
<212> DNA
<213> Artificial Sequence

<220>
<223> Primers

<400> 696
tttgatctcc agcttggtcc cctggccaaa                                        30

<210> 697
<211> 30
<212> DNA
<213> Artificial Sequence

<220>
<223> Primers

<400> 697
tttgatatcc actttggtcc cagggccgaa                                        30

<210> 698
<211> 30
<212> DNA
<213> Artificial Sequence

<220>
<223> Primers

<400> 698
tttgatctcc accttggtcc ctccgccgaa                                        30

<210> 699
<211> 30
<212> DNA
<213> Artificial Sequence

<220>
<223> Primers

<400> 699
tttaatctcc agtcgtgtcc cttggccgaa                                        30

<210> 700
<211> 59
<212> DNA
<213> Artificial Sequence

<220>
<223> Primers

<400> 700
caggttcagc tggtgcagtc tggagctgag gtgaagaagc ctggggcctc agtgaaggt      59

<210> 701
<211> 59
<212> DNA
<213> Artificial Sequence

<220>
<223> Primers

<400> 701
caggtgcagc tggtgcagtc tggggctgag gtgaagaagc ctggggcctc agtgaaggt      59

<210> 702
<211> 59
<212> DNA
<213> Artificial Sequence

<220>
<223> Primers

<400> 702
caggtccagc tggtacagtc tggggctgag gtgaagaagc ctggggcctc agtgaaggt      59

<210> 703
<211> 59
<212> DNA
<213> Artificial Sequence

<220>
<223> Primers

<400> 703

caggttcagc tggtgcagtc tggggctgag gtgaagaagc ctggggcctc agtgaaggt 59

<210> 704
<211> 59
<212> DNA
<213> Artificial Sequence

<220>
<223> Primers

<400> 704
cagatgcagc tggtgcagtc tggggctgag gtgaagaaga ctgggtcctc agtgaaggt 59

<210> 705
<211> 59
<212> DNA
<213> Artificial Sequence

<220>
<223> Primers

<400> 705
caggtgcagc tggtgcagtc tggggctgag gtgaagaagc ctggggcctc agtgaaggt 59

<210> 706
<211> 59
<212> DNA
<213> Artificial Sequence

<220>
<223> Primers

<400> 706
caaatgcagc tggtgcagtc tgggcctgag gtgaagaagc ctgggacctc agtgaaggt 59

<210> 707
<211> 59
<212> DNA
<213> Artificial Sequence

<220>
<223> Primers

<400> 707
caggtgcagc tggtgcagtc tggggctgag gtgaagaagc ctgggtcctc ggtgaaggt 59

<210> 708
<211> 59
<212> DNA
<213> Artificial Sequence

<220>
<223> Primers

<400> 708
caggtgcagc tggtgcagtc tggggctgag gtgaagaagc ctggggcctc agtgaaggt 59

<210> 709
<211> 59
<212> DNA
<213> Artificial Sequence

<220>
<223> Primers

<400> 709
caggtcacct tgaaggagtc tggtcctgtg ctggtgaaac ccacagagac cctcacgct 59

<210> 710
<211> 59
<212> DNA
<213> Artificial Sequence

<220>
<223> Primers

<400> 710
cagatcacct tgaaggagtc tggtcctacg ctggtgaaac ccacacagac cctcacgct 59

<210> 711
<211> 59
<212> DNA
<213> Artificial Sequence

<220>
<223> Primers

<400> 711
caggtcacct tgagggagtc tggtcctgcg ctggtgaaac ccacacagac cctcacact 59

<210> 712
<211> 59
<212> DNA
<213> Artificial Sequence

<220>
<223> Primers

<400> 712
caggtgcagc tggtggagtc tgggggaggc ttggtcaagc ctggagggtc cctgagact 59

<210> 713
<211> 59
<212> DNA
<213> Artificial Sequence

<220>
<223> Primers

<400> 713
gaggtgcagc tggtggagtc tgggggaggc ttggtacagc ctggggggtc cctgagact 59

<210> 714
<211> 59
<212> DNA
<213> Artificial Sequence

<220>
<223> Primers

<400> 714
gaggtgcagc tggtggagtc tgggggaggc ttggtaaagc ctggggggtc ccttagact 59

<210> 715
<211> 59
<212> DNA
<213> Artificial Sequence

<220>
<223> Primers

<400> 715
gaggtgcagc tggtggagtc tgggggaggc ttggtacagc ctggggggtc cctgagact 59

<210> 716
<211> 59

<212> DNA
<213> Artificial Sequence

<220>
<223> Primers

<400> 716
gaggtgcagc tggtggagtc tgggggaggt gtggtacggc ctggggggtc cctgagact 59

<210> 717
<211> 59
<212> DNA
<213> Artificial Sequence

<220>
<223> Primers

<400> 717
gaggtgcagc tggtggagtc tgggggaggc ctggtcaagc ctggggggtc cctgagact 59

<210> 718
<211> 59
<212> DNA
<213> Artificial Sequence

<220>
<223> Primers

<400> 718
gaggtgcagc tgttggagtc tgggggaggc ttggtacagc ctggggggtc cctgagact 59

<210> 719
<211> 59
<212> DNA
<213> Artificial Sequence

<220>
<223> Primers

<400> 719
caggtgcagc tggtggagtc tgggggaggc gtggtccagc ctgggaggtc cctgagact 59

<210> 720
<211> 59
<212> DNA
<213> Artificial Sequence

<220>
<223> Primers

<400> 720
caggtgcagc tggtggagtc tgggggaggc gtggtccagc ctgggaggtc cctgagact 59

<210> 721
<211> 59
<212> DNA
<213> Artificial Sequence

<220>
<223> Primers

<400> 721
gaggtgcagc tggtggagtc tgggggaggc ttggtacagc ctgggggatc cctgagact 59

<210> 722
<211> 59
<212> DNA
<213> Artificial Sequence

<220>
<223> Primers

<400> 722
gaggtgcagc tggtggagtc tgggggaggc ttggtacagc ctagggggtc cctgagact 59

<210> 723
<211> 59
<212> DNA
<213> Artificial Sequence

<220>
<223> Primers

<400> 723
gaagtgcagc tggtggagtc tgggggagtc gtggtacagc ctggggggtc cctgagact 59

<210> 724
<211> 59
<212> DNA
<213> Artificial Sequence

<220>
<223> Primers

<400> 724
gaggtgcagc tggtggagtc tgggggaggc ttggtacagc ctggggggtc cctgagact 59

<210> 725
<211> 59
<212> DNA
<213> Artificial Sequence

<220>
<223> Primers

<400> 725
gaggtgcagc tggtggagtc tgggggaggc ttggtacagc cagggcggtc cctgagact 59

<210> 726
<211> 59
<212> DNA
<213> Artificial Sequence

<220>
<223> Primers

<400> 726
gaggtgcagc tggtggagtc tggaggaggc ttgatccagc ctggggggtc cctgagact 59

<210> 727
<211> 59
<212> DNA
<213> Artificial Sequence

<220>
<223> Primers

<400> 727
gaggtgcagc tggtggagtc tggggaaggc ttggtccagc ctggggggtc cctgagact 59

<210> 728
<211> 59
<212> DNA
<213> Artificial Sequence

<220>

<223> Primers

<400> 728
gaggtgcagc tggtggagtc tggaggaggc ttgatccagc ctggggggtc cctgagact    59

<210> 729
<211> 59
<212> DNA
<213> Artificial Sequence

<220>
<223> Primers

<400> 729
gaggtgcagc tggtggagtc tgggggaggc ttggtccagc ctggggggtc cctgagact    59

<210> 730
<211> 59
<212> DNA
<213> Artificial Sequence

<220>
<223> Primers

<400> 730
gaggtgcagc tggtggagtc tgggggaggc ttggtccagc ctggagggtc cctgagact    59

<210> 731
<211> 59
<212> DNA
<213> Artificial Sequence

<220>
<223> Primers

<400> 731
gaggtgcagc tggtggagtc cgggggaggc ttggtccagc ctggggggtc cctgaaact    59

<210> 732
<211> 59
<212> DNA
<213> Artificial Sequence

<220>
<223> Primers

<400> 732
gaggtgcagc tggtggagtc cgggggaggc ttagttcagc ctggggggtc cctgagact    59

<210> 733
<211> 59
<212> DNA
<213> Artificial Sequence

<220>
<223> Primers

<400> 733
gaagtgcagc tggtggagtc tgggggaggc ttggtacagc ctggcaggtc cctgagact    59

<210> 734
<211> 59
<212> DNA
<213> Artificial Sequence

<220>
<223> Primers

<400> 734
caggtgcagc tgcaggagtc gggcccagga ctggtgaagc cttcggacac cctgtccct 59

<210> 735
<211> 59
<212> DNA
<213> Artificial Sequence

<220>
<223> Primers

<400> 735
caggtgcagc tgcaggagtc gggcccagga ctggtgaagc cttcacagac cctgtccct 59

<210> 736
<211> 59
<212> DNA
<213> Artificial Sequence

<220>
<223> Primers

<400> 736
caggtgcagc tacagcagtg gggcgcagga ctgttgaagc cttcggagac cctgtccct 59

<210> 737
<211> 59
<212> DNA
<213> Artificial Sequence

<220>
<223> Primers

<400> 737
cagctgcagc tgcaggagtc gggcccagga ctggtgaagc cttcggagac cctgtccct 59

<210> 738
<211> 59
<212> DNA
<213> Artificial Sequence

<220>
<223> Primers

<400> 738
caggtgcagc tgcaggagtc gggcccagga ctggtgaagc cttcggagac cctgtccct 59

<210> 739
<211> 59
<212> DNA
<213> Artificial Sequence

<220>
<223> Primers

<400> 739
caggtgcagc tgcaggagtc gggcccagga ctggtgaagc cttcggagac cctgtccct 59

<210> 740
<211> 59
<212> DNA
<213> Artificial Sequence

<220>
<223> Primers

<400> 740
caggtgcagc tgcaggagtc gggcccagga ctggtgaagc cttcggagac cctgtccct 59

<210> 741
<211> 59
<212> DNA
<213> Artificial Sequence

<220>
<223> Primers

<400> 741
gaggtgcagc tggtgcagtc tggagcagag gtgaaaaagc ccggggagtc tctgaagat 59

<210> 742
<211> 59
<212> DNA
<213> Artificial Sequence

<220>
<223> Primers

<400> 742
caggtacagc tgcagcagtc aggtccagga ctggtgaagc cctcgcagac cctctcact 59

<210> 743
<211> 59
<212> DNA
<213> Artificial Sequence

<220>
<223> Primers

<400> 743
caggtgcagc tggtgcagtc tggccatgag gtgaagcagc ctggggcctc agtgaaggt 59

<210> 744
<211> 52
<212> DNA
<213> Artificial Sequence

<220>
<223> Primers

<400> 744
ggtaaaggtg taaccagaag ccttgcagga gaccttcact gaggccccag gc 52

<210> 745
<211> 52
<212> DNA
<213> Artificial Sequence

<220>
<223> Primers

<400> 745
ggtgaaggtg tatccagaag ccttgcagga gaccttcact gaggccccag gc 52

<210> 746
<211> 52
<212> DNA
<213> Artificial Sequence

<220>
<223> Primers

<400> 746
agtgagggtg tatccggaaa ccttgcagga gaccttcact gaggccccag gc 52

<210> 747

<211> 52
<212> DNA
<213> Artificial Sequence

<220>
<223> Primers

<400> 747
agtgaaggtg tatccagaag ccttgcagga aaccttcact gaggccccag gc          52

<210> 748
<211> 52
<212> DNA
<213> Artificial Sequence

<220>
<223> Primers

<400> 748
ggtgaaggtg tatccggaag ccttgcagga aaccttcact gaggacccag tc          52

<210> 749
<211> 52
<212> DNA
<213> Artificial Sequence

<220>
<223> Primers

<400> 749
ggtgaaggtg tatccagatg ccttgcagga aaccttcact gaggccccag gc          52

<210> 750
<211> 52
<212> DNA
<213> Artificial Sequence

<220>
<223> Primers

<400> 750
agtaaaggtg aatccagaag ccttgcagga gaccttcact gaggtcccag gc          52

<210> 751
<211> 52
<212> DNA
<213> Artificial Sequence

<220>
<223> Primers

<400> 751
gctgaaggtg cctccagaag ccttgcagga gaccttcacc gaggacccag gc          52

<210> 752
<211> 52
<212> DNA
<213> Artificial Sequence

<220>
<223> Primers

<400> 752
ggtgaaggtg tatccagaag ccttgcagga gaccttcact gaggccccag gc          52

<210> 753
<211> 52
<212> DNA

<213> Artificial Sequence

<220>
<223> Primers

<400> 753
gctgagtgag aacccagaga cggtgcaggt cagcgtgagg gtctctgtgg gt          52

<210> 754
<211> 52
<212> DNA
<213> Artificial Sequence

<220>
<223> Primers

<400> 754
gctgagtgag aacccagaga aggtgcaggt cagcgtgagg gtctgtgtgg gt          52

<210> 755
<211> 52
<212> DNA
<213> Artificial Sequence

<220>
<223> Primers

<400> 755
gctgagtgag aacccagaga aggtgcaggt cagtgtgagg gtctgtgtgg gt          52

<210> 756
<211> 52
<212> DNA
<213> Artificial Sequence

<220>
<223> Primers

<400> 756
actgaaggtg aatccagagg ctgcacagga gagtctcagg gaccctccag gc          52

<210> 757
<211> 52
<212> DNA
<213> Artificial Sequence

<220>
<223> Primers

<400> 757
actgaaggtg aatccagagg ctgcacagga gagtctcagg gaccccccag gc          52

<210> 758
<211> 52
<212> DNA
<213> Artificial Sequence

<220>
<223> Primers

<400> 758
actgaaagtg aatccagagg ctgcacagga gagtctaagg gaccccccag gc          52

<210> 759
<211> 52
<212> DNA
<213> Artificial Sequence

<220>
<223> Primers

<400> 759
actgaaggtg aatccagagg ctgcacagga gagtctcagg gaccccccag gc          52

<210> 760
<211> 52
<212> DNA
<213> Artificial Sequence

<220>
<223> Primers

<400> 760
atcaaaggtg aatccagagg ctgcacagga gagtctcagg gaccccccag gc          52

<210> 761
<211> 52
<212> DNA
<213> Artificial Sequence

<220>
<223> Primers

<400> 761
actgaaggtg aatccagagg ctgcacagga gagtctcagg gaccccccag gc          52

<210> 762
<211> 52
<212> DNA
<213> Artificial Sequence

<220>
<223> Primers

<400> 762
gctaaaggtg aatccagagg ctgcacagga gagtctcagg gaccccccag gc          52

<210> 763
<211> 52
<212> DNA
<213> Artificial Sequence

<220>
<223> Primers

<400> 763
actgaaggtg aatccagagg ctgcacagga gagtctcagg gacctcccag gc          52

<210> 764
<211> 52
<212> DNA
<213> Artificial Sequence

<220>
<223> Primers

<400> 764
actgaaggtg aatccagacg ctgcacagga gagtctcagg gacctcccag gc          52

<210> 765
<211> 52
<212> DNA
<213> Artificial Sequence

<220>
<223> Primers

<400> 765
actgaaggtg aatccagagg ctgcacagga gagtctcagg gatcccccag gc          52

<210> 766
<211> 52
<212> DNA
<213> Artificial Sequence

<220>
<223> Primers

<400> 766
actgacggtg aatccagagg ctgcacagga gagtctcagg gaccccctag gc          52

<210> 767
<211> 52
<212> DNA
<213> Artificial Sequence

<220>
<223> Primers

<400> 767
atcaaaggtg aatccagagg ctgcacagga gagtctcagg gaccccccag gc          52

<210> 768
<211> 52
<212> DNA
<213> Artificial Sequence

<220>
<223> Primers

<400> 768
actgaaggtg aatccagagg ctgcacagga gagtctcagg gaccccccag gc          52

<210> 769
<211> 52
<212> DNA
<213> Artificial Sequence

<220>
<223> Primers

<400> 769
accaaaggtg aatccagaag ctgtacagga gagtctcagg gaccgccctg gc          52

<210> 770
<211> 52
<212> DNA
<213> Artificial Sequence

<220>
<223> Primers

<400> 770
actgacggtg aacccagagg ctgcacagga gagtctcagg gaccccccag gc          52

<210> 771
<211> 52
<212> DNA
<213> Artificial Sequence

<220>
<223> Primers

<400> 771

actgaaggtg aatccagagg ctgcacagga gagtctcagg gaccccccag gc          52

<210> 772
<211> 52
<212> DNA
<213> Artificial Sequence

<220>
<223> Primers

<400> 772
actgacggtg aacccagagg ctgcacagga gagtctcagg gaccccccag gc          52

<210> 773
<211> 52
<212> DNA
<213> Artificial Sequence

<220>
<223> Primers

<400> 773
actaaaggtg aatccagagg ctgcacagga gagtctcagg gaccccccag gc          52

<210> 774
<211> 52
<212> DNA
<213> Artificial Sequence

<220>
<223> Primers

<400> 774
actgaaggtg aatccagagg ctgcacagga gagtctcagg gaccctccag gc          52

<210> 775
<211> 52
<212> DNA
<213> Artificial Sequence

<220>
<223> Primers

<400> 775
actgaaggtg aacccagagg ctgcacagga gagtttcagg gaccccccag gc          52

<210> 776
<211> 52
<212> DNA
<213> Artificial Sequence

<220>
<223> Primers

<400> 776
actgaaggtg aatccagagg ctgcacagga gagtctcagg gaccccccag gc          52

<210> 777
<211> 52
<212> DNA
<213> Artificial Sequence

<220>
<223> Primers

<400> 777
atcaaaggtg aatccagagg ctgcacagga gagtctcagg gacctgccag gc          52

<210> 778
<211> 52
<212> DNA
<213> Artificial Sequence

<220>
<223> Primers

<400> 778
gctgatggag taaccagaga cagcgcaggt gagggacagg gtgtccgaag gc 52

<210> 779
<211> 52
<212> DNA
<213> Artificial Sequence

<220>
<223> Primers

<400> 779
gctgatggag ccaccagaga cagtacaggt gagggacagg gtctgtgaag gc 52

<210> 780
<211> 52
<212> DNA
<213> Artificial Sequence

<220>
<223> Primers

<400> 780
actgaaggac ccaccataga cagcgcaggt gagggacagg gtctccgaag gc 52

<210> 781
<211> 52
<212> DNA
<213> Artificial Sequence

<220>
<223> Primers

<400> 781
gctgatggag ccaccagaga cagtgcaggt gagggacagg gtctccgaag gc 52

<210> 782
<211> 52
<212> DNA
<213> Artificial Sequence

<220>
<223> Primers

<400> 782
actgatggag ccaccagaga cagtgcaggt gagggacagg gtctccgaag gc 52

<210> 783
<211> 52
<212> DNA
<213> Artificial Sequence

<220>
<223> Primers

<400> 783
actgatggag ccaccagaga cagtgcaggt gagggacagg gtctccgaag gc 52

<210> 784
<211> 52

<212> DNA
<213> Artificial Sequence

<220>
<223> Primers

<400> 784
gctgacggag ccaccagaga cagtgcaggt gagggacagg gtctccgaag gc          52

<210> 785
<211> 52
<212> DNA
<213> Artificial Sequence

<220>
<223> Primers

<400> 785
ggtaaagctg tatccagaac ccttacagga gatcttcaga gactccccgg gc          52

<210> 786
<211> 52
<212> DNA
<213> Artificial Sequence

<220>
<223> Primers

<400> 786
agagacactg tccccggaga tggcacaggt gagtgagagg gtctgcgagg gc          52

<210> 787
<211> 52
<212> DNA
<213> Artificial Sequence

<220>
<223> Primers

<400> 787
ggtgaaactg taaccagaag ccttgcagga gaccttcact gaggccccag gc          52

<210> 788
<211> 31
<212> DNA
<213> Artificial Sequence

<220>
<223> Primers

<400> 788
tgggtgcgac aggcccctgg acaagggctt g                                 31

<210> 789
<211> 31
<212> DNA
<213> Artificial Sequence

<220>
<223> Primers

<400> 789
tgggtgcgac aggcccctgg acaagggctt g                                 31

<210> 790
<211> 31
<212> DNA
<213> Artificial Sequence

<220>
<223> Primers

<400> 790
tgggtgcgac aggctcctgg aaaagggctt g                                    31

<210> 791
<211> 31
<212> DNA
<213> Artificial Sequence

<220>
<223> Primers

<400> 791
tgggtgcgcc aggcccccgg acaaaggctt g                                    31

<210> 792
<211> 31
<212> DNA
<213> Artificial Sequence

<220>
<223> Primers

<400> 792
tgggtgcgac aggcccccgg acaagcgctt g                                    31

<210> 793
<211> 31
<212> DNA
<213> Artificial Sequence

<220>
<223> Primers

<400> 793
tgggtgcgac aggcccctgg acaagggctt g                                    31

<210> 794
<211> 31
<212> DNA
<213> Artificial Sequence

<220>
<223> Primers

<400> 794
tgggtgcgac aggctcgtgg acaacgcctt g                                    31

<210> 795
<211> 31
<212> DNA
<213> Artificial Sequence

<220>
<223> Primers

<400> 795
tgggtgcgac aggcccctgg acaagggctt g                                    31

<210> 796
<211> 31
<212> DNA
<213> Artificial Sequence

<220>

<223> Primers

<400> 796
tgggtgcgac aggccactgg acaagggctt g                                      31

<210> 797
<211> 31
<212> DNA
<213> Artificial Sequence

<220>
<223> Primers

<400> 797
tggatccgtc agcccccagg gaaggccctg g                                      31

<210> 798
<211> 31
<212> DNA
<213> Artificial Sequence

<220>
<223> Primers

<400> 798
tggatccgtc agcccccagg aaaggccctg g                                      31

<210> 799
<211> 31
<212> DNA
<213> Artificial Sequence

<220>
<223> Primers

<400> 799
tggatccgtc agcccccagg gaaggccctg g                                      31

<210> 800
<211> 31
<212> DNA
<213> Artificial Sequence

<220>
<223> Primers

<400> 800
tggatccgcc aggctccagg gaaggggctg g                                      31

<210> 801
<211> 31
<212> DNA
<213> Artificial Sequence

<220>
<223> Primers

<400> 801
tgggtccgcc aagctacagg aaaaggtctg g                                      31

<210> 802
<211> 31
<212> DNA
<213> Artificial Sequence

<220>
<223> Primers

<400> 802
tgggtccgcc aggctccagg gaaggggctg g                                    31

<210> 803
<211> 31
<212> DNA
<213> Artificial Sequence

<220>
<223> Primers

<400> 803
tgggcccgca aggctccagg aaaggggctg g                                    31

<210> 804
<211> 31
<212> DNA
<213> Artificial Sequence

<220>
<223> Primers

<400> 804
tgggtccgcc aagctccagg gaaggggctg g                                    31

<210> 805
<211> 31
<212> DNA
<213> Artificial Sequence

<220>
<223> Primers

<400> 805
tgggtccgcc aggctccagg gaaggggctg g                                    31

<210> 806
<211> 31
<212> DNA
<213> Artificial Sequence

<220>
<223> Primers

<400> 806
tgggtccgcc aggctccagg gaaggggctg g                                    31

<210> 807
<211> 31
<212> DNA
<213> Artificial Sequence

<220>
<223> Primers

<400> 807
tgggtccgcc aggctccagg caaggggctg g                                    31

<210> 808
<211> 31
<212> DNA
<213> Artificial Sequence

<220>
<223> Primers

<400> 808
tgggtccgcc aggctccagg caaggggctg g                                    31

<210> 809
<211> 31
<212> DNA
<213> Artificial Sequence

<220>
<223> Primers

<400> 809
tgggtccatc aggctccagg aaaggggctg g                                    31

<210> 810
<211> 31
<212> DNA
<213> Artificial Sequence

<220>
<223> Primers

<400> 810
tggatccgcc aggctccagg gaaggggctg g                                    31

<210> 811
<211> 31
<212> DNA
<213> Artificial Sequence

<220>
<223> Primers

<400> 811
tgggtccgtc aagctccggg gaagggtctg g                                    31

<210> 812
<211> 31
<212> DNA
<213> Artificial Sequence

<220>
<223> Primers

<400> 812
tgggtccgcc aggctccagg gaaggggctg g                                    31

<210> 813
<211> 31
<212> DNA
<213> Artificial Sequence

<220>
<223> Primers

<400> 813
tggttccgcc aggctccagg gaaggggctg g                                    31

<210> 814
<211> 31
<212> DNA
<213> Artificial Sequence

<220>
<223> Primers

<400> 814
tgggtccgcc aggctccagg gaaggggctg g                                    31

<210> 815

<211> 31
<212> DNA
<213> Artificial Sequence

<220>
<223> Primers

<400> 815
tgggtccgcc aggctccagg gaagggactg g                                    31

<210> 816
<211> 31
<212> DNA
<213> Artificial Sequence

<220>
<223> Primers

<400> 816
tgggtccgcc aggctccagg gaaggggctg g                                    31

<210> 817
<211> 31
<212> DNA
<213> Artificial Sequence

<220>
<223> Primers

<400> 817
tgggtccgcc aggctccagg gaaggggctg g                                    31

<210> 818
<211> 31
<212> DNA
<213> Artificial Sequence

<220>
<223> Primers

<400> 818
tgggtccgcc aggctccagg gaaggggctg g                                    31

<210> 819
<211> 31
<212> DNA
<213> Artificial Sequence

<220>
<223> Primers

<400> 819
tgggtccgcc aggcttccgg gaaagggctg g                                    31

<210> 820
<211> 31
<212> DNA
<213> Artificial Sequence

<220>
<223> Primers

<400> 820
tgggtccgcc aagctccagg gaaggggctg g                                    31

<210> 821
<211> 31
<212> DNA

<213> Artificial Sequence

<220>
<223> Primers

<400> 821
tgggtccggc aagctccagg gaagggcctg g                                    31

<210> 822
<211> 31
<212> DNA
<213> Artificial Sequence

<220>
<223> Primers

<400> 822
tggatccggc agcccccagg gaagggactg g                                    31

<210> 823
<211> 31
<212> DNA
<213> Artificial Sequence

<220>
<223> Primers

<400> 823
tggatccgcc agcacccagg gaagggcctg g                                    31

<210> 824
<211> 31
<212> DNA
<213> Artificial Sequence

<220>
<223> Primers

<400> 824
tggatccgcc agcccccagg gaaggggctg g                                    31

<210> 825
<211> 31
<212> DNA
<213> Artificial Sequence

<220>
<223> Primers

<400> 825
tggatccgcc agcccccagg gaaggggctg g                                    31

<210> 826
<211> 31
<212> DNA
<213> Artificial Sequence

<220>
<223> Primers

<400> 826
tggatccggc agcccgccgg gaagggactg g                                    31

<210> 827
<211> 31
<212> DNA
<213> Artificial Sequence

<220>
<223> Primers

<400> 827
tggatccggc agcccccagg gaagggactg g                                    31

<210> 828
<211> 31
<212> DNA
<213> Artificial Sequence

<220>
<223> Primers

<400> 828
tggatccggc agcccccagg gaagggactg g                                    31

<210> 829
<211> 31
<212> DNA
<213> Artificial Sequence

<220>
<223> Primers

<400> 829
tgggtgcgcc agatgcccgg gaaaggcctg g                                    31

<210> 830
<211> 31
<212> DNA
<213> Artificial Sequence

<220>
<223> Primers

<400> 830
tggatcaggc agtccccatc gagaggcctt g                                    31

<210> 831
<211> 31
<212> DNA
<213> Artificial Sequence

<220>
<223> Primers

<400> 831
tgggtgccac aggcccctgg acaagggctt g                                    31

<210> 832
<211> 32
<212> DNA
<213> Artificial Sequence

<220>
<223> Primers

<400> 832
tcccatccac tcaagccctt gtccaggggc ct                                   32

<210> 833
<211> 32
<212> DNA
<213> Artificial Sequence

<220>
<223> Primers

<400> 833
tcccatccac tcaagccctt gtccaggggc ct                                    32

<210> 834
<211> 32
<212> DNA
<213> Artificial Sequence

<220>
<223> Primers

<400> 834
tcccatccac tcaagccctt ttccaggagc ct                                    32

<210> 835
<211> 32
<212> DNA
<213> Artificial Sequence

<220>
<223> Primers

<400> 835
tcccatccac tcaagccttt gtccgggggc ct                                    32

<210> 836
<211> 32
<212> DNA
<213> Artificial Sequence

<220>
<223> Primers

<400> 836
tcccatccac tcaagcgctt gtccgggggc ct                                    32

<210> 837
<211> 32
<212> DNA
<213> Artificial Sequence

<220>
<223> Primers

<400> 837
tcccatccac tcaagccctt gtccaggggc ct                                    32

<210> 838
<211> 32
<212> DNA
<213> Artificial Sequence

<220>
<223> Primers

<400> 838
tcctatccac tcaaggcgtt gtccacgagc ct                                    32

<210> 839
<211> 32
<212> DNA
<213> Artificial Sequence

<220>
<223> Primers

<400> 839

tcccatccac tcaagccctt gtccaggggc ct                                    32

<210> 840
<211> 32
<212> DNA
<213> Artificial Sequence

<220>
<223> Primers

<400> 840
tcccatccac tcaagccctt gtccagtggc ct                                    32

<210> 841
<211> 32
<212> DNA
<213> Artificial Sequence

<220>
<223> Primers

<400> 841
tgcaagccac tccagggcct tccctggggg ct                                    32

<210> 842
<211> 32
<212> DNA
<213> Artificial Sequence

<220>
<223> Primers

<400> 842
tgcaagccac tccagggcct ttcctggggg ct                                    32

<210> 843
<211> 32
<212> DNA
<213> Artificial Sequence

<220>
<223> Primers

<400> 843
tgcaagccac tccagggcct tccctggggg ct                                    32

<210> 844
<211> 32
<212> DNA
<213> Artificial Sequence

<220>
<223> Primers

<400> 844
tgaaacccac tccagcccct tccctggagc ct                                    32

<210> 845
<211> 32
<212> DNA
<213> Artificial Sequence

<220>
<223> Primers

<400> 845
tgagacccac tccagacctt ttcctgtagc tt                                    32

<210> 846
<211> 32
<212> DNA
<213> Artificial Sequence

<220>
<223> Primers

<400> 846
gccaacccac tccagcccct tccctggagc ct                                    32

<210> 847
<211> 32
<212> DNA
<213> Artificial Sequence

<220>
<223> Primers

<400> 847
cgatacccac tccagcccct ttcctggagc ct                                    32

<210> 848
<211> 32
<212> DNA
<213> Artificial Sequence

<220>
<223> Primers

<400> 848
agagacccac tccagcccct tccctggagc tt                                    32

<210> 849
<211> 32
<212> DNA
<213> Artificial Sequence

<220>
<223> Primers

<400> 849
tgagacccac tccagcccct tccctggagc ct                                    32

<210> 850
<211> 32
<212> DNA
<213> Artificial Sequence

<220>
<223> Primers

<400> 850
tgagacccac tccagcccct tccctggagc ct                                    32

<210> 851
<211> 32
<212> DNA
<213> Artificial Sequence

<220>
<223> Primers

<400> 851
tgccacccac tccagcccct tgcctggagc ct                                    32

<210> 852
<211> 32

<212> DNA
<213> Artificial Sequence

<220>
<223> Primers

<400> 852
tgccacccac tccagcccct tgcctggagc ct                                    32

<210> 853
<211> 32
<212> DNA
<213> Artificial Sequence

<220>
<223> Primers

<400> 853
cgatacccac tccagcccct ttcctggagc ct                                    32

<210> 854
<211> 32
<212> DNA
<213> Artificial Sequence

<220>
<223> Primers

<400> 854
tgagacccac tccagcccct tccctggagc ct                                    32

<210> 855
<211> 32
<212> DNA
<213> Artificial Sequence

<220>
<223> Primers

<400> 855
agagacccac tccagaccct tccccggagc tt                                    32

<210> 856
<211> 32
<212> DNA
<213> Artificial Sequence

<220>
<223> Primers

<400> 856
tgaaacccac tccagcccct tccctggagc ct                                    32

<210> 857
<211> 32
<212> DNA
<213> Artificial Sequence

<220>
<223> Primers

<400> 857
acctacccac tccagcccct tccctggagc ct                                    32

<210> 858
<211> 32
<212> DNA
<213> Artificial Sequence

<220>
<223> Primers

<400> 858
tgagacccac tccagcccct tccctggagc ct                                32

<210> 859
<211> 32
<212> DNA
<213> Artificial Sequence

<220>
<223> Primers

<400> 859
tgaaacatat tccagtccct tccctggagc ct                                32

<210> 860
<211> 32
<212> DNA
<213> Artificial Sequence

<220>
<223> Primers

<400> 860
tgagacccac tccagcccct tccctggagc ct                                32

<210> 861
<211> 32
<212> DNA
<213> Artificial Sequence

<220>
<223> Primers

<400> 861
ggccacccac tccagcccct tccctggagc ct                                32

<210> 862
<211> 32
<212> DNA
<213> Artificial Sequence

<220>
<223> Primers

<400> 862
gccaacccac tccagcccct tccctggagc ct                                32

<210> 863
<211> 32
<212> DNA
<213> Artificial Sequence

<220>
<223> Primers

<400> 863
gccaacccac tccagccctt tcccggaagc ct                                32

<210> 864
<211> 32
<212> DNA
<213> Artificial Sequence

<220>

<223> Primers

<400> 864
tgagacccac accagcccct tccctggagc tt                                    32

<210> 865
<211> 32
<212> DNA
<213> Artificial Sequence

<220>
<223> Primers

<400> 865
tgagacccac tccaggccct tccctggagc tt                                    32

<210> 866
<211> 32
<212> DNA
<213> Artificial Sequence

<220>
<223> Primers

<400> 866
cccaatccac tccagtccct tccctggggg ct                                    32

<210> 867
<211> 32
<212> DNA
<213> Artificial Sequence

<220>
<223> Primers

<400> 867
cccaatccac tccaggccct tccctgggtg ct                                    32

<210> 868
<211> 32
<212> DNA
<213> Artificial Sequence

<220>
<223> Primers

<400> 868
cccaatccac tccagcccct tccctggggg ct                                    32

<210> 869
<211> 32
<212> DNA
<213> Artificial Sequence

<220>
<223> Primers

<400> 869
cccaatccac tccagcccct tccctggggg ct                                    32

<210> 870
<211> 32
<212> DNA
<213> Artificial Sequence

<220>
<223> Primers

<400> 870
cccaatccac tccagtccct tcccggcggg ct                                    32

<210> 871
<211> 32
<212> DNA
<213> Artificial Sequence

<220>
<223> Primers

<400> 871
cccaatccac tccagtccct tccctggggg ct                                    32

<210> 872
<211> 32
<212> DNA
<213> Artificial Sequence

<220>
<223> Primers

<400> 872
cccaatccac tccagtccct tccctggggg ct                                    32

<210> 873
<211> 32
<212> DNA
<213> Artificial Sequence

<220>
<223> Primers

<400> 873
ccccatccac tccaggcctt tcccgggcat ct                                    32

<210> 874
<211> 32
<212> DNA
<213> Artificial Sequence

<220>
<223> Primers

<400> 874
tcccagccac tcaaggcctc tcgatgggga ct                                    32

<210> 875
<211> 32
<212> DNA
<213> Artificial Sequence

<220>
<223> Primers

<400> 875
tcccatccac tcaagccctt gtccaggggc ct                                    32

<210> 876
<211> 67
<212> DNA
<213> Artificial Sequence

<220>
<223> Primers

<400> 876
agagtcacca tgaccacaga cacatccacg agcacagcct acatggagct gaggagcctg 60

agatctg                                                            67

<210> 877
<211> 67
<212> DNA
<213> Artificial Sequence

<220>
<223> Primers

<400> 877
agggtcacca tgaccaggga cacgtccatc agcacagcct acatggagct gagcaggctg 60
agatctg                                                            67

<210> 878
<211> 67
<212> DNA
<213> Artificial Sequence

<220>
<223> Primers

<400> 878
agagtcacca tgaccgagga cacatctaca gacacagcct acatggagct gagcagcctg 60
agatctg                                                            67

<210> 879
<211> 67
<212> DNA
<213> Artificial Sequence

<220>
<223> Primers

<400> 879
agagtcacca ttaccaggga cacatccgcg agcacagcct acatggagct gagcagcctg 60
agatctg                                                            67

<210> 880
<211> 67
<212> DNA
<213> Artificial Sequence

<220>
<223> Primers

<400> 880
agagtcacca ttaccaggga caggtctatg agcacagcct acatggagct gagcagcctg 60
agatctg                                                            67

<210> 881
<211> 67
<212> DNA
<213> Artificial Sequence

<220>
<223> Primers

<400> 881
agagtcacca tgaccaggga cacgtccacg agcacagtct acatggagct gagcagcctg 60
agatctg                                                            67

<210> 882
<211> 67
<212> DNA
<213> Artificial Sequence

<220>

<223> Primers

<400> 882
agagtcacca ttaccaggga catgtccaca agcacagcct acatggagct gagcagcctg 60
agatccg 67

<210> 883
<211> 67
<212> DNA
<213> Artificial Sequence

<220>
<223> Primers

<400> 883
agagtcacga ttaccgcgga caaatccacg agcacagcct acatggagct gagcagcctg 60
agatctg 67

<210> 884
<211> 67
<212> DNA
<213> Artificial Sequence

<220>
<223> Primers

<400> 884
agagtcacca tgaccaggaa cacctccata agcacagcct acatggagct gagcagcctg 60
agatctg 67

<210> 885
<211> 67
<212> DNA
<213> Artificial Sequence

<220>
<223> Primers

<400> 885
aggctcacca tctccaagga cacctccaaa agccaggtgg tccttaccat gaccaacatg 60
gaccctg 67

<210> 886
<211> 67
<212> DNA
<213> Artificial Sequence

<220>
<223> Primers

<400> 886
aggctcacca tcaccaagga cacctccaaa aaccaggtgg tccttacaat gaccaacatg 60
gaccctg 67

<210> 887
<211> 67
<212> DNA
<213> Artificial Sequence

<220>
<223> Primers

<400> 887
aggctcacca tctccaagga cacctccaaa aaccaggtgg tccttacaat gaccaacatg 60
gaccctg 67

<210> 888
<211> 67

<212> DNA
<213> Artificial Sequence

<220>
<223> Primers

<400> 888
cgattcacca tctccaggga caacgccaag aactcactgt atctgcaaat gaacagcctg 60
agagccg 67

<210> 889
<211> 67
<212> DNA
<213> Artificial Sequence

<220>
<223> Primers

<400> 889
cgattcacca tctccagaga aaatgccaag aactccttgt atcttcaaat gaacagcctg 60
agagccg 67

<210> 890
<211> 67
<212> DNA
<213> Artificial Sequence

<220>
<223> Primers

<400> 890
agattcacca tctcaagaga tgattcaaaa aacacgctgt atctgcaaat gaacagcctg 60
aaaaccg 67

<210> 891
<211> 67
<212> DNA
<213> Artificial Sequence

<220>
<223> Primers

<400> 891
cgattcatca tctccagaga caattccagg aactccctgt atctgcaaaa gaacagacgg 60
agagccg 67

<210> 892
<211> 67
<212> DNA
<213> Artificial Sequence

<220>
<223> Primers

<400> 892
cgattcacca tctccagaga caacgccaag aactccctgt atctgcaaat gaacagtctg 60
agagccg 67

<210> 893
<211> 67
<212> DNA
<213> Artificial Sequence

<220>
<223> Primers

<400> 893
cgattcacca tctccagaga caacgccaag aactcactgt atctgcaaat gaacagcctg 60

agagccg                                                             67

<210> 894
<211> 67
<212> DNA
<213> Artificial Sequence

<220>
<223> Primers

<400> 894
cggttcacca tctccagaga caattccaag aacacgctgt atctgcaaat gaacagcctg 60
agagccg                                                             67

<210> 895
<211> 67
<212> DNA
<213> Artificial Sequence

<220>
<223> Primers

<400> 895
cgattcacca tctccagaga caattccaag aacacgctgt atctgcaaat gaacagcctg 60
agagctg                                                             67

<210> 896
<211> 67
<212> DNA
<213> Artificial Sequence

<220>
<223> Primers

<400> 896
cgattcacca tctccagaga caattccaag aacacgctgt atctgcaaat gaacagcctg 60
agagccg                                                             67

<210> 897
<211> 67
<212> DNA
<213> Artificial Sequence

<220>
<223> Primers

<400> 897
cgattcatca tctccagaga caattccagg aacaccctgt atctgcaaac gaatagcctg 60
agggccg                                                             67

<210> 898
<211> 67
<212> DNA
<213> Artificial Sequence

<220>
<223> Primers

<400> 898
agattcacca tctccagaga caattccaag aacacgctgt atcttcaaat gaacaacctg 60
agagctg                                                             67

<210> 899
<211> 67
<212> DNA
<213> Artificial Sequence

<220>

<223> Primers

<400> 899
cgattcacca tctccagaga caacagcaaa aactccctgt atctgcaaat gaacagtctg 60
agaactg 67

<210> 900
<211> 67
<212> DNA
<213> Artificial Sequence

<220>
<223> Primers

<400> 900
cgattcacca tctccagaga caatgccaag aactcactgt atctgcaaat gaacagcctg 60
agagacg 67

<210> 901
<211> 67
<212> DNA
<213> Artificial Sequence

<220>
<223> Primers

<400> 901
agattcacca tctcaagaga tgattccaaa agcatcgcct atctgcaaat gaacagcctg 60
aaaaccg 67

<210> 902
<211> 67
<212> DNA
<213> Artificial Sequence

<220>
<223> Primers

<400> 902
cgattcacca tctccagaga caattccaag aacacgctgt atcttcaaat gaacagcctg 60
agagccg 67

<210> 903
<211> 67
<212> DNA
<213> Artificial Sequence

<220>
<223> Primers

<400> 903
agattcacca tctccagaga caattccaag aacacgctgt atcttcaaat gggcagcctg 60
agagctg 67

<210> 904
<211> 67
<212> DNA
<213> Artificial Sequence

<220>
<223> Primers

<400> 904
cgattcacca tctccagaga caattccaag aacacgctgt atcttcaaat gaacagcctg 60
agagctg 67

<210> 905
<211> 67

<212> DNA
<213> Artificial Sequence

<220>
<223> Primers

<400> 905
cgattcacca tctccagaga caacgccaag aactcactgt atctgcaaat gaacagcctg 60
agagccg 67

<210> 906
<211> 67
<212> DNA
<213> Artificial Sequence

<220>
<223> Primers

<400> 906
agattcacca tctcaagaga tgattcaaag aactcactgt atctgcaaat gaacagcctg 60
aaaaccg 67

<210> 907
<211> 67
<212> DNA
<213> Artificial Sequence

<220>
<223> Primers

<400> 907
aggttcacca tctccagaga tgattcaaag aacacggcgt atctgcaaat gaacagcctg 60
aaaaccg 67

<210> 908
<211> 67
<212> DNA
<213> Artificial Sequence

<220>
<223> Primers

<400> 908
cgattcacca tctccagaga caacgccaag aacacgctgt atctgcaaat gaacagtctg 60
agagccg 67

<210> 909
<211> 67
<212> DNA
<213> Artificial Sequence

<220>
<223> Primers

<400> 909
cgattcacca tctccagaga caacgccaag aactccctgt atctgcaaat gaacagtctg 60
agagctg 67

<210> 910
<211> 67
<212> DNA
<213> Artificial Sequence

<220>
<223> Primers

<400> 910
cgagtcacca tgtcagtaga cacgtccaag aaccagttct ccctgaagct gagctctgtg 60

accgccg                                                             67

<210> 911
<211> 67
<212> DNA
<213> Artificial Sequence

<220>
<223> Primers

<400> 911
cgagttacca tatcagtaga cacgtctaag aaccagttct ccctgaagct gagctctgtg 60
actgccg                                                             67

<210> 912
<211> 67
<212> DNA
<213> Artificial Sequence

<220>
<223> Primers

<400> 912
cgagtcacca tatcagtaga cacgtccaag aaccagttct ccctgaagct gagctctgtg 60
accgccg                                                             67

<210> 913
<211> 67
<212> DNA
<213> Artificial Sequence

<220>
<223> Primers

<400> 913
cgagtcacca tatccgtaga cacgtccaag aaccagttct ccctgaagct gagctctgtg 60
accgccg                                                             67

<210> 914
<211> 67
<212> DNA
<213> Artificial Sequence

<220>
<223> Primers

<400> 914
cgagtcacca tgtcagtaga cacgtccaag aaccagttct ccctgaagct gagctctgtg 60
accgccg                                                             67

<210> 915
<211> 67
<212> DNA
<213> Artificial Sequence

<220>
<223> Primers

<400> 915
cgagtcacca tatcagtaga cacgtccaag aaccagttct ccctgaagct gagctctgtg 60
accgctg                                                             67

<210> 916
<211> 67
<212> DNA
<213> Artificial Sequence

<220>

<223> Primers

<400> 916
cgagtcacca tatcagtaga cacgtccaag aaccagttct ccctgaagct gagctctgtg 60
accgctg 67

<210> 917
<211> 67
<212> DNA
<213> Artificial Sequence

<220>
<223> Primers

<400> 917
caggtcacca tctcagccga caagtccatc agcaccgcct acctgcagtg gagcagcctg 60
aaggcct 67

<210> 918
<211> 67
<212> DNA
<213> Artificial Sequence

<220>
<223> Primers

<400> 918
cgaataacca tcaacccaga cacatccaag aaccagttct ccctgcagct gaactctgtg 60
actcccg 67

<210> 919
<211> 67
<212> DNA
<213> Artificial Sequence

<220>
<223> Primers

<400> 919
cggtttgtct tctccatgga cacctctgcc agcacagcat acctgcagat cagcagccta 60
aaggctg 67

<210> 920
<211> 50
<212> DNA
<213> Artificial Sequence

<220>
<223> Primers

<400> 920
tctcgcacag taatacacgg ccgtgtcgtc agatctcagg ctcctcagct 50

<210> 921
<211> 50
<212> DNA
<213> Artificial Sequence

<220>
<223> Primers

<400> 921
tctcgcacag taatacacgg ccgtgtcgtc agatctcagc ctgctcagct 50

<210> 922
<211> 50
<212> DNA
<213> Artificial Sequence

<220>
<223> Primers

<400> 922
tgttgcacag taatacacgg ccgtgtcctc agatctcagg ctgctcagct 50

<210> 923
<211> 50
<212> DNA
<213> Artificial Sequence

<220>
<223> Primers

<400> 923
tctcgcacag taatacacag ccatgtcctc agatctcagg ctgctcagct 50

<210> 924
<211> 50
<212> DNA
<213> Artificial Sequence

<220>
<223> Primers

<400> 924
tcttgcacag taatacatgg ctgtgtcctc agatctcagg ctgctcagct 50

<210> 925
<211> 50
<212> DNA
<213> Artificial Sequence

<220>
<223> Primers

<400> 925
tctcgcacag taatacacgg ccgtgtcctc agatctcagg ctgctcagct 50

<210> 926
<211> 50
<212> DNA
<213> Artificial Sequence

<220>
<223> Primers

<400> 926
tgccgcacag taatacacgg ccgtgtcctc ggatctcagg ctgctcagct 50

<210> 927
<211> 50
<212> DNA
<213> Artificial Sequence

<220>
<223> Primers

<400> 927
tctcgcacag taatacacgg ccgtgtcctc agatctcagg ctgctcagct 50

<210> 928
<211> 50
<212> DNA
<213> Artificial Sequence

<220>

<223> Primers

<400> 928
tctcgcacag taatacacgg ccgtgtcctc agatctcagg ctgctcagct          50

<210> 929
<211> 50
<212> DNA
<213> Artificial Sequence

<220>
<223> Primers

<400> 929
ccgtgcacag taatatgtgg ctgtgtccac agggtccatg ttggtcatgg          50

<210> 930
<211> 50
<212> DNA
<213> Artificial Sequence

<220>
<223> Primers

<400> 930
gtgtgcacag taatatgtgg ctgtgtccac agggtccatg ttggtcattg          50

<210> 931
<211> 50
<212> DNA
<213> Artificial Sequence

<220>
<223> Primers

<400> 931
ccgtgcacaa taatacgtgg ctgtgtccac agggtccatg ttggtcattg          50

<210> 932
<211> 50
<212> DNA
<213> Artificial Sequence

<220>
<223> Primers

<400> 932
tctcgcacag taatacacgg ccgtgtcctc ggctctcagg ctgttcattt          50

<210> 933
<211> 50
<212> DNA
<213> Artificial Sequence

<220>
<223> Primers

<400> 933
tcttgcacag taatacacag ccgtgtcccc ggctctcagg ctgttcattt          50

<210> 934
<211> 50
<212> DNA
<213> Artificial Sequence

<220>
<223> Primers

<400> 934
tgtggtacag taatacacgg ctgtgtcctc ggttttcagg ctgttcattt 50

<210> 935
<211> 50
<212> DNA
<213> Artificial Sequence

<220>
<223> Primers

<400> 935
tctcacacag taatacacag ccatgtcctc ggctctccgt ctgttctttt 50

<210> 936
<211> 50
<212> DNA
<213> Artificial Sequence

<220>
<223> Primers

<400> 936
tctcgcacag tgatacaagg ccgtgtcctc ggctctcaga ctgttcattt 50

<210> 937
<211> 50
<212> DNA
<213> Artificial Sequence

<220>
<223> Primers

<400> 937
tctcgcacag taatacacag ccgtgtcctc ggctctcagg ctgttcattt 50

<210> 938
<211> 50
<212> DNA
<213> Artificial Sequence

<220>
<223> Primers

<400> 938
tttcgcacag taatatacgg ccgtgtcctc ggctctcagg ctgttcattt 50

<210> 939
<211> 50
<212> DNA
<213> Artificial Sequence

<220>
<223> Primers

<400> 939
tctcgcacag taatacacag ccgtgtcctc agctctcagg ctgttcattt 50

<210> 940
<211> 49
<212> DNA
<213> Artificial Sequence

<220>
<223> Primers

<400> 940
ctcgcacagt aatacacagc cgtgtcctcg gctctcaggc tgttcattt 49

<210> 941
<211> 50
<212> DNA
<213> Artificial Sequence

<220>
<223> Primers

<400> 941
tctcacacag taatacacag ccgtgtcctc ggccctcagg ctattcgttt          50

<210> 942
<211> 50
<212> DNA
<213> Artificial Sequence

<220>
<223> Primers

<400> 942
tctggcacag taatacacgg ccgtgccctc agctctcagg ttgttcattt          50

<210> 943
<211> 50
<212> DNA
<213> Artificial Sequence

<220>
<223> Primers

<400> 943
ttttgcacag taatacaagg cggtgtcctc agttctcaga ctgttcattt          50

<210> 944
<211> 50
<212> DNA
<213> Artificial Sequence

<220>
<223> Primers

<400> 944
tctcgcacag taatacacag ccgtgtcctc gtctctcagg ctgttcattt          50

<210> 945
<211> 50
<212> DNA
<213> Artificial Sequence

<220>
<223> Primers

<400> 945
tctagtacag taatacacgg ctgtgtcctc ggttttcagg ctgttcattt          50

<210> 946
<211> 50
<212> DNA
<213> Artificial Sequence

<220>
<223> Primers

<400> 946
tctcgcacag taatacacgg ccgtgtcctc ggctctcagg ctgttcattt          50

<210> 947

<211> 50
<212> DNA
<213> Artificial Sequence

<220>
<223> Primers

<400> 947
tctcgcacag taatacacag ccatgtcctc agctctcagg ctgcccattt          50

<210> 948
<211> 50
<212> DNA
<213> Artificial Sequence

<220>
<223> Primers

<400> 948
tctcgcacag taatacacag ccgtgtcctc agctctcagg ctgttcattt          50

<210> 949
<211> 50
<212> DNA
<213> Artificial Sequence

<220>
<223> Primers

<400> 949
tctcgcacag taatacacag ccgtgtcctc ggctctcagg ctgttcattt          50

<210> 950
<211> 50
<212> DNA
<213> Artificial Sequence

<220>
<223> Primers

<400> 950
tctagcacag taatacacgg ccgtgtcctc ggttttcagg ctgttcattt          50

<210> 951
<211> 50
<212> DNA
<213> Artificial Sequence

<220>
<223> Primers

<400> 951
tctagtacag taatacacgg ccgtgtcctc ggttttcagg ctgttcattt          50

<210> 952
<211> 50
<212> DNA
<213> Artificial Sequence

<220>
<223> Primers

<400> 952
tcttgcacag taatacacag ccgtgtcctc ggctctcaga ctgttcattt          50

<210> 953
<211> 50
<212> DNA

<213> Artificial Sequence

<220>
<223> Primers

<400> 953
ttttgcacag taatacaagg ccgtgtcctc agctctcaga ctgttcattt 50

<210> 954
<211> 50
<212> DNA
<213> Artificial Sequence

<220>
<223> Primers

<400> 954
tctcgcacag taatacacgg ccgtgtccac ggcggtcaca gagctcagct 50

<210> 955
<211> 50
<212> DNA
<213> Artificial Sequence

<220>
<223> Primers

<400> 955
tctcgcacag taatacacgg ccgtgtccgc ggcagtcaca gagctcagct 50

<210> 956
<211> 50
<212> DNA
<213> Artificial Sequence

<220>
<223> Primers

<400> 956
tctcgcacag taatacacag ccgtgtccgc ggcggtcaca gagctcagct 50

<210> 957
<211> 50
<212> DNA
<213> Artificial Sequence

<220>
<223> Primers

<400> 957
tctcgcacag taatacacag ccgtgtctgc ggcggtcaca gagctcagct 50

<210> 958
<211> 50
<212> DNA
<213> Artificial Sequence

<220>
<223> Primers

<400> 958
tctcgcacag taatacacgg ccgtgtccgc ggcggtcaca gagctcagct 50

<210> 959
<211> 50
<212> DNA
<213> Artificial Sequence

<220>
<223> Primers

<400> 959
tctcgcacag taatacacgg ccgtgtccgc agcggtcaca gagctcagct          50

<210> 960
<211> 50
<212> DNA
<213> Artificial Sequence

<220>
<223> Primers

<400> 960
tctcgcacag taatacacgg ccgtgtccgc agcggtcaca gagctcagct          50

<210> 961
<211> 50
<212> DNA
<213> Artificial Sequence

<220>
<223> Primers

<400> 961
tctcgcacag taatacatgg cggtgtccga ggccttcagg ctgctccact          50

<210> 962
<211> 50
<212> DNA
<213> Artificial Sequence

<220>
<223> Primers

<400> 962
tcttgcacag taatacacag ccgtgtcctc gggagtcaca gagttcagct          50

<210> 963
<211> 50
<212> DNA
<213> Artificial Sequence

<220>
<223> Primers

<400> 963
tctcgcacag taatacatgg ccatgtcctc agcctttagg ctgctgatct          50

<210> 964
<211> 33
<212> DNA
<213> Artificial Sequence

<220>
<223> Primers

<400> 964
tggggccagg gcaccctggt caccgtctcc tca                            33

<210> 965
<211> 33
<212> DNA
<213> Artificial Sequence

<220>
<223> Primers

<400> 965
tggggccgtg gcaccctggt cactgtctcc tca                                    33

<210> 966
<211> 33
<212> DNA
<213> Artificial Sequence

<220>
<223> Primers

<400> 966
tggggccaag ggacaatggt caccgtctct tca                                    33

<210> 967
<211> 33
<212> DNA
<213> Artificial Sequence

<220>
<223> Primers

<400> 967
tggggccaag gaaccctggt caccgtctcc tca                                    33

<210> 968
<211> 33
<212> DNA
<213> Artificial Sequence

<220>
<223> Primers

<400> 968
tggggccaag gaaccctggt caccgtctcc tca                                    33

<210> 969
<211> 33
<212> DNA
<213> Artificial Sequence

<220>
<223> Primers

<400> 969
tgggggcaag ggaccacggt caccgtctcc tca                                    33

<210> 970
<211> 33
<212> DNA
<213> Artificial Sequence

<220>
<223> Primers

<400> 970
tgaggagacg gtgaccaggg tgccctggcc cca                                    33

<210> 971
<211> 33
<212> DNA
<213> Artificial Sequence

<220>
<223> Primers

<400> 971

tgaggagaca gtgaccaggg tgccacggcc cca                                    33

<210> 972
<211> 33
<212> DNA
<213> Artificial Sequence

<220>
<223> Primers

<400> 972
tgaagagacg gtgaccattg tcccttggcc cca                                    33

<210> 973
<211> 33
<212> DNA
<213> Artificial Sequence

<220>
<223> Primers

<400> 973
tgaggagacg gtgaccaggg ttccttggcc cca                                    33

<210> 974
<211> 33
<212> DNA
<213> Artificial Sequence

<220>
<223> Primers

<400> 974
tgaggagacg gtgaccaggg ttccttggcc cca                                    33

<210> 975
<211> 33
<212> DNA
<213> Artificial Sequence

<220>
<223> Primers

<400> 975
tgaggagacg gtgaccgtgg tcccttgccc cca                                    33

<210> 976
<211> 51
<212> DNA
<213> Artificial Sequence

<220>
<223> Primers

<400> 976
caggttcagc tggtgcagtc tggagctgag gtgaagaagc ctggggcctc a                51

<210> 977
<211> 51
<212> DNA
<213> Artificial Sequence

<220>
<223> Primers

<400> 977
caggtgcagc tggtgcagtc tggggctgag gtgaagaagc ctggggcctc a                51

<210> 978
<211> 51
<212> DNA
<213> Artificial Sequence

<220>
<223> Primers

<400> 978
caggtccagc tggtacagtc tggggctgag gtgaagaagc ctggggcctc a          51

<210> 979
<211> 51
<212> DNA
<213> Artificial Sequence

<220>
<223> Primers

<400> 979
caggttcagc tggtgcagtc tggggctgag gtgaagaagc ctggggcctc a          51

<210> 980
<211> 51
<212> DNA
<213> Artificial Sequence

<220>
<223> Primers

<400> 980
cagatgcagc tggtgcagtc tggggctgag gtgaagaaga ctgggtcctc a          51

<210> 981
<211> 51
<212> DNA
<213> Artificial Sequence

<220>
<223> Primers

<400> 981
caggtgcagc tggtgcagtc tggggctgag gtgaagaagc ctggggcctc a          51

<210> 982
<211> 51
<212> DNA
<213> Artificial Sequence

<220>
<223> Primers

<400> 982
caaatgcagc tggtgcagtc tgggcctgag gtgaagaagc ctgggacctc a          51

<210> 983
<211> 51
<212> DNA
<213> Artificial Sequence

<220>
<223> Primers

<400> 983
caggtgcagc tggtgcagtc tggggctgag gtgaagaagc ctgggtcctc g          51

<210> 984
<211> 51

<212> DNA
<213> Artificial Sequence

<220>
<223> Primers

<400> 984
caggtgcagc tggtgcagtc tggggctgag gtgaagaagc ctggggcctc a          51

<210> 985
<211> 51
<212> DNA
<213> Artificial Sequence

<220>
<223> Primers

<400> 985
caggtcacct tgaaggagtc tggtcctgtg ctggtgaaac ccacagagac c          51

<210> 986
<211> 51
<212> DNA
<213> Artificial Sequence

<220>
<223> Primers

<400> 986
cagatcacct tgaaggagtc tggtcctacg ctggtgaaac ccacacagac c          51

<210> 987
<211> 51
<212> DNA
<213> Artificial Sequence

<220>
<223> Primers

<400> 987
caggtcacct tgagggagtc tggtcctgcg ctggtgaaac ccacacagac c          51

<210> 988
<211> 51
<212> DNA
<213> Artificial Sequence

<220>
<223> Primers

<400> 988
caggtgcagc tggtggagtc tgggggaggc ttggtcaagc ctggagggtc c          51

<210> 989
<211> 51
<212> DNA
<213> Artificial Sequence

<220>
<223> Primers

<400> 989
gaggtgcagc tggtggagtc tgggggaggc ttggtacagc ctggggggtc c          51

<210> 990
<211> 51
<212> DNA
<213> Artificial Sequence

<220>
<223> Primers

<400> 990
gaggtgcagc tggtggagtc tgggggaggc ttggtaaagc ctggggggtc c 51

<210> 991
<211> 51
<212> DNA
<213> Artificial Sequence

<220>
<223> Primers

<400> 991
gaggtgcagc tggtggagtc tgggggaggc ttggtacagc ctggggggtc c 51

<210> 992
<211> 51
<212> DNA
<213> Artificial Sequence

<220>
<223> Primers

<400> 992
gaggtgcagc tggtggagtc tgggggaggt gtggtacggc ctggggggtc c 51

<210> 993
<211> 51
<212> DNA
<213> Artificial Sequence

<220>
<223> Primers

<400> 993
gaggtgcagc tggtggagtc tgggggaggc ctggtcaagc ctggggggtc c 51

<210> 994
<211> 51
<212> DNA
<213> Artificial Sequence

<220>
<223> Primers

<400> 994
gaggtgcagc tgttggagtc tgggggaggc ttggtacagc ctggggggtc c 51

<210> 995
<211> 51
<212> DNA
<213> Artificial Sequence

<220>
<223> Primers

<400> 995
caggtgcagc tggtggagtc tgggggaggc gtggtccagc ctgggaggtc c 51

<210> 996
<211> 51
<212> DNA
<213> Artificial Sequence

<220>

<223> Primers

<400> 996
caggtgcagc tggtggagtc tgggggaggc gtggtccagc ctgggaggtc c          51

<210> 997
<211> 51
<212> DNA
<213> Artificial Sequence

<220>
<223> Primers

<400> 997
gaggtgcagc tggtggagtc tgggggaggc ttggtacagc ctggggggtc c          51

<210> 998
<211> 51
<212> DNA
<213> Artificial Sequence

<220>
<223> Primers

<400> 998
gaggtgcagc tggtggagtc tgggggaggc ttggtacagc ctagggggtc c          51

<210> 999
<211> 51
<212> DNA
<213> Artificial Sequence

<220>
<223> Primers

<400> 999
gaagtgcagc tggtggagtc tgggggagtc gtggtacagc ctggggggtc c          51

<210> 1000
<211> 51
<212> DNA
<213> Artificial Sequence

<220>
<223> Primers

<400> 1000
gaggtgcagc tggtggagtc tgggggaggc ttggtacagc ctggggggtc c          51

<210> 1001
<211> 51
<212> DNA
<213> Artificial Sequence

<220>
<223> Primers

<400> 1001
gaggtgcagc tggtggagtc tgggggaggc ttggtacagc cagggcggtc c          51

<210> 1002
<211> 51
<212> DNA
<213> Artificial Sequence

<220>
<223> Primers

<400> 1002
gaggtgcagc tggtggagtc tggaggaggc ttgatccagc ctggggggtc c 51

<210> 1003
<211> 51
<212> DNA
<213> Artificial Sequence

<220>
<223> Primers

<400> 1003
gaggtgcagc tggtggagtc tggggaaggc ttggtccagc ctggggggtc c 51

<210> 1004
<211> 51
<212> DNA
<213> Artificial Sequence

<220>
<223> Primers

<400> 1004
gaggtgcagc tggtggagtc tggaggaggc ttgatccagc ctggggggtc c 51

<210> 1005
<211> 51
<212> DNA
<213> Artificial Sequence

<220>
<223> Primers

<400> 1005
gaggtgcagc tggtggagtc tgggggaggc ttggtccagc ctggggggtc c 51

<210> 1006
<211> 51
<212> DNA
<213> Artificial Sequence

<220>
<223> Primers

<400> 1006
gaggtgcagc tggtggagtc tgggggaggc ttggtccagc ctggagggtc c 51

<210> 1007
<211> 51
<212> DNA
<213> Artificial Sequence

<220>
<223> Primers

<400> 1007
gaggtgcagc tggtggagtc cgggggaggc ttggtccagc ctggggggtc c 51

<210> 1008
<211> 51
<212> DNA
<213> Artificial Sequence

<220>
<223> Primers

<400> 1008
gaggtgcagc tggtggagtc cgggggaggc ttagttcagc ctggggggtc c 51

<210> 1009
<211> 51
<212> DNA
<213> Artificial Sequence

<220>
<223> Primers

<400> 1009
gaagtgcagc tggtggagtc tgggggaggc ttggtacagc ctggcaggtc c 51

<210> 1010
<211> 51
<212> DNA
<213> Artificial Sequence

<220>
<223> Primers

<400> 1010
caggtgcagc tgcaggagtc gggcccagga ctggtgaagc cttcggacac c 51

<210> 1011
<211> 51
<212> DNA
<213> Artificial Sequence

<220>
<223> Primers

<400> 1011
caggtgcagc tgcaggagtc gggcccagga ctggtgaagc cttcacagac c 51

<210> 1012
<211> 51
<212> DNA
<213> Artificial Sequence

<220>
<223> Primers

<400> 1012
caggtgcagc tacagcagtg gggcgcagga ctgttgaagc cttcggagac c 51

<210> 1013
<211> 51
<212> DNA
<213> Artificial Sequence

<220>
<223> Primers

<400> 1013
cagctgcagc tgcaggagtc gggcccagga ctggtgaagc cttcggagac c 51

<210> 1014
<211> 51
<212> DNA
<213> Artificial Sequence

<220>
<223> Primers

<400> 1014
caggtgcagc tgcaggagtc gggcccagga ctggtgaagc cttcggagac c 51

<210> 1015

<211> 51
<212> DNA
<213> Artificial Sequence

<220>
<223> Primers

<400> 1015
caggtgcagc tgcaggagtc gggcccagga ctggtgaagc cttcggagac c          51

<210> 1016
<211> 51
<212> DNA
<213> Artificial Sequence

<220>
<223> Primers

<400> 1016
caggtgcagc tgcaggagtc gggcccagga ctggtgaagc cttcggagac c          51

<210> 1017
<211> 51
<212> DNA
<213> Artificial Sequence

<220>
<223> Primers

<400> 1017
gaggtgcagc tggtgcagtc tggagcagag gtgaaaaagc ccggggagtc t          51

<210> 1018
<211> 51
<212> DNA
<213> Artificial Sequence

<220>
<223> Primers

<400> 1018
caggtacagc tgcagcagtc aggtccagga ctggtgaagc cctcgcagac c          51

<210> 1019
<211> 51
<212> DNA
<213> Artificial Sequence

<220>
<223> Primers

<400> 1019
caggtgcagc tggtgcagtc tggccatgag gtgaagcagc ctggggcctc a          51

<210> 1020
<211> 45
<212> DNA
<213> Artificial Sequence

<220>
<223> Primers

<400> 1020
agaagccttg caggagacct tcactgaggc cccaggcttc ttcac                 45

<210> 1021
<211> 45
<212> DNA

<213> Artificial Sequence

<220>
<223> Primers

<400> 1021
agaagccttg caggagacct tcactgaggc cccaggcttc ttcac                45

<210> 1022
<211> 45
<212> DNA
<213> Artificial Sequence

<220>
<223> Primers

<400> 1022
ggaaaccttg caggagacct tcactgaggc cccaggcttc ttcac                45

<210> 1023
<211> 45
<212> DNA
<213> Artificial Sequence

<220>
<223> Primers

<400> 1023
agaagccttg caggaaacct tcactgaggc cccaggcttc ttcac                45

<210> 1024
<211> 45
<212> DNA
<213> Artificial Sequence

<220>
<223> Primers

<400> 1024
ggaagccttg caggaaacct tcactgagga cccagtcttc ttcac                45

<210> 1025
<211> 45
<212> DNA
<213> Artificial Sequence

<220>
<223> Primers

<400> 1025
agatgccttg caggaaacct tcactgaggc cccaggcttc ttcac                45

<210> 1026
<211> 45
<212> DNA
<213> Artificial Sequence

<220>
<223> Primers

<400> 1026
agaagccttg caggagacct tcactgaggt cccaggcttc ttcac                45

<210> 1027
<211> 45
<212> DNA
<213> Artificial Sequence

<220>
<223> Primers

<400> 1027
agaagccttg caggagacct tcaccgagga cccaggcttc ttcac 45

<210> 1028
<211> 45
<212> DNA
<213> Artificial Sequence

<220>
<223> Primers

<400> 1028
agaagccttg caggagacct tcactgaggc cccaggcttc ttcac 45

<210> 1029
<211> 45
<212> DNA
<213> Artificial Sequence

<220>
<223> Primers

<400> 1029
agagacggtg caggtcagcg tgagggtctc tgtgggtttc accag 45

<210> 1030
<211> 45
<212> DNA
<213> Artificial Sequence

<220>
<223> Primers

<400> 1030
agagaaggtg caggtcagcg tgagggtctg tgtgggtttc accag 45

<210> 1031
<211> 45
<212> DNA
<213> Artificial Sequence

<220>
<223> Primers

<400> 1031
agagaaggtg caggtcagtg tgagggtctg tgtgggtttc accag 45

<210> 1032
<211> 45
<212> DNA
<213> Artificial Sequence

<220>
<223> Primers

<400> 1032
agaggctgca caggagagtc tcagggaccc tccaggcttg accaa 45

<210> 1033
<211> 45
<212> DNA
<213> Artificial Sequence

<220>
<223> Primers

<400> 1033
agaggctgca caggagagtc tcagggaccc cccaggctgt accaa                45

<210> 1034
<211> 45
<212> DNA
<213> Artificial Sequence

<220>
<223> Primers

<400> 1034
agaggctgca caggagagtc taagggaccc cccaggcttt accaa                45

<210> 1035
<211> 45
<212> DNA
<213> Artificial Sequence

<220>
<223> Primers

<400> 1035
agaggctgca caggagagtc tcagggaccc cccaggctgt accaa                45

<210> 1036
<211> 45
<212> DNA
<213> Artificial Sequence

<220>
<223> Primers

<400> 1036
agaggctgca caggagagtc tcagggaccc cccaggccgt accac                45

<210> 1037
<211> 45
<212> DNA
<213> Artificial Sequence

<220>
<223> Primers

<400> 1037
agaggctgca caggagagtc tcagggaccc cccaggcttg accag                45

<210> 1038
<211> 45
<212> DNA
<213> Artificial Sequence

<220>
<223> Primers

<400> 1038
agaggctgca caggagagtc tcagggaccc cccaggctgt accaa                45

<210> 1039
<211> 45
<212> DNA
<213> Artificial Sequence

<220>
<223> Primers

<400> 1039

agaggctgca caggagagtc tcagggacct cccaggctgg accac 45

<210> 1040
<211> 45
<212> DNA
<213> Artificial Sequence

<220>
<223> Primers

<400> 1040
agacgctgca caggagagtc tcagggacct cccaggctgg accac 45

<210> 1041
<211> 45
<212> DNA
<213> Artificial Sequence

<220>
<223> Primers

<400> 1041
agaggctgca caggagagtc tcagggatcc cccaggctgt accaa 45

<210> 1042
<211> 45
<212> DNA
<213> Artificial Sequence

<220>
<223> Primers

<400> 1042
agaggctgca caggagagtc tcagggaccc cctaggctgt accaa 45

<210> 1043
<211> 45
<212> DNA
<213> Artificial Sequence

<220>
<223> Primers

<400> 1043
agaggctgca caggagagtc tcagggaccc cccaggctgt accac 45

<210> 1044
<211> 45
<212> DNA
<213> Artificial Sequence

<220>
<223> Primers

<400> 1044
agaggctgca caggagagtc tcagggaccc cccaggctgt accaa 45

<210> 1045
<211> 45
<212> DNA
<213> Artificial Sequence

<220>
<223> Primers

<400> 1045
agaagctgta caggagagtc tcagggaccg ccctggctgt accaa 45

<210> 1046
<211> 45
<212> DNA
<213> Artificial Sequence

<220>
<223> Primers

<400> 1046
agaggctgca caggagagtc tcagggaccc cccaggctgg atcaa                45

<210> 1047
<211> 45
<212> DNA
<213> Artificial Sequence

<220>
<223> Primers

<400> 1047
agaggctgca caggagagtc tcagggaccc cccaggctgg accaa                45

<210> 1048
<211> 45
<212> DNA
<213> Artificial Sequence

<220>
<223> Primers

<400> 1048
agaggctgca caggagagtc tcagggaccc cccaggctgg atcaa                45

<210> 1049
<211> 45
<212> DNA
<213> Artificial Sequence

<220>
<223> Primers

<400> 1049
agaggctgca caggagagtc tcagggaccc cccaggctgg accaa                45

<210> 1050
<211> 45
<212> DNA
<213> Artificial Sequence

<220>
<223> Primers

<400> 1050
agaggctgca caggagagtc tcagggaccc tccaggctgg accaa                45

<210> 1051
<211> 45
<212> DNA
<213> Artificial Sequence

<220>
<223> Primers

<400> 1051
agaggctgca caggagagtt tcagggaccc cccaggctgg accaa                45

<210> 1052
<211> 45

<212> DNA
<213> Artificial Sequence

<220>
<223> Primers

<400> 1052
agaggctgca caggagagtc tcagggaccc cccaggctga actaa                45

<210> 1053
<211> 45
<212> DNA
<213> Artificial Sequence

<220>
<223> Primers

<400> 1053
agaggctgca caggagagtc tcagggacct gccaggctgt accaa                45

<210> 1054
<211> 45
<212> DNA
<213> Artificial Sequence

<220>
<223> Primers

<400> 1054
agagacagcg caggtgaggg acagggtgtc cgaaggcttc accag                45

<210> 1055
<211> 45
<212> DNA
<213> Artificial Sequence

<220>
<223> Primers

<400> 1055
agagacagta caggtgaggg acagggtctg tgaaggcttc accag                45

<210> 1056
<211> 45
<212> DNA
<213> Artificial Sequence

<220>
<223> Primers

<400> 1056
atagacagcg caggtgaggg acagggtctc cgaaggcttc aacag                45

<210> 1057
<211> 45
<212> DNA
<213> Artificial Sequence

<220>
<223> Primers

<400> 1057
agagacagtg caggtgaggg acagggtctc cgaaggcttc accag                45

<210> 1058
<211> 45
<212> DNA
<213> Artificial Sequence

<220>
<223> Primers

<400> 1058
agagacagtg caggtgaggg acagggtctc cgaaggcttc accag                45

<210> 1059
<211> 45
<212> DNA
<213> Artificial Sequence

<220>
<223> Primers

<400> 1059
agagacagtg caggtgaggg acagggtctc cgaaggcttc accag                45

<210> 1060
<211> 45
<212> DNA
<213> Artificial Sequence

<220>
<223> Primers

<400> 1060
agagacagtg caggtgaggg acagggtctc cgaaggcttc accag                45

<210> 1061
<211> 45
<212> DNA
<213> Artificial Sequence

<220>
<223> Primers

<400> 1061
agaaccctta caggagatct tcagagactc cccgggcttt ttcac                45

<210> 1062
<211> 45
<212> DNA
<213> Artificial Sequence

<220>
<223> Primers

<400> 1062
ggagatggca caggtgagtg agagggtctg cgagggcttc accag                45

<210> 1063
<211> 45
<212> DNA
<213> Artificial Sequence

<220>
<223> Primers

<400> 1063
agaagccttg caggagacct tcactgaggc cccaggctgc ttcac                45

<210> 1064
<211> 42
<212> DNA
<213> Artificial Sequence

<220>

<223> Primers

<400> 1064
tatggtatca gctgggtgcg acaggcccct ggacaagggc tt                42

<210> 1065
<211> 42
<212> DNA
<213> Artificial Sequence

<220>
<223> Primers

<400> 1065
tactatatgc actgggtgcg acaggcccct ggacaagggc tt                42

<210> 1066
<211> 42
<212> DNA
<213> Artificial Sequence

<220>
<223> Primers

<400> 1066
ttatccatgc actgggtgcg acaggctcct ggaaaagggc tt                42

<210> 1067
<211> 42
<212> DNA
<213> Artificial Sequence

<220>
<223> Primers

<400> 1067
tatgctatgc attgggtgcg ccaggccccc ggacaaaggc tt                42

<210> 1068
<211> 42
<212> DNA
<213> Artificial Sequence

<220>
<223> Primers

<400> 1068
cgctacctgc actgggtgcg acaggccccc ggacaagcgc tt                42

<210> 1069
<211> 42
<212> DNA
<213> Artificial Sequence

<220>
<223> Primers

<400> 1069
tactatatgc actgggtgcg acaggcccct ggacaagggc tt                42

<210> 1070
<211> 42
<212> DNA
<213> Artificial Sequence

<220>
<223> Primers

<400> 1070
tctgctatgc agtgggtgcg acaggctcgt ggacaacgcc tt                42

<210> 1071
<211> 42
<212> DNA
<213> Artificial Sequence

<220>
<223> Primers

<400> 1071
tatgctatca gctgggtgcg acaggcccct ggacaagggc tt                42

<210> 1072
<211> 42
<212> DNA
<213> Artificial Sequence

<220>
<223> Primers

<400> 1072
tatgatatca actgggtgcg acaggccact ggacaagggc tt                42

<210> 1073
<211> 42
<212> DNA
<213> Artificial Sequence

<220>
<223> Primers

<400> 1073
atgggtgtga gctggatccg tcagccccca gggaaggccc tg                42

<210> 1074
<211> 42
<212> DNA
<213> Artificial Sequence

<220>
<223> Primers

<400> 1074
gtgggtgtgg gctggatccg tcagccccca ggaaaggccc tg                42

<210> 1075
<211> 42
<212> DNA
<213> Artificial Sequence

<220>
<223> Primers

<400> 1075
atgtgtgtga gctggatccg tcagccccca gggaaggccc tg                42

<210> 1076
<211> 42
<212> DNA
<213> Artificial Sequence

<220>
<223> Primers

<400> 1076
tactacatga gctggatccg ccaggctcca gggaaggggc tg                42

<210> 1077
<211> 42
<212> DNA
<213> Artificial Sequence

<220>
<223> Primers

<400> 1077
tacgacatgc actgggtccg ccaagctaca ggaaaaggtc tg                42

<210> 1078
<211> 42
<212> DNA
<213> Artificial Sequence

<220>
<223> Primers

<400> 1078
gcctggatga gctgggtccg ccaggctcca gggaaggggc tg                42

<210> 1079
<211> 42
<212> DNA
<213> Artificial Sequence

<220>
<223> Primers

<400> 1079
agtgacatga actgggcccg caaggctcca ggaaaggggc tg                42

<210> 1080
<211> 42
<212> DNA
<213> Artificial Sequence

<220>
<223> Primers

<400> 1080
tatggcatga gctgggtccg ccaagctcca gggaaggggc tg                42

<210> 1081
<211> 42
<212> DNA
<213> Artificial Sequence

<220>
<223> Primers

<400> 1081
tatagcatga actgggtccg ccaggctcca gggaaggggc tg                42

<210> 1082
<211> 42
<212> DNA
<213> Artificial Sequence

<220>
<223> Primers

<400> 1082
tatgccatga gctgggtccg ccaggctcca gggaaggggc tg                42

<210> 1083

<211> 42
<212> DNA
<213> Artificial Sequence

<220>
<223> Primers

<400> 1083
tatggcatgc actgggtccg ccaggctcca ggcaaggggc tg                42

<210> 1084
<211> 42
<212> DNA
<213> Artificial Sequence

<220>
<223> Primers

<400> 1084
tatggcatgc actgggtccg ccaggctcca ggcaaggggc tg                42

<210> 1085
<211> 42
<212> DNA
<213> Artificial Sequence

<220>
<223> Primers

<400> 1085
agtgacatga actgggtcca tcaggctcca ggaaaggggc tg                42

<210> 1086
<211> 42
<212> DNA
<213> Artificial Sequence

<220>
<223> Primers

<400> 1086
aatgagatga gctggatccg ccaggctcca gggaaggggc tg                42

<210> 1087
<211> 42
<212> DNA
<213> Artificial Sequence

<220>
<223> Primers

<400> 1087
tataccatgc actgggtccg tcaagctccg gggaagggtc tg                42

<210> 1088
<211> 42
<212> DNA
<213> Artificial Sequence

<220>
<223> Primers

<400> 1088
tatagcatga actgggtccg ccaggctcca gggaaggggc tg                42

<210> 1089
<211> 42
<212> DNA

<213> Artificial Sequence

<220>
<223> Primers

<400> 1089
tatgctatga gctggttccg ccaggctcca gggaaggggc tg                42

<210> 1090
<211> 42
<212> DNA
<213> Artificial Sequence

<220>
<223> Primers

<400> 1090
aactacatga gctgggtccg ccaggctcca gggaaggggc tg                42

<210> 1091
<211> 42
<212> DNA
<213> Artificial Sequence

<220>
<223> Primers

<400> 1091
tatgctatgc actgggtccg ccaggctcca gggaagggac tg                42

<210> 1092
<211> 42
<212> DNA
<213> Artificial Sequence

<220>
<223> Primers

<400> 1092
aactacatga gctgggtccg ccaggctcca gggaaggggc tg                42

<210> 1093
<211> 42
<212> DNA
<213> Artificial Sequence

<220>
<223> Primers

<400> 1093
tattggatga gctgggtccg ccaggctcca gggaaggggc tg                42

<210> 1094
<211> 42
<212> DNA
<213> Artificial Sequence

<220>
<223> Primers

<400> 1094
cactacatgg actgggtccg ccaggctcca gggaaggggc tg                42

<210> 1095
<211> 42
<212> DNA
<213> Artificial Sequence

<220>
<223> Primers

<400> 1095
tctgctatgc actgggtccg ccaggcttcc gggaaagggc tg                42

<210> 1096
<211> 42
<212> DNA
<213> Artificial Sequence

<220>
<223> Primers

<400> 1096
tactggatgc actgggtccg ccaagctcca gggaaggggc tg                42

<210> 1097
<211> 42
<212> DNA
<213> Artificial Sequence

<220>
<223> Primers

<400> 1097
tatgccatgc actgggtccg gcaagctcca gggaagggcc tg                42

<210> 1098
<211> 42
<212> DNA
<213> Artificial Sequence

<220>
<223> Primers

<400> 1098
aactggtggg gctggatccg gcagccccca gggaagggac tg                42

<210> 1099
<211> 42
<212> DNA
<213> Artificial Sequence

<220>
<223> Primers

<400> 1099
tactactgga gctggatccg ccagcaccca gggaagggcc tg                42

<210> 1100
<211> 42
<212> DNA
<213> Artificial Sequence

<220>
<223> Primers

<400> 1100
tactactgga gctggatccg ccagccccca gggaaggggc tg                42

<210> 1101
<211> 42
<212> DNA
<213> Artificial Sequence

<220>
<223> Primers

<400> 1101
tactactggg gctggatccg ccagccccca gggaaggggc tg                42

<210> 1102
<211> 42
<212> DNA
<213> Artificial Sequence

<220>
<223> Primers

<400> 1102
tactactgga gctggatccg gcagcccgcc gggaagggac tg                42

<210> 1103
<211> 42
<212> DNA
<213> Artificial Sequence

<220>
<223> Primers

<400> 1103
tactactgga gctggatccg gcagccccca gggaagggac tg                42

<210> 1104
<211> 42
<212> DNA
<213> Artificial Sequence

<220>
<223> Primers

<400> 1104
tactactgga gctggatccg gcagccccca gggaagggac tg                42

<210> 1105
<211> 42
<212> DNA
<213> Artificial Sequence

<220>
<223> Primers

<400> 1105
tactggatcg gctgggtgcg ccagatgccc gggaaaggcc tg                42

<210> 1106
<211> 42
<212> DNA
<213> Artificial Sequence

<220>
<223> Primers

<400> 1106
gctgcttgga actggatcag gcagtcccca tcgagaggcc tt                42

<210> 1107
<211> 42
<212> DNA
<213> Artificial Sequence

<220>
<223> Primers

<400> 1107

tatggtatga attgggtgcc acaggcccct ggacaagggc tt 42

<210> 1108
<211> 39
<212> DNA
<213> Artificial Sequence

<220>
<223> Primers

<400> 1108
gatccatccc atccactcaa gcccttgtcc aggggcctg 39

<210> 1109
<211> 39
<212> DNA
<213> Artificial Sequence

<220>
<223> Primers

<400> 1109
gatccatccc atccactcaa gcccttgtcc aggggcctg 39

<210> 1110
<211> 39
<212> DNA
<213> Artificial Sequence

<220>
<223> Primers

<400> 1110
aaaacctccc atccactcaa gcccttttcc aggagcctg 39

<210> 1111
<211> 39
<212> DNA
<213> Artificial Sequence

<220>
<223> Primers

<400> 1111
gctccatccc atccactcaa gcctttgtcc gggggcctg 39

<210> 1112
<211> 39
<212> DNA
<213> Artificial Sequence

<220>
<223> Primers

<400> 1112
gatccatccc atccactcaa gcgcttgtcc gggggcctg 39

<210> 1113
<211> 39
<212> DNA
<213> Artificial Sequence

<220>
<223> Primers

<400> 1113
gattattccc atccactcaa gcccttgtcc aggggcctg 39

<210> 1114
<211> 39
<212> DNA
<213> Artificial Sequence

<220>
<223> Primers

<400> 1114
gatccatcct atccactcaa ggcgttgtcc acgagcctg                              39

<210> 1115
<211> 39
<212> DNA
<213> Artificial Sequence

<220>
<223> Primers

<400> 1115
gatccctccc atccactcaa gcccttgtcc aggggcctg                              39

<210> 1116
<211> 39
<212> DNA
<213> Artificial Sequence

<220>
<223> Primers

<400> 1116
catccatccc atccactcaa gcccttgtcc agtggcctg                              39

<210> 1117
<211> 39
<212> DNA
<213> Artificial Sequence

<220>
<223> Primers

<400> 1117
aatgtgtgca agccactcca gggccttccc tgggggctg                              39

<210> 1118
<211> 39
<212> DNA
<213> Artificial Sequence

<220>
<223> Primers

<400> 1118
aatgagtgca agccactcca gggcctttcc tgggggctg                              39

<210> 1119
<211> 39
<212> DNA
<213> Artificial Sequence

<220>
<223> Primers

<400> 1119
aatgagtgca agccactcca gggccttccc tgggggctg                              39

<210> 1120
<211> 39

<212> DNA
<213> Artificial Sequence

<220>
<223> Primers

<400> 1120
aatgtatgaa acccactcca gccccttccc tggagcctg 39

<210> 1121
<211> 39
<212> DNA
<213> Artificial Sequence

<220>
<223> Primers

<400> 1121
aatagctgag acccactcca gaccttttcc tgtagcttg 39

<210> 1122
<211> 39
<212> DNA
<213> Artificial Sequence

<220>
<223> Primers

<400> 1122
aatacggcca acccactcca gccccttccc tggagcctg 39

<210> 1123
<211> 39
<212> DNA
<213> Artificial Sequence

<220>
<223> Primers

<400> 1123
aacacccgat acccactcca gcccctttcc tggagcctt 39

<210> 1124
<211> 39
<212> DNA
<213> Artificial Sequence

<220>
<223> Primers

<400> 1124
aataccagag acccactcca gccccttccc tggagcttg 39

<210> 1125
<211> 39
<212> DNA
<213> Artificial Sequence

<220>
<223> Primers

<400> 1125
aatggatgag acccactcca gccccttccc tggagcctg 39

<210> 1126
<211> 39
<212> DNA
<213> Artificial Sequence

<220>
<223> Primers

<400> 1126
aatagctgag acccactcca gccccttccc tggagcctg                                39

<210> 1127
<211> 39
<212> DNA
<213> Artificial Sequence

<220>
<223> Primers

<400> 1127
tataactgcc acccactcca gccccttgcc tggagcctg                                39

<210> 1128
<211> 39
<212> DNA
<213> Artificial Sequence

<220>
<223> Primers

<400> 1128
tataactgcc acccactcca gccccttgcc tggagcctg                                39

<210> 1129
<211> 39
<212> DNA
<213> Artificial Sequence

<220>
<223> Primers

<400> 1129
aacacccgat acccactcca gcccctttcc tggagcctg                                39

<210> 1130
<211> 39
<212> DNA
<213> Artificial Sequence

<220>
<223> Primers

<400> 1130
aatggatgag acccactcca gccccttccc tggagcctg                                39

<210> 1131
<211> 38
<212> DNA
<213> Artificial Sequence

<220>
<223> Primers

<400> 1131
ataagagaga cccactccag acccttcccc ggagcttg                                 38

<210> 1132
<211> 39
<212> DNA
<213> Artificial Sequence

<220>

<223> Primers

<400> 1132
aatgtatgaa acccactcca gccccttccc tggagcctg                                39

<210> 1133
<211> 39
<212> DNA
<213> Artificial Sequence

<220>
<223> Primers

<400> 1133
aatgaaacct acccactcca gccccttccc tggagcctg                                39

<210> 1134
<211> 39
<212> DNA
<213> Artificial Sequence

<220>
<223> Primers

<400> 1134
aataactgag acccactcca gccccttccc tggagcctg                                39

<210> 1135
<211> 39
<212> DNA
<213> Artificial Sequence

<220>
<223> Primers

<400> 1135
aatagctgaa acatattcca gtcccttccc tggagcctg                                39

<210> 1136
<211> 39
<212> DNA
<213> Artificial Sequence

<220>
<223> Primers

<400> 1136
aataactgag acccactcca gccccttccc tggagcctg                                39

<210> 1137
<211> 39
<212> DNA
<213> Artificial Sequence

<220>
<223> Primers

<400> 1137
tatgttggcc acccactcca gccccttccc tggagcctg                                39

<210> 1138
<211> 39
<212> DNA
<213> Artificial Sequence

<220>
<223> Primers

<400> 1138
agtacggcca acccactcca gccccttccc tggagcctg 39

<210> 1139
<211> 39
<212> DNA
<213> Artificial Sequence

<220>
<223> Primers

<400> 1139
aatacggcca acccactcca gccctttccc ggaagcctg 39

<210> 1140
<211> 39
<212> DNA
<213> Artificial Sequence

<220>
<223> Primers

<400> 1140
aatacgtgag acccacacca gccccttccc tggagcttg 39

<210> 1141
<211> 39
<212> DNA
<213> Artificial Sequence

<220>
<223> Primers

<400> 1141
aatacctgag acccactcca ggcccttccc tggagcttg 39

<210> 1142
<211> 39
<212> DNA
<213> Artificial Sequence

<220>
<223> Primers

<400> 1142
   gatgtaccca atccactcca gtcccttccc tgggggctg 39

<210> 1143
<211> 39
<212> DNA
<213> Artificial Sequence

<220>
<223> Primers

<400> 1143
gatgtaccca atccactcca ggcccttccc tgggtgctg 39

<210> 1144
<211> 39
<212> DNA
<213> Artificial Sequence

<220>
<223> Primers

<400> 1144
   gatttcccca atccactcca gccccttccc tgggggctg 39

<210> 1145
<211> 39
<212> DNA
<213> Artificial Sequence

<220>
<223> Primers

<400> 1145
   gatactccca atccactcca gccccttccc tgggggctg                              39

<210> 1146
<211> 39
<212> DNA
<213> Artificial Sequence

<220>
<223> Primers

<400> 1146
gatacgccca atccactcca gtcccttccc ggcgggctg                               39

<210> 1147
<211> 39
<212> DNA
<213> Artificial Sequence

<220>
<223> Primers

<400> 1147
   gatataccca atccactcca gtcccttccc tgggggctg                              39

<210> 1148
<211> 39
<212> DNA
<213> Artificial Sequence

<220>
<223> Primers

<400> 1148
   gatataccca atccactcca gtcccttccc tgggggctg                              39

<210> 1149
<211> 39
<212> DNA
<213> Artificial Sequence

<220>
<223> Primers

<400> 1149
gatgatcccc atccactcca ggcctttccc gggcatctg                               39

<210> 1150
<211> 39
<212> DNA
<213> Artificial Sequence

<220>
<223> Primers

<400> 1150
tgtccttccc agccactcaa ggcctctcga tggggactg                               39

<210> 1151

<211> 39
<212> DNA
<213> Artificial Sequence

<220>
<223> Primers

<400> 1151
gaaccatccc atccactcaa gcccttgtcc aggggcctg 39

<210> 1152
<211> 73
<212> DNA
<213> Artificial Sequence

<220>
<223> Primers

<400> 1152
acaaactatg cacagaagct ccagggcaga gtcaccatga ccacagacac atccacgagc 60
acagcctaca tgg 73

<210> 1153
<211> 73
<212> DNA
<213> Artificial Sequence

<220>
<223> Primers

<400> 1153
acaaactatg cacagaagtt tcagggcagg gtcaccatga ccagggacac gtccatcagc 60
acagcctaca tgg 73

<210> 1154
<211> 73
<212> DNA
<213> Artificial Sequence

<220>
<223> Primers

<400> 1154
acaatctacg cacagaagtt ccagggcaga gtcaccatga ccgaggacac atctacagac 60
acagcctaca tgg 73

<210> 1155
<211> 73
<212> DNA
<213> Artificial Sequence

<220>
<223> Primers

<400> 1155
acaaaatatt cacaggagtt ccagggcaga gtcaccatta ccagggacac atccgcgagc 60
acagcctaca tgg 73

<210> 1156
<211> 73
<212> DNA
<213> Artificial Sequence

<220>
<223> Primers

<400> 1156
accaactacg cacagaaatt ccaggacaga gtcaccatta ccagggacag gtctatgagc 60

acagcctaca tgg                                                          73

<210> 1157
<211> 73
<212> DNA
<213> Artificial Sequence

<220>
<223> Primers

<400> 1157
acaagctacg cacagaagtt ccagggcaga gtcaccatga ccagggacac gtccacgagc 60
acagtctaca tgg                                                          73

<210> 1158
<211> 73
<212> DNA
<213> Artificial Sequence

<220>
<223> Primers

<400> 1158
acaaactacg cacagaagtt ccaggaaaga gtcaccatta ccagggacat gtccacaagc 60
acagcctaca tgg                                                          73

<210> 1159
<211> 73
<212> DNA
<213> Artificial Sequence

<220>
<223> Primers

<400> 1159
gcaaactacg cacagaagtt ccagggcaga gtcacgatta ccgcggacaa atccacgagc 60
acagcctaca tgg                                                          73

<210> 1160
<211> 73
<212> DNA
<213> Artificial Sequence

<220>
<223> Primers

<400> 1160
acaggctatg cacagaagtt ccagggcaga gtcaccatga ccaggaacac ctccataagc 60
acagcctaca tgg                                                          73

<210> 1161
<211> 73
<212> DNA
<213> Artificial Sequence

<220>
<223> Primers

<400> 1161
aaatcctaca gcacatctct gaagagcagg ctcaccatct ccaaggacac ctccaaaagc 60
caggtggtcc tta                                                          73

<210> 1162
<211> 73
<212> DNA
<213> Artificial Sequence

<220>

<223> Primers

<400> 1162
aagcgctaca gcccatctct gaagagcagg ctcaccatca ccaaggacac ctccaaaaac 60
caggtggtcc tta 73

<210> 1163
<211> 73
<212> DNA
<213> Artificial Sequence

<220>
<223> Primers

<400> 1163
aaatactaca gcacatctct gaagaccagg ctcaccatct ccaaggacac ctccaaaaac 60
caggtggtcc tta 73

<210> 1164
<211> 73
<212> DNA
<213> Artificial Sequence

<220>
<223> Primers

<400> 1164
atatactacg cagactctgt gaagggccga ttcaccatct ccagggacaa cgccaagaac 60
tcactgtatc tgc 73

<210> 1165
<211> 73
<212> DNA
<213> Artificial Sequence

<220>
<223> Primers

<400> 1165
acatactatc caggctccgt gaagggccga ttcaccatct ccagagaaaa tgccaagaac 60
tccttgtatc ttc 73

<210> 1166
<211> 73
<212> DNA
<213> Artificial Sequence

<220>
<223> Primers

<400> 1166
acagactacg ctgcacccgt gaaaggcaga ttcaccatct caagagatga ttcaaaaaac 60
acgctgtatc tgc 73

<210> 1167
<211> 73
<212> DNA
<213> Artificial Sequence

<220>
<223> Primers

<400> 1167
acgcactatg tggactccgt gaagcgccga ttcatcatct ccagagacaa ttccaggaac 60
tccctgtatc tgc 73

<210> 1168
<211> 73

<212> DNA
<213> Artificial Sequence

<220>
<223> Primers

<400> 1168
acaggttatg cagactctgt gaagggccga ttcaccatct ccagagacaa cgccaagaac 60
tccctgtatc tgc 73

<210> 1169
<211> 73
<212> DNA
<213> Artificial Sequence

<220>
<223> Primers

<400> 1169
atatactacg cagactcagt gaagggccga ttcaccatct ccagagacaa cgccaagaac 60
tcactgtatc tgc 73

<210> 1170
<211> 73
<212> DNA
<213> Artificial Sequence

<220>
<223> Primers

<400> 1170
acatactacg cagactccgt gaagggccgg ttcaccatct ccagagacaa ttccaagaac 60
acgctgtatc tgc 73

<210> 1171
<211> 73
<212> DNA
<213> Artificial Sequence

<220>
<223> Primers

<400> 1171
aaatactatg cagactccgt gaagggccga ttcaccatct ccagagacaa ttccaagaac 60
acgctgtatc tgc 73

<210> 1172
<211> 73
<212> DNA
<213> Artificial Sequence

<220>
<223> Primers

<400> 1172
aaatactatg cagactccgt gaagggccga ttcaccatct ccagagacaa ttccaagaac 60
acgctgtatc tgc 73

<210> 1173
<211> 73
<212> DNA
<213> Artificial Sequence

<220>
<223> Primers

<400> 1173
acgcactatg cagactctgt gaagggccga ttcatcatct ccagagacaa ttccaggaac 60

accctgtatc tgc                                                        73

<210> 1174
<211> 73
<212> DNA
<213> Artificial Sequence

<220>
<223> Primers

<400> 1174
acatactacg cagactccag gaagggcaga ttcaccatct ccagagacaa ttccaagaac 60
acgctgtatc ttc                                                        73

<210> 1175
<211> 73
<212> DNA
<213> Artificial Sequence

<220>
<223> Primers

<400> 1175
acatactatg cagactctgt gaagggccga ttcaccatct ccagagacaa cagcaaaaac 60
tccctgtatc tgc                                                        73

<210> 1176
<211> 73
<212> DNA
<213> Artificial Sequence

<220>
<223> Primers

<400> 1176
atatactacg cagactctgt gaagggccga ttcaccatct ccagagacaa tgccaagaac 60
tcactgtatc tgc                                                        73

<210> 1177
<211> 73
<212> DNA
<213> Artificial Sequence

<220>
<223> Primers

<400> 1177
acagaatacg ccgcgtctgt gaaaggcaga ttcaccatct caagagatga ttccaaaagc 60
atcgcctatc tgc                                                        73

<210> 1178
<211> 73
<212> DNA
<213> Artificial Sequence

<220>
<223> Primers

<400> 1178
acatactacg cagactccgt gaagggccga ttcaccatct ccagagacaa ttccaagaac 60
acgctgtatc ttc                                                        73

<210> 1179
<211> 73
<212> DNA
<213> Artificial Sequence

<220>

<223> Primers

<400> 1179
acatattatg cagactctgt gaagggcaga ttcaccatct ccagagacaa ttccaagaac 60
acgctgtatc ttc 73

<210> 1180
<211> 73
<212> DNA
<213> Artificial Sequence

<220>
<223> Primers

<400> 1180
acatactacg cagactccgt gaagggccga ttcaccatct ccagagacaa ttccaagaac 60
acgctgtatc ttc 73

<210> 1181
<211> 73
<212> DNA
<213> Artificial Sequence

<220>
<223> Primers

<400> 1181
aaatactatg tggactctgt gaagggccga ttcaccatct ccagagacaa cgccaagaac 60
tcactgtatc tgc 73

<210> 1182
<211> 73
<212> DNA
<213> Artificial Sequence

<220>
<223> Primers

<400> 1182
acagaatacg ccgcgtctgt gaaaggcaga ttcaccatct caagagatga ttcaaagaac 60
tcactgtatc tgc 73

<210> 1183
<211> 73
<212> DNA
<213> Artificial Sequence

<220>
<223> Primers

<400> 1183
acagcatatg ctgcgtcggt gaaaggcagg ttcaccatct ccagagatga ttcaaagaac 60
acggcgtatc tgc 73

<210> 1184
<211> 73
<212> DNA
<213> Artificial Sequence

<220>
<223> Primers

<400> 1184
acaagctacg cggactccgt gaagggccga ttcaccatct ccagagacaa cgccaagaac 60
acgctgtatc tgc 73

<210> 1185
<211> 73

<212> DNA
<213> Artificial Sequence

<220>
<223> Primers

<400> 1185
ataggctatg cggactctgt gaagggccga ttcaccatct ccagagacaa cgccaagaac 60
tccctgtatc tgc 73

<210> 1186
<211> 73
<212> DNA
<213> Artificial Sequence

<220>
<223> Primers

<400> 1186
acctactaca acccgtccct caagagtcga gtcaccatgt cagtagacac gtccaagaac 60
cagttctccc tga 73

<210> 1187
<211> 73
<212> DNA
<213> Artificial Sequence

<220>
<223> Primers

<400> 1187
acctactaca acccgtccct caagagtcga gttaccatat cagtagacac gtctaagaac 60
cagttctccc tga 73

<210> 1188
<211> 73
<212> DNA
<213> Artificial Sequence

<220>
<223> Primers

<400> 1188
accaactaca acccgtccct caagagtcga gtcaccatat cagtagacac gtccaagaac 60
cagttctccc tga 73

<210> 1189
<211> 73
<212> DNA
<213> Artificial Sequence

<220>
<223> Primers

<400> 1189
acctactaca acccgtccct caagagtcga gtcaccatat ccgtagacac gtccaagaac 60
cagttctccc tga 73

<210> 1190
<211> 73
<212> DNA
<213> Artificial Sequence

<220>
<223> Primers

<400> 1190
accaactaca acccctccct caagagtcga gtcaccatgt cagtagacac gtccaagaac 60

cagttctccc tga                                                          73

<210> 1191
<211> 73
<212> DNA
<213> Artificial Sequence

<220>
<223> Primers

<400> 1191
accaactaca acccctccct caagagtcga gtcaccatat cagtagacac gtccaagaac 60
cagttctccc tga                                                          73

<210> 1192
<211> 73
<212> DNA
<213> Artificial Sequence

<220>
<223> Primers

<400> 1192
accaactaca acccctccct caagagtcga gtcaccatat cagtagacac gtccaagaac 60
cagttctccc tga                                                          73

<210> 1193
<211> 73
<212> DNA
<213> Artificial Sequence

<220>
<223> Primers

<400> 1193
accagataca gcccgtcctt ccaaggccag gtcaccatct cagccgacaa gtccatcagc 60
accgcctacc tgc                                                          73

<210> 1194
<211> 73
<212> DNA
<213> Artificial Sequence

<220>
<223> Primers

<400> 1194
aatgattatg cagtatctgt gaaaagtcga ataaccatca acccagacac atccaagaac 60
cagttctccc tgc                                                          73

<210> 1195
<211> 73
<212> DNA
<213> Artificial Sequence

<220>
<223> Primers

<400> 1195
ccaacatatg cccagggctt cacaggacgg tttgtcttct ccatggacac ctctgccagc 60
acagcatacc tgc                                                          73

<210> 1196
<211> 71
<212> DNA
<213> Artificial Sequence

<220>

<223> Primers

<400> 1196
tctcgcacag taatacacgg ccgtgtcgtc agatctcagg ctcctcagct ccatgtaggc 60
tgtgctcgtg g 71

<210> 1197
<211> 71
<212> DNA
<213> Artificial Sequence

<220>
<223> Primers

<400> 1197
tctcgcacag taatacacgg ccgtgtcgtc agatctcagc ctgctcagct ccatgtaggc 60
tgtgctgatg g 71

<210> 1198
<211> 71
<212> DNA
<213> Artificial Sequence

<220>
<223> Primers

<400> 1198
tgttgcacag taatacacgg ccgtgtcctc agatctcagg ctgctcagct ccatgtaggc 60
tgtgtctgta g 71

<210> 1199
<211> 71
<212> DNA
<213> Artificial Sequence

<220>
<223> Primers

<400> 1199
tctcgcacag taatacacag ccatgtcctc agatctcagg ctgctcagct ccatgtaggc 60
tgtgctcgcg g 71

<210> 1200
<211> 71
<212> DNA
<213> Artificial Sequence

<220>
<223> Primers

<400> 1200
tcttgcacag taatacatgg ctgtgtcctc agatctcagg ctgctcagct ccatgtaggc 60
tgtgctcata g 71

<210> 1201
<211> 71
<212> DNA
<213> Artificial Sequence

<220>
<223> Primers

<400> 1201
tctcgcacag taatacacgg ccgtgtcctc agatctcagg ctgctcagct ccatgtagac 60
tgtgctcgtg g 71

<210> 1202
<211> 71

<212> DNA
<213> Artificial Sequence

<220>
<223> Primers

<400> 1202
tgccgcacag taatacacgg ccgtgtcctc ggatctcagg ctgctcagct ccatgtaggc 60
tgtgcttgtg g 71

<210> 1203
<211> 71
<212> DNA
<213> Artificial Sequence

<220>
<223> Primers

<400> 1203
tctcgcacag taatacacgg ccgtgtcctc agatctcagg ctgctcagct ccatgtaggc 60
tgtgctcgtg g 71

<210> 1204
<211> 71
<212> DNA
<213> Artificial Sequence

<220>
<223> Primers

<400> 1204
tctcgcacag taatacacgg ccgtgtcctc agatctcagg ctgctcagct ccatgtaggc 60
tgtgcttatg g 71

<210> 1205
<211> 71
<212> DNA
<213> Artificial Sequence

<220>
<223> Primers

<400> 1205
ccgtgcacag taatatgtgg ctgtgtccac agggtccatg ttggtcatgg taaggaccac 60
ctggcttttg g 71

<210> 1206
<211> 71
<212> DNA
<213> Artificial Sequence

<220>
<223> Primers

<400> 1206
gtgtgcacag taatatgtgg ctgtgtccac agggtccatg ttggtcattg taaggaccac 60
ctggttttg g 71

<210> 1207
<211> 71
<212> DNA
<213> Artificial Sequence

<220>
<223> Primers

<400> 1207
ccgtgcacaa taatacgtgg ctgtgtccac agggtccatg ttggtcattg taaggaccac 60

ctggttttg g                                                              71

<210> 1208
<211> 71
<212> DNA
<213> Artificial Sequence

<220>
<223> Primers

<400> 1208
tctcgcacag taatacacgg ccgtgtcctc ggctctcagg ctgttcattt gcagatacag 60
tgagttcttg g                                                            71

<210> 1209
<211> 71
<212> DNA
<213> Artificial Sequence

<220>
<223> Primers

<400> 1209
tcttgcacag taatacacag ccgtgtcccc ggctctcagg ctgttcattt gaagatacaa 60
ggagttcttg g                                                            71

<210> 1210
<211> 71
<212> DNA
<213> Artificial Sequence

<220>
<223> Primers

<400> 1210
tgtggtacag taatacacgg ctgtgtcctc ggttttcagg ctgttcattt gcagatacag 60
cgtgtttttt g                                                            71

<210> 1211
<211> 71
<212> DNA
<213> Artificial Sequence

<220>
<223> Primers

<400> 1211
tctcacacag taatacacag ccatgtcctc ggctctccgt ctgttctttt gcagatacag 60
ggagttcctg g                                                            71

<210> 1212
<211> 71
<212> DNA
<213> Artificial Sequence

<220>
<223> Primers

<400> 1212
tctcgcacag tgatacaagg ccgtgtcctc ggctctcaga ctgttcattt gcagatacag 60
ggagttcttg g                                                            71

<210> 1213
<211> 71
<212> DNA
<213> Artificial Sequence

<220>

<223> Primers

<400> 1213
tctcgcacag taatacacag ccgtgtcctc ggctctcagg ctgttcattt gcagatacag 60
tgagttcttg g 71

<210> 1214
<211> 71
<212> DNA
<213> Artificial Sequence

<220>
<223> Primers

<400> 1214
tttcgcacag taatatacgg ccgtgtcctc ggctctcagg ctgttcattt gcagatacag 60
cgtgttcttg g 71

<210> 1215
<211> 71
<212> DNA
<213> Artificial Sequence

<220>
<223> Primers

<400> 1215
tctcgcacag taatacacag ccgtgtcctc agctctcagg ctgttcattt gcagatacag 60
cgtgttcttg g 71

<210> 1216
<211> 71
<212> DNA
<213> Artificial Sequence

<220>
<223> Primers

<400> 1216
tctcgcacag taatacacag ccgtgtcctc ggctctcagg ctgttcattt gcagatacag 60
cgtgttcttg g 71

<210> 1217
<211> 71
<212> DNA
<213> Artificial Sequence

<220>
<223> Primers

<400> 1217
tctcacacag taatacacag ccgtgtcctc ggccctcagg ctattcgttt gcagatacag 60
ggtgttcctg g 71

<210> 1218
<211> 71
<212> DNA
<213> Artificial Sequence

<220>
<223> Primers

<400> 1218
tctggcacag taatacacgg ccgtgccctc agctctcagg ttgttcattt gaagatacag 60
cgtgttcttg g 71

<210> 1219
<211> 71

<212> DNA
<213> Artificial Sequence

<220>
<223> Primers

<400> 1219
   ttttgcacag taatacaagg cggtgtcctc agttctcaga ctgttcattt gcagatacag 60
   ggagtttttg c 71

<210> 1220
<211> 71
<212> DNA
<213> Artificial Sequence

<220>
<223> Primers

<400> 1220
tctcgcacag taatacacag ccgtgtcctc gtctctcagg ctgttcattt gcagatacag 60
tgagttcttg g 71

<210> 1221
<211> 71
<212> DNA
<213> Artificial Sequence

<220>
<223> Primers

<400> 1221
tctagtacag taatacacgg ctgtgtcctc ggttttcagg ctgttcattt gcagataggc 60
gatgcttttg g 71

<210> 1222
<211> 71
<212> DNA
<213> Artificial Sequence

<220>
<223> Primers

<400> 1222
tctcgcacag taatacacgg ccgtgtcctc ggctctcagg ctgttcattt gaagatacag 60
cgtgttcttg g 71

<210> 1223
<211> 71
<212> DNA
<213> Artificial Sequence

<220>
<223> Primers

<400> 1223
tctcgcacag taatacacag ccatgtcctc agctctcagg ctgcccattt gaagatacag 60
cgtgttcttg g 71

<210> 1224
<211> 71
<212> DNA
<213> Artificial Sequence

<220>
<223> Primers

<400> 1224
tctcgcacag taatacacag ccgtgtcctc agctctcagg ctgttcattt gaagatacag 60

cgtgttcttg g                                                          71

<210> 1225
<211> 71
<212> DNA
<213> Artificial Sequence

<220>
<223> Primers

<400> 1225
tctcgcacag taatacacag ccgtgtcctc ggctctcagg ctgttcattt gcagatacag 60
tgagttcttg g                                                          71

<210> 1226
<211> 71
<212> DNA
<213> Artificial Sequence

<220>
<223> Primers

<400> 1226
tctagcacag taatacacgg ccgtgtcctc ggttttcagg ctgttcattt gcagatacag 60
tgagttcttt g                                                          71

<210> 1227
<211> 71
<212> DNA
<213> Artificial Sequence

<220>
<223> Primers

<400> 1227
tctagtacag taatacacgg ccgtgtcctc ggttttcagg ctgttcattt gcagatacgc 60
cgtgttcttt g                                                          71

<210> 1228
<211> 71
<212> DNA
<213> Artificial Sequence

<220>
<223> Primers

<400> 1228
tcttgcacag taatacacag ccgtgtcctc ggctctcaga ctgttcattt gcagatacag 60
cgtgttcttg g                                                          71

<210> 1229
<211> 71
<212> DNA
<213> Artificial Sequence

<220>
<223> Primers

<400> 1229
ttttgcacag taatacaagg ccgtgtcctc agctctcaga ctgttcattt gcagatacag 60
ggagttcttg g                                                          71

<210> 1230
<211> 71
<212> DNA
<213> Artificial Sequence

<220>

<223> Primers

<400> 1230
tctcgcacag taatacacgg ccgtgtccac ggcggtcaca gagctcagct tcagggagaa 60
ctggttcttg g 71

<210> 1231
<211> 71
<212> DNA
<213> Artificial Sequence

<220>
<223> Primers

<400> 1231
tctcgcacag taatacacgg ccgtgtccgc ggcagtcaca gagctcagct tcagggagaa 60
ctggttctta g 71

<210> 1232
<211> 71
<212> DNA
<213> Artificial Sequence

<220>
<223> Primers

<400> 1232
tctcgcacag taatacacag ccgtgtccgc ggcggtcaca gagctcagct tcagggagaa 60
ctggttcttg g 71

<210> 1233
<211> 71
<212> DNA
<213> Artificial Sequence

<220>
<223> Primers

<400> 1233
tctcgcacag taatacacag ccgtgtctgc ggcggtcaca gagctcagct tcagggagaa 60
ctggttcttg g 71

<210> 1234
<211> 71
<212> DNA
<213> Artificial Sequence

<220>
<223> Primers

<400> 1234
tctcgcacag taatacacgg ccgtgtccgc ggcggtcaca gagctcagct tcagggagaa 60
ctggttcttg g 71

<210> 1235
<211> 71
<212> DNA
<213> Artificial Sequence

<220>
<223> Primers

<400> 1235
tctcgcacag taatacacgg ccgtgtccgc agcggtcaca gagctcagct tcagggagaa 60
ctggttcttg g 71

<210> 1236
<211> 71

<212> DNA
<213> Artificial Sequence

<220>
<223> Primers

<400> 1236
tctcgcacag taatacacgg ccgtgtccgc agcggtcaca gagctcagct tcagggagaa 60
ctggttcttg g 71

<210> 1237
<211> 71
<212> DNA
<213> Artificial Sequence

<220>
<223> Primers

<400> 1237
tctcgcacag taatacatgg cggtgtccga ggccttcagg ctgctccact gcaggtaggc 60
ggtgctgatg g 71

<210> 1238
<211> 71
<212> DNA
<213> Artificial Sequence

<220>
<223> Primers

<400> 1238
tcttgcacag taatacacag ccgtgtcctc gggagtcaca gagttcagct gcagggagaa 60
ctggttcttg g 71

<210> 1239
<211> 71
<212> DNA
<213> Artificial Sequence

<220>
<223> Primers

<400> 1239
tctcgcacag taatacatgg ccatgtcctc agcctttagg ctgctgatct gcaggtatgc 60
tgtgctggca g 71

<210> 1240
<211> 21
<212> DNA
<213> Artificial Sequence

<220>
<223> Primers

<400> 1240
gatgttgtga tgacwcagtc t 21

<210> 1241
<211> 21
<212> DNA
<213> Artificial Sequence

<220>
<223> Primers

<400> 1241
gacatccaga tgayccagtc t 21

<210> 1242
<211> 21
<212> DNA
<213> Artificial Sequence

<220>
<223> Primers

<400> 1242
gccatccagw tgacccagtc t                                                21

<210> 1243
<211> 21
<212> DNA
<213> Artificial Sequence

<220>
<223> Primers

<400> 1243
gaaatagtga tgaygcagtc t                                                21

<210> 1244
<211> 21
<212> DNA
<213> Artificial Sequence

<220>
<223> Primers

<400> 1244
gaaattgtgt tgacrcagtc t                                                21

<210> 1245
<211> 21
<212> DNA
<213> Artificial Sequence

<220>
<223> Primers

<400> 1245
gakattgtga tgacccagac t                                                21

<210> 1246
<211> 21
<212> DNA
<213> Artificial Sequence

<220>
<223> Primers

<400> 1246
gaaattgtrm tgacwcagtc t                                                21

<210> 1247
<211> 21
<212> DNA
<213> Artificial Sequence

<220>
<223> Primers

<400> 1247
gayatygtga tgacycagtc t                                                21

<210> 1248
<211> 21

<212> DNA
<213> Artificial Sequence

<220>
<223> Primers

<400> 1248
gaaacgacac tcacgcagtc t                                                21

<210> 1249
<211> 21
<212> DNA
<213> Artificial Sequence

<220>
<223> Primers

<400> 1249
gacatccagt tgacccagtc t                                                21

<210> 1250
<211> 21
<212> DNA
<213> Artificial Sequence

<220>
<223> Primers

<400> 1250
aacatccaga tgacccagtc t                                                21

<210> 1251
<211> 21
<212> DNA
<213> Artificial Sequence

<220>
<223> Primers

<400> 1251
gccatccgga tgacccagtc t                                                21

<210> 1252
<211> 21
<212> DNA
<213> Artificial Sequence

<220>
<223> Primers

<400> 1252
gtcatctgga tgacccagtc t                                                21

<210> 1253
<211> 21
<212> DNA
<213> Artificial Sequence

<220>
<223> Primers

<400> 1253
gcaggagatg gaggccggct s                                                21

<210> 1254
<211> 21
<212> DNA
<213> Artificial Sequence

<220>
<223> Primers

<400> 1254
gcaggagagg gtgrctcttt c                                                21

<210> 1255
<211> 21
<212> DNA
<213> Artificial Sequence

<220>
<223> Primers

<400> 1255
acaastgatg gtgactctgt c                                                21

<210> 1256
<211> 21
<212> DNA
<213> Artificial Sequence

<220>
<223> Primers

<400> 1256
gaaggagatg gaggccggct g                                                21

<210> 1257
<211> 21
<212> DNA
<213> Artificial Sequence

<220>
<223> Primers

<400> 1257
gcaggagatg gaggcctgct c                                                21

<210> 1258
<211> 21
<212> DNA
<213> Artificial Sequence

<220>
<223> Primers

<400> 1258
gcaggagatg ttgactttgt c                                                21

<210> 1259
<211> 21
<212> DNA
<213> Artificial Sequence

<220>
<223> Primers

<400> 1259
gcaggtgatg gtgactttct c                                                21

<210> 1260
<211> 21
<212> DNA
<213> Artificial Sequence

<220>

<223> Primers

<400> 1260
gcagttgatg gtggccctct c                                                21

<210> 1261
<211> 21
<212> DNA
<213> Artificial Sequence

<220>
<223> Primers

<400> 1261
gcaagtgatg gtgactctgt c                                                21

<210> 1262
<211> 21
<212> DNA
<213> Artificial Sequence

<220>
<223> Primers

<400> 1262
gcaaatgata ctgactctgt c                                                21

<210> 1263
<211> 21
<212> DNA
<213> Artificial Sequence

<220>
<223> Primers

<400> 1263
tggyttcagc agaggccagg c                                                21

<210> 1264
<211> 21
<212> DNA
<213> Artificial Sequence

<220>
<223> Primers

<400> 1264
tggtacctgc agaagccagg s                                                21

<210> 1265
<211> 21
<212> DNA
<213> Artificial Sequence

<220>
<223> Primers

<400> 1265
tggtatcrgc agaaaccagg g                                                21

<210> 1266
<211> 21
<212> DNA
<213> Artificial Sequence

<220>
<223> Primers

<400> 1266
tggtaccarc agaaaccagg a                                                21

<210> 1267
<211> 21
<212> DNA
<213> Artificial Sequence

<220>
<223> Primers

<400> 1267
tggtaccarc agaaacctgg c                                                21

<210> 1268
<211> 21
<212> DNA
<213> Artificial Sequence

<220>
<223> Primers

<400> 1268
tggtaycwgc agaaaccwgg g                                                21

<210> 1269
<211> 21
<212> DNA
<213> Artificial Sequence

<220>
<223> Primers

<400> 1269
tggtatcagc araaaccwgg s                                                21

<210> 1270
<211> 19
<212> DNA
<213> Artificial Sequence

<220>
<223> Primers

<400> 1270
tggtaycagc araaaccag                                                   19

<210> 1271
<211> 21
<212> DNA
<213> Artificial Sequence

<220>
<223> Primers

<400> 1271
tggtttctgc agaaagccag g                                                21

<210> 1272
<211> 21
<212> DNA
<213> Artificial Sequence

<220>
<223> Primers

<400> 1272
tggtttcagc agaaaccagg g                                                21

<210> 1273
<211> 21
<212> DNA
<213> Artificial Sequence

<220>
<223> Primers

<400> 1273
atagatcagg agctgtggag r                                                21

<210> 1274
<211> 21
<212> DNA
<213> Artificial Sequence

<220>
<223> Primers

<400> 1274
atagatcagg agcttaggrg c                                                21

<210> 1275
<211> 21
<212> DNA
<213> Artificial Sequence

<220>
<223> Primers

<400> 1275
atagatgagg agcctgggmg c                                                21

<210> 1276
<211> 21
<212> DNA
<213> Artificial Sequence

<220>
<223> Primers

<400> 1276
rtagatcagg mgcttagggg c                                                21

<210> 1277
<211> 21
<212> DNA
<213> Artificial Sequence

<220>
<223> Primers

<400> 1277
atagatcagg wgcttaggra c                                                21

<210> 1278
<211> 21
<212> DNA
<213> Artificial Sequence

<220>
<223> Primers

<400> 1278
atagatgaag agcttagggg c                                                21

<210> 1279

<211> 21
<212> DNA
<213> Artificial Sequence

<220>
<223> Primers

<400> 1279
ataaattagg agtcttggag g                                                21

<210> 1280
<211> 21
<212> DNA
<213> Artificial Sequence

<220>
<223> Primers

<400> 1280
gtaaatgagc agcttaggag g                                                21

<210> 1281
<211> 21
<212> DNA
<213> Artificial Sequence

<220>
<223> Primers

<400> 1281
atagatcagg agtgtggaga c                                                21

<210> 1282
<211> 21
<212> DNA
<213> Artificial Sequence

<220>
<223> Primers

<400> 1282
atagatcagg agctcagggg c                                                21

<210> 1283
<211> 21
<212> DNA
<213> Artificial Sequence

<220>
<223> Primers

<400> 1283
atagatcagg gacttagggg c                                                21

<210> 1284
<211> 21
<212> DNA
<213> Artificial Sequence

<220>
<223> Primers

<400> 1284
atagaggaag agcttagggg a                                                21

<210> 1285
<211> 21
<212> DNA

<213> Artificial Sequence

<220>
<223> Primers

<400> 1285
cttgatgagg agctttggag a                                                21

<210> 1286
<211> 21
<212> DNA
<213> Artificial Sequence

<220>
<223> Primers

<400> 1286
ataaattagg cgccttggag a                                                21

<210> 1287
<211> 21
<212> DNA
<213> Artificial Sequence

<220>
<223> Primers

<400> 1287
cttgatgagg agctttgggg c                                                21

<210> 1288
<211> 21
<212> DNA
<213> Artificial Sequence

<220>
<223> Primers

<400> 1288
ttgaataatg aaaatagcag c                                                21

<210> 1289
<211> 21
<212> DNA
<213> Artificial Sequence

<220>
<223> Primers

<400> 1289
ggggtcccag acagattcag y                                                21

<210> 1290
<211> 21
<212> DNA
<213> Artificial Sequence

<220>
<223> Primers

<400> 1290
ggggtcccat caaggttcag y                                                21

<210> 1291
<211> 21
<212> DNA
<213> Artificial Sequence

<220>
<223> Primers

<400> 1291
ggyatcccag ccaggttcag t                                                21

<210> 1292
<211> 21
<212> DNA
<213> Artificial Sequence

<220>
<223> Primers

<400> 1292
ggrgtcccwg acaggttcag t                                                21

<210> 1293
<211> 21
<212> DNA
<213> Artificial Sequence

<220>
<223> Primers

<400> 1293
agcatcccag ccaggttcag t                                                21

<210> 1294
<211> 21
<212> DNA
<213> Artificial Sequence

<220>
<223> Primers

<400> 1294
ggggtcccct cgaggttcag t                                                21

<210> 1295
<211> 21
<212> DNA
<213> Artificial Sequence

<220>
<223> Primers

<400> 1295
ggaatcccac ctcgattcag t                                                21

<210> 1296
<211> 21
<212> DNA
<213> Artificial Sequence

<220>
<223> Primers

<400> 1296
ggggtccctg accgattcag t                                                21

<210> 1297
<211> 21
<212> DNA
<213> Artificial Sequence

<220>
<223> Primers

<400> 1297
ggcatcccag acaggttcag t                                                21

<210> 1298
<211> 21
<212> DNA
<213> Artificial Sequence

<220>
<223> Primers

<400> 1298
ggggtctcat cgaggttcag t                                                21

<210> 1299
<211> 21
<212> DNA
<213> Artificial Sequence

<220>
<223> Primers

<400> 1299
ggagtgccag ataggttcag t                                                21

<210> 1300
<211> 21
<212> DNA
<213> Artificial Sequence

<220>
<223> Primers

<400> 1300
kcagtaataa accccaacat c                                                21

<210> 1301
<211> 21
<212> DNA
<213> Artificial Sequence

<220>
<223> Primers

<400> 1301
acagtaatay gttgcagcat c                                                21

<210> 1302
<211> 21
<212> DNA
<213> Artificial Sequence

<220>
<223> Primers

<400> 1302
acmgtaataa gttgcaacat c                                                21

<210> 1303
<211> 21
<212> DNA
<213> Artificial Sequence

<220>
<223> Primers

<400> 1303

rcagtaataa gttgcaaaat c                                                    21

<210> 1304
<211> 21
<212> DNA
<213> Artificial Sequence

<220>
<223> Primers

<400> 1304
acagtaataa rctgcaaaat c                                                    21

<210> 1305
<211> 21
<212> DNA
<213> Artificial Sequence

<220>
<223> Primers

<400> 1305
acartagtaa gttgcaaaat c                                                    21

<210> 1306
<211> 21
<212> DNA
<213> Artificial Sequence

<220>
<223> Primers

<400> 1306
gcagtaataa actccaamat c                                                    21

<210> 1307
<211> 21
<212> DNA
<213> Artificial Sequence

<220>
<223> Primers

<400> 1307
gcagtaataa accccgacat c                                                    21

<210> 1308
<211> 21
<212> DNA
<213> Artificial Sequence

<220>
<223> Primers

<400> 1308
acagaagtaa tatgcagcat c                                                    21

<210> 1309
<211> 21
<212> DNA
<213> Artificial Sequence

<220>
<223> Primers

<400> 1309
acagtaatat gttgcaatat c                                                    21

<210> 1310
<211> 21
<212> DNA
<213> Artificial Sequence

<220>
<223> Primers

<400> 1310
acagtaatac actgcaaaat c                                                21

<210> 1311
<211> 21
<212> DNA
<213> Artificial Sequence

<220>
<223> Primers

<400> 1311
acagtaataa actgccacat c                                                21

<210> 1312
<211> 21
<212> DNA
<213> Artificial Sequence

<220>
<223> Primers

<400> 1312
ttyggccarg ggaccaagst g                                                21

<210> 1313
<211> 21
<212> DNA
<213> Artificial Sequence

<220>
<223> Primers

<400> 1313
ttcggccaag ggacacgact g                                                21

<210> 1314
<211> 21
<212> DNA
<213> Artificial Sequence

<220>
<223> Primers

<400> 1314
ttcggccctg ggaccaaagt g                                                21

<210> 1315
<211> 21
<212> DNA
<213> Artificial Sequence

<220>
<223> Primers

<400> 1315
ttcggcggag ggaccaaggt g                                                21

<210> 1316
<211> 21

<212> DNA
<213> Artificial Sequence

<220>
<223> Primers

<400> 1316
tttgatytcc accttggtcc c                                                21

<210> 1317
<211> 21
<212> DNA
<213> Artificial Sequence

<220>
<223> Primers

<400> 1317
tttgatctcc agcttggtcc c                                                21

<210> 1318
<211> 21
<212> DNA
<213> Artificial Sequence

<220>
<223> Primers

<400> 1318
tttgatatcc actttggtcc c                                                21

<210> 1319
<211> 21
<212> DNA
<213> Artificial Sequence

<220>
<223> Primers

<400> 1319
tttaatctcc agtcgtgtcc c                                                21

<210> 1320
<211> 21
<212> DNA
<213> Artificial Sequence

<220>
<223> Primers

<400> 1320
caggtkcagc tggtgcagtc t                                                21

<210> 1321
<211> 21
<212> DNA
<213> Artificial Sequence

<220>
<223> Primers

<400> 1321
gaggtgcagc tgktggagtc t                                                21

<210> 1322
<211> 21
<212> DNA
<213> Artificial Sequence

<220>
<223> Primers

<400> 1322
cagstgcagc tgcaggagtc g                                                21

<210> 1323
<211> 21
<212> DNA
<213> Artificial Sequence

<220>
<223> Primers

<400> 1323
caggtcacct tgarggagtc t                                                21

<210> 1324
<211> 21
<212> DNA
<213> Artificial Sequence

<220>
<223> Primers

<400> 1324
caratgcagc tggtgcagtc t                                                21

<210> 1325
<211> 20
<212> DNA
<213> Artificial Sequence

<220>
<223> Primers

<400> 1325
gargtgcagc tggtgsagtc                                                  20

<210> 1326
<211> 21
<212> DNA
<213> Artificial Sequence

<220>
<223> Primers

<400> 1326
cagatcacct tgaaggagtc t                                                21

<210> 1327
<211> 21
<212> DNA
<213> Artificial Sequence

<220>
<223> Primers

<400> 1327
caggtscagc tggtrsagtc t                                                21

<210> 1328
<211> 21
<212> DNA
<213> Artificial Sequence

<220>

<223> Primers

<400> 1328
caggtacagc tgcagcagtc a                                                21

<210> 1329
<211> 21
<212> DNA
<213> Artificial Sequence

<220>
<223> Primers

<400> 1329
caggtgcagc tacagcagtg g                                                21

<210> 1330
<211> 21
<212> DNA
<213> Artificial Sequence

<220>
<223> Primers

<400> 1330
rgtgaaggtg tatccagaag c                                                21

<210> 1331
<211> 21
<212> DNA
<213> Artificial Sequence

<220>
<223> Primers

<400> 1331
gctgagtgag aacccagaga m                                                21

<210> 1332
<211> 21
<212> DNA
<213> Artificial Sequence

<220>
<223> Primers

<400> 1332
actgaargtg aatccagagg c                                                21

<210> 1333
<211> 21
<212> DNA
<213> Artificial Sequence

<220>
<223> Primers

<400> 1333
actgacggtg aayccagagg c                                                21

<210> 1334
<211> 21
<212> DNA
<213> Artificial Sequence

<220>
<223> Primers

<400> 1334
gctgayggag ccaccagaga c                                                21

<210> 1335
<211> 21
<212> DNA
<213> Artificial Sequence

<220>
<223> Primers

<400> 1335
rgtaaaggtg wawccagaag c                                                21

<210> 1336
<211> 21
<212> DNA
<213> Artificial Sequence

<220>
<223> Primers

<400> 1336
actraaggtg aayccagagg c                                                21

<210> 1337
<211> 21
<212> DNA
<213> Artificial Sequence

<220>
<223> Primers

<400> 1337
ggtraarctg tawccagaas c                                                21

<210> 1338
<211> 21
<212> DNA
<213> Artificial Sequence

<220>
<223> Primers

<400> 1338
aycaaaggtg aatccagarg c                                                21

<210> 1339
<211> 21
<212> DNA
<213> Artificial Sequence

<220>
<223> Primers

<400> 1339
rctraaggtg aatccagasg c                                                21

<210> 1340
<211> 21
<212> DNA
<213> Artificial Sequence

<220>
<223> Primers

<400> 1340
ggtgaaggtg tatccrgawg c                                                21

<210> 1341
<211> 21
<212> DNA
<213> Artificial Sequence

<220>
<223> Primers

<400> 1341
actgaaggac ccaccataga c                                                21

<210> 1342
<211> 21
<212> DNA
<213> Artificial Sequence

<220>
<223> Primers

<400> 1342
actgatggag ccaccagaga c                                                21

<210> 1343
<211> 21
<212> DNA
<213> Artificial Sequence

<220>
<223> Primers

<400> 1343
gctgatggag taaccagaga c                                                21

<210> 1344
<211> 21
<212> DNA
<213> Artificial Sequence

<220>
<223> Primers

<400> 1344
agtgagggtg tatccggaaa c                                                21

<210> 1345
<211> 21
<212> DNA
<213> Artificial Sequence

<220>
<223> Primers

<400> 1345
gctgaaggtg cctccagaag c                                                21

<210> 1346
<211> 21
<212> DNA
<213> Artificial Sequence

<220>
<223> Primers

<400> 1346
agagacactg tccccggaga t                                                21

<210> 1347

<211> 21
<212> DNA
<213> Artificial Sequence

<220>
<223> Primers

<400> 1347
tgggtgcgac aggcycctgg a                                                21

<210> 1348
<211> 21
<212> DNA
<213> Artificial Sequence

<220>
<223> Primers

<400> 1348
tgggtgcgmc aggcccccgg a                                                21

<210> 1349
<211> 21
<212> DNA
<213> Artificial Sequence

<220>
<223> Primers

<400> 1349
tggatccgtc agcccccagg r                                                21

<210> 1350
<211> 21
<212> DNA
<213> Artificial Sequence

<220>
<223> Primers

<400> 1350
tggrtccgcc aggctccagg g                                                21

<210> 1351
<211> 21
<212> DNA
<213> Artificial Sequence

<220>
<223> Primers

<400> 1351
tggatccgsc agcccccagg g                                                21

<210> 1352
<211> 21
<212> DNA
<213> Artificial Sequence

<220>
<223> Primers

<400> 1352
tgggtccgsc aagctccagg g                                                21

<210> 1353
<211> 21
<212> DNA

<213> Artificial Sequence

<220>
<223> Primers

<400> 1353
tgggtccrtc argctccrgg r                                                21

<210> 1354
<211> 21
<212> DNA
<213> Artificial Sequence

<220>
<223> Primers

<400> 1354
tgggtscgmc argcyacwgg a                                                21

<210> 1355
<211> 21
<212> DNA
<213> Artificial Sequence

<220>
<223> Primers

<400> 1355
tggktccgcc aggctccagg s                                                21

<210> 1356
<211> 21
<212> DNA
<213> Artificial Sequence

<220>
<223> Primers

<400> 1356
tggatcaggc agtccccatc g                                                21

<210> 1357
<211> 21
<212> DNA
<213> Artificial Sequence

<220>
<223> Primers

<400> 1357
tgggcccgca aggctccagg a                                                21

<210> 1358
<211> 21
<212> DNA
<213> Artificial Sequence

<220>
<223> Primers

<400> 1358
tggatccgcc agcacccagg g                                                21

<210> 1359
<211> 21
<212> DNA
<213> Artificial Sequence

<220>
<223> Primers

<400> 1359
tgggtccgcc aggcttccgg g                                                21

<210> 1360
<211> 21
<212> DNA
<213> Artificial Sequence

<220>
<223> Primers

<400> 1360
tgggtgcgcc agatgcccgg g                                                21

<210> 1361
<211> 21
<212> DNA
<213> Artificial Sequence

<220>
<223> Primers

<400> 1361
tgggtgcgac aggctcgtgg a                                                21

<210> 1362
<211> 21
<212> DNA
<213> Artificial Sequence

<220>
<223> Primers

<400> 1362
tggatccggc agcccgccgg g                                                21

<210> 1363
<211> 21
<212> DNA
<213> Artificial Sequence

<220>
<223> Primers

<400> 1363
tgggtgccac aggcccctgg a                                                21

<210> 1364
<211> 21
<212> DNA
<213> Artificial Sequence

<220>
<223> Primers

<400> 1364
tcccatccac tcaagccytt g                                                21

<210> 1365
<211> 20
<212> DNA
<213> Artificial Sequence

<220>
<223> Primers

<400> 1365
tcccatccac tcaagcsctt                                                    20

<210> 1366
<211> 21
<212> DNA
<213> Artificial Sequence

<220>
<223> Primers

<400> 1366
wgagacccac tccagcccct t                                                  21

<210> 1367
<211> 21
<212> DNA
<213> Artificial Sequence

<220>
<223> Primers

<400> 1367
cccaatccac tccagkccct t                                                  21

<210> 1368
<211> 21
<212> DNA
<213> Artificial Sequence

<220>
<223> Primers

<400> 1368
tgagacccac tccagrccct t                                                  21

<210> 1369
<211> 21
<212> DNA
<213> Artificial Sequence

<220>
<223> Primers

<400> 1369
gccaacccac tccagcccyt t                                                  21

<210> 1370
<211> 21
<212> DNA
<213> Artificial Sequence

<220>
<223> Primers

<400> 1370
kgccacccac tccagcccct t                                                  21

<210> 1371
<211> 20
<212> DNA
<213> Artificial Sequence

<220>
<223> Primers

<400> 1371

tcccagccac tcaaggcctc 20

<210> 1372
<211> 20
<212> DNA
<213> Artificial Sequence

<220>
<223> Primers

<400> 1372
ccccatccac tccaggcctt 20

<210> 1373
<211> 21
<212> DNA
<213> Artificial Sequence

<220>
<223> Primers

<400> 1373
tgaracccac wccagcccct t 21

<210> 1374
<211> 21
<212> DNA
<213> Artificial Sequence

<220>
<223> Primers

<400> 1374
mgakacccac tccagmccct t 21

<210> 1375
<211> 21
<212> DNA
<213> Artificial Sequence

<220>
<223> Primers

<400> 1375
yccmatccac tcmagcccyt t 21

<210> 1376
<211> 21
<212> DNA
<213> Artificial Sequence

<220>
<223> Primers

<400> 1376
tcctatccac tcaaggcgtt g 21

<210> 1377
<211> 21
<212> DNA
<213> Artificial Sequence

<220>
<223> Primers

<400> 1377
tgcaagccac tccagggcct t 21

<210> 1378
<211> 21
<212> DNA
<213> Artificial Sequence

<220>
<223> Primers

<400> 1378
tgaaacatat tccagtccct t                                                21

<210> 1379
<211> 21
<212> DNA
<213> Artificial Sequence

<220>
<223> Primers

<400> 1379
cgatacccac tccagcccct t                                                21

<210> 1380
<211> 21
<212> DNA
<213> Artificial Sequence

<220>
<223> Primers

<400> 1380
agagtcacca tgaccaggra c                                                21

<210> 1381
<211> 21
<212> DNA
<213> Artificial Sequence

<220>
<223> Primers

<400> 1381
aggctcacca tcwccaagga c                                                21

<210> 1382
<211> 21
<212> DNA
<213> Artificial Sequence

<220>
<223> Primers

<400> 1382
cgagtyacca tatcagtaga c                                                21

<210> 1383
<211> 21
<212> DNA
<213> Artificial Sequence

<220>
<223> Primers

<400> 1383
cgattcacca tctccagrga c                                                21

<210> 1384
<211> 20

<212> DNA
<213> Artificial Sequence

<220>
<223> Primers

<400> 1384
agattcacca tctcmagaga                                                    20

<210> 1385
<211> 20
<212> DNA
<213> Artificial Sequence

<220>
<223> Primers

<400> 1385
mggttcacca tctccagaga                                                    20

<210> 1386
<211> 20
<212> DNA
<213> Artificial Sequence

<220>
<223> Primers

<400> 1386
cgattcayca tctccagaga                                                    20

<210> 1387
<211> 21
<212> DNA
<213> Artificial Sequence

<220>
<223> Primers

<400> 1387
cgagtcacca trtcmgtaga c                                                  21

<210> 1388
<211> 21
<212> DNA
<213> Artificial Sequence

<220>
<223> Primers

<400> 1388
agrgtcacca tkaccaggga c                                                  21

<210> 1389
<211> 21
<212> DNA
<213> Artificial Sequence

<220>
<223> Primers

<400> 1389
caggtcacca tctcagccga c                                                  21

<210> 1390
<211> 21
<212> DNA
<213> Artificial Sequence

<220>
<223> Primers

<400> 1390
cgaataacca tcaacccaga c                                                21

<210> 1391
<211> 21
<212> DNA
<213> Artificial Sequence

<220>
<223> Primers

<400> 1391
cggtttgtct tctccatgga c                                                21

<210> 1392
<211> 21
<212> DNA
<213> Artificial Sequence

<220>
<223> Primers

<400> 1392
agagtcacca tgaccgagga c                                                21

<210> 1393
<211> 21
<212> DNA
<213> Artificial Sequence

<220>
<223> Primers

<400> 1393
agagtcacga ttaccgcgga c                                                21

<210> 1394
<211> 21
<212> DNA
<213> Artificial Sequence

<220>
<223> Primers

<400> 1394
agagtcacca tgaccacaga c                                                21

<210> 1395
<211> 21
<212> DNA
<213> Artificial Sequence

<220>
<223> Primers

<400> 1395
tctagyacag taatacacgg c                                                21

<210> 1396
<211> 21
<212> DNA
<213> Artificial Sequence

<220>

<223> Primers

<400> 1396
tctcgcacag taatacaygg c                                                21

<210> 1397
<211> 21
<212> DNA
<213> Artificial Sequence

<220>
<223> Primers

<400> 1397
tctygcacag taatacacag c                                                21

<210> 1398
<211> 21
<212> DNA
<213> Artificial Sequence

<220>
<223> Primers

<400> 1398
tgyygcacag taatacacgg c                                                21

<210> 1399
<211> 21
<212> DNA
<213> Artificial Sequence

<220>
<223> Primers

<400> 1399
ccgtgcacar taataygtgg c                                                21

<210> 1400
<211> 21
<212> DNA
<213> Artificial Sequence

<220>
<223> Primers

<400> 1400
tctggcacag taatacacgg c                                                21

<210> 1401
<211> 21
<212> DNA
<213> Artificial Sequence

<220>
<223> Primers

<400> 1401
tgtggtacag taatacacgg c                                                21

<210> 1402
<211> 21
<212> DNA
<213> Artificial Sequence

<220>
<223> Primers

<400> 1402
tctcgcacag tgatacaagg c                                                21

<210> 1403
<211> 21
<212> DNA
<213> Artificial Sequence

<220>
<223> Primers

<400> 1403
ttttgcacag taatacaagg c                                                21

<210> 1404
<211> 21
<212> DNA
<213> Artificial Sequence

<220>
<223> Primers

<400> 1404
tcttgcacag taatacatgg c                                                21

<210> 1405
<211> 21
<212> DNA
<213> Artificial Sequence

<220>
<223> Primers

<400> 1405
gtgtgcacag taatatgtgg c                                                21

<210> 1406
<211> 21
<212> DNA
<213> Artificial Sequence

<220>
<223> Primers

<400> 1406
tttcgcacag taatatacgg c                                                21

<210> 1407
<211> 21
<212> DNA
<213> Artificial Sequence

<220>
<223> Primers

<400> 1407
tctcacacag taatacacag c                                                21

<210> 1408
<211> 21
<212> DNA
<213> Artificial Sequence

<220>
<223> Primers

<400> 1408
caggtkcagc tggtgcagtc t                                                21

<210> 1409
<211> 21
<212> DNA
<213> Artificial Sequence

<220>
<223> Primers

<400> 1409
gaggtgcagc tgktggagtc t                                                21

<210> 1410
<211> 21
<212> DNA
<213> Artificial Sequence

<220>
<223> Primers

<400> 1410
cagstgcagc tgcaggagtc g                                                21

<210> 1411
<211> 21
<212> DNA
<213> Artificial Sequence

<220>
<223> Primers

<400> 1411
caggtcacct tgarggagtc t                                                21

<210> 1412
<211> 21
<212> DNA
<213> Artificial Sequence

<220>
<223> Primers

<400> 1412
caratgcagc tggtgcagtc t                                                21

<210> 1413
<211> 20
<212> DNA
<213> Artificial Sequence

<220>
<223> Primers

<400> 1413
gargtgcagc tggtgsagtc                                                  20

<210> 1414
<211> 21
<212> DNA
<213> Artificial Sequence

<220>
<223> Primers

<400> 1414
cagatcacct tgaaggagtc t                                                21

<210> 1415

<211> 21
<212> DNA
<213> Artificial Sequence

<220>
<223> Primers

<400> 1415
caggtscagc tggtrsagtc t                                                21

<210> 1416
<211> 21
<212> DNA
<213> Artificial Sequence

<220>
<223> Primers

<400> 1416
caggtacagc tgcagcagtc a                                                21

<210> 1417
<211> 21
<212> DNA
<213> Artificial Sequence

<220>
<223> Primers

<400> 1417
caggtgcagc tacagcagtg g                                                21

<210> 1418
<211> 21
<212> DNA
<213> Artificial Sequence

<220>
<223> Primers

<400> 1418
rgaarccttg caggagacct t                                                21

<210> 1419
<211> 21
<212> DNA
<213> Artificial Sequence

<220>
<223> Primers

<400> 1419
rgaagccttg caggaaacct t                                                21

<210> 1420
<211> 21
<212> DNA
<213> Artificial Sequence

<220>
<223> Primers

<400> 1420
agatgccttg caggaaacct t                                                21

<210> 1421
<211> 21
<212> DNA

<213> Artificial Sequence

<220>
<223> Primers

<400> 1421
agagamggtg caggtcagcg t                                                21

<210> 1422
<211> 21
<212> DNA
<213> Artificial Sequence

<220>
<223> Primers

<400> 1422
agasgctgca caggagagtc t                                                21

<210> 1423
<211> 21
<212> DNA
<213> Artificial Sequence

<220>
<223> Primers

<400> 1423
agagacagtr caggtgaggg a                                                21

<210> 1424
<211> 21
<212> DNA
<213> Artificial Sequence

<220>
<223> Primers

<400> 1424
akagacagcg caggtgaggg a                                                21

<210> 1425
<211> 21
<212> DNA
<213> Artificial Sequence

<220>
<223> Primers

<400> 1425
agagaaggtg caggtcagtg t                                                21

<210> 1426
<211> 21
<212> DNA
<213> Artificial Sequence

<220>
<223> Primers

<400> 1426
agaagctgta caggagagtc t                                                21

<210> 1427
<211> 21
<212> DNA
<213> Artificial Sequence

<220>
<223> Primers

<400> 1427
agaggctgca caggagagtt t                                                21

<210> 1428
<211> 21
<212> DNA
<213> Artificial Sequence

<220>
<223> Primers

<400> 1428
agaaccctta caggagatct t                                                21

<210> 1429
<211> 21
<212> DNA
<213> Artificial Sequence

<220>
<223> Primers

<400> 1429
ggagatggca caggtgagtg a                                                21

<210> 1430
<211> 21
<212> DNA
<213> Artificial Sequence

<220>
<223> Primers

<400> 1430
tatggyatsa gctgggtgcg m                                                21

<210> 1431
<211> 21
<212> DNA
<213> Artificial Sequence

<220>
<223> Primers

<400> 1431
atgkgtgtga gctggatccg t                                                21

<210> 1432
<211> 21
<212> DNA
<213> Artificial Sequence

<220>
<223> Primers

<400> 1432
tactactggr gctggatccg s                                                21

<210> 1433
<211> 21
<212> DNA
<213> Artificial Sequence

<220>
<223> Primers

<400> 1433
tatgcyatsa gctgggtscg m                                                21

<210> 1434
<211> 21
<212> DNA
<213> Artificial Sequence

<220>
<223> Primers

<400> 1434
tctgctatgc astgggtscg m                                                21

<210> 1435
<211> 21
<212> DNA
<213> Artificial Sequence

<220>
<223> Primers

<400> 1435
tatgcyatgc aytgggtscg s                                                21

<210> 1436
<211> 21
<212> DNA
<213> Artificial Sequence

<220>
<223> Primers

<400> 1436
cgctacctgc actgggtgcg a                                                21

<210> 1437
<211> 21
<212> DNA
<213> Artificial Sequence

<220>
<223> Primers

<400> 1437
ttatccatgc actgggtgcg a                                                21

<210> 1438
<211> 21
<212> DNA
<213> Artificial Sequence

<220>
<223> Primers

<400> 1438
gcctggatga gctgggtccg c                                                21

<210> 1439
<211> 21
<212> DNA
<213> Artificial Sequence

<220>
<223> Primers

<400> 1439

gctgcttgga actggatcag g                                                21

<210> 1440
<211> 21
<212> DNA
<213> Artificial Sequence

<220>
<223> Primers

<400> 1440
aatgagatga gctggatccg c                                                21

<210> 1441
<211> 21
<212> DNA
<213> Artificial Sequence

<220>
<223> Primers

<400> 1441
aactacatga gctgggtccg c                                                21

<210> 1442
<211> 21
<212> DNA
<213> Artificial Sequence

<220>
<223> Primers

<400> 1442
aactggtggg gctggatccg g                                                21

<210> 1443
<211> 21
<212> DNA
<213> Artificial Sequence

<220>
<223> Primers

<400> 1443
gtgggtgtgg gctggatccg t                                                21

<210> 1444
<211> 21
<212> DNA
<213> Artificial Sequence

<220>
<223> Primers

<400> 1444
cactacatgg actgggtccg c                                                21

<210> 1445
<211> 21
<212> DNA
<213> Artificial Sequence

<220>
<223> Primers

<400> 1445
agtgacatga actgggcccg c                                                21

<210> 1446
<211> 21
<212> DNA
<213> Artificial Sequence

<220>
<223> Primers

<400> 1446
agtgacatga actgggtcca t                                                21

<210> 1447
<211> 21
<212> DNA
<213> Artificial Sequence

<220>
<223> Primers

<400> 1447
tataccatgc actgggtccg t                                                21

<210> 1448
<211> 21
<212> DNA
<213> Artificial Sequence

<220>
<223> Primers

<400> 1448
tatgctatgc actgggtccg c                                                21

<210> 1449
<211> 21
<212> DNA
<213> Artificial Sequence

<220>
<223> Primers

<400> 1449
tatgctatga gctggttccg c                                                21

<210> 1450
<211> 21
<212> DNA
<213> Artificial Sequence

<220>
<223> Primers

<400> 1450
tatagcatga actgggtccg c                                                21

<210> 1451
<211> 21
<212> DNA
<213> Artificial Sequence

<220>
<223> Primers

<400> 1451
tatggcatgc actgggtccg c                                                21

<210> 1452
<211> 21

<212> DNA
<213> Artificial Sequence

<220>
<223> Primers

<400> 1452
tattggatga gctgggtccg c                                                21

<210> 1453
<211> 21
<212> DNA
<213> Artificial Sequence

<220>
<223> Primers

<400> 1453
tacgacatgc actgggtccg c                                                21

<210> 1454
<211> 21
<212> DNA
<213> Artificial Sequence

<220>
<223> Primers

<400> 1454
tactacatga gctggatccg c                                                21

<210> 1455
<211> 21
<212> DNA
<213> Artificial Sequence

<220>
<223> Primers

<400> 1455
tactggatgc actgggtccg c                                                21

<210> 1456
<211> 21
<212> DNA
<213> Artificial Sequence

<220>
<223> Primers

<400> 1456
tactggatcg gctgggtgcg c                                                21

<210> 1457
<211> 21
<212> DNA
<213> Artificial Sequence

<220>
<223> Primers

<400> 1457
tactatatgc actgggtgcg a                                                21

<210> 1458
<211> 21
<212> DNA
<213> Artificial Sequence

<220>
<223> Primers

<400> 1458
tatgatatca actgggtgcg a                                                21

<210> 1459
<211> 21
<212> DNA
<213> Artificial Sequence

<220>
<223> Primers

<400> 1459
tatggtatga attgggtgcc a                                                21

<210> 1460
<211> 21
<212> DNA
<213> Artificial Sequence

<220>
<223> Primers

<400> 1460
aatascwgag acccactcca g                                                21

<210> 1461
<211> 21
<212> DNA
<213> Artificial Sequence

<220>
<223> Primers

<400> 1461
aataaswgag acccactcca g                                                21

<210> 1462
<211> 21
<212> DNA
<213> Artificial Sequence

<220>
<223> Primers

<400> 1462
gmtccatccc atccactcaa g                                                21

<210> 1463
<211> 21
<212> DNA
<213> Artificial Sequence

<220>
<223> Primers

<400> 1463
gatackccca atccactcca g                                                21

<210> 1464
<211> 21
<212> DNA
<213> Artificial Sequence

<220>

<223> Primers

<400> 1464
gatrtaccca atccactcca g                                                21

<210> 1465
<211> 21
<212> DNA
<213> Artificial Sequence

<220>
<223> Primers

<400> 1465
aatgwgtgca agccactcca g                                                21

<210> 1466
<211> 21
<212> DNA
<213> Artificial Sequence

<220>
<223> Primers

<400> 1466
aayaccygak acccactcca g                                                21

<210> 1467
<211> 21
<212> DNA
<213> Artificial Sequence

<220>
<223> Primers

<400> 1467
aatgkatgar acccactcca g                                                21

<210> 1468
<211> 21
<212> DNA
<213> Artificial Sequence

<220>
<223> Primers

<400> 1468
artacggcca acccactcca g                                                21

<210> 1469
<211> 21
<212> DNA
<213> Artificial Sequence

<220>
<223> Primers

<400> 1469
aaaacctccc atccactcaa g                                                21

<210> 1470
<211> 21
<212> DNA
<213> Artificial Sequence

<220>
<223> Primers

<400> 1470
gattattccc atccactcaa g                                                21

<210> 1471
<211> 21
<212> DNA
<213> Artificial Sequence

<220>
<223> Primers

<400> 1471
gatccatcct atccactcaa g                                                21

<210> 1472
<211> 21
<212> DNA
<213> Artificial Sequence

<220>
<223> Primers

<400> 1472
gaaccatccc atccactcaa g                                                21

<210> 1473
<211> 21
<212> DNA
<213> Artificial Sequence

<220>
<223> Primers

<400> 1473
gatccctccc atccactcaa g                                                21

<210> 1474
<211> 21
<212> DNA
<213> Artificial Sequence

<220>
<223> Primers

<400> 1474
catccatccc atccactcaa g                                                21

<210> 1475
<211> 21
<212> DNA
<213> Artificial Sequence

<220>
<223> Primers

<400> 1475
tgtccttccc agccactcaa g                                                21

<210> 1476
<211> 21
<212> DNA
<213> Artificial Sequence

<220>
<223> Primers

<400> 1476
aatacgtgag acccacacca g                                                21

<210> 1477
<211> 21
<212> DNA
<213> Artificial Sequence

<220>
<223> Primers

<400> 1477
aatagctgaa acatattcca g                                                21

<210> 1478
<211> 21
<212> DNA
<213> Artificial Sequence

<220>
<223> Primers

<400> 1478
gatttcccca atccactcca g                                                21

<210> 1479
<211> 21
<212> DNA
<213> Artificial Sequence

<220>
<223> Primers

<400> 1479
gatgatcccc atccactcca g                                                21

<210> 1480
<211> 21
<212> DNA
<213> Artificial Sequence

<220>
<223> Primers

<400> 1480
tataactgcc acccactcca g                                                21

<210> 1481
<211> 21
<212> DNA
<213> Artificial Sequence

<220>
<223> Primers

<400> 1481
aatgaaacct acccactcca g                                                21

<210> 1482
<211> 21
<212> DNA
<213> Artificial Sequence

<220>
<223> Primers

<400> 1482
tatgttggcc acccactcca g                                                21

<210> 1483

<211> 21
<212> DNA
<213> Artificial Sequence

<220>
<223> Primers

<400> 1483
accaactaca acccstccct c                                                21

<210> 1484
<211> 21
<212> DNA
<213> Artificial Sequence

<220>
<223> Primers

<400> 1484
atatactacg cagactcwgt g                                                21

<210> 1485
<211> 21
<212> DNA
<213> Artificial Sequence

<220>
<223> Primers

<400> 1485
acatactayg cagactcygt g                                                21

<210> 1486
<211> 21
<212> DNA
<213> Artificial Sequence

<220>
<223> Primers

<400> 1486
acmaactacg cacagaartt c                                                21

<210> 1487
<211> 20
<212> DNA
<213> Artificial Sequence

<220>
<223> Primers

<400> 1487
acaaactatg cacagaagyt                                                  20

<210> 1488
<211> 21
<212> DNA
<213> Artificial Sequence

<220>
<223> Primers

<400> 1488
acargctayg cacagaagtt c                                                21

<210> 1489
<211> 21
<212> DNA

<213> Artificial Sequence

<220>
<223> Primers

<400> 1489
ayaggytatg crgactctgt g                                                21

<210> 1490
<211> 21
<212> DNA
<213> Artificial Sequence

<220>
<223> Primers

<400> 1490
aaatmctaca gcacatctct g                                                21

<210> 1491
<211> 21
<212> DNA
<213> Artificial Sequence

<220>
<223> Primers

<400> 1491
aaatactatg tggactctgt g                                                21

<210> 1492
<211> 21
<212> DNA
<213> Artificial Sequence

<220>
<223> Primers

<400> 1492
ccaacatatg cccagggctt c                                                21

<210> 1493
<211> 21
<212> DNA
<213> Artificial Sequence

<220>
<223> Primers

<400> 1493
gcaaactacg cacagaagtt c                                                21

<210> 1494
<211> 21
<212> DNA
<213> Artificial Sequence

<220>
<223> Primers

<400> 1494
aaatactatg cagactccgt g                                                21

<210> 1495
<211> 21
<212> DNA
<213> Artificial Sequence

<220>
<223> Primers

<400> 1495
aagcgctaca gcccatctct g                                                21

<210> 1496
<211> 21
<212> DNA
<213> Artificial Sequence

<220>
<223> Primers

<400> 1496
aatgattatg cagtatctgt g                                                21

<210> 1497
<211> 21
<212> DNA
<213> Artificial Sequence

<220>
<223> Primers

<400> 1497
accagataca gcccgtcctt c                                                21

<210> 1498
<211> 21
<212> DNA
<213> Artificial Sequence

<220>
<223> Primers

<400> 1498
acagaatacg ccgcgtctgt g                                                21

<210> 1499
<211> 21
<212> DNA
<213> Artificial Sequence

<220>
<223> Primers

<400> 1499
acgcactatg cagactctgt g                                                21

<210> 1500
<211> 21
<212> DNA
<213> Artificial Sequence

<220>
<223> Primers

<400> 1500
acgcactatg tggactccgt g                                                21

<210> 1501
<211> 21
<212> DNA
<213> Artificial Sequence

<220>
<223> Primers

<400> 1501
acaatctacg cacagaagtt c                                                21

<210> 1502
<211> 21
<212> DNA
<213> Artificial Sequence

<220>
<223> Primers

<400> 1502
acaaaatatt cacaggagtt c                                                21

<210> 1503
<211> 21
<212> DNA
<213> Artificial Sequence

<220>
<223> Primers

<400> 1503
acatactacg cagactccag g                                                21

<210> 1504
<211> 21
<212> DNA
<213> Artificial Sequence

<220>
<223> Primers

<400> 1504
acaagctacg cggactccgt g                                                21

<210> 1505
<211> 21
<212> DNA
<213> Artificial Sequence

<220>
<223> Primers

<400> 1505
acatattatg cagactctgt g                                                21

<210> 1506
<211> 21
<212> DNA
<213> Artificial Sequence

<220>
<223> Primers

<400> 1506
acagactacg ctgcacccgt g                                                21

<210> 1507
<211> 21
<212> DNA
<213> Artificial Sequence

<220>
<223> Primers

<400> 1507

acagcatatg ctgcgtcggt g 21

<210> 1508
<211> 21
<212> DNA
<213> Artificial Sequence

<220>
<223> Primers

<400> 1508
acatactatc caggctccgt g 21

<210> 1509
<211> 21
<212> DNA
<213> Artificial Sequence

<220>
<223> Primers

<400> 1509
acctactaca acccgtccct c 21

<210> 1510
<211> 21
<212> DNA
<213> Artificial Sequence

<220>
<223> Primers

<400> 1510
tstygcacag taatacacgg c 21

<210> 1511
<211> 21
<212> DNA
<213> Artificial Sequence

<220>
<223> Primers

<400> 1511
tctygcacag taatacatgg c 21

<210> 1512
<211> 21
<212> DNA
<213> Artificial Sequence

<220>
<223> Primers

<400> 1512
tctagyacag taatacacgg c 21

<210> 1513
<211> 21
<212> DNA
<213> Artificial Sequence

<220>
<223> Primers

<400> 1513
ccgtgcacar taataygtgg c 21

<210> 1514
<211> 21
<212> DNA
<213> Artificial Sequence

<220>
<223> Primers

<400> 1514
tctygcacag taatacacag c                                                21

<210> 1515
<211> 21
<212> DNA
<213> Artificial Sequence

<220>
<223> Primers

<400> 1515
gtgtgcacag taatatgtgg c                                                21

<210> 1516
<211> 21
<212> DNA
<213> Artificial Sequence

<220>
<223> Primers

<400> 1516
tgccgcacag taatacacgg c                                                21

<210> 1517
<211> 21
<212> DNA
<213> Artificial Sequence

<220>
<223> Primers

<400> 1517
tgtggtacag taatacacgg c                                                21

<210> 1518
<211> 21
<212> DNA
<213> Artificial Sequence

<220>
<223> Primers

<400> 1518
tctcacacag taatacacag c                                                21

<210> 1519
<211> 21
<212> DNA
<213> Artificial Sequence

<220>
<223> Primers

<400> 1519
tctcgcacag tgatacaagg c                                                21

<210> 1520
<211> 21

<212> DNA
<213> Artificial Sequence

<220>
<223> Primers

<400> 1520
tttcgcacag taatatacgg c                                                21

<210> 1521
<211> 21
<212> DNA
<213> Artificial Sequence

<220>
<223> Primers

<400> 1521
tctggcacag taatacacgg c                                                21

<210> 1522
<211> 21
<212> DNA
<213> Artificial Sequence

<220>
<223> Primers

<400> 1522
ttttgcacag taatacaagg c                                                21

<210> 1523
<211> 21
<212> DNA
<213> Artificial Sequence

<220>
<223> Primers

<400> 1523
tggggccarg gmaccctggt c                                                21

<210> 1524
<211> 21
<212> DNA
<213> Artificial Sequence

<220>
<223> Primers

<400> 1524
tggggscaag ggacmayggt c                                                21

<210> 1525
<211> 21
<212> DNA
<213> Artificial Sequence

<220>
<223> Primers

<400> 1525
tggggccgtg gcaccctggt c                                                21

<210> 1526
<211> 21
<212> DNA
<213> Artificial Sequence

<220>
<223> Primers

<400> 1526
tgaggagacr gtgaccaggg t                                                21

<210> 1527
<211> 21
<212> DNA
<213> Artificial Sequence

<220>
<223> Primers

<400> 1527
tgargagacg gtgaccrtkg t                                                21

<210> 1528
<211> 21
<212> DNA
<213> Artificial Sequence

<220>
<223> Primers

<400> 1528
tgaggagacg gtgaccaggg t                                                21

<210> 1529
<211> 21
<212> DNA
<213> Artificial Sequence

<220>
<223> Primers

<400> 1529
gatgttgtga tgacwcagtc t                                                21

<210> 1530
<211> 21
<212> DNA
<213> Artificial Sequence

<220>
<223> Primers

<400> 1530
gacatccaga tgayccagtc t                                                21

<210> 1531
<211> 21
<212> DNA
<213> Artificial Sequence

<220>
<223> Primers

<400> 1531
gccatccagw tgacccagtc t                                                21

<210> 1532
<211> 21
<212> DNA
<213> Artificial Sequence

<220>

<223> Primers

<400> 1532
gaaatagtga tgaygcagtc t                                                21

<210> 1533
<211> 21
<212> DNA
<213> Artificial Sequence

<220>
<223> Primers

<400> 1533
gaaattgtgt tgacrcagtc t                                                21

<210> 1534
<211> 21
<212> DNA
<213> Artificial Sequence

<220>
<223> Primers

<400> 1534
gakattgtga tgacccagac t                                                21

<210> 1535
<211> 21
<212> DNA
<213> Artificial Sequence

<220>
<223> Primers

<400> 1535
gaaattgtrm tgacwcagtc t                                                21

<210> 1536
<211> 21
<212> DNA
<213> Artificial Sequence

<220>
<223> Primers

<400> 1536
gayatygtga tgacycagtc t                                                21

<210> 1537
<211> 21
<212> DNA
<213> Artificial Sequence

<220>
<223> Primers

<400> 1537
gaaacgacac tcacgcagtc t                                                21

<210> 1538
<211> 21
<212> DNA
<213> Artificial Sequence

<220>
<223> Primers

<400> 1538
gacatccagt tgacccagtc t                                                21

<210> 1539
<211> 21
<212> DNA
<213> Artificial Sequence

<220>
<223> Primers

<400> 1539
aacatccaga tgacccagtc t                                                21

<210> 1540
<211> 21
<212> DNA
<213> Artificial Sequence

<220>
<223> Primers

<400> 1540
gccatccgga tgacccagtc t                                                21

<210> 1541
<211> 21
<212> DNA
<213> Artificial Sequence

<220>
<223> Primers

<400> 1541
gtcatctgga tgacccagtc t                                                21

<210> 1542
<211> 21
<212> DNA
<213> Artificial Sequence

<220>
<223> Primers

<400> 1542
tttgatytcc accttggtcc c                                                21

<210> 1543
<211> 21
<212> DNA
<213> Artificial Sequence

<220>
<223> Primers

<400> 1543
tttgatctcc agcttggtcc c                                                21

<210> 1544
<211> 21
<212> DNA
<213> Artificial Sequence

<220>
<223> Primers

<400> 1544
tttgatatcc actttggtcc c                                                21

<210> 1545
<211> 21
<212> DNA
<213> Artificial Sequence

<220>
<223> Primers

<400> 1545
tttaatctcc agtcgtgtcc c                                                21

<210> 1546
<211> 21
<212> DNA
<213> Artificial Sequence

<220>
<223> Primers

<400> 1546
caggtkcagc tggtgcagtc t                                                21

<210> 1547
<211> 21
<212> DNA
<213> Artificial Sequence

<220>
<223> Primers

<400> 1547
gaggtgcagc tgktggagtc t                                                21

<210> 1548
<211> 21
<212> DNA
<213> Artificial Sequence

<220>
<223> Primers

<400> 1548
cagstgcagc tgcaggagtc g                                                21

<210> 1549
<211> 21
<212> DNA
<213> Artificial Sequence

<220>
<223> Primers

<400> 1549
caggtcacct tgarggagtc t                                                21

<210> 1550
<211> 21
<212> DNA
<213> Artificial Sequence

<220>
<223> Primers

<400> 1550
caratgcagc tggtgcagtc t                                                21

<210> 1551

<211> 20
<212> DNA
<213> Artificial Sequence

<220>
<223> Primers

<400> 1551
gargtgcagc tggtgsagtc                                                    20

<210> 1552
<211> 21
<212> DNA
<213> Artificial Sequence

<220>
<223> Primers

<400> 1552
cagatcacct tgaaggagtc t                                                  21

<210> 1553
<211> 21
<212> DNA
<213> Artificial Sequence

<220>
<223> Primers

<400> 1553
caggtscagc tggtrsagtc t                                                  21

<210> 1554
<211> 21
<212> DNA
<213> Artificial Sequence

<220>
<223> Primers

<400> 1554
caggtacagc tgcagcagtc a                                                  21

<210> 1555
<211> 21
<212> DNA
<213> Artificial Sequence

<220>
<223> Primers

<400> 1555
caggtgcagc tacagcagtg g                                                  21

<210> 1556
<211> 21
<212> DNA
<213> Artificial Sequence

<220>
<223> Primers

<400> 1556
tgaggagacr gtgaccaggg t                                                  21

<210> 1557
<211> 21
<212> DNA

<213> Artificial Sequence

<220>
<223> Primers

<400> 1557
tgargagacg gtgaccrtkg t                                                21

<210> 1558
<211> 21
<212> DNA
<213> Artificial Sequence

<220>
<223> Primers

<400> 1558
tgaggagacg gtgaccaggg t                                                21

<210> 1559
<211> 21
<212> DNA
<213> Artificial Sequence

<220>
<223> Primers

<400> 1559
gatgttgtga tgacwcagtc t                                                21

<210> 1560
<211> 21
<212> DNA
<213> Artificial Sequence

<220>
<223> Primers

<400> 1560
gacatccaga tgayccagtc t                                                21

<210> 1561
<211> 21
<212> DNA
<213> Artificial Sequence

<220>
<223> Primers

<400> 1561
gccatccagw tgacccagtc t                                                21

<210> 1562
<211> 21
<212> DNA
<213> Artificial Sequence

<220>
<223> Primers

<400> 1562
gaaatagtga tgaygcagtc t                                                21

<210> 1563
<211> 21
<212> DNA
<213> Artificial Sequence

<220>
<223> Primers

<400> 1563
gaaattgtgt tgacrcagtc t                                                21

<210> 1564
<211> 21
<212> DNA
<213> Artificial Sequence

<220>
<223> Primers

<400> 1564
gakattgtga tgacccagac t                                                21

<210> 1565
<211> 21
<212> DNA
<213> Artificial Sequence

<220>
<223> Primers

<400> 1565
gaaattgtrm tgacwcagtc t                                                21

<210> 1566
<211> 21
<212> DNA
<213> Artificial Sequence

<220>
<223> Primers

<400> 1566
gayatygtga tgacycagtc t                                                21

<210> 1567
<211> 21
<212> DNA
<213> Artificial Sequence

<220>
<223> Primers

<400> 1567
gaaacgacac tcacgcagtc t                                                21

<210> 1568
<211> 21
<212> DNA
<213> Artificial Sequence

<220>
<223> Primers

<400> 1568
gacatccagt tgacccagtc t                                                21

<210> 1569
<211> 21
<212> DNA
<213> Artificial Sequence

<220>
<223> Primers

<400> 1569
aacatccaga tgacccagtc t                                                21

<210> 1570
<211> 21
<212> DNA
<213> Artificial Sequence

<220>
<223> Primers

<400> 1570
gccatccgga tgacccagtc t                                                21

<210> 1571
<211> 21
<212> DNA
<213> Artificial Sequence

<220>
<223> Primers

<400> 1571
gtcatctgga tgacccagtc t                                                21

<210> 1572
<211> 21
<212> DNA
<213> Artificial Sequence

<220>
<223> Primers

<400> 1572
tttgatytcc accttggtcc c                                                21

<210> 1573
<211> 21
<212> DNA
<213> Artificial Sequence

<220>
<223> Primers

<400> 1573
tttgatctcc agcttggtcc c                                                21

<210> 1574
<211> 21
<212> DNA
<213> Artificial Sequence

<220>
<223> Primers

<400> 1574
tttgatatcc actttggtcc c                                                21

<210> 1575
<211> 21
<212> DNA
<213> Artificial Sequence

<220>
<223> Primers

<400> 1575

tttaatctcc agtcgtgtcc c                                                21

<210> 1576
<211> 21
<212> DNA
<213> Artificial Sequence

<220>
<223> Primers

<400> 1576
caggtkcagc tggtgcagtc t                                                21

<210> 1577
<211> 21
<212> DNA
<213> Artificial Sequence

<220>
<223> Primers

<400> 1577
gaggtgcagc tgktggagtc t                                                21

<210> 1578
<211> 21
<212> DNA
<213> Artificial Sequence

<220>
<223> Primers

<400> 1578
cagstgcagc tgcaggagtc g                                                21

<210> 1579
<211> 21
<212> DNA
<213> Artificial Sequence

<220>
<223> Primers

<400> 1579
caggtcacct tgarggagtc t                                                21

<210> 1580
<211> 21
<212> DNA
<213> Artificial Sequence

<220>
<223> Primers

<400> 1580
caratgcagc tggtgcagtc t                                                21

<210> 1581
<211> 20
<212> DNA
<213> Artificial Sequence

<220>
<223> Primers

<400> 1581
gargtgcagc tggtgsagtc                                                  20

<210> 1582
<211> 21
<212> DNA
<213> Artificial Sequence

<220>
<223> Primers

<400> 1582
cagatcacct tgaaggagtc t                                                21

<210> 1583
<211> 21
<212> DNA
<213> Artificial Sequence

<220>
<223> Primers

<400> 1583
caggtscagc tggtrsagtc t                                                21

<210> 1584
<211> 21
<212> DNA
<213> Artificial Sequence

<220>
<223> Primers

<400> 1584
caggtacagc tgcagcagtc a                                                21

<210> 1585
<211> 21
<212> DNA
<213> Artificial Sequence

<220>
<223> Primers

<400> 1585
caggtgcagc tacagcagtg g                                                21

<210> 1586
<211> 21
<212> DNA
<213> Artificial Sequence

<220>
<223> Primers

<400> 1586
tgaggagacr gtgaccaggg t                                                21

<210> 1587
<211> 21
<212> DNA
<213> Artificial Sequence

<220>
<223> Primers

<400> 1587
tgargagacg gtgaccrtkg t                                                21

<210> 1588
<211> 21

<212> DNA
<213> Artificial Sequence

<220>
<223> Primers

<400> 1588
tgaggagacg gtgaccaggg t                                                21

<210> 1589
<211> 46
<212> DNA
<213> Artificial Sequence

<220>
<223> Primers

<400> 1589
ggtcgttcca ttttactccc actccgatgt tgtgatgacw cagtct                     46

<210> 1590
<211> 46
<212> DNA
<213> Artificial Sequence

<220>
<223> Primers

<400> 1590
ggtcgttcca ttttactccc actccgacat ccagatgayc cagtct                     46

<210> 1591
<211> 46
<212> DNA
<213> Artificial Sequence

<220>
<223> Primers

<400> 1591
ggtcgttcca ttttactccc actccgccat ccagwtgacc cagtct                     46

<210> 1592
<211> 46
<212> DNA
<213> Artificial Sequence

<220>
<223> Primers

<400> 1592
ggtcgttcca ttttactccc actccgaaat agtgatgayg cagtct                     46

<210> 1593
<211> 46
<212> DNA
<213> Artificial Sequence

<220>
<223> Primers

<400> 1593
ggtcgttcca ttttactccc actccgaaat tgtgttgacr cagtct                     46

<210> 1594
<211> 46
<212> DNA
<213> Artificial Sequence

<220>
<223> Primers

<400> 1594
ggtcgttcca ttttactccc actccgakat tgtgatgacc cagact 46

<210> 1595
<211> 46
<212> DNA
<213> Artificial Sequence

<220>
<223> Primers

<400> 1595
ggtcgttcca ttttactccc actccgaaat tgtrmtgacw cagtct 46

<210> 1596
<211> 46
<212> DNA
<213> Artificial Sequence

<220>
<223> Primers

<400> 1596
ggtcgttcca ttttactccc actccgayat ygtgatgacy cagtct 46

<210> 1597
<211> 46
<212> DNA
<213> Artificial Sequence

<220>
<223> Primers

<400> 1597
ggtcgttcca ttttactccc actccgaaac gacactcacg cagtct 46

<210> 1598
<211> 46
<212> DNA
<213> Artificial Sequence

<220>
<223> Primers

<400> 1598
ggtcgttcca ttttactccc actccgacat ccagttgacc cagtct 46

<210> 1599
<211> 46
<212> DNA
<213> Artificial Sequence

<220>
<223> Primers

<400> 1599
ggtcgttcca ttttactccc actccaacat ccagatgacc cagtct 46

<210> 1600
<211> 46
<212> DNA
<213> Artificial Sequence

<220>

<223> Primers

<400> 1600
ggtcgttcca ttttactccc actccgccat ccggatgacc cagtct 46

<210> 1601
<211> 46
<212> DNA
<213> Artificial Sequence

<220>
<223> Primers

<400> 1601
ggtcgttcca ttttactccc actccgtcat ctggatgacc cagtct 46

<210> 1602
<211> 38
<212> DNA
<213> Artificial Sequence

<220>
<223> Primers

<400> 1602
taatactttg gctggccctg caggagatgg aggccggc 38

<210> 1603
<211> 40
<212> DNA
<213> Artificial Sequence

<220>
<223> Primers

<400> 1603
taatactttg gctggccctg caggagaggg tgrctctttc 40

<210> 1604
<211> 40
<212> DNA
<213> Artificial Sequence

<220>
<223> Primers

<400> 1604
taatactttg gctggcccta caastgatgg tgactctgtc 40

<210> 1605
<211> 40
<212> DNA
<213> Artificial Sequence

<220>
<223> Primers

<400> 1605
taatactttg gctggccctg aaggagatgg aggccggctg 40

<210> 1606
<211> 40
<212> DNA
<213> Artificial Sequence

<220>
<223> Primers

<400> 1606
taatactttg gctggccctg caggagatgg aggcctgctc                                40

<210> 1607
<211> 40
<212> DNA
<213> Artificial Sequence

<220>
<223> Primers

<400> 1607
taatactttg gctggccctg caggagatgt tgactttgtc                                40

<210> 1608
<211> 40
<212> DNA
<213> Artificial Sequence

<220>
<223> Primers

<400> 1608
taatactttg gctggccctg caggtgatgg tgactttctc                                40

<210> 1609
<211> 40
<212> DNA
<213> Artificial Sequence

<220>
<223> Primers

<400> 1609
taatactttg gctggccctg cagttgatgg tggccctctc                                40

<210> 1610
<211> 40
<212> DNA
<213> Artificial Sequence

<220>
<223> Primers

<400> 1610
taatactttg gctggccctg caagtgatgg tgactctgtc                                40

<210> 1611
<211> 40
<212> DNA
<213> Artificial Sequence

<220>
<223> Primers

<400> 1611
taatactttg gctggccctg caaatgatac tgactctgtc                                40

<210> 1612
<211> 50
<212> DNA
<213> Artificial Sequence

<220>
<223> Primers

<400> 1612
ccagccaaag tattagcaac aacctacact ggyttcagca gaggccaggc                     50

<210> 1613
<211> 50
<212> DNA
<213> Artificial Sequence

<220>
<223> Primers

<400> 1613
ccagccaaag tattagcaac aacctacact ggtacctgca gaagccaggs 50

<210> 1614
<211> 50
<212> DNA
<213> Artificial Sequence

<220>
<223> Primers

<400> 1614
ccagccaaag tattagcaac aacctacact ggtatcrgca gaaaccaggg 50

<210> 1615
<211> 50
<212> DNA
<213> Artificial Sequence

<220>
<223> Primers

<400> 1615
ccagccaaag tattagcaac aacctacact ggtaccarca gaaaccagga 50

<210> 1616
<211> 50
<212> DNA
<213> Artificial Sequence

<220>
<223> Primers

<400> 1616
ccagccaaag tattagcaac aacctacact ggtaccarca gaaacctggc 50

<210> 1617
<211> 50
<212> DNA
<213> Artificial Sequence

<220>
<223> Primers

<400> 1617
ccagccaaag tattagcaac aacctacact ggtaycwgca gaaaccwggg 50

<210> 1618
<211> 50
<212> DNA
<213> Artificial Sequence

<220>
<223> Primers

<400> 1618
ccagccaaag tattagcaac aacctacact ggtatcagca raaaccwggs 50

<210> 1619

<211> 48
<212> DNA
<213> Artificial Sequence

<220>
<223> Primers

<400> 1619
ccagccaaag tattagcaac aacctacact ggtaycagca raaaccag                48

<210> 1620
<211> 50
<212> DNA
<213> Artificial Sequence

<220>
<223> Primers

<400> 1620
ccagccaaag tattagcaac aacctacact ggtttctgca gaaagccagg              50

<210> 1621
<211> 50
<212> DNA
<213> Artificial Sequence

<220>
<223> Primers

<400> 1621
ccagccaaag tattagcaac aacctacact ggtttcagca gaaaccaggg              50

<210> 1622
<211> 38
<212> DNA
<213> Artificial Sequence

<220>
<223> Primers

<400> 1622
gatggactgg aaaacataat agatcaggag ctgtggag                           38

<210> 1623
<211> 39
<212> DNA
<213> Artificial Sequence

<220>
<223> Primers

<400> 1623
gatggactgg aaaacataat agatcaggag cttaggrgc                          39

<210> 1624
<211> 39
<212> DNA
<213> Artificial Sequence

<220>
<223> Primers

<400> 1624
gatggactgg aaaacataat agatgaggag cctgggmgc                          39

<210> 1625
<211> 39
<212> DNA

<213> Artificial Sequence

<220>
<223> Primers

<400> 1625
gatggactgg aaaacatart agatcaggmg cttaggggc                                39

<210> 1626
<211> 39
<212> DNA
<213> Artificial Sequence

<220>
<223> Primers

<400> 1626
gatggactgg aaaacataat agatcaggwg cttaggrac                                39

<210> 1627
<211> 39
<212> DNA
<213> Artificial Sequence

<220>
<223> Primers

<400> 1627
gatggactgg aaaacataat agatgaagag cttaggggc                                39

<210> 1628
<211> 39
<212> DNA
<213> Artificial Sequence

<220>
<223> Primers

<400> 1628
gatggactgg aaaacataat aaattaggag tcttggagg                                39

<210> 1629
<211> 39
<212> DNA
<213> Artificial Sequence

<220>
<223> Primers

<400> 1629
gatggactgg aaaacatagt aaatgagcag cttaggagg                                39

<210> 1630
<211> 39
<212> DNA
<213> Artificial Sequence

<220>
<223> Primers

<400> 1630
gatggactgg aaaacataat agatcaggag tgtggagac                                39

<210> 1631
<211> 39
<212> DNA
<213> Artificial Sequence

<220>
<223> Primers

<400> 1631
gatggactgg aaaacataat agatcaggag ctcaggggc 39

<210> 1632
<211> 39
<212> DNA
<213> Artificial Sequence

<220>
<223> Primers

<400> 1632
gatggactgg aaaacataat agatcaggga cttaggggc 39

<210> 1633
<211> 39
<212> DNA
<213> Artificial Sequence

<220>
<223> Primers

<400> 1633
gatggactgg aaaacataat agaggaagag cttagggga 39

<210> 1634
<211> 39
<212> DNA
<213> Artificial Sequence

<220>
<223> Primers

<400> 1634
gatggactgg aaaacatact tgatgaggag ctttggaga 39

<210> 1635
<211> 39
<212> DNA
<213> Artificial Sequence

<220>
<223> Primers

<400> 1635
gatggactgg aaaacataat aaattaggcg ccttggaga 39

<210> 1636
<211> 39
<212> DNA
<213> Artificial Sequence

<220>
<223> Primers

<400> 1636
gatggactgg aaaacatact tgatgaggag ctttggggc 39

<210> 1637
<211> 39
<212> DNA
<213> Artificial Sequence

<220>
<223> Primers

<400> 1637
gatggactgg aaaacatatt gaataatgaa aatagcagc 39

<210> 1638
<211> 39
<212> DNA
<213> Artificial Sequence

<220>
<223> Primers

<400> 1638
gttttccagt ccatctctgg ggtcccagac agattcagy 39

<210> 1639
<211> 39
<212> DNA
<213> Artificial Sequence

<220>
<223> Primers

<400> 1639
gttttccagt ccatctctgg ggtcccatca aggttcagy 39

<210> 1640
<211> 49
<212> DNA
<213> Artificial Sequence

<220>
<223> Primers

<400> 1640
gctggtggtg ccgttctata gccatagcca ggtkcagctg gtgcagtct 49

<210> 1641
<211> 49
<212> DNA
<213> Artificial Sequence

<220>
<223> Primers

<400> 1641
gctggtggtg ccgttctata gccatagcga ggtgcagctg ktggagtct 49

<210> 1642
<211> 49
<212> DNA
<213> Artificial Sequence

<220>
<223> Primers

<400> 1642
gctggtggtg ccgttctata gccatagcca gstgcagctg caggagtcg 49

<210> 1643
<211> 49
<212> DNA
<213> Artificial Sequence

<220>
<223> Primers

<400> 1643

gctggtggtg ccgttctata gccatagcca ggtcaccttg arggagtct 49

<210> 1644
<211> 49
<212> DNA
<213> Artificial Sequence

<220>
<223> Primers

<400> 1644
gctggtggtg ccgttctata gccatagcca ratgcagctg gtgcagtct 49

<210> 1645
<211> 48
<212> DNA
<213> Artificial Sequence

<220>
<223> Primers

<400> 1645
gctggtggtg ccgttctata gccatagcga rgtgcagctg gtgsagtc 48

<210> 1646
<211> 49
<212> DNA
<213> Artificial Sequence

<220>
<223> Primers

<400> 1646
gctggtggtg ccgttctata gccatagcca gatcaccttg aaggagtct 49

<210> 1647
<211> 49
<212> DNA
<213> Artificial Sequence

<220>
<223> Primers

<400> 1647
gctggtggtg ccgttctata gccatagcca ggtscagctg gtrsagtct 49

<210> 1648
<211> 49
<212> DNA
<213> Artificial Sequence

<220>
<223> Primers

<400> 1648
gctggtggtg ccgttctata gccatagcca ggtacagctg cagcagtca 49

<210> 1649
<211> 49
<212> DNA
<213> Artificial Sequence

<220>
<223> Primers

<400> 1649
gctggtggtg ccgttctata gccatagcca ggtgcagcta cagcagtgg 49

<210> 1650
<211> 36
<212> DNA
<213> Artificial Sequence

<220>
<223> Primers

<400> 1650
gttcatggag taatcrgtga aggtgtatcc agaagc                                36

<210> 1651
<211> 36
<212> DNA
<213> Artificial Sequence

<220>
<223> Primers

<400> 1651
gttcatggag taatcgctga gtgagaaccc agagam                                36

<210> 1652
<211> 36
<212> DNA
<213> Artificial Sequence

<220>
<223> Primers

<400> 1652
gttcatggag taatcactga argtgaatcc agaggc                                36

<210> 1653
<211> 36
<212> DNA
<213> Artificial Sequence

<220>
<223> Primers

<400> 1653
gttcatggag taatcactga cggtgaaycc agaggc                                36

<210> 1654
<211> 36
<212> DNA
<213> Artificial Sequence

<220>
<223> Primers

<400> 1654
gttcatggag taatcgctga yggagccacc agagac                                36

<210> 1655
<211> 36
<212> DNA
<213> Artificial Sequence

<220>
<223> Primers

<400> 1655
gttcatggag taatcrgtaa aggtgwawcc agaagc                                36

<210> 1656
<211> 36

<212> DNA
<213> Artificial Sequence

<220>
<223> Primers

<400> 1656
gttcatggag taatcactra aggtgaaycc agaggc                                36

<210> 1657
<211> 36
<212> DNA
<213> Artificial Sequence

<220>
<223> Primers

<400> 1657
gttcatggag taatcggtra arctgtawcc agaasc                                36

<210> 1658
<211> 36
<212> DNA
<213> Artificial Sequence

<220>
<223> Primers

<400> 1658
gttcatggag taatcaycaa aggtgaatcc agargc                                36

<210> 1659
<211> 36
<212> DNA
<213> Artificial Sequence

<220>
<223> Primers

<400> 1659
gttcatggag taatcrctra aggtgaatcc agasgc                                36

<210> 1660
<211> 36
<212> DNA
<213> Artificial Sequence

<220>
<223> Primers

<400> 1660
gttcatggag taatcggtga aggtgtatcc rgawgc                                36

<210> 1661
<211> 36
<212> DNA
<213> Artificial Sequence

<220>
<223> Primers

<400> 1661
gttcatggag taatcactga aggacccacc atagac                                36

<210> 1662
<211> 36
<212> DNA
<213> Artificial Sequence

<220>
<223> Primers

<400> 1662
gttcatggag taatcactga tggagccacc agagac                                36

<210> 1663
<211> 36
<212> DNA
<213> Artificial Sequence

<220>
<223> Primers

<400> 1663
gttcatggag taatcgctga tggagtaacc agagac                                36

<210> 1664
<211> 36
<212> DNA
<213> Artificial Sequence

<220>
<223> Primers

<400> 1664
gttcatggag taatcagtga gggtgtatcc ggaaac                                36

<210> 1665
<211> 36
<212> DNA
<213> Artificial Sequence

<220>
<223> Primers

<400> 1665
gttcatggag taatcgctga aggtgcctcc agaagc                                36

<210> 1666
<211> 36
<212> DNA
<213> Artificial Sequence

<220>
<223> Primers

<400> 1666
gttcatggag taatcagaga cactgtcccc ggagat                                36

<210> 1667
<211> 36
<212> DNA
<213> Artificial Sequence

<220>
<223> Primers

<400> 1667
gattactcca tgaactgggt gcgacaggcy cctgga                                36

<210> 1668
<211> 36
<212> DNA
<213> Artificial Sequence

<220>

<223> Primers

<400> 1668
gattactcca tgaactgggt gcgmcaggcc cccgga                                36

<210> 1669
<211> 36
<212> DNA
<213> Artificial Sequence

<220>
<223> Primers

<400> 1669
gattactcca tgaactggat ccgtcagccc ccaggr                                36

<210> 1670
<211> 36
<212> DNA
<213> Artificial Sequence

<220>
<223> Primers

<400> 1670
gattactcca tgaactggrt ccgccaggct ccaggg                                36

<210> 1671
<211> 36
<212> DNA
<213> Artificial Sequence

<220>
<223> Primers

<400> 1671
gattactcca tgaactggat ccgscagccc ccaggg                                36

<210> 1672
<211> 36
<212> DNA
<213> Artificial Sequence

<220>
<223> Primers

<400> 1672
gattactcca tgaactgggt ccgscaagct ccaggg                                36

<210> 1673
<211> 36
<212> DNA
<213> Artificial Sequence

<220>
<223> Primers

<400> 1673
gattactcca tgaactgggt ccrtcargct ccrggr                                36

<210> 1674
<211> 36
<212> DNA
<213> Artificial Sequence

<220>
<223> Primers

<400> 1674
gattactcca tgaactgggt scgmcargcy acwgga                                36

<210> 1675
<211> 36
<212> DNA
<213> Artificial Sequence

<220>
<223> Primers

<400> 1675
gattactcca tgaactggkt ccgccaggct ccaggs                                36

<210> 1676
<211> 36
<212> DNA
<213> Artificial Sequence

<220>
<223> Primers

<400> 1676
gattactcca tgaactggat caggcagtcc ccatcg                                36

<210> 1677
<211> 36
<212> DNA
<213> Artificial Sequence

<220>
<223> Primers

<400> 1677
gattactcca tgaactgggc ccgcaaggct ccagga                                36

<210> 1678
<211> 36
<212> DNA
<213> Artificial Sequence

<220>
<223> Primers

<400> 1678
gattactcca tgaactggat ccgccagcac ccaggg                                36

<210> 1679
<211> 36
<212> DNA
<213> Artificial Sequence

<220>
<223> Primers

<400> 1679
gattactcca tgaactgggt ccgccaggct tccggg                                36

<210> 1680
<211> 36
<212> DNA
<213> Artificial Sequence

<220>
<223> Primers

<400> 1680
gattactcca tgaactgggt gcgccagatg cccggg                                36

<210> 1681
<211> 36
<212> DNA
<213> Artificial Sequence

<220>
<223> Primers

<400> 1681
gattactcca tgaactgggt gcgacaggct cgtgga                                36

<210> 1682
<211> 36
<212> DNA
<213> Artificial Sequence

<220>
<223> Primers

<400> 1682
gattactcca tgaactggat ccggcagccc gccggg                                36

<210> 1683
<211> 36
<212> DNA
<213> Artificial Sequence

<220>
<223> Primers

<400> 1683
gattactcca tgaactgggt gccacaggcc cctgga                                36

<210> 1684
<211> 51
<212> DNA
<213> Artificial Sequence

<220>
<223> Primers

<400> 1684
tgtgtaatca ttagctttgt ttctaataaa tcccatccac tcaagccytt g               51

<210> 1685
<211> 53
<212> DNA
<213> Artificial Sequence

<220>
<223> Primers

<400> 1685
tgttgtgtaa tcattagctt tgtttctaat aaatcccatc cactcaagcs ctt             53

<210> 1686
<211> 54
<212> DNA
<213> Artificial Sequence

<220>
<223> Primers

<400> 1686
tgttgtgtaa tcattagctt tgtttctaat aaawgagacc cactccagcc cctt            54

<210> 1687

<211> 54
<212> DNA
<213> Artificial Sequence

<220>
<223> Primers

<400> 1687
tgttgtgtaa tcattagctt tgtttctaat aaacccaatc cactccagkc cctt          54

<210> 1688
<211> 54
<212> DNA
<213> Artificial Sequence

<220>
<223> Primers

<400> 1688
tgttgtgtaa tcattagctt tgtttctaat aaatgagacc cactccagrc cctt          54

<210> 1689
<211> 54
<212> DNA
<213> Artificial Sequence

<220>
<223> Primers

<400> 1689
tgttgtgtaa tcattagctt tgtttctaat aaagccaacc cactccagcc cytt          54

<210> 1690
<211> 54
<212> DNA
<213> Artificial Sequence

<220>
<223> Primers

<400> 1690
tgttgtgtaa tcattagctt tgtttctaat aaakgccacc cactccagcc cctt          54

<210> 1691
<211> 53
<212> DNA
<213> Artificial Sequence

<220>
<223> Primers

<400> 1691
tgttgtgtaa tcattagctt tgtttctaat aaatcccagc cactcaaggc ctc          53

<210> 1692
<211> 53
<212> DNA
<213> Artificial Sequence

<220>
<223> Primers

<400> 1692
tgttgtgtaa tcattagctt tgtttctaat aaaccccatc cactccaggc ctt          53

<210> 1693
<211> 54
<212> DNA

<213> Artificial Sequence

<220>
<223> Primers

<400> 1693
tgttgtgtaa tcattagctt tgtttctaat aaatgaracc cacwccagcc cctt        54

<210> 1694
<211> 54
<212> DNA
<213> Artificial Sequence

<220>
<223> Primers

<400> 1694
tgttgtgtaa tcattagctt tgtttctaat aaamgakacc cactccagmc cctt        54

<210> 1695
<211> 54
<212> DNA
<213> Artificial Sequence

<220>
<223> Primers

<400> 1695
tgttgtgtaa tcattagctt tgtttctaat aaayccmatc cactcmagcc cytt        54

<210> 1696
<211> 54
<212> DNA
<213> Artificial Sequence

<220>
<223> Primers

<400> 1696
tgttgtgtaa tcattagctt tgtttctaat aaatcctatc cactcaaggc gttg        54

<210> 1697
<211> 54
<212> DNA
<213> Artificial Sequence

<220>
<223> Primers

<400> 1697
tgttgtgtaa tcattagctt tgtttctaat aaatgcaagc cactccaggg cctt        54

<210> 1698
<211> 54
<212> DNA
<213> Artificial Sequence

<220>
<223> Primers

<400> 1698
tgttgtgtaa tcattagctt tgtttctaat aaatgaaaca tattccagtc cctt        54

<210> 1699
<211> 54
<212> DNA
<213> Artificial Sequence

<220>
<223> Primers

<400> 1699
tgttgtgtaa tcattagctt tgtttctaat aaacgatacc cactccagcc cctt 54

<210> 1700
<211> 63
<212> DNA
<213> Artificial Sequence

<220>
<223> Primers

<400> 1700
gctaatgatt acacaacaga gtacagtgca tctgtgaagg gtagagtcac catgaccagg 60
rac 63

<210> 1701
<211> 63
<212> DNA
<213> Artificial Sequence

<220>
<223> Primers

<400> 1701
gctaatgatt acacaacaga gtacagtgca tctgtgaagg gtaggctcac catcwccaag 60
gac 63

<210> 1702
<211> 63
<212> DNA
<213> Artificial Sequence

<220>
<223> Primers

<400> 1702
gctaatgatt acacaacaga gtacagtgca tctgtgaagg gtcgagtyac catatcagta 60
gac 63

<210> 1703
<211> 63
<212> DNA
<213> Artificial Sequence

<220>
<223> Primers

<400> 1703
gctaatgatt acacaacaga gtacagtgca tctgtgaagg gtcgattcac catctccagr 60
gac 63

<210> 1704
<211> 62
<212> DNA
<213> Artificial Sequence

<220>
<223> Primers

<400> 1704
gctaatgatt acacaacaga gtacagtgca tctgtgaagg gtagattcac catctcmaga 60
ga 62

<210> 1705
<211> 62

<212> DNA
<213> Artificial Sequence

<220>
<223> Primers

<400> 1705
gctaatgatt acacaacaga gtacagtgca tctgtgaagg gtmggttcac catctccaga 60
ga 62

<210> 1706
<211> 62
<212> DNA
<213> Artificial Sequence

<220>
<223> Primers

<400> 1706
gctaatgatt acacaacaga gtacagtgca tctgtgaagg gtcgattcay catctccaga 60
ga 62

<210> 1707
<211> 63
<212> DNA
<213> Artificial Sequence

<220>
<223> Primers

<400> 1707
gctaatgatt acacaacaga gtacagtgca tctgtgaagg gtcgagtcac catrtcmgta 60
gac 63

<210> 1708
<211> 63
<212> DNA
<213> Artificial Sequence

<220>
<223> Primers

<400> 1708
gctaatgatt acacaacaga gtacagtgca tctgtgaagg gtagrgtcac catkaccagg 60
gac 63

<210> 1709
<211> 63
<212> DNA
<213> Artificial Sequence

<220>
<223> Primers

<400> 1709
gctaatgatt acacaacaga gtacagtgca tctgtgaagg gtcaggtcac catctcagcc 60
gac 63

<210> 1710
<211> 63
<212> DNA
<213> Artificial Sequence

<220>
<223> Primers

<400> 1710
gctaatgatt acacaacaga gtacagtgca tctgtgaagg gtcgaataac catcaaccca 60

gac                                                                 63

<210> 1711
<211> 62
<212> DNA
<213> Artificial Sequence

<220>
<223> Primers

<400> 1711
ctaatgatta cacaacagag tacagtgcat ctgtgaaggg tcggtttgtc ttctccatgg 60
ac                                                                  62

<210> 1712
<211> 63
<212> DNA
<213> Artificial Sequence

<220>
<223> Primers

<400> 1712
gctaatgatt acacaacaga gtacagtgca tctgtgaagg gtagagtcac catgaccgag 60
gac                                                                 63

<210> 1713
<211> 63
<212> DNA
<213> Artificial Sequence

<220>
<223> Primers

<400> 1713
gctaatgatt acacaacaga gtacagtgca tctgtgaagg gtagagtcac gattaccgcg 60
gac                                                                 63

<210> 1714
<211> 63
<212> DNA
<213> Artificial Sequence

<220>
<223> Primers

<400> 1714
gctaatgatt acacaacaga gtacagtgca tctgtgaagg gtagagtcac catgaccaca 60
gac                                                                 63

<210> 1715
<211> 45
<212> DNA
<213> Artificial Sequence

<220>
<223> Primers

<400> 1715
gtccatagca tgatacctag ggtatctagy acagtaatac acggc                   45

<210> 1716
<211> 45
<212> DNA
<213> Artificial Sequence

<220>
<223> Primers

<400> 1716
gtccatagca tgatacctag ggtatctcgc acagtaatac ayggc 45

<210> 1717
<211> 45
<212> DNA
<213> Artificial Sequence

<220>
<223> Primers

<400> 1717
gtccatagca tgatacctag ggtatctygc acagtaatac acagc 45

<210> 1718
<211> 45
<212> DNA
<213> Artificial Sequence

<220>
<223> Primers

<400> 1718
gtccatagca tgatacctag ggtatgyygc acagtaatac acggc 45

<210> 1719
<211> 45
<212> DNA
<213> Artificial Sequence

<220>
<223> Primers

<400> 1719
gtccatagca tgatacctag ggtaccgtgc acartaatay gtggc 45

<210> 1720
<211> 45
<212> DNA
<213> Artificial Sequence

<220>
<223> Primers

<400> 1720
gtccatagca tgatacctag ggtatctggc acagtaatac acggc 45

<210> 1721
<211> 45
<212> DNA
<213> Artificial Sequence

<220>
<223> Primers

<400> 1721
gtccatagca tgatacctag ggtatgtggt acagtaatac acggc 45

<210> 1722
<211> 45
<212> DNA
<213> Artificial Sequence

<220>
<223> Primers

<400> 1722

gtccatagca tgatacctag ggtatctcgc acagtgatac aaggc 45

<210> 1723
<211> 45
<212> DNA
<213> Artificial Sequence

<220>
<223> Primers

<400> 1723
gtccatagca tgatacctag ggtattttgc acagtaatac aaggc 45

<210> 1724
<211> 45
<212> DNA
<213> Artificial Sequence

<220>
<223> Primers

<400> 1724
gtccatagca tgatacctag ggtatcttgc acagtaatac atggc 45

<210> 1725
<211> 45
<212> DNA
<213> Artificial Sequence

<220>
<223> Primers

<400> 1725
gtccatagca tgatacctag ggtagtgtgc acagtaatat gtggc 45

<210> 1726
<211> 45
<212> DNA
<213> Artificial Sequence

<220>
<223> Primers

<400> 1726
gtccatagca tgatacctag ggtatttcgc acagtaatat acggc 45

<210> 1727
<211> 45
<212> DNA
<213> Artificial Sequence

<220>
<223> Primers

<400> 1727
gtccatagca tgatacctag ggtatctcac acagtaatac acagc 45

<210> 1728
<211> 45
<212> DNA
<213> Artificial Sequence

<220>
<223> Primers

<400> 1728
cctaggtatc atgctatgga ctcctggggc carggmaccc tggtc 45

<210> 1729
<211> 45
<212> DNA
<213> Artificial Sequence

<220>
<223> Primers

<400> 1729
cctaggtatc atgctatgga ctcctggggs caagggacma yggtc                45

<210> 1730
<211> 45
<212> DNA
<213> Artificial Sequence

<220>
<223> Primers

<400> 1730
cctaggtatc atgctatgga ctcctggggc cgtggcaccc tggtc                45

<210> 1731
<211> 49
<212> DNA
<213> Artificial Sequence

<220>
<223> Primers

<400> 1731
ggaagaccga tgggcccttg gtggaggctg aggagacrgt gaccagggt            49

<210> 1732
<211> 49
<212> DNA
<213> Artificial Sequence

<220>
<223> Primers

<400> 1732
ggaagaccga tgggcccttg gtggaggctg argagacggt gaccrtkgt            49

<210> 1733
<211> 49
<212> DNA
<213> Artificial Sequence

<220>
<223> Primers

<400> 1733
ggaagaccga tgggcccttg gtggaggctg aggagacggt gaccagggt            49

<210> 1734
<211> 22
<212> DNA
<213> Artificial Sequence

<220>
<223> Primers

<400> 1734
ggtcgttcca ttttactccc ac                                         22

<210> 1735
<211> 23

<212> DNA
<213> Artificial Sequence

<220>
<223> Primers

<400> 1735
gctggtggtg ccgttctata gcc                                               23

<210> 1736
<211> 48
<212> DNA
<213> Artificial Sequence

<220>
<223> Primers

<400> 1736
ggtcgttcca ttttactccc actccgccat ccagttgact cagtctcc                    48

<210> 1737
<211> 54
<212> DNA
<213> Artificial Sequence

<220>
<223> Primers

<400> 1737
gatgaagaca gatggtgcag ccacagtacg tttgatctcc agcttggtcc ctcc             54

<210> 1738
<211> 50
<212> DNA
<213> Artificial Sequence

<220>
<223> Primers

<400> 1738
ggtcgttcca ttttactccc actccgaaat tgtgttgaca cagtctccag                  50

<210> 1739
<211> 53
<212> DNA
<213> Artificial Sequence

<220>
<223> Primers

<400> 1739
gatgaagaca gatggtgcag ccacagtacg tttgatatcc actttggtcc ctc              53

<210> 1740
<211> 56
<212> DNA
<213> Artificial Sequence

<220>
<223> Primers

<400> 1740
gctggtggtg ccgttctata gccatagcga ggtgaagctg gtggagtctg gaggag           56

<210> 1741
<211> 55
<212> DNA
<213> Artificial Sequence

<220>
<223> Primers

<400> 1741
ggaagaccga tgggcccttg gtggaggctg aggagacggt gactgaggtt ccttg 55

<210> 1742
<211> 58
<212> DNA
<213> Artificial Sequence

<220>
<223> Primers

<400> 1742
ggtcgttcca ttttactccc actccgatat tgtgctaact cagtctccag ccaccctg 58

<210> 1743
<211> 62
<212> DNA
<213> Artificial Sequence

<220>
<223> Primers

<400> 1743
gatgaagaca gatggtgcag ccacagtacg tttcagctcc agcttggtcc cagcaccgaa 60
cg 62

<210> 1744
<211> 39
<212> DNA
<213> Artificial Sequence

<220>
<223> Primers

<400> 1744
gttttccagt ccatctctgg yatcccagcc aggttcagt 39

<210> 1745
<211> 39
<212> DNA
<213> Artificial Sequence

<220>
<223> Primers

<400> 1745
gttttccagt ccatctctgg rgtcccwgac aggttcagt 39

<210> 1746
<211> 39
<212> DNA
<213> Artificial Sequence

<220>
<223> Primers

<400> 1746
gttttccagt ccatctctag catcccagcc aggttcagt 39

<210> 1747
<211> 39

<212>  DNA
<213>  Artificial Sequence

<220>
<223>  Primers

<400>  1747
gttttccagt ccatctctgg ggtcccctcg aggttcagt                              39


<210>  1748
<211>  39
<212>  DNA
<213>  Artificial Sequence

<220>
<223>  Primers

<400>  1748
gttttccagt ccatctctgg aatcccacct cgattcagt                              39


<210>  1749
<211>  39
<212>  DNA
<213>  Artificial Sequence

<220>
<223>  Primers

<400>  1749
gttttccagt ccatctctgg ggtccctgac cgattcagt                              39


<210>  1750
<211>  39
<212>  DNA
<213>  Artificial Sequence

<220>
<223>  Primers

<400>  1750
gttttccagt ccatctctgg catcccagac aggttcagt                              39


<210>  1751
<211>  39
<212>  DNA
<213>  Artificial Sequence

<220>
<223>  Primers

<400>  1751
gttttccagt ccatctctgg ggtctcatcg aggttcagt                              39


<210>  1752
<211>  39
<212>  DNA
<213>  Artificial Sequence

<220>
<223>  Primers

<400>  1752
gttttccagt ccatctctgg agtgccagat aggttcagt                              39

<210>  1753
<211>  39
<212>  DNA
<213>  Artificial Sequence

<220>
<223>  Primers

<400>  1753
ccagctgtta ctctgttgkc agtaataaac cccaacatc                              39


<210>  1754
<211>  39
<212>  DNA
<213>  Artificial Sequence

<220>
<223>  Primers

<400>  1754
ccagctgtta ctctgttgac agtaataygt tgcagcatc                              39


<210>  1755
<211>  39
<212>  DNA
<213>  Artificial Sequence

<220>
<223>  Primers

<400>  1755
ccagctgtta ctctgttgac mgtaataagt tgcaacatc                              39


<210>  1756
<211>  39
<212>  DNA
<213>  Artificial Sequence

<220>
<223>  Primers

<400> 1756
ccagctgtta ctctgttgrc agtaataagt tgcaaaatc                              39


<210>  1757
<211>  39
<212>  DNA
<213>  Artificial Sequence

<220>
<223>  Primers

<400>  1757
ccagctgtta ctctgttgac agtaataarc tgcaaaatc                              39


<210>  1758
<211>  39
<212>  DNA
<213>  Artificial Sequence

<220>

<223>  Primers

<400>  1758
ccagctgtta ctctgttgac artagtaagt tgcaaaatc                              39


<210>  1759
<211>  39
<212>  DNA
<213>  Artificial Sequence

<220>
<223>  Primers

<400>  1759
ccagctgtta ctctgttggc agtaataaac tccaamatc                              39


<210>  1760
<211>  39
<212>  DNA
<213>  Artificial Sequence

<220>
<223>  Primers

<400>  1760
ccagctgtta ctctgttggc agtaataaac cccgacatc                              39


<210>  1761
<211>  39
<212>  DNA
<213>  artificial Sequence

<220>
<223>  Primers

<400>  1761
ccagctgtta ctctgttgac agaagtaata tgcagcatc                              39


<210>  1762
<211>  39
<212>  DNA
<213>  Artificial Sequence

<220>
<223>  Primers

<400>  1762
ccagctgtta ctctgttgac agtaatatgt tgcaatatc                              39


<210>  1763
<211>  39
<212>  DNA
<213>  Artificial Sequence

<220>
<223>  Primers

<400>  1763
ccagctgtta ctctgttgac agtaatacac tgcaaaatc                              39


<210>  1764
<211>  39

<212>  DNA
<213>  Artificial Sequence

<220>
<223>  Primers

<400>  1764
ccagctgtta ctctgttgac agtaataaac tgccacatc                              39


<210>  1765
<211>  45
<212>  DNA
<213>  Artificial Sequence

<220>
<223>  Primers

<400>  1765
cagagtaaca gctggccgct cacgttyggc cargggacca agstg                       45


<210>  1766
<211>  45
<212>  DNA
<213>  Artificial Sequence

<220>
<223>  Primers

<400>  1766
cagagtaaca gctggccgct cacgttcggc caagggacac gactg                       45


<210>  1767
<211>  45
<212>  DNA
<213>  Artificial Sequence

<220>
<223>  Primers

<400>  1767
cagagtaaca gctggccgct cacgttcggc cctgggacca aagtg                       45


<210>  1768
<211>  45
<212>  DNA
<213>  Artificial Sequence

<220>
<223>  Primers

<400>  1768
cagagtaaca gctggccgct cacgttcggc ggagggacca aggtg                       45


<210>  1769
<211>  47
<212>  DNA
<213>  Artificial Sequence

<220>
<223>  Primers

<400>  1769
gatgaagaca gatggtgcag ccacagtacg tttgatytcc accttgg                     47

<210>  1770
<211>  47
<212>  DNA
<213>  Artificial Sequence

<220>
<223>  Primers

<400>  1770
gatgaagaca gatggtgcag ccacagtacg tttgatctcc agcttgg                47


<210>  1771
<211>  47
<212>  DNA
<213>  Artificial Sequence

<220>
<223>  Primers

<400>  1771
gatgaagaca gatggtgcag ccacagtacg tttgatatcc actttgg                47


<210>  1772
<211>  47
<212>  DNA
<213>  Artificial Sequence

<220>
<223>  Primers

<400>  1772
gatgaagaca gatggtgcag ccacagtacg tttaatctcc agtcgtg                47


<210>  1773
<211>  360
<212>  DNA
<213>  Mus musculus

<400>  1773
gaggtgaagc tggtggagtc tggaggaggc ttggtacagc ctgggggttc tctgagtctc      60

tcctgtgcag cttctggatt caccttcact gattactcca tgaactgggt ccgccagcct     120

ccagggaagg cacttgagtg gttgggtttt attagaaaca aagctaatga ttacacaaca     180

gagtacagtg catctgtgaa gggtcggttc accatctcca gagataattc ccaaagcatc     240

ctctatcttc aaatgaatgc cctgagagct gaggacagtg ccacttatta ctgtgtaaga     300

taccctaggt atcatgctat ggactcctgg ggtcaaggaa cctcagtcac cgtctcctca     360


<210>  1774
<211>  321
<212>  DNA
<213>  Mus musculus

<400>  1774
gatattgtgc taactcagtc tccagccacc ctgtctgtga ctccaggaga tagcgtcaat      60

ctttcctgca gggccagcca aagtattagc aacaacctac actggtatca acaaaaatca     120

catgagtctc caaggcttct catcaagtat gttttccagt ccatctctgg gatcccctcc     180

aggttcagtg gcagtggatc agggacagat ttcactctca gtatcaacag tgtggagact 240

gaagattttg gaatgtattt ctgtcaacag agtaacagct ggccgctcac gttcggtgct 300

gggaccaagc tggagctgaa a 321

<210> 1775
<211> 120
<212> PRT
<213> Mus musculus

<400> 1775

Glu Val Lys Leu Val Glu Ser Gly Gly Gly Leu Val Gln Pro Gly Gly
1               5                   10                  15

Ser Leu Ser Leu Ser Cys Ala Ala Ser Gly Phe Thr Phe Thr Asp Tyr
            20                  25                  30

Ser Met Asn Trp Val Arg Gln Pro Pro Gly Lys Ala Leu Glu Trp Leu
        35                  40                  45

Gly Phe Ile Arg Asn Lys Ala Asn Asp Tyr Thr Thr Glu Tyr Ser Ala
    50                  55                  60

Ser Val Lys Gly Arg Phe Thr Ile Ser Arg Asp Asn Ser Gln Ser Ile
65                  70                  75                  80

Leu Tyr Leu Gln Met Asn Ala Leu Arg Ala Glu Asp Ser Ala Thr Tyr
                85                  90                  95

Tyr Cys Val Arg Tyr Pro Arg Tyr His Ala Met Asp Ser Trp Gly Gln
            100                 105                 110

Gly Thr Ser Val Thr Val Ser Ser
        115                 120

<210> 1776
<211> 107
<212> PRT
<213> Mus musculus

<400> 1776

Asp Ile Val Leu Thr Gln Ser Pro Ala Thr Leu Ser Val Thr Pro Gly
1               5                   10                  15

Asp Ser Val Asn Leu Ser Cys Arg Ala Ser Gln Ser Ile Ser Asn Asn
            20                  25                  30

Leu His Trp Tyr Gln Gln Lys Ser His Glu Ser Pro Arg Leu Leu Ile
        35                  40                  45

Lys Tyr Val Phe Gln Ser Ile Ser Gly Ile Pro Ser Arg Phe Ser Gly
    50                  55                  60

Ser Gly Ser Gly Thr Asp Phe Thr Leu Ser Ile Asn Ser Val Glu Thr
65                  70                  75                  80

Glu Asp Phe Gly Met Tyr Phe Cys Gln Gln Ser Asn Ser Trp Pro Leu
                85                  90                  95

Thr Phe Gly Ala Gly Thr Lys Leu Glu Leu Lys
            100                 105

<210>  1777
<211>  120
<212>  PRT
<213>  Artificial Sequence

<220>
<223>  VH region

<400>  1777

Glu Val Gln Leu Val Glu Ser Gly Gly Gly Val Val Arg Pro Gly Gly
1               5                   10                  15

Ser Leu Arg Leu Ser Cys Ala Ala Ser Gly Phe Thr Val Ser Asp Tyr
            20                  25                  30

Ser Met Asn Trp Val Arg Gln Ala Pro Gly Lys Gly Leu Glu Trp Ile
        35                  40                  45

Gly Phe Ile Arg Asn Lys Ala Asn Asp Tyr Thr Thr Glu Tyr Ser Ala
    50                  55                  60

Ser Val Lys Gly Arg Phe Thr Ile Ser Arg Asp Asp Ser Lys Asn Thr
65                  70                  75                  80

Leu Tyr Leu Gln Met Asn Ser Leu Lys Thr Glu Asp Thr Ala Val Tyr
                85                  90                  95

Tyr Cys Thr Thr Tyr Pro Arg Tyr His Ala Met Asp Ser Trp Gly Gln
            100                 105                 110

Gly Thr Met Val Thr Val Ser Ser
        115                 120

<210>  1778
<211>  120
<212>  PRT
<213>  Artificial Sequence

<220>
<223>  VH region

<400>  1778

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Ser Met Asn Trp Val Arg Gln Ala Pro Gly Lys Gly Leu Glu Trp Val
        35                  40                  45

Ala Phe Ile Arg Asn Lys Ala Asn Asp Tyr Thr Thr Glu Tyr Ser Ala
    50                  55                  60

Ser Val Lys Gly Arg Phe Thr Ile Ser Arg Asp Asn Ser Lys Asn Thr
65                  70                  75                  80

Leu Tyr Leu Gln Met Asn Ser Leu Arg Ala Glu Asp Thr Ala Met Tyr
                85                  90                  95

Tyr Cys Ala Arg Tyr Pro Arg Tyr His Ala Met Asp Ser Trp Gly Gln
            100                 105                 110

Gly Thr Leu Val Thr Val Ser Ser
        115                 120

<210> 1779
<211> 120
<212> PRT
<213> Artificial Sequence

<220>
<223> VH region

<400> 1779

Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Asp Tyr
            20                  25                  30

Ser Met Asn Trp Val Arg Gln Ala Ser Gly Lys Gly Leu Glu Trp Val
        35                  40                  45

Gly Phe Ile Arg Asn Lys Ala Asn Asp Tyr Thr Thr Glu Tyr Ser Ala
    50                  55                  60

Ser Val Lys Gly Arg Phe Thr Ile Ser Arg Asp Asp Ser Lys Asn Thr
65                  70                  75                  80

Leu Tyr Leu Gln Met Asn Ser Leu Lys Thr Glu Asp Thr Ala Val Tyr
                85                  90                  95

Tyr Cys Thr Thr Tyr Pro Arg Tyr His Ala Met Asp Ser Trp Gly Gln
            100                 105                 110

Gly Thr Leu Val Thr Val Ser Ser
        115                 120


<210>  1780
<211>  107
<212>  PRT
<213>  Artificial Sequence

<220>
<223>  VL region

<400>  1780

Ala Ile Gln Leu Thr Gln Ser Pro Ser Ser Leu Ser Ala Ser Val Gly
1               5                   10                  15

Asp Arg Val Thr Ile Thr Cys Arg Ala Ser Gln Ser Ile Ser Asn Asn
            20                  25                  30

Leu His Trp Tyr Leu Gln Lys Pro Gly Gln Ser Pro Gln Leu Leu Ile
        35                  40                  45

Tyr Tyr Val Phe Gln Ser Ile Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Asn Ser Trp Pro Leu
                85                  90                  95

Thr Phe Gly Gly Gly Thr Lys Leu Glu Ile Lys
            100                 105


<210>  1781
<211>  107
<212>  PRT
<213>  Artificial Sequence

<220>
<223>  VL region

<400>  1781

Glu Ile Val Leu Thr Gln Ser Pro Ala Thr Leu Ser Val Ser Pro Gly
1               5                   10                  15

Glu Arg Ala Thr Leu Ser Cys Arg Ala Ser Gln Ser Ile Ser Asn Asn
            20                  25                  30

Leu His Trp Tyr Gln Gln Lys Pro Gly Lys Ala Pro Lys Ser Leu Ile
        35                  40                  45

Tyr Tyr Val Phe Gln Ser Ile Ser Gly Val Pro Ser Arg Phe Ser Gly
    50                  55                  60

Ser Gly Ser Gly Thr Asp Phe Thr Leu Thr Ile Asn Ser Leu Glu Ala
65                  70                  75                  80

Glu Asp Ala Ala Thr Tyr Tyr Cys Gln Gln Ser Asn Ser Trp Pro Leu
                85                  90                  95

Thr Phe Gly Gly Gly Thr Lys Val Asp Ile Lys
            100                 105